(19) Europäisches Patentamt
European Patent Office
Office européen des brevets

(11) EP 4 596 547 A1

(12) **EUROPEAN PATENT APPLICATION**
published in accordance with Art. 153(4) EPC

(43) Date of publication:
**06.08.2025 Bulletin 2025/32**

(21) Application number: **23872612.9**

(22) Date of filing: **29.09.2023**

(51) International Patent Classification (IPC):
***C07D 471/04*** *(2006.01)* ***A01N 43/90*** *(2006.01)*
***A01P 7/02*** *(2006.01)* ***A01P 7/04*** *(2006.01)*
***A61K 31/437*** *(2006.01)* ***A61P 33/14*** *(2006.01)*

(52) Cooperative Patent Classification (CPC):
**A01N 43/90; A01P 7/02; A01P 7/04; A61K 31/437; A61P 33/14; C07D 471/04**

(86) International application number:
**PCT/JP2023/035679**

(87) International publication number:
**WO 2024/071393 (04.04.2024 Gazette 2024/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **30.09.2022 JP 2022158626**

(71) Applicant: **Sumitomo Chemical Company, Limited**
**Tokyo 103-6020 (JP)**

(72) Inventors:
- **SATO, Natsumi**
  **Takarazuka-shi, Hyogo 665-8555 (JP)**
- **SHIODA, Takayuki**
  **Takarazuka-shi, Hyogo 665-8555 (JP)**
- **SHIMAMURA, Ayano**
  **Tokyo 103-6020 (JP)**
- **SATO YANAGIHARA, Saki**
  **Osaka-shi, Osaka 541-8550 (JP)**

(74) Representative: **Vossius & Partner**
**Patentanwälte Rechtsanwälte mbB**
**Siebertstrasse 3**
**81675 München (DE)**

# (54) HETEROCYCLIC COMPOUND AND HARMFUL ARTHROPOD-CONTROLLING COMPOSITION CONTAINING SAME

(57) The present invention provides compounds having an excellent control activity against harmful arthropods.
A compound represented by formula (I) or an N-oxide thereof having an excellent control activity against harmful arthropods

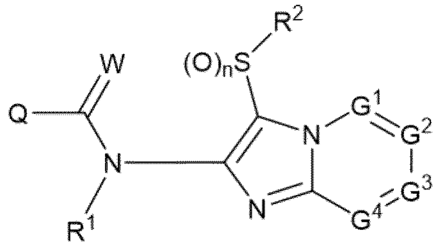

(I)

[wherein the symbols have the meanings described in the description].



Processed by Luminess, 75001 PARIS (FR)

## Description

TECHNICAL FIELD

The present application claims priority to and the benefit of JP-A-2022-158626, filed on September 30, 2022, the entire contents of which are incorporated herein by reference.

The present invention relates to heterocyclic compounds and compositions for controlling harmful arthropods containing the same.

BACKGROUND ART

Various compounds have been investigated so far for controlling harmful arthropods. For example, Patent Document 1 describes that certain compounds have a harmful arthropod control effect.

PRIOR ART DOCUMENT

PATENT DOCUMENT

Patent Document 1: WO 2016/129684 A

SUMMARY OF THE INVENTION

PROBLEMS TO BE SOLVED BY THE INVENTION

An object of the present invention is to provide a compound having excellent control activity against harmful arthropods.

MEANS FOR SOLVING THE PROBLEMS

The present invention is as follows.

[1] A compound represented by a formula (I) (hereinafter, referred to as a present compound N) or an N-oxide thereof (hereinafter, a compound represented by the formula (I) or an N-oxide thereof is referred to as a present compound),

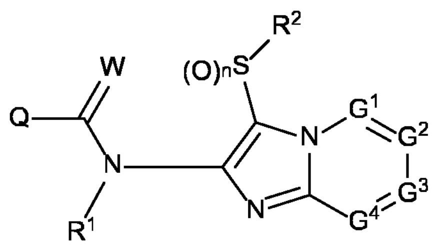

(I)

[wherein:

W represents an oxygen atom or a sulfur atom;

$R^1$ represents a C1-C6 chain hydrocarbon group optionally substituted with one or more substituent(s) selected from Group D, a C3-C7 cycloalkyl group optionally substituted with one or more substituent(s) selected from Group E, a phenyl group optionally substituted with one or more substituent(s) selected from Group F, a 5- or 6-membered aromatic heterocyclic group optionally substituted with one or more substituent(s) selected from Group F, $C(O)R^4$, $C(O)OR^4$, or $C(O)NR^4R^5$;

$R^4$ and $R^5$ are identical to or different from each other, and each represents a C1-C6 chain hydrocarbon group optionally substituted with one or more halogen atoms, a phenyl group optionally substituted with one or more substituent(s) selected from Group F, a 5- or 6-membered heterocyclic group optionally substituted with one or more substituent(s) selected from Group F, or a hydrogen atom;

$R^2$ represents a C1-C6 alkyl group optionally substituted with one or more halogen atoms, a cyclopropyl group, a cyclopropylmethyl group, or $NR^6R^7$;

$R^6$ and $R^7$ are identical to or different from each other, and each represents a C1-C6 alkyl group optionally

substituted with one or more halogen atoms, a C3-C6 cycloalkyl group optionally substituted with one or more halogen atoms, or a hydrogen atom; or

$R^6$ and $R^7$ may be combined with the nitrogen atom to which they are attached to form an aziridinyl group, an azetidinyl group, a pyrrolidinyl group, or a piperidyl group;

n represents 0, 1, or 2;

$G^1$ represents a nitrogen atom or $CR^{3a}$;

$G^2$ represents a nitrogen atom or $CR^{3b}$;

$G^3$ represents a nitrogen atom or $CR^{3c}$;

$G^4$ represents a nitrogen atom or $CR^{3d}$;

$R^{3a}$, $R^{3b}$, $R^{3c}$, and $R^{3d}$ are identical to or different from each other, and each represents a C1-C6 chain hydrocarbon group optionally substituted with one or more substituent(s) selected from Group H, a C3-C7 cycloalkyl group optionally substituted with one or more substituent(s) selected from Group J, a phenyl group optionally substituted with one or more substituent(s) selected from Group K, a 5- or 6-membered aromatic heterocyclic group optionally substituted with one or more substituent (s) selected from Group K, $OR^8$, $NR^8R^9$, $NR^{8a}R^{9a}$, $NR^{10}NR^8R^9$, $NR^{10}OR^9$, $NR^9C(O)R^{11}$, $NR^{10}NR^9C(O)R^{11}$, $NR^9C(O)OR^{12}$, $NR^{10}NR^9C(O)OR^{12}$, $NR^9C(O)NR^{13}R^{14}$, $NR^{10}NR^9C(O)NR^{13}R^{14}$, $N{=}CHNR^{13}R^{14}$, $N{=}S(O)_pR^{15}R^{16}$, $C(O)R^{11}$, $C(O)OR^{17}$, $C(O)NR^{13}R^{14}$, $C(O)NR^9S(O)_2R^{18}$, $CR^{19}{=}NOR^{17}$, $NR^9CR^{10}{=}NOR^{17}$, $S(O)_mR^{18}$, a cyano group, a nitro group, a hydrogen atom, or a halogen atom;

p represents 0 or 1;

m represents 0, 1, or 2;

$R^8$ represents a C1-C6 chain hydrocarbon group optionally substituted with one or more substituent(s) selected from Group L, a C3-C7 cycloalkyl group optionally substituted with one or more substituent(s) selected from Group M, a C3-C7 cycloalkenyl group optionally substituted with one or more substituent(s) selected from Group M, a phenyl group optionally substituted with one or more substituent(s) selected from Group $J^2$, a 6-membered aromatic heterocyclic group optionally substituted with one or more substituent(s) selected from Group $J^2$, a hydrogen atom, or $S(O)_2R^{18}$;

$R^{11}$ represents a C1-C6 chain hydrocarbon group optionally substituted with one or more halogen atom(s), a C3-C7 cycloalkyl group optionally substituted with one or more halogen atom(s), a (C3-C6 cycloalkyl)C1-C3 alkyl group optionally substituted with one or more halogen atom(s), a phenyl group optionally substituted with one or more substituent(s) selected from Group $J^2$, a 5- or 6-membered aromatic heterocyclic group optionally substituted with one or more substituent(s) selected from Group $J^2$, or a hydrogen atom;

$R^{12}$ represents a C1-C6 chain hydrocarbon group optionally substituted with one or more halogen atom(s), a C3-C7 cycloalkyl group optionally substituted with one or more halogen atom(s), a (C3-C6 cycloalkyl)C1-C3 alkyl group optionally substituted with one or more halogen atom(s), or a phenyl C1-C3 alkyl group {wherein the phenyl portion of said phenyl C1-C3 alkyl group may be substituted with one or more substituent(s) selected from Group $J^2$};

$R^{13}$ represents a C1-C6 alkyl group optionally substituted with one or more halogen atom(s), or a hydrogen atom;

$R^{14}$ represents a C1-C6 chain hydrocarbon group optionally substituted with one or more substituent(s) selected from Group L, a C3-C7 cycloalkyl group optionally substituted with one or more substituent(s) selected from Group M, $S(O)_2R^{18}$, or a hydrogen atom;

$R^{15}$ and $R^{16}$ are identical to or different from each other, and each represents a C1-C6 alkyl group optionally substituted with one or more halogen atom(s);

$R^{17}$ represents a C1-C6 chain hydrocarbon group optionally substituted with one or more halogen atom(s), a phenyl group optionally substituted with one or more substituent(s) selected from Group $J^2$, or a hydrogen atom;

$R^{18}$ represents a C1-C6 chain hydrocarbon group optionally substituted with one or more halogen atom(s), or a phenyl group optionally substituted with one or more substituent(s) selected from Group $J^2$;

$R^{19}$ represents a C1-C6 chain hydrocarbon group optionally substituted with one or more halogen atom(s), a halogen atom, $OR^{20}$, $NR^{21}R^{22}$, or a hydrogen atom;

$R^{20}$ represents a C1-C6 chain hydrocarbon group optionally substituted with one or more halogen atom(s);

$R^9$, $R^{10}$, $R^{21}$, and $R^{22}$ are identical to or different from each other, and each represents a C1-C6 chain hydrocarbon group optionally substituted with one or more halogen atom(s), or a hydrogen atom;

$R^{8a}$ and $R^{9a}$ are combined with the nitrogen atom which they are attached to form a 3-7 membered nonaromatic heterocyclic group optionally substituted with one or more substituent(s) selected from Group P;

Q represents $Q^1$ or $Q^2$;

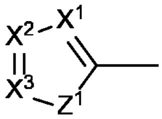

$Q^1$

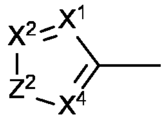

$Q^2$

wherein,
$Z^1$ represents $NR^{3j}$, an oxygen atom, or a sulfur atom;
$Z^2$ represents $NR^{3k}$, an oxygen atom, or a sulfur atom;
when Q is $Q^1$ and $Z^1$ represents $NR^{3j}$, $X^1$ represents a nitrogen atom or $CR^{3e}$, $X^2$ represents a nitrogen atom or $CR^{3f}$, and $X^3$ represents a nitrogen atom or $CR^{3g}$;
when Q is $Q^1$ and $Z^1$ represents an oxygen atom, $X^1$ represents a nitrogen atom or $CR^{3e}$, $X^2$ represents a nitrogen atom or $CR^{3f}$, and $X^3$ represents a nitrogen atom or $CR^{3g}$ (provided that not all of $X^1$, $X^2$, and $X^3$ represent nitrogen atoms);
when Q is $Q^1$ and $Z^1$ represents a sulfur atom, $X^1$ represents a nitrogen atom or $CR^{3e}$, $X^2$ represents a nitrogen atom or $CR^{3f}$, and $X^3$ represents a nitrogen atom or $CR^{3g}$ (provided that not all of $X^1$, $X^2$, and $X^3$ represent nitrogen atoms);
when Q is $Q^2$ and $Z^2$ represents $NR^{3k}$, $X^1$ represents a nitrogen atom or $CR^{3e}$, $X^2$ represents a nitrogen atom or $CR^{3f}$, and $X^4$ represents a nitrogen atom or $CR^{3h}$;
when Q is $Q^2$ and $Z^2$ represents an oxygen atom, $X^1$ represents a nitrogen atom or $CR^{3e}$, $X^2$ represents a nitrogen atom or $CR^{3f}$, and $X^4$ represents a nitrogen atom or $CR^{3h}$ (provided that not all of $X^1$, $X^2$, and $X^4$ represent nitrogen atoms);
when Q is $Q^2$ and $Z^2$ represents a sulfur atom, $X^1$ represents a nitrogen atom or $CR^{3e}$, $X^2$ represents a nitrogen atom or $CR^{3f}$, and $X^4$ represents a nitrogen atom or $CR^{3h}$ (provided that not all of $X^1$, $X^2$, and $X^4$ represent nitrogen atoms);
$R^{3j}$ represents a methyl group optionally substituted with one or more substituent(s) selected from Group T, a C2-C4 chain hydrocarbon group optionally substituted with one or more substituent(s) selected from Group U, a C5-C6 chain hydrocarbon group optionally substituted with one or more substituent(s) selected from Group V, a C3-C7 cycloalkyl group optionally substituted with one or more substituent(s) selected from Group $K^2$, a phenyl group optionally substituted with one or more substituent(s) selected from Group X, a 5- or 6-membered aromatic heterocyclic group optionally substituted with one or more substituent (s) selected from Group X, $C(O)R^{23}$, $C(O)OR^{24}$, $C(O)NR^{25}R^{26}$, $C(O)NR^{27}S(O)_2R^{28}$, $CR^{29}=NOR^{24}$, or $S(O)_kR^{28}$;
k represents 0, 1, or 2;
$R^{23}$ represents a C1-C6 chain hydrocarbon group optionally substituted with one or more halogen atom(s), a C3-C7 cycloalkyl group optionally substituted with one or more halogen atom(s), a (C3-C6 cycloalkyl)C1-C3 alkyl group optionally substituted with one or more halogen atom(s), a phenyl group optionally substituted with one or more substituent(s) selected from Group X, a 5- or 6-membered aromatic heterocyclic group optionally substituted with one or more substituent(s) selected from Group X, or a hydrogen atom;
$R^{24}$ represents a C1-C6 chain hydrocarbon group optionally substituted with one or more halogen atom(s), a phenyl group optionally substituted with one or more substituent(s) selected from Group X, or a hydrogen atom;
$R^{25}$ represents a C1-C6 alkyl group optionally substituted with one or more halogen atom(s), or a hydrogen atom;
$R^{26}$ represents a C1-C6 chain hydrocarbon group optionally substituted with one or more substituent(s) selected from Group V, a C3-C7 cycloalkyl group optionally substituted with one or more substituent(s) selected from Group $K^2$, $S(O)_vR^{28}$, or a hydrogen atom;
v represents 0, 1, or 2;
$R^{28}$ represents a C1-C6 chain hydrocarbon group optionally substituted with one or more halogen atom(s), or a phenyl group optionally substituted with one or more substituent(s) selected from Group X;
$R^{29}$ represents a C1-C6 chain hydrocarbon group optionally substituted with one or more halogen atom(s), a halogen atom, $OR^{30}$, $NR^{31}R^{32}$, or a hydrogen atom;
$R^{30}$ represents a C1-C6 chain hydrocarbon group optionally substituted with one or more halogen atom(s);
$R^{27}$, $R^{31}$, and $R^{32}$ are identical to or different from each other, and each represents a C1-C6 chain hydrocarbon group optionally substituted with one or more halogen atom(s), or a hydrogen atom;
$R^{3k}$ represents a C1-C6 chain hydrocarbon group optionally substituted with one or more substituent(s) selected from Group Y, a C3-C7 cycloalkyl group optionally substituted with one or more substituent(s) selected from Group $M^2$, a phenyl group optionally substituted with one or more substituent(s) selected from Group $A^2$, a 5- or 6-membered aromatic heterocyclic group optionally substituted with one or more substituent(s) selected from Group $A^2$, $C(O)R^{52}$, $C(O)OR^{53}$, $C(O)NR^{54}R^{55}$, $C(O)NR^{56}S(O)_2R^{57}$, $CR^{58}=NOR^{53}$, $S(O)_tR^{57}$, or a hydrogen

atom;
t represents 0, 1, or 2;
$R^{52}$ represents a C1-C6 chain hydrocarbon group optionally substituted with one or more halogen atom(s), a C3-C7 cycloalkyl group optionally substituted with one or more halogen atom(s), a (C3-C6 cycloalkyl)C1-C3 alkyl group optionally substituted with one or more halogen atom(s), a phenyl group optionally substituted with one or more substituent(s) selected from Group $A^2$, a 5- or 6-membered aromatic heterocyclic group optionally substituted with one or more substituent(s) selected from Group $A^2$, or a hydrogen atom;
$R^{53}$ represents a C1-C6 chain hydrocarbon group optionally substituted with one or more halogen atom(s), a phenyl group optionally substituted with one or more substituent(s) selected from Group $A^2$, or a hydrogen atom;
$R^{54}$ represents a C1-C6 alkyl group optionally substituted with one or more halogen atom(s), or a hydrogen atom;
$R^{55}$ represents a C1-C6 chain hydrocarbon group optionally substituted with one or more substituent(s) selected from Group Y, a C3-C7 cycloalkyl group optionally substituted with one or more substituent(s) selected from Group $M^2$, $S(O)_yR^{28}$, or a hydrogen atom;
y represents 0, 1, or 2;
$R^{57}$ represents a C1-C6 chain hydrocarbon group optionally substituted with one or more halogen atom(s), or a phenyl group optionally substituted with one or more substituent(s) selected from Group $A^2$;
$R^{58}$ represents a C1-C6 chain hydrocarbon group optionally substituted with one or more halogen atom(s), a halogen atom, $OR^{59}$, $NR^{60}R^{61}$, or a hydrogen atom;
$R^{59}$ represents a C1-C6 chain hydrocarbon group optionally substituted with one or more halogen atom(s);
$R^{56}$, $R^{60}$, and $R^{61}$ are identical to or different from each other, and each represents a C1-C6 chain hydrocarbon group optionally substituted with one or more halogen atom(s), or a hydrogen atom;
$R^{3e}$, $R^{3f}$, $R^{3g}$, and $R^{3h}$ are identical to or different from each other, and each represents a C1-C6 chain hydrocarbon group optionally substituted with one or more substituent(s) selected from Group $B^2$, a C3-C7 cycloalkyl group optionally substituted with one or more substituent(s) selected from Group $D^2$, a phenyl group optionally substituted with one or more substituent(s) selected from Group $E^2$, a 5- or 6-membered aromatic heterocyclic group optionally substituted with one or more substituent (s) selected from Group $E^2$, $OR^{33}$, $NR^{33}R^{34}$, $NR^{33a}R^{34a}$, $NR^{35}NR^{33}R^{34}$, $NR^{35}OR^{34}$, $NR^{34}C(O)R^{36}$, $NR^{35}NR^{34}C(O)R^{36}$, $NR^{34}C(O)OR^{37}$, $NR^{35}NR^{34}C(O)OR^{37}$, $NR^{34}C(O)NR^{38}R^{39}$, $NR^{35}NR^{34}C(O)NR^{38}R^{39}$, $N{=}CHNR^{38}R^{39}$, $N{=}S(O)_qR^{40}R^{41}$, $C(O)R^{36}$, $C(O)OR^{42}$, $C(O)NR^{38}R^{39}$, $C(O)NR^{34}S(O)_2R^{43}$, $CR^{44}{=}NOR^{42}$, $NR^{34}CR^{35}{=}NOR^{42}$, $S(O)_rR^{43}$, a cyano group, a nitro group, a halogen atom, or a hydrogen atom;
q represents 0 or 1;
r represents 0, 1, or 2;
$R^{33}$ represents a C1-C6 chain hydrocarbon group optionally substituted with one or more substituent(s) selected from Group $B^2$, a C3-C7 cycloalkyl group optionally substituted with one or more substituent(s) selected from Group $D^2$, a C3-C7 cycloalkenyl group optionally substituted with one or more substituent(s) selected from Group $D^2$, a phenyl group optionally substituted with one or more substituent(s) selected from Group $E^2$, a 6-membered aromatic heterocyclic group optionally substituted with one or more substituent(s) selected from Group $E^2$, a hydrogen atom, or $S(O)_2R^{43}$;
$R^{36}$ represents a C1-C6 chain hydrocarbon group optionally substituted with one or more halogen atom(s), a C3-C7 cycloalkyl group optionally substituted with one or more halogen atom(s), a (C3-C6 cycloalkyl)C1-C3 alkyl group optionally substituted with one or more halogen atom(s), a phenyl group optionally substituted with one or more substituent(s) selected from Group $E^2$, a 5- or 6-membered aromatic heterocyclic group optionally substituted with one or more substituent(s) selected from Group $E^2$, or a hydrogen atom;
$R^{37}$ represents a C1-C6 chain hydrocarbon group optionally substituted with one or more halogen atom(s), a C3-C7 cycloalkyl group optionally substituted with one or more halogen atom(s), a (C3-C6 cycloalkyl)C1-C3 alkyl group optionally substituted with one or more halogen atom(s), or a phenyl C1-C3 alkyl group {wherein the phenyl portion of said phenyl C1-C3 alkyl group may be substituted with one or more substituent(s) selected from Group $E^2$};
$R^{38}$ represents a C1-C6 alkyl group optionally substituted with one or more halogen atom(s), or a hydrogen atom;
$R^{39}$ represents a C1-C6 chain hydrocarbon group optionally substituted with one or more substituent(s) selected from Group $B^2$, a C3-C7 cycloalkyl group optionally substituted with one or more substituent(s) selected from Group $D^2$, $S(O)_2R^{43}$, or a hydrogen atom;
$R^{40}$ and $R^{41}$ are identical to or different from each other, and each represents a C1-C6 alkyl group optionally substituted with one or more halogen atom(s);
$R^{42}$ represents a C1-C6 chain hydrocarbon group optionally substituted with one or more halogen atom(s), a phenyl group optionally substituted with one or more substituent(s) selected from Group $E^2$, or a hydrogen atom;
$R^{43}$ represents a C1-C6 chain hydrocarbon group optionally substituted with one or more halogen atom(s), or a phenyl group optionally substituted with one or more substituent(s) selected from Group $E^2$;

$R^{44}$ represents a C1-C6 chain hydrocarbon group optionally substituted with one or more halogen atom(s), a halogen atom, $OR^{45}$, $NR^{46}R^{47}$, or a hydrogen atom;
$R^{45}$ represents a C1-C6 chain hydrocarbon group optionally substituted with one or more halogen atom(s);
$R^{34}$, $R^{35}$, $R^{46}$, and $R^{47}$ are identical to or different from each other, and each represents a C1-C6 chain hydrocarbon group optionally substituted with one or more halogen atom(s), or a hydrogen atom;
$R^{33a}$ and $R^{34a}$ are combined with the nitrogen atom to which they are attached to form a 3-7 membered nonaromatic heterocyclic group optionally substituted with one or more substituent(s) selected from Group $F^2$;
Group D: a group consisting of a C3-C6 cycloalkyl group optionally substituted with one or more substituent(s) selected from the group consisting of halogen atoms and C1-C3 alkyl groups, a C1-C6 alkoxy group optionally substituted with one or more halogen atom(s), a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), a C2-C6 alkylcarbonyl group optionally substituted with one or more halogen atom(s), a C2-C6 alkoxycarbonyl group optionally substituted with one or more halogen atom(s), a phenyl group optionally substituted with one or more substituent(s) selected from Group $L^2$, a 5- or 6-membered aromatic heterocyclic group optionally substituted with one or more substituent(s) selected from Group $L^2$, a hydroxy group, a cyano group, and a halogen atom;
Group E: a group consisting of a C1-C6 alkyl group optionally substituted with one or more halogen atom(s), a C1-C6 alkoxy group optionally substituted with one or more halogen atom(s), a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), and a halogen atom;
Group F: a group consisting of a C1-C6 alkyl group optionally substituted with one or more halogen atom(s), a C1-C6 alkoxy group optionally substituted with one or more halogen atom(s), a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), a C1-C6 alkylamino group optionally substituted with one or more halogen atom(s), a di(C1-C6 alkyl)amino group optionally substituted with one or more halogen atom(s), a C2-C6 alkylcarbonyl group optionally substituted with one or more halogen atom(s), a C2-C6 alkoxycarbonyl group optionally substituted with one or more halogen atom(s), a hydroxy group, a sulfanyl group, an amino group, a cyano group, a nitro group, and a halogen atom;
Group H: a group consisting of a C1-C6 alkoxy group optionally substituted with one or more halogen atom(s), a C3-C6 alkenyloxy group optionally substituted with one or more halogen atom(s), a C3-C6 alkynyloxy group optionally substituted with one or more halogen atom(s), a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), a C3-C6 cycloalkyl group optionally substituted with one or more halogen atom(s), a cyano group, a hydroxy group, and a halogen atom;
Group J: a group consisting of a C1-C6 chain hydrocarbon group optionally substituted with one or more halogen atom(s), a C1-C6 alkoxy group optionally substituted with one or more halogen atom(s), a C3-C6 alkenyloxy group optionally substituted with one or more halogen atom(s), a C3-C6 alkynyloxy group optionally substituted with one or more halogen atom(s), a halogen atom, an oxo group, a hydroxy group, a cyano group, and a nitro group;
Group K: a group consisting of a C1-C6 alkyl group optionally substituted with one or more halogen atom(s), a 5- or 6-membered aromatic heterocyclic group optionally substituted with one or more substituent(s) selected from Group $J^2$, $OR^{49}$, $NR^{48}R^{49}$, $C(O)R^{49}$, $C(O)NR^{48}R^{49}$, $OC(O)R^{48}$, $OC(O)OR^{48}$, $NR^{49}C(O)R^{48}$, $NR^{49}C(O)OR^{48}$, $C(O)OR^{49}$, a halogen atom, a nitro group, a cyano group, and an amino group;
$R^{48}$ represents a C1-C6 alkyl group optionally substituted with one or more halogen atom(s), or a C3-C6 cycloalkyl group optionally substituted with one or more halogen atom(s),
$R^{49}$ represents a C1-C6 alkyl group optionally substituted with one or more halogen atom(s), a C3-C6 cycloalkyl group optionally substituted with one or more halogen atom(s), or a hydrogen atom;
Group L: a group consisting of a C1-C6 alkoxy group optionally substituted with one or more halogen atom(s), a phenyl group optionally substituted with one or more substituent(s) selected from Group $J^2$, a 5- or 6-membered aromatic heterocyclic group optionally substituted with one or more substituent(s) selected from Group $J^2$, a C3-C7 cycloalkyl group optionally substituted with one or more halogen atom(s), a 3-7 membered nonaromatic heterocyclic group optionally substituted with one or more substituent(s) selected from Group $H^2$, an amino group, $NHR^{50}$, $NR^{50}R^{51}$, a halogen atom, and a cyano group;
$R^{50}$ and $R^{51}$ are identical to or different from each other, and each represents a C1-C6 alkyl group optionally substituted with one or more halogen atom(s);
Group M: a group consisting of a C1-C6 alkyl group optionally substituted with one or more halogen atom(s), a

halogen atom, and a cyano group;

Group P: a group consisting of a C1-C6 chain hydrocarbon group optionally substituted with one or more halogen atom(s), a C1-C6 alkoxy group optionally substituted with one or more halogen atom(s), a C3-C6 alkenyloxy group optionally substituted with one or more halogen atom(s), a C3-C6 alkynyloxy group optionally substituted with one or more halogen atom(s), a halogen atom, an oxo group, a hydroxy group, a cyano group, and a nitro group;

Group T: a group consisting of a C3-C6 cycloalkyl group optionally substituted with one or more substituent(s) selected from the group consisting of fluorine atoms and C1-C3 alkyl groups, a C1-C6 alkoxy group optionally substituted with one or more halogen atom(s), a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), a hydroxy group, a sulfanyl group, and a halogen atom;

Group U: a group consisting of a C3-C6 cycloalkyl group optionally substituted with one or more substituent(s) selected from the group consisting of a fluorine atom and a C1-C3 alkyl group, a C1-C6 alkylsulfanyl group optionally substituted with one or more halogen atoms, a C1-C6 alkylsulfinyl group optionally substituted with one or more halogen atoms, a C1-C6 alkylsulfonyl group optionally substituted with one or more halogen atoms, a sulfanyl group, and a fluorine atom;

Group V: a group consisting of a C3-C6 cycloalkyl group optionally substituted with one or more substituent(s) selected from the group consisting of halogen atoms and C1-C3 alkyl groups, a C1-C6 alkoxy group optionally substituted with one or more halogen atom(s), a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), a C2-C6 alkylcarbonyl group optionally substituted with one or more halogen atom(s), a C2-C6 alkoxycarbonyl group optionally substituted with one or more halogen atom(s), a hydroxy group, a sulfanyl group, a cyano group, and a halogen atom;

Group X: a group consisting of a C1-C6 alkyl group optionally substituted with one or more halogen atom(s), a C1-C6 alkoxy group optionally substituted with one or more halogen atom(s), a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), a C1-C6 alkylamino group optionally substituted with one or more halogen atom(s), a di(C1-C6 alkyl)amino group optionally substituted with one or more halogen atom(s), a C2-C6 alkylcarbonyl group optionally substituted with one or more halogen atom(s), a C2-C6 alkoxycarbonyl group optionally substituted with one or more halogen atom(s), a hydroxy group, a sulfanyl group, an amino group, a cyano group, a nitro group, and a halogen atom;

Group Y: a group consisting of a C3-C6 cycloalkyl group optionally substituted with one or more substituent(s) selected from the group consisting of halogen atoms and C1-C3 alkyl groups, a C1-C6 alkoxy group optionally substituted with one or more halogen atom(s), a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), a C2-C6 alkylcarbonyl group optionally substituted with one or more halogen atom(s), a C2-C6 alkoxycarbonyl group optionally substituted with one or more halogen atom(s), a hydroxy group, a sulfanyl group, a cyano group, and a halogen atom;

Group $A^2$: a group consisting of a C1-C6 alkyl group optionally substituted with one or more halogen atom(s), a C1-C6 alkoxy group optionally substituted with one or more halogen atom(s), a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), a C1-C6 alkylamino group optionally substituted with one or more halogen atom(s), a di(C1-C6 alkyl) amino group optionally substituted with one or more halogen atom(s), a C2-C6 alkylcarbonyl group optionally substituted with one or more halogen atom(s), a C2-C6 alkoxycarbonyl group optionally substituted with one or more halogen atom(s), a hydroxy group, a sulfanyl group, an amino group, a cyano group, a nitro group, and a halogen atom;

Group $B^2$: a group consisting of a C3-C6 cycloalkyl group optionally substituted with one or more substituent(s) selected from the group consisting of halogen atoms and C1-C3 alkyl groups, a C1-C6 alkoxy group optionally substituted with one or more halogen atom(s), a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), a C2-C6 alkylcarbonyl group optionally substituted with one or more halogen atom(s), a C2-C6 alkoxycarbonyl group optionally substituted with one or more halogen atom(s), a hydroxy group, a sulfanyl group, a cyano group, and a halogen atom;

Group $D^2$: a group consisting of a C1-C6 alkyl group optionally substituted with one or more halogen atom(s), a C1-C6 alkoxy group optionally substituted with one or more halogen atom(s), a C1-C6 alkylsulfanyl group optionally substituted with one or more halogen atom(s), a C1-C6 alkylsulfinyl group optionally substituted with

one or more halogen atom(s), a C1-C6 alkylsulfonyl group optionally substituted with one or more halogen atom(s), and a halogen atom;

Group $E^2$: a group consisting of a C1-C6 alkyl group optionally substituted with one or more halogen atom(s), a C1-C6 alkoxy group optionally substituted with one or more halogen atom(s), a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), a C1-C6 alkylamino group optionally substituted with one or more halogen atom(s), a di(C1-C6 alkyl) amino group optionally substituted with one or more halogen atom(s), a C2-C6 alkylcarbonyl group optionally substituted with one or more halogen atom(s), a C2-C6 alkoxycarbonyl group optionally substituted with one or more halogen atom(s), a hydroxy group, a sulfanyl group, an amino group, a cyano group, a nitro group, and a halogen atom;

Group $F^2$: a group consisting of a C1-C6 chain hydrocarbon group optionally substituted with one or more halogen atom(s), a C1-C6 alkoxy group optionally substituted with one or more halogen atom(s), a C3-C6 alkenyloxy group optionally substituted with one or more halogen atom(s), a C3-C6 alkynyloxy group optionally substituted with one or more halogen atom(s), a halogen atom, an oxo group, a hydroxy group, a cyano group, and a nitro group;

Group $H^2$: a group consisting of a C1-C6 chain hydrocarbon group optionally substituted with one or more halogen atom(s), a C1-C6 alkoxy group optionally substituted with one or more halogen atom(s), a C3-C6 alkenyloxy group optionally substituted with one or more halogen atom(s), a C3-C6 alkynyloxy group optionally substituted with one or more halogen atom(s), and a halogen atom;

Group $J^2$: a group consisting of a C1-C6 chain hydrocarbon group optionally substituted with one or more halogen atom(s), a hydroxy group, a C1-C6 alkoxy group optionally substituted with one or more halogen atom(s), a C3-C6 alkenyloxy group optionally substituted with one or more halogen atom(s), a C3-C6 alkynyloxy group optionally substituted with one or more halogen atom(s), a sulfanyl group, a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), an amino group, $NHR^{50}$, $NR^{50}R^{51}$, $C(O)R^{50}$, $OC(O)R^{50}$, $C(O)OR^{50}$, a cyano group, a nitro group, and a halogen atom;

Group $K^2$: a group consisting of a C1-C6 alkyl group optionally substituted with one or more halogen atom(s), a C1-C6 alkoxy group optionally substituted with one or more halogen atom(s), a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), and a fluorine atom;

Group $L^2$: a group consisting of a C1-C6 alkyl group optionally substituted with one or more halogen atom(s), a C1-C6 alkoxy group optionally substituted with one or more halogen atom(s), a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), a C1-C6 alkylamino group optionally substituted with one or more halogen atom(s), a di(C1-C6 alkyl)amino optionally substituted with one or more halogen atom(s) group, a C2-C6 alkylcarbonyl group optionally substituted with one or more halogen atom(s), a C2-C6 alkoxycarbonyl group optionally substituted with one or more halogen atom(s), a hydroxy group, a sulfanyl group, an amino group, a cyano group, a nitro group, and a halogen atom;

Group $M^2$: a group consisting of a C1-C6 alkyl group optionally substituted with one or more halogen atom(s), a C1-C6 alkoxy group optionally substituted with one or more halogen atom(s), a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), and a halogen atom.]

[2] The compound according to [1] or an N-oxide thereof, wherein $G^1$ is $CR^{3a}$, $G^2$ is $CR^{3b}$, $G^3$ is $CR^{3c}$, and $G^4$ is a nitrogen atom or $CR^{3d}$.

[3] The compound according to [1] or an N-oxide thereof, wherein $G^1$ is $CR^{3a}$, $G^2$ is $CR^{3b}$, $G^3$ is $CR^{3c}$, and $G^4$ is $CR^{3d}$.

[4] The compound according to any one of [1] to [3] or an N-oxide thereof, wherein

Q is $Q^1$,

in $Q^1$, a combination of $Z^1$, $X^1$, $X^2$ and $X^3$ is:

a combination where $Z^1$ is an oxygen atom, $X^1$ is a nitrogen atom, $X^2$ is $CR^{3f}$, and $X^3$ is $CR^{3g}$;

a combination where $Z^1$ is an oxygen atom, $X^1$ is $CR^{3e}$, $X^2$ is a nitrogen atom, and $X^3$ is $CR^{3g}$;

a combination where $Z^1$ is an oxygen atom, $X^1$ is $CR^{3e}$, $X^2$ is $CR^{3f}$, and $X^3$ is a nitrogen atom;

a combination where $Z^1$ is a sulfur atom, $X^1$ is $CR^{3e}$, $X^2$ is a nitrogen atom, and $X^3$ is $CR^{3g}$;

a combination where $Z^1$ is a sulfur atom, $X^1$ is a nitrogen atom, $X^2$ is $CR^{3f}$, and $X^3$ is $CR^{3g}$;
a combination where $Z^1$ is $NR^{3j}$, $X^1$ is a nitrogen atom, $X^2$ is $CR^{3f}$, and $X^3$ is $CR^{3g}$;
a combination where $Z^1$ is $NR^{3j}$, $X^1$ is $CR^{3e}$, $X^2$ is a nitrogen atom, and $X^3$ is $CR^{3g}$; or
a combination where $Z^1$ is $NR^{3j}$, $X^1$ is $CR^{3e}$, $X^2$ is $CR^{3f}$, and $X^3$ is a nitrogen atom.

[5] The compound according to any one of [1] to [3] or an N-oxide thereof, wherein Q is $Q^2$, in $Q^2$, a combination of $Z^2$, $X^1$, $X^2$ and $X^4$ is:

a combination where $Z^2$ is an oxygen atom, $X^1$ is a nitrogen atom, $X^2$ is $CR^{3f}$, and $X^4$ is $CR^{3h}$;
a combination where $Z^2$ is an oxygen atom, $X^1$ is $CR^{3e}$, $X^2$ is a nitrogen atom, and $X^4$ is $CR^{3h}$;
a combination where $Z^2$ is an oxygen atom, $X^1$ is $CR^{3e}$, $X^2$ is $CR^{3f}$, and $X^4$ is a nitrogen atom;
a combination where $Z^2$ is $NR^{3k}$, $X^1$ is a nitrogen atom, $X^2$ is $CR^{3f}$, and $X^4$ is $CR^{3h}$;
a combination where $Z^2$ is $NR^{3k}$, $X^1$ is $CR^{3e}$, $X^2$ is a nitrogen atom, and $X^4$ is $CR^{3h}$;
a combination where $Z^2$ is $NR^{3k}$, $X^1$ is $CR^{3e}$, $X^2$ is $CR^{3f}$, and $X^4$ is a nitrogen atom;
a combination where $Z^2$ is $NR^{3k}$, $X^1$ is $CR^{3e}$, $X^2$ is $CR^{3f}$, and $X^4$ is $CR^{3h}$; or
a combination where $Z^2$ is $NR^{3k}$, $X^1$ is a nitrogen atom, $X^2$ is a nitrogen atom, and $X^4$ is $CR^{3h}$.

[6] The compound according to any one of [1] to [5] or an N-oxide thereof, wherein $R^2$ is an ethyl group.
[7] The compound according to any one of [1] to [6] or an N-oxide thereof, wherein W is an oxygen atom.
[8] A composition for controlling a harmful arthropod comprising the compound according to any one of [1] to [7] or an N-oxide thereof.
[9] A composition comprising one or more ingredients selected from the group consisting of the following Groups (a), (b), (c) and (d), and the compound according to any one of [1] to [7] or an N-oxide thereof:

Group (a): a group consisting of insecticidal ingredients, miticidal ingredients, and nematicidal ingredients;
Group (b): fungicidal ingredients;
Group (c): plant growth modulating ingredients; and
Group (d): repellent ingredients.

[10] A method for controlling a harmful arthropod, which comprises applying an effective amount of the compound according to any one of [1] to [7] or an N-oxide thereof, or an effective amount of the composition according to [9] to a harmful arthropod or a habitat where the harmful arthropod lives.
[11] A seed or vegetative reproductive organ carrying an effective amount of the compound according to any one of [1] to [7] or an N-oxide thereof or an effective amount of the composition according to [9].
[12] A compound represented by a formula (II) or a salt thereof (hereinafter, a compound represented by the formula (II) or a salt thereof is referred to as an intermediate A),

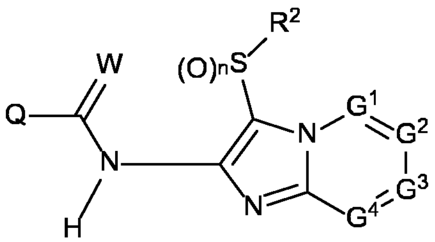 (II)

wherein the symbols have the same meanings as defined in [1].

EFFECT OF THE INVENTION

The present invention can control harmful arthropods.

MODE FOR CARRYING OUT THE INVENTION

The substituent in the present invention will be described.
The halogen atom means a fluorine atom, a chlorine atom, a bromine atom, or an iodine atom.
When the substituent is substituted with two or more halogen atoms or substituents, the halogen atoms or substituents may be the same as or different from each other.

The notation of "CX-CY" in the present description means that the number of carbon atoms is X to Y. For example, the notation of "C1-C6" means that the number of carbon atoms is 1 to 6.

The chain hydrocarbon group represents an alkyl group, an alkenyl group, or an alkynyl group.

Examples of the alkyl group include a methyl group, an ethyl group, a propyl group, an isopropyl group, a 1,1-dimethylpropyl group, a 1,2-dimethylpropyl group, a 1-ethylpropyl group, a butyl group, a sec-butyl group, a tert-butyl group, a pentyl group, and a hexyl group.

Examples of the alkenyl group include a vinyl group, a 1-propenyl group, a 2-propenyl group, a 1-methyl-1-propenyl group, a 1-methyl-2-propenyl group, a 1,2-dimethyl-1-propenyl group, a 1-ethyl -2-propenyl group, a 3-butenyl group, a 4-pentenyl group, and a 5-hexenyl group.

Examples of the alkynyl group include an ethynyl group, a 1-propynyl group, a 2-propynyl group, a 1-methyl-2-propynyl group, a 1,1-dimethyl-2-propynyl group, a 1-ethyl-2-propynyl group, a 2-butynyl group, a 4-pentynyl group, and a 5-hexynyl group.

Examples of the alkoxy group include a methoxy group, an ethoxy group, a propoxy group, an isopropoxy group, a butoxy group, a tert-butoxy group, a pentyloxy group, and a hexyloxy group.

Examples of the alkenyloxy group include a 2-propenyloxy group, a 2-butenyloxy group, and a 5-hexenyloxy group.

Examples of the alkynyloxy group include a 2-propynyloxy group, a 2-butynyloxy group, and a 5-hexynyloxy group.

Examples of the cycloalkyl group include a cyclopropyl group, a cyclobutyl group, a cyclopentyl group, a cyclohexyl group, and a cycloheptyl group.

Examples of the cycloalkenyl group include a cyclopropenyl group, a cyclobutenyl group, a cyclopentenyl group, a cyclohexenyl group, and a cycloheptenyl group.

The 3-7 membered nonaromatic heterocyclic group represents an aziridine ring, an azetidine ring, a pyrrolidine ring, an imidazoline ring, an imidazolidine ring, a piperidine ring, a tetrahydropyrimidine ring, a hexahydropyrimidine ring, a piperazine ring, an azepane ring, an oxazolidine ring, an isoxazolidine ring, a 1,3-oxazinane ring, a morpholine ring, a 1,4-oxazepane ring, a thiazolidine ring, an isothiazolidine ring, a 1,3-thiazinane ring, a thiomorpholine ring, or a 1,4-thiazepane ring.

Examples of the 3-7 membered nonaromatic heterocyclic group optionally substituted with one or more substituent(s) selected from Group P, the 3-7 membered nonaromatic heterocyclic group optionally substituted with one or more substituent(s) selected from Group $F^2$, or the 3-7 membered nonaromatic heterocyclic group optionally substituted with one or more substituent(s) selected from Group $H^2$ include the following groups.

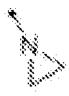
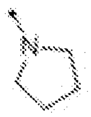
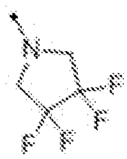
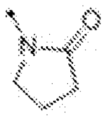
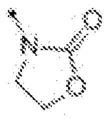
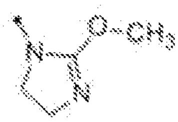
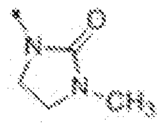

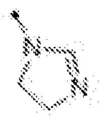
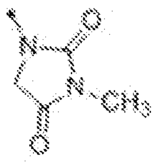
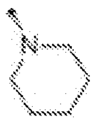
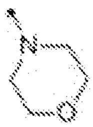
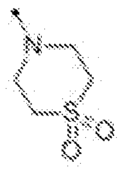

The 5- or 6-membered aromatic heterocyclic group represents a 5-membered aromatic heterocyclic group or a 6-membered aromatic heterocyclic group. The 5-membered aromatic heterocyclic group represents a pyrrolyl group, a furyl group, a thienyl group, a pyrazolyl group, an imidazolyl group, a triazolyl group, a tetrazolyl group, an oxazolyl group, an isoxazolyl group, a thiazolyl group, an isothiazolyl group, an oxadiazolyl group, or a thiadiazolyl group, and the 6-membered aromatic heterocyclic group represents a pyridyl group, a pyridazinyl group, a pyrimidinyl group, a pyrazinyl group, a triazinyl group, or a tetrazinyl group.

The 5- or 6-membered heterocyclic group represents a 5- or 6-membered aromatic heterocyclic group or a 5- or 6-membered nonaromatic heterocyclic group, for example, a pyrrolyl group, a furyl group, a thienyl group, a pyrazolyl group, an imidazolyl group, a triazolyl group, a tetrazolyl group, an oxazolyl group, an isoxazolyl group, a thiazolyl group, an isothiazolyl group, an oxadiazolyl group, a thiadiazolyl group, a pyridyl group, a pyridazinyl group, a pyrazinyl group, a triazinyl group, a tetrazinyl group, a pyrrolidinyl group, an imidazolinyl group, an imidazolidinyl group, a piperidyl group, a tetrahydropyrimidinyl group, a hexahydropyrimidinyl group, a piperazinyl group, an oxazolidinyl group, an isoxazolidinyl group, a 1,3-oxazinanyl group, a morpholinyl group, a thiazolidinyl group, an isothiazolidinyl group, a 1,3-thiazinanyl group, and a thiomorpholinyl group.

Examples of the (C3-C6 cycloalkyl) C1-C3 alkyl group optionally substituted with one or more halogen atoms include a cyclopropylmethyl group, a (2-fluorocyclopropyl)methyl group, a cyclopropyl(fluoro)methyl group, and a (2-fluorocyclopropyl) (fluoro)methyl group.

Examples of the phenyl C1-C3 alkyl group {the phenyl moiety in the phenyl C1-C3 alkyl group may be substituted with one or more substituent(s) selected from Group $J^2$} or the phenyl C1-C3 alkyl group {the phenyl moiety in the phenyl C1-C3 alkyl group may be substituted with one or more substituent(s) selected from Group $E^2$} include a benzyl group, a 2-fluorobenzyl group, a 4-chlorobenzyl group, a 4-(trifluoromethyl)benzyl group, and a 2-[4-(trifluoromethyl)phenyl]ethyl group.

Examples of the alkylsulfanyl group include a methylsulfanyl group, an ethylsulfanyl group, a propylsulfanyl group, and an isopropylsulfanyl group.

Examples of the alkylsulfinyl group include a methylsulfinyl group, an ethylsulfinyl group, a propylsulfinyl group, and an isopropylsulfinyl group.

Examples of the alkylsulfonyl group include a methylsulfonyl group, an ethylsulfonyl group, a propylsulfonyl group, and an isopropylsulfonyl group.

Examples of the alkylamino group include a methylamino group, an ethylamino group, an isopropylamino group, and a hexylamino group.

Examples of the dialkylamino group include a dimethylamino group, an ethylmethylamino group, an isopropylmethylamino group, and a dihexylamino group.

Examples of the alkylcarbonyl group include an acetyl group, a propanoyl group, a 2-methylpropanoyl group, and a hexanoyl group.

Examples of the alkoxycarbonyl group include a methoxycarbonyl group, an ethoxycarbonyl group, an isopropoxycarbonyl group, and a pentyloxycarbonyl group.

Examples of the N-oxide of the compound represented by the formula (I) include a compound represented by the following formula.

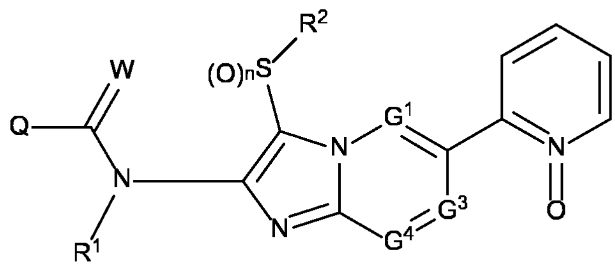

[In the formula, symbols represent the same meaning as described above.]

The present compound and the intermediate A may have one or more stereoisomers. Stereoisomers include enantiomers, diastereomers, geometric isomers, and the like. The present compound and the intermediate A include each stereoisomer and a stereoisomer mixture in an any ratio.

The present compound and the compound represented by the formula (II) may form an acid addition salt. Examples of the acid forming the acid addition salt include inorganic acids such as hydrogen chloride, phosphoric acid, and sulfuric acid, and organic acids such as acetic acid, trifluoroacetic acid, benzoic acid, and p-toluenesulfonic acid. The acid addition salt is obtained by mixing the present compound or a compound represented by the formula (II) with an acid.

Examples of an embodiment of the present compound N include the following compounds.

[Embodiment 1] In the present compound N, a compound wherein $R^1$ is a C1-C6 chain hydrocarbon group optionally substituted with one or more substituent(s) selected from Group D.

[Embodiment 2] In the present compound N, a compound wherein $R^1$ is a C1-C6 alkyl group.

[Embodiment 3] In the present compound N, a compound wherein $R^1$ is a methyl group.

[Embodiment 4] In the present compound N, a compound wherein $R^2$ is a C1-C6 alkyl group.

[Embodiment 5] In the present compound N, a compound wherein $R^2$ is an ethyl group.

[Embodiment 6] In the present compound N, a compound wherein $R^2$ is $NR^6R^7$, and $R^6$ and $R^7$ are the same as or different from each other and are a C1-C6 alkyl group or a hydrogen atom.

[Embodiment 7] In the present compound N, a compound wherein $R^2$ is $NR^6R^7$, and $R^6$ and $R^7$ are combined with the nitrogen atom to which they are attached to form an aziridinyl group, an azetidinyl group, a pyrrolidinyl group, or a piperidinyl group.

[Embodiment 8] In the present compound N, a compound wherein W is an oxygen atom.

[Embodiment 9] In the present compound N, a compound wherein a combination of $G^1$, $G^2$, $G^3$, and $G^4$ is: a

combination wherein $G^1$ is $CR^{3a}$, $G^2$ is $CR^{3b}$, $G^3$ is $CR^{3c}$, and $G^4$ is a nitrogen atom; a combination wherein $G^1$ is $CR^{3a}$, $G^2$ is $CR^{3b}$, $G^3$ is a nitrogen atom, and $G^4$ is $CR^{3d}$; a combination wherein $G^1$ is $CR^{3a}$, $G^2$ is a nitrogen atom, $G^3$ is $CR^{3c}$, and $G^4$ is $CR^{3d}$; a combination wherein $G^1$ is a nitrogen atom, $G^2$ is $CR^{3b}$, $G^3$ is $CR^{3c}$, and $G^4$ is $CR^{3d}$; or a combination wherein $G^1$ is $CR^{3a}$, $G^2$ is $CR^{3b}$, $G^3$ is $CR^{3c}$, and $G^4$ is $CR^{3d}$, $R^{3a}$, $R^{3c}$, and $R^{3d}$ are a hydrogen atom, and $R^{3b}$ is a C1-C6 alkyl group optionally substituted with one or more halogen atoms, a C3-C7 cycloalkyl group optionally substituted with one or more substituent(s) selected from the group consisting of a halogen atom and a cyano group, a phenyl group optionally substituted with one or more halogen atoms, a 5- or 6-membered aromatic heterocyclic group optionally substituted with one or more halogen atoms, or a halogen atom.

[Embodiment 10] In the present compound N, a compound wherein $G^1$ is $CR^{3a}$, $G^2$ is $CR^{3b}$, $G^3$ is $CR^{3c}$, $G^4$ is a nitrogen atom or $CR^{3d}$, $R^{3a}$, $R^{3c}$, and $R^{3d}$ are a hydrogen atom, and $R^{3b}$ is a C1-C6 alkyl group optionally substituted with one or more halogen atoms or a halogen atom.

[Embodiment 11] In the present compound N, a compound wherein $G^1$ is $CR^{3a}$, $G^2$ is $CR^{3b}$, G3 is $CR^{3c}$, $G^4$ is $CR^{3d}$, $R^{3a}$, $R^{3c}$, and $R^{3d}$ are a hydrogen atom, and $R^{3b}$ is a C1-C6 alkyl group optionally substituted with one or more halogen atoms or a halogen atom.

[Embodiment 12] In the present compound N, a compound wherein a combination of $G^1$, $G^2$, $G^3$, and $G^4$ is: a combination wherein $G^1$ is $CR^{3a}$, $G^2$ is $CR^{3b}$, $G^3$ is $CR^{3c}$, and $G^4$ is a nitrogen atom; a combination wherein $G^1$ is $CR^{3a}$, $G^2$ is $CR^{3b}$, $G^3$ is a nitrogen atom, and $G^4$ is $CR^{3d}$; a combination wherein $G^1$ is $CR^{3a}$, $G^2$ is a nitrogen atom, $G^3$ is $CR^{3c}$, and $G^4$ is $CR^{3d}$; a combination wherein $G^1$ is a nitrogen atom, $G^2$ is $CR^{3b}$, $G^3$ is $CR^{3c}$, and $G^4$ is $CR^{3d}$; or a combination wherein $G^1$ is $CR^{3a}$, $G^2$ is $CR^{3b}$, $G^3$ is $CR^{3c}$, and $G^4$ is $CR^{3d}$, $R^{3a}$, $R^{3b}$, and $R^{3d}$ are a hydrogen atom, and $R^{3c}$ is a halogen atom.

[Embodiment 13] In the present compound N, a compound wherein $G^1$ is $CR^{3a}$, $G^2$ is $CR^{3b}$, $G^3$ is $CR^{3c}$, $G^4$ is a nitrogen atom or $CR^{3d}$, $R^{3a}$, $R^{3b}$, and $R^{3d}$ are a hydrogen atom, and $R^{3c}$ is a halogen atom.

[Embodiment 14] In the present compound N, a compound wherein $G^1$ is $CR^{3a}$, $G^2$ is $CR^{3b}$, $G^3$ is $CR^{3c}$, $G^4$ is $CR^{3d}$, $R^{3a}$, $R^{3b}$, and $R^{3d}$ are a hydrogen atom, and $R^{3c}$ is a halogen atom.

[Embodiment 15] In Embodiment 4, a compound wherein $R^1$ is a C1-C6 chain hydrocarbon group optionally substituted with one or more substituent(s) selected from Group D.

[Embodiment 16] In Embodiment 5, a compound wherein $R^1$ is a C1-C6 chain hydrocarbon group optionally substituted with one or more substituent(s) selected from Group D.

[Embodiment 17] In Embodiment 6, a compound wherein $R^1$ is a C1-C6 chain hydrocarbon group optionally substituted with one or more substituent(s) selected from Group D.

[Embodiment 18] In Embodiment 7, a compound wherein $R^1$ is a C1-C6 chain hydrocarbon group optionally substituted with one or more substituent(s) selected from Group D.

[Embodiment 19] In Embodiment 8, a compound wherein $R^1$ is a C1-C6 chain hydrocarbon group optionally substituted with one or more substituent(s) selected from Group D.

[Embodiment 20] In Embodiment 9, a compound wherein $R^1$ is a C1-C6 chain hydrocarbon group optionally substituted with one or more substituent(s) selected from Group D.

[Embodiment 21] In Embodiment 10, a compound wherein $R^1$ is a C1-C6 chain hydrocarbon group optionally substituted with one or more substituent(s) selected from Group D.

[Embodiment 22] In Embodiment 11, a compound wherein $R^1$ is a C1-C6 chain hydrocarbon group optionally substituted with one or more substituent(s) selected from Group D.

[Embodiment 23] In Embodiment 12, a compound wherein $R^1$ is a C1-C6 chain hydrocarbon group optionally substituted with one or more substituent(s) selected from Group D.

[Embodiment 24] In Embodiment 13, a compound wherein $R^1$ is a C1-C6 chain hydrocarbon group optionally substituted with one or more substituent(s) selected from Group D.

[Embodiment 25] In Embodiment 14, a compound wherein $R^1$ is a C1-C6 chain hydrocarbon group optionally substituted with one or more substituent(s) selected from Group D.

[Embodiment 26] In Embodiment 4, a compound wherein $R^1$ is a C1-C6 alkyl group.

[Embodiment 27] In Embodiment 5, a compound wherein $R^1$ is a C1-C6 alkyl group.

[Embodiment 28] In Embodiment 6, a compound wherein $R^1$ is a C1-C6 alkyl group.

[Embodiment 29] In Embodiment 7, a compound wherein $R^1$ is a C1-C6 alkyl group.

[Embodiment 30] In Embodiment 8, a compound wherein $R^1$ is a C1-C6 alkyl group.

[Embodiment 31] In Embodiment 9, a compound wherein $R^1$ is a C1-C6 alkyl group.

[Embodiment 32] In Embodiment 10, a compound wherein $R^1$ is a C1-C6 alkyl group.

[Embodiment 33] In Embodiment 11, a compound wherein $R^1$ is a C1-C6 alkyl group.

[Embodiment 34] In Embodiment 12, a compound wherein $R^1$ is a C1-C6 alkyl group.

[Embodiment 35] In Embodiment 13, a compound wherein $R^1$ is a C1-C6 alkyl group.

[Embodiment 36] In Embodiment 14, a compound wherein $R^1$ is a C1-C6 alkyl group.

[Embodiment 37] In Embodiment 4, a compound wherein $R^1$ is a methyl group.

[Embodiment 38] In Embodiment 5, a compound wherein $R^1$ is a methyl group.
[Embodiment 39] In Embodiment 6, a compound wherein $R^1$ is a methyl group.
[Embodiment 40] In Embodiment 7, a compound wherein $R^1$ is a methyl group.
[Embodiment 41] In Embodiment 8, a compound wherein $R^1$ is a methyl group.
[Embodiment 42] In Embodiment 9, a compound wherein $R^1$ is a methyl group.
[Embodiment 43] In Embodiment 10, a compound wherein $R^1$ is a methyl group.
[Embodiment 44] In Embodiment 11, a compound wherein $R^1$ is a methyl group.
[Embodiment 45] In Embodiment 12, a compound wherein $R^1$ is a methyl group.
[Embodiment 46] In Embodiment 13, a compound wherein $R^1$ is a methyl group.
[Embodiment 47] In Embodiment 14, a compound wherein $R^1$ is a methyl group.
[Embodiment 48] In Embodiment 8, a compound wherein $R^2$ is a C1-C6 alkyl group.
[Embodiment 49] In Embodiment 9, a compound wherein $R^2$ is a C1-C6 alkyl group.
[Embodiment 50] In Embodiment 10, a compound wherein $R^2$ is a C1-C6 alkyl group.
[Embodiment 51] In Embodiment 11, a compound wherein $R^2$ is a C1-C6 alkyl group.
[Embodiment 52] In Embodiment 12, a compound wherein $R^2$ is a C1-C6 alkyl group.
[Embodiment 53] In Embodiment 13, a compound wherein $R^2$ is a C1-C6 alkyl group.
[Embodiment 54] In Embodiment 14, a compound wherein $R^2$ is a C1-C6 alkyl group.
[Embodiment 55] In Embodiment 8, a compound wherein $R^2$ is an ethyl group.
[Embodiment 56] In Embodiment 9, a compound wherein $R^2$ is an ethyl group.
[Embodiment 57] In Embodiment 10, a compound wherein $R^2$ is an ethyl group.
[Embodiment 58] In Embodiment 11, a compound wherein $R^2$ is an ethyl group.
[Embodiment 59] In Embodiment 12, a compound wherein $R^2$ is an ethyl group.
[Embodiment 60] In Embodiment 13, a compound wherein $R^2$ is an ethyl group.
[Embodiment 61] In Embodiment 14, a compound wherein $R^2$ is an ethyl group.
[Embodiment 62] In Embodiment 8, a compound wherein $R^2$ is $NR^6R^7$, and $R^6$ and $R^7$ are the same as or different from each other and are a C1-C6 alkyl group or a hydrogen atom.
[Embodiment 63] In Embodiment 9, a compound wherein $R^2$ is $NR^6R^7$, and $R^6$ and $R^7$ are the same as or different from each other and are a C1-C6 alkyl group or a hydrogen atom.
[Embodiment 64] In Embodiment 10, a compound wherein $R^2$ is $NR^6R^7$, and $R^6$ and $R^7$ are the same as or different from each other and are a C1-C6 alkyl group or a hydrogen atom.
[Embodiment 65] In Embodiment 11, a compound wherein $R^2$ is $NR^6R^7$, and $R^6$ and $R^7$ are the same as or different from each other and are a C1-C6 alkyl group or a hydrogen atom.
[Embodiment 66] In Embodiment 12, a compound wherein $R^2$ is $NR^6R^7$, and $R^6$ and $R^7$ are the same as or different from each other and are a C1-C6 alkyl group or a hydrogen atom.
[Embodiment 67] In Embodiment 13, a compound wherein $R^2$ is $NR^6R^7$, and $R^6$ and $R^7$ are the same as or different from each other and are a C1-C6 alkyl group or a hydrogen atom.
[Embodiment 68] In Embodiment 14, a compound wherein $R^2$ is $NR^6R^7$, and $R^6$ and $R^7$ are the same as or different from each other and are a C1-C6 alkyl group or a hydrogen atom.
[Embodiment 69] In Embodiment 8, a compound wherein $R^2$ is $NR^6R^7$, and $R^6$ and $R^7$ are combined with the nitrogen atom to which they are attached to form an aziridinyl group, an azetidinyl group, a pyrrolidinyl group, or a piperidinyl group.
[Embodiment 70] In Embodiment 9, a compound wherein $R^2$ is $NR^6R^7$, and $R^6$ and $R^7$ are combined with the nitrogen atom to which they are attached to form an aziridinyl group, an azetidinyl group, a pyrrolidinyl group, or a piperidinyl group.
[Embodiment 71] In Embodiment 10, a compound wherein $R^2$ is $NR^6R^7$, and $R^6$ and $R^7$ are combined with the nitrogen atom to which they are attached to form an aziridinyl group, an azetidinyl group, a pyrrolidinyl group, or a piperidinyl group.
[Embodiment 72] In Embodiment 11, a compound wherein $R^2$ is $NR^6R^7$, and $R^6$ and $R^7$ are combined with the nitrogen atom to which they are attached to form an aziridinyl group, an azetidinyl group, a pyrrolidinyl group, or a piperidinyl group.
[Embodiment 73] In Embodiment 12, a compound wherein $R^2$ is $NR^6R^7$, and $R^6$ and $R^7$ are combined with the nitrogen atom to which they are attached to form an aziridinyl group, an azetidinyl group, a pyrrolidinyl group, or a piperidinyl group.
[Embodiment 74] In Embodiment 13, a compound wherein $R^2$ is $NR^6R^7$, and $R^6$ and $R^7$ are combined with the nitrogen atom to which they are attached to form an aziridinyl group, an azetidinyl group, a pyrrolidinyl group, or a piperidinyl group.
[Embodiment 75] In Embodiment 14, a compound wherein $R^2$ is $NR^6R^7$, and $R^6$ and $R^7$ are combined with the nitrogen atom to which they are attached to form an aziridinyl group, an azetidinyl group, a pyrrolidinyl group, or a piperidinyl

group.
[Embodiment 76] In Embodiment 9, a compound wherein W is an oxygen atom.
[Embodiment 77] In Embodiment 10, a compound wherein W is an oxygen atom.
[Embodiment 78] In Embodiment 11, a compound wherein W is an oxygen atom.
[Embodiment 79] In Embodiment 12, a compound wherein W is an oxygen atom.
[Embodiment 80] In Embodiment 13, a compound wherein W is an oxygen atom.
[Embodiment 81] In Embodiment 14, a compound wherein W is an oxygen atom.
[Embodiment 82] In the present compound N, a compound wherein $R^1$ is a C1-C6 alkyl group and $R^2$ is an ethyl group.
[Embodiment 83] In the present compound N, a compound wherein $R^1$ is a C1-C6 alkyl group and W is an oxygen atom.
[Embodiment 84] In the present compound N, a compound wherein $R^2$ is a C1-C6 alkyl group and W is an oxygen atom.
[Embodiment 85] In the present compound N, a compound wherein $R^2$ is an ethyl group and W is an oxygen atom.
[Embodiment 86] In the present compound N, a compound wherein $R^1$ is a methyl group and $R^2$ is an ethyl group.
[Embodiment 87] In the present compound N, a compound wherein $R^1$ is a methyl group and W is an oxygen atom.
[Embodiment 88] In the present compound N, a compound wherein $R^1$ is a C1-C6 alkyl group, $R^2$ is $NR^6R^7$, and $R^6$ and $R^7$ are the same as or different from each other and are a C1-C6 alkyl group or a hydrogen atom.
[Embodiment 89] In the present compound N, a compound wherein $R^1$ is a C1-C6 alkyl group, $R^2$ is $NR^6R^7$, and $R^6$ and $R^7$ are combined with the nitrogen atom to which they are attached to form an aziridinyl group, an azetidinyl group, a pyrrolidinyl group, or a piperidinyl group.
[Embodiment 90] In the present compound N, a compound wherein $R^1$ is a methyl group, $R^2$ is $NR^6R^7$, and $R^6$ and $R^7$ are the same as or different from each other and are a C1-C6 alkyl group or a hydrogen atom.
[Embodiment 91] In the present compound N, a compound wherein $R^1$ is a methyl group, $R^2$ is $NR^6R^7$, and $R^6$ and $R^7$ are combined with the nitrogen atom to which they are attached to form an aziridinyl group, an azetidinyl group, a pyrrolidinyl group, or a piperidinyl group.
[Embodiment 92] In the present compound N, a compound wherein $R^2$ is $NR^6R^7$, $R^6$ and $R^7$ are the same as or different from each other and are a C1-C6 alkyl group or a hydrogen atom, and W is an oxygen atom.
[Embodiment 93] In the present compound N, a compound wherein $R^2$ is $NR^6R^7$, $R^6$ and $R^7$ are combined with the nitrogen atom to which they are attached to form an aziridinyl group, an azetidinyl group, a pyrrolidinyl group, or a piperidinyl group, and W is an oxygen atom.
[Embodiment 94] In Embodiment 8, a compound wherein $R^1$ is a C1-C6 alkyl group, and $R^2$ is an ethyl group.
[Embodiment 95] In Embodiment 9, a compound wherein $R^1$ is a C1-C6 alkyl group, and $R^2$ is an ethyl group.
[Embodiment 96] In Embodiment 10, a compound wherein $R^1$ is a C1-C6 alkyl group, and $R^2$ is an ethyl group.
[Embodiment 97] In Embodiment 11, a compound wherein $R^1$ is a C1-C6 alkyl group, and $R^2$ is an ethyl group.
[Embodiment 98] In Embodiment 12, a compound wherein $R^1$ is a C1-C6 alkyl group, and $R^2$ is an ethyl group.
[Embodiment 99] In Embodiment 13, a compound wherein $R^1$ is a C1-C6 alkyl group, and $R^2$ is an ethyl group.
[Embodiment 100] In Embodiment 14, a compound wherein $R^1$ is a C1-C6 alkyl group, and $R^2$ is an ethyl group.
[Embodiment 101] In Embodiment 4, a compound wherein $R^1$ is a C1-C6 alkyl group, and W is an oxygen atom.
[Embodiment 102] In Embodiment 5, a compound wherein $R^1$ is a C1-C6 alkyl group, and W is an oxygen atom.
[Embodiment 103] In Embodiment 6, a compound wherein $R^1$ is a C1-C6 alkyl group, and W is an oxygen atom.
[Embodiment 104] In Embodiment 7, a compound wherein $R^1$ is a C1-C6 alkyl group, and W is an oxygen atom.
[Embodiment 105] In Embodiment 9, a compound wherein $R^1$ is a C1-C6 alkyl group, and W is an oxygen atom.
[Embodiment 106] In Embodiment 10, a compound wherein $R^1$ is a C1-C6 alkyl group, and W is an oxygen atom.
[Embodiment 107] In Embodiment 11, a compound wherein $R^1$ is a C1-C6 alkyl group, and W is an oxygen atom.
[Embodiment 108] In Embodiment 12, a compound wherein $R^1$ is a C1-C6 alkyl group, and W is an oxygen atom.
[Embodiment 109] In Embodiment 13, a compound wherein $R^1$ is a C1-C6 alkyl group, and W is an oxygen atom.
[Embodiment 110] In Embodiment 14, a compound wherein $R^1$ is a C1-C6 alkyl group, and W is an oxygen atom.
[Embodiment 111] In Embodiment 8, a compound wherein $R^1$ is a methyl group, and $R^2$ is an ethyl group.
[Embodiment 112] In Embodiment 9, a compound wherein $R^1$ is a methyl group, and $R^2$ is an ethyl group.
[Embodiment 113] In Embodiment 10, a compound wherein $R^1$ is a methyl group, and $R^2$ is an ethyl group.
[Embodiment 114] In Embodiment 11, a compound wherein $R^1$ is a methyl group, and $R^2$ is an ethyl group.
[Embodiment 115] In Embodiment 12, a compound wherein $R^1$ is a methyl group, and $R^2$ is an ethyl group.
[Embodiment 116] In Embodiment 13, a compound wherein $R^1$ is a methyl group, and $R^2$ is an ethyl group.
[Embodiment 117] In Embodiment 14, a compound wherein $R^1$ is a methyl group, and $R^2$ is an ethyl group.
[Embodiment 118] In Embodiment 4, a compound wherein $R^1$ is a methyl group, and W is an oxygen atom.
[Embodiment 119] In Embodiment 5, a compound wherein $R^1$ is a methyl group, and W is an oxygen atom.
[Embodiment 120] In Embodiment 6, a compound wherein $R^1$ is a methyl group, and W is an oxygen atom.
[Embodiment 121] In Embodiment 7, a compound wherein $R^1$ is a methyl group, and W is an oxygen atom.

[Embodiment 122] In Embodiment 9, a compound wherein $R^1$ is a methyl group, and W is an oxygen atom.
[Embodiment 123] In Embodiment 10, a compound wherein $R^1$ is a methyl group, and W is an oxygen atom.
[Embodiment 124] In Embodiment 11, a compound wherein $R^1$ is a methyl group, and W is an oxygen atom.
[Embodiment 125] In Embodiment 12, a compound wherein $R^1$ is a methyl group, and W is an oxygen atom.
[Embodiment 126] In Embodiment 13, a compound wherein $R^1$ is a methyl group, and W is an oxygen atom.
[Embodiment 127] In Embodiment 14, a compound wherein $R^1$ is a methyl group, and W is an oxygen atom.
[Embodiment 128] In Embodiment 1, a compound wherein $R^2$ is a C1-C6 alkyl group, and W is an oxygen atom.
[Embodiment 129] In Embodiment 2, a compound wherein $R^2$ is a C1-C6 alkyl group, and W is an oxygen atom.
[Embodiment 130] In Embodiment 3, a compound wherein $R^2$ is a C1-C6 alkyl group, and W is an oxygen atom.
[Embodiment 131] In Embodiment 9, a compound wherein $R^2$ is a C1-C6 alkyl group, and W is an oxygen atom.
[Embodiment 132] In Embodiment 10, a compound wherein $R^2$ is a C1-C6 alkyl group, and W is an oxygen atom.
[Embodiment 133] In Embodiment 11, a compound wherein $R^2$ is a C1-C6 alkyl group, and W is an oxygen atom.
[Embodiment 134] In Embodiment 12, a compound wherein $R^2$ is a C1-C6 alkyl group, and W is an oxygen atom.
[Embodiment 135] In Embodiment 13, a compound wherein $R^2$ is a C1-C6 alkyl group, and W is an oxygen atom.
[Embodiment 136] In Embodiment 14, a compound wherein $R^2$ is a C1-C6 alkyl group, and W is an oxygen atom.
[Embodiment 137] In Embodiment 1, a compound wherein $R^2$ is an ethyl group, and W is an oxygen atom.
[Embodiment 138] In Embodiment 2, a compound wherein $R^2$ is an ethyl group, and W is an oxygen atom.
[Embodiment 139] In Embodiment 3, a compound wherein $R^2$ is an ethyl group, and W is an oxygen atom.
[Embodiment 140] In Embodiment 9, a compound wherein $R^2$ is an ethyl group, and W is an oxygen atom.
[Embodiment 141] In Embodiment 10, a compound wherein $R^2$ is an ethyl group, and W is an oxygen atom.
[Embodiment 142] In Embodiment 11, a compound wherein $R^2$ is an ethyl group, and W is an oxygen atom.
[Embodiment 143] In Embodiment 12, a compound wherein $R^2$ is an ethyl group, and W is an oxygen atom.
[Embodiment 144] In Embodiment 13, a compound wherein $R^2$ is an ethyl group, and W is an oxygen atom.
[Embodiment 145] In Embodiment 14, a compound wherein $R^2$ is an ethyl group, and W is an oxygen atom.
[Embodiment 146] In Embodiment 8, a compound wherein $R^1$ is a C1-C6 alkyl group, $R^2$ is $NR^6R^7$, and $R^6$ and $R^7$ are the same as or different from each other and are a C1-C6 alkyl group or a hydrogen atom.
[Embodiment 147] In Embodiment 9, a compound wherein $R^1$ is a C1-C6 alkyl group, $R^2$ is $NR^6R^7$, and $R^6$ and $R^7$ are the same as or different from each other and are a C1-C6 alkyl group or a hydrogen atom.
[Embodiment 148] In Embodiment 10, a compound wherein $R^1$ is a C1-C6 alkyl group, $R^2$ is $NR^6R^7$, and $R^6$ and $R^7$ are the same as or different from each other and are a C1-C6 alkyl group or a hydrogen atom.
[Embodiment 149] In Embodiment 11, a compound wherein $R^1$ is a C1-C6 alkyl group, $R^2$ is $NR^6R^7$, and $R^6$ and $R^7$ are the same as or different from each other and are a C1-C6 alkyl group or a hydrogen atom.
[Embodiment 150] In Embodiment 12, a compound wherein $R^1$ is a C1-C6 alkyl group, $R^2$ is $NR^6R^7$, and $R^6$ and $R^7$ are the same as or different from each other and are a C1-C6 alkyl group or a hydrogen atom.
[Embodiment 151] In Embodiment 13, a compound wherein $R^1$ is a C1-C6 alkyl group, $R^2$ is $NR^6R^7$, and $R^6$ and $R^7$ are the same as or different from each other and are a C1-C6 alkyl group or a hydrogen atom.
[Embodiment 152] In Embodiment 14, a compound wherein $R^1$ is a C1-C6 alkyl group, $R^2$ is $NR^6R^7$, and $R^6$ and $R^7$ are the same as or different from each other and are a C1-C6 alkyl group or a hydrogen atom.
[Embodiment 153] In Embodiment 8, a compound wherein $R^1$ is a C1-C6 alkyl group, $R^2$ is $NR^6R^7$, and $R^6$ and $R^7$ are combined with the nitrogen atom to which they are attached to form an aziridinyl group, an azetidinyl group, a pyrrolidinyl group, or a piperidinyl group.
[Embodiment 154] In Embodiment 9, a compound wherein $R^1$ is a C1-C6 alkyl group, $R^2$ is $NR^6R^7$, and $R^6$ and $R^7$ are combined with the nitrogen atom to which they are attached to form an aziridinyl group, an azetidinyl group, a pyrrolidinyl group, or a piperidinyl group.
[Embodiment 155] In Embodiment 10, a compound wherein $R^1$ is a C1-C6 alkyl group, $R^2$ is $NR^6R^7$, and $R^6$ and $R^7$ are combined with the nitrogen atom to which they are attached to form an aziridinyl group, an azetidinyl group, a pyrrolidinyl group, or a piperidinyl group.
[Embodiment 156] In Embodiment 11, a compound wherein $R^1$ is a C1-C6 alkyl group, $R^2$ is $NR^6R^7$, and $R^6$ and $R^7$ are combined with the nitrogen atom to which they are attached to form an aziridinyl group, an azetidinyl group, a pyrrolidinyl group, or a piperidinyl group.
[Embodiment 157] In Embodiment 12, a compound wherein $R^1$ is a C1-C6 alkyl group, $R^2$ is $NR^6R^7$, and $R^6$ and $R^7$ are combined with the nitrogen atom to which they are attached to form an aziridinyl group, an azetidinyl group, a pyrrolidinyl group, or a piperidinyl group.
[Embodiment 158] In Embodiment 13, a compound wherein $R^1$ is a C1-C6 alkyl group, $R^2$ is $NR^6R^7$, and $R^6$ and $R^7$ are combined with the nitrogen atom to which they are attached to form an aziridinyl group, an azetidinyl group, a pyrrolidinyl group, or a piperidinyl group.
[Embodiment 159] In Embodiment 14, a compound wherein $R^1$ is a C1-C6 alkyl group, $R^2$ is $NR^6R^7$, and $R^6$ and $R^7$ are combined with the nitrogen atom to which they are attached to form an aziridinyl group, an azetidinyl group, a

pyrrolidinyl group, or a piperidinyl group.

[Embodiment 160] In Embodiment 8, a compound wherein $R^1$ is a methyl group, $R^2$ is $NR^6R^7$, and $R^6$ and $R^7$ are the same as or different from each other and are a C1-C6 alkyl group or a hydrogen atom.

[Embodiment 161] In Embodiment 9, a compound wherein $R^1$ is a methyl group, $R^2$ is $NR^6R^7$, and $R^6$ and $R^7$ are the same as or different from each other and are a C1-C6 alkyl group or a hydrogen atom.

[Embodiment 162] In Embodiment 10, a compound wherein $R^1$ is a methyl group, $R^2$ is $NR^6R^7$, and $R^6$ and $R^7$ are the same as or different from each other and are a C1-C6 alkyl group or a hydrogen atom.

[Embodiment 163] In Embodiment 11, a compound wherein $R^1$ is a methyl group, $R^2$ is $NR^6R^7$, and $R^6$ and $R^7$ are the same as or different from each other and are a C1-C6 alkyl group or a hydrogen atom.

[Embodiment 164] In Embodiment 12, a compound wherein $R^1$ is a methyl group, $R^2$ is $NR^6R^7$, and $R^6$ and $R^7$ are the same as or different from each other and are a C1-C6 alkyl group or a hydrogen atom.

[Embodiment 165] In Embodiment 13, a compound wherein $R^1$ is a methyl group, $R^2$ is $NR^6R^7$, and $R^6$ and $R^7$ are the same as or different from each other and are a C1-C6 alkyl group or a hydrogen atom.

[Embodiment 166] In Embodiment 14, a compound wherein $R^1$ is a methyl group, $R^2$ is $NR^6R^7$, and $R^6$ and $R^7$ are the same as or different from each other and are a C1-C6 alkyl group or a hydrogen atom.

[Embodiment 167] In Embodiment 8, a compound wherein $R^1$ is a methyl group, $R^2$ is $NR^6R^7$, and $R^6$ and $R^7$ are combined with the nitrogen atom to which they are attached to form an aziridinyl group, an azetidinyl group, a pyrrolidinyl group, or a piperidinyl group.

[Embodiment 168] In Embodiment 9, a compound wherein $R^1$ is a methyl group, $R^2$ is $NR^6R^7$, and $R^6$ and $R^7$ are combined with the nitrogen atom to which they are attached to form an aziridinyl group, an azetidinyl group, a pyrrolidinyl group, or a piperidinyl group.

[Embodiment 169] In Embodiment 10, a compound wherein $R^1$ is a methyl group, $R^2$ is $NR^6R^7$, and $R^6$ and $R^7$ are combined with the nitrogen atom to which they are attached to form an aziridinyl group, an azetidinyl group, a pyrrolidinyl group, or a piperidinyl group.

[Embodiment 170] In Embodiment 11, a compound wherein $R^1$ is a methyl group, $R^2$ is $NR^6R^7$, and $R^6$ and $R^7$ are combined with the nitrogen atom to which they are attached to form an aziridinyl group, an azetidinyl group, a pyrrolidinyl group, or a piperidinyl group.

[Embodiment 171] In Embodiment 12, a compound wherein $R^1$ is a methyl group, $R^2$ is $NR^6R^7$, and $R^6$ and $R^7$ are combined with the nitrogen atom to which they are attached to form an aziridinyl group, an azetidinyl group, a pyrrolidinyl group, or a piperidinyl group.

[Embodiment 172] In Embodiment 13, a compound wherein $R^1$ is a methyl group, $R^2$ is $NR^6R^7$, and $R^6$ and $R^7$ are combined with the nitrogen atom to which they are attached to form an aziridinyl group, an azetidinyl group, a pyrrolidinyl group, or a piperidinyl group.

[Embodiment 173] In Embodiment 14, a compound wherein $R^1$ is a methyl group, $R^2$ is $NR^6R^7$, and $R^6$ and $R^7$ are combined with the nitrogen atom to which they are attached to form an aziridinyl group, an azetidinyl group, a pyrrolidinyl group, or a piperidinyl group.

[Embodiment 174] In Embodiment 1, a compound wherein $R^2$ is $NR^6R^7$, $R^6$ and $R^7$ are the same as or different from each other and are a C1-C6 alkyl group or a hydrogen atom, and W is an oxygen atom.

[Embodiment 175] In Embodiment 2, a compound wherein $R^2$ is $NR^6R^7$, $R^6$ and $R^7$ are the same as or different from each other and are a C1-C6 alkyl group or a hydrogen atom, and W is an oxygen atom.

[Embodiment 176] In Embodiment 3, a compound wherein $R^2$ is $NR^6R^7$, $R^6$ and $R^7$ are the same as or different from each other and are a C1-C6 alkyl group or a hydrogen atom, and W is an oxygen atom.

[Embodiment 177] In Embodiment 9, a compound wherein $R^2$ is $NR^6R^7$, $R^6$ and $R^7$ are the same as or different from each other and are a C1-C6 alkyl group or a hydrogen atom, and W is an oxygen atom.

[Embodiment 178] In Embodiment 10, a compound wherein $R^2$ is $NR^6R^7$, $R^6$ and $R^7$ are the same as or different from each other and are a C1-C6 alkyl group or a hydrogen atom, and W is an oxygen atom.

[Embodiment 179] In Embodiment 11, a compound wherein $R^2$ is $NR^6R^7$, $R^6$ and $R^7$ are the same as or different from each other and are a C1-C6 alkyl group or a hydrogen atom, and W is an oxygen atom.

[Embodiment 180] In Embodiment 12, a compound wherein $R^2$ is $NR^6R^7$, $R^6$ and $R^7$ are the same as or different from each other and are a C1-C6 alkyl group or a hydrogen atom, and W is an oxygen atom.

[Embodiment 181] In Embodiment 13, a compound wherein $R^2$ is $NR^6R^7$, $R^6$ and $R^7$ are the same as or different from each other and are a C1-C6 alkyl group or a hydrogen atom, and W is an oxygen atom.

[Embodiment 182] In Embodiment 14, a compound wherein $R^2$ is $NR^6R^7$, $R^6$ and $R^7$ are the same as or different from each other and are a C1-C6 alkyl group or a hydrogen atom, and W is an oxygen atom.

[Embodiment 183] In Embodiment 1, a compound wherein $R^2$ is $NR^6R^7$, $R^6$ and $R^7$ are combined with the nitrogen atom to which they are attached to form an aziridinyl group, an azetidinyl group, a pyrrolidinyl group, or a piperidinyl group, and W is an oxygen atom.

[Embodiment 184] In Embodiment 2, a compound wherein $R^2$ is $NR^6R^7$, $R^6$ and $R^7$ are combined with the nitrogen

atom to which they are attached to form an aziridinyl group, an azetidinyl group, a pyrrolidinyl group, or a piperidinyl group, and W is an oxygen atom.

[Embodiment 185] In Embodiment 3, a compound wherein $R^2$ is $NR^6R^7$, $R^6$ and $R^7$ are combined with the nitrogen atom to which they are attached to form an aziridinyl group, an azetidinyl group, a pyrrolidinyl group, or a piperidinyl group, and W is an oxygen atom.

[Embodiment 186] In Embodiment 9, a compound wherein $R^2$ is $NR^6R^7$, $R^6$ and $R^7$ are combined with the nitrogen atom to which they are attached thereto, to form an aziridinyl group, an azetidinyl group, a pyrrolidinyl group, or a piperidinyl group, and W is an oxygen atom.

[Embodiment 187] In Embodiment 10, a compound wherein $R^2$ is $NR^6R^7$, $R^6$ and $R^7$ are combined with the nitrogen atom to which they are attached to form an aziridinyl group, an azetidinyl group, a pyrrolidinyl group, or a piperidinyl group, and W is an oxygen atom.

[Embodiment 188] In Embodiment 11, a compound wherein $R^2$ is $NR^6R^7$, $R^6$ and $R^7$ are combined with the nitrogen atom to which they are attached to form an aziridinyl group, an azetidinyl group, a pyrrolidinyl group, or a piperidinyl group, and W is an oxygen atom.

[Embodiment 189] In Embodiment 12, a compound wherein $R^2$ is $NR^6R^7$, $R^6$ and $R^7$ are combined with the nitrogen atom to which they are attached to form an aziridinyl group, an azetidinyl group, a pyrrolidinyl group, or a piperidinyl group, and W is an oxygen atom.

[Embodiment 190] In Embodiment 13, a compound wherein $R^2$ is $NR^6R^7$, $R^6$ and $R^7$ are combined with the nitrogen atom to which they are attached to form an aziridinyl group, an azetidinyl group, a pyrrolidinyl group, or a piperidinyl group, and W is an oxygen atom.

[Embodiment 191] In Embodiment 14, a compound wherein $R^2$ is $NR^6R^7$, $R^6$ and $R^7$ are combined with the nitrogen atom to which they are attached to form an aziridinyl group, an azetidinyl group, a pyrrolidinyl group, or a piperidinyl group, and W is an oxygen atom.

[Embodiment 192] In any one of Embodiments 1 to 191 or the present compound N, a compound wherein Q is $Q^1$, a combination of $Z^1$, $X^1$, $X^2$ and $X^3$ is: a combination where $Z^1$ is an oxygen atom, $X^1$ is a nitrogen atom, $X^2$ is $CR^{3f}$, and $X^3$ is $CR^{3g}$; a combination where $Z^1$ is an oxygen atom, $X^1$ is $CR^{3e}$, $X^2$ is a nitrogen atom, and $X^3$ is $CR^{3g}$; a combination where $Z^1$ is an oxygen atom, $X^1$ is $CR^{3e}$, $X^2$ is $CR^{3f}$, and $X^3$ is a nitrogen atom; a combination where $Z^1$ is an oxygen atom, $X^1$ is $CR^{3e}$, $X^2$ is $CR^{3f}$, and $X^3$ is $CR^{3g}$; a combination where $Z^1$ is $NR^{3j}$, $X^1$ is a nitrogen atom, $X^2$ is $CR^{3f}$, and $X^3$ is $CR^{3g}$; a combination where $Z^1$ is $NR^{3j}$, $X^1$ is $CR^{3e}$, $X^2$ is a nitrogen atom, and $X^3$ is $CR^{3g}$; or a combination where $Z^1$ is $NR^{3j}$, $X^1$ is $CR^{3e}$, $X^2$ is $CR^{3f}$, and $X^3$ is a nitrogen atom, $R^{3e}$ is a hydrogen atom, $R^{3f}$ and $R^{3g}$ are the same as or different from each other and are a C1-C6 alkyl group optionally substituted with one or more halogen atoms, a hydrogen atom, or a halogen atom, and $R^{3j}$ is a methyl group optionally substituted with one or more halogen atoms, a C2-C4 alkyl group optionally substituted with one or more fluorine atoms, a C5-C6 alkyl group optionally substituted with one or more halogen atoms, or a C3-C7 cycloalkyl group optionally substituted with one or more halogen atoms.

[Embodiment 193] In any one of Embodiments 1 to 191 or the present compound, a compound wherein Q is $Q^2$, a combination of $Z^2$, $X^1$, $X^2$ and $X^4$ is: a combination where $Z^2$ is an oxygen atom, $X^1$ is a nitrogen atom, $X^2$ is $CR^{3f}$, and $X^4$ is $CR^{3h}$; a combination where Z is an oxygen atom, $X^1$ is $CR^{3e}$, $X^2$ is a nitrogen atom, and $X^4$ is $CR^{3h}$; a combination where $Z^2$ is an oxygen atom, $X^1$ is $CR^{3e}$, $X^2$ is $CR^{3f}$, and $X^4$ is a nitrogen atom; a combination where $Z^2$ is $NR^{3k}$, $X^1$ is a nitrogen atom, $X^2$ is $CR^{3f}$, and $X^4$ is $CR^{3h}$; a combination where $Z^2$ is $NR^{3k}$, $X^1$ is $CR^{3e}$, $X^2$ is a nitrogen atom, and $X^4$ is $CR^{3h}$; a combination where $Z^2$ is $NR^{3k}$, $X^1$ is $CR^{3e}$, $X^2$ is $CR^{3f}$, and $X^4$ is a nitrogen atom; or a combination where Z is $NR^{3k}$, $X^1$ is $CR^{3e}$, $X^2$ is $CR^{3f}$, and $X^4$ is $CR^{3h}$, $R^{3e}$ and $R^{3h}$ are the same as or different from each other and are a C1-C6 alkyl group optionally substituted with one or more halogen atoms, or a hydrogen atom, $R^{3f}$ is a C1-C6 alkyl group optionally substituted with one or more halogen atoms, $OR^{33}$, a hydrogen atom, or a halogen atom, and $R^{3k}$ is a C1-C6 alkyl group optionally substituted with one or more halogen atoms, or a C3-C7 cycloalkyl group optionally substituted with one or more halogen atoms.

[Embodiment 194] In any one of Embodiments 1 to 191 or the present compound N, a compound wherein Q is $Q^1$, a combination of $Z^1$, $X^1$, $X^2$, and $X^3$ is: a combination wherein $Z^1$ is an oxygen atom, $X^1$ is a nitrogen atom, $X^2$ is $CR^{3f}$, and $X^3$ is $CR^{3g}$; a combination wherein $Z^1$ is an oxygen atom, $X^1$ is $CR^{3e}$, $X^2$ is a nitrogen atom, and $X^3$ is $CR^{3g}$; a combination wherein $Z^1$ is an oxygen atom, $X^1$ is $CR^{3e}$, $X^2$ is $CR^{3f}$, and $X^3$ is a nitrogen atom; or a combination wherein $Z^1$ is an oxygen atom, $X^1$ is $CR^{3e}$, $X^2$ is $CR^{3f}$, and $X^3$ is $CR^{3g}$, $R^{3e}$ is a hydrogen atom, and $R^{3f}$ and $R^{3g}$ are the same as or different from each other and are a C1-C6 alkyl group optionally substituted with one or more halogen atoms, a hydrogen atom, or a halogen atom.

[Embodiment 195] In any one of Embodiments 1 to 191 or the present compound, a compound wherein Q is $Q^1$, a combination of $Z^1$, $X^1$, $X^2$ and $X^3$ is: a combination where $Z^1$ is $NR^{3j}$, $X^1$ is a nitrogen atom, $X^2$ is $CR^{3f}$, and $X^3$ is $CR^{3g}$; a combination where $Z^1$ is $NR^{3j}$, $X^1$ is $CR^{3e}$, $X^2$ is a nitrogen atom, and $X^3$ is $CR^{3g}$; or a combination where $Z^1$ is $NR^{3j}$, $X^1$ is $CR^{3e}$, $X^2$ is $CR^{3f}$, and $X^3$ is a nitrogen atom, $R^{3e}$ is a hydrogen atom, $R^{3f}$ and $R^{3g}$ are the same as or different from each other and are a C1-C6 alkyl group optionally substituted with one or more halogen atoms, a hydrogen atom, or a halogen atom, and $R^{3j}$ is a methyl group optionally substituted with one or more halogen atoms, a C2-C4 alkyl group

optionally substituted with one or more fluorine atoms, a C5-C6 alkyl group optionally substituted with one or more halogen atoms, or a C3-C7 cycloalkyl group optionally substituted with one or more halogen atoms.

[Embodiment 196] In any one of Embodiments 1 to 191 or the present compound, a compound wherein Q is $Q^2$, a combination of $Z^2$, $X^1$, $X^2$ and $X^4$ is: a combination where $Z^2$ is an oxygen atom, $X^1$ is a nitrogen atom, $X^2$ is $CR^{3f}$, and $X^4$ is $CR^{3h}$; a combination where $Z^2$ is an oxygen atom, $X^1$ is $CR^{3e}$, $X^2$ is a nitrogen atom, and $X^4$ is $CR^{3h}$; or a combination where $Z^2$ is an oxygen atom, $X^1$ is $CR^{3e}$, $X^2$ is $CR^{3f}$, and $X^4$ is a nitrogen atom, $R^{3e}$ and $R^{3h}$ are the same as or different from each other and are a C1-C6 alkyl group optionally substituted with one or more halogen atoms or a hydrogen atom, and $R^{3f}$ is the same as or different from each other and is a C1-C6 alkyl group optionally substituted with one or more halogen atoms, a hydrogen atom, or a halogen atom.

[Embodiment 197] In any one of Embodiments 1 to 191 or the present compound N, a compound wherein Q is $Q^2$, a combination of $Z^2$, $X^1$, $X^2$ and $X^4$ is: a combination where $Z^2$ is $NR^{3k}$, $X^1$ is a nitrogen atom, $X^2$ is $CR^{3f}$, and $X^4$ is $CR^{3h}$; a combination where $Z^2$ is $NR^{3k}$, $X^1$ is $CR^{3e}$, $X^2$ is a nitrogen atom, and $X^4$ is $CR^{3h}$; a combination where $Z^2$ is $NR^{3k}$, $X^1$ is $CR^{3e}$, $X^2$ is $CR^{3f}$, and $X^4$ is a nitrogen atom; or a combination where $Z^2$ is $NR^{3k}$, $X^1$ is $CR^{3e}$, $X^2$ is $CR^{3f}$, and $X^4$ is $CR^{3h}$, $R^{3e}$ and $R^{3h}$ are the same as or different from each other and are a C1-C6 alkyl group optionally substituted with one or more halogen atoms, or a hydrogen atom, $R^{3f}$ is a C1-C6 alkyl group optionally substituted with one or more halogen atoms, $OR^{33}$, a hydrogen atom, or a halogen atom.

[Embodiment 198] In any one of Embodiments 1 to 191 or the present compound N, a compound wherein Q is $Q^2$, a combination of $Z^2$, $X^1$, $X^2$ and $X^4$ is: a combination where $Z^2$ is $NR^{3k}$, $X^1$ is a nitrogen atom, $X^2$ is $CR^{3f}$, and $X^4$ is $CR^{3h}$; a combination where $Z^2$ is $NR^{3k}$, $X^1$ is $CR^{3e}$, $X^2$ is a nitrogen atom, and $X^4$ is $CR^{3h}$; or a combination where $Z^2$ is $NR^{3k}$, $X^1$ is $CR^{3e}$, $X^2$ is $CR^{3f}$, and $X^4$ is a nitrogen atom; or a combination where $Z^2$ is $NR^{3k}$, $X^1$ is $CR^{3e}$, $X^2$ is $CR^{3f}$, and $X^4$ is $CR^{3h}$, $R^{3e}$ and $R^{3h}$ are the same as or different from each other and are a C1-C6 alkyl group optionally substituted with one or more halogen atoms, or a hydrogen atom, $R^{3f}$ is a C1-C6 alkyl group optionally substituted with one or more halogen atoms, a hydrogen atom, or a halogen atom.

[Embodiment 199] In any one of Embodiments 1 to 191 or the present compound N, a compound wherein Q is $Q^1$, $Z^1$ is $NR^{3j}$, $X^1$ is $CR^{3e}$, $X^2$ is $CR^{3f}$, $X^3$ is a nitrogen atom, $R^{3e}$ is a hydrogen atom, $R^{3f}$ is a C1-C6 alkyl group optionally substituted with one or more halogen atoms, a hydrogen atom, or a halogen atom, and $R^{3j}$ is a methyl group optionally substituted with one or more halogen atoms, a C2-C4 alkyl group optionally substituted with one or more fluorine atoms, a C5-C6 alkyl group optionally substituted with one or more halogen atoms, or a C3-C7 cycloalkyl group optionally substituted with one or more halogen atoms.

[Embodiment 200] In any one of Embodiments 1 to 191 or the present compound N, a compound wherein Q is $Q^1$, $Z^1$ is an oxygen atom, $X^1$ is $CR^{3e}$, $X^2$ is $CR^{3f}$, $X^3$ is a nitrogen atom, $R^{3e}$ is a hydrogen atom, and $R^{3f}$ is a C1-C6 alkyl group optionally substituted with one or more halogen atoms, a hydrogen atom, or a halogen atom.

[Embodiment 201] In any one of Embodiments 1 to 191 or the present compound N, a compound wherein Q is $Q^1$, Z is an oxygen atom, $X^1$ is $CR^{3e}$, $X^2$ is $CR^{3f}$, $X^3$ is $CR^{3g}$, $R^{3e}$ and $R^{3f}$ are a hydrogen atom, and $R^{3g}$ is a C1-C6 alkyl group optionally substituted with one or more halogen atoms, a hydrogen atom, or a halogen atom.

[Embodiment 202] In any one of Embodiments 1 to 191 or the present compound N, a compound wherein Q is $Q^2$, $Z^2$ is $NR^{3k}$, $X^1$ is $CR^{3e}$, $X^2$ is $CR^{3f}$, $X^4$ is a nitrogen atom, $R^{3e}$ is a hydrogen atom, $R^{3f}$ is a C1-C6 alkyl group optionally substituted with one or more halogen atoms, or a halogen atom, and $R^{3k}$ is a C1-C6 alkyl group optionally substituted with one or more halogen atoms, or a C3-C7 cycloalkyl group optionally substituted with one or more halogen atoms.

[Embodiment 203] In any one of Embodiments 1 to 191 or the present compound N, a compound wherein Q is $Q^2$, and Z is $NR^{3j}$, $X^1$ is $CR^{3e}$, $X^2$ is a nitrogen atom, $X^4$ is $CR^{3h}$, $R^{3e}$ and $R^{3h}$ are a hydrogen atom, and $R^{3j}$ is a C1-C6 alkyl group optionally substituted with one or more halogen atoms or a C3-C7 cycloalkyl group optionally substituted with one or more halogen atoms.

[Embodiment 204] In any one of Embodiments 1 to 191 or the present compound N, a compound wherein Q is $Q^2$, $Z^2$ is an oxygen atom, $X^1$ is $CR^{3e}$, $X^2$ is a nitrogen atom, $X^4$ is $CR^{3h}$, $R^{3e}$ and $R^{3h}$ are the same as or different from each other and are a C1-C6 alkyl group optionally substituted with one or more halogen atoms, or a hydrogen atom.

[Embodiment 205] In any one of Embodiments 1 to 191 or the present compound N, a compound wherein Q is $Q^2$, $Z^2$ is an oxygen atom, $X^1$ is $CR^{3e}$, $X^2$ is $CR^{3f}$, $X^4$ is a nitrogen atom, $R^{3e}$ is a hydrogen atom, and $R^{3f}$ is a C1-C6 alkyl group optionally substituted with one or more halogen atoms, a hydrogen atom, or a halogen atom.

[Embodiment 206] In any one of Embodiments 1 to 191 or the present compound N, a compound wherein Q is $Q^1$, $Z^1$ is a sulfur atom, $X^1$ is $CR^{3e}$, $X^2$ is a nitrogen atom, $X^3$ is $CR^{3g}$, $R^{3e}$ is a hydrogen atom, and $R^{3g}$ is a C1-C6 alkyl group optionally substituted with one or more halogen atoms, a hydrogen atom, or a halogen atom.

[Embodiment 207] In Embodiments 1 to 191 or the present compound N, a compound wherein Q is $Q^1$, $Z^1$ is a sulfur atom, $X^1$ is a nitrogen atom, $X^2$ is $CR^{3f}$, $X^3$ is $CR^{3g}$, $R^{3e}$ is a hydrogen atom, and $R^{3g}$ is a C1-C6 alkyl group optionally substituted with one or more halogen atoms, a hydrogen atom, or a halogen atom.

Examples of the embodiment of the compound represented by the formula (II) (hereinafter, also referred to as Intermediate C) include the following compounds.

[Embodiment C1] In Intermediate C, a compound wherein $R^2$ is a C1-C6 alkyl group.
[Embodiment C2] In Intermediate C, a compound wherein $R^2$ is an ethyl group.
[Embodiment C3] In Intermediate C, a compound wherein $R^2$ is $NR^6R^7$, and $R^6$ and $R^7$ are the same as or different from each other and are a C1-C6 alkyl group or a hydrogen atom.
[Embodiment C4] In Intermediate C, a compound wherein $R^2$ is $NR^6R^7$, and $R^6$ and $R^7$ are combined with the nitrogen atom to which they are attached to form an aziridinyl group, an azetidinyl group, a pyrrolidinyl group, or a piperidinyl group.
[Embodiment C5] In Intermediate C, a compound wherein W is an oxygen atom.
[Embodiment C6] In Intermediate C, a compound wherein a combination of $G^1$, $G^2$, $G^3$, and $G^4$ is: a combination wherein $G^1$ is $CR^{3a}$, $G^2$ is $CR^{3b}$, $G^3$ is $CR^{3c}$, and $G^4$ is a nitrogen atom; a combination wherein $G^1$ is $CR^{3a}$, $G^2$ is $CR^{3b}$, $G^3$ is a nitrogen atom, and $G^4$ is $CR^{3d}$; a combination wherein $G^1$ is $CR^{3a}$, $G^2$ is a nitrogen atom, $G^3$ is $CR^{3c}$, and $G^4$ is $CR^{3d}$; a combination wherein $G^1$ is a nitrogen atom, $G^2$ is $CR^{3b}$, $G^3$ is $CR^{3c}$, and $G^4$ is $CR^{3d}$; or a combination wherein $G^1$ is $CR^{3a}$, $G^2$ is $CR^{3b}$, $G^3$ is $CR^{3c}$, and $G^4$ is $CR^{3d}$, $R^{3a}$, $R^{3c}$, and $R^{3d}$ are a hydrogen atom, and $R^{3b}$ is a C1-C6 alkyl group optionally substituted with one or more halogen atoms, a C3-C7 cycloalkyl group optionally substituted with one or more substituent(s) selected from the group consisting of a halogen atom and a cyano group, a phenyl group optionally substituted with one or more halogen atoms, a 5- or 6-membered aromatic heterocyclic group optionally substituted with one or more halogen atoms, or a halogen atom.
[Embodiment C7] In Intermediate C, a compound wherein $G^1$ is $CR^{3a}$, $G^2$ is $CR^{3b}$, $G^3$ is $CR^{3c}$, $G^4$ is a nitrogen atom or $CR^{3d}$, $R^{3a}$, $R^{3c}$, and $R^{3d}$ are a hydrogen atom, and $R^{3b}$ is a C1-C6 alkyl group optionally substituted with one or more halogen atoms or a halogen atom.
[Embodiment C8] In Intermediate C, a compound wherein $G^1$ is $CR^{3a}$, $G^2$ is $CR^{3b}$, $G^3$ is $CR^{3c}$, $G^4$ is $CR^{3d}$, $R^{3a}$, $R^{3c}$, and $R^{3d}$ are a hydrogen atom, and $R^{3b}$ is a C1-C6 alkyl group optionally substituted with one or more halogen atoms or a halogen atom.
[Embodiment C9] In Intermediate C, a compound wherein a combination of $G^1$, $G^2$, $G^3$, and $G^4$ is a combination wherein $G^1$ is $CR^{3a}$, $G^2$ is $CR^{3b}$, $G^3$ is $CR^{3c}$, and $G^4$ is a nitrogen atom; a combination wherein $G^1$ is $CR^{3a}$, $G^2$ is $CR^{3b}$, $G^3$ is a nitrogen atom, and $G^4$ is $CR^{3d}$; a combination wherein $G^1$ is $CR^{3a}$, $G^2$ is a nitrogen atom, $G^3$ is $CR^{3c}$, and $G^4$ is $CR^{3d}$; a combination wherein $G^1$ is a nitrogen atom, $G^2$ is $CR^{3b}$, $G^3$ is $CR^{3c}$, and $G^4$ is $CR^{3d}$; or a combination wherein $G^1$ is $CR^{3a}$, $G^2$ is $CR^{3b}$, $G^3$ is $CR^{3c}$, and $G^4$ is $CR^{3d}$, $R^{3a}$, $R^{3b}$, and $R^{3d}$ are a hydrogen atom, and $R^{3c}$ is a halogen atom.
[Embodiment C10] In Intermediate C, a compound wherein $G^1$ is $CR^{3a}$, $G^2$ is $CR^{3b}$, $G^3$ is $CR^{3c}$, $G^4$ is a nitrogen atom or $CR^{3d}$, $R^{3a}$, $R^{3b}$, and $R^{3d}$ are a hydrogen atom, and $R^{3c}$ is a halogen atom.
[Embodiment C11] In Intermediate C, a compound wherein $G^1$ is $CR^{3a}$, $G^2$ is $CR^{3b}$, $G^3$ is $CR^{3c}$, $G^4$ is $CR^{3d}$, $R^{3a}$, $R^{3b}$, and $R^{3d}$ are a hydrogen atom, and $R^{3c}$ is a halogen atom.
[Embodiment C12] In Intermediate C, a compound wherein n is 0.
[Embodiment C13] In Embodiment C1, a compound wherein n is 0.
[Embodiment C14] In Embodiment C2, a compound wherein n is 0.
[Embodiment C15] In Embodiment C3, a compound wherein n is 0.
[Embodiment C16] In Embodiment C4, a compound wherein n is 0.
[Embodiment C17] In Embodiment C5, a compound wherein n is 0.
[Embodiment C18] In Embodiment C6, a compound wherein n is 0.
[Embodiment C19] In Embodiment C7, a compound wherein n is 0.
[Embodiment C20] In Embodiment C8, a compound wherein n is 0.
[Embodiment C21] In Embodiment C9, a compound wherein n is 0.
[Embodiment C22] In Embodiment C10, a compound wherein n is 0.
[Embodiment C23] In Embodiment C11, a compound wherein n is 0.
[Embodiment C24] In Embodiment C5, a compound wherein $R^2$ is a C1-C6 alkyl group.
[Embodiment C25] In Embodiment C6, a compound wherein $R^2$ is a C1-C6 alkyl group.
[Embodiment C26] In Embodiment C7, a compound wherein $R^2$ is a C1-C6 alkyl group.
[Embodiment C27] In Embodiment C8, a compound wherein $R^2$ is a C1-C6 alkyl group.
[Embodiment C28] In Embodiment C9, a compound wherein $R^2$ is a C1-C6 alkyl group.
[Embodiment C29] In Embodiment C10, a compound wherein $R^2$ is a C1-C6 alkyl group.
[Embodiment C30] In Embodiment C11, a compound wherein $R^2$ is a C1-C6 alkyl group.
[Embodiment C31] In Embodiment C5, a compound wherein $R^2$ is an ethyl group.
[Embodiment C32] In Embodiment C6, a compound wherein $R^2$ is an ethyl group.
[Embodiment C33] In Embodiment C7, a compound wherein $R^2$ is an ethyl group.
[Embodiment C34] In Embodiment C8, a compound wherein $R^2$ is an ethyl group.
[Embodiment C35] In Embodiment C9, a compound wherein $R^2$ is an ethyl group.
[Embodiment C36] In Embodiment C10, a compound wherein $R^2$ is an ethyl group.
[Embodiment C37] In Embodiment C11, a compound wherein $R^2$ is an ethyl group.

[Embodiment C38] In Embodiment C5, a compound wherein $R^2$ is $NR^6R^7$, and $R^6$ and $R^7$ are the same as or different from each other and are a C1-C6 alkyl group or a hydrogen atom.
[Embodiment C39] In Embodiment C6, a compound wherein $R^2$ is $NR^6R^7$, and $R^6$ and $R^7$ are the same as or different from each other and are a C1-C6 alkyl group or a hydrogen atom.
[Embodiment C40] In Embodiment C7, a compound wherein $R^2$ is $NR^6R^7$, and $R^6$ and $R^7$ are the same as or different from each other and are a C1-C6 alkyl group or a hydrogen atom.
[Embodiment C41] In Embodiment C8, a compound wherein $R^2$ is $NR^6R^7$, and $R^6$ and $R^7$ are the same as or different from each other and are a C1-C6 alkyl group or a hydrogen atom.
[Embodiment C42] In Embodiment C9, a compound wherein $R^2$ is $NR^6R^7$, and $R^6$ and $R^7$ are the same as or different from each other and are a C1-C6 alkyl group or a hydrogen atom.
[Embodiment C43] In Embodiment C10, a compound wherein $R^2$ is $NR^6R^7$, and $R^6$ and $R^7$ are the same as or different from each other and are a C1-C6 alkyl group or a hydrogen atom.
[Embodiment C44] In Embodiment C11, a compound wherein $R^2$ is $NR^6R^7$, and $R^6$ and $R^7$ are the same as or different from each other and are a C1-C6 alkyl group or a hydrogen atom.
[Embodiment C45] In Embodiment C5, a compound wherein $R^2$ is $NR^6R^7$, and $R^6$ and $R^7$ are combined with the nitrogen atom to which they are attached to form an aziridinyl group, an azetidinyl group, a pyrrolidinyl group, or a piperidinyl group.
[Embodiment C46] In Embodiment C6, a compound wherein $R^2$ is $NR^6R^7$, and $R^6$ and $R^7$ are combined with the nitrogen atom to which they are attached to form an aziridinyl group, an azetidinyl group, a pyrrolidinyl group, or a piperidinyl group.
[Embodiment C47] In Embodiment C7, a compound wherein $R^2$ is $NR^6R^7$, and $R^6$ and $R^7$ are combined with the nitrogen atom to which they are attached to form an aziridinyl group, an azetidinyl group, a pyrrolidinyl group, or a piperidinyl group.
[Embodiment C48] In Embodiment C8, a compound wherein $R^2$ is $NR^6R^7$, and $R^6$ and $R^7$ are combined with the nitrogen atom to which they are attached to form an aziridinyl group, an azetidinyl group, a pyrrolidinyl group, or a piperidinyl group.
[Embodiment C49] In Embodiment C9, a compound wherein $R^2$ is $NR^6R^7$, and $R^6$ and $R^7$ are combined with the nitrogen atom to which they are attached to form an aziridinyl group, an azetidinyl group, a pyrrolidinyl group, or a piperidinyl group.
[Embodiment C50] In Embodiment C10, a compound wherein $R^2$ is $NR^6R^7$, and $R^6$ and $R^7$ are combined with the nitrogen atom to which they are attached to form an aziridinyl group, an azetidinyl group, a pyrrolidinyl group, or a piperidinyl group.
[Embodiment C51] In Embodiment C11, a compound wherein $R^2$ is $NR^6R^7$, and $R^6$ and $R^7$ are combined with the nitrogen atom to which they are attached to form an aziridinyl group, an azetidinyl group, a pyrrolidinyl group, or a piperidinyl group.
[Embodiment C52] In Embodiment C6, a compound wherein W is an oxygen atom.
[Embodiment C53] In Embodiment C7, a compound wherein W is an oxygen atom.
[Embodiment C54] In Embodiment C8, a compound wherein W is an oxygen atom.
[Embodiment C55] In Embodiment C9, a compound wherein W is an oxygen atom.
[Embodiment C56] In Embodiment C10, a compound wherein W is an oxygen atom.
[Embodiment C57] In Embodiment C11, a compound wherein W is an oxygen atom.
[Embodiment C58] In Embodiment C6, a compound wherein $R^2$ is an ethyl group, and W is an oxygen atom.
[Embodiment C59] In Embodiment C7, a compound wherein $R^2$ is an ethyl group, and W is an oxygen atom.
[Embodiment C60] In Embodiment C8, a compound wherein $R^2$ is an ethyl group, and W is an oxygen atom.
[Embodiment C61] In Embodiment C9, a compound wherein $R^2$ is an ethyl group, and W is an oxygen atom.
[Embodiment C62] In Embodiment C10, a compound wherein $R^2$ is an ethyl group, and W is an oxygen atom.
[Embodiment C63] In Embodiment C11, a compound wherein $R^2$ is an ethyl group, and W is an oxygen atom.
[Embodiment C64] In Embodiment C12, a compound wherein $R^2$ is an ethyl group, and W is an oxygen atom.
[Embodiment C65] In Embodiment C6, a compound wherein $R^2$ is $NR^6R^7$, $R^6$ and $R^7$ are the same as or different from each other and are a C1-C6 alkyl group or a hydrogen atom, and W is an oxygen atom.
[Embodiment C66] In Embodiment C7, a compound wherein $R^2$ is $NR^6R^7$, $R^6$ and $R^7$ are the same as or different from each other and are a C1-C6 alkyl group or a hydrogen atom, and W is an oxygen atom.
[Embodiment C67] In Embodiment C8, a compound wherein $R^2$ is $NR^6R^7$, $R^6$ and $R^7$ are the same as or different from each other and are a C1-C6 alkyl group or a hydrogen atom, and W is an oxygen atom.
[Embodiment C68] In Embodiment C9, a compound wherein $R^2$ is $NR^6R^7$, $R^6$ and $R^7$ are the same as or different from each other and are a C1-C6 alkyl group or a hydrogen atom, and W is an oxygen atom.
[Embodiment C69] In Embodiment C10, a compound wherein $R^2$ is $NR^6R^7$, $R^6$ and $R^7$ are the same as or different from each other and are a C1-C6 alkyl group or a hydrogen atom, and W is an oxygen atom.

[Embodiment C70] In Embodiment C11, a compound wherein $R^2$ is $NR^6R^7$, $R^6$ and $R^7$ are the same as or different from each other and are a C1-C6 alkyl group or a hydrogen atom, and W is an oxygen atom.
[Embodiment C71] In Embodiment C12, a compound wherein $R^2$ is $NR^6R^7$, $R^6$ and $R^7$ are the same as or different from each other and are a C1-C6 alkyl group or a hydrogen atom, and W is an oxygen atom.
[Embodiment C72] In Embodiment C6, a compound wherein $R^2$ is $NR^6R^7$, $R^6$ and $R^7$ are combined with the nitrogen atom to which they are attached to form an aziridinyl group, an azetidinyl group, a pyrrolidinyl group, or a piperidinyl group, and W is an oxygen atom.
[Embodiment C73] In Embodiment C7, a compound wherein $R^2$ is $NR^6R^7$, $R^6$ and $R^7$ are combined with the nitrogen atom to which they are attached to form an aziridinyl group, an azetidinyl group, a pyrrolidinyl group, or a piperidinyl group, and W is an oxygen atom.
[Embodiment C74] In Embodiment C8, a compound wherein $R^2$ is $NR^6R^7$, $R^6$ and $R^7$ are combined with the nitrogen atom to which they are attached to form an aziridinyl group, an azetidinyl group, a pyrrolidinyl group, or a piperidinyl group, and W is an oxygen atom.
[Embodiment C75] In Embodiment C9, a compound wherein $R^2$ is $NR^6R^7$, $R^6$ and $R^7$ are combined with the nitrogen atom to which they are attached to form an aziridinyl group, an azetidinyl group, a pyrrolidinyl group, or a piperidinyl group, and W is an oxygen atom.
[Embodiment C76] In Embodiment C10, a compound wherein $R^2$ is $NR^6R^7$, $R^6$ and $R^7$ are combined with the nitrogen atom to which they are attached to form an aziridinyl group, an azetidinyl group, a pyrrolidinyl group, or a piperidinyl group, and W is an oxygen atom.
[Embodiment C77] In Embodiment C11, a compound wherein $R^2$ is $NR^6R^7$, $R^6$ and $R^7$ are combined with the nitrogen atom to which they are attached to form an aziridinyl group, an azetidinyl group, a pyrrolidinyl group, or a piperidinyl group, and W is an oxygen atom.
[Embodiment C78] In Embodiment C12, a compound wherein $R^2$ is $NR^6R^7$, $R^6$ and $R^7$ are combined with the nitrogen atom to which they are attached to form an aziridinyl group, an azetidinyl group, a pyrrolidinyl group, or a piperidinyl group, and W is an oxygen atom.
[Embodiment C79] In Embodiment C5, a compound wherein $R^2$ is an ethyl group, and n is 0.
[Embodiment C80] In Embodiment C6, a compound wherein $R^2$ is an ethyl group, and n is 0.
[Embodiment C81] In Embodiment C7, a compound wherein $R^2$ is an ethyl group, and n is 0.
[Embodiment C82] In Embodiment C8, a compound wherein $R^2$ is an ethyl group, and n is 0.
[Embodiment C83] In Embodiment C9, a compound wherein $R^2$ is an ethyl group, and n is 0.
[Embodiment C84] In Embodiment C10, a compound wherein $R^2$ is an ethyl group, and n is 0.
[Embodiment C85] In Embodiment C11, a compound wherein $R^2$ is an ethyl group, and n is 0.
[Embodiment C86] In Embodiment C1, a compound wherein W is an oxygen atom, and n is 0.
[Embodiment C87] In Embodiment C2, a compound wherein W is an oxygen atom, and n is 0.
[Embodiment C88] In Embodiment C3, a compound wherein W is an oxygen atom, and n is 0.
[Embodiment C89] In Embodiment C4, a compound wherein W is an oxygen atom, and n is 0.
[Embodiment C90] In Embodiment C6, a compound wherein W is an oxygen atom, and n is 0.
[Embodiment C91] In Embodiment C7, a compound wherein W is an oxygen atom, and n is 0.
[Embodiment C92] In Embodiment C8, a compound wherein W is an oxygen atom, and n is 0.
[Embodiment C93] In Embodiment C9, a compound wherein W is an oxygen atom, and n is 0.
[Embodiment C94] In Embodiment C10, a compound wherein W is an oxygen atom, and n is 0.
[Embodiment C95] In Embodiment C11, a compound wherein W is an oxygen atom, and n is 0.
[Embodiment C96] In any one of Embodiments C1 to C95 or Intermediate C, a compound wherein Q is $Q^1$, $Z^1$ is $NR^{3j}$, $X^1$ is $CR^{3e}$, $X^2$ is $CR^{3f}$, $X^3$ is a nitrogen atom, $R^{3e}$ is a hydrogen atom, $R^{3f}$ is a C1-C6 alkyl group optionally substituted with one or more halogen atoms, a hydrogen atom, or a halogen atom, and $R^{3j}$ is a methyl group optionally substituted with one or more halogen atoms, a C2-C4 alkyl group optionally substituted with one or more fluorine atoms, a C5-C6 alkyl group optionally substituted with one or more halogen atoms, or a C3-C7 cycloalkyl group optionally substituted with one or more halogen atoms.
[Embodiment C97] In any one of Embodiments C1 to C95 or Intermediate C, a compound wherein Q is $Q^1$, $Z^1$ is an oxygen atom, $X^1$ is $CR^{3e}$, $X^2$ is $CR^{3f}$, $X^3$ is a nitrogen atom, $R^{3e}$ is a hydrogen atom, and $R^{3f}$ is a C1-C6 alkyl group optionally substituted with one or more halogen atoms, a hydrogen atom, or a halogen atom.
[Embodiment C98] In any one of Embodiments C1 to C95 or Intermediate C, a compound wherein Q is $Q^1$, $Z^1$ is an oxygen atom, $X^1$ is $CR^{3e}$, $X^2$ is $CR^{3f}$, $X^3$ is $CR^{3g}$, $R^{3e}$ and $R^{3f}$ are a hydrogen atom, and $R^{3g}$ is a C1-C6 alkyl group optionally substituted with one or more halogen atoms, a hydrogen atom, or a halogen atom.
[Embodiment C99] In any one of Embodiments C1 to C95 or Intermediate C, a compound wherein Q is $Q^2$, $Z^2$ is $NR^{3k}$, $X^1$ is $CR^{3e}$, $X^2$ is $CR^{3f}$, $X^4$ is a nitrogen atom, $R^{3e}$ is a hydrogen atom, $R^{3f}$ is a C1-C6 alkyl group optionally substituted with one or more halogen atoms, a hydrogen atom, or a halogen atom, and $R^{3k}$ is a C1-C6 alkyl group optionally substituted with one or more halogen atoms, or a C3-C7 cycloalkyl group optionally substituted with one or more

halogen atoms.

[Embodiment C100] In any one of Embodiments C1 to C95 or Intermediate C, a compound wherein Q is $Q^2$, and Z is $NR^{3j}$, $X^1$ is $CR^{3e}$, $X^2$ is a nitrogen atom, $X^4$ is $CR^{3h}$, $R^{3e}$ and $R^{3h}$ are a hydrogen atom, and $R^{3j}$ is a C1-C6 alkyl group optionally substituted with one or more halogen atoms or a C3-C7 cycloalkyl group optionally substituted with one or more halogen atoms.

[Embodiment C101] In any one of Embodiments C1 to C95 or Intermediate C, a compound wherein Q is $Q^2$, $Z^2$ is an oxygen atom, $X^1$ is $CR^{3e}$, $X^2$ is a nitrogen atom, $X^4$ is $CR^{3h}$, $R^{3e}$ and $R^{3h}$ are the same as or different from each other and are a C1-C6 alkyl group optionally substituted with one or more halogen atoms, or a hydrogen atom.

[Embodiment C102] In any one of Embodiments C1 to C95 or Intermediate C, a compound wherein Q is $Q^2$, $Z^2$ is an oxygen atom, $X^1$ is $CR^{3e}$, $X^2$ is $CR^{3f}$, $X^4$ is a nitrogen atom, $R^{3e}$ is a hydrogen atom, and $R^{3f}$ is a C1-C6 alkyl group optionally substituted with one or more halogen atoms, a hydrogen atom, or a halogen atom.

[Embodiment C103] In any one of Embodiments C1 to C95 or Intermediate C, a compound wherein Q is $Q^1$, $Z^1$ is a sulfur atom, $X^1$ is $CR^{3e}$, $X^2$ is a nitrogen atom, $X^3$ is $CR^{3g}$, $R^{3e}$ is a hydrogen atom, and $R^{3g}$ is a C1-C6 alkyl group optionally substituted with one or more halogen atoms, a hydrogen atom, or a halogen atom.

[Embodiment C104] In any one of Embodiments C1 to C95 or Intermediate C, a compound wherein Q is $Q^1$, $Z^1$ is a sulfur atom, $X^1$ is a nitrogen atom, $X^2$ is $CR^{3f}$, $X^3$ is $CR^{3g}$, $R^{3e}$ is a hydrogen atom, and $R^{3g}$ is a C1-C6 alkyl group optionally substituted with one or more halogen atoms, a hydrogen atom, or a halogen atom.

Then, a method for producing the present compound will be described.

Production method 1

A compound represented by a formula (I-b) (hereinafter, referred to as a compound (I-b)) or a compound represented by a formula (I-c) (hereinafter, referred to as a compound (I-c)) can be produced by reacting a compound represented by a formula (I-a) (hereinafter, referred to as a compound (I-a)) with an oxidizing agent.

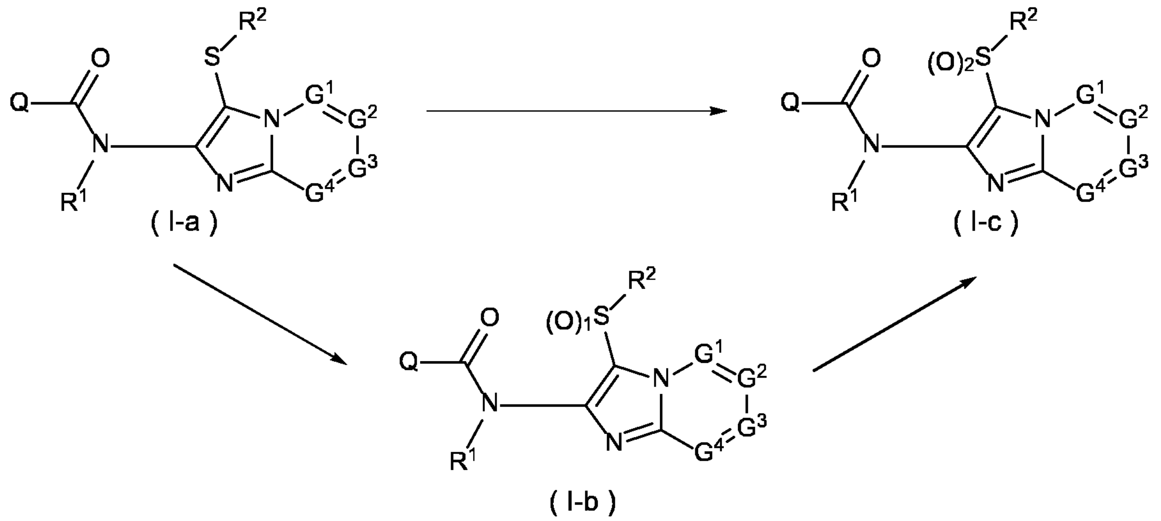

[In the formula, symbols represent the same meaning as described above.]

First, a method for producing the compound (I-b) from the compound (I-a) will be described.

The reaction is typically performed in a solvent. Examples of the solvent used in the reaction include: a halogenated hydrocarbon such as dichloromethane and chloroform (hereinafter, referred to as halogenated hydrocarbons); a nitrile such as acetonitrile (hereinafter, referred to as nitriles); an alcohol such as methanol and ethanol (hereinafter, referred to as alcohols); acetic acid; water; and mixtures of two or more thereof.

Examples of the oxidizing agent used in the reaction include sodium periodate, m-chloroperbenzoic acid (hereinafter, referred to as mCPBA), and hydrogen peroxide.

When hydrogen peroxide is used as the oxidizing agent, a base or a catalyst may be added as necessary.

Examples of the base used in the reaction include sodium carbonate. When a base is used in the reaction, the base is typically used in a ratio of 0.01 to 1 mol relative to 1 mol of the compound (I-a).

Examples of the catalyst used in the reaction include tungstic acid and sodium tungstate. When a catalyst is used in the reaction, the catalyst is typically used in a ratio of 0.01 to 0.5 mol relative to 1 mol of the compound (I-a).

For the reaction, the oxidizing agent is typically used in a ratio of 1 to 1.2 mol relative to 1 mol of the compound (I-a).

The reaction temperature is typically in the range of -20 to 80°C. The reaction time is typically in the range of 0.1 to 12 hours.

After completion of the reaction, water is added to the reaction mixture, the mixture is extracted with an organic solvent, and the organic layer is washed with an aqueous solution of a reducing agent (for example, sodium sulfite and sodium thiosulfate) and an aqueous solution of a base (for example, sodium hydrogen carbonate) as necessary. The organic layer is dried and concentrated to allow to provide the compound (I-b).

Then, a method for producing the compound (I-c) from the compound (I-b) will be described.

The reaction is typically performed in a solvent. Examples of the solvent used in the reaction include halogenated hydrocarbons, nitriles, alcohols, acetic acid, water, and mixtures of two or more thereof.

Examples of the oxidizing agent used in the reaction include mCPBA and hydrogen peroxide.

When hydrogen peroxide is used as the oxidizing agent, a base or a catalyst may be added as necessary.

Examples of the base used in the reaction include sodium carbonate. When a base is used in the reaction, the base is typically used in a ratio of 0.01 to 1 mol relative to 1 mol of the compound (I-b).

Examples of the catalyst used in the reaction include sodium tungstate. When a catalyst is used in the reaction, the catalyst is typically used in a ratio of 0.01 to 0.5 mol relative to 1 mol of the compound (I-b).

In the reaction, the oxidizing agent is typically used in a ratio of 1 to 2 mol relative to 1 mol of the compound (I-b).

The reaction temperature is typically in the range of -20 to 120°C. The reaction time is typically in the range of 0.1 to 12 hours.

After completion of the reaction, water is added to the reaction mixture, the mixture is extracted with an organic solvent, and the organic layer is washed with an aqueous solution of a reducing agent (for example, sodium sulfite and sodium thiosulfate) and an aqueous solution of a base (for example, sodium hydrogen carbonate) as necessary. This organic layer is dried and concentrated to allow to provide the compound (I-c).

The compound (I-c) can be produced by reacting the compound (I-a) with an oxidizing agent as a single-step reaction (one-pot).

The reaction can be performed in accordance with the method for producing the compound (I-c) from the compound (I-b), using the oxidizing agent in a ratio of typically 2 to 5 mol relative to 1 mol of the compound (I-a).

Production method 2

A compound represented by a formula (I-1-A) (hereinafter, referred to as a compound (I-1-A)) can be produced by reacting a compound represented by a formula (M1-1-A) (hereinafter, referred to as a compound (M1-1-A)) with a compound represented by a formula (M2-1) (hereinafter, referred to as a compound (M2-1)), in the presence of a condensing agent.

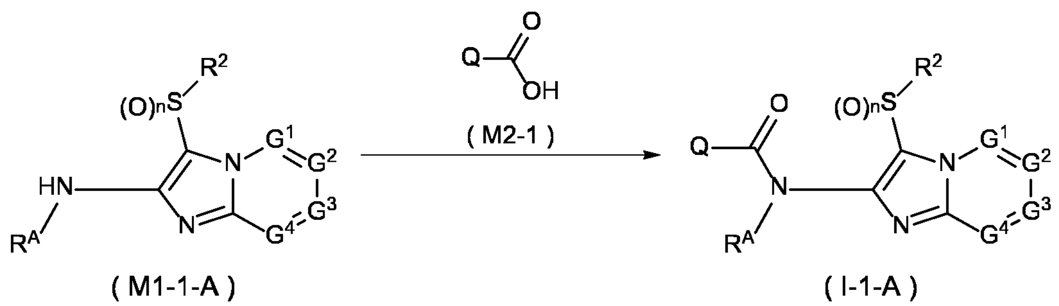
( M1-1-A ) ( M2-1 ) ( I-1-A )

[In the formula, $R^A$ represents a C1-C6 chain hydrocarbon group optionally substituted with one or more substituent(s) selected from Group D, a C3-C7 cycloalkyl group optionally substituted with one or more substituent(s) selected from Group E, a phenyl group optionally substituted with one or more substituent(s) selected from Group F, or a 5- or 6-membered aromatic heterocyclic group optionally substituted with one or more substituent(s) selected from Group F, other symbols represents the same meaning as described above.]

The reaction is typically performed in a solvent. Examples of the solvent used in the reaction include: an ether such as tetrahydrofuran (hereinafter, referred to as THF) and methyl tert-butyl ether (hereinafter, referred to as MTBE) (hereinafter, referred to as ethers); halogenated hydrocarbons; an aromatic hydrocarbon such as toluene and xylene (hereinafter, referred to as aromatic hydrocarbons); an ester such as ethyl acetate and butyl acetate (hereinafter, referred to as esters); nitriles; an aprotic polar solvent such as N-methylpyrrolidone (hereinafter, referred to as NMP), N,N-dimethylformamide (hereinafter, referred to as DMF), and dimethyl sulfoxide (hereinafter, referred to as DMSO) (hereinafter, referred to as aprotic polar solvent); a nitrogen-containing aromatic compound such as pyridine, picoline, lutidine,

and quinoline (hereinafter, referred to as nitrogen-containing aromatic compounds), and mixtures of two or more thereof.

Examples of the condensing agent used in the reaction include carbodiimides such as 1-ethyl-3-[3-(dimethylamino)propyl]carbodiimide hydrochloride and 1,3-dicyclohexylcarbodiimide.

For the reaction, a catalyst can be used as necessary. Examples of the catalyst used in the reaction include 1-hydroxybenzotriazole. When a catalyst is used in the reaction, the catalyst is typically used in a ratio of 0.01 to 0.5 mol relative to 1 mol of the compound (M1-1-A).

For the reaction, a base can be used as necessary. Examples of the base used in the reaction include an organic base (hereinafter, referred to as organic bases) such as triethylamine, N,N-diisopropylethylamine, pyridine, and 4-(dimethylamino)pyridine. When a base is used in the reaction, the base is typically used in a ratio of 1 to 2 mol relative to 1 mol of the compound (M1-1-A).

For the reaction, the compound (M2-1) is typically used in a ratio of 1 to 2 mol, and the condensing agent is typically used in a ratio of 1 to 2 mol, relative to 1 mol of the compound (M1-1-A).

The reaction temperature is typically in the range of 0 to 200°C. The reaction time is typically in the range of 0.1 to 12 hours.

After completion of the reaction, water is added to the reaction mixture, the mixture is extracted with an organic solvent, and the organic layer is subjected to post-treatment operations such as drying and concentration, thereby allowing to provide the compound (I-1-A).

The compound (M2-1) is a commercially available compound, or can be produced using a known method.

Production method 3

The compound (I-1-A) can also be produced by reacting the compound (M1-1-A) with a compound represented by a formula (M2-2) (hereinafter, referred to as a compound (M2-2)).

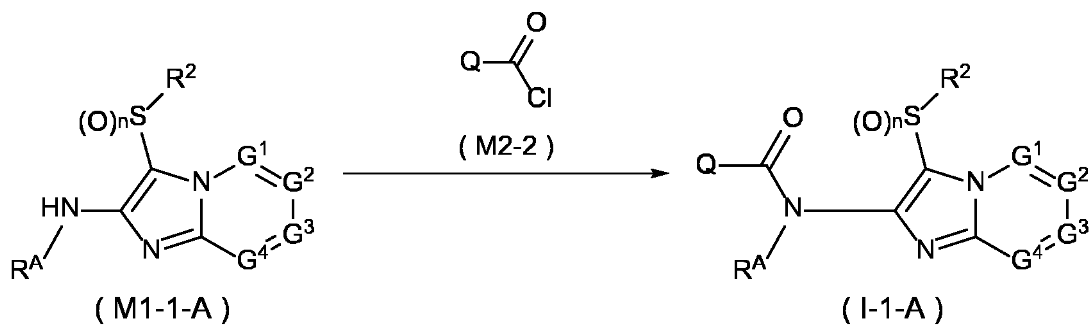

[In the formula, symbols represent the same meaning as described above.]

The reaction is typically performed in a solvent. Examples of the solvent used in the reaction include ethers; an aliphatic hydrocarbon such as hexane, heptane, or octane (hereinafter, referred to as aliphatic hydrocarbons); aromatic hydrocarbons; halogenated hydrocarbons; esters; nitriles; aprotic polar solvents; and mixtures of two or more thereof.

For the reaction, a base can be used as necessary. Examples of the base used in the reaction include an alkali metal carbonate such as sodium carbonate and potassium carbonate (hereinafter, referred to as alkali metal carbonates); an alkali metal hydroxide such as sodium hydroxide and potassium hydroxide (hereinafter, referred to as alkali metal hydroxides); and organic bases. When a base is used in the reaction, the base is typically used in a ratio of 1 to 2 mol relative to 1 mol of the compound (M1-1-A).

For the reaction, the compound (M2-2) is typically used in a ratio of 0.8 to 1.2 mol relative to 1 mol of the compound (M1-1-A).

The reaction temperature is typically in the range of -20 to 200°C. The reaction time is typically in the range of 0.1 to 24 hours.

After completion of the reaction, water is added to the reaction mixture, the mixture is extracted with an organic solvent, and the organic layer is subjected to post-treatment operations such as drying and concentration, thereby allowing to provide the compound (I-1-A).

The compound (M2-2) is a commercially available compound, or can be produced using a known method.

Production method 4

A compound represented by a formula (I-2-B) (hereinafter, referred to as a compound (I-2-B)) can be produced by reacting a compound represented by a formula (II) (hereinafter, referred to as a compound (II)) with a compound represented by a formula (R-1) (hereinafter, referred to as a compound (R-1)), in the presence of a base.

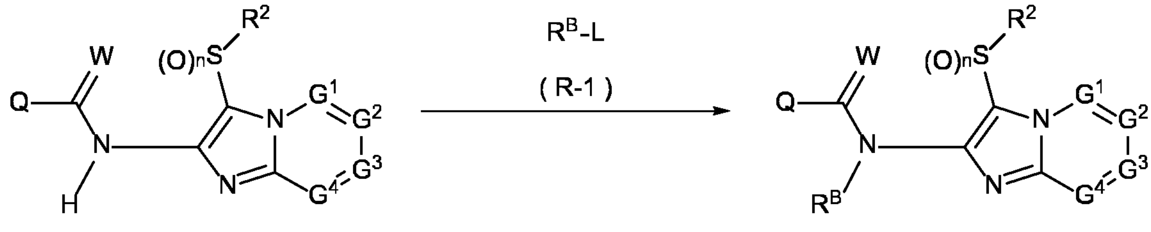


( II ) ( I-2-B )

[In the formula, $R^B$ represents a C1-C6 chain hydrocarbon group optionally substituted with one or more substituent(s) selected from Group D, a C3-C7 cycloalkyl group optionally substituted with one or more substituent(s) selected from Group E, $C(O)R^4$, $C(O)OR^4$, or $C(O)NR^4R^5$, L represents a leaving group such as a chlorine atom or a bromine atom, and other symbols represent the same meaning as described above.]

The reaction is typically performed in a solvent. Examples of the solvent used in the reaction include ethers, aromatic hydrocarbons, nitriles, aprotic polar solvents, and mixtures of two or more thereof.

Examples of the base used in the reaction include an alkali metal hydride such as sodium hydride (hereinafter, referred to as alkali metal hydrides), alkali metal carbonates, and organic bases.

For the reaction, the compound (R-1) is typically used in a ratio of 1 to 5 mol, and the base is typically used in a ratio of 1 to 2 mol, relative to 1 mol of the compound (II).

The reaction temperature is typically in the range of 0 to 100°C. The reaction time is typically in the range of 0.1 to 24 hours.

After completion of the reaction, water is added to the reaction mixture, the mixture is extracted with an organic solvent, and the organic layer is subjected to post-treatment operations such as drying and concentration, thereby allowing to provide the compound (I-2-B).

The compound (R-1) is a commercially available compound, or can be produced using a known method.

Production method 5

A compound represented by a formula (I-1) (hereinafter, referred to as a compound (I-1)) can be produced by reacting a compound represented by a formula (M1-2) (hereinafter, referred to as a compound (M1-2)) with a compound represented by a formula (M2-3) (hereinafter, referred to as a compound (M2-3)).

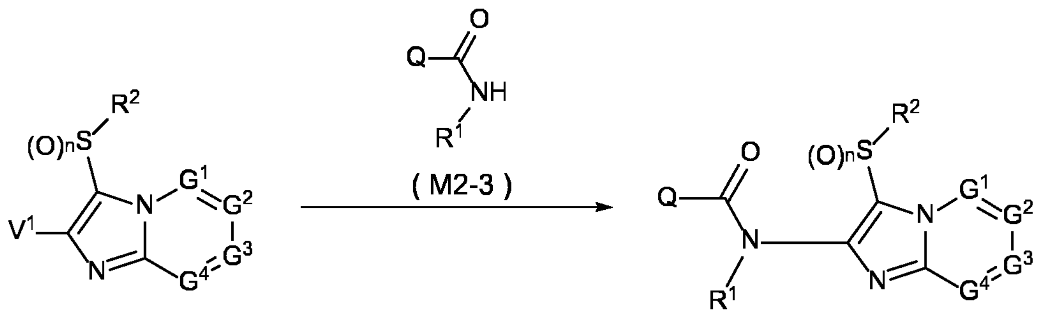


( M1-2 ) ( I-1 )

[In the formula, $V^1$ represents a halogen atom, and other symbols represent the same meaning as described above.]

When $V^1$ is a fluorine atom, the compound (I-1) can be produced by reacting the compound (M1-2) with the compound (M2-3), in the presence of a base.

The reaction is typically performed in a solvent. Examples of the solvent used in the reaction include ethers, aromatic hydrocarbons, nitriles, aprotic polar solvents, water, and mixtures of two or more thereof.

Examples of the base used in the reaction include alkali metal carbonates and alkali metal hydrides.

For the reaction, the compound (M2-3) is typically used in a ratio of 0.8 to 1.2 mol, and the base is typically used in a ratio of 1 to 2 mol, relative to 1 mol of the compound (M1-2).

The reaction temperature is typically in the range of 0 to 200°C. The reaction time is typically in the range of 0.5 to 24 hours.

After completion of the reaction, water is added to the reaction mixture, the mixture is extracted with an organic solvent, and the organic layer is subjected to post-treatment operations such as drying and concentration, thereby allowing

to provide the compound (I-1).

When $V^1$ is a chlorine atom, a bromine atom, or an iodine atom, the compound (I-1) can be produced by reacting the compound (M1-2) with the compound (M2-3), in the presence of a base and a copper catalyst or a palladium catalyst.

The reaction is typically performed in a solvent. Examples of the solvent used in the reaction include ethers, aromatic hydrocarbons, nitriles, aprotic polar solvents, water, and mixtures of two or more thereof.

Examples of the base used in the reaction include alkali metal carbonates; phosphates such as trisodium phosphate and tripotassium phosphate; alkali metal hydrides; organic bases; and cyclic amines such as 1,4-diazabicyclo [2,2,2]octane and diazabicycloundecene.

Examples of the copper catalyst used in the reaction include copper (I) iodide, copper (I) bromide, copper (I) chloride, and copper (I) oxide. When a copper catalyst is used in the reaction, the copper catalyst is typically used in a ratio of 0.01 to 0.5 mol relative to 1 mol of the compound (M1-2).

Examples of the palladium catalyst used in the reaction include palladium (II) acetate and tris(dibenzylideneacetone)dipalladium (0). When a palladium catalyst is used in the reaction, the palladium catalyst is typically used in a ratio of 0.01 to 0.2 mol relative to 1 mol of the compound (M1-2).

For the reaction, a ligand can be used as necessary. Examples of the ligand used in the reaction include acetylacetone, salen, phenanthroline, triphenylphosphine, and 4,5'-bis(diphenylphosphino)-9,9'-dimethylxanthene. When a ligand is used in the reaction, the ligand is typically used in a ratio of 0.01 to 0.5 mol relative to 1 mol of the compound (M1-2).

For the reaction, the compound (M2-3) is typically used in a ratio of 0.8 to 1.2 mol, and the base is typically used in a ratio of 1 to 2 mol, relative to 1 mol of the compound (M1-2).

The reaction temperature is typically in the range of 0 to 200°C. The reaction time is typically in the range of 0.5 to 24 hours.

After completion of the reaction, water is added to the reaction mixture, the mixture is extracted with an organic solvent, and the organic layer is subjected to post-treatment operations such as drying and concentration, thereby allowing to provide the compound (I-1).

In the compound (M2-3), the compound in which $R^1$ is $R^A$ or $R^B$ is a commercially available compound, or can be produced using a known method.

Production method 6

The compound (I-a) can be produced according to the following scheme.

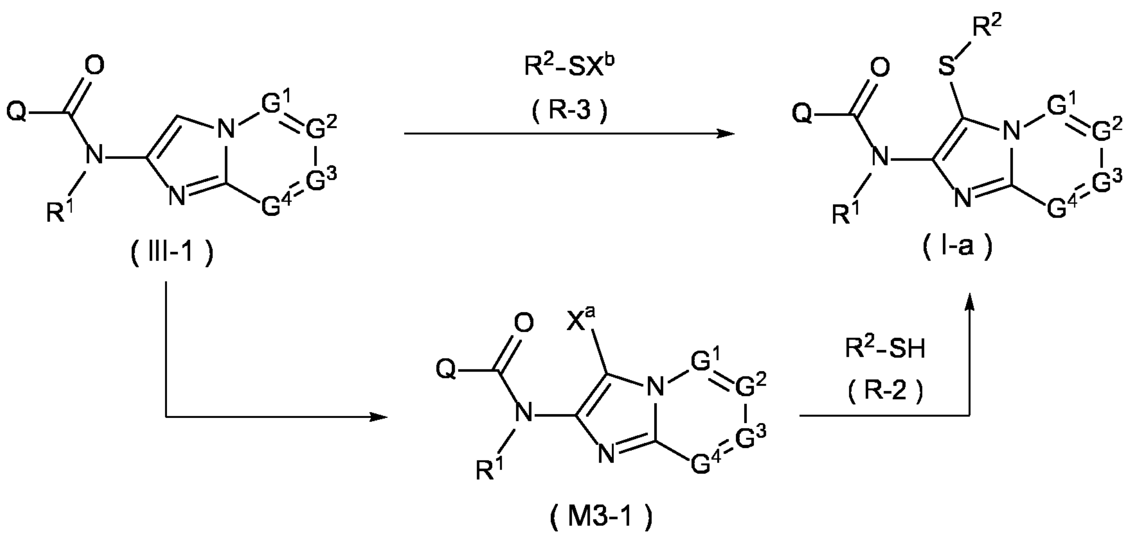


[In the formula, $X^a$ represents a chlorine atom, a bromine atom, or an iodine atom, $X^b$ represents $SR^2$ or a hydrogen atom, and other symbols represent the same meaning as described above.]

First, there will be described a step (hereinafter, referred to as a step 6-A) of producing a compound represented by a formula (M3-1) (hereinafter, referred to as a compound (M3-1)) from a compound represented by a formula (III-1) (hereinafter, referred to as a compound (III-1)).

The compound (M3-1) can be produced by reacting the compound (III-1) with a halogenating agent.

The reaction is typically performed in a solvent. Examples of the solvent used in the reaction include alcohols, nitriles, ethers, aromatic hydrocarbons, aprotic polar solvents, halogenated hydrocarbons, water, and mixtures of two or

more thereof.

Examples of the halogenating agent include chlorine, bromine, iodine, N-chlorosuccinimide, N-bromosuccinimide, and N-iodosuccinimide.

For the reaction, the halogenating agent is typically used in a ratio of 1 to 20 mol relative to 1 mol of the compound (III-1).

The reaction temperature is typically in the range of -20°C to 200°C. The reaction time is typically in the range of 0.1 to 72 hours.

After completion of the reaction, water is added to the reaction mixture, the mixture is extracted with an organic solvent, and the organic layer is subjected to post-treatment operations such as drying and concentration, thereby allowing to provide the compound (M3-1).

Then, a step of producing the compound (I-a) from the compound (M3-1) will be described.

The compound (I-a) can be produced by reacting the compound (M3-1) with a compound represented by a formula (R-2) (hereinafter, referred to as a compound (R-2)), in the presence of a metal catalyst and a base.

The reaction is typically performed in a solvent. Examples of the solvent used in the reaction include alcohols, nitriles, ethers, aromatic hydrocarbons, aprotic polar solvents, water, and mixtures of two or more thereof.

Examples of the metal catalyst used in the reaction include: palladium catalysts such as tetrakis(triphenylphosphine)palladium (0), 1,1'-bis(diphenylphosphino)ferrocene palladium (II) dichloride, tris(dibenzylideneacetone)dipalladium (0), and palladium (II) acetate; nickel catalysts such as bis(cyclooctadiene)nickel (0) and nickel (II) chloride; and copper catalysts such as copper (I) iodide and copper (I) chloride.

Examples of the base used in the reaction include alkali metal hydrides, alkali metal carbonates, and organic bases.

For the reaction, a ligand can be used. Examples of the ligand include

> triphenylphosphine, xantphos, 2,2'-bis(diphenylphosphino)-1,1'-binaphthyl, 1,1'-bis(diphenylphosphino)ferrocene, 2-dicyclohexylphosphino-2',4',6'-riisopropylbiphenyl,
> 2-dicyclohexylphosphino-2',6'-dimethoxybiphenyl,
> 1,2-bis(diphenylphosphino)ethane, 2,2'-bipyridine,
> 2-aminoethanol, 8-hydroxyquinoline, and 1,10-phenanthroline. When a ligand is used in the reaction, the ligand is typically used in a ratio of 0.01 to 1 mol relative to 1 mol of the compound (M3-1).

For the reaction, the compound (R-2) is typically used in a ratio of 1 to 20 mol, the metal catalyst is typically used in a ratio of 0.01 to 0.5 mol, and the base is typically used in a ratio of 0.1 to 5 mol, relative to 1 mol of the compound (M3-1).

The reaction temperature is typically in the range of -20°C to 200°C. The reaction time is typically in the range of 0.1 to 72 hours.

After completion of the reaction, water is added to the reaction mixture, the mixture is extracted with an organic solvent, and the organic layer is subjected to post-treatment operations such as drying and concentration, thereby allowing to provide the compound (I-a).

The compound (R-2) is known or can be produced in accordance with a known method.

Then, a step of producing the compound (I-a) from the compound (III-1) will be described.

The compound (I-a) can also be produced by reacting the compound (III-1) with a compound represented by a formula (R-3) (hereinafter, referred to as a compound (R-3)), in the presence of a halogenating agent.

The reaction is typically performed in a solvent. Examples of the solvent used in the reaction include alcohols, nitriles, ethers, aromatic hydrocarbons, aprotic polar solvents, water, and mixtures of two or more thereof.

Examples of the halogenating agent include bromine, iodine, sodium bromide, potassium bromide, sodium iodide, and potassium iodide.

For the reaction, an oxidizing agent can be used as necessary. Examples of the oxidizing agent used in the reaction include hydrogen peroxide, tert-butyl hydroperoxide, and DMSO. When an oxidizing agent is used in the reaction, the oxidizing agent is typically used in a ratio of 1 to 20 mol relative to 1 mol of the compound (III-1).

For the reaction, the compound (R-3) is typically used in a ratio of 0.5 to 10 mol, and the halogenating agent is typically used in a ratio of 0.05 to 10 mol, relative to 1 mol of the compound (III-1).

The reaction temperature is typically in the range of 0°C to 200°C. The reaction time is typically in the range of 0.1 to 72 hours.

After completion of the reaction, water is added to the reaction mixture, the mixture is extracted with an organic solvent, and the organic layer is subjected to post-treatment operations such as drying and concentration, thereby allowing to provide the compound (I-a).

The compound (R-3) is known or can be produced in accordance with a known method.

Production method 7

A compound represented by a formula (I-S) (hereinafter, referred to as a compound (I-S)) can be produced by reacting the compound (I-1) with a sulfurizing agent.

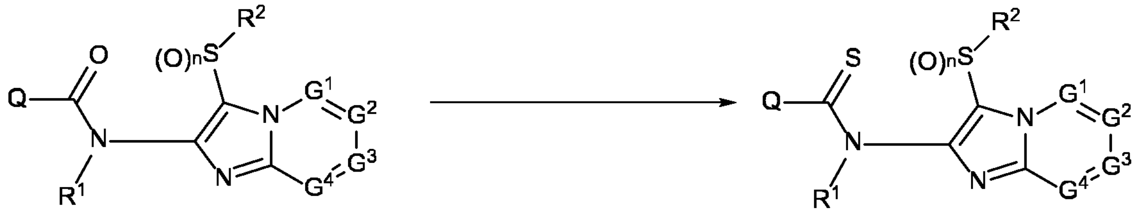

( I-1 ) ( I-S )

[In the formula, symbols represent the same meaning as described above.]

The reaction is performed in a solvent or in the absence of a solvent. Examples of the solvent include ethers, halogenated hydrocarbons, aromatic hydrocarbons, nitriles, nitrogen-containing aromatic compounds, and mixtures of two or more thereof.

Examples of the sulfurizing agent include diphosphorus pentasulfide and Lawesson's reagent (2,4-bis(4-methoxyphenyl)-1,3-dithia-2,4-diphosphetane-2,4-disulfide).

For the reaction, the sulfurizing agent is typically used in a ratio of 1 to 3 mol relative to 1 mol of the compound (I-1).

The reaction temperature is typically in the range of 0°C to 200°C. The reaction time is typically in the range of 1 to 24 hours.

After completion of the reaction, water is added to the reaction mixture, the mixture is extracted with an organic solvent, and the organic layer is subjected to post-treatment operations such as drying and concentration, thereby allowing to provide the compound (I-S).

Production method 8

A compound represented by a formula (II-S) (hereinafter, referred to as a compound (II-S)) can be produced by reacting the compound (II-O) with a sulfurizing agent.

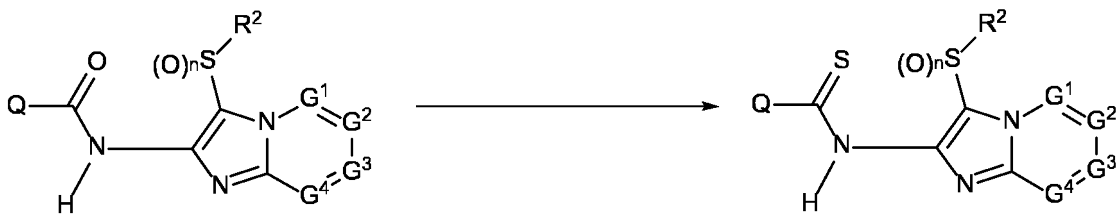

( II-O ) ( II-S )

[In the formula, symbols represent the same meaning as described above.]

The reaction can be performed in accordance with Production method 7, using the compound (II-O) in place of the compound (I-1).

The production of a compound represented by a formula (II-S-B) from the compound (II-S) can be performed in accordance with Production method 4.

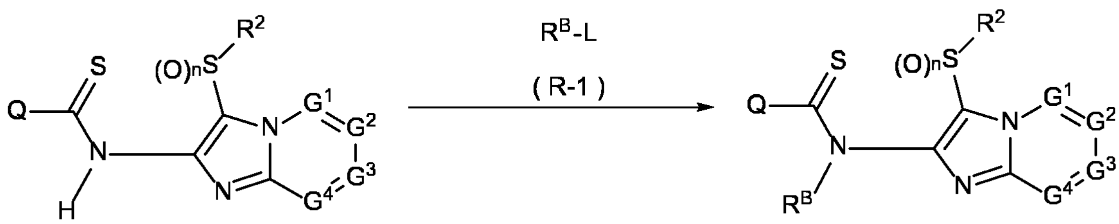


( II-S ) ( II-S-B )

[In the formula, symbols represent the same meaning as described above.]

Production method 9

The N-oxide of the compound represented by the formula (I) can be produced by reacting the compound represented by the formula (I) with an oxidizing agent. The reaction can be performed, for example, in accordance with the method described in Production method 1 or the method described in US 2018/0009778 A, or WO 2016/121970 A.
Hereinafter, a method for producing a production intermediate will be described.

Reference production method 1

A compound represented by a formula (M1-1-D) (hereinafter, referred to as a compound (M1-1-D)) can be produced by reacting a compound represented by a formula (M1-3) (hereinafter, referred to as a compound (M1-3)) with a compound represented by a formula (R-5) (hereinafter, referred to as a compound (R-5)), in the presence of a base.

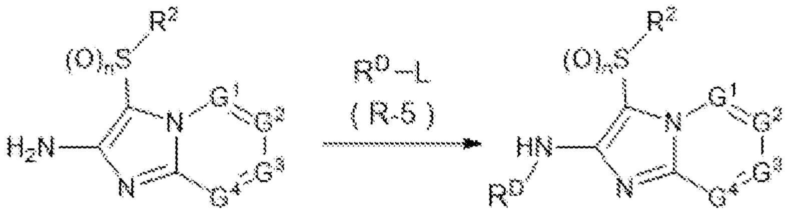


( M1-3 )    ( M1-1-D )

[In the formula, $R^D$ represents a C1-C6 chain hydrocarbon group optionally substituted with one or more substituent(s) selected from Group D, or a C3-C7 cycloalkyl group optionally substituted with one or more substituent(s) selected from Group E, and other symbols represent the same meaning as described above.]
The reaction is typically performed in a solvent. Examples of the solvent used in the reaction include ethers, aromatic hydrocarbons, nitriles, aprotic polar solvents, and mixtures of two or more thereof.
Examples of the base used in the reaction include alkali metal hydrides, alkali metal carbonates, and organic bases.
For the reaction, the compound (R-5) is typically used in a ratio of 1 to 5 mol, and the base is typically used in a ratio of 1 to 2 mol, relative to 1 mol of the compound (M1-3).
The reaction temperature is typically in the range of 0 to 100°C. The reaction time is typically in the range of 0.1 to 24 hours.
After completion of the reaction, water is added to the reaction mixture, the mixture is extracted with an organic solvent, and the organic layer is subjected to post-treatment operations such as drying and concentration, thereby allowing to provide the compound (M1-1-D).

Reference production method 2

A compound (M1-1-A) can be produced by reacting a compound represented by a formula (M1-2-2) (hereinafter, referred to as a compound (M1-2-2)) with a compound represented by a formula (R-6) (hereinafter, referred to as a compound (R-6)).

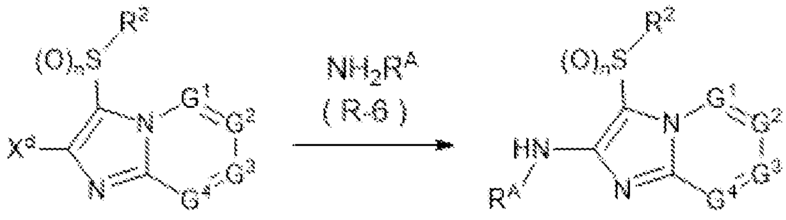


( M1-2-2 )    ( M1-1-A )

[In the formula, $X^d$ represents a fluorine atom or a chlorine atom, and other symbols represent the same meaning as described above.]
The reaction is typically performed in a solvent. Examples of the solvent used in the reaction include aprotic polar solvents, alcohols, and mixtures of two or more thereof.
For the reaction, a base can be used as necessary. Examples of the base used in the reaction include organic

bases and alkali metal carbonates. When a base is used in the reaction, the base is typically used in a ratio of 0.1 to 5 mol relative to 1 mol of the compound (M1-2-2).

For the reaction, the compound (R-6) is typically used in a ratio of 1 to 100 mol relative to 1 mol of the compound (M1-2-2).

The reaction temperature is typically in the range of 25°C to 200°C. The reaction time is typically in the range of 0.1 to 48 hours.

After completion of the reaction, water is added to the reaction mixture, the mixture is extracted with an organic solvent, and the organic layer is subjected to post-treatment operations such as drying and concentration, thereby allowing to provide the compound (M1-1-A).

The compound (R-6) is a commercially available compound, or can be produced using a known method.

Reference production method 3

A compound (M1-3) can be produced by reacting the compound (M1-2-2) with ammonia.

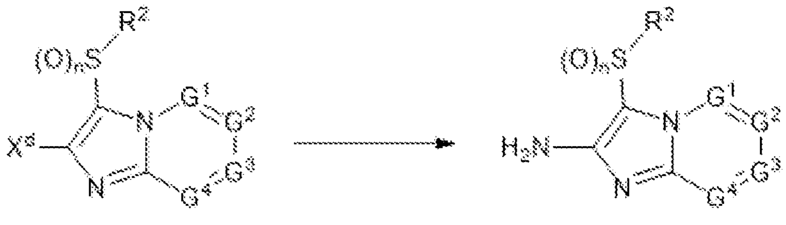

(M1-2-2) (M1-3)

[In the formula, symbols represent the same meaning as described above.]

The reaction is typically performed in a solvent. Examples of the solvent used in the reaction include ethers, nitriles, aprotic polar solvents, alcohols, water, and mixtures of two or more thereof.

For the reaction, a base can be used as necessary. Examples of the base used in the reaction include organic bases and alkali metal carbonates. When a base is used in the reaction, the base is typically used in a ratio of 0.1 to 5 mol relative to 1 mol of the compound (M1-2-2).

Ammonia can also be used as a solution such as ammonia water or an ammonia methanol solution.

For the reaction, ammonia is typically used in a ratio of 1 to 100 mol relative to 1 mol of the compound (M1-2-2).

The reaction temperature is typically in the range of -20°C to 100°C. The reaction time is typically in the range of 0.1 to 48 hours.

After completion of the reaction, water is added to the reaction mixture, the mixture is extracted with an organic solvent, and the organic layer is subjected to post-treatment operations such as drying and concentration, thereby allowing to provide the compound (M1-3).

Reference production method 4

A compound represented by a formula (M1-2b) and a compound represented by a formula (M1-2c) can be produced by reacting a compound represented by a formula (M1-2a) (hereinafter, referred to as a compound (M1-2a)) with an oxidizing agent. The compound represented by the formula (M1-2c) can also be produced by reacting the compound represented by the formula (M1-2b) with an oxidizing agent.

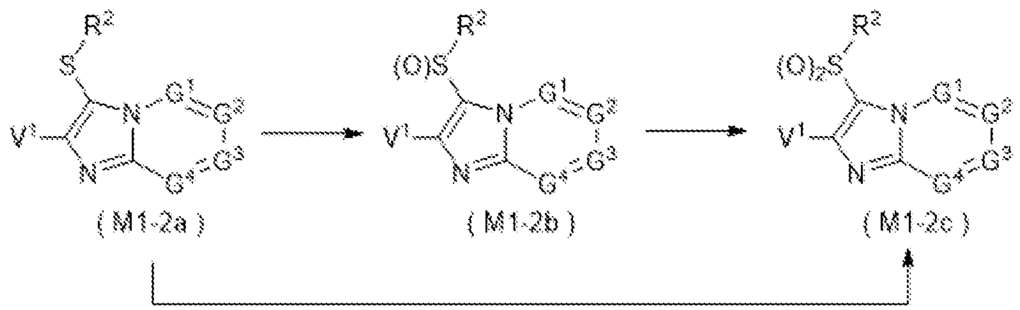

(M1-2a) (M1-2b) (M1-2c)

[In the formula, symbols represent the same meaning as described above.]

These reactions can be performed in accordance with Production method 1.

Reference production method 5

A compound (M1-2a) can be produced according to the following scheme.

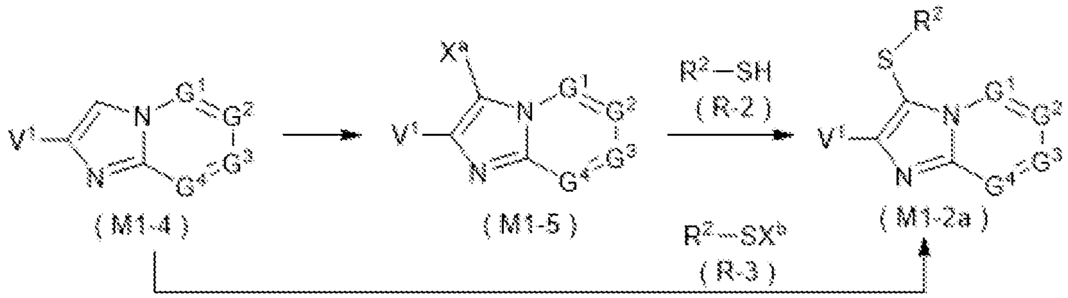


(M1-4) (M1-5) (M1-2a)

[In the formula, symbols represent the same meaning as described above.]

These reactions can be performed in accordance with Production method 6.

A compound represented by a formula (M1-4) (hereinafter, referred to as a compound (M1-4)) is known, or can be produced in accordance with a method described in WO 2015/157093 A, WO 2016/109706 A, Organic & Biomolecular Chemistry, 2017, 15, 4199., Europian Journal of Medicinal Chemistry, 2016, 123, 916., and the like.

Reference production method 6

A compound (II-O) can be produced by reacting the compound (M1-3) with the compound (M2-1) or the compound (M2-2).

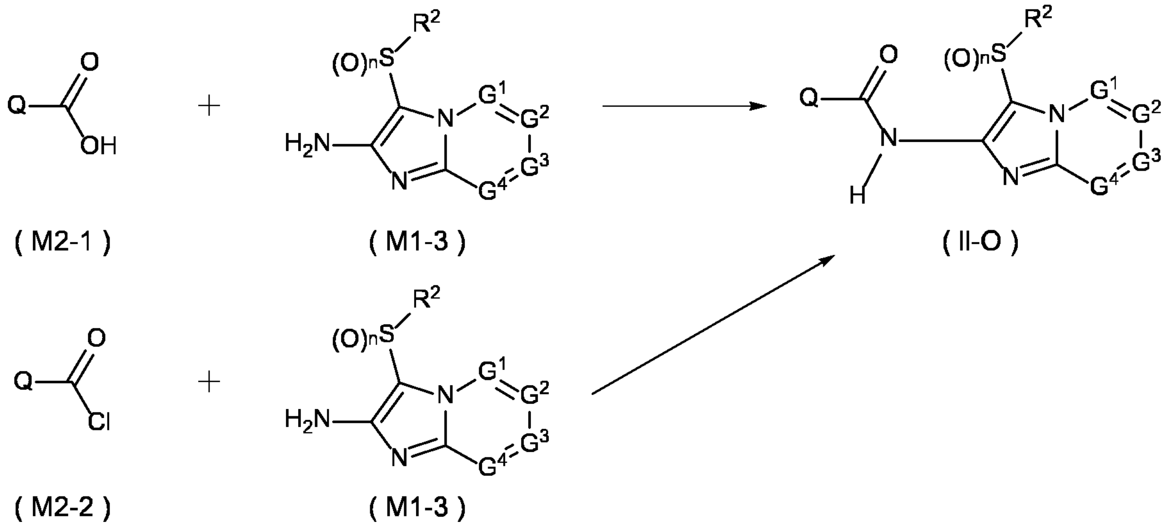

(M2-1) (M1-3) (II-O)

(M2-2) (M1-3)

[In the formula, symbols represent the same meaning as described above.]

These reactions can be performed in accordance with Production method 2 or Production method 3, using the compound (M1-3) in place of the compound (M1-1-A).

Reference production method 7

A compound represented by a formula (M3-1-A) can be produced by reacting a compound represented by a formula (M1-6-A) (hereinafter, referred to as a compound (M1-6-A)) with the compound (M2-1) or compound (M2-2).

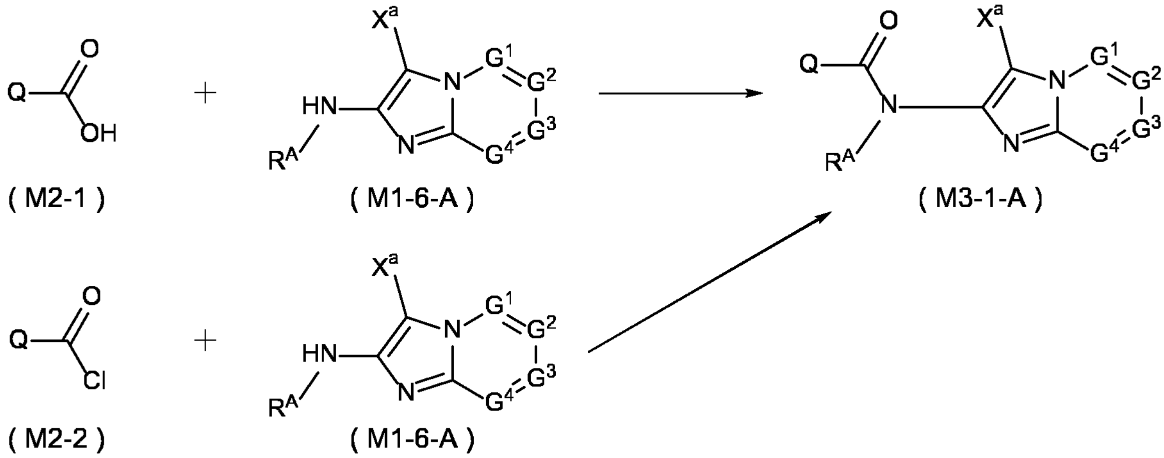

( M2-1 ) ( M1-6-A ) ( M3-1-A )

( M2-2 ) ( M1-6-A )

[In the formula, symbols represent the same meaning as described above.]

These reactions can be performed in accordance with Production method 2 or Production method 3, using the compound (M1-6-A) in place of the compound (M1-1-A).

Reference production method 8

A compound represented by a formula (M1-6-D) (hereinafter, referred to as a compound (M1-6-D)) can be produced by the following scheme.

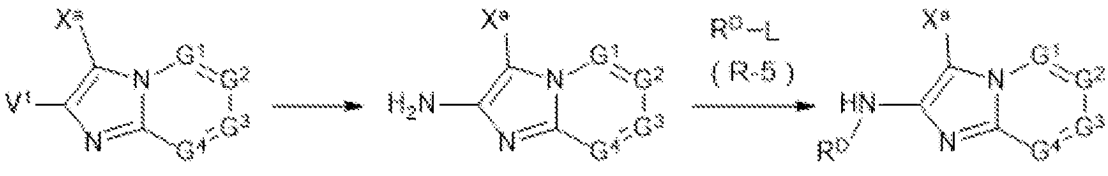


( M1-5 ) ( M1-7 ) ( M1-6-D )

[In the formula, symbols represent the same meaning as described above.]

First, a method for producing a compound represented by a formula (M1-7) (hereinafter, referred to as a compound (M1-7)) will be described.

The compound (M1-7) can be produced by reacting a compound represented by a formula (M1-5) (hereinafter, referred to as a compound (M1-5)) with ammonia. The reaction can be performed in accordance with Reference production method 3, using the compound (M1-5) in place of the compound (M1-2-2).

Then, a method for producing the compound (M1-6-D) will be described.

The compound (M1-6-D) can be produced by reacting the compound (M1-7) with the compound (R-5), in the presence of a base. The reaction can be performed in accordance with Reference production method 1, using the compound (M1-7) in place of the compound (M1-3).

Reference production method 9

A compound (M1-6-A) can be produced according to the following scheme.

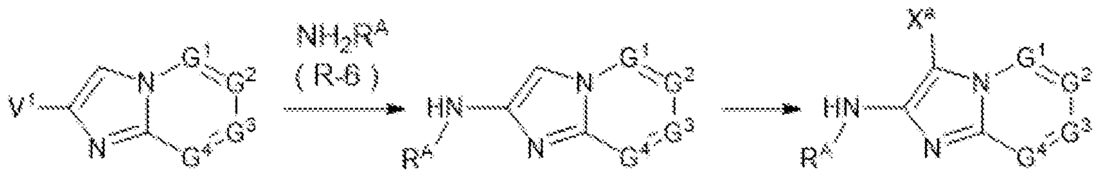


( M1-4 ) ( M1-8-A ) ( M1-6-A )

[In the formula, symbols represent the same meaning as described above.]

First, a method for producing a compound represented by a formula (M1-8-A) (hereinafter, referred to as a compound (M1-8-A)) will be described.

The compound (M1-8-A) can be produced by reacting the compound (M1-4) with the compound (R-6), in the presence of a base. The reaction can be performed in accordance with Reference production method 2, using the compound (M1-4) in place of the compound (M1-2-2).

Then, a method for producing the compound (M1-6-A) will be described.

The compound (M1-6-A) can be produced by reacting the compound (M1-8-A) with a halogenating agent. The reaction can be performed in accordance with a step 6-A of Production method 6, using the compound (M1-8-A) in place of the compound (III-1).

Reference production method 10

A compound represented by a formula (M3-1-B) (hereinafter, referred to as a compound (M3-1-B)) can be produced by the following scheme.

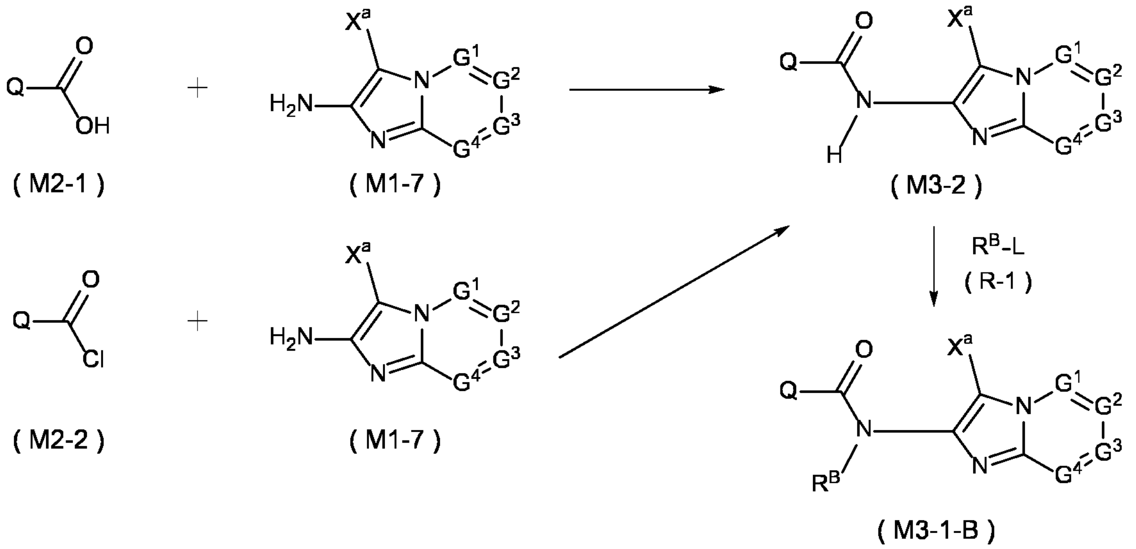

( M2-1 ) ( M1-7 ) ( M3-2 ) ( M2-2 ) ( M1-7 ) ( M3-1-B )

[In the formula, symbols represent the same meaning as described above.]

First, a method for producing a compound represented by a formula (M3-2) (hereinafter, referred to as a compound (M3-2)) will be described.

A compound (M3-2) can be produced by reacting the compound (M1-7) with the compound (M2-1) or the compound (M2-2). These reactions can be performed in accordance with Production method 2 or Production method 3, using the compound (M1-7) in place of the compound (M1-1-A).

Then, a method for producing the compound (M3-1-B) will be described.

The compound (M3-1-B) can be produced by reacting the compound (M3-2) with the compound (R-1), in the presence of a base. The reaction can be performed in accordance with Production method 4, using the compound (M3-2) in place of the compound (II).

Reference production method 11

A compound represented by a formula (III-1-A) (hereinafter, referred to as a compound (III-1-A)) can be produced by the following scheme.

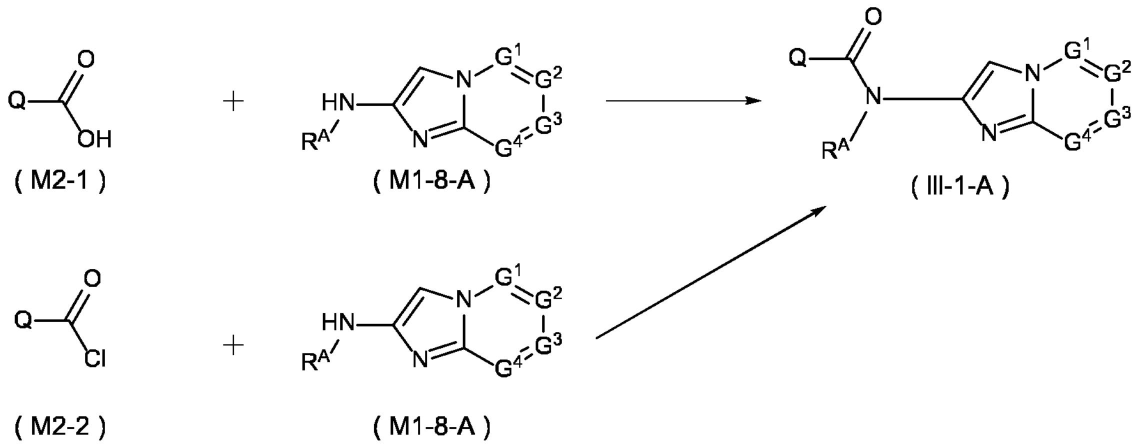

(M2-1) (M1-8-A) (III-1-A)

(M2-2) (M1-8-A)

[In the formula, symbols represent the same meaning as described above.]

A compound (III-1-A) can be produced by reacting the compound (M1-8-A) with the compound (M2-1) or the compound (M2-2). These reactions can be performed in accordance with Production method 2 or Production method 3, using the compound (M1-8-A) in place of the compound (M1-1-A).

Reference production method 12

A compound represented by a formula (III-1-B) (hereinafter, referred to as a compound (III-1-B)) can be produced by the following scheme.

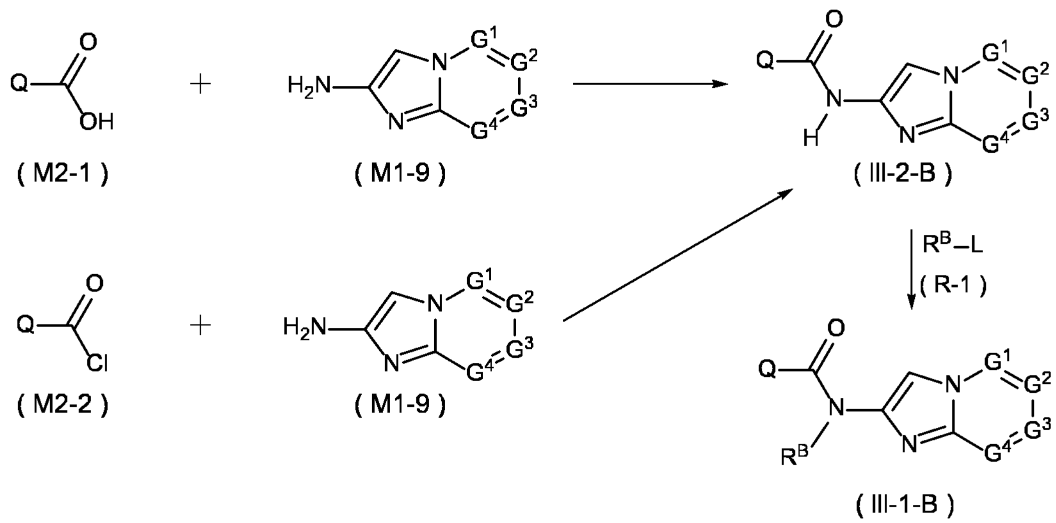


(M2-1) (M1-9) (III-2-B)

(M2-2) (M1-9) (III-1-B)

[In the formula, symbols represent the same meaning as described above.]

First, a method for producing a compound represented by a formula (III-2-B) (hereinafter, referred to as a compound (III-2-B)) will be described.

The compound (III-2-B) can be produced by reacting a compound represented by a formula (M1-9) (hereinafter, referred to as a compound (M1-9)) with the compound (M2-1) or compound (M2-2). These reactions can be performed in accordance with Production method 2 or Production method 3, using the compound (M1-9) in place of the compound (M1-1-A).

Then, a method for producing the compound (III-1-B) will be described.

The compound (III-1-B) can be produced by reacting the compound (III-2-B) with the compound (R-1), in the presence of a base. The reaction can be performed in accordance with Production method 4, using the compound (III-2-B) in place of the compound (II).

Reference production method 13

A compound (M1-9) can be produced by reacting the compound (M1-4) with ammonia.

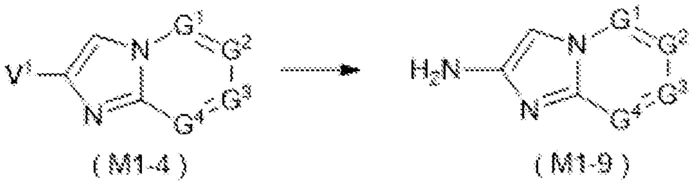

[In the formula, symbols represent the same meaning as described above.]

The reaction can be performed in accordance with Reference production method 3, using the compound (M1-4) in place of the compound (M1-2-2).

The Present compound may be mixed with or used in combination with one or more ingredient(s) selected from the group consisting of the following Group (a), Group (b), Group (c), and Group (d) (hereinafter referred to as "Present ingredient").

When the Present compound is mixed with or used in combination with the Present ingredient, they are used simultaneously, separately, or at time intervals with each other.

When the Present compound is used simultaneously with the Present ingredient, the Present compound and the Present ingredient may be contained in separate formulations or contained in one formulation.

One aspect of the present invention provides a composition comprising one or more ingredient(s) selected from the group consisting of Group (a), Group (b), Group (c), and Group (d), and the Present compound (hereinafter referred to as "Composition A").

Group (a) is a group consisting of acetylcholinesterase inhibitors (for example, carbamate insecticides and organophosphate insecticides), GABA-gated chloride channel blockers (for example, phenylpyrazole insecticides), sodium channel modulators (for example, pyrethroid insecticides), nicotinic acetylcholine receptor competitive modulators (for example, neonicotinoid insecticides), nicotinic acetylcholine receptor allosteric modulators, glutamate-gated chloride channel allosteric modulators (for example, macrolide insecticides), juvenile hormone mimics, multisite inhibitors, chordotonal organ TRPV channel modulators, mite growth inhibitors, microbial disruptors of insect midgut membranes, inhibitors of mitochondrial ATP synthase, uncouplers of oxidative phosphorylation, nicotinic acetylcholine receptor channel blockers (for example, nereistoxin insecticides), inhibitors of chitin biosynthesis, moulting disruptors, ecdysone receptor agonists, octopamine receptor agonists, mitochondrial complexes I, II, III, and IV electron transport inhibitors, voltage-dependent sodium channel blockers, inhibitors of acetyl CoA carboxylase, ryanodine receptor modulators (for example, diamide insecticides), chordotonal organ modulators, and microbial insecticides, and other insecticidal active ingredients, miticidal active ingredients, and nematicidal active ingredients. These ingredients are described in the classification on the basis of action mechanism by IRAC.

Group (b) is a group consisting of nucleic acids synthesis inhibitors (for example, phenylamide fungicides and acylamino acid fungicides), cell division and cytoskeleton inhibitors (for example, MBC fungicides), respiration inhibitors (for example, QoI fungicides, and QiI fungicides), amino acids synthesis and protein synthesis inhibitors (for example, anilino-pyrimidine fungicides), signal transduction inhibitors, lipid synthesis and membrane synthesis inhibitors, sterol biosynthesis inhibitors (for example, DMI fungicides such as triazole fungicides), cell wall biosynthesis inhibitors, melanin synthesis inhibitors, plant defense inducers, fungicides with multi-site contact activity, microbial fungicides, and other fungicidal active ingredients. These ingredients are described in the classification on the basis of action mechanism by FRAC.

Group (c) is a group of plant growth regulatory ingredients (including mycorrhizal fungi and root nodule bacteria).

Group (d) is a group of repellent ingredients.

Hereinafter, examples of the combination of the Present ingredient and the Present compound are described. For example, "alanycarb + SX" indicates a combination of alanycarb and SX.

The abbreviation of "SX" indicates any one of the Present compound selected from the Compound groups SX1 to SX1908 described in Examples. Also, all of the following Present ingredient are known ingredients, and may be obtained from commercially available formulations, or may be prepared by known methods. When the Present ingredient is a microorganism, it may also be available from a bacterial authority depository. Further, the number in parentheses represents the CAS RN (registered trademark).

Combinations of the Present ingredient in the above Group (a) and the Present compound:
abamectin + SX, acephate + SX, acequinocyl + SX, acetamiprid + SX, acetoprole + SX, acrinathrin + SX, acynonapyr + SX, afidopyropen + SX, afoxolaner + SX, alanycarb + SX, aldicarb + SX, allethrin + SX, alpha-cypermethrin + SX, alpha-endosulfan + SX, aluminium phosphide + SX, amitraz + SX, azadirachtin + SX, azamethiphos + SX, azinphos-ethyl + SX, azinphos-methyl + SX, azocyclotin + SX, bark of Celastrus angulatus + SX, bendiocarb + SX, benfluthrin + SX, benfuracarb + SX, bensultap + SX, benzoximate + SX, benzpyrimoxan + SX, beta-cyfluthrin + SX, beta-cypermethrin + SX, bifenazate + SX, bifenthrin + SX, bioallethrin + SX, bioresmethrin + SX, bistrifluron + SX, borax + SX, boric acid + SX, broflanilide + SX, bromopropylate + SX, buprofezin + SX, butocarboxim + SX, butoxycarboxim + SX, cadusafos + SX,

calcium phosphide + SX, carbaryl + SX, carbofuran + SX, carbosulfan + SX, cartap hydrochloride + SX, cartap + SX, chinomethionat + SX, chlorantraniliprole + SX, chlordane + SX, chlorethoxyfos + SX, chlorfenapyr + SX, chlorfenvinphos + SX, chlorfluazuron + SX, chlormephos + SX, chloropicrin + SX, chlorpyrifos + SX, chlorpyrifos-methyl + SX, chromafenozide + SX, clofentezine + SX, clothianidin + SX, concanamycin A + SX, coumaphos + SX, cryolite + SX, cyanophos + SX, cyantraniliprole + SX, cyclaniliprole + SX, cyclobutrifluram + SX, cycloprothrin + SX, cycloxaprid + SX, cyenopyrafen + SX, cyetpyrafen + SX, cyflumetofen + SX, cyfluthrin + SX, cyhalodiamide + SX, cyhalothrin + SX, cyhexatin + SX, cypermethrin + SX, cyphenothrin + SX, cyproflanilide + SX, cyromazine + SX, dazomet + SX, deltamethrin + SX, demeton-S-methyl + SX, diafenthiuron + SX, diazinon + SX, dichlorvos + SX, dicloromezotiaz + SX, dicofol + SX, dicrotophos + SX, diflovidazin + SX, diflubenzuron + SX, dimefluthrin + SX, dimethoate + SX, dimethylvinphos + SX, dimpropyridaz + SX, dinotefuran + SX, disodium octaborate + SX, disulfoton + SX, DNOC(2-methyl-4,6-dinitrophenol) + SX, doramectin + SX, dried leaves of Dryopteris filix-mas + SX, emamectin-benzoate + SX, empenthrin + SX, endosulfan + SX, EPN(O-ethyl O-(4-nitrophenyl) phenylphosphonothioate) + SX, epsilon-metofluthrin + SX, epsilon-momfluorothrin + SX, esfenvalerate + SX, ethiofencarb + SX, ethion + SX, ethiprole + SX, ethoprophos + SX, etofenprox + SX, etoxazole + SX, extract of Artemisia absinthium + SX, extract of Azadirachta indica + SX, extract of Cassia nigricans + SX, extract of clitoria ternatea + SX, extract of Symphytum officinale + SX, extract of Chenopodium ambrosioides + SX, extract of Tanacetum vulgare + SX, extract of Urtica dioica + SX, extract of Viscum album + SX, famphur + SX, fenamiphos + SX, fenazaquin + SX, fenbutatin oxide + SX, fenitrothion + SX, fenmezoditiaz + SX, fenobucarb + SX, fenoxycarb + SX, fenpropathrin + SX, fenpyroximate + SX, fenthion + SX, fenvalerate + SX, fipronil + SX, flometoquin + SX, flonicamid + SX, fluacrypyrim + SX, fluazaindolizine + SX, fluazuron + SX, flubendiamide + SX, fluchlordiniliprole + SX, flucycloxuron + SX, flucythrinate + SX, fluensulfone + SX, flufenoprox + SX, flufenoxuron + SX, flufiprole + SX, flumethrin + SX, flupentiofenox + SX, flupyradifurone + SX, flupyrimin + SX, fluralaner + SX, fluvalinate + SX, fluxametamide + SX, formetanate + SX, fosthiazate + SX, furamethrin + SX, furathiocarb + SX, gamma-cyhalothrin + SX, GS-omega/kappa HXTX-Hv1a peptide + SX, halfenprox + SX, halofenozide + SX, heptafluthrin + SX, heptenophos + SX, hexaflumuron + SX, hexythiazox + SX, potassium salt of hop beta acid + SX, hydramethylnon + SX, hydroprene + SX, imicyafos + SX, imidacloprid + SX, imidaclothiz + SX, imiprothrin + SX, indazapyroxamet + SX, indoxacarb + SX, isocycloseram + SX, isofenphos + SX, isoprocarb + SX, isopropyl-O-(methoxyaminothiophosphoryl) salicylate + SX, isoxathion + SX, ivermectin + SX, kadethrin + SX, kappa-tefluthrin + SX, kappa-bifenthrin + SX, kinoprene + SX, lambda-cyhalothrin + SX, lenoremycin + SX, lepimectin + SX, lime sulfur + SX, lotilaner + SX, lufenuron + SX, machine oil + SX, malathion + SX, mecarbam + SX, meperfluthrin + SX, metaflumizone + SX, metam + SX, methamidophos + SX, methidathion + SX, methiocarb + SX, methomyl + SX, methoprene + SX, methoxychlor + SX, methoxyfenozide + SX, methyl bromide + SX, metofluthrin + SX, metolcarb + SX, metoxadiazone + SX, mevinphos + SX, milbemectin + SX, milbemycin oxime + SX, mivorilaner + SX, modoflaner + SX, momfluorothrin + SX, monocrotophos + SX, moxidectin + SX, naled + SX, nicofluprole + SX, nicotine + SX, nicotine-sulfate + SX, nitenpyram + SX, novaluron + SX, noviflumuron + SX, oil of the seeds of Chenopodium anthelminticum + SX, omethoate + SX, oxamyl + SX, oxazosulfyl + SX, oxydemeton-methyl + SX, parathion + SX, parathion-methyl + SX, permethrin + SX, phenothrin + SX, phenthoate + SX, phorate + SX, phosalone + SX, phosmet + SX, phosphamidon + SX, phosphine + SX, phoxim + SX, pirimicarb + SX, pirimiphos-methyl + SX, prallethrin + SX, profenofos + SX, profluthrin + SX, propargite + SX, propetamphos + SX, propoxur + SX, propylene glycol alginate + SX, prothiofos + SX, pyflubumide + SX, pymetrozine + SX, pyraclofos + SX, pyrethrins + SX, pyridaben + SX, pyridalyl + SX, pyridaphenthion + SX, pyrifluquinazone + SX, pyrimidifen + SX, pyriminostrobin + SX, pyriprole + SX, pyriproxyfen + SX, quinalphos + SX, resmethrin + SX, rotenone + SX, ryanodine + SX, sarolaner + SX, selamectin + SX, sigma-cypermethrin + SX, silafluofen + SX, sodium borate + SX, sodium metaborate + SX, spidoxamat + SX, spinetoram + SX, spinosad + SX, spirobudifen + SX, spirodiclofen + SX, spiromesifen + SX, spiropidion + SX, spirotetramat + SX, sulfluramid + SX, sulfotep + SX, sulfoxaflor + SX, sulfur + SX, sulfuryl fluoride + SX, tartar emetic + SX, tau-fluvalinate + SX, tebufenozide + SX, tebufenpyrad + SX, tebupirimfos + SX, teflubenzuron + SX, tefluthrin + SX, temephos + SX, terbufos + SX, terpene constituents of the extract of chenopodium ambrosioides near ambrosioides + SX, tetrachlorantraniliprole + SX, tetrachlorvinphos + SX, tetradifon + SX, tetramethrin + SX, tetramethylfluthrin + SX, tetraniliprole + SX, theta-cypermethrin + SX, thiacloprid + SX, thiamethoxam + SX, thiocyclam + SX, thiodicarb + SX, thiofanox + SX, thiometon + SX, thiosultap-disodium + SX, thiosultap-monosodium + SX, tigolaner + SX, tiorantraniliprole + SX, tioxazafen + SX, tolfenpyrad + SX, tralomethrin + SX, transfluthrin + SX, triazamate + SX, triazophos + SX, trichlorfon + SX, trifluenfuronate + SX, triflumezopyrim + SX, triflumuron + SX, trimethacarb + SX, tyclopyrazoflor + SX, umifoxolaner + SX, vamidothion + SX, wood extract of Quassia amara + SX, XMC (3,5-dimethylphenyl N-methylcarbamate) + SX, xylylcarb + SX, zeta-cypermethrin + SX, zinc phosphide + SX, 4-[5-(3,5-dichlorophenyl)-5-(trifluoromethyl)-4,5-dihydro-1,2-oxazol-3-yl]-2-methyl-N-(1-oxothietan-3-yl)benzamide (1241050-20-3) + SX, 3-methoxy-N-(5-{5-(trifluoromethyl)-5-[3-(trifluoromethyl) phenyl]-4,5-dihydro-1,2-oxazol-3-yl}indan-1-yl)propanamide (1118626-57-5) + SX, 2-({2-fluoro-4-methyl-5-[(2,2,2-trifluoroethyl)sulfinyl]phenyl}imino)-3-(2,2,2-trifluoroethyl)-1,3-thiazolidin-4-one (1445683-71-5) + SX, (2Z)-2-({2-fluoro-4-methyl-5-[(R)-(2,2,2-trifluoroethyl)sulfinyl]phenyl}imino)-3-(2,2,2-trifluoroethyl)-1,3-thiazolidin-4-one (2377084-09-6) + SX, N-{4-chloro-3-[(1-cyanocyclopropyl)carbamoyl]phenyl}-1-methyl-4-(methanesulfonyl)-3-(1,1,2,2,2-pentafluoroethyl)-1H-pyrazole-3-carboxamide (1400768-21-9) + SX, N-[3-chloro-1-(pyridin-3-yl)-1H-pyrazol-4-yl]-2-(methanesulfonyl)propanamide (2396747-83-2) + SX, 1,4-dimethyl-2-[2-(pyridin-3-yl)-2H-indazol-5-yl]-1,2,4-triazolidine-3,5-dione (2171099-09-3) + SX, 2-isopropyl-5-[(3,4,4-trifluoro-3-buten-1-yl)sulfonyl]-1,3,4-thiadiazole (2058052-95-0) + SX, N-({2-fluoro-4-[(2S,3S)-2-hydroxy-3-(3,4,5-trichlorophenyl)-3-(trifluoromethyl)pyrrolidin-1-yl]phenyl}methyl)cyclopropanecarboxamide + SX, 7-fluoro-N-[1-(methylsulfanyl)-2-methylpropan-2-yl]-2-(pyridin-3-yl)-2H-indazole-4-carboxamide + SX, 7-fluoro-N-[1-(methanesulfinyl)-2-methylpropan-2-yl]-2-(pyridin-3-yl)-2H-indazole-4-carboxamide + SX, 7-fluoro-N-[1-(methanesulfonyl)-2-methylpropan-2-yl]-2-(pyridin-3-yl)-2H-indazole-4-carboxamide + SX, N-[1-(difluoromethyl)cyclopropyl]-2-(pyridin-3-yl)-2H-indazole-4-carboxamide + SX, 2,9-dihydro-9-(methoxymethyl)-2-(pyridin-3-yl)-10H-pyrazolo[3,4-f]pyrido[2,3-b] [1,4]oxazepin-10-one (2607927-97-7) + SX, BT crop protein Cry1Ab + SX, BT crop protein CrylAc + SX, BT crop protein CrylFa + SX, BT crop protein Cry1A.105 + SX, BT crop protein Cry2Ab + SX, BT crop protein Vip3A + SX, BT crop protein mCry3A + SX, BT crop protein Cry3Ab + SX, BT crop protein Cry3Bb + SX, BT crop protein Cry34Ab1/Cry35Ab1 + SX, Adoxophyes orana granulosis virus strain BV-0001 + SX, Anticarsia gemmatalis MNPV(multiple nucleocapsid nucleopolyhedrovirus) + SX, Autographa californica MNPV + SX, Cydia pomonella GV(granulovirus) strain V15 + SX, Cydia pomonella GV strain V22 + SX, Cryptophlebia leucotreta GV + SX, Dendrolimus punctatus cypovirus + SX, Helicoverpa armigera NPV(nucleopolyhedrovirus) strain BV-0003 + SX, Helicoverpa zea NPV + SX, Lymantria dispar NPV + SX, Mamestra brassicae NPV + SX, Mamestra configurata NPV + SX, Neodiprion abietis NPV + SX, Neodiprion lecontei NPV + SX, Neodiprion sertifer NPV + SX, Nosema locustae + SX, Orgyia pseudotsugata NPV + SX, Pieris rapae GV + SX, Plodia interpunctella GV + SX, Spodoptera exigua MNPV + SX, Spodoptera littoralis MNPV + SX, Spodoptera litura NPV + SX, Arthrobotrys dactyloides + SX, Bacillus firmus strain GB-126 + SX, Bacillus firmus strain I-1582 + SX, Bacillus firmus strain NCIM2637 + SX, Bacillus megaterium + SX, Bacillus sp. strain AQ175 + SX, Bacillus sp. strain AQ177 + SX, Bacillus sp. strain AQ178 + SX, Bacillus sphaericus strain 2362 serotype H5a5b + SX, Bacillus sphaericus strain ABTS1743 + SX, Bacillus thuringiensis strain AQ52 + SX, Bacillus thuringiensis strain BD#32 + SX, Bacillus thuringiensis strain CR-371 + SX, Bacillus thuringiensis subsp. Aizawai strain ABTS-1857 + SX, Bacillus thuringiensis subsp. Aizawai strain AM65-52 + SX, Bacillus thuringiensis subsp. Aizawai strain GC-91 + SX, Bacillus thuringiensis subsp. Aizawai strain NB200 + SX, Bacillus thuringiensis subsp. Aizawai Serotype strain H-7 + SX, Bacillus thuringiensis subsp. Kurstaki strain ABTS351 + SX, Bacillus thuringiensis subsp. Kurstaki strain BMP123 + SX, Bacillus thuringiensis subsp. Kurstaki strain CCT1306) + SX, Bacillus thuringiensis subsp. Kurstaki strain EG2348 + SX, Bacillus thuringiensis subsp. Kurstaki strain EG7841 + SX, Bacillus thuringiensis subsp. Kurstaki strain EVB113-19 + SX, Bacillus thuringiensis subsp. Kurstaki strain F810 + SX, Bacillus thuringiensis subsp. Kurstaki strain HD-1 + SX, Bacillus thuringiensis subsp. Kurstaki strain PB54 + SX, Bacillus thuringiensis subsp. Kurstaki strain SA-11 + SX, Bacillus thuringiensis subsp. Kurstaki strain SA-12 + SX, Bacillus thuringiensis subsp. Tenebriosis strain NB176 + SX, Bacillus thuringiensis subsp. Thuringiensis strain MPPL002 + SX, Bacillus thuringiensis subsp. morrisoni + SX, Bacillus thuringiensis var. colmeri + SX, Bacillus thuringiensis var. darmstadiensis strain 24-91 + SX, Bacillus thuringiensis var. dendrolimus + SX, Bacillus thuringiensis var. galleriae + SX, Bacillus thuringiensis var. israelensis strain BMP144 + SX, Bacillus thuringiensis var. israelensis serotype strain H-14 + SX, Bacillus thuringiensis var. japonensis strain buibui + SX, Bacillus thuringiensis var. san diego strain M-7 + SX, Bacillus thuringiensis var. 7216 + SX, Bacillus thuringiensis var. aegypti + SX, Bacillus thuringiensis var. T36 + SX, Beauveria bassiana strain ANT-03 + SX, Beauveria bassiana strain ATCC74040 + SX, Beauveria bassiana strain GHA + SX, Beauveria brongniartii + SX, Burkholderia rinojensis strain A396 + SX, Chromobacterium subtsugae strain PRAA4-1T + SX, Dactyllela ellipsospora + SX, Dectylaria thaumasia + SX, Hirsutella minnesotensis + SX, Hirsutella rhossiliensis + SX, Hirsutella thompsonii + SX, Lagenidium giganteum + SX, Lecanicillium lecanii strain KV01 + SX, Lecanicillium lecanii conidia of strain DAOM198499 + SX, Lecanicillium lecanii conidia of strain DAOM216596 + SX, Lecanicillium muscarium strain Ve6 + SX, Metarhizium anisopliae strain F52 + SX, Metarhizium anisopliae var. acridum + SX, Metarhizium anisopliae var. anisopliae BIPESCO 5/F52 + SX, Metarhizium flavoviride + SX, Monacrosporium phymatopagum + SX, Paecilomyces fumosoroseus Apopka strain 97 + SX, Paecilomyces lilacinus strain 251 + SX, Paecilomyces tenuipes strain T1 + SX, Paenibacillus popilliae + SX, Pasteuria nishizawae strain Pn1 + SX, Pasteuria penetrans + SX, Pasteuria usgae + SX, Pasteuria thornei + SX, Serratia entomophila + SX, Verticillium chlamydosporium + SX, Verticillium lecani strain NCIM1312 + SX, Wolbachia pipientis + SX.

Combination of the Present ingredient in the above Group (b) and the Present compound:

acibenzolar-S-methyl + SX, aldimorph + SX, ametoctradin + SX, aminopyrifen + SX, amisulbrom + SX, anilazine + SX, azaconazole + SX, azoxystrobin + SX, basic copper sulfate + SX, benalaxyl + SX, benalaxyl-M + SX, benodanil + SX, benomyl + SX, benthiavalicarb + SX, benthiavalicarb-isopropyl + SX, benzovindiflupyr + SX, binapacryl + SX, biphenyl + SX, bitertanol + SX, bixafen + SX, blasticidin-S + SX, Bordeaux mixture + SX, boscalid + SX, bromothalonil + SX, bromuconazole + SX, bupirimate + SX, captafol + SX, captan + SX, carbendazim + SX, carboxin + SX, carpropamid + SX, chinomethionat + SX, chitin + SX, chloroinconazide + SX, chloroneb + SX, chlorothalonil + SX, chlozolinate + SX, colletochlorin B + SX, copper (II) acetate + SX, copper (II) hydroxide + SX, copper oxychloride + SX, copper(II) sulfate + SX, coumoxystrobin + SX, cyazofamid + SX, cyflufenamid + SX, cymoxanil + SX, cyproconazole + SX, cyprodinil + SX, dichlobentiazox + SX, dichlofluanid + SX, diclocymet + SX, diclomezine + SX, dicloran + SX, diethofencarb + SX, difenoconazole + SX, diflumetorim + SX, dimethachlone + SX, dimethirimol + SX, dimethomorph + SX, dimoxystrobin +

SX, diniconazole + SX, diniconazole-M + SX, dinocap + SX, dipotassium hydrogenphosphite + SX, dipymetitrone + SX, dithianon + SX, dodecylbenzenesulphonic acid bisethylenediamine copper(II) salt + SX, dodemorph + SX, dodine + SX, edifenphos + SX, enoxastrobin + SX, epoxiconazole + SX, etaconazole + SX, ethaboxam + SX, ethirimol + SX, etridiazole + SX, extract of Allium sativum + SX, extract of the cotyledons of lupine plantlets ("BLAD") + SX, extract of Equisetum arvense + SX, extract of Melaleuca alternifolia + SX, extract of Reynoutria sachalinensis + SX, extract of Tropaeolum majus + SX, famoxadone + SX, fenamidone + SX, fenaminstrobin + SX, fenarimol + SX, fenbuconazole + SX, fenfuram + SX, fenhexamid + SX, fenoxanil + SX, fenpiclonil + SX, fenpicoxamid + SX, fenpropidin + SX, fenpropimorph + SX, fenpyrazamine + SX, fentin acetate + SX, fentin chloride + SX, fentin hydroxide + SX, ferbam + SX, ferimzone + SX, florylpicoxamid + SX, fluazinam + SX, flubeneteram + SX, fludioxonil + SX, flufenoxadiazam + SX, flufenoxystrobin + SX, fluindapyr + SX, flumetylsulforim + SX, flumorph + SX, fluopicolide + SX, fluopyram + SX, fluopimomide + SX, fluoroimide + SX, fluoxapiprolin + SX, fluoxastrobin + SX, fluoxytioconazole + SX, fluquinconazole + SX, flusilazole + SX, flusulfamide + SX, flutianil + SX, flutolanil + SX, flutriafol + SX, fluxapyroxad + SX, folpet + SX, fosetyl + SX, fosetyl-aluminium + SX, fuberidazole + SX, furalaxyl + SX, furametpyr + SX, guazatine + SX, hexaconazole + SX, hymexazole + SX, imazalil + SX, imibenconazole + SX, iminoctadine + SX, iminoctadine triacetate + SX, inpyrfluxam + SX, iodocarb + SX, ipconazole + SX, ipfentrifluconazole + SX, ipflufenoquin + SX, iprobenfos + SX, iprodione + SX, iprovalicarb + SX, isofetamid + SX, isoflucypram + SX, isoprothiolane + SX, isopyrazam + SX, isotianil + SX, kasugamycin + SX, kresoxim-methyl + SX, laminarin + SX, leaves and bark of Quercus + SX, mancozeb + SX, mandestrobin + SX, mandipropamid + SX, maneb + SX, mefentrifluconazole + SX, mepanipyrim + SX, mepronil + SX, meptyldinocap + SX, metalaxyl + SX, metalaxyl-M + SX, metarylpicoxamid + SX, metconazole + SX, methasulfocarb + SX, metiram + SX, metominostrobin + SX, metrafenone + SX, metyltetraprole + SX, myclobutanil + SX, naftifine + SX, nuarimol + SX, octhilinone + SX, ofurace + SX, orysastrobin + SX, oxadixyl + SX, oxathiapiprolin + SX, oxine-copper + SX, oxolinic acid + SX, oxpoconazole + SX, oxpoconazole fumarate + SX, oxycarboxin + SX, oxytetracycline + SX, pefurazoate + SX, penconazole + SX, pencycuron + SX, penflufen + SX, penthiopyrad + SX, phenamacril + SX, phosphorous acid + SX, phthalide + SX, picarbutrazox + SX, picoxystrobin + SX, piperalin + SX, polyoxins + SX, potassium hydrogencarbonate + SX, potassium dihydrogenphosphite + SX, probenazole + SX, prochloraz + SX, procymidone + SX, propamidine + SX, propamocarb + SX, propiconazole + SX, propineb + SX, proquinazid + SX, prothiocarb + SX, prothioconazole + SX, pydiflumetofen + SX, pyraclostrobin + SX, pyrametostrobin + SX, pyraoxystrobin + SX, pyrapropoyne + SX, pyraziflumid + SX, pyrazophos + SX, pyribencarb + SX, pyributicarb + SX, pyridachlometyl + SX, pyrifenox + SX, pyrimethanil + SX, pyrimorph + SX, pyriofenone + SX, pyrisoxazole + SX, pyroquilon + SX, Quillaja extract + SX, quinconazole + SX, quinofumelin + SX, quinoxyfen + SX, quintozene + SX, Saponins of Chenopodium quinoa + SX, seboctylamine + SX, sedaxane + SX, silthiofam + SX, simeconazole + SX, sodium hydrogencarbonate + SX, spiroxamine + SX, streptomycin + SX, sulfur + SX, tebuconazole + SX, tebufloquin + SX, teclofthalam + SX, tecnazene + SX, terbinafine + SX, tetraconazole + SX, thiabendazole + SX, thifluzamide + SX, thiophanate + SX, thiophanate-methyl + SX, thiram + SX, thymol + SX, tiadinil + SX, tolclofos-methyl + SX, tolfenpyrad + SX, tolprocarb + SX, tolylfluanid + SX, triadimefon + SX, triadimenol + SX, triazoxide + SX, triclopyricarb + SX, tricyclazole + SX, tridemorph + SX, trifloxystrobin + SX, triflumizole + SX, triforine + SX, triticonazole + SX, validamycin + SX, valifenalate + SX, vinclozolin + SX, yellow mustard powder + SX, zinc thiazole + SX, zineb + SX, ziram + SX, zoxamide + SX, N'-[4-({3-[(4-chlorophenyl)methyl]-1,2,4-thiadiazol-5-yl}oxy)-2,5-dimethylphenyl]-N-ethyl-N-methylmethanimidamide (1202781-91-6) + SX, N'-{4-[(4,5-dichlorothiazol-2-yl)oxy]-2,5-dimethylphenyl}-N-ethyl-N-methylmethanimidamide (929908-57-6) + SX, N'-(2,5-dimethyl-4-phenoxyphenyl)-N-ethyl-N-methylmethanimidamide (1052688-31-9) + SX, N'-[5-chloro-4-(2-fluorophenoxy)-2-methylphenyl]-N-ethyl-N-methylmethanimidamide (2055589-28-9) + SX, N'-[2-chloro-4-(2-fluorophenoxy)-5-methylphenyl]-N-ethyl-N-methylmethanimidamide (2055756-21-1) + SX, N'-(2-chloro-4-phenoxy-5-methylphenyl)-N-ethyl-N-methylmethanimidamide (2062599-39-5) + SX, N'-[4-(1-hydroxy-1-phenyl-2,2,2-trifluoroethyl)-2-methyl-5-methoxyphenyl]-N-isopropyl-N-methylmethanimidamide (2101814-55-3) + SX, N'-[5-bromo-6-(1-methyl-2-propoxyethoxy)-2-methylpyridin-3-yl]-N-ethyl-N-methylmethanimidamide (1817828-69-5) + SX, 4-(2-bromo-4-fluorophenyl)-N-(2-chloro-6-fluorophenyl)-1,3-dimethyl-1H-pyrazol-5-amine (1362477-26-6) + SX, 2-[6-(3-fluoro-4-methoxyphenyl)-5-methylpyridin-2-yl]quinazoline (1257056-97-5) + SX, ethyl (2Z)-3-amino-2-cyano-3-phenylacrylate (39491-78-6) + SX, N-[(2-chlorothiazol-5-yl)methyl]-N-ethyl-6-methoxy-3-nitropyridin-2-amine (1446247-98-8) + SX, 5-(4-chlorobenzyl)-2-(chloromethyl)-2-methyl-1-(1H-1,2,4-triazol-1-ylmethyl)cyclopentan-1-ol (1394057-11-4) + SX, (1R, 2S, 5S)-5-(4-chlorobenzyl)-2-(chloromethyl)-2-methyl-1-(1H-1,2,4-triazol-1-ylmethyl)cyclopentan-1-ol (1801930-06-2) + SX, (1S, 2R, 5R)-5-(4-chlorobenzyl)-2-(chloromethyl)-2-methyl-1-(1H-1,2,4-triazol-1-ylmethyl)cyclopentan-1-ol (1801930-07-3) + SX, 2-(chloromethyl)-5-(4-fluorobenzyl)-2-methyl-1-(1H-1,2,4-triazol-1-ylmethyl)cyclopentan-1-ol (1394057-13-6) + SX, (1R, 2S, 5S)-2-(chloromethyl)-5-(4-fluorobenzyl)-2-methyl-1-(1H-1,2,4-triazol-1-ylmethyl)cyclopentan-1-ol (1801930-08-4) + SX, (1S, 2R, 5R)-2-(chloromethyl)-5-(4-fluorobenzyl)-2-methyl-1-(1H-1,2,4-triazol-1-ylmethyl)cyclopentan-1-ol (1801930-09-5) + SX, methyl 3-[(4-chlorophenyl)methyl]-2-hydroxy-1-methyl-2-(1H-1,2,4-triazol-1-ylmethyl)cyclopentan-1-carboxylate (1791398-02-1) + SX, 1-(2,4-difluorophenyl)-2-(1H-1,2,4-triazol-1-yl)-1-[1-(4-bromo-2,6-difluorophenoxy)cyclopropyl] ethanol (2019215-86-0) + SX, 1-(2,4-difluorophenyl)-2-(1H-1,2,4-triazol-1-yl)-1-[1-(4-chloro-2,6-difluorophenoxy)cyclopropyl]ethanol (2019215-84-8) + SX, 1-[2-(1-chlorocyclopropyl)-3-(2-fluorophenyl)-2-hydroxypropyl]-1H-imidazole-5-carbonitrile (2018316-13-5) + SX, 1-[2-(1-chlorocyclopropyl)-3-(2,3-difluorophenyl)-2-hydroxypropyl]-1H-imidazole-5-carbonitrile (2018317-25-2) + SX, 2-[6-(4-bromophenoxy)-2-(trifluoromethyl)pyridin-3-yl]-1-(1H-1,2,4-triazol-1-yl)propan-2-ol (2082661-43-4) + SX, 2-[6-(4-chlorophenoxy)-2-(trifluoromethyl)pyridin-3-yl]-1-(1H-1,2,4-triazol-1-yl)propan-2-ol (2082660-27-1) + SX, methyl ({2-methyl-5-[1-(4-methoxy-2-methylphenyl)-1H-pyrazol-3-yl]phenyl}methyl)carbamate (1605879-98-8) + SX, 2-(difluoromethyl)-N-[1,1,3-trimethyl-2,3-dihydro-1H-inden-4-yl]pyridine-3-carboxamide (1616239-21-4) + SX, 2-(difluoromethyl)-N-[3-ethyl-1,1-dimethyl-2,3-dihydro-1H-inden-4-yl]pyridine-3-carboxamide (1847460-02-9) + SX, 2-(difluoromethyl)-N-[3-propyl-1,1-dimethyl-2,3-dihydro-1H-inden-4-yl]pyridine-3-carboxamide (1847460-05-2) + SX, (2E,3Z)-5-{[1-(4-chlorophenyl)-1H-pyrazol-3-yl]oxy}-2-(methoxyimino)-N,3-dimethylpent-3-enamide (1445331-27-0) + SX, (2E,3Z)-5-{[1-(2,4-dichlorophenyl)-1H-pyrazol-3-yl]oxy}-2-(methoxyimino)-N,3-dimethylpent-3-enamide (1445331-54-3) + SX, 5-chloro-4-({2-[6-(4-chlorophenoxy)pyridin-3-yl]ethyl}amino)-6-methylpyrimidine (1605340-92-8) + SX, N-(1-benzyl-1,3-dimethylbutyl)-8-fluoroquinoline-3-carboxamide (2132414-04-9) + SX, N-(1-benzyl-3,3,3-trifluoro-1-methylpropyl)-8-fluoroquinoline-3-carboxamide (2132414-00-5) + SX, 4,4-dimethyl-2-({4-[5-(trifluoromethyl)-1,2,4-oxadiazol-3-yl]phenyl}methyl)isoxazolidin-3-one (2098918-25-1) + SX, 5,5-dimethyl-2-({4-[5-(trifluoromethyl)-1,2,4-oxadiazol-3-yl]phenyl}methyl)isoxazolidin-3-one (2098918-26-2) + SX, N-ethyl-2-methyl-N-({4-[5-(trifluoromethyl)-1,2,4-oxadiazol-3-yl]phenyl}methyl)propanamide + SX, N,2-dimethoxy-N-({4-[5-(trifluoromethyl)-1,2,4-oxadiazol-3-yl]phenyl}methyl)propanamide + SX, N-methoxy-N-({4-[5-(trifluoromethyl)-1,2,4-oxadiazol-3-yl]phenyl}methyl)cyclopropanecarboxamide + SX, N-methoxy-N'-methyl-N-({4-[5-(trifluoromethyl)-1,2,4-oxadiazol-3-yl]phenyl}methyl)urea + SX, N'-ethyl-N-methoxy-N-({4-[5-(trifluoromethyl)-1,2,4-oxadiazol-3-yl]phenyl}methyl)urea + SX, N,N'-dimethoxy-N-({4-[5-(trifluoromethyl)-1,2,4-oxadiazol-3-yl]phenyl}methyl)urea + SX, N-acetyl-2-(ethanesulfonyl)-N-[2-(methoxycarbonyl)-4-(trifluoromethoxy)phenyl]-4-(trifluoromethyl)benzamide (2043675-28-9) + SX, 3-(4-bromo-7-fluoroindol-1-yl)butan-2-yl N-[(3-hydroxy-4-methoxypyridin-2-yl)carbonyl]-L-alaninate + SX, 3-(7-bromoindol-1-yl)butan-2-yl N-[(3-hydroxy-4-methoxypyridin-2-yl)carbonyl]-L-alaninate + SX, 3-(7-bromo-4-fluoroindol-1-yl)butan-2-yl N-[(3-hydroxy-4-methoxypyridin-2-yl)carbonyl]-L-alaninate + SX, 3-(3,5-dichloropyridin-2-yl)butan-2-yl N-[(3-hydroxy-4-methoxypyridin-2-yl)carbonyl]-L-alaninate + SX, 3-(3,5-dichloropyridin-2-yl)butan-2-yl N-{[3-(acetoxymethoxy)-4-methoxypyridin-2-yl]carbonyl}-L-alaninate + SX, (1S)-1-[1-(naphthalen-1-yl)cyclopropyl]ethyl N-[(3-hydroxy-4-methoxypyridin-2-yl)carbonyl]-L-alaninate + SX, (1S)-1-[1-(naphthalen-1-yl)cyclopropyl]ethyl N-[(3-acetoxy-4-methoxypyridin-2-yl)carbonyl]-L-alaninate + SX, (1S)-1-[1-(naphthalen-1-yl)cyclopropyl]ethyl N-{[3-(acetoxymethoxy)-4-methoxypyridin-2-yl]carbonyl}-L-alaninate + SX, Agrobacterium radiobactor strain K1026 + SX, N-({4-[5-(trifluoromethyl)-1,2,4-oxadiazol-3-yl]phenyl}methyl)cyclopropanecarboxamide + SX, N-allyl-N-({4-[5-(trifluoromethyl)-1,2,4-oxadiazol-3-yl]phenyl}methyl)acetamide + SX, N-allyl-N-({4-[5-(trifluoromethyl)-1,2,4-oxadiazol-3-yl]phenyl}methyl)propanamide + SX, N-({4-[5-(trifluoromethyl)-1,2,4-oxadiazol-3-yl]phenyl}methyl)propanamide + SX, 3,3,3-trifluoro-N-({2-fluoro-4-[5-(trifluoromethyl)-1,2,4-oxadiazol-3-yl]phenyl}methyl)propanamide + SX, 3,3,3-trifluoro-N-({3-fluoro-4-[5-(trifluoromethyl)-1,2,4-oxadiazol-3-yl]phenyl}methyl)propanamide + SX, 3,3,3-trifluoro-N-({2,3-difluoro-4-[5-(trifluoromethyl)-1,2,4-oxadiazol-3-yl]phenyl}methyl)propanamide + SX, N-({2,3-difluoro-4-[5-(trifluoromethyl)-1,2,4-oxadiazol-3-yl]phenyl}methyl)butanamide + SX, N-methoxy-N-methyl-N'-({ 4-[5-(trifluoromethyl)-1,2,4-oxadiazol-3-yl]phenyl}methyl)urea + SX, N,N-diethyl-N'-({ 4-[5-(trifluoromethyl)-1,2,4-oxadiazol-3-yl]phenyl}methyl)urea + SX, N-methyl-N'-({ 4-[5-(trifluoromethyl)-1,2,4-oxadiazol-3-yl]phenyl}methyl)urea + SX, 1-({4-[5-(trifluoromethyl)-1,2,4-oxadiazol-3-yl]phenyl}methyl)pyrrolidin-2-one + SX, 1-({4-[5-(trifluoromethyl)-1,2,4-oxadiazol-3-yl]phenyl}methyl)piperidin-2-one + SX, 4-({4-[5-(trifluoromethyl)-1,2,4-oxadiazol-3-yl]phenyl}methyl)morpholin-3-one + SX, 2-({4-[5-(trifluoromethyl)-1,2,4-oxadiazol-3-yl]phenyl}methyl)isoxazolidin-3-one + SX, 3,3-dimethyl-1-({4-[5-(trifluoromethyl)-1,2,4-oxadiazol-3-yl]phenyl}methyl)piperidin-2-one + SX, 2-({4-[5-(trifluoromethyl)-1,2,4-oxadiazol-3-yl]phenyl}methyl)-1,2-oxazinan-3-one + SX, 1-({3-fluoro-4-[5-(trifluoromethyl)-1,2,4-oxadiazol-3-yl]phenyl}methyl)azepan-2-one + SX, 4,4-dimethyl-1-({4-[5-(trifluoromethyl)-1,2,4-oxadiazol-3-yl]phenyl}methyl)pyrrolidin-2-one + SX, 5-methyl-1-({4-[5-(trifluoromethyl)-1,2,4-oxadiazol-3-yl]phenyl}methyl)pyrrolidin-2-one + SX, ethyl 1-({4-[5-(trifluoromethyl)-1,2,4-oxadiazol-3-yl]phenyl}methyl)-1H-pyrazole-4-carboxylate + SX, N-methyl-1-({4-[5-(trifluoromethyl)-1,2,4-oxadiazol-3-yl]phenyl}methyl)-1H-pyrazole-4-carboxamide + SX, N-propyl-1-({4-[5-(trifluoromethyl)-1,2,4-oxadiazol-3-yl]phenyl}methyl)-1H-pyrazole-4-carboxamide + SX, N-methoxy-1-({4-[5-(trifluoromethyl)-1,2,4-oxadiazol-3-yl]phenyl}methyl)-1H-pyrazole-4-carboxamide + SX, N-methoxy-N-methyl-1-({4-[5-(trifluoromethyl)-1,2,4-oxadiazol-3-yl]phenyl}methyl)-1H-pyrazole-4-carboxamide + SX, N,N-dimethyl-1-({4-[5-(trifluoromethyl)-1,2,4-oxadiazol-3-yl]phenyl}methyl)-1H-1,2,4-triazol-3-amine + SX, N-methyl-4-[5-(trifluoromethyl)-1,2,4-oxadiazol-3-yl]benzamide + SX, methyl 2-[2-chloro-4-(4-chlorophenoxy)phenyl]-2-hydroxy-3-(1H-1,2,4-triazol-1-yl)propanoate + SX, ethyl 2-[2-chloro-4-(4-chlorophenoxy)phenyl]-2-hydroxy-3-(1H-1,2,4-triazol-1-yl)propanoate + SX, methyl 2-[2-(trifluoromethyl)-4-(4-chlorophenoxy)phenyl]-2-hydroxy-3-(1H-1,2,4-triazol-1-yl)propanoate + SX, 1-(2,3-dimethylpyridin-5-yl)-4,4-difluoro-3,3-dimethyl-3,4-dihydroisoquinoline + SX, 1-[2-(difluoromethyl)-3-methylpyridin-5-yl]-4,4-difluoro-3,3-dimethyl-3,4-dihydroisoquinoline + SX, 2,2-difluoro-N-[6-({[1-(1-methyl-1H-tetrazol-5-yl)benzimidazol-2-yl]oxy}methyl)pyridin-2-yl]-2-phenoxyacetamide + SX, 1-[2-(1-chlorocyclopropyl)-3-(3-chloro-2-fluorophenyl)-2-hydroxypropyl]-1H-imidazole-5-carbonitrile + SX, ethyl 1-[(4-{[2-(trifluoromethyl)-1,3-dioxolan-2-yl]methoxy}phenyl)methyl]-1H-pyrazole-4-carboxylate + SX, ethyl 1-[(4-{[(1Z)-2-ethoxy-3,3,3-trifluoro-1-propen-1-yl]oxy}phenyl)methyl]-1H-pyrazole-4-carboxylate + SX, 6-chloro-3-(3-cyclopropyl-2-fluorophenoxy)-N-[2-(2,4-dimethylphenyl)-2,2-difluoroethyl]-5-methylpyridazine-4-carboxamide + SX, 6-chloro-3-(3-cyclopropyl-2-fluorophenoxy)-N-[2-(3,4-dimethylphenyl)-2,2-difluoroethyl]-5-methylpyridazine-4-carboxamide + SX, 6-chloro-N-[2-(2-chloro-4-methylphenyl)-2,2-difluoroethyl]-3-(3-cyclopropyl-2-fluorophenoxy)-5-methylpyridazine-4-carboxamide + SX, 2-[cyano(2,6-difluoropyridin-4-yl)amino]-N-(2,2-dimethylcyclobutyl)-5-methylthiazole-4-carboxamide + SX, 2-[cyano(2,6-difluoropyridin-4-yl)amino]-N-(spiro[3.4]octan-1-yl)-5-methylthiazole-4-carboxamide + SX, 2-[cyano(2,6-difluoropyridin-4-yl)amino]-N-hexyl-5-methylthiazole-4-carboxamide + SX, 2-[acetyl(2,6-difluoropyridin-4-yl)amino]-N-(2,2-dimethylcyclobutyl)-5-methylthiazole-4-carboxamide + SX, 2-[(2-methoxyacetyl)(2,6-difluoropyridin-4-yl)amino]-N-(2,2-dimethylcyclobutyl)-5-methylthiazole-4-carboxamide + SX, 2-[(2-methylpropanoyl)(2,6-difluoropyridin-4-yl)amino]-N-(2,2-dimethylcyclobutyl)-5-methylthiazole-4-carboxamide + SX, 5-[5-(difluoromethyl)-1,3,4-oxadiazol-2-yl]-N-[1-(2-fluorophenyl)ethyl]pyrimidin-2-amine + SX, 5-[5-(difluoromethyl)-1,3,4-oxadiazol-2-yl]-N-[1-(2,6-difluorophenyl)ethyl]pyrimidin-2-amine + SX, 5-[5-(difluoromethyl)-1,3,4-oxadiazol-2-yl]-N-[1-(3,5-difluorophenyl)ethyl]pyrimidin-2-amine + SX, 5-[5-(difluoromethyl)-1,3,4-oxadiazol-2-yl]-N-[1-(6-chloropyridin-3-yl)ethyl] pyrimidin-2-amine + SX, 5-[5-(difluoromethyl)-1,3,4-oxadiazol-2-yl]-N-[1-(2-fluorophenyl)cyclopropyl]pyrimidin-2-amine + SX, 5-[5-(difluoromethyl)-1,3,4-oxadiazol-2-yl]-N-[1-(2,6-difluorophenyl)cyclopropyl]pyrimidin-2-amine + SX, 5-[5-(difluoromethyl)-1,3,4-oxadiazol-2-yl]-N-[1-(2-fluoro-3-methoxyphenyl)cyclopropyl]pyrimidin-2-amine + SX, 5-[5-(difluoromethyl)-1,3,4-oxadiazol-2-yl]-2-{[1-(2,6-difluorophenyl)cyclopropyl]oxy}pyrimidine + SX, 3-[3-(3-cyclopropyl-2-fluorophenoxy)-6-methylpyridazin-4-yl]-5-[(2,4-dimethylphenyl)methyl]-5,6-dihydro-4H-1,2,4-oxadiazine + SX, (5S)-3-[3-(3-cyclopropyl-2-fluorophenoxy)-6-methylpyridazin-4-yl]-5-[(2,4-dimethylphenyl)methyl]-5,6-dihydro-4H-1,2,4-oxadiazine + SX, 3-[3-(3-chloro-2-fluorophenoxy)-6-methylpyridazin-4-yl]-5-[(4-bromo-2-methylphenyl)methyl]-5,6-dihydro-4H-1,2,4-oxadiazine + SX, 3-[3-(3-chloro-2-fluorophenoxy)-6-methylpyridazin-4-yl]-5-[(2-chloro-4-methylphenyl) methyl]-5,6-dihydro-4H-1,2,4-oxadiazine + SX, (5S)-3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, (5R)-3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, Agrobacterium radiobactor strain K84 + SX, Bacillus amyloliquefaciens strain PTA-4838 (Aveo(registered trademark) EZ Nematicide) + SX, Bacillus amyloliquefaciens strain AT332 + SX, Bacillus amyloliquefaciens strain B3 + SX, Bacillus amyloliquefaciens strain D747 + SX, Bacillus amyloliquefaciens strain DB101 + SX, Bacillus amyloliquefaciens strain DB102 + SX, Bacillus amyloliquefaciens strain GB03 + SX, Bacillus amyloliquefaciens strain FZB24 + SX, Bacillus amyloliquefaciens strain FZB42 + SX, Bacillus amyloliquefaciens strain IN937a + SX, Bacillus amyloliquefaciens strain MBI600 + SX, Bacillus amyloliquefaciens strain QST713 + SX, Bacillus amyloliquefaciens isolate strain B246 + SX, Bacillus amyloliquefaciens strain F727 + SX, Bacillus amyloliquefaciens subsp. plantarum strain D747 + SX, Bacillus licheniformis strain HB-2 + SX, Bacillus licheniformis strain SB3086 + SX, Bacillus pumilus strain AQ717 + SX, Bacillus pumilus strain BUF-33 + SX, Bacillus pumilus strain GB34 + SX, Bacillus pumilus strain QST2808 + SX, Bacillus simplex strain CGF2856 + SX, Bacillus subtilis strain AQ153 + SX, Bacillus subtilis strain AQ743 + SX, Bacillus subtilis strain BU1814 + SX, Bacillus subtilis strain D747 + SX, Bacillus subtilis strain DB101 + SX, Bacillus subtilis strain FZB24 + SX, Bacillus subtilis strain GB03 + SX, Bacillus subtilis strain HAI0404 + SX, Bacillus subtilis strain IAB/BS03 + SX, Bacillus subtilis strain MBI600 + SX, Bacillus subtilis strain QST30002/AQ30002 + SX, Bacillus subtilis strain QST30004/AQ30004 + SX, Bacillus subtilis strain QST713 + SX, Bacillus subtilis strain QST714 + SX, Bacillus subtilis var. Amyloliquefaciens strain FZB24 + SX, Bacillus subtilis strain Y1336 + SX, Burkholderia cepacia + SX, Burkholderia cepacia type Wisconsin strain J82 + SX, Burkholderia cepacia type Wisconsin strain M54 + SX, Candida oleophila strain O + SX, Candida saitoana + SX, Chaetomium cupreum + SX, Clonostachys rosea + SX, Coniothyrium minitans strain CGMCC8325 + SX, Coniothyrium minitans strain CON/M/91-8 + SX, cryptococcus albidus + SX, Erwinia carotovora subsp. carotovora strain CGE234M403 + SX, Fusarium oxysporum strain Fo47 + SX, Gliocladium catenulatum strain J1446 + SX, Paenibacillus polymyxa strain AC-1 + SX, Paenibacillus polymyxa strain BS-0105 + SX, Pantoea agglomerans strain E325 + SX, Phlebiopsis gigantea strain VRA1992 + SX, Pseudomonas aureofaciens strain TX-1 + SX, Pseudomonas chlororaphis strain 63-28 + SX, Pseudomonas chlororaphis strain AFS009 + SX, Pseudomonas chlororaphis strain MA342 + SX, Pseudomonas fluorescens strain 1629RS + SX, Pseudomonas fluorescens strain A506 + SX, Pseudomonas fluorescens strain CL145A + SX, Pseudomonas fluorescens strain G7090 + SX, Pseudomonas sp. strain CAB-02 + SX, Pseudomonas syringae strain 742RS + SX, Pseudomonas syringae strain MA-4 + SX, Pseudozyma flocculosa strain PF-A22UL + SX, Pseudomonas rhodesiae strain HAI-0804 + SX, Pythium oligandrum strain DV74 + SX, Pythium oligandrum strain M1 + SX, Streptomyces griseoviridis strain K61 + SX, Streptomyces lydicus strain WYCD108US + SX, Streptomyces lydicus strain WYEC108 + SX, Talaromyces flavus strain SAY-Y-94-01 + SX, Talaromyces flavus strain V117b + SX, Trichoderma asperellum strain ICC012 + SX, Trichoderma asperellum SKT-1 + SX, Trichoderma asperellum strain T25 + SX, Trichoderma asperellum strain T34 + SX, Trichoderma asperellum strain TV1 + SX, Trichoderma atroviride strain CNCM 1-1237 + SX, Trichoderma atroviride strain LC52 + SX, Trichoderma atroviride strain IMI 206040 + SX, Trichoderma atroviride strain SC1 + SX, Trichoderma atroviride strain SKT-1 + SX, Trichoderma atroviride strain T11 + SX, Trichoderma gamsii strain ICC080 + SX, Trichoderma harzianum strain 21 + SX, Trichoderma harzianum strain DB104 + SX, Trichoderma harzianum strain DSM 14944 + SX, Trichoderma harzianum strain ESALQ-1303 + SX, Trichoderma harzianum strain ESALQ-1306 + SX, Trichoderma harzianum strain IIHR-Th-2 + SX, Trichoderma harzianum strain ITEM908 + SX, Trichoderma harzianum

strain kd + SX, Trichoderma harzianum strain MO1 + SX, Trichoderma harzianum strain SF + SX, Trichoderma harzianum strain T22 + SX, Trichoderma harzianum strain T39 + SX, Trichoderma harzianum strain T78 + SX, Trichoderma harzianum strain TH35 + SX, Trichoderma polysporum strain IMI206039 + SX, trichoderma stromaticum + SX, Trichoderma virens strain G-41 + SX, Trichoderma virens strain GL-21 + SX, Trichoderma viride + SX, Variovorax paradoxus strain CGF4526 + SX, Harpin protein + SX.

Combination of the Present ingredient in the above Group (c) and the Present compound:

1-methylcyclopropene + SX, 1,3-diphenylurea + SX, 2,3,5-triiodobenzoic acid + SX, IAA ((1H-indol-3-yl)acetic acid) + SX, IBA (4-(1H-indol-3-yl)butyric acid) + SX, MCPA (2-(4-chloro-2-methylphenoxy)acetic acid) + SX, MCPB (4-(4-chloro-2-methylphenoxy)butyric acid) + SX, 4-CPA (4-chlorophenoxyacetic acid) + SX, 5-aminolevulinic acid hydrochloride + SX, 6-benzylaminopurine + SX, abscisic acid + SX, AVG (aminoethoxyvinylglycine) + SX, anisiflupurin + SX, ancymidol + SX, butralin + SX, calcium carbonate + SX, calcium chloride + SX, calcium formate + SX, calcium peroxide + SX, calcium polysulfide + SX, calcium sulfate + SX, chlormequat-chloride + SX, chlorpropham + SX, choline chloride + SX, cloprop + SX, cyanamide + SX, cyclanilide + SX, daminozide + SX, decan-1-ol + SX, dichlorprop + SX, dikegulac + SX, dimethipin + SX, diquat + SX, ethephon + SX, ethychlozate + SX, flumetralin + SX, flurprimidol + SX, forchlorfenuron + SX, formononetin + SX, Gibberellin A + SX, Gibberellin A3 + SX, inabenfide + SX, Kinetin + SX, lipochitooligosaccharide SP104 + SX, maleic hydrazide + SX, mefluidide + SX, mepiquat-chloride + SX, oxidized glutathione + SX, paclobutrazol + SX, pendimethalin + SX, prohexadione-calcium + SX, prohydrojasmon + SX, pyraflufen-ethyl + SX, sintofen + SX, sodium 1-naphthaleneacetate + SX, sodium cyanate + SX, thidiazuron + SX, triapenthenol + SX, tribufos + SX, trinexapac-ethyl + SX, uniconazole-P + SX, 2-(naphthalen-1-yl)acetamide + SX, [4-oxo-4-(2-phenylethyl)amino]butylate + SX, methyl 5-(trifluoromethyl)benzo[b]thiophene-2-carboxylate + SX, 3-[(6-chloro-4-phenylquinazolin-2-yl)amino]propan-1-ol + SX, Claroideoglomus etunicatum + SX, Claroideoglomus claroideum + SX, Funneliformis mosseae + SX, Gigaspora margarita + SX, Gigaspora rosea + SX, Glomus aggregatum + SX, Glomus deserticola + SX, Glomus monosporum + SX, Paraglomus brasillianum + SX, Rhizophagus clarus + SX, Rhizophagus intraradices RTI-801 + SX, Rhizophagus irregularis DAOM 197198 + SX, Azorhizobium caulinodans + SX, Azospirillum amazonense + SX, Azospirillum brasilense XOH + SX, Azospirillum brasilense Ab-V5 + SX, Azospirillum brasilense Ab-V6 + SX, Azospirillum caulinodans + SX, Azospirillum halopraeferens + SX, Azospirillum irakense + SX, Azospirillum lipoferum + SX, Bradyrhizobium elkanii SEMIA 587 + SX, Bradyrhizobium elkanii SEMIA 5019 + SX, Bradyrhizobium japonicum TA-11 + SX, Bradyrhizobium japonicum USDA 110 + SX, Bradyrhizobium liaoningense + SX, Bradyrhizobium lupini + SX, Delftia acidovorans RAY209 + SX, Mesorhizobium ciceri + SX, Mesorhizobium huakii + SX, Mesorhizobium loti + SX, Rhizobium etli + SX, Rhizobium galegae + SX, Rhizobium leguminosarum bv. Phaseoli + SX, Rhizobium leguminosarum bv. Trifolii + SX, Rhizobium leguminosarum bv. Viciae + SX, Rhizobium trifolii + SX, Rhizobium tropici + SX, Sinorhizobium fredii + SX, Sinorhizobium meliloti + SX, Zucchini Yellow Mosaik Virus weak strain + SX.

Combination of the Present ingredient in the above Group (d) and the Present compound:

anthraquinone + SX, deet + SX, icaridin + SX.

The ratio of the Present compound to the Present ingredient includes, but not limited thereto, as a ratio by weight (the Present compound : the Present ingredient) 1,000:1 to 1:1,000, 500:1 to 1:500, 100:1 to 1:100, 50:1, 20:1, 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, 1:10, 1:20, and 1:50, and the others.

The Present compound has an efficacy against pests. Examples of the pests include plant phytopathogenic microorganism, harmful arthropods such as harmful insects and harmful mites, harmful nematicides, and harmful mollusks.

Hemiptera:

from the family Delphacidae, for example, small brown planthopper (Laodelphax striatellus), brown planthopper (Nilaparvata lugens), white-backed planthopper (Sogatella furcifera), corn planthopper (Peregrinus maidis), cereal leafhopper ( Javesella pellucida), sugarcane leafhopper (Perkinsiella saccharicida), and Tagosodes orizicolus;

from the family Cicadellidae, for example,

green rice leafhopper (Nephotettix cincticeps), green paddy leafhopper (Nephotettix virescens), rice leafhopper (Nephotettix nigropictus), zigzag-striped leafhopper (Recilia dorsalis), tea green leafhopper (Empoasca onukii), potato leafhopper (Empoasca fabae), corn leafhopper (Dalbulus maidis), rice leafhopper (Cofana spectra), and Amrasca biguttula biguttula;

from the family Aphrophoridae, for example, European spittlebug (Philaenus spumarius);

from the family Cercopidae, for example, Mahanarva posticata and Mahanarva fimbriolata;

from the family Aphididae, for example,

bean aphid (Aphis fabae), soybean aphid (Aphis glycines), cotton aphid (Aphis gossypii), green apple aphid (Aphis pomi), apple aphid (Aphis spiraecola), green peach aphid (Myzus persicae), leaf-curling plum aphid (Brachycaudus helichrysi), cabbage aphid (Brevicoryne brassicae), rosy apple aphid(Dysaphis plantaginea),

false cabbage aphid (Lipaphis erysimi), potato aphid (Macrosiphum euphorbiae), foxglove aphid (Aulacorthum solani), lettuce aphid (Nasonovia ribisnigri), grain aphid (Rhopalosiphum padi), corn aphid (Rhopalosiphum maidis), brown citrus aphid (Toxoptera citricida), mealy plum aphid (Hyalopterus pruni), cane aphid (Melanaphis sacchari), black rice root aphid (Tetraneura nigriabdominalis), sugarcane cottony aphid (Ceratovacuna lanigera), apple woolly aphid (Eriosoma lanigerum), and English grain aphid (Sitobion avenae);
from the family Phylloxeridae, for example,
grapevine phylloxera (Daktulosphaira vitifoliae), Pecan phylloxera (Phylloxera devastatrix), Pecan leaf phylloxera (Phylloxera notabilis), and Southern pecan leaf phylloxera (Phylloxera russelae);
from the family Adelgidae, for example, hemlock woolly aphid (Adelges tsugae), balsam woolly aphid (Adelges piceae), and Aphrastasia pectinatae;
from the family Pentatomidae, for example, black rice bug (Scotinophara lurida), black paddy bug (Scotinophara coarctata), common green stink bug (Nezara antennata), white-spotted spined bug (Eysarcoris aeneus), lewis spined bug (Eysarcoris lewisi), white-spotted bug (Eysarcoris ventralis), Eysarcoris annamita, brown marmorated stink bug (Halyomorpha halys), green plant bug (Nezara viridula), brown stink bug(Euschistus heros), red banded stink bug(Piezodorus guildinii), Oebalus pugnax, and Dichelops melacanthus;
from the family Cydnidae, for example, Scaptocoris castanea;
from the family Alydidae, for example, bean bug (Riptortus clavatus), corbett rice bug (Leptocorisa chinensis), and rice bug (Leptocorisa acuta);
from the family Coreidae, for example, Cletus punctiger and Australian leaf-footed bug (Leptoglossus australis);
from the family Lygaeidae, for example, oriental chinch bug (Cavelerius saccharivorus), seed bug (Togo hemipterus), and chinch bug (Blissus leucopterus);
from the family Miridae, for example, rice leaf bug (Trigonotylus caelestialium), sorghum plant bug (Stenotus rubrovittatus), wheat leaf bug (Stenodema calcarata), and American tarnished plant bug (Lygus lineolaris);
from the family Aleyrodidae, for example, greenhouse whitefly (Trialeurodes vaporariorum), tobacco whitefly (Bemisia tabaci), citrus whitefly (Dialeurodes citri), citrus spiny whitefly (Aleurocanthus spiniferus), tea spiny whitefly (Aleurocanthus camelliae), and Pealius euryae;
from the family Diaspididae, for example, Abgrallaspis cyanophylli, red scale (Aonidiella aurantii), San José scale (Diaspidiotus perniciosus), white peach scale (Pseudaulacaspis pentagona), arrowhead scale (Unaspis yanonensis), and citrus snow scale (Unaspis citri);
from the family Coccidae, for example, pink wax scale (Ceroplastes rubens);
from the family Margarodidae, for example, fluted scale (Icerya purchasi) and seychelles fluted scale (Icerya seychellarum) ;
from the family Pseudococcidae, for example, solanum mealybug (Phenacoccus solani), cotton mealybug (Phenacoccus solenopsis), Japanese mealybug (Planococcus kraunhiae), white peach scale (Pseudococcus comstocki), citrus mealybug (Planococcus citri), currant mealybug (Pseudococcus calceolariae), long-tailed mealybug (Pseudococcus longispinus), and tuttle mealybug (Brevennia rehi);
from the family Psyllidae, for example, citrus psylla (Diaphorina citri), two-spotted citrus psyllid (Trioza erytreae), pear sucker (Cacopsylla pyrisuga), Cacopsylla chinensis, potato psyllid (Bactericera cockerelli), and Pear psylla (Cacopsylla pyricola);
from the family Tingidae, for example, sycamore lace bug (Corythucha ciliata), aster tingid (Corythucha marmorata), Japanese pear lace bug (Stephanitis nashi), and azalea lace bug (Stephanitis pyrioides);
from the family Cimicidae, for example, common bed bug (Cimex lectularius) and tropical bed bug (Cimex hemipterus);
from the family Cicadidae, for example, Quesada gigas;
from the family Reduviidae, for example, Triatoma infestans, large kissing bug (Triatoma rubrofasciata), Triatoma dimidiata, and Rhodonius prolixus;
and the others.

Lepidoptera:

from the family Crambidae, for example, rice stem borer (Chilo suppressalis), Dark-headed stem borer (Chilo polychrysus), white stem borer (Scirpophaga innotata), yellow paddy borer (Scirpophaga incertulas), Rupela albina, rice leaf roller (Cnaphalocrocis medinalis), Marasmia patnalis, rice leaf roller (Marasmia exigua), cotton leaf roller (Notarcha derogata), corn borer (Ostrinia furnacalis), European corn borer (Ostrinia nubilalis), cabbage webworm (Hellula undalis), grape leafroller (Herpetogramma luctuosale), bluegrass webworm (Parapediasia teterrellus), rice case-worm (Nymphula depunctalis), Sugarcane borer (Diatraea saccharalis), and eggplant fruit borer (Leucinodes orbonalis);
from the family Pyralidae, for example, lesser cornstalk borer (Elasmopalpus lignosellus), mealworm moth

(Plodia interpunctella), persimmon bark borer (Euzophera batangensis), and fig moth (Cadra cautella);
from the family Noctuidae, for example, cotton worm (Spodoptera litura), beet armyworm (Spodoptera exigua), rice armyworm (Mythimna separata), cabbage moth (Mamestra brassicae), pink borer (Sesamia inferens), grass armyworm (Spodoptera mauritia), green rice caterpillar (Naranga aenescens), Spodoptera frugiperda, true armyworm (Spodoptera exempta), semitropical armyworm (Spodoptera eridania), black cutworm (Agrotis ipsilon), beet worm (Autographa nigrisigna), rice looper (Plusia festucae), Soybean looper (Chrysodeixis includens), Trichoplusia spp., Heliothis spp.(such as tobacco budworm (Heliothis virescens)), Helicoverpa spp. (such as tobacco budworm (Helicoverpa armigera), corn earworm (Helicoverpa zea)), velvet-bean caterpillar (Anticarsia gemmatalis), cotton leafworm (Alabama argillacea), and hop vine borer (Hydraecia immanis);
from the family Pieridae, for example, common cabbage worm (Pieris rapae);
from the family Tortricidae, for example, oriental fruit moth (Grapholita molesta), Grapholita dimorpha, soybean moth (Leguminivora glycinivorella), Matsumuraeses azukivora, summer fruit tortrix (Adoxophyes orana fasciata), smaller tea tortrix (Adoxophyes honmai), Japanese tea tortrix (Homona magnanima), apple tortrix (Archips fuscocupreanus), codling moth (Cydia pomonella), sugarcane shoot borer (Tetramoera schistaceana), bean shoot borer (Epinotia aporema), citrus fruit borer (Citripestis sagittiferella), and European grapevine moth (Lobesia botrana);
from the family Gracillariidae, for example, tea leaf roller (Caloptilia theivora) and Asiatic apple leaf miner (Phyllonorycter ringoniella);
from the family Carposinidae, for example, peach fruit moth (Carposina sasakii);
from the family Lyonetiidae, for example, coffee leaf miner (Leucoptera coffeella), peach leaf miner (Lyonetia clerkella), and Lyonetia prunifoliella;
from the family Lymantriidae, for example, Lymantria spp. (such as gypsy moth (Lymantria dispar)) and Euproctis spp. (such as tea lymantriid (Euproctis pseudoconspersa));
from the family Plutellidae, for example, diamondback moth (Plutella xylostella);
from the family Gelechiidae, for example, peach worm (Anarsia lineatella), sweetpotato leaf folder (Helcystogramma triannulella), pink bollworm (Pectinophora gossypiella), potato moth (Phthorimaea operculella), and Tuta absoluta;
from the family Arctiidae, for example, American white moth (Hyphantria cunea);
from the family Castniidae, for example, giant sugarcane borer (Telchin licus);
from the family Cossidae, for example, Cossus insularis;
from the family Geometridae, for example, Ascotis selenaria;
from the family Limacodidae, for example, blue-striped nettle grub (Parasa lepida) ;
from the family Stathmopodidae, for example, persimmon fruit moth (Stathmopoda masinissa);
from the family Sphingidae, for example, tobacco hornworm (Acherontia lachesis);
from the family Sesiidae, for example, Nokona feralis, cherry borer (Synanthedon hector), and Synanthedon tenuis: from the family Hesperiidae, for example, rice skipper (Parnara guttata);
from the family Tineidae, for example, casemaking clothes moth (Tinea translucens), common clothes moth (Tineola bisselliella);
and the others.

Thysanoptera:

from the family Thripidae, for example, western flower thrips (Frankliniella occidentalis), oriental thrips (Thrips palmi), yellow tea thrips (Scirtothrips dorsalis), onion thrips (Thrips tabaci), eastern flower thrips (Frankliniella intonsa), rice thrips (Stenchaetothrips biformis), Echinothrips americanus, and avocado thrips (Scirtothrips perseae);
from the family Phlaeothripidae, for example, aculeated rice thrips (Haplothrips aculeatus);
and the others.

Diptera:

from the family Anthomyiidae, for example, seedcorn maggot (Delia platura), onion maggot (Delia antiqua), and beet leaf miner (Pegomya cunicularia);
from the family Ulidiidae, for example, sugarbeet root maggot (Tetanops myopaeformis);
from the family Agromyzidae, for example, rice leaf miner (Agromyza oryzae), tomato leaf miner (Liriomyza sativae), chrysanthemum leaf miner (Liriomyza trifolii), and pea leafminer (Chromatomyia horticola);
from the family Chloropidae, for example, rice stem maggot (Chlorops oryzae);
from the family Tephritidae, for example, melon fly (Bactrocera cucurbitae), oriental fruit fly (Bactrocera dorsalis),

Malaysian fruit fly (Bactrocera latifrons), olive fruit fly (Bactrocera oleae), Queensland fruit fly (Bactrocera tryoni), Mediterranean fruit fly (Ceratitis capitata), apple maggot (Rhagoletis pomonella), and Japanese cherry fruit fly (Rhacochlaena japonica);
from the family Ephydridae, for example, smaller rice leaf miner (Hydrellia griseola), whorl maggot (Hydrellia philippina), and paddy stem maggot (Hydrellia sasakii);
from the family Drosophilidae, for example, cherry drosophila (Drosophila suzukii), and common fruit fly (Drosophila melanogaster);
from the family Phoridae, for example, Megaselia spiracularis;
from the family Psychodidae, for example, Clogmia albipunctata;
from the family Sciaridae, for example, Bradysia difformis;
from the family Cecidomyiidae, for example, hessian fly (Mayetiola destructor) and paddy gall fly (Orseolia oryzae);
from the family Diopsidae, for example, Diopsis macrophthalma;
from the family Glossinidae, for example, Glossina palpalis and Glossina morsitans;
from the family Simuliidae, for example, Simulium japonicum and Simulium damnosum;
from the subfamily Phlebotominae;
from the family Tipulidae, for example, rice crane fly (Tipula aino), common cranefly (Tipula oleracea), and European cranefly (Tipula paludosa);
from the family Culicidae, for example, southern house mosquito (Culex pipiens pallens), Culex tritaeniorhynchus ,Culex pipiens f. molestus, brown house mosquito (Culex quinquefasciatus), northern house mosquito (Culex pipiens pipiens), Culex vishnui, Asian tiger mosquito (Aedes albopictus), dengue mosquito (Aedes aegypti), Chinese malaria mosquito (Anopheles sinensis), Anopheles gambiae, Anopheles stephensi, Anopheles coluzzii, Anopheles albimanus, Anopheles sundaicus, Anopheles arabiensis, Anopheles funestus, Anopheles darlingi, Anopheles farauti, and Anopheles minimus;
from the family Simulidae, for example, Prosimulium yezoensis and Simulium ornatum;
from the family Tabanidae, for example, Tabanus trigonus; from the family Muscidae, for example, house fly (Musca domestica), false stable fly (Muscina stabulans), biting house fly (Stomoxys calcitrans), and buffalo fly (Haematobia irritans);
from the family Calliphoridae;
from the family Sarcophagidae;
from the family Chironomidae, for example, Chironomus plumosus, Chironomus yoshimatsui, and Glyptotendipes tokunagai;
from the family Fannidae;
and the others.

Coleoptera:

from the family Chrysomelidae, for example, Diabrotica spp. (such as western corn rootworm (Diabrotica virgifera virgifera), southern corn rootworm (Diabrotica undecimpunctata howardi), northern corn rootworm (Diabrotica barberi), Mexican corn rootworm (Diabrotica virgifera zeae), banded cucumber beetle (Diabrotica balteata), cucurbit beetle (Diabrotica speciosa)), bean leaf beetle (Cerotoma trifurcata), barley leaf beetle (Oulema melanopus), cucurbit leaf beetle (Aulacophora femoralis), striped flea beetle (Phyllotreta striolata), cabbage flea beetle (Phyllotreta cruciferae), western black flea beetle (Phyllotreta pusilla), cabbage stem flea beetle (Psylliodes chrysocephala), hop flea beetle (Psylliodes punctulata), Colorado potato beetle (Leptinotarsa decemlineata), rice leaf beetle (Oulema oryzae), grape colaspis (Colaspis brunnea), corn flea beetle (Chaetocnema pulicaria), sweet-potato flea beetle (Chaetocnema confinis), potato flea beetle (Epitrix cucumeris), rice leaf beetle (Dicladispa armigera), southern corn leaf beetle (Myochrous denticollis), Laccoptera quadrimaculata, and tobacco flea beetle (Epitrix hirtipennis);
from the family Carabidae, for example, Seedcorn beetle (Stenolophus lecontei) and slender seed-corn ground beetle (Clivina impressifrons);
from the family Scarabaeidae, for example, cupreus chafer (Anomala cuprea), soybean beetle (Anomala rufocuprea), Anomala albopilosa, Japanese beetle (Popillia japonica), yellowish elongate chafer (Heptophylla picea), European Chafer(Rhizotrogus majalis), Tomarus gibbosus, Holotrichia spp., Phyllophaga spp. (such as June beetle (Phyllophaga crinita)), and Diloboderus spp. (such as Diloboderus abderus);
from the family Anthriibidae, for example, coffee bean weevil (Araecerus coffeae);
from the family Aponidae, for example, sweet-potato weevil (Cylas formicarius);
from the family Bruchidae, for example, Mexican bean weevil (Zabrotes subfasciatus);
from the family Scolytidae, for example, pine beetle (Tomicus piniperda) and coffee berry borer (Hypothenemus

hampei) ;
from the family Curculionidae, for example, West Indian sweet-potato weevil (Euscepes postfasciatus), alfalfa weevil (Hypera postica), maize wevil (Sitophilus zeamais), rice weevil (Sitophilus oryzae), grain weevil (Sitophilus granarius), rice plant weevil (Echinocnemus squameus), rice water weevil (Lissorhoptrus oryzophilus), Rhabdoscelus lineaticollis, boll weevil (Anthonomus grandis), nunting billbug (Sphenophorus venatus), southern corn billbug (Sphenophorus callosus), soybean stalk weevil (Sternechus subsignatus), sugarcane weevil (Sphenophorus levis), rusty gourd-shaped weevil (Scepticus griseus), brown gourd-shaped weevil (Scepticus uniformis), Aracanthus spp. (such as Aracanthus mourei), and cotton root borer (Eutinobothrus brasiliensis);
from the family Tenebrionidae, for example, red meal beetle (Tribolium castaneum), mason beetle (Tribolium confusum), and lesser mealworm (Alphitobius diaperinus);
from the family Coccinellidae, for example, twenty-eight-spotted ladybird (Epilachna vigintioctopunctata);
from the family Bostrychidae, for example, common powder-post beetle (Lyctus brunneus), and lesser grain borer (Rhizopertha dominica);
from the family Ptinidae;
from the family Cerambycidae, for example, citrus long-horned beetle (Anoplophora malasiaca), Migdolus fryanus, and peach borer (Aromia bungii);
from the family Elateridae, for example, Melanotus okinawensis, barley wireworm (Agriotes fuscicollis), Melanotus legatus, Anchastus spp., Conoderus spp., Ctenicera spp., Limonius spp., and Aeolus spp.;
from the family Staphylinidae, for example, Paederus fuscipes;
from the family Dermestidae, for example, varied carpet beetle (Anthrenus verbasci), hide beetle (Dermestes maculates), and khapra beetle (Trogoderma granarium);
from the family Anobiidae, for example, tobacco beetle (Lasioderma serricorne), and biscuit beetle (Stegobium paniceum) ;
from the family Laemophloeidae, for example, flat grain beetle (Cryptolestes ferrugineus);
from the family Silvanidae, for example, saw-toothed grain beetle (Oryzaephilus surinamensis);
from the family Nitidulidae, for example, blossom beetle (Brassicogethes aeneus);
and the others.

Orthoptera:

from the family Acrididae, for example, oriental migratory locust (Locusta migratoria), Moroccan locust (Dociostaurus maroccanus), Australian plague locust (Chortoicetes terminifera), red locust (Nomadacris septemfasciata), brown locust(Locustana pardalina), tree locust (Anacridium melanorhodon), Italian locust (Calliptamus italicus), differential grasshopper (Melanoplus differentialis), two-striped grasshopper (Melanoplus bivittatus), migratory grasshopper (Melanoplus sanguinipes), red-legged grasshopper (Melanoplus femurrubrum), clear-winged grasshopper (Camnula pellucida), desert locust (Schistocerca gregaria), Yellow-winged locust(Gastrimargus musicus), spur-throated locust (Austracris guttulosa), Japanese grasshopper (Oxya yezoensis), rice grasshopper (Oxya japonica), and Bombay locust (Patanga succincta);
from the family Gryllotalpidae, for example, oriental mole cricket (Gryllotalpa orientalis);
from the family Gryllidae, for example, house cricket (Acheta domestica), and emma field cricket (Teleogryllus emma) ;
from the family Tettigoniidae, for example, mormon cricket (Anabrus simplex);
and the others.

Hymenoptera:

from the family Tenthredinidae, for example, beet sawfly (Athalia rosae) and nippon cabbage sawfly (Athalia japonica);
from the family Formicidae, for example, Solenopsis spp. (such as red imported fire ant (Solenopsis invicta) , tropical fire ant (Solenopsis geminata)), Atta spp. (such as brown leaf-cutting ant (Atta capiguara), Acromyrmex spp., Paraponera clavata, black house ant (Ochetellus glaber), little red ant (Monomorium pharaonis), Argentine ant (Linepithema humile), Formica japonica, Pristomyrmex punctutus, Pheidole noda, big-headed ant (Pheidole megacephala), Camponotus spp. (such as Camponotus japonicus, Camponotus obscuripes), Pogonomyrmex spp. (such as western harvester ant (Pogonomyrmex occidentalis)), Wasmania spp. (such as Wasmania auropunctata), and long-legged ant (Anoplolepis gracilipes);
from the family Vespidae, for example, Asian giant hornet (Vespa mandarinia, Vespa simillima, Vespa analis, Asian hornet(Vespa velutina), and Polistes jokahamae;
from the family Siricidae, for example, pine wood wasp (Urocerus gigas);

from the family Bethylidae;
and the others.

Blattodea :

from the family Ectobiidae, for example, German cockroach (Blattella germanica);
from the family Blattidae, for example, smoky-brown cockroach (Periplaneta fuliginosa), American cockroach (Periplaneta americana), Australian cockroach (Periplaneta australasiae), brown cockroach (Periplaneta brunnea), and black cockroach (Blatta orientalis);
from the family Termitidae, for example, Japanese termite (Reticulitermes speratus), Formosan termite (Coptotermes formosanus), western drywood termite (Incisitermes minor), Cryptotermes domesticus, Odontotermes formosanus, Neotermes koshunensis, Glyptotermes satsumensis, Glyptotermes nakajimai, Glyptotermes fuscus, Hodotermopsis sjostedti, Coptotermes guangzhouensis, Reticulitermes amamianus, Reticulitermes miyatakei, Reticulitermes kanmonensis, Nasutitermes takasagoensis, Pericapritermes nitobei, Sinocapritermes mushae, and Cornitermes cumulans;
and the others.

Siphonaptera:

from the family Pulicidae, for example, human flea (Pulex irritans), cat flea (Ctenocephalides felis), dog flea (Ctenocephalides canis), oriental rat flea (Xenopsylla cheopis), and chicken flea (Echidnophaga gallinacea);
from the family Pulicidae, for example, chigoe flea (Tunga penetrans);
from the family Ceratophyllidae, for example, European rat flea (Nosopsyllus fasciatus); and the others.

Psocodae:

from the family Pediculidae, for example, head louse (Pediculus humanus capitis);
from the family Pthiridae, for example, crab louse (Pthirus pubis);
from the family Haematopinidae, for example, short-nosed cattle louse (Haematopinus eurysternus) and pig louse (Haematopinus suis);
from the family Linognathidae, for example, blue cattle louse (Linognathus vituli), sheep face louse (Linognathus ovillus), and capillate louse (Solenopotes capillatus);
from the family Bovicoliidae, for example, cattle biting louse (Bovicola bovis), sheep biting louse (Bovicola ovis), Bovicola breviceps, Damalinia forficula, and Werneckiella spp.;
from the family Trichodectidae, for example, dog biting louse (Trichodectes canis) and cat louse (Felicola subrostratus);
from the family Menoponidae, for example, common chicken louse (Menopon gallinae), chicken body louse (Menacanthus stramineus), and Trinoton spp;
from the family Trimenoponidae, for example, Cummingsia spp.;
from the family Trogiidae, for example, death watch (Trogium pulsatorium);
from the family Liposcelidae or Liposcelididae, for example, book louse (Liposcelis corrodens), Liposcelis bostrychophila, Liposcelis pearmani and Liposcelis entomophila;
and the other.

Thysanura:

from the family Lepismatidae, for example, oriental silverfish (Ctenolepisma villosa) andmoth fish (Lepisma saccharina);
and the others.

Acari:

from the family Tetranychidae, for example, common red spider mite (Tetranychus urticae), kanzawa spider mite (Tetranychus kanzawai), red spider mite (Tetranychus evansi), citrus red mite (Panonychus citri), fruit-tree red spider mite (Panonychus ulmi), and Oligonychus spp.;
from the family Eriophyidae, for example, Japanese citrus rust mite (Aculops pelekassi), Phyllocoptruta citri, tomato mite (Aculops lycopersici), purple mite (Calacarus carinatus), tea rust mite (Acaphylla theavagrans), Eriophyes chibaensis, apple bud mite (Aculus schlechtendali), Aceria diospyri, Aceria tosichella, and Shevtchenkella sp.;
from the family Tarsonemidae, for example, broad mite (Polyphagotarsonemus latus);
from the family Tenuipalpidae, for example, Brevipalpus phoenicis;
from the family Tuckerellidae;
from the family Ixodidae, for example, Haemaphysalis longicornis, Haemaphysalis flava, Haemaphysalis japonica, Haemaphysalis campanulata, American dog tick (Dermacentor variabilis), Dermacentor taiwanensis, Rocky Mountain wood tick (Dermacentor andersoni), netted tick (Dermacentor reticulatus), Ixodes ovatus, Ixodes persulcatus, black-legged tick (Ixodes scapularis), Ixodes pacificus, Ixodes holocyclus, Ixodes ricinus, lone star tick (Amblyomma americanum), gulf coast tick (Amblyomma maculatum), Rhipicephalus microplus, cattle tick (Rhipicephalus annulatus), brown dog tick (Rhipicephalus sanguineus), Rhipicephalus appendiculatus, and Rhipicephalus decoloratus; from the family Argasidae, for example, fowl tick (Argas persicus), Ornithodoros hermsi, and Ornithodoros turicata;
from the family Acaridae, for example, cereal mite (Tyrophagus putrescentiae) and grassland mite (Tyrophagus similis);
from the family Pyroglyphidae, for example, American house dust mite (Dermatophagoides farinae) and European house dust mite (Dermatophagoides pteronyssinus);
from the family Cheyletidae, for example, Cheyletus eruditus, Cheyletus malaccensis, Chelacaropsis moorei, and Cheyletiella yasguri;
from the family Psoroptidae, for example, sheep scab mite (Psoroptes ovis), horse psoroptic mange mite (Psoroptes equi), Knemidocoptes mutans, ear mange mite (Otodectes cynotis), and Chorioptes spp.;
from the family Sarcoptidae, for example, Notoedres cati, Notoedres muris, and itch mite (Sarcoptes scabiei);
from the family Listrophoridae, for example, Listrophorus gibbus;
from the family Dermanyssidae, for example,bird mite (Dermanyssus gallinae);
from the family Macronyssidae, for example, feather mite (Ornithonyssus sylviarum) and tropical rat mite (Ornithonyssus bacoti);
from the family Varroidae, for example, Varroa jacobsoni; from the family Demodicidae, for example, dog follicle mite (Demodex canis) and cat follicle mite (Demodex cati);
from the family Trombiculidae, for example, Leptotrombidium akamushi, Leptotrombidium pallidum, and Leptotrombidium scutellare;
and the others.

Araneae:

from the family Eutichuridae, for example, Cheiracanthium japonicum;
from the family Theridiidae, for example, red-back spider (Latrodectus hasseltii);
and the others

Polydesmida:

from the family Paradoxosomatidae, for example, flat-backed millipede (Oxidus gracilis)and Nedyopus tambanus;
and the others.

Isopoda:

from the family Armadillidiidae, common pill bug (Armadillidium vulgare);
and the others.

Chilopoda:

from the family Scutigeridae, for example, Thereuonema hilgendorfi;
from the family Scolopendridae, giant tropical centipede (Scolopendra subspinipes);
from the family Ethopolyidae, Bothropolys rugosus;
and the others.

Gastropoda:

from the family Limacidae, for example, tree slug (Limax marginatus) and garden tawny slug (Limax flavus);

from the family Philomycidae, for example, Meghimatium bilineatum;
from the family Ampullariidae, for example, golden apple snail (Pomacea canaliculata);
from the family Lymnaeidae, for example, Austropeplea ollula;
and the others.

Nematoda:

from the family Aphelenchoididae, for example, rice white-tip nematode (Aphelenchoides besseyi);
from the family Pratylenchidae, for example, root lesion nematode (Pratylenchus coffeae), Pratylenchus brachyurus, California meadow nematode (Pratylenchus neglectus), and Radopholus similis;
from the family Heteroderidae, for example, javanese root-knot nematode (Meloidogyne javanica), southern root-knot nematode (Meloidogyne incognita), guava root-knot nematodes (Meloidogyne enterolobii), northern root-knot nematode (Meloidogyne hapla), soybean cyst nematode (Heterodera glycines), potato cyst nematode (Globodera rostochiensis), and white potato cyst nematode (Globodera pallida);
from the family Hoplolaimidae, for example, Rotylenchulus reniformis;
from the family Anguinidae, for example, strawberry bud nematode (Nothotylenchus acris), and stem nematode (Ditylenchus dipsaci);
from the family Tylenchulidae, for example, citrus nematode (Tylenchulus semipenetrans);
from the family Longidoridae, for example, dagger nematode (Xiphinema index);
from the family Trichodoridae;
from the family Parasitaphelenchidae, for example, pine wilt disease (Bursaphelenchus xylophilus);
and the others.

The harmful arthropods such as harmful insects and harmful mites, harmful mollusks and harmful nematodes may be those having a reduced susceptibility to or a developed resistance to an insecticide, a mitecide, a molluscicide or a nematicide.

The method for controlling harmful arthropods of the present invention is carried out by applying an effective amount of the Present compound or the Composition A to a harmful arthropod directly and/or a habitat thereof (for example, plant bodies, soil, an interior of a house, animal bodies). Examples of the method for controlling harmful arthropods of the present invention include foliage treatment, soil treatment, root treatment, shower treatment, smoking treatment, water surface treatment and seed treatment.

The Present compound or the Composition A is usually used by mixing it with inert carrier(s) such as solid carrier(s), liquid carrier(s), and gaseous carrier(s), surfactant(s), and the like, and as needed, adding thereto auxiliary agent(s) for formulation such as binder(s), dispersant(s), and stabilizer(s) to be formulated into an aqueous suspension formulation, an oily suspension formulation, an oil solution, an emulsifiable concentrate, an emulsion formulation, a microemulsion formulation, a microcapsule formulation, a wettable powder, a granular wettable powder, a dust formulation, a granule, a tablet, an aerosol formulation, a resin formulation, or the like. In addition to these formulations, the Present compound or the Composition A may be used by formulating it into a dosage form described in Manual on development and use of FAO and WHO Specifications for pesticides, FAO Plant Production and Protection Papers- 271-276, prepared by the FAO/WHO Joint Meeting on Pesticide Specifications, 2016, ISSN:0259-2517.

These formulations usually comprise 0.0001 to 99% by weight ratio of the Present compound or the Composition A.

Examples of the solid carrier include fine powders and granules of clays (for example, pyrophyllite clay and kaolin clay), talc, calcium carbonate, diatomaceous earth, zeolite, bentonite, acid white clay, attapulgite, white carbon, ammonium sulfate, vermiculite, perlite, pumice, silica sand, chemical fertilizers (for example, ammonium sulfate, ammonium phosphate, ammonium nitrate, urea, and ammonium chloride), and the others; as well as resins (for example, polyethylene, polypropylene, polyester, polyurethane, polyamide, and polyvinyl chloride).

Examples of the liquid carrier include water, alcohols (for example, ethanol, cyclohexanol, benzyl alcohol, propylene glycol, and polyethylene glycol), ketones (for example, acetone and cyclohexanone), aromatic hydrocarbons (for example, xylene, phenyl xylyl ethane, and methylnaphthalene), aliphatic hydrocarbons (for example, hexane and cyclohexane), esters (for example, ethyl acetate, methyl oleate, and propylene carbonate), nitriles (for example, acetonitrile), ethers (for example, ethylene glycol dimethyl ether), amides (for example, N,N-dimethylformamide and N,N-dimethyloctanamide), sulfoxides (for example, dimethyl sulfoxide), lactams (for example, N-methylpyrrolidone and N-octylpyrrolidone), fatty acids (for example, oleic acid), and vegetable oils (for example, soybean oil).

Examples of the gaseous carrier include fluorocarbon, butane gas, LPG (liquefied petroleum gas), dimethyl ether, nitrogen, and carbon dioxide.

Examples of the surfactant include nonionic surfactants (for example, polyoxyethylene alkyl ethers, polyoxyethylene alkyl aryl ethers, and polyethylene glycol fatty acid esters), and anionic surfactants (for example, alkyl

sulfonates, alkyl aryl sulfonates, and alkyl sulfates).

Examples of the other auxiliary agent for formulation include binders, dispersants, colorants, and stabilizers, and the specific examples thereof include polysaccharides (for example, starch, gum arabic, cellulose derivatives, and alginic acid), lignin derivatives, water-soluble synthetic polymers (for example, polyvinyl alcohol, polyvinyl pyrrolidone, and polyacrylic acids), acidic isopropyl phosphate, and dibutylhydroxytoluene.

Moreover, some adjuvants can be employed to improve or help the efficacy of the Present compound. The specific examples thereof include Nimbus (registered trademark), Assist (registered trademark), Aureo (registered trademark), Iharol (registered trademark), Silwet L-77 (registered trademark), BreakThru (registered trademark), SundanceII (registered trademark), Induce (registered trademark), Penetrator (registered trademark), AgriDex (registered trademark), Lutensol A8 (registered trademark), N092P-7 (registered trademark), Triton (registered trademark), Nufilm (registered trademark), Emulgator NP7 (registered trademark), Emulad (registered trademark), TRITON X 45 (registered trademark), AGRAL 90 (registered trademark), AGROTIN (registered trademark), ARPON (registered trademark), EnSpray N (registered trademark), and BANOLE (registered trademark).

As used herein, examples of the plant include whole plant, stem and leaf, flower, ear, fruit, tree stem, branch, crown, seed, vegetative reproductive organ, and seedling.

A vegetative reproduction organ means a part of plant such as root, stem, and leaf which has a growth capability even when said part is separated from the plant body and placed into soil. Examples of the vegetative reproduction organ include tuberous root, creeping root, bulb, corm or solid bulb, tuber, rhizome, stolon, rhizophore, cane cuttings, propagule, and vine cutting. Stolon is also referred to as "runner", and propagule is also referred to as "propagulum" and categorized into broad bud and bulbil. Vine cutting means a shoot (collective term of leaf and stem) of sweet potato, glutinous yam, or the like. Bulb, corm or solid bulb, tuber, rhizome, cane cuttings, rhizophore, and tuberous root are also collectively referred to as "bulb". For example, cultivation of potato starts with planting a tuber into soil, and the tuber to be used is generally referred to as "seed potato".

Examples of a method for controlling harmful arthropods by applying an effective amount of the Present compound or the Composition A to soils include a method of applying an effective amount of the Present compound or the Composition A to soils before planting plants or after planting plants, a method of applying an effective amount of the Present compound or the Composition A to a root part of a crop to be protected from damage such as ingestion by harmful arthropods, and a method of controlling harmful arthropods that ingest a plant by permeating and transferring an effective amount of the Present compound or the Composition A from a root into the interior of the plant body. Specifically, examples of the application method include planting hole treatment (spraying into planting holes, soil mixing after planting hole treatment), plant foot treatment (plant foot spraying, soil mixing after plant foot treatment, irrigation at plant foot, plant foot treatment at a later seeding raising stage), planting furrow treatment (planting furrow spraying, soil mixing after planting furrow treatment), planting row treatment (planting row spraying, soil mixing after planting row treatment, planting row spraying at a growing stage), planting row treatment at the time of sowing (planting row spraying at the time of sowing, soil mixing after planting row treatment at the time of sowing), broadcast treatment (overall soil surface spraying, soil mixing after broadcast treatment), side-article treatment, treatment of water surface (application to water surface, application to water surface after flooding), other soil spraying treatment (spraying of a granular formulation on leaves at a growing stage, spraying under a canopy or around a tree stem, spraying on the soil surface, mixing with surface soil, spraying into seed holes, spraying on the ground surfaces of furrows, spraying between plants), other irrigation treatment (soil irrigation, irrigation at a seedling raising stage, chemical solution injection treatment, irrigation of a plant part just above the ground, chemical solution drip irrigation, chemigation), seedling raising box treatment (spraying into a seedling raising box, irrigation of a seedling raising box, flooding into a seedling raising box with chemical solution), seedling raising tray treatment (spraying on a seedling raising tray, irrigation of a seedling raising tray, flooding into a seedling raising tray with chemical solution), seedbed treatment (spraying on a seedbed, irrigation of a seedbed, spraying on a lowland rice nursery, immersion of seedlings), seedbed soil incorporation treatment (mixing with seedbed soil, mixing with seedbed soil before sowing, spraying at sowing before covering with soils, spraying at sowing after covering with soils, mixing with covering with soils), and other treatment (mixing with culture soil, plowing under, mixing with surface soil, mixing with soil at the place where raindrops fall from a canopy, treatment at a planting position, spraying of a granule formulation on flower clusters, mixing with a paste fertilizer).

Examples of the application to seeds (or seed treatments) include an application of the Present compound or the Composition A to seeds or vegetative reproductive organs, and specific examples thereof include spraying treatment in which a suspension of the Present compound or the Composition A is sprayed onto seed surface or the vegetative reproductive organ surface in the form of mist; smearing treatment in which the Present compound or the Composition A is coated a surface of seeds or the vegetative reproductive organ; a soaking treatment in which the seeds are soaked into the solution of the Present compound or the Composition A for a certain time; and a method for coating the seeds or the vegetative reproductive organ with a carrier containing the Present compound or the Composition A (film coating treatment, pellet coating treatment). Examples of the above-described vegetative reproductive organ include particularly seed potato.

When the Composition A is applied to seeds or vegetative reproductive organs, the Composition A may be also applied to seeds or vegetative reproductive organs as a single formulation, or the Composition A may be applied to seeds or vegetative reproductive organs as multiple different formulations by multiple times. Examples of the method in which the Composition A is applied as multiple different formulations by multiple times include, for example, a method in which the formulations comprising as an active component the Present compound only are applied, and seeds or vegetative reproductive organs are air dried, followed by applying the formulations comprising the Present ingredient: and a method in which the formulations comprising as an active component the present compound and the Present ingredients are applied, and seeds or vegetative reproductive organs are air dried, followed by applying the formulations comprising the Present ingredients other than the already-applied Present ingredients, are included.

As used herein, seeds or vegetative reproductive organs carrying the present compound or the Composition A means seeds or vegetative reproductive organs in the state where the present compound or the Composition A is adhered to a surface of the seeds or the vegetative reproductive organ. The above-described seeds or vegetative reproductive organs carrying the present compound or the Composition A may be adhered by any other materials that are different from the present compound or the Composition A before or after being adhered the present compound or the Composition A to the seeds or vegetative reproductive organs.

Also, when the Composition A is adhered in a form of layer(s) to a surface of seeds or vegetative reproductive organ, the layer(s) is/are composed of one layer or a multiple layers. Also, when multiple layers are formed, each of the layer may be composed of a layer comprising one or more active ingredients, or a combination of a layer comprising one or more active ingredients and a layer not comprising an active ingredient.

Seeds or vegetative reproductive organs carrying the present compound or the Composition A can be obtained, for example, by applying the formulations comprising the present compound or the Composition A by the above-described application method to seeds to seeds or vegetative reproductive organs.

When the Present compound or the Composition A is applied for harmful arthropods control in agricultural fields, the application dose thereof is usually within a range of 1 to 10,000 g of the Present compound per 10,000 $m^2$. In the case of being applied to seeds or vegetative reproductive organs, the dose of application dose thereof is usually within a range of 0.001 to 100 g of the Present compound per 1 Kg of seeds. When the Present compound or the Composition A is formulated into an emulsifiable concentrate, a wettable powder or a flowable etc., they are usually applied by diluting them with water so as to make an effective concentration of the active ingredients 0.01 to 10,000 ppm, and the dust formulation or the granular formulation, etc., is usually applied as itself without diluting them.

Also, the resin preparation which is processed into a sheet or a string may be applied by winding a plant with a sheet or a string of the resin preparation, putting a string of the resin preparation around a crop so that the plant is surrounded by the string, or laying a sheet of the resin preparation on the soil surface near the root of a plant.

When the Present compound or the Composition A is used to control harmful arthropods that live inside a house, the application dose as an amount of the Present compound is usually within a range from 0.01 to 1,000 mg per 1 $m^2$ of an area to be treated, in the case of using it on a planar area. In the case of using it spatially, the application dose as an amount of the Present compound is usually within a range from 0.01 to 500 mg per 1 $m^3$ of the space to be treated. When the Present compound or the Composition A is formulated into emulsifiable concentrates, wettable powders, flowables or the others, such formulations are usually applied after diluting it with water in such a way that a concentration of the active ingredient is within a range from 0.1 to 10,000 ppm. In the case of being formulated into oil solutions, aerosols, smoking agents, poison baits and the others, such formulations are used as itself without diluting it.

When the Present compound or the composition is used for controlling external parasites of livestock such as cows, horses, pigs, sheep, goats and chickens and small animals such as dogs, cats, rats and mice, the composition of the present invention may be applied to the animals by a known method in the veterinary field. Specifically, when systemic control is intended, the composition of the present invention is administered to the animals as a tablet, a mixture with feed or a suppository, or by injection (including intramuscular, subcutaneous, intravenous and intraperitoneal injections). On the other hand, when non-systemic control is intended, the composition of the present invention is applied to the animals by means of spraying of the oil solution or aqueous solution, pour-on or spot-on treatment, or washing of the animal with a shampoo formulation, or by putting a collar or ear tag made of the resin formulations to the animal. In the case of being administered to an animal body, the dose of the compound of the Present compound is usually within a range from 0.1 to 1,000 mg per 1 kg of an animal body weight.

Also, the composition of the Present compound or the Composition A may be used as an agent for controlling harmful arthropods in agricultural lands such as paddy fields, fields, turfs, and orchards. Examples of the plants include the followings:

corn (dent corn, flint corn, flour corn, popcorn, waxy corn, sweet corn, and field corn), rice (long grain rice, short grain rice, medium grain rice, japonica rice, tropical japonica rice, indica rice, javanica rice, paddy rice, upland rice, floating rice, direct-seeded rice, transplanted rice, and glutinous rice), wheat (bread wheat (hard wheat, soft wheat, medium wheat, red wheat, and white wheat), durum wheat, spelt wheat, and club wheat, winter wheat and spring wheat of them), barley (two-rowed barley (= barley for brewery), six-rowed barley, hull-less barley, and pearl barley, winter barley and spring barley of

them), rye (winter rye and spring rye), triticale (winter triticale and spring triticale), oat (winter oat and spring oat), sorghum, cotton (upland cotton and Pima cotton), soybean (ripe seed harvest soybean, green soybeans, and early harvest soybeans, indeterminate type, determinate type, and semi-determinate type of them), peanut, buckwheat, beet (beets for sugar production, beets for feed, beets for root vegetable, beets for leaf vegetable, and beets for fuel), rapeseed (winter rapeseed and spring rapeseed), canola (winter canola and spring canola), sunflower (sunflowers for oil extraction, edible sunflowers, and sunflowers for ornamental purpose), sugar cane, tobacco, tea, mulberry, solanaceous vegetables (for example, eggplant, tomato, pimento, pepper, and potato), cucurbitaceous vegetables (for example, cucumber, pumpkin, zucchini, water melon, and melon), cruciferous vegetables (for example, Japanese radish, white turnip, horseradish, kohlrabi, Chinese cabbage, cabbage, leaf mustard, broccoli, and cauliflower), asteraceous vegetables (for example, burdock, crown daisy, artichoke, and lettuce), liliaceous vegetables (for example, welsh onion, onion, garlic, and asparagus), ammiaceous vegetables (for example, carrot, parsley, celery, and parsnip), chenopodiaceous vegetables (for example, spinach and Swiss chard), lamiaceous vegetables (for example, perilla, mint, and basil), strawberry, sweet potato, glutinous yam, eddoe, pomaceous fruits (for example, apple, pear, Japanese pear, Chinese white pear, Chinese quince, and quince), stone fleshy fruits (for example, peach, plum, nectarine, Japanese apricot (Prunus mume), cherry fruit, apricot, and prune), citrus fruits (for example, Citrus unshiu, orange, lemon, lime, and grapefruit), nuts (for example, chestnuts, walnuts, hazelnuts, almond, pistachio, cashew nuts, and macadamia nuts), berry fruits (for example, blueberry, cranberry, blackberry, and raspberry), grapes, Japanese persimmon, fig, olive, Japanese plum, banana, coffee, date palm, coconuts, ornamental plants, forest plants, turfs, grasses, and the others.

The above plants are not specifically limited as long as they are generally cultivated cultivars. The above plants also include plants which may be produced by natural breeding, plants which may be generated by mutation, F1 hybrid plants, and genetically modified crops. Examples of the genetically modified crops include plants which have resistance to HPPD (4-hydroxyphenylpyruvate dioxygenase enzyme) inhibitors such as isoxaflutole, ALS (acetolactate synthase) inhibitors such as imazethapyr and thifensulfuron-methyl, EPSP (5-enolpyruvylshikimate-3-phosphate synthase) inhibitors, glutamine synthetase inhibitors, PPO (protoporphyrinogen oxidase) inhibitors, or herbicide such as bromoxynil and dicamba; plants which can synthesize a selective toxin known in Bacillus spp. such as Bacillus thuringiensis or the like; and plants which can synthesize a gene fragment or the like which is partially identical to an endogenous gene derived from a harmful insect, and induce a gene silencing (RNAi; RNA interference) in the target harmful insect to achieve a specific insecticidal activity.

[EXAMPLES]

Hereinafter, the present invention will be described in more detail with reference to production examples, formulation examples, test examples, and the like, but the present invention is not limited to these examples.

In the present specification, Me represents a methyl group, Et represents an ethyl group, Pr represents a propyl group, i-Pr represents an isopropyl group, t-Bu represents a tert-butyl group, $C_2F_5$ represents a perfluoroethyl group, c-Pr represents a cyclopropyl group, $CF_3$ represents a trifluoromethyl group, $CF_2H$ represents a difluoromethyl group, Ph represents a phenyl group, Py2 represents a 2-pyridyl group, Py3 represents a 3-pyridyl group, and Py4 represents a 4-pyridyl group. When c-Pr, Ph, Py2, Py3, and Py4 have a substituent, the substituent is described together with a substitution position before the symbol. For example, 1-CN-c-Pr represents a 1-cyanocyclopropyl group, 3,4-$F_2$-Ph represents a 3,4-difluorophenyl group, 2-$C_2F_5$-Ph represents a 2-(perfluoroethyl)phenyl group, 4-$SO_2CF_3$-Ph represents a 4- (trifluoromethanesulfonyl)phenyl group, 3,4-$(OCF_3)_2$-Ph represents a 3,4-bis(trifluoromethoxy)phenyl group, 3-$SCF_3$-4-$OCF_3$-Ph represents a 3- [(trifluoromethyl)thio]-4-(trifluoromethoxy)phenyl group, 4-$CF_3$-Py2 is a 4-(trifluoromethyl)-2-pyridyl group, 3-$CF_2$H-1-i-Pr-1H-pyrrol-2-yl represents a 3-difluoromethyl-1-isopropyl-2-1H-pyrrolyl group, 4-$CF_2$H-1-Me-1H-imidazole-2-yl represents a 4-difluoromethyl-1-methyl-2-1H-imidazolyl group, and 3-$CF_2$H-1-Me-1H-pyrazol-5-yl represents a 3-difluoromethyl-1-methyl-5-1H-pyrazolyl group.

First, production examples of the present compound and production intermediates thereof will be shown.

When the physical property of the compound is measured by liquid chromatography/mass spectrometry (hereinafter, referred to as LCMS), the measured molecular ion value $[M+H]^+$ or $[M-H]^-$ and retention time (hereinafter, referred to as RT) are described. The conditions of liquid chromatography (hereinafter, referred to as LC) are as follows.

[LC conditions]

Column: L-column2 ODS, inner diameter: 4.6 mm, length: 30 mm, particle diameter: 3 μm (Chemicals Evaluation and Research Institute, Japan)
UV measurement wavelength: 254 nm
Mobile phase: A solution: 0.1% formic acid aqueous solution, B solution: 0.1% formic acid acetonitrile

Flow rate: 2.0 mL/min
Gradient conditions: feeding is performed at the concentration gradient described in [Table LC1].

[Table LC1]

| Time (minutes) | A solution (%) | B solution (%) |
|---|---|---|
| 0.01 | 90 | 10 |
| 2.00 | 0 | 100 |
| 4.00 | 0 | 100 |
| 4.01 | 90 | 10 |

Reference production exapmle 1

1.6 g of 1-ethyl-3-[3-(dimethylamino)propyl]carbodiimide hydrochloride and 1.5 g of 6-iodo-3-(ethylthio)imidazo [1,2-a] pyridine-2-amine (hereinafter, referred to as an intermediate Z) produced by the method described in WO 2018/052136 were added to a mixture of 0.7 g of 5-methylisoxazole-5-carboxylic acid and 10 mL of pyridine, and the mixture was stirred at 80°C for 4 hours. Water was added to the resulting mixture, and the precipitated solid was separated by filtration and dried to provide 1.8 g of an intermediate 1 represented by the following formula.

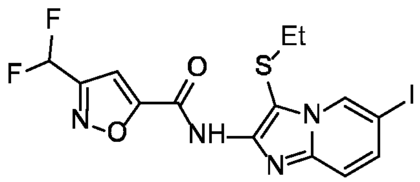

Intermediate 1: $^1H$-NMR ($CDCl_3$): 8.73 (1H, br s), 8.64-8.63 (1H, m), 7.54-7.51 (1H, m), 7.49-7.47 (1H, m), 7.32 (1H, s), 6.86 (1H, t), 2.75 (2H, q), 1.25 (3H, t).

Reference production example 1-1

The compound produced in accordance with Reference production example 1 and physical properties thereof are shown below.
A compound represented by a formula (A-1)

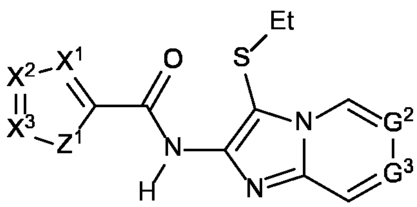

(A-1)

wherein the combination of $Z^1$, $X^1$, $X^2$, $X^3$, $G^2$, and $G^3$ is any of the combinations shown in [Table A-1].

[Table A-1]

| Intermediate | $Z^1$ | $X^1$ | $X^2$ | $X^3$ | $G^2$ | $G^3$ |
|---|---|---|---|---|---|---|
| 2 | N-Me | CH | CH | N | C-I | CH |
| 3 | N-Me | CH | $C-CF_3$ | N | C-I | CH |
| 4 | $N-CF_2H$ | CH | CH | N | C-I | CH |
| 5 | O | CH | CH | $C-CF_3$ | C-I | CH |
| 6 | S | CH | CH | $C-CF_3$ | C-I | CH |
| 7 | S | CH | N | $C-CF_3$ | C-I | CH |

(continued)

| Intermediate | $Z^1$ | $X^1$ | $X^2$ | $X^3$ | $G^2$ | $G^3$ |
|---|---|---|---|---|---|---|
| 8 | S | CH | N | C-$CF_3$ | C-Br | CH |
| 9 | S | CH | N | C-$CF_3$ | C-$CF_3$ | CH |
| 10 | S | CH | N | C-$CF_3$ | CH | C-I |
| 11 | S | CH | N | C-$CF_3$ | CH | C-Br |
| 12 | S | CH | N | C-Cl | C-I | CH |
| 13 | S | N | C-$CF_3$ | CH | C-I | CH |

Intermediate 2: $^1$H-NMR ($CDCl_3$) δ: 8.62-8.61 (1H, m), 8.19 (1H, br s), 7.54 (1H, d), 7.53-7.46 (2H, m), 6.73 (1H, d), 4.27 (3H, s), 2.72 (2H, q), 1.24 (3H, t).
Intermediate 3: $^1$H-NMR ($CDCl_3$) δ: 8.62-8.61 (1H, m), 8.25 (1H, brs), 7.51-7.49 (2H, m), 6.97 (1H, s), 4.31 (3H, s), 2.73 (2H, q), 1.25-1.23 (3H, m).
Intermediate 4: LCMS: 464 [M+H]+, RT = 1.79 min
Intermediate 5: $^1$H-NMR ($CDCl_3$) δ: 8.64-8.63 (1H, m), 8.56 (1H, br s), 7.52-7.49 (1H, m), 7.46-7.44 (1H, m), 7.36-7.35 (1H, m), 6.98-6.97 (1H, m), 2.76 (2H, q), 1.25 (3H, t).
Intermediate 6: LCMS: 498 [M+H]+, RT = 2.06 min
Intermediate 7: $^1$H-NMR ($CDCl_3$) δ: 8.64-8.64 (1H, m), 8.41-8.41 (1H, m), 8.31 (1H, br s), 7.54-7.52 (1H, m), 7.45-7.43 (1H, m), 2.76 (2H, q), 1.24 (3H, t).
Intermediate 8: $^1$H-NMR ($CDCl_3$) δ: 8.54-8.54 (2H, m), 8.44-8.44 (1H, m), 7.54 (1H, dd), 7.41 (1H, dd), 2.77 (2H, q), 1.24 (3H, t).
Intermediate 9: $^1$H-NMR ($CDCl_3$) δ: 8.77-8.76 (1H, m), 8.47 (1H, br s), 8.45-8.44 (1H, m), 7.76 (1H, d), 7.50 (1H, dd), 2.80 (2H, q), 1.26 (3H, t).
Intermediate 10: LCMS: 499 [M+H]+, RT = 1.91 min
Intermediate 11: LCMS: 451 [M+H]+, RT = 1.86 min
Intermediate 12: LCMS: 465 [M+H]+, RT = 1.83 min
Intermediate 13: $^1$H-NMR ($CDCl_3$) δ: 9.53 (1H, br s), 8.64-8.64 (1H, m), 8.07-8.07 (1H, m), 7.53-7.50 (1H, m), 7.49-7.46 (1H, m), 2.75 (2H, q), 1.25 (3H, t).

Reference production example 1-2

The compound produced in accordance with Reference production example 1 and physical properties thereof are shown below.

A compound represented by a formula (A-2)

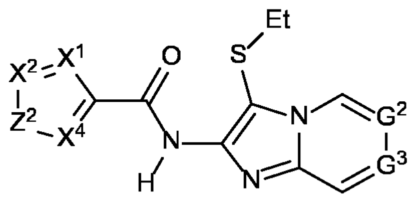

(A-2)

wherein the combination of $Z^2$, $X^1$, $X^2$, $X^4$, $G^2$, and $G^3$ is any of the combinations shown in [Table A-2].

[Table A-2]

| Intermediate | $Z^2$ | $X^1$ | $X^2$ | $X^4$ | $G^2$ | $G^3$ |
|---|---|---|---|---|---|---|
| 14 | N-Me | C-$CF_3$ | N | CH | C-I | CH |
| 15 | N-Me | C-$CF_2H$ | N | CH | C-Br | CH |
| 16 | N-Me | CH | C-$CF_3$ | N | C-I | CH |
| 17 | N-Me | CH | C-Br | N | C-I | CH |
| 18 | N-Me | CH | C-$OCF_2H$ | N | C-I | CH |
| 19 | N-$CF_2H$ | CH | CH | N | C-I | CH |

(continued)

| Intermediate | $Z^2$ | $X^1$ | $X^2$ | $X^4$ | $G^2$ | $G^3$ |
|---|---|---|---|---|---|---|
| 20 | N-c-Pr | CH | CH | N | C-I | CH |
| 21 | N-$CH_2CF_3$ | CH | CH | N | C-I | CH |
| 22 | N-$CF_2H$ | CH | C-Me | N | C-I | CH |
| 23 | N-$CF_3$ | CH | N | CH | C-I | CH |
| 24 | N-$CF_2H$ | CH | N | CH | C-I | CH |
| 25 | N-c-Pr | CH | N | CH | C-I | CH |
| 26 | N-Me | CH | N | C-$CF_3$ | C-I | CH |
| 27 | N-$CH_2CF_3$ | N | N | CH | C-I | CH |
| 28 | N-Me | C-$CF_3$ | CH | CH | C-I | CH |
| 29 | O | CH | C-$CF_3$ | N | C-I | CH |
| 30 | O | CH | C-i-Pr | N | C-I | CH |
| 31 | O | CH | C-i-Pr | N | C-Br | CH |
| 32 | O | CH | C-i-Pr | N | C-$CF_3$ | CH |
| 33 | O | CH | C-i-Pr | N | CH | C-I |
| 34 | O | CH | C-i-Pr | N | CH | C-Br |

Intermediate 14: $^1$H-NMR ($CDCl_3$) δ: 8.60-8.60 (1H, m), 8.29 (1H, br s), 8.12 (1H, s), 7.47-7.46 (2H, m), 4.01 (3H, s), 2.70 (2H, q), 1.20 (3H, t).
Intermediate 15: $^1$H-NMR ($CDCl_3$) δ: 8.59 (1H, br s), 8.51-8.50 (1H, m), 8.10 (1H, s), 7.56 (1H, d), 7.35 (1H, dd), 6.93 (1H, t), 3.98 (3H, s), 2.70 (2H, q), 1.21 (3H, t).
Intermediate 16: LCMS: 496 [M+H] +, RT = 1.95 min
Intermediate 17: $^1$H-NMR ($CDCl_3$) δ: 9.13 (1H, br s), 8.61-8.60 (1H, m), 7.46-7.45 (2H, m), 6.99 (1H, s), 3.97 (3H, s), 2.71 (2H, q), 1.22 (3H, t).
Intermediate 18: LCMS: 494 [M+H] +, RT = 1.85 min
Intermediate 19: LCMS: 464 [M+H] +, RT = 1.69 min
Intermediate 20: LCMS: 454 [M+H] +, RT = 1.70 min
Intermediate 21: LCMS: 496 [M+H] +, RT = 1.83 min
Intermediate 22: LCMS: 478 [M+H] +, RT = 1.86 min
Intermediate 23: $^1$H-NMR ($CDCl_3$) δ: 8.63-8.63 (1H, m), 8.47 (1H, s), 8.19 (1H, br s), 8.15 (1H, s), 7.52-7.49 (1H, m), 7.42 (1H, d), 2.74 (2H, q), 1.23 (3H, t).
Intermediate 24: LCMS: 464 [M+H] +, RT = 1.63 min
Intermediate 25: LCMS: 454 [M+H] +, RT = 1.55 min
Intermediate 26: LCMS: 496 [M+H] +, RT = 1.75 min
Intermediate 27: LCMS: 497 [M+H] +, RT = 1.79 min
Intermediate 28: LCMS: 495 [M+H] +, RT = 1.69 min
Intermediate 29: LCMS: 483 [M+H] +, RT = 2.02 min
Intermediate 30: LCMS: 457 [M+H] +, RT = 1.96 min
Intermediate 31: LCMS: 409 [M+H] +, RT = 1.93 min
Intermediate 32: LCMS: 399 [M+H] +, RT = 1.98 min
Intermediate 33: LCMS: 457 [M+H] +, RT = 1.94 min
Intermediate 34: LCMS: 409 [M+H] +, RT = 1.90 min

Production example 1

0.31 mL of methyl iodide was added to a mixture of 1.2 g of Intermediate 1, 2.4 g of cesium carbonate, and 5 mL of DMF under ice cooling, and the mixture was stirred at room temperature for 5 hours. Water was added to the resulting mixture, and the mixture was extracted with ethyl acetate. The resulting organic layer was dried over anhydrous sodium sulfate and concentrated under reduced pressure. The resulting residue was subjected to a silica gel chromatography to provide 1.1 g of a present compound 1.

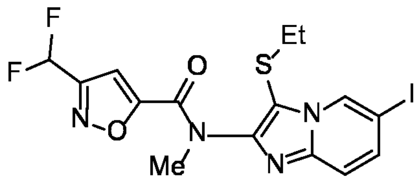

Present compound 1: $^1$H-NMR ($CDCl_3$) $\delta$: 8.60-8.59 (1H, m), 7.56-7.53 (1H, m), 7.39-7.36 (1H, m), 6.75-6.56 (1H, m), 6.65 (1H, t), 3.56 (3H, s), 2.59 (2H, q), 1.17 (3H, t).

Production example 1-1

The compound produced in accordance with Production example 1 and physical properties thereof are shown below.

A compound represented by a formula (B-1)

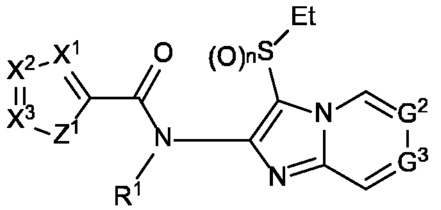

(B-1)

wherein the combination of n, $Z^1$, $X^1$, $X^2$, $X^3$, $G^2$, $G^3$, and $R^1$ is any of the combinations shown in [Table B-1]

[Table B-1]

| Present compound | n | $Z^1$ | $X^1$ | $X^2$ | $X^3$ | $G^2$ | $G^3$ | $R^1$ |
|---|---|---|---|---|---|---|---|---|
| 2 | 0 | N-Me | CH | CH | N | C-I | CH | Me |
| 3 | 0 | N-Me | CH | C-$CF_3$ | N | C-I | CH | Me |
| 4 | 0 | N-$CF_2$H | CH | CH | N | C-I | CH | Me |
| 5 | 0 | O | CH | CH | C-$CF_3$ | C-I | CH | Me |
| 6 | 0 | S | CH | CH | C-$CF_3$ | C-I | CH | Me |
| 7 | 0 | S | CH | N | C-$CF_3$ | C-I | CH | Me |
| 8 | 0 | S | CH | N | C-$CF_3$ | C-Br | CH | Me |
| 9 | 0 | S | CH | N | C-$CF_3$ | C-$CF_3$ | CH | Me |
| 10 | 0 | S | CH | N | C-$CF_3$ | CH | C-I | Me |
| 11 | 0 | S | CH | N | C-$CF_3$ | CH | C-Br | Me |
| 12 | 0 | S | CH | N | C-Cl | C-I | CH | Me |

(continued)

| Present compound | n | $Z^1$ | $X^1$ | $X^2$ | $X^3$ | $G^2$ | $G^3$ | $R^1$ |
|---|---|---|---|---|---|---|---|---|
| 13 | 0 | S | N | C-$CF_3$ | CH | C-I | CH | Me |

Present compound 2: $^1$H-NMR ($CDCl_3$) δ: 8.46 (1H, s), 7.53 (1H, d), 7.41 (1H, d), 7.08 (1H, s), 5.63 (1H, s), 4.16 (3H, s), 3.51 (3H, s), 2.37 (2H, q), 1.13 (3H, t).
Present compound 3: $^1$H-NMR ($CDCl_3$) δ: 8.49-8.48 (1H, m), 7.57-7.55 (1H, m), 7.43-7.40 (1H, m), 5.93 (1H, s), 4.19 (3H, s), 3.52 (3H, s), 2.46 (2H, q), 1.14 (3H, t).
Present compound 4: $^1$H-NMR ($CDCl_3$) δ: 8.48-8.48 (1H, m), 7.92 (1H, t), 7.56 (1H, dd), 7.43 (1H, dd), 7.37 (1H, s), 5.74 (1H, s), 3.51 (3H, s), 2.40 (2H, q), 1.12 (3H, t).
Present compound 5: $^1$H-NMR ($CDCl_3$) δ: 8.60-8.60 (1H, m), 7.54-7.52 (1H, m), 7.38-7.36 (1H, m), 6.66-6.62 (1H, m), 6.66-6.66 (1H, m), 3.53 (3H, s), 2.54 (2H, q), 1.15 (3H, t).
Present compound 6: $^1$H-NMR ($CDCl_3$) δ: 8.56-8.55 (1H, m), 7.59-7.56 (1H, m), 7.46-7.43 (1H, m), 7.08-7.06 (1H, m), 6.79-6.77 (1H, m), 3.50 (3H, s), 2.46 (2H, q), 1.12 (3H, t).
Present compound 7: $^1$H-NMR ($CDCl_3$) δ: 8.57-8.56(1H, m), 7.61-7.58(1H, m), 7.50-7.50(1H, m), 7.46-7.43(1H, m), 3.53(3H, s), 2.55(2H, q), 1.15(3H, t).
Present compound 8: $^1$H-NMR ($CDCl_3$) δ: 8.47-8.46 (1H, m), 7.56 (1H, dd), 7.51 (1H, d), 7.49 (1H, dd), 3.53 (3H, s), 2.55 (2H, q), 1.15 (3H, t).
Present compound 9: $^1$H-NMR ($CDCl_3$) δ: 8.69-8.68 (1H, m), 7.77 (1H, d), 7.57 (1H, dd), 7.49-7.49 (1H, m), 3.55 (3H, s), 2.58 (2H, q), 1.16 (3H, t).
Present compound 10: LCMS: 513 [M+H] +, RT = 2.02 min
Present compound 11: $^1$H-NMR ($CDCl_3$) δ: 8.21 (1H, dd), 7.85 (1H, dd), 7.54-7.53 (1H, m), 7.15 (1H, dd), 3.52 (3H, s), 2.53 (2H, q), 1.13 (3H, t).
Present compound 12: $^1$H-NMR ($CDCl_3$) δ: 8.59-8.59 (1H, m), 7.61-7.58 (1H, m), 7.46-7.43 (1H, m), 7.35 (1H, s), 3.48 (3H, s), 2.57 (2H, q), 1.16 (3H, t).
Present compound 13: $^1$H-NMR ($CDCl_3$) δ: 8.63-8.62 (1H, m), 7.78 (1H, s), 7.51-7.48 (1H, m), 7.33-7.31 (1H, m), 3.62 (3H, s), 2.63 (2H, q), 1.17 (3H, t).

Production example 1-2

The compound produced in accordance with Production example 1 and physical properties thereof are shown below.

A compound represented by a formula (B-2)

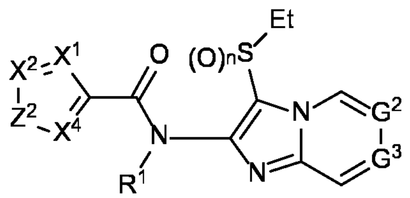

(B-2)

wherein the combination of n, $Z^2$, $X^1$, $X^2$, $X^4$, $G^2$, $G^3$, and $R^1$ is any of the combinations shown in [Table B-2]

[Table B-2]

| Present compound | n | $Z^2$ | $X^1$ | $X^2$ | $X^4$ | $G^2$ | $G^3$ | $R^1$ |
|---|---|---|---|---|---|---|---|---|
| 14 | 0 | N-Me | C-$CF_3$ | N | CH | C-I | CH | Me |
| 15 | 0 | N-Me | C-$CF_2H$ | N | CH | C-Br | CH | Me |
| 16 | 0 | N-Me | CH | C-$CF_3$ | N | C-I | CH | Me |
| 17 | 0 | N-Me | CH | C-Br | N | C-I | CH | Me |
| 18 | 0 | N-Me | CH | C-$OCF_2H$ | N | C-I | CH | Me |
| 19 | 0 | N-$CF_2H$ | CH | CH | N | C-I | CH | Me |
| 20 | 0 | N-c-Pr | CH | CH | N | C-I | CH | Me |
| 21 | 0 | N-$CH_2CF_3$ | CH | CH | N | C-I | CH | Me |

(continued)

| Present compound | n | $Z^2$ | $X^1$ | $X^2$ | $X^4$ | $G^2$ | $G^3$ | $R^1$ |
|---|---|---|---|---|---|---|---|---|
| 22 | 0 | N-$CF_2H$ | CH | C-Me | N | C-I | CH | Me |
| 23 | 0 | N-$CF_3$ | CH | N | CH | C-I | CH | Me |
| 24 | 0 | N-$CF_2H$ | CH | N | CH | C-I | CH | Me |
| 25 | 0 | N-c-Pr | CH | N | CH | C-I | CH | Me |
| 26 | 0 | N-Me | CH | N | C-$CF_3$ | C-I | CH | Me |
| 27 | 0 | N-$CH_2CF_3$ | N | N | CH | C-I | CH | Me |
| 28 | 0 | N-Me | C-$CF_3$ | CH | CH | C-I | CH | Me |
| 29 | 0 | O | CH | C-$CF_3$ | N | C-I | CH | Me |
| 30 | 0 | O | CH | C-i-Pr | N | C-I | CH | Me |
| 31 | 0 | O | CH | C-i-Pr | N | C-Br | CH | Me |
| 32 | 0 | O | CH | C-i-Pr | N | C-$CF_3$ | CH | Me |
| 33 | 0 | O | CH | C-i-Pr | N | CH | C-I | Me |

(continued)

| Present compound | n | $Z^2$ | $X^1$ | $X^2$ | $X^4$ | $G^2$ | $G^3$ | $R^1$ |
|---|---|---|---|---|---|---|---|---|
| 34 | 0 | O | CH | C-i-Pr | N | CH | C-Br | Me |

Present compound 14: $^1$H-NMR ($CDCl_3$) δ: 8.53-8.52 (1H, m), 7.55-7.53 (1H, m), 7.42-7.39 (1H, m), 7.05 (1H, s), 3.71 (3H, s), 3.46 (3H, s), 2.52 (2H, q), 1.13 (3H, t).
Present compound 15: $^1$H-NMR ($CDCl_3$) δ: 8.45-8.45 (1H, m), 7.55 (1H, d), 7.45 (1H, dd), 7.21 (1H, t), 6.77 (1H, s), 3.69 (3H, s), 3.45 (3H, s), 2.48 (2H, q), 1.11 (3H, t).
Present compound 16: $^1$H-NMR ($CDCl_3$) δ: 8.57-8.57 (1H, m), 7.52-7.49 (1H, m), 7.41-7.38 (1H, m), 6.74 (1H, s), 3.74 (3H, s), 3.54 (3H, s), 2.53 (2H, q), 1.13 (3H, t).
Present compound 17: $^1$H-NMR ($CDCl_3$) δ: 8.57-8.57 (1H, m), 7.50 (1H, dd), 7.40 (1H, d), 6.26 (1H, s), 3.67 (3H, s), 3.52 (3H, s), 2.52 (2H, q), 1.12 (3H, t).
Present compound 18: $^1$H-NMR ($CDCl_3$) δ: 8.57-8.56 (1H, m), 7.49 (1H, dd), 7.39 (1H, dd), 6.37 (1H, t), 5.93 (1H, s), 3.52 (3H, s), 3.51 (3H, s), 2.52 (2H, q), 1.12 (3H, t).
Present compound 19: $^1$H-NMR ($CDCl_3$) δ: 8.54-8.54 (1H, m), 7.58 (1H, d), 7.52-7.50 (1H, m), 7.40-7.38 (1H, m), 6.92 (1H, t), 6.43 (1H, s), 3.55 (3H, s), 2.47 (2H, q), 1.11 (3H, t).
Present compound 20: LCMS: 468 [M+H] +, RT = 1.67 min
Present compound 21: $^1$H-NMR ($CDCl_3$) δ: 8.53-8.52 (1H, m), 7.49 (1H, dd), 7.37 (1H, dd), 7.27 (1H, s), 6.49-6.49 (1H, m), 4.44 (2H, q), 3.54 (3H, s), 2.45 (2H, q), 1.11 (3H, t).
Present compound 22: LCMS: 492 [M+H] +, RT = 1.77 min
Present compound 23: $^1$H-NMR ($CDCl_3$) δ: 8.56-8.55 (1H, m), 8.01 (1H, s), 7.59-7.56 (1H, m), 7.45-7.42 (1H, m), 7.00 (1H, s), 3.48 (3H, s), 2.51 (2H, q), 1.12 (3H, t).
Present compound 24: $^1$H-NMR ($CDCl_3$) δ: 8.55-8.55 (1H, m), 7.93 (1H, s), 7.58-7.55 (1H, m), 7.45-7.43 (1H, m), 7.02 (1H, t), 6.99 (1H, s), 3.48 (3H, s), 2.47 (2H, q), 1.10 (3H, t).
Present compound 25: LCMS: 468 [M+H] +, RT = 1.69 min
Present compound 26: $^1$H-NMR ($CDCl_3$) δ: 8.47-8.47 (1H, m), 7.50 (1H, dd), 7.37 (1H, dd), 7.03 (1H, s), 3.89 (3H, s), 3.50 (3H, s), 2.56 (2H, q), 1.15 (3H, t).
Present compound 27: $^1$H-NMR ($CDCl_3$) δ: 8.58-8.58 (1H, m), 8.10 (1H, s), 7.50-7.47 (1H, m), 7.37-7.34 (1H, m), 4.88 (2H, q), 3.58 (3H, s), 2.56 (2H, q), 1.15 (3H, t).
Present compound 28: $^1$H-NMR ($CDCl_3$) δ: 8.53-8.52 (1H, m), 7.51 (1H, dd), 7.40 (1H, dd), 6.79-6.79 (1H, m), 6.25 (1H, d), 3.44 (3H, s), 3.39 (3H, s), 2.45 (2H, q), 1.10 (3H, t).
Present compound 29: $^1$H-NMR ($CDCl_3$) δ: 8.59-8.59 (1H, m), 7.53-7.51 (1H, m), 7.36-7.34 (1H, m), 7.04 (1H, s), 3.58 (3H, s), 2.66 (2H, q), 1.22-1.17 (3H, m).
Present compound 30: $^1$H-NMR ($CDCl_3$) δ: 8.58-8.57 (1H, m), 7.48 (1H, dd), 7.36 (1H, dd), 6.29 (1H, s), 3.55 (3H, s), 3.01-2.94 (1H, m), 2.60 (2H, q), 1.28-1.20 (6H, m), 1.17 (3H, t).
Present compound 31: $^1$H-NMR ($CDCl_3$) δ: 8.48-8.47 (1H, m), 7.47 (1H, dd), 7.37 (1H, dd), 6.29 (1H, s), 3.55 (3H, s), 3.01-2.94 (1H, m), 2.60 (2H, q), 1.26-1.22 (6H, m), 1.17 (3H, t).
Present compound 32: LCMS: 413 [M+H] +, RT = 1.96 min
Present compound 33: $^1$H-NMR ($CDCl_3$) δ: 8.09 (1H, d), 7.97-7.97 (1H, m), 7.19 (1H, dd), 6.28 (1H, s), 3.54 (3H, s), 3.01-2.92 (1H, m), 2.60-2.54 (2H, m), 1.24-1.18 (6H, m), 1.15 (3H, t).
Present compound 34: LCMS: 423 [M+H] +, RT = 1.90 min

Production example 2

1.5 g of mCPBA(purity 65%, 35% water included) was added to a mixture of 0.87 g of the present compound 1 and 10 mL of chloroform under ice cooling, and the mixture was stirred at room temperature for 2 hours. To the resulting mixture, a saturated aqueous sodium bicarbonate solution and an aqueous sodium thiosulfate solution were sequentially added, and the mixture was stirred at room temperature for 3 hours and then extracted with chloroform. The resulting organic layer was dried over anhydrous sodium sulfate and concentrated under reduced pressure. The resulting residue was subjected to a silica gel chromatography to provide 0.76 g of a present compound 35.

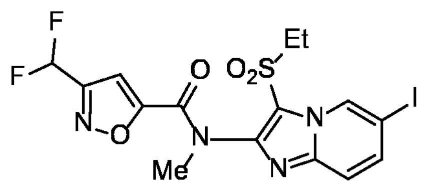

Present compound 35: LCMS: 511 [M+H]+, RT = 1.73 min

Production example 2-1

The compound produced in accordance with Production example 2 and physical properties thereof are shown below.

A compound represented by a formula (B-3)

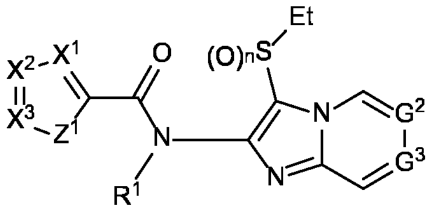

( B-3 )

wherein the combination of n, $Z^1$, $X^1$, $X^2$, $X^3$, $G^2$, $G^3$, and $R^1$ is any of the combinations shown in [Table B-3].

[Table B-3]

| Present compound | n | $Z^1$ | $X^1$ | $X^2$ | $X^3$ | $G^2$ | $G^3$ | $R^1$ |
|---|---|---|---|---|---|---|---|---|
| 36 | 2 | N-Me | CH | $C-CF_3$ | N | C-I | CH | Me |
| 37 | 1 | $N-CF_2H$ | CH | CH | N | C-I | CH | Me |
| 38 | 2 | $N-CF_2H$ | CH | CH | N | C-I | CH | Me |
| 39 | 2 | O | CH | CH | $C-CF_3$ | C-I | CH | Me |
| 40 | 2 | S | CH | N | $C-CF_3$ | C-I | CH | Me |
| 41 | 1 | S | CH | N | $C-CF_3$ | C-Br | CH | Me |
| 42 | 2 | S | CH | N | $C-CF_3$ | C-Br | CH | Me |
| 43 | 1 | S | CH | N | $C-CF_3$ | $C-CF_3$ | CH | Me |
| 44 | 2 | S | CH | N | $C-CF_3$ | $C-CF_3$ | CH | Me |
| 45 | 1 | S | CH | N | $C-CF_3$ | CH | C-I | Me |
| 46 | 2 | S | CH | N | $C-CF_3$ | CH | C-I | Me |
| 47 | 1 | S | CH | N | $C-CF_3$ | CH | C-Br | Me |
| 48 | 2 | S | CH | N | $C-CF_3$ | CH | C-Br | Me |
| 49 | 2 | S | CH | N | C-Cl | C-I | CH | Me |
| 50 | 2 | S | N | $C-CF_3$ | CH | C-I | CH | Me |

Present compound 36: LCMS: 542 [M+H]+, RT = 1.87 min
Present compound 37: LCMS: 494 [M+H]+, RT = 1.44 min
Present compound 38: LCMS: 510 [M+H]+, RT = 1.61 min
Present compound 39: LCMS: 528 [M+H]+, RT = 1.83 min
Present compound 40: LCMS: 545 [M+H]+, RT = 1.80 min
Present compound 41: LCMS: 481 [M+H]+, RT = 1.71 min
Present compound 42: LCMS: 497 [M+H]+, RT = 1.77 min
Present compound 43: LCMS: 471 [M+H]+, RT = 1.81 min
Present compound 44: LCMS: 487 [M+H]+, RT = 1.85 min
Present compound 45: LCMS: 529 [M+H]+, RT = 1.76 min
Present compound 46: LCMS: 545 [M+H]+, RT = 1.82 min
Present compound 47: LCMS: 481 [M+H]+, RT = 1.73 min
Present compound 48: LCMS: 497 [M+H]+, RT = 1.79 min
Present compound 49: LCMS: 511 [M+H]+, RT = 1.73 min
Present compound 50: LCMS: 545 [M+H]+, RT = 1.79 min

Production example 2-2

The compound produced in accordance with Production example 2 and physical properties thereof are shown below.

A compound represented by a formula (B-4)

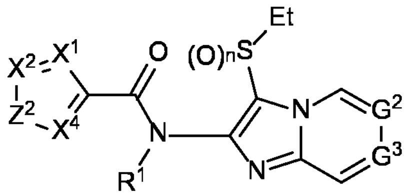

( B-4 )

wherein the combination of n, $Z^2$, $X^1$, $X^2$, $X^4$, $G^2$, $G^3$, and $R^1$ is any of the combinations shown in [Table B-4]

[Table B-4]

| Present compound | n | $Z^2$ | $X^1$ | $X^2$ | $X^4$ | $G^2$ | $G^3$ | $R^1$ |
|---|---|---|---|---|---|---|---|---|
| 51 | 2 | N-Me | $C-CF_3$ | N | CH | C-I | CH | Me |
| 52 | 2 | N-Me | CH | $C-CF_3$ | N | C-I | CH | Me |
| 53 | 2 | N-Me | CH | C-Br | N | C-I | CH | Me |
| 54 | 1 | N-Me | CH | $C-OCF_2H$ | N | C-I | CH | Me |
| 55 | 2 | N-Me | CH | $C-OCF_2H$ | N | C-I | CH | Me |
| 56 | 2 | $N-CF_2H$ | CH | CH | N | C-I | CH | Me |
| 57 | 2 | N-c-Pr | CH | CH | N | C-I | CH | Me |
| 58 | 1 | $N-CH_2CF_3$ | CH | CH | N | C-I | CH | Me |
| 59 | 2 | $N-CH_2CF_3$ | CH | CH | N | C-I | CH | Me |
| 60 | 1 | $N-CF_3$ | CH | N | CH | C-I | CH | Me |
| 61 | 2 | $N-CF_3$ | CH | N | CH | C-I | CH | Me |
| 62 | 1 | $N-CF_2H$ | CH | N | CH | C-I | CH | Me |
| 63 | 2 | $N-CF_2H$ | CH | N | CH | C-I | CH | Me |
| 64 | 2 | N-c-Pr | CH | N | CH | C-I | CH | Me |
| 65 | 1 | N-Me | CH | N | $C-CF_3$ | C-I | CH | Me |
| 66 | 2 | N-Me | CH | N | $C-CF_3$ | C-I | CH | Me |
| 67 | 2 | $N-CH_2CF_3$ | N | N | CH | C-I | CH | Me |
| 68 | 1 | N-Me | $C-CF_3$ | CH | CH | C-I | CH | Me |
| 69 | 2 | N-Me | $C-CF_3$ | CH | CH | C-I | CH | Me |
| 70 | 1 | O | CH | C-i-Pr | N | C-I | CH | Me |
| 71 | 2 | O | CH | C-i-Pr | N | C-I | CH | Me |
| 72 | 1 | O | CH | C-i-Pr | N | C-Br | CH | Me |
| 73 | 2 | O | CH | C-i-Pr | N | C-Br | CH | Me |
| 74 | 1 | O | CH | C-i-Pr | N | CH | C-I | Me |
| 75 | 2 | O | CH | C-i-Pr | N | CH | C-I | Me |
| 76 | 1 | O | CH | C-i-Pr | N | CH | C-Br | Me |

(continued)

| Present compound | n | $Z^2$ | $X^1$ | $X^2$ | $X^4$ | $G^2$ | $G^3$ | $R^1$ |
|---|---|---|---|---|---|---|---|---|
| 77 | 2 | O | CH | C-i-Pr | N | CH | C-Br | Me |

Present compound 51: LCMS: 542 [M+H]+, RT = 1.69 min
Present compound 52: LCMS: 542 [M+H]+, RT = 1.80 min
Present compound 53: LCMS: 552 [M+H]+, RT = 1.66 min
Present compound 54: LCMS: 524 [M+H]+, RT = 1.43 min
Present compound 55: LCMS: 540 [M+H]+, RT = 1.60 min
Present compound 56: LCMS: 510 [M+H]+, RT = 1.63 min
Present compound 57: LCMS: 500 [M+H]+, RT = 1.60 min
Present compound 58: LCMS: 526 [M+H]+, RT = 1.41 min
Present compound 59: LCMS: 542 [M+H]+, RT = 1.57 min
Present compound 60: LCMS: 512 [M+H]+, RT = 1.49 min
Present compound 61: LCMS: 528 [M+H]+, RT = 1.71 min
Present compound 62: LCMS: 494 [M+H]+, RT = 1.40 min
Present compound 63: LCMS: 510 [M+H]+, RT = 1.57 min
Present compound 64: LCMS: 500 [M+H]+, RT = 1.44 min
Present compound 65: LCMS: 526 [M+H]+, RT = 1.44 min
Present compound 66: LCMS: 542 [M+H]+, RT = 1.60 min
Present compound 67: LCMS: 543 [M+H]+, RT = 1.64 min
Present compound 68: LCMS: 525 [M+H]+, RT = 1.48 min
Present compound 69: LCMS: 541 [M+H]+, RT = 1.65 min
Present compound 70: LCMS: 487 [M+H]+, RT = 1.62 min
Present compound 71: LCMS: 503 [M+H]+, RT = 1.78 min
Present compound 72: LCMS: 439 [M+H]+, RT = 1.59 min
Present compound 73: LCMS: 455 [M+H]+, RT = 1.75 min
Present compound 74: LCMS: 487 [M+H]+, RT = 1.64 min
Present compound 75: LCMS: 503 [M+H]+, RT = 1.79 min
Present compound 76: LCMS: 439 [M+H]+, RT = 1.60 min
Present compound 77: LCMS: 455 [M+H]+, RT = 1.77 min

Then, examples of the present compound produced in accordance with any of the production examples described in Examples and the production methods described in the present specification are shown below.

A compound represented by a formula (L-1) (hereinafter, referred to as a compound (L-1)),

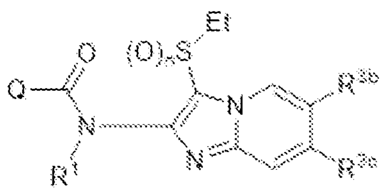

(L-1)

wherein n is 0, $R^1$ is a hydrogen atom, $R^{3b}$ is a hydrogen atom, $R^{3c}$ is a hydrogen atom, and Q is any of the substituents shown in [Table L1] to [Table L13] (hereinafter, referred to as a compound group SX1).

[Table L1]

| |
|---|
| furan-2-yl |

[Table L2]

| |
|---|
| 4-$CF_2H$-furan-2-yl |

| |
|---|
| isoxazol-5-yl |
| oxazol-5-yl |
| oxazol-2-yl |
| 1,2,4-oxadiazol-5-yl |
| 1,3,4-oxadiazol-2-yl |
| thiophen-2-yl |
| isothiazol-5-yl |
| thiazol-5-yl |
| thiazol-2-yl |
| 1,2,4-thiadiazol-5-yl |
| 1,3,4-thiadiazol-2-yl |
| 1-$CF_3$-1H-pyrrol-2-yl |
| 1-$CF_3$-1H-imidazol-2-yl |
| 1-$CF_3$-1H-imidazol-5-yl |
| 1-$CF_3$-1H-pyrazol-5-yl |
| 1-$CF_2H$-1H-pyrrol-2-yl |
| 1-$CF_2H$-1H-imidazol-2-yl |
| 1-$CF_2H$-1H-imidazol-5-yl |
| 1-$CF_2H$-1H-pyrazol-5-yl |
| 1-(fluoromethyl)-1H-pyrrol-2-yl |
| 1-(fluoromethyl)-1H-imidazol-2-yl |
| 1-(fluoromethyl)-1H-imidazol-5-yl |
| 1-(fluoromethyl)-1H-pyrazol-5-yl |
| 1-Me-1H-pyrrol-2-yl |
| 1-Me-1H-imidazol-2-yl |
| 1-Me-1H-imidazol-5-yl |
| 1-Me-1H-pyrazol-5-yl |
| 1-Me-1H-1,2,4-triazol-5-yl |
| 4-Me-4H-1,2,4-triazol-3-yl |
| 1-i-Pr-1H-pyrrol-2-yl |
| 1-i-Pr-1H-imidazol-2-yl |
| 1-i-Pr-1H-imidazol-5-yl |
| 1-i-Pr-1H-pyrazol-5-yl |
| 1-c-Pr-1H-pyrrol-2-yl |
| 1-c-Pr-1H-imidazol-2-yl |
| 1-c-Pr-1H-imidazol-5-yl |
| 1-c-Pr-1H-pyrazol-5-yl |
| 1-c-Pr-1H-1,2,4-triazol-5-yl |
| 4-c-Pr-4H-1,2,4-triazol-3-yl |
| 4-$CF_3$-furan-2-yl |
| 3-$CF_3$-isoxazol-5-yl |
| 2-$CF_3$-oxazol-5-yl |
| 5-$CF_3$-oxazol-2-yl |
| 3-$CF_3$-1,2,4-oxadiazol-5-yl |
| 5-$CF_3$-1,3,4-oxadiazol-2-yl |
| 4-$CF_3$-oxazol-2yl |
| 4-$CF_3$-oxazol-5-yl |
| 4-$CF_3$-isoxazol-5-yl |
| 3-$CF_3$-furan-2-yl |
| 5-$CF_3$-furan-2-yl |

| |
|---|
| 3-$CF_2H$-isoxazol-5-yl |
| 2-$CF_2H$-oxazol-5-yl |
| 5-$CF_2H$-oxazol-2-yl |
| 3-$CF_2H$-1,2,4-oxadiazol-5-yl |
| 5-$CF_2H$-1,3,4-oxadiazol-2-yl |
| 5-$CF_2H$-thiophen-2-yl |
| 3-$CF_2H$-isothiazol-5-yl |
| 4-$CF_2H$-oxazol-2yl |
| 4-$CF_2H$-oxazol-5-yl |
| 4-$CF_2H$-isoxazol-5-yl |
| 3-$CF_2H$-furan-2-yl |
| 5-$CF_2H$-furan-2-yl |
| 4-(fluoromethyl)furan-2-yl |
| 3-(fluoromethyl)isoxazol-5-yl |
| 2-(fluoromethyl)oxazol-5-yl |
| 5-(fluoromethyl)oxazol-2-yl |
| 4-(fluoromethyl)oxazol-2yl |
| 4-(fluoromethyl)oxazol-5-yl |
| 4-(fluoromethyl)isoxazol-5-yl |
| 3-(fluoromethyl)furan-2-yl |
| 5-(fluoromethyl)furan-2-yl |
| 3-Me-isoxazol-5-yl |
| 2-Me-oxazol-5-yl |
| 5-Me-oxazol-2-yl |
| 3-Me-1,2,4-oxadiazol-5-yl |
| 5-Me-1,3,4-oxadiazol-2-yl |
| 4-Me-oxazol-2yl |
| 4-Me-oxazol-5-yl |
| 4-Me-isoxazol-5-yl |
| 4-c-Pr-furan-2-yl |
| 3-c-Pr-isoxazol-5-yl |
| 2-c-Pr-oxazol-5-yl |
| 5-c-Pr-oxazol-2-yl |
| 3-c-Pr-1,2,4-oxadiazol-5-yl |
| 5-c-Pr-1,3,4-oxadiazol-2-yl |
| 4-c-Pr-oxazol-2yl |
| 4-c-Pr-oxazol-5-yl |
| 4-c-Pr-isoxazol-5-yl |
| 3-c-Pr-furan-2-yl |
| 5-c-Pr-furan-2-yl |
| 4-i-Pr-furan-2-yl |
| 3-i-Pr-isoxazol-5-yl |
| 2-i-Pr-oxazol-5-yl |
| 5-i-Pr-oxazol-2-yl |
| 4-i-Pr-oxazol-2-yl |
| 4-i-Pr-oxazol-5-yl |
| 4-i-Pr-isoxazol-5-yl |
| 3-i-Pr-furan-2-yl |
| 5-i-Pr-furan-2-yl |
| 3-Cl-isoxazol-5-yl |

[Table L3]

| |
|---|
| 3-Br-isoxazol-5-yl |

[Table L4]

| |
|---|
| 1-i-Pr-4-$CF_3$-1H-imidazol-5-yl |

| |
|---|
| 2-Cl-oxazol-5-yl |
| 2-Br-oxazol-5-yl |
| 5-Cl-oxazol-2-yl |
| 5-Br-oxazol-2-yl |
| 3-F-furan-2-yl |
| 3-Cl-furan-2-yl |
| 3-Br-furan-2-yl |
| 4-F-furan-2-yl |
| 4-Cl-furan-2-yl |
| 4-Br-furan-2-yl |
| 5-F-furan-2-yl |
| 5-Cl-furan-2-yl |
| 5-Br-furan-2-yl |
| 1-Me-3-$CF_3$-1H-pyrrol-2-yl |
| 1-Me-4-$CF_3$-1H-pyrrol-2-yl |
| 1-Me-5-$CF_3$-1H-pyrrol-2-yl |
| 1-Me-4-$CF_3$-1H-imidazol-2-yl |
| 1-Me-5-$CF_3$-1H-imidazol-2-yl |
| 1-Me-2-$CF_3$-1H-imidazol-5-yl |
| 1-Me-4-$CF_3$-1H-imidazol-5-yl |
| 1-Me-3-$CF_3$-1H-pyrazol-5-yl |
| 1-Me-4-$CF_3$-1H-pyrazol-5-yl |
| 1-Me-3-$CF_3$-1H-1,2,4-triazol-5-yl |
| 4-Me-5-$CF_3$-4H-1,2,4-triazol-3-yl |
| 3-$CF_2H$-1-Me-1H-pyrrol-2-yl |
| 4-$CF_2H$-1-Me-1H-pyrrole-2-yl |
| 5-$CF_2H$-1-Me-1H-pyrrol-2-yl |
| 4-$CF_2H$-1-Me-1H-imidazole-2-yl |
| 5-$CF_2H$-1-Me-1H-imidazol-2-yl |
| 2-$CF_2H$-1-Me-1H-imidazol-5-yl |
| 4-$CF_2H$-1-Me-1H-imidazol-5-yl |
| 3-$CF_2H$-1-Me-1H-pyrazol-5-yl |
| 4-$CF_2H$-1-Me-1H-pyrazol-5-yl |
| 3-$CF_2H$-1-Me-1H-1,2,4-triazol-5-yl |
| 5-$CF_2H$-4-Me-4H-1,2,4-triazol-3-yl |
| 3-(fluoromethyl)-1-Me-1H-pyrrol-2-yl |
| 4-(fluoromethyl)-1-Me-1H-pyrrol-2-yl |
| 5-(fluoromethyl)-1-Me-1H-pyrrol-2-yl |
| 4-(fluoromethyl)-1-Me-1H-imidazol-2-yl |
| 5-(fluoromethyl)-1-Me-1H-imidazol-2-yl |
| 2-(fluoromethyl)-1-Me-1H-imidazol-5-yl |
| 4-(fluoromethyl)-1-Me-1H-imidazol-5-yl |
| 3-(fluoromethyl)-1-Me-1H-pyrazol-5-yl |
| 4-(fluoromethyl)-1-Me-1H-pyrazol-5-yl |
| 1-i-Pr-3-$CF_3$-1H-pyrrol-2-yl |
| 1-i-Pr-4-$CF_3$-1H-pyrrol-2-yl |
| 1-i-Pr-5-$CF_3$-1H-pyrrol-2-yl |
| 1-i-Pr-4-$CF_3$-1H-imidazol-2-yl |
| 1-i-Pr-5-$CF_3$-1H-imidazol-2-yl |
| 1-i-Pr-2-$CF_3$-1H-imidazol-5-yl |

| |
|---|
| 1-i-Pr-3-$CF_3$-1H-pyrazol-5-yl |
| 1-i-Pr-4-$CF_3$-1H-pyrazol-5-yl |
| 3-$CF_2H$-1-i-Pr-1H-pyrrol-2-yl |
| 4-$CF_2H$-1-i-Pr-1H-pyrrol-2-yl |
| 5-$CF_2H$-1-i-Pr-1H-pyrrol-2-yl |
| 4-$CF_2H$-1-i-Pr-1H-imidazol-2-yl |
| 5-$CF_2H$-1-i-Pr-1H-imidazol-2-yl |
| 2-$CF_2H$-1-i-Pr-1H-imidazol-5-yl |
| 4-$CF_2H$-1-i-Pr-1H-imidazol-5-yl |
| 3-$CF_2H$-1-i-Pr-1H-pyrazol-5-yl |
| 4-$CF_2H$-1-i-Pr-1H-pyrazol-5-yl |
| 3-(fluoromethyl)-1-i-Pr-1H-pyrrol-2-yl |
| 4-(fluoromethyl)-1-i-Pr-1H-pyrrol-2-yl |
| 5-(fluoromethyl)-1-i-Pr-1H-pyrrol-2-yl |
| 4-(fluoromethyl)-1-i-Pr-1H-imidazol-2-yl |
| 5-(fluoromethyl)-1-i-Pr-1H-imidazol-2-yl |
| 2-(fluoromethyl)-1-i-Pr-1H-imidazol-5-yl |
| 4-(fluoromethyl)-1-i-Pr-1H-imidazol-5-yl |
| 3-(fluoromethyl)-1-i-Pr-1H-pyrazol-5-yl |
| 4-(fluoromethyl)-1-i-Pr-1H-pyrazol-5-yl |
| 1-c-Pr-3-$CF_3$-1H-pyrrol-2-yl |
| 1-c-Pr-4-$CF_3$-1H-pyrrol-2-yl |
| 1-c-Pr-5-$CF_3$-1H-pyrrol-2-yl |
| 1-c-Pr-4-$CF_3$-1H-imidazol-2-yl |
| 1-c-Pr-5-$CF_3$-1H-imidazol-2-yl |
| 1-c-Pr-2-$CF_3$-1H-imidazol-5-yl |
| 1-c-Pr-4-$CF_3$-1H-imidazol-5-yl |
| 1-c-Pr-3-$CF_3$-1H-pyrazol-5-yl |
| 1-c-Pr-4-$CF_3$-1H-pyrazol-5-yl |
| 1-c-Pr-3-$CF_2H$-1H-pyrrol-2-yl |
| 1-c-Pr-4-$CF_2H$-1H-pyrrol-2-yl |
| 1-c-Pr-5-$CF_2H$-1H-pyrrol-2-yl |
| 1-c-Pr-4-$CF_2H$-1H-imidazol-2-yl |
| 1-c-Pr-5-$CF_2H$-1H-imidazol-2-yl |
| 1-c-Pr-2-$CF_2H$-1H-imidazol-5-yl |
| 1-c-Pr-4-$CF_2H$-1H-imidazol-5-yl |
| 1-c-Pr-3-$CF_2H$-1H-pyrazol-5-yl |
| 1-c-Pr-4-$CF_2H$-1H-pyrazol-5-yl |
| 1-c-Pr-3-(fluoromethyl)-1H-pyrrol-2-yl |
| 1-c-Pr-4-(fluoromethyl)-1H-pyrrol-2-yl |
| 1-c-Pr-5-(fluoromethyl)-1H-pyrrol-2-yl |
| 1-c-Pr-4-(fluoromethyl)-1H-imidazol-2-yl |
| 1-c-Pr-5-(fluoromethyl)-1H-imidazol-2-yl |
| 1-c-Pr-2-(fluoromethyl)-1H-imidazol-5-yl |
| 1-c-Pr-4-(fluoromethyl)-1H-imidazol-5-yl |
| 1-c-Pr-3-(fluoromethyl)-1H-pyrazol-5-yl |
| 1-c-Pr-4-(fluoromethyl)-1H-pyrazol-5-yl |
| 1-Me-3-$OCF_3$-1H-pyrrol-2-yl |
| 1-Me-3-$OCF_3$-1H-pyrazol-5-yl |
| 1-i-Pr-3-$OCF_3$-1H-pyrrol-2-yl |

[Table L5]

[Table L6]

| |
|---|
| 1-i-Pr-3-OCF$_3$-1H-pyrazol-5-yl |
| 1-c-Pr-3-OCF$_3$-1H-pyrrol-2-yl |
| 1-c-Pr-3-OCF$_3$-1H-pyrazol-5-yl |
| 3-F-1-Me-1H-pyrrol-2-yl |
| 4-F-1-Me-1H-pyrrol-2-yl |
| 3-Cl-1-Me-1H-pyrrol-2-yl |
| 4-Cl-1-Me-1H-pyrrol-2-yl |
| 3-Br-1-Me-1H-pyrrol-2-yl |
| 4-Br-1-Me-1H-pyrrol-2-yl |
| 3-F-1-i-Pr-1H-pyrrol-2-yl |
| 4-F-1-i-Pr-1H-pyrrol-2-yl |
| 3-Cl-1-i-Pr-1H-pyrrol-2-yl |
| 4-Cl-1-i-Pr-1H-pyrrol-2-yl |
| 3-Br-1-i-Pr-1H-pyrrol-2-yl |
| 4-Br-1-i-Pr-1H-pyrrol-2-yl |
| 1-c-Pr-3-F-1H-pyrrol-2-yl |
| 1-c-Pr-4-F-1H-pyrrol-2-yl |
| 3-Cl-1-c-Pr-1H-pyrrol-2-yl |
| 4-Cl-1-c-Pr-1H-pyrrol-2-yl |
| 3-Br-1-c-Pr-1H-pyrrol-2-yl |
| 4-Br-1-c-Pr-1H-pyrrol-2-yl |
| 3-Me-1-CF$_3$-1H-pyrrol-2-yl |
| 4-Me-1-CF$_3$-1H-pyrrol-2-yl |
| 5-Me-1-CF$_3$-1H-pyrrol-2-yl |
| 5-Me-1-CF$_3$-1H-imidazol-2-yl |
| 4-Me-1-CF$_3$-1H-imidazol-2-yl |
| 2-Me-1-CF$_3$-1H-imidazol-5-yl |
| 4-Me-1-CF$_3$-1H-imidazol-5-yl |
| 3-Me-1-CF$_3$-1H-pyrazol-5-yl |
| 4-Me-1-CF$_3$-1H-pyrazol-5-yl |
| 1-CF$_2$H-3-Me-1H-pyrrol-2-yl |
| 1-CF$_2$H-4-Me- -1H-pyrrol-2-yl |
| 1-CF$_2$H-5-Me-1H-pyrrol-2-yl |
| 1-CF$_2$H-5-Me-1H-imidazol-2-yl |
| 1-CF$_2$H-4-Me-1H-imidazol-2-yl |
| 1-CF$_2$H-2-Me-1H-imidazol-5-yl |
| 1-CF$_2$H-4-Me-1H-imidazol-5-yl |
| 1-CF$_2$H-3-Me-1H-pyrazol-5-yl |
| 1-CF$_2$H-4-Me-1H-pyrazol-5-yl |
| 1-(fluoromethyl)-3-Me-1H-pvrrol-2-yl |
| 1-(fluoromethyl)-4-Me-1H-pyrrol-2-yl |
| 1-(fluoromethyl)-5-Me-1H-pyrrol-2-yl |
| 1-(fluoromethyl)-5-Me-1H-imidazol-2-yl |
| 1-(fluoromethyl)-4-Me-1H-imidazol-2-yl |
| 1-(fluoromethyl)-2-Me-1H-imidazol-5-yl |
| 1-(fluoromethyl)-4-Me-1H-imidazol-5-yl |
| 1-(fluoromethyl)-3-Me-1H-pyrazol-5-yl |
| 1-(fluoromethyl)-4-Me-1H-pyrazol-5-yl |
| 3-i-Pr-1-CF$_3$-1H-pyrrol-2-yl |
| 4-i-Pr-1-CF$_3$-1H-pyrrol-2-yl |
| 5-i-Pr-1-CF$_3$-1H-pyrrol-2-yl |

| |
|---|
| 5-i-Pr-1-$CF_3$-1H-imidazol-2-yl |
| 4-i-Pr-1-$CF_3$-1H-imidazol-2-yl |
| 2-i-Pr-1-$CF_3$-1H-imidazol-5-yl |
| 4-i-Pr-1-$CF_3$-1H-imidazol-5-yl |
| 3-i-Pr-1-$CF_3$-1H-pyrazol-5-yl |
| 4-i-Pr-1-$CF_3$-1H-pyrazol-5-yl |
| 1-$CF_2H$-3-i-Pr-1H-pyrrol-2-yl |
| 1-$CF_2H$-4-i-Pr-1H-pyrrol-2-yl |
| 1-$CF_2H$-5-i-Pr-1H-pyrrol-2-yl |
| 1-$CF_2H$-5-i-Pr-1H-imidazol-2-yl |
| 1-$CF_2H$-4-i-Pr-1H-imidazol-2-yl |
| 1-$CF_2H$-2-i-Pr-1H-imidazol-5-yl |
| 1-$CF_2H$-4-i-Pr-1H-imidazol-5-yl |
| 1-$CF_2H$-3-i-Pr-1H-pyrazol-5-yl |
| 1-$CF_2H$-4-i-Pr-1H-pyrazol-5-yl |
| 1-(fluoromethyl)-3-i-Pr-1H-pyrrol-2-yl |
| 1-(fluoromethyl)-4-i-Pr-1H-pyrrol-2-yl |
| 1-(fluoromethyl)-5-i-Pr-1H-pyrrol-2-yl |
| 1-(fluoromethyl)-5-i-Pr-1H-imidazol-2-yl |
| 1-(fluoromethyl)-4-i-Pr-1H-imidazol-2-yl |
| 1-(fluoromethyl)-2-i-Pr-1H-imidazol-5-yl |
| 1-(fluoromethyl)-4-i-Pr-1H-imidazol-5-yl |
| 1-(fluoromethyl)-3-i-Pr-1H-pyrazol-5-yl |
| 1-(fluoromethyl)-4-i-Pr-1H-pyrazol-5-yl |
| 3-c-Pr-1-$CF_3$-1H-pyrrol-2-yl |
| 4-c-Pr-1-$CF_3$-1H-pyrrol-2-yl |
| 5-c-Pr-1-$CF_3$-1H-pyrrol-2-yl |
| 5-c-Pr-1-$CF_3$-1H-imidazol-2-yl |
| 4-c-Pr-1-$CF_3$-1H-imidazol-2-yl |
| 2-c-Pr-1-$CF_3$-1H-imidazol-5-yl |
| 4-c-Pr-1-$CF_3$-1H-imidazol-5-yl |
| 3-c-Pr-1-$CF_3$-1H-pyrazol-5-yl |
| 4-c-Pr-1-$CF_3$-1H-pyrazol-5-yl |
| 3-c-Pr-1-$CF_2H$-1H-pyrrol-2-yl |
| 4-c-Pr-1-$CF_2H$-1H-pyrrol-2-yl |
| 5-c-Pr-1-$CF_2H$-1H-pyrrol-2-yl |
| 5-c-Pr-1-$CF_2H$-1H-imidazol-2-yl |
| 4-c-Pr-1-$CF_2H$-1H-imidazol-2-yl |
| 2-c-Pr-1-$CF_2H$-1H-imidazol-5-yl |
| 4-c-Pr-1-$CF_2H$-1H-imidazol-5-yl |
| 3-c-Pr-1-$CF_2H$-1H-pyrazol-5-yl |
| 4-c-Pr-1-$CF_2H$-1H-pyrazol-5-yl |
| 5-c-Pr-1-(fluoromethyl)-1H-imidazol-2-yl |
| 4-c-Pr-1-(fluoromethyl)-1H-imidazol-2-yl |
| 2-c-Pr-1-(fluoromethyl)-1H-imidazol-5-yl |
| 4-c-Pr-1-(fluoromethyl)-1H-imidazol-5-yl |
| 3-c-Pr-1-(fluoromethyl)-1H-pyrazol-5-yl |
| 4-c-Pr-1-(fluoromethyl)-1H-pyrazol-5-yl |
| furan-3-yl |
| thiophen-3-yl |
| isoxazol-3-yl |

[Table L7]

| |
|---|
| isoxazol-4-yl |
| oxazol-4-yl |
| isothiazol-3-yl |

(continued)

| |
|---|
| isothiazol-4-yl |
| thiazol-4-yl |
| 1,2,4-oxadiazol-3-yl |
| 1,2,5-oxadiazol-3-yl |
| 1,2,4-thiadiazol-3-yl |
| 1,2,5-thiadiazol-3-yl |
| 1-Me-1H-pyrrol-3-yl |
| 1-Me-1H-pyrazol-3-yl |
| 1-Me-1H-pyrazol-4-yl |
| 1-Me-1H-imidazol-4-yl |
| 2-Me-2H-1,2,3-triazol-4-yl |
| 1-Me-1H-1,2,4-triazol-3-yl |
| 1-Me-1H-1,2,3-triazol-4-yl |
| 1-i-Pr-1H-pyrrol-3-yl |
| 1-i-Pr-1H-pyrazol-3-yl |
| 1-i-Pr-1H-pyrazol-4-yl |
| 1-i-Pr-1H-imidazol-4-yl |
| 2-i-Pr-2H-1,2,3-triazol-4-yl |
| 1-i-Pr-1H-1,2,4-triazol-3-yl |
| 1-i-Pr-1H-1,2,3-triazol-4-yl |
| 1-c-Pr-1H-pyrrol-3-yl |
| 1-c-Pr-1H-pyrazol-3-yl |
| 1-c-Pr-1H-pyrazol-4-yl |
| 1-c-Pr-1H-imidazol-4-yl |
| 2-c-Pr-2H-1,2,3-triazol-4-yl |
| 1-c-Pr-1H-1,2,4-triazol-3-yl |
| 1-c-Pr-1H-1,2,3-triazol-4-yl |
| 1-$CF_3$-1H-pyrrol-3-yl |
| 1-$CF_3$-1H-pyrazol-3-yl |
| 1-$CF_3$-1H-pyrazol-4-yl |
| 1-$CF_3$-1H-imidazol-4-yl |
| 2-$CF_3$-2H-1,2,3-triazol-4-yl |
| 1-$CF_3$-1H-1,2,4-triazol-3-yl |
| 1-$CF_3$-1H-1,2,3-triazol-4-yl |
| 1-$CF_2H$-1H-pyrrol-3-yl |
| 1-$CF_2H$-1H-pyrazol-3-yl |
| 1-$CF_2H$-1H-pyrazol-4-yl |
| 1-$CF_2H$-1H-imidazol-4-yl |
| 2-$CF_2H$-2H-1,2,3-triazol-4-yl |
| 1-$CF_2H$-1H-1,2,4-triazol-3-yl |

(continued)

| |
|---|
| 1-$CF_2H$-1H-1,2,3-triazol-4-yl |
| 1-(fluoromethyl)-1H-pyrrol-3-yl |
| 1-(fluoromethyl)-1H-pyrazol-3-yl |
| 1-(fluoromethyl)-1H-pyrazol-4-yl |
| 1-(fluoromethyl)-1H-imidazol-4-yl |
| 4-Me-isoxazol-3-yl |
| 5-Me-isoxazol-3-yl |
| 3-Me-isoxazol-4-yl |

[Table L8]

| |
|---|
| 5-Me-isoxazol-4-yl |
| 2-Me-oxazol-4-yl |
| 5-Me-oxazol-4-yl |
| 5-Me-1,2,4-oxadiazol-3-yl |
| 4-Me-1,2,5-oxadiazol-3-yl |
| 2-i-Pr-furan-3-yl |
| 4-i-Pr-furan-3-yl |
| 5-i-Pr-furan-3-yl |
| 4-i-Pr-isoxazol-3-yl |
| 5-i-Pr-isoxazol-3-yl |
| 3-i-Pr-isoxazol-4-yl |
| 5-i-Pr-isoxazol-4-yl |
| 2-i-Pr-oxazol-4-yl |
| 5-i-Pr-oxazol-4-yl |
| 2-c-Pr-furan-3-yl |
| 4-c-Pr-furan-3-yl |
| 5-c-Pr-furan-3-yl |
| 4-c-Pr-isoxazol-3-yl |
| 5-c-Pr-isoxazol-3-yl |
| 3-c-Pr-isoxazol-4-yl |
| 5-c-Pr-isoxazol-4-yl |
| 2-c-Pr-oxazol-4-yl |
| 5-c-Pr-oxazol-4-yl |
| 2-$CF_3$-furan-3-yl |
| 4-$CF_3$-furan-3-yl |
| 5-$CF_3$-furan-3-yl |
| 4-$CF_3$-isoxazol-3-yl |
| 5-$CF_3$-isoxazol-3-yl |
| 3-$CF_3$-isoxazol-4-yl |
| 5-$CF_3$-isoxazol-4-yl |

(continued)

| |
|---|
| 2-$CF_3$-oxazol-4-yl |
| 5-$CF_3$-oxazol-4-yl |
| 4-$CF_3$-isothiazol-3-yl |
| 5-$CF_3$-isothiazol-3-yl |
| 3-$CF_3$-isothiazol-4-yl |
| 5-$CF_3$-isothiazol-4-yl |
| 5-$CF_3$-1,2,4-oxadiazol-3-yl |
| 4-$CF_3$-1,2,5-oxadiazol-3-yl |
| 5-$CF_3$-1,2,4-thiadiazol-3-yl |
| 4-$CF_3$-1,2,5-thiadiazol-3-yl |
| 2-$CF_2H$-furan-3-yl |
| 4-$CF_2H$-furan-3-yl |
| 5-$CF_2H$-furan-3-yl |
| 4-$CF_2H$-isoxazol-3-yl |
| 5-$CF_2H$-isoxazol-3-yl |
| 3-$CF_2H$-isoxazol-4-yl |
| 5-$CF_2H$-isoxazol-4-yl |
| 2-$CF_2H$-oxazol-4-yl |
| 5-$CF_2H$-oxazol-4-yl |
| 4-$CF_2H$-isothiazol-3-yl |
| 5-$CF_2H$-isothiazol-3-yl |

[Table L9]

| |
|---|
| 3-$CF_2H$-isothiazol-4-yl |
| 5-$CF_2H$-isothiazol-4-yl |
| 5-$CF_2H$-1,2,4-oxadiazol-3-yl |
| 4-$CF_2H$-1,2,5-oxadiazol-3-yl |
| 5-$CF_2H$-1,2,4-thiadiazol-3-yl |
| 4-$CF_2H$-1,2,5-thiadiazol-3-yl |
| 2-(fluoromethyl)-furan-3-yl |
| 4-(fluoromethyl)-furan-3-yl |
| 5-(fluoromethyl)-furan-3-yl |
| 4-(fluoromethyl)-isoxazol-3-yl |
| 5-(fluoromethyl)-isoxazol-3-yl |
| 3-(fluoromethyl)-isoxazol-4-yl |
| 5-(fluoromethyl)-isoxazol-4-yl |
| 2-(fluoromethyl)-oxazol-4-yl |
| 5-(fluoromethyl)-oxazol-4-yl |
| 2-F-furan-3-yl |
| 4-F-furan-3-yl |

(continued)

| |
|---|
| 5-F-furan-3-yl |
| 2-Cl-furan-3-yl |
| 4-Cl-furan-3-yl |
| 5-Cl-furan-3-yl |
| 2-Br-furan-3-yl |
| 4-Br-furan-3-yl |
| 5-Br-furan-3-yl |
| 2-c-Pr-1-Me-1H-pyrrol-3-yl |
| 4-c-Pr-1-Me-1H-pyrrol-3-yl |
| 5-c-Pr-1-Me-1H-pyrrol-3-yl |
| 4-c-Pr-1-Me-1H-pyrazol-3-yl |
| 5-c-Pr-1-Me-1H-pyrazol-3-yl |
| 3-c-Pr-1-Me-1H-pyrazol-4-yl |
| 5-c-Pr-1-Me-1H-pyrazol-4-yl |
| 2-c-Pr-1-Me-1H-imidazol-4-yl |
| 5-c-Pr-1-Me-1H-imidazol-4-yl |
| 5-c-Pr-2-Me-2H-1,2,3-triazol-4-yl |
| 5-c-Pr-1-Me-1H-1,2,4-triazol-3-yl |
| 5-c-Pr-1-Me-1H-1,2,3-triazol-4-yl |
| 1,2-dimethyl-1H-pyrrol-3-yl |
| 1,4-dimethyl-1H-pyrrol-3-yl |
| 1,5-dimethyl-1H-pyrrol-3-yl |
| 1,4-dimethyl-1H-pyrazol-3-yl |
| 1,5-dimethyl-1H-pyrazol-3-yl |
| 1,3-dimethyl-1H-pyrazol-4-yl |
| 1,5-dimethyl-1H-pyrazol-4-yl |
| 1,2-dimethyl-1H-imidazol-4-yl |
| 1,5-dimethyl-1H-imidazol-4-yl |
| 2,5-dimethyl-2H-1,2,3-triazol-4-yl |
| 1,5-dimethyl-1H-1,2,4-triazol-3-yl |
| 1,5-dimethyl-1H-1,2,3-triazol-4-yl |
| 2-i-Pr-1-Me-1H-pyrrol-3-yl |
| 4-i-Pr-1-Me-1H-pyrrol-3-yl |
| 5-i-Pr-1-Me-1H-pyrrol-3-yl |

[Table L10]

| |
|---|
| 4-i-Pr-1-Me-1H-pyrazol-3-yl |
| 5-i-Pr-1-Me-1H-pyrazol-3-yl |
| 3-i-Pr-1-Me-1H-pyrazol-4-yl |
| 5-i-Pr-1-Me-1H-pyrazol-4-yl |

(continued)

| |
|---|
| 2-i-Pr-1-Me-1H-imidazol-4-yl |
| 5-i-Pr-1-Me-1H-imidazol-4-yl |
| 5-i-Pr-2-Me-2H-1,2,3-triazol-4-yl |
| 5-i-Pr-1-Me-1H-1,2,4-triazol-3-yl |
| 5-i-Pr-1-Me-1H-1,2,3-triazol-4-yl |
| 1-Me-2-$CF_3$-1H-pyrrol-3-yl |
| 1-Me-4-$CF_3$-1H-pyrrol-3-yl |
| 1-Me-5-$CF_3$-1H-pyrrol-3-yl |
| 1-Me-4-$CF_3$-1H-pyrazol-3-yl |
| 1-Me-5-$CF_3$-1H-pyrazol-3-yl |
| 1-Me-3-$CF_3$-1H-pyrazol-4-yl |
| 1-Me-5-$CF_3$-1H-pyrazol-4-yl |
| 1-Me-2-$CF_3$-1H-imidazol-4-yl |
| 1-Me-5-$CF_3$-1H-imidazol-4-yl |
| 2-Me-5-$CF_3$-2H-1,2,3-triazol-4-yl |
| 1-Me-5-$CF_3$-1H-1,2,4-triazol-3-yl |
| 1-Me-5-$CF_3$-1H-1,2,3-triazol-4-yl |
| 2-$CF_2$H-1-Me-1H-pyrrol-3-yl |
| 4-$CF_2$H-1-Me-1H-pyrrol-3-yl |
| 5-$CF_2$H-1-Me-1H-pyrrol-3-yl |
| 4-$CF_2$H-1-Me-1H-pyrazol-3-yl |
| 5-$CF_2$H-1-Me-1H-pyrazol-3-yl |
| 3-$CF_2$H-1-Me-1H-pyrazol-4-yl |
| 5-$CF_2$H-1-Me-1H-pyrazol-4-yl |
| 2-$CF_2$H-1-Me-1H-imidazol-4-yl |
| 5-$CF_2$H-1-Me-1H-imidazol-4-yl |
| 5-$CF_2$H-2-Me-2H-1,2,3-triazol-4-yl |
| 5-$CF_2$H-1-Me-1H-1,2,4-triazol-3-yl |
| 5-$CF_2$H-1-Me-1H-1,2,3-triazol-4-yl |
| 2-fluoromethyl-1-Me-1H-pyrrol-3-yl |
| 4-fluoromethyl-1-Me-1H-pyrrol-3-yl |
| 5-fluoromethyl-1-Me-1H-pyrrol-3-yl |
| 4-fluoromethyl-1-Me-1H-pyrazol-3-yl |
| 5-fluoromethyl-1-Me-1H-pyrazol-3-yl |
| 3-fluoromethyl-1-Me-1H-pyrazol-4-yl |
| 5-fluoromethyl-1-Me-1H-pyrazol-4-yl |
| 2-fluoromethyl-1-Me-1H-imidazol-4-yl |
| 5-fluoromethyl-1-Me-1H-imidazol-4-yl |
| 4-i-Pr-1-$CF_3$-1H-pyrazol-3-yl |
| 5-i-Pr-1-$CF_3$-1H-pyrazol-3-yl |

(continued)

| |
|---|
| 3-i-Pr-1-$CF_3$-1H-pyrazol-4-yl |
| 5-i-Pr-1-$CF_3$-1H-pyrazol-4-yl |
| 2-i-Pr-1-$CF_3$-1H-imidazol-4-yl |
| 5-i-Pr-1-$CF_3$-1H-imidazol-4-yl |
| 2-c-Pr-1-$CF_3$-1H-pyrrol-3-yl |
| 4-c-Pr-1-$CF_3$-1H-pyrrol-3-yl |
| 5-c-Pr-1-$CF_3$-1H-pyrrol-3-yl |

[Table L11]

| |
|---|
| 4-c-Pr-1-$CF_3$-1H-pyrrol-3-yl |
| 5-c-Pr-1-$CF_3$-1H-pyrrol-3-yl |
| 4-c-Pr-1-$CF_3$-1H-pyrazol-3-yl |
| 5-c-Pr-1-$CF_3$-1H-pyrazol-3-yl |
| 3-c-Pr-1-$CF_3$-1H-pyrazol-4-yl |
| 5-c-Pr-1-$CF_3$-1H-pyrazol-4-yl |
| 2-c-Pr-1-$CF_3$-1H-imidazol-4-yl |
| 5-c-Pr-1-$CF_3$-1H-imidazol-4-yl |
| 2-Me-1-$CF_3$-1H-pyrrol-3-yl |
| 4-Me-1-$CF_3$-1H-pyrrol-3-yl |
| 5-Me-1-$CF_3$-1H-pyrrol-3-yl |
| 4-Me-1-$CF_3$-1H-pyrazol-3-yl |
| 5-Me-1-$CF_3$-1H-pyrazol-3-yl |
| 3-Me-1-$CF_3$-1H-pyrazol-4-yl |
| 5-Me-1-$CF_3$-1H-pyrazol-4-yl |
| 2-Me-1-$CF_3$-1H-imidazol-4-yl |
| 5-Me-1-$CF_3$-1H-imidazol-4-yl |
| 5-Me-2-$CF_3$-2H-1,2,3-triazol-4-yl |
| 5-Me-1-$CF_3$-1H-1,2,4-triazol-3-yl |
| 5-Me-1-$CF_3$-1H-1,2,3-triazol-4-yl |
| 2-i-Pr-1-$CF_3$-1H-pyrrol-3-yl |
| 4-i-Pr-1-$CF_3$-1H-pyrrol-3-yl |
| 5-i-Pr-1-$CF_3$-1H-pyrrol-3-yl |
| 4-i-Pr-1-$CF_3$-1H-pyrazol-3-yl |
| 5-i-Pr-1-$CF_3$-1H-pyrazol-3-yl |
| 3-i-Pr-1-$CF_3$-1H-pyrazol-4-yl |
| 5-i-Pr-1-$CF_3$-1H-pyrazol-4-yl |
| 2-i-Pr-1-$CF_3$-1H-imidazol-4-yl |
| 5-i-Pr-1-$CF_3$-1H-imidazol-4-yl |
| 2-c-Pr-1-$CF_2H$-1H-pyrrol-3-yl |
| 4-c-Pr-1-$CF_2H$-1H-pyrrol-3-yl |

(continued)

| |
|---|
| 5-c-Pr-1-$CF_2$H-1H-pyrrol-3-yl |
| 4-c-Pr-1-$CF_2$H-1H-pyrazol-3-yl |
| 5-c-Pr-1-$CF_2$H-1H-pyrazol-3-yl |
| 3-c-Pr-1-$CF_2$H-1H-pyrazol-4-yl |
| 5-c-Pr-1-$CF_2$H-1H-pyrazol-4-yl |
| 2-c-Pr-1-$CF_2$H-1H-imidazol-4-yl |
| 5-c-Pr-1-$CF_2$H-1H-imidazol-4-yl |
| 1-$CF_2$H-2-Me-1H-pyrrol-3-yl |
| 1-$CF_2$H-4-Me-1H-pyrrol-3-yl |
| 1-$CF_2$H-5-Me-1H-pyrrol-3-yl |
| 1-$CF_2$H-4-Me-1H-pyrazol-3-yl |
| 1-$CF_2$H-5-Me-1H-pyrazol-3-yl |
| 1-$CF_2$H-3-Me-1H-pyrazol-4-yl |
| 1-$CF_2$H-5-Me-1H-pyrazol-4-yl |
| 1-$CF_2$H-2-Me-1H-imidazol-4-yl |
| 1-$CF_2$H-5-Me-1H-imidazol-4-yl |
| 1-$CF_2$H-2-i-Pr-1H-pyrrol-3-yl |
| 1-$CF_2$H-4-i-Pr-1H-pyrrol-3-yl |
| 1-$CF_2$H-5-i-Pr-1H-pyrrol-3-yl |
| 1-$CF_2$H-4-i-Pr-1H-pyrazol-3-yl |

[Table L12]

| |
|---|
| 1-$CF_2$H-5-i-Pr-1H-pyrazol-3-yl |
| 1-$CF_2$H-3-i-Pr-1H-pyrazol-4-yl |
| 1-$CF_2$H-5-i-Pr-1H-pyrazol-4-yl |
| 1-$CF_2$H-2-i-Pr-1H-imidazol-4-yl |
| 1-$CF_2$H-5-i-Pr-1H-imidazol-4-yl |
| 2-c-Pr-1-fluoromethyl-1H-pyrrol-3-yl |
| 4-c-Pr-1-fluoromethyl-1H-pyrrol-3-yl |
| 5-c-Pr-1-fluoromethyl-1H-pyrrol-3-yl |
| 4-c-Pr-1-fluoromethyl-1H-pyrazol-3-yl |
| 5-c-Pr-1-fluoromethyl-1H-pyrazol-3-yl |
| 3-c-Pr-1-fluoromethyl-1H-pyrazol-4-yl |
| 5-c-Pr-1-fluoromethyl-1H-pyrazol-4-yl |
| 2-c-Pr-1-fluoromethyl-1H-imidazol-4-yl |
| 5-c-Pr-1-fluoromethyl-1H-imidazol-4-yl |
| 1-fluoromethyl-2-Me-1H-pyrrol-3-yl |
| 1-fluoromethyl-4-Me-1H-pyrrol-3-yl |
| 1-fluoromethyl-5-Me-1H-pyrrol-3-yl |
| 1-fluoromethyl-4-Me-1H-pyrazol-3-yl |

(continued)

| |
|---|
| 1-fluoromethyl-5-Me-1H-pyrazol-3-yl |
| 1-fluoromethyl-3-Me-1H-pyrazol-4-yl |
| 1-fluoromethyl-5-Me-1H-pyrazol-4-yl |
| 1-fluoromethyl-2-Me-1H-imidazol-4-yl |
| 1-fluoromethyl-5-Me-1H-imidazol-4-yl |
| 1-fluoromethyl-2-i-Pr-1H-pyrrol-3-yl |
| 1-fluoromethyl-4-i-Pr-1H-pyrrol-3-yl |
| 1-fluoromethyl-5-i-Pr-1H-pyrrol-3-yl |
| 1-fluoromethyl-4-i-Pr-1H-pyrazol-3-yl |
| 1-fluoromethyl-5-i-Pr-1H-pyrazol-3-yl |
| 1-fluoromethyl-3-i-Pr-1H-pyrazol-4-yl |
| 1-fluoromethyl-5-i-Pr-1H-pyrazol-4-yl |
| 1-fluoromethyl-2-i-Pr-1H-imidazol-4-yl |
| 1-fluoromethyl-5-i-Pr-1H-imidazol-4-yl |
| 4-F-1-Me-1H-pyrrol-3-yl |
| 5-F-1-Me-1H-pyrrol-3-yl |
| 4-Cl-1-Me-1H-pyrrol-3-yl |
| 5-Cl-1-Me-1H-pyrrol-3-yl |
| 4-Br-1-Me-1H-pyrrol-3-yl |
| 5-Br-1-Me-1H-pyrrol-3-yl |
| 4-F-1-Me-1H-pyrazol-3-yl |
| 5-F-1-Me-1H-pyrazol-3-yl |
| 4-Cl-1-Me-1H-pyrazol-3-yl |
| 5-Cl-1-Me-1H-pyrazol-3-yl |
| 4-Br-1-Me-1H-pyrazol-3-yl |
| 5-Br-1-Me-1H-pyrazol-3-yl |
| 1-Me-4-$OCF_3$-1H-pyrazol-3-yl |
| 1-Me-5-$OCF_3$-1H-pyrazol-3-yl |
| 1-Me-3-$OCF_3$-1H-pyrazol-4-yl |
| 1-Me-5-$OCF_3$-1H-pyrazol-4-yl |
| 1-Me-3-$OCF_3$-1H-pyrazol-5-yl |
| 1-Me-4-$OCF_3$-1H-pyrazol-5-yl |
| 4-$OCF_2H$-1-Me-1H-pyrazol-3-yl |

[Table L13]

| |
|---|
| 5-$OCF_2H$-1-Me-1H-pyrazol-3-yl |
| 3-$OCF_2H$-1-Me-1H-pyrazol-4-yl |
| 5-$OCF_2H$-1-Me-1H-pyrazol-4-yl |
| 3-$OCF_2H$-1-Me-1H-pyrazol-5-yl |
| 4-$OCF_2H$-1-Me-1H-pyrazol-5-yl |

(continued)

| |
|---|
| 2-F-1-Me-1H-imidazol-4-yl |
| 2-Cl-1-Me-1H-imidazol-4-yl |
| 2-Br-1-Me-1H-imidazol-4-yl |

A compound wherein in the compound (L-1), n is 0, $R^1$ is a methyl group, $R^{3b}$ is a hydrogen atom, $R^{3c}$ is a hydrogen atom, and Q is any of the substituents shown in [Table L1] to [Table L13] (hereinafter, referred to as a compound group SX4).

A compound wherein in the compound (L-1), n is 1, $R^1$ is a methyl group, $R^{3b}$ is a hydrogen atom, $R^{3c}$ is a hydrogen atom, and Q is any of the substituents shown in [Table L1] to [Table L13] (hereinafter, referred to as a compound group SX5).

A compound wherein in the compound (L-1), n is 2, $R^1$ is a methyl group, $R^{3b}$ is a hydrogen atom, $R^{3c}$ is a hydrogen atom, and Q is any of the substituents shown in [Table L1] to [Table L13] (hereinafter, referred to as a compound group SX6).

A compound wherein in the compound (L-1), n is 0, $R^1$ is an ethyl group, $R^{3b}$ is a hydrogen atom, $R^{3c}$ is a hydrogen atom, and Q is any of the substituents shown in [Table L1] to [Table L13] (hereinafter, referred to as a compound group SX7).

A compound wherein in the compound (L-1), n is 1, $R^1$ is an ethyl group, $R^{3b}$ is a hydrogen atom, $R^{3c}$ is a hydrogen atom, and Q is any of the substituents shown in [Table L1] to [Table L13] (hereinafter, referred to as a compound group SX8).

A compound wherein in the compound (L-1), n is 2, $R^1$ is an ethyl group, $R^{3b}$ is a hydrogen atom, $R^{3c}$ is a hydrogen atom, and Q is any of the substituents shown in [Table L1] to [Table L13] (hereinafter, referred to as a compound group SX9).

A compound wherein in the compound (L-1), n is 0, $R^1$ is an isopropyl group, $R^{3b}$ is a hydrogen atom, $R^{3c}$ is a hydrogen atom, and Q is any of the substituents shown in [Table L1] to [Table L13] (hereinafter, referred to as a compound group SX10).

A compound wherein in the compound (L-1), n is 1, $R^1$ is an isopropyl group, $R^{3b}$ is a hydrogen atom, $R^{3c}$ is a hydrogen atom, and Q is any of the substituents shown in [Table L1] to [Table L13] (hereinafter, referred to as a compound group SX11).

A compound wherein in the compound (L-1), n is 2, $R^1$ is an isopropyl group, $R^{3b}$ is a hydrogen atom, $R^{3c}$ is a hydrogen atom, and Q is any of the substituents shown in [Table L1] to [Table L13] (hereinafter, referred to as a compound group SX12).

A compound wherein in the compound (L-1), n is 0, $R^1$ is a methylcyano group, $R^{3b}$ is a hydrogen atom, $R^{3c}$ is a hydrogen atom, and Q is any of the substituents shown in [Table L1] to [Table L13] (hereinafter, referred to as a compound group SX13).

A compound wherein in the compound (L-1), n is 1, $R^1$ is a methylcyano group, $R^{3b}$ is a hydrogen atom, $R^{3c}$ is a hydrogen atom, and Q is any of the substituents shown in [Table L1] to [Table L13] (hereinafter, referred to as a compound group SX14).

A compound wherein in the compound (L-1), n is 2, $R^1$ is a methylcyano group, $R^{3b}$ is a hydrogen atom, $R^{3c}$ is a hydrogen atom, and Q is any of the substituents shown in [Table L1] to [Table L13] (hereinafter, referred to as a compound group SX15).

A compound wherein in the compound (L-1), n is 0, $R^1$ is a benzyl group, $R^{3b}$ is a hydrogen atom, $R^{3c}$ is a hydrogen atom, and Q is any of the substituents shown in [Table L1] to [Table L13] (hereinafter, referred to as a compound group SX16).

A compound wherein in the compound (L-1), n is 1, $R^1$ is a benzyl group, $R^{3b}$ is a hydrogen atom, $R^{3c}$ is a hydrogen atom, and **Q** is any of the substituents shown in [Table L1] to [Table L13] (hereinafter, referred to as a compound group SX17).

**A** compound wherein in the compound (L-1), n is 2, $R^1$ is a benzyl group, $R^{3b}$ is a hydrogen atom, $R^{3c}$ is a hydrogen atom, and **Q** is any of the substituents shown in [Table L1] to [Table L13] (hereinafter, referred to as a compound group SX18).

A compound wherein in the compound (L-1), n is 0, $R^1$ is a methyl group, $R^{3b}$ is a fluorine atom, $R^{3c}$ is a hydrogen atom, and Q is any of the substituents shown in [Table L1] to [Table L13] (hereinafter, referred to as a compound group SX22).

A compound wherein in the compound (L-1), n is 1, $R^1$ is a methyl group, $R^{3b}$ is a fluorine atom, $R^{3c}$ is a hydrogen atom, and Q is any of the substituents shown in [Table L1] to [Table L13] (hereinafter, referred to as a compound group SX23).

A compound wherein in the compound (L-1), n is 2, $R^1$ is a methyl group, $R^{3b}$ is a fluorine atom, $R^{3c}$ is a hydrogen atom, and Q is any of the substituents shown in [Table L1] to [Table L13] (hereinafter, referred to as a compound group SX24).

A compound wherein in the compound (L-1), n is 0, $R^1$ is an ethyl group, $R^{3b}$ is a fluorine atom, $R^{3c}$ is a hydrogen atom, and **Q** is any of the substituents shown in [Table L1] to [Table L13] (hereinafter, referred to as a compound group SX25).

**A** compound wherein in the compound (L-1), n is 1, $R^1$ is an ethyl group, $R^{3b}$ is a fluorine atom, $R^{3c}$ is a hydrogen atom, and **Q** is any of the substituents shown in [Table L1] to [Table L13] (hereinafter, referred to as a compound group SX26).

A compound wherein in the compound (L-1), n is 2, $R^1$ is an ethyl group, $R^{3b}$ is a fluorine atom, $R^{3c}$ is a hydrogen atom, and Q is any of the substituents shown in [Table L1] to [Table L13] (hereinafter, referred to as a compound group SX27).

A compound wherein in the compound (L-1), n is 0, $R^1$ is an isopropyl group, $R^{3b}$ is a fluorine atom, $R^{3c}$ is a hydrogen atom, and Q is any of the substituents shown in [Table L1] to [Table L13] (hereinafter, referred to as a compound group SX28).

A compound wherein in the compound (L-1), n is 1, $R^1$ is an isopropyl group, $R^{3b}$ is a fluorine atom, $R^{3c}$ is a hydrogen atom, and Q is any of the substituents shown in [Table L1] to [Table L13] (hereinafter, referred to as a compound group SX29).

A compound wherein in the compound (L-1), n is 2, $R^1$ is an isopropyl group, $R^{3b}$ is a fluorine atom, $R^{3c}$ is a hydrogen atom, and Q is any of the substituents shown in [Table L1] to [Table L13] (hereinafter, referred to as a compound group SX30).

A compound wherein in the compound (L-1), n is 0, $R^1$ is a methylcyano group, $R^{3b}$ is a fluorine atom, $R^{3c}$ is a hydrogen atom, and Q is any of the substituents shown in [Table L1] to [Table L13] (hereinafter, referred to as a compound group SX31).

A compound wherein in the compound (L-1), n is 1, $R^1$ is a methylcyano group, $R^{3b}$ is a fluorine atom, $R^{3c}$ is a hydrogen atom, and Q is any of the substituents shown in [Table L1] to [Table L13] (hereinafter, referred to as a compound group SX32).

A compound wherein in the compound (L-1), n is 2, $R^1$ is a methylcyano group, $R^{3b}$ is a fluorine atom, $R^{3c}$ is a hydrogen atom, and Q is any of the substituents shown in [Table L1] to [Table L13] (hereinafter, referred to as a compound group SX33).

A compound wherein in the compound (L-1), n is 0, $R^1$ is a benzyl group, $R^{3b}$ is a fluorine atom, $R^{3c}$ is a hydrogen atom, and Q is any of the substituents shown in [Table L1] to [Table L13] (hereinafter, referred to as a compound group SX34).

A compound wherein in the compound (L-1), n is 1, $R^1$ is a benzyl group, $R^{3b}$ is a fluorine atom, $R^{3c}$ is a hydrogen atom, and **Q** is any of the substituents shown in [Table L1] to [Table L13] (hereinafter, referred to as a compound group SX35).

**A** compound wherein in the compound (L-1), n is 2, $R^1$ is a benzyl group, $R^{3b}$ is a fluorine atom, $R^{3c}$ is a hydrogen atom, and **Q** is any of the substituents shown in [Table L1] to [Table L13] (hereinafter, referred to as a compound group SX36).

A compound wherein in the compound (L-1), n is 0, $R^1$ is a methyl group, $R^{3b}$ is a chlorine atom, $R^{3c}$ is a hydrogen atom, and Q is any of the substituents shown in [Table L1] to [Table L13] (hereinafter, referred to as a compound group SX40).

A compound wherein in the compound (L-1), n is 1, $R^1$ is a methyl group, $R^{3b}$ is a chlorine atom, $R^{3c}$ is a hydrogen atom, and Q is any of the substituents shown in [Table L1] to [Table L13] (hereinafter, referred to as a compound group **SX41).**

A compound wherein in the compound (L-1), n is 2, $R^1$ is a methyl group, $R^{3b}$ is a chlorine atom, $R^{3c}$ is a hydrogen atom, and Q is any of the substituents shown in [Table L1] to [Table L13] (hereinafter, referred to as a compound group SX42).

A compound wherein in the compound (L-1), n is 0, $R^1$ is an ethyl group, $R^{3b}$ is a chlorine atom, $R^{3c}$ is a hydrogen atom, and Q is any of the substituents shown in [Table L1] to [Table L13] (hereinafter, referred to as a compound group SX43).

A compound wherein in the compound (L-1), n is 1, $R^1$ is an ethyl group, $R^{3b}$ is a chlorine atom, $R^{3c}$ is a hydrogen atom, and Q is any of the substituents shown in [Table L1] to [Table L13] (hereinafter, referred to as a compound group SX44).

A compound wherein in the compound (L-1), n is 2, $R^1$ is an ethyl group, $R^{3b}$ is a chlorine atom, $R^{3c}$ is a hydrogen atom, and Q is any of the substituents shown in [Table L1] to [Table L13] (hereinafter, referred to as a compound group SX45).

A compound wherein in the compound (L-1), n is 0, $R^1$ is an isopropyl group, $R^{3b}$ is a chlorine atom, $R^{3c}$ is a

hydrogen atom, and Q is any of the substituents shown in [Table L1] to [Table L13] (hereinafter, referred to as a compound group SX46).

A compound wherein in the compound (L-1), n is 1, $R^1$ is an isopropyl group, $R^{3b}$ is a chlorine atom, $R^{3c}$ is a hydrogen atom, and Q is any of the substituents shown in [Table L1] to [Table L13] (hereinafter, referred to as a compound group SX47).

A compound wherein in the compound (L-1), n is 2, $R^1$ is an isopropyl group, $R^{3b}$ is a chlorine atom, $R^{3c}$ is a hydrogen atom, and Q is any of the substituents shown in [Table L1] to [Table L13] (hereinafter, referred to as a compound group SX48).

A compound wherein in the compound (L-1), n is 0, $R^1$ is a methylcyano group, $R^{3b}$ is a chlorine atom, $R^{3c}$ is a hydrogen atom, and Q is any of the substituents shown in [Table L1] to [Table L13] (hereinafter, referred to as a compound group SX49).

A compound wherein in the compound (L-1), n is 1, $R^1$ is a methylcyano group, $R^{3b}$ is a chlorine atom, $R^{3c}$ is a hydrogen atom, and Q is any of the substituents shown in [Table L1] to [Table L13] (hereinafter, referred to as a compound group SX50).

A compound wherein in the compound (L-1), n is 2, $R^1$ is a methylcyano group, $R^{3b}$ is a chlorine atom, $R^{3c}$ is a hydrogen atom, and Q is any of the substituents shown in [Table L1] to [Table L13] (hereinafter, referred to as a compound group SX51).

A compound wherein in the compound (L-1), n is 0, $R^1$ is a benzyl group, $R^{3b}$ is a chlorine atom, $R^{3c}$ is a hydrogen atom, and Q is any of the substituents shown in [Table L1] to [Table L13] (hereinafter, referred to as a compound group SX52).

A compound wherein in the compound (L-1), n is 1, $R^1$ is a benzyl group, $R^{3b}$ is a chlorine atom, $R^{3c}$ is a hydrogen atom, and Q is any of the substituents shown in [Table L1] to [Table L13] (hereinafter, referred to as a compound group SX53).

A compound wherein in the compound (L-1), n is 2, $R^1$ is a benzyl group, $R^{3b}$ is a chlorine atom, $R^{3c}$ is a hydrogen atom, and Q is any of the substituents shown in [Table L1] to [Table L13] (hereinafter, referred to as a compound group SX54).

A compound wherein in the compound (L-1), n is 0, $R^1$ is a methyl group, $R^{3b}$ is a bromine atom, $R^{3c}$ is a hydrogen atom, and Q is any of the substituents shown in [Table L1] to [Table L13] (hereinafter, referred to as a compound group SX58).

A compound wherein in the compound (L-1), n is 1, $R^1$ is a methyl group, $R^{3b}$ is a bromine atom, $R^{3c}$ is a hydrogen atom, and Q is any of the substituents shown in [Table L1] to [Table L13] (hereinafter, referred to as a compound group SX59).

A compound wherein in the compound (L-1), n is 2, $R^1$ is a methyl group, $R^{3b}$ is a bromine atom, $R^{3c}$ is a hydrogen atom, and Q is any of the substituents shown in [Table L1] to [Table L13] (hereinafter, referred to as a compound group SX60).

A compound wherein in the compound (L-1), n is 0, $R^1$ is an ethyl group, $R^{3b}$ is a bromine atom, $R^{3c}$ is a hydrogen atom, and Q is any of the substituents shown in [Table L1] to [Table L13] (hereinafter, referred to as a compound group SX61).

A compound wherein in the compound (L-1), n is 1, $R^1$ is an ethyl group, $R^{3b}$ is a bromine atom, $R^{3c}$ is a hydrogen atom, and Q is any of the substituents shown in [Table L1] to [Table L13] (hereinafter, referred to as a compound group SX62).

A compound wherein in the compound (L-1), n is 2, $R^1$ is an ethyl group, $R^{3b}$ is a bromine atom, $R^{3c}$ is a hydrogen atom, and Q is any of the substituents shown in [Table L1] to [Table L13] (hereinafter, referred to as a compound group SX63).

A compound wherein in the compound (L-1), n is 0, $R^1$ is an isopropyl group, $R^{3b}$ is a bromine atom, $R^{3c}$ is a hydrogen atom, and Q is any of the substituents shown in [Table L1] to [Table L13] (hereinafter, referred to as a compound group SX64).

A compound wherein in the compound (L-1), n is 1, $R^1$ is an isopropyl group, $R^{3b}$ is a bromine atom, $R^{3c}$ is a hydrogen atom, and Q is any of the substituents shown in [Table L1] to [Table L13] (hereinafter, referred to as a compound group SX65).

A compound wherein in the compound (L-1), n is 2, $R^1$ is an isopropyl group, $R^{3b}$ is a bromine atom, $R^{3c}$ is a hydrogen atom, and Q is any of the substituents shown in [Table L1] to [Table L13] (hereinafter, referred to as a compound group SX66).

A compound wherein in the compound (L-1), n is 0, $R^1$ is a methylcyano group, $R^{3b}$ is a bromine atom, $R^{3c}$ is a hydrogen atom, and Q is any of the substituents shown in [Table L1] to [Table L13] (hereinafter, referred to as a compound group SX67).

A compound wherein in the compound (L-1), n is 1, $R^1$ is a methylcyano group, $R^{3b}$ is a bromine atom, $R^{3c}$ is a hydrogen atom, and Q is any of the substituents shown in [Table L1] to [Table L13] (hereinafter, referred to as a compound

group SX68).

A compound wherein in the compound (L-1), n is 2, $R^1$ is a methylcyano group, $R^{3b}$ is a bromine atom, $R^{3c}$ is a hydrogen atom, and Q is any of the substituents shown in [Table L1] to [Table L13] (hereinafter, referred to as a compound group SX69).

A compound wherein in the compound (L-1), n is 0, $R^1$ is a benzyl group, $R^{3b}$ is a bromine atom, $R^{3c}$ is a hydrogen atom, and Q is any of the substituents shown in [Table L1] to [Table L13] (hereinafter, referred to as a compound group SX70).

A compound wherein in the compound (L-1), n is 1, $R^1$ is a benzyl group, $R^{3b}$ is a bromine atom, $R^{3c}$ is a hydrogen atom, and Q is any of the substituents shown in [Table L1] to [Table L13] (hereinafter, referred to as a compound group SX71).

A compound wherein in the compound (L-1), n is 2, $R^1$ is a benzyl group, $R^{3b}$ is a bromine atom, $R^{3c}$ is a hydrogen atom, and Q is any of the substituents shown in [Table L1] to [Table L13] (hereinafter, referred to as a compound group SX72).

A compound wherein in the compound (L-1), n is 0, $R^1$ is a methyl group, $R^{3b}$ is an iodine atom, $R^{3c}$ is a hydrogen atom, and Q is any of the substituents shown in [Table L1] to [Table L13] (hereinafter, referred to as a compound group SX76).

A compound wherein in the compound (L-1), n is 1, $R^1$ is a methyl group, $R^{3b}$ is an iodine atom, $R^{3c}$ is a hydrogen atom, and Q is any of the substituents shown in [Table L1] to [Table L13] (hereinafter, referred to as a compound group SX77).

A compound wherein in the compound (L-1), n is 2, $R^1$ is a methyl group, $R^{3b}$ is an iodine atom, $R^{3c}$ is a hydrogen atom, and Q is any of the substituents shown in [Table L1] to [Table L13] (hereinafter, referred to as a compound group SX78).

A compound wherein in the compound (L-1), n is 0, $R^1$ is an ethyl group, $R^{3b}$ is an iodine atom, $R^{3c}$ is a hydrogen atom, and Q is any of the substituents shown in [Table L1] to [Table L13] (hereinafter, referred to as a compound group SX79).

A compound wherein in the compound (L-1), n is 1, $R^1$ is an ethyl group, $R^{3b}$ is an iodine atom, $R^{3c}$ is a hydrogen atom, and Q is any of the substituents shown in [Table L1] to [Table L13] (hereinafter, referred to as a compound group SX80).

A compound wherein in the compound (L-1), n is 2, $R^1$ is an ethyl group, $R^{3b}$ is an iodine atom, $R^{3c}$ is a hydrogen atom, and Q is any of the substituents shown in [Table L1] to [Table L13] (hereinafter, referred to as a compound group SX81).

A compound wherein in the compound (L-1), n is 0, $R^1$ is an isopropyl group, $R^{3b}$ is an iodine atom, $R^{3c}$ is a hydrogen atom, and Q is any of the substituents shown in [Table L1] to [Table L13] (hereinafter, referred to as a compound group SX82).

A compound wherein in the compound (L-1), n is 1, $R^1$ is an isopropyl group, $R^{3b}$ is an iodine atom, $R^{3c}$ is a hydrogen atom, and Q is any of the substituents shown in [Table L1] to [Table L13] (hereinafter, referred to as a compound group SX83).

A compound wherein in the compound (L-1), n is 2, $R^1$ is an isopropyl group, $R^{3b}$ is an iodine atom, $R^{3c}$ is a hydrogen atom, and Q is any of the substituents shown in [Table L1] to [Table L13] (hereinafter, referred to as a compound group SX84).

A compound wherein in the compound (L-1), n is 0, $R^1$ is a methylcyano group, $R^{3b}$ is an iodine atom, $R^{3c}$ is a hydrogen atom, and Q is any of the substituents shown in [Table L1] to [Table L13] (hereinafter, referred to as a compound group SX85).

A compound wherein in the compound (L-1), n is 1, $R^1$ is a methylcyano group, $R^{3b}$ is an iodine atom, $R^{3c}$ is a hydrogen atom, and Q is any of the substituents shown in [Table L1] to [Table L13] (hereinafter, referred to as a compound group SX86).

A compound wherein in the compound (L-1), n is 2, $R^1$ is a methylcyano group, $R^{3b}$ is an iodine atom, $R^{3c}$ is a hydrogen atom, and Q is any of the substituents shown in [Table L1] to [Table L13] (hereinafter, referred to as a compound group SX87).

A compound wherein in the compound (L-1), n is 0, $R^1$ is a benzyl group, $R^{3b}$ is an iodine atom, $R^{3c}$ is a hydrogen atom, and Q is any of the substituents shown in [Table L1] to [Table L13] (hereinafter, referred to as a compound group SX88).

A compound wherein in the compound (L-1), n is 1, $R^1$ is a benzyl group, $R^{3b}$ is an iodine atom, $R^{3c}$ is a hydrogen atom, and Q is any of the substituents shown in [Table L1] to [Table L13] (hereinafter, referred to as a compound group SX89).

A compound wherein in the compound (L-1), n is 2, $R^1$ is a benzyl group, $R^{3b}$ is an iodine atom, $R^{3c}$ is a hydrogen atom, and Q is any of the substituents shown in [Table L1] to [Table L13] (hereinafter, referred to as a compound group SX90).

A compound wherein in the compound (L-1), n is 0, $R^1$ is a methyl group, $R^{3b}$ is a trifluoromethyl group, $R^{3c}$ is a hydrogen atom, and Q is any of the substituents shown in [Table L1] to [Table L13] (hereinafter, referred to as a compound group SX94).

A compound wherein in the compound (L-1), n is 1, $R^1$ is a methyl group, $R^{3b}$ is a trifluoromethyl group, $R^{3c}$ is a hydrogen atom, and Q is any of the substituents shown in [Table L1] to [Table L13] (hereinafter, referred to as a compound group SX95).

A compound wherein in the compound (L-1), n is 2, $R^1$ is a methyl group, $R^{3b}$ is a trifluoromethyl group, $R^{3c}$ is a hydrogen atom, and Q is any of the substituents shown in [Table L1] to [Table L13] (hereinafter, referred to as a compound group SX96).

A compound wherein in the compound (L-1), n is 0, $R^1$ is an ethyl group, $R^{3b}$ is a trifluoromethyl group, $R^{3c}$ is a hydrogen atom, and Q is any of the substituents shown in [Table L1] to [Table L13] (hereinafter, referred to as a compound group SX97).

A compound wherein in the compound (L-1), n is 1, $R^1$ is an ethyl group, $R^{3b}$ is a trifluoromethyl group, $R^{3c}$ is a hydrogen atom, and Q is any of the substituents shown in [Table L1] to [Table L13] (hereinafter, referred to as a compound group SX98).

A compound wherein in the compound (L-1), n is 2, $R^1$ is an ethyl group, $R^{3b}$ is a trifluoromethyl group, $R^{3c}$ is a hydrogen atom, and Q is any of the substituents shown in [Table L1] to [Table L13] (hereinafter, referred to as a compound group SX99).

A compound wherein in the compound (L-1), n is 0, $R^1$ is an isopropyl group, $R^{3b}$ is a trifluoromethyl group, $R^{3c}$ is a hydrogen atom, and Q is any of the substituents shown in [Table L1] to [Table L13] (hereinafter, referred to as a compound group SX100).

A compound wherein in the compound (L-1), n is 1, $R^1$ is an isopropyl group, $R^{3b}$ is a trifluoromethyl group, $R^{3c}$ is a hydrogen atom, and Q is any of the substituents shown in [Table L1] to [Table L13] (hereinafter, referred to as a compound group SX101).

A compound wherein in the compound (L-1), n is 2, $R^1$ is an isopropyl group, $R^{3b}$ is a trifluoromethyl group, $R^{3c}$ is a hydrogen atom, and Q is any of the substituents shown in [Table L1] to [Table L13] (hereinafter, referred to as a compound group SX102).

A compound wherein in the compound (L-1), n is 0, $R^1$ is a methylcyano group, $R^{3b}$ is a trifluoromethyl group, $R^{3c}$ is a hydrogen atom, and Q is any of the substituents shown in [Table L1] to [Table L13] (hereinafter, referred to as a compound group SX103).

A compound wherein in the compound (L-1), n is 1, $R^1$ is a methylcyano group, $R^{3b}$ is a trifluoromethyl group, $R^{3c}$ is a hydrogen atom, and Q is any of the substituents shown in [Table L1] to [Table L13] (hereinafter, referred to as a compound group SX104).

A compound wherein in the compound (L-1), n is 2, $R^1$ is a methylcyano group, $R^{3b}$ is a trifluoromethyl group, $R^{3c}$ is a hydrogen atom, and Q is any of the substituents shown in [Table L1] to [Table L13] (hereinafter, referred to as a compound group SX105).

A compound wherein in the compound (L-1), n is 0, $R^1$ is a benzyl group, $R^{3b}$ is a trifluoromethyl group, $R^{3c}$ is a hydrogen atom, and Q is any of the substituents shown in [Table L1] to [Table L13] (hereinafter, referred to as a compound group SX106).

A compound wherein in the compound (L-1), n is 1, $R^1$ is a benzyl group, $R^{3b}$ is a trifluoromethyl group, $R^{3c}$ is a hydrogen atom, and Q is any of the substituents shown in [Table L1] to [Table L13] (hereinafter, referred to as a compound group SX107).

A compound wherein in the compound (L-1), n is 2, $R^1$ is a benzyl group, $R^{3b}$ is a trifluoromethyl group, $R^{3c}$ is a hydrogen atom, and Q is any of the substituents shown in [Table L1] to [Table L13] (hereinafter, referred to as a compound group SX108).

A compound wherein in the compound (L-1), n is 0, $R^1$ is a methyl group, $R^{3b}$ is a difluoromethyl group, $R^{3c}$ is a hydrogen atom, and Q is any of the substituents shown in [Table L1] to [Table L13] (hereinafter, referred to as a compound group SX112).

A compound wherein in the compound (L-1), n is 1, $R^1$ is a methyl group, $R^{3b}$ is a difluoromethyl group, $R^{3c}$ is a hydrogen atom, and Q is any of the substituents shown in [Table L1] to [Table L13] (hereinafter, referred to as a compound group SX113).

A compound wherein in the compound (L-1), n is 2, $R^1$ is a methyl group, $R^{3b}$ is a difluoromethyl group, $R^{3c}$ is a hydrogen atom, and Q is any of the substituents shown in [Table L1] to [Table L13] (hereinafter, referred to as a compound group SX114).

A compound wherein in the compound (L-1), n is 0, $R^1$ is an ethyl group, $R^{3b}$ is a difluoromethyl group, $R^{3c}$ is a hydrogen atom, and Q is any of the substituents shown in [Table L1] to [Table L13] (hereinafter, referred to as a compound group SX115).

A compound wherein in the compound (L-1), n is 1, $R^1$ is an ethyl group, $R^{3b}$ is a difluoromethyl group, $R^{3c}$ is a

hydrogen atom, and Q is any of the substituents shown in [Table L1] to [Table L13] (hereinafter, referred to as a compound group SX116).

A compound wherein in the compound (L-1), n is 2, $R^1$ is an ethyl group, $R^{3b}$ is a difluoromethyl group, $R^{3c}$ is a hydrogen atom, and Q is any of the substituents shown in [Table L1] to [Table L13] (hereinafter, referred to as a compound group SX117).

A compound wherein in the compound (L-1), n is 0, $R^1$ is an isopropyl group, $R^{3b}$ is a difluoromethyl group, $R^{3c}$ is a hydrogen atom, and Q is any of the substituents shown in [Table L1] to [Table L13] (hereinafter, referred to as a compound group SX118).

A compound wherein in the compound (L-1), n is 1, $R^1$ is an isopropyl group, $R^{3b}$ is a difluoromethyl group, $R^{3c}$ is a hydrogen atom, and Q is any of the substituents shown in [Table L1] to [Table L13] (hereinafter, referred to as a compound group SX119).

A compound wherein in the compound (L-1), n is 2, $R^1$ is an isopropyl group, $R^{3b}$ is a difluoromethyl group, $R^{3c}$ is a hydrogen atom, and Q is any of the substituents shown in [Table L1] to [Table L13] (hereinafter, referred to as a compound group SX120).

A compound wherein in the compound (L-1), n is 0, $R^1$ is a methylcyano group, $R^{3b}$ is a difluoromethyl group, $R^{3c}$ is a hydrogen atom, and Q is any of the substituents shown in [Table L1] to [Table L13] (hereinafter, referred to as a compound group SX121).

A compound wherein in the compound (L-1), n is 1, $R^1$ is a methylcyano group, $R^{3b}$ is a difluoromethyl group, $R^{3c}$ is a hydrogen atom, and Q is any of the substituents shown in [Table L1] to [Table L13] (hereinafter, referred to as a compound group SX122).

A compound wherein in the compound (L-1), n is 2, $R^1$ is a methylcyano group, $R^{3b}$ is a difluoromethyl group, $R^{3c}$ is a hydrogen atom, and Q is any of the substituents shown in [Table L1] to [Table L13] (hereinafter, referred to as a compound group SX123).

A compound wherein in the compound (L-1), n is 0, $R^1$ is a benzyl group, $R^{3b}$ is a difluoromethyl group, $R^{3c}$ is a hydrogen atom, and Q is any of the substituents shown in [Table L1] to [Table L13] (hereinafter, referred to as a compound group SX124).

A compound wherein in the compound (L-1), n is 1, $R^1$ is a benzyl group, $R^{3b}$ is a difluoromethyl group, $R^{3c}$ is a hydrogen atom, and Q is any of the substituents shown in [Table L1] to [Table L13] (hereinafter, referred to as a compound group SX125).

A compound wherein in the compound (L-1), n is 2, $R^1$ is a benzyl group, $R^{313}$ is a difluoromethyl group, $R^{3c}$ is a hydrogen atom, and Q is any of the substituents shown in [Table L1] to [Table L13] (hereinafter, referred to as a compound group SX126).

A compound wherein in the compound (L-1), n is 0, $R^1$ is a methyl group, $R^{3b}$ is a hydrogen atom, $R^{3c}$ is a fluorine atom, and Q is any of the substituents shown in [Table L1] to [Table L13] (hereinafter, referred to as a compound group SX130).

A compound wherein in the compound (L-1), n is 1, $R^1$ is a methyl group, $R^{3b}$ is a hydrogen atom, $R^{3c}$ is a fluorine atom, and Q is any of the substituents shown in [Table L1] to [Table L13] (hereinafter, referred to as a compound group SX131).

A compound wherein in the compound (L-1), n is 2, $R^1$ is a methyl group, $R^{3b}$ is a hydrogen atom, $R^{3c}$ is a fluorine atom, and Q is any of the substituents shown in [Table L1] to [Table L13] (hereinafter, referred to as a compound group SX132).

A compound wherein in the compound (L-1), n is 0, $R^1$ is an ethyl group, $R^{3b}$ is a hydrogen atom, $R^{3c}$ is a fluorine atom, and Q is any of the substituents shown in [Table L1] to [Table L13] (hereinafter, referred to as a compound group SX133).

A compound wherein in the compound (L-1), n is 1, $R^1$ is an ethyl group, $R^{3b}$ is a hydrogen atom, $R^{3c}$ is a fluorine atom, and Q is any of the substituents shown in [Table L1] to [Table L13] (hereinafter, referred to as a compound group SX134).

A compound wherein in the compound (L-1), n is 2, $R^1$ is an ethyl group, $R^{3b}$ is a hydrogen atom, $R^{3c}$ is a fluorine atom, and Q is any of the substituents shown in [Table L1] to [Table L13] (hereinafter, referred to as a compound group SX135).

A compound wherein in the compound (L-1), n is 0, $R^1$ is an isopropyl group, $R^{3b}$ is a hydrogen atom, $R^{3c}$ is a fluorine atom, and Q is any of the substituents shown in [Table L1] to [Table L13] (hereinafter, referred to as a compound group SX136).

A compound wherein in the compound (L-1), n is 1, $R^1$ is an isopropyl group, $R^{3b}$ is a hydrogen atom, $R^{3c}$ is a fluorine atom, and Q is any of the substituents shown in [Table L1] to [Table L13] (hereinafter, referred to as a compound group SX137).

A compound wherein in the compound (L-1), n is 2, $R^1$ is an isopropyl group, $R^{3b}$ is a hydrogen atom, $R^{3c}$ is a fluorine atom, and Q is any of the substituents shown in [Table L1] to [Table L13] (hereinafter, referred to as a compound

group SX138).

A compound wherein in the compound (L-1), n is 0, $R^1$ is a methylcyano group, $R^{3b}$ is a hydrogen atom, $R^{3c}$ is a fluorine atom, and Q is any of the substituents shown in [Table L1] to [Table L13] (hereinafter, referred to as a compound group SX139).

A compound wherein in the compound (L-1), n is 1, $R^1$ is a methylcyano group, $R^{3b}$ is a hydrogen atom, $R^{3c}$ is a fluorine atom, and Q is any of the substituents shown in [Table L1] to [Table L13] (hereinafter, referred to as a compound group SX140).

A compound wherein in the compound (L-1), n is 2, $R^1$ is a methylcyano group, $R^{3b}$ is a hydrogen atom, $R^{3c}$ is a fluorine atom, and Q is any of the substituents shown in [Table L1] to [Table L13] (hereinafter, referred to as a compound group SX141).

A compound wherein in the compound (L-1), n is 0, $R^1$ is a benzyl group, $R^{3b}$ is a hydrogen atom, $R^{3c}$ is a fluorine atom, and Q is any of the substituents shown in [Table L1] to [Table L13] (hereinafter, referred to as a compound group SX142).

A compound wherein in the compound (L-1), n is 1, $R^1$ is a benzyl group, $R^{3b}$ is a hydrogen atom, $R^{3c}$ is a fluorine atom, and Q is any of the substituents shown in [Table L1] to [Table L13] (hereinafter, referred to as a compound group SX143).

A compound wherein in the compound (L-1), n is 2, $R^1$ is a benzyl group, $R^{3b}$ is a hydrogen atom, $R^{3c}$ is a fluorine atom, and Q is any of the substituents shown in [Table L1] to [Table L13] (hereinafter, referred to as a compound group SX144).

A compound wherein in the compound (L-1), n is 0, $R^1$ is a methyl group, $R^{3b}$ is a fluorine atom, $R^{3c}$ is a fluorine atom, and Q is any of the substituents shown in [Table L1] to [Table L13] (hereinafter, referred to as a compound group SX148).

A compound wherein in the compound (L-1), n is 1, $R^1$ is a methyl group, $R^{3b}$ is a fluorine atom, $R^{3c}$ is a fluorine atom, and Q is any of the substituents shown in [Table L1] to [Table L13] (hereinafter, referred to as a compound group SX149).

A compound wherein in the compound (L-1), n is 2, $R^1$ is a methyl group, $R^{3b}$ is a fluorine atom, $R^{3c}$ is a fluorine atom, and Q is any of the substituents shown in [Table L1] to [Table L13] (hereinafter, referred to as a compound group SX150).

A compound wherein in the compound (L-1), n is 0, $R^1$ is an ethyl group, $R^{3b}$ is a fluorine atom, $R^{3c}$ is a fluorine atom, and Q is any of the substituents shown in [Table L1] to [Table L13] (hereinafter, referred to as a compound group SX151).

A compound wherein in the compound (L-1), n is 1, $R^1$ is an ethyl group, $R^{3b}$ is a fluorine atom, $R^{3c}$ is a fluorine atom, and Q is any of the substituents shown in [Table L1] to [Table L13] (hereinafter, referred to as a compound group SX152).

A compound wherein in the compound (L-1), n is 2, $R^1$ is an ethyl group, $R^{3b}$ is a fluorine atom, $R^{3c}$ is a fluorine atom, and Q is any of the substituents shown in [Table L1] to [Table L13] (hereinafter, referred to as a compound group SX153).

A compound wherein in the compound (L-1), n is 0, $R^1$ is an isopropyl group, $R^{3b}$ is a fluorine atom, $R^{3c}$ is a fluorine atom, and Q is any of the substituents shown in [Table L1] to [Table L13] (hereinafter, referred to as a compound group SX154).

A compound wherein in the compound (L-1), n is 1, $R^1$ is an isopropyl group, $R^{3b}$ is a fluorine atom, $R^{3c}$ is a fluorine atom, and Q is any of the substituents shown in [Table L1] to [Table L13] (hereinafter, referred to as a compound group SX155).

A compound wherein in the compound (L-1), n is 2, $R^1$ is an isopropyl group, $R^{3b}$ is a fluorine atom, $R^{3c}$ is a fluorine atom, and Q is any of the substituents shown in [Table L1] to [Table L13] (hereinafter, referred to as a compound group SX156).

A compound wherein in the compound (L-1), n is 0, $R^1$ is a methylcyano group, $R^{3b}$ is a fluorine atom, $R^{3c}$ is a fluorine atom, and Q is any of the substituents shown in [Table L1] to [Table L13] (hereinafter, referred to as a compound group SX157).

A compound wherein in the compound (L-1), n is 1, $R^1$ is a methylcyano group, $R^{3b}$ is a fluorine atom, $R^{3c}$ is a fluorine atom, and Q is any of the substituents shown in [Table L1] to [Table L13] (hereinafter, referred to as a compound group SX158).

A compound wherein in the compound (L-1), n is 2, $R^1$ is a methylcyano group, $R^{3b}$ is a fluorine atom, $R^{3c}$ is a fluorine atom, and Q is any of the substituents shown in [Table L1] to [Table L13] (hereinafter, referred to as a compound group SX159).

A compound wherein in the compound (L-1), n is 0, $R^1$ is a benzyl group, $R^{3b}$ is a fluorine atom, $R^{3c}$ is a fluorine atom, and Q is any of the substituents shown in [Table L1] to [Table L13] (hereinafter, referred to as a compound group SX160).

A compound wherein in the compound (L-1), n is 1, $R^1$ is a benzyl group, $R^{3b}$ is a fluorine atom, $R^{3c}$ is a fluorine atom, and Q is any of the substituents shown in [Table L1] to [Table L13] (hereinafter, referred to as a compound group SX161).

A compound wherein in the compound (L-1), n is 2, $R^1$ is a benzyl group, $R^{3b}$ is a fluorine atom, $R^{3c}$ is a fluorine atom, and Q is any of the substituents shown in [Table L1] to [Table L13] (hereinafter, referred to as a compound group SX162).

A compound wherein in the compound (L-1), n is 0, $R^1$ is a methyl group, $R^{3b}$ is a chlorine atom, $R^{3c}$ is a fluorine atom, and Q is any of the substituents shown in [Table L1] to [Table L13] (hereinafter, referred to as a compound group SX166).

A compound wherein in the compound (L-1), n is 1, $R^1$ is a methyl group, $R^{3b}$ is a chlorine atom, $R^{3c}$ is a fluorine atom, and Q is any of the substituents shown in [Table L1] to [Table L13] (hereinafter, referred to as a compound group SX167).

A compound wherein in the compound (L-1), n is 2, $R^1$ is a methyl group, $R^{3b}$ is a chlorine atom, $R^{3c}$ is a fluorine atom, and Q is any of the substituents shown in [Table L1] to [Table L13] (hereinafter, referred to as a compound group SX168).

A compound wherein in the compound (L-1), n is 0, $R^1$ is an ethyl group, $R^{3b}$ is a chlorine atom, $R^{3c}$ is a fluorine atom, and Q is any of the substituents shown in [Table L1] to [Table L13] (hereinafter, referred to as a compound group SX169).

A compound wherein in the compound (L-1), n is 1, $R^1$ is an ethyl group, $R^{3b}$ is a chlorine atom, $R^{3c}$ is a fluorine atom, and Q is any of the substituents shown in [Table L1] to [Table L13] (hereinafter, referred to as a compound group SX170).

A compound wherein in the compound (L-1), n is 2, $R^1$ is an ethyl group, $R^{3b}$ is a chlorine atom, $R^{3c}$ is a fluorine atom, and Q is any of the substituents shown in [Table L1] to [Table L13] (hereinafter, referred to as compound group SX171).

A compound wherein in the compound (L-1), n is 0, $R^1$ is an isopropyl group, $R^{3b}$ is a chlorine atom, $R^{3c}$ is a fluorine atom, and Q is any of the substituents shown in [Table L1] to [Table L13] (hereinafter, referred to as a compound group SX172).

A compound wherein in the compound (L-1), n is 1, $R^1$ is an isopropyl group, $R^{3b}$ is a chlorine atom, $R^{3c}$ is a fluorine atom, and Q is any of the substituents shown in [Table L1] to [Table L13] (hereinafter, referred to as a compound group SX173).

A compound wherein in the compound (L-1), n is 2, $R^1$ is an isopropyl group, $R^{3b}$ is a chlorine atom, $R^{3c}$ is a fluorine atom, and Q is any of the substituents shown in [Table L1] to [Table L13] (hereinafter, referred to as a compound group SX174).

A compound wherein in the compound (L-1), n is 0, $R^1$ is a methylcyano group, $R^{3b}$ is a chlorine atom, $R^{3c}$ is a fluorine atom, and Q is any of the substituents shown in [Table L1] to [Table L13] (hereinafter, referred to as a compound group SX175).

A compound wherein in the compound (L-1), n is 1, $R^1$ is a methylcyano group, $R^{3b}$ is a chlorine atom, $R^{3c}$ is a fluorine atom, and Q is any of the substituents shown in [Table L1] to [Table L13] (hereinafter, referred to as compound group SX176).

A compound wherein in the compound (L-1), n is 2, $R^1$ is a methylcyano group, $R^{3b}$ is a chlorine atom, $R^{3c}$ is a fluorine atom, and Q is any of the substituents shown in [Table L1] to [Table L13] (hereinafter, referred to as a compound group SX177).

A compound wherein in the compound (L-1), n is 0, $R^1$ is a benzyl group, $R^{3b}$ is a chlorine atom, $R^{3c}$ is a fluorine atom, and Q is any of the substituents shown in [Table L1] to [Table L13] (hereinafter, referred to as a compound group SX178).

A compound wherein in the compound (L-1), n is 1, $R^1$ is a benzyl group, $R^{3b}$ is a chlorine atom, $R^{3c}$ is a fluorine atom, and Q is any of the substituents shown in [Table L1] to [Table L13] (hereinafter, referred to as a compound group SX179).

A compound wherein in the compound (L-1), n is 2, $R^1$ is a benzyl group, $R^{3b}$ is a chlorine atom, $R^{3c}$ is a fluorine atom, and Q is any of the substituents shown in [Table L1] to [Table L13] (hereinafter, referred to as a compound group SX180).

A compound wherein in the compound (L-1), n is 0, $R^1$ is a methyl group, $R^{3b}$ is a bromine atom, $R^{3c}$ is a fluorine atom, and Q is any of the substituents shown in [Table L1] to [Table L13] (hereinafter, referred to as a compound group SX184).

A compound wherein in the compound (L-1), n is 1, $R^1$ is a methyl group, $R^{3b}$ is a bromine atom, $R^{3c}$ is a fluorine atom, and Q is any of the substituents shown in [Table L1] to [Table L13] (hereinafter, referred to as a compound group SX185).

A compound wherein in the compound (L-1), n is 2, $R^1$ is a methyl group, $R^{3b}$ is a bromine atom, $R^{3c}$ is a fluorine

atom, and Q is any of the substituents shown in [Table L1] to [Table L13] (hereinafter, referred to as a compound group SX186).

A compound wherein in the compound (L-1), n is 0, $R^1$ is an ethyl group, $R^{3b}$ is a bromine atom, $R^{3c}$ is a fluorine atom, and Q is any of the substituents shown in [Table L1] to [Table L13] (hereinafter, referred to as a compound group SX187).

A compound wherein in the compound (L-1), n is 1, $R^1$ is an ethyl group, $R^{3b}$ is a bromine atom, $R^{3c}$ is a fluorine atom, and Q is any of the substituents shown in [Table L1] to [Table L13] (hereinafter, referred to as a compound group SX188).

A compound wherein in the compound (L-1), n is 2, $R^1$ is an ethyl group, $R^{3b}$ is a bromine atom, $R^{3c}$ is a fluorine atom, and Q is any of the substituents shown in [Table L1] to [Table L13] (hereinafter, referred to as a compound group SX189).

A compound wherein in the compound (L-1), n is 0, $R^1$ is an isopropyl group, $R^{3b}$ is a bromine atom, $R^{3c}$ is a fluorine atom, and Q is any of the substituents shown in [Table L1] to [Table L13] (hereinafter, referred to as a compound group SX190).

A compound wherein in the compound (L-1), n is 1, $R^1$ is an isopropyl group, $R^{3b}$ is a bromine atom, $R^{3c}$ is a fluorine atom, and Q is any of the substituents shown in [Table L1] to [Table L13] (hereinafter, referred to as a compound group SX191).

A compound wherein in the compound (L-1), n is 2, $R^1$ is an isopropyl group, $R^{3b}$ is a bromine atom, $R^{3c}$ is a fluorine atom, and Q is any of the substituents shown in [Table L1] to [Table L13] (hereinafter, referred to as a compound group SX192).

A compound wherein in the compound (L-1), n is 0, $R^1$ is a methylcyano group, $R^{3b}$ is a bromine atom, $R^{3c}$ is a fluorine atom, and Q is any of the substituents shown in [Table L1] to [Table L13] (hereinafter, referred to as compound group SX193).

A compound wherein in the compound (L-1), n is 1, $R^1$ is a methylcyano group, $R^{3b}$ is a bromine atom, $R^{3c}$ is a fluorine atom, and Q is any of the substituents shown in [Table L1] to [Table L13] (hereinafter, referred to as compound group SX194).

A compound wherein in the compound (L-1), n is 2, $R^1$ is a methylcyano group, $R^{3b}$ is a bromine atom, $R^{3c}$ is a fluorine atom, and Q is any of the substituents shown in [Table L1] to [Table L13] (hereinafter, referred to as a compound group SX195).

A compound wherein in the compound (L-1), n is 0, $R^1$ is a benzyl group, $R^{3b}$ is a bromine atom, $R^{3c}$ is a fluorine atom, and Q is any of the substituents shown in [Table L1] to [Table L13] (hereinafter, referred to as a compound group SX196).

A compound wherein in the compound (L-1), n is 1, $R^1$ is a benzyl group, $R^{3b}$ is a bromine atom, $R^{3c}$ is a fluorine atom, and Q is any of the substituents shown in [Table L1] to [Table L13] (hereinafter, referred to as a compound group SX197).

A compound wherein in the compound (L-1), n is 2, $R^1$ is a benzyl group, $R^{3b}$ is a bromine atom, $R^{3c}$ is a fluorine atom, and Q is any of the substituents shown in [Table L1] to [Table L13] (hereinafter, referred to as a compound group SX198).

A compound wherein in the compound (L-1), n is 0, $R^1$ is a methyl group, $R^{3b}$ is an iodine atom, $R^{3c}$ is a fluorine atom, and Q is any of the substituents shown in [Table L1] to [Table L13] (hereinafter, referred to as a compound group SX202).

A compound wherein in the compound (L-1), n is 1, $R^1$ is a methyl group, $R^{3b}$ is an iodine atom, $R^{3c}$ is a fluorine atom, and Q is any of the substituents shown in [Table L1] to [Table L13] (hereinafter, referred to as a compound group SX203).

A compound wherein in the compound (L-1), n is 2, $R^1$ is a methyl group, $R^{3b}$ is an iodine atom, $R^{3c}$ is a fluorine atom, and Q is any of the substituents shown in [Table L1] to [Table L13] (hereinafter, referred to as a compound group SX204).

A compound wherein in the compound (L-1), n is 0, $R^1$ is an ethyl group, $R^{3b}$ is an iodine atom, $R^{3c}$ is a fluorine atom, and Q is any of the substituents shown in [Table L1] to [Table L13] (hereinafter, referred to as a compound group SX205).

A compound wherein in the compound (L-1), n is 1, $R^1$ is an ethyl group, $R^{3b}$ is an iodine atom, $R^{3c}$ is a fluorine atom, and Q is any of the substituents shown in [Table L1] to [Table L13] (hereinafter, referred to as a compound group SX206).

A compound wherein in the compound (L-1), n is 2, $R^1$ is an ethyl group, $R^{3b}$ is an iodine atom, $R^{3c}$ is a fluorine atom, and Q is any of the substituents shown in [Table L1] to [Table L13] (hereinafter, referred to as a compound group SX207).

A compound wherein in the compound (L-1), n is 0, $R^1$ is an isopropyl group, $R^{3b}$ is an iodine atom, $R^{3c}$ is a fluorine atom, and Q is any of the substituents shown in [Table L1] to [Table L13] (hereinafter, referred to as a compound group

SX208).

A compound wherein in the compound (L-1), n is 1, $R^1$ is an isopropyl group, $R^{3b}$ is an iodine atom, $R^{3c}$ is a fluorine atom, and Q is any of the substituents shown in [Table L1] to [Table L13] (hereinafter, referred to as a compound group SX209).

A compound wherein in the compound (L-1), n is 2, $R^1$ is an isopropyl group, $R^{3b}$ is an iodine atom, $R^{3c}$ is a fluorine atom, and Q is any of the substituents shown in [Table L1] to [Table L13] (hereinafter, referred to as a compound group SX210).

A compound wherein in the compound (L-1), n is 0, $R^1$ is a methylcyano group, $R^{3b}$ is an iodine atom, $R^{3c}$ is a fluorine atom, and Q is any of the substituents shown in [Table L1] to [Table L13] (hereinafter, referred to as a compound group SX211).

A compound wherein in the compound (L-1), n is 1, $R^1$ is a methylcyano group, $R^{3b}$ is an iodine atom, $R^{3c}$ is a fluorine atom, and Q is any of the substituents shown in [Table L1] to [Table L13] (hereinafter, referred to as compound group SX212).

A compound wherein in the compound (L-1), n is 2, $R^1$ is a methylcyano group, $R^{3b}$ is an iodine atom, $R^{3c}$ is a fluorine atom, and Q is any of the substituents shown in [Table L1] to [Table L13] (hereinafter, referred to as a compound group SX213).

A compound wherein in the compound (L-1), n is 0, $R^1$ is a benzyl group, $R^{3b}$ is an iodine atom, $R^{3c}$ is a fluorine atom, and Q is any of the substituents shown in [Table L1] to [Table L13] (hereinafter, referred to as a compound group SX214).

A compound wherein in the compound (L-1), n is 1, $R^1$ is a benzyl group, $R^{3b}$ is an iodine atom, $R^{3c}$ is a fluorine atom, and Q is any of the substituents shown in [Table L1] to [Table L13] (hereinafter, referred to as a compound group SX215).

A compound wherein in the compound (L-1), n is 2, $R^1$ is a benzyl group, $R^{3b}$ is an iodine atom, $R^{3c}$ is a fluorine atom, and Q is any of the substituents shown in [Table L1] to [Table L13] (hereinafter, referred to as a compound group SX216).

A compound wherein in the compound (L-1), n is 0, $R^1$ is a methyl group, $R^{3b}$ is a trifluoromethyl group, $R^{3c}$ is a fluorine atom, and Q is any of the substituents shown in [Table L1] to [Table L13] (hereinafter, referred to as a compound group SX220).

A compound wherein in the compound (L-1), n is 1, $R^1$ is a methyl group, $R^{3b}$ is a trifluoromethyl group, $R^{3c}$ is a fluorine atom, and Q is any of the substituents shown in [Table L1] to [Table L13] (hereinafter, referred to as a compound group SX221).

A compound wherein in the compound (L-1), n is 2, $R^1$ is a methyl group, $R^{3b}$ is a trifluoromethyl group, $R^{3c}$ is a fluorine atom, and Q is any of the substituents shown in [Table L1] to [Table L13] (hereinafter, referred to as a compound group SX222).

A compound wherein in the compound (L-1), n is 0, $R^1$ is an ethyl group, $R^{3b}$ is a trifluoromethyl group, $R^{3c}$ is a fluorine atom, and Q is any of the substituents shown in [Table L1] to [Table L13] (hereinafter, referred to as a compound group SX223).

A compound wherein in the compound (L-1), n is 1, $R^1$ is an ethyl group, $R^{3b}$ is a trifluoromethyl group, $R^{3c}$ is a fluorine atom, and Q is any of the substituents shown in [Table L1] to [Table L13] (hereinafter, referred to as a compound group SX224).

A compound wherein in the compound (L-1), n is 2, $R^1$ is an ethyl group, $R^{3b}$ is a trifluoromethyl group, $R^{3c}$ is a fluorine atom, and Q is any of the substituents shown in [Table L1] to [Table L13] (hereinafter, referred to as a compound group SX225).

A compound wherein in the compound (L-1), n is 0, $R^1$ is an isopropyl group, $R^{3b}$ is a trifluoromethyl group, $R^{3c}$ is a fluorine atom, and Q is any of the substituents shown in [Table L1] to [Table L13] (hereinafter, referred to as a compound group SX226).

A compound wherein in the compound (L-1), n is 1, $R^1$ is an isopropyl group, $R^{3b}$ is a trifluoromethyl group, $R^{3c}$ is a fluorine atom, and Q is any of the substituents shown in [Table L1] to [Table L13] (hereinafter, referred to as a compound group SX227).

A compound wherein in the compound (L-1), n is 2, $R^1$ is an isopropyl group, $R^{3b}$ is a trifluoromethyl group, $R^{3c}$ is a fluorine atom, and Q is any of the substituents shown in [Table L1] to [Table L13] (hereinafter, referred to as a compound group SX228).

A compound wherein in the compound (L-1), n is 0, $R^1$ is a methylcyano group, $R^{3b}$ is a trifluoromethyl group, $R^{3c}$ is a fluorine atom, and Q is any of the substituents shown in [Table L1] to [Table L13] (hereinafter, referred to as a compound group SX229).

A compound wherein in the compound (L-1), n is 1, $R^1$ is a methylcyano group, $R^{3b}$ is a trifluoromethyl group, $R^{3c}$ is a fluorine atom, and Q is any of the substituents shown in [Table L1] to [Table L13] (hereinafter, referred to as a compound group SX230).

A compound wherein in the compound (L-1), n is 2, $R^1$ is a methylcyano group, $R^{3b}$ is a trifluoromethyl group, $R^{3c}$ is a fluorine atom, and Q is any of the substituents shown in [Table L1] to [Table L13] (hereinafter, referred to as a compound group SX231).

A compound wherein in the compound (L-1), n is 0, $R^1$ is a benzyl group, $R^{3b}$ is a trifluoromethyl group, $R^{3c}$ is a fluorine atom, and Q is any of the substituents shown in [Table L1] to [Table L13] (hereinafter, referred to as a compound group SX232).

A compound wherein in the compound (L-1), n is 1, $R^1$ is a benzyl group, $R^{3b}$ is a trifluoromethyl group, $R^{3c}$ is a fluorine atom, and Q is any of the substituents shown in [Table L1] to [Table L13] (hereinafter, referred to as a compound group SX233).

A compound wherein in the compound (L-1), n is 2, $R^1$ is a benzyl group, $R^{3b}$ is a trifluoromethyl group, $R^{3c}$ is a fluorine atom, and Q is any of the substituents shown in [Table L1] to [Table L13] (hereinafter, referred to as a compound group SX234).

A compound wherein in the compound (L-1), n is 0, $R^1$ is a methyl group, $R^{3b}$ is a difluoromethyl group, $R^{3c}$ is a fluorine atom, and Q is any of the substituents shown in [Table L1] to [Table L13] (hereinafter, referred to as a compound group SX238).

A compound wherein in the compound (L-1), n is 1, $R^1$ is a methyl group, $R^{3b}$ is a difluoromethyl group, $R^{3c}$ is a fluorine atom, and Q is any of the substituents shown in [Table L1] to [Table L13] (hereinafter, referred to as a compound group SX239).

A compound wherein in the compound (L-1), n is 2, $R^1$ is a methyl group, $R^{3b}$ is a difluoromethyl group, $R^{3c}$ is a fluorine atom, and Q is any of the substituents shown in [Table L1] to [Table L13] (hereinafter, referred to as a compound group SX240).

A compound wherein in the compound (L-1), n is 0, $R^1$ is an ethyl group, $R^{3b}$ is a difluoromethyl group, $R^{3c}$ is a fluorine atom, and Q is any of the substituents shown in [Table L1] to [Table L13] (hereinafter, referred to as a compound group SX241).

A compound wherein in the compound (L-1), n is 1, $R^1$ is an ethyl group, $R^{3b}$ is a difluoromethyl group, $R^{3c}$ is a fluorine atom, and Q is any of the substituents shown in [Table L1] to [Table L13] (hereinafter, referred to as a compound group SX242).

A compound wherein in the compound (L-1), n is 2, $R^1$ is an ethyl group, $R^{3b}$ is a difluoromethyl group, $R^{3c}$ is a fluorine atom, and Q is any of the substituents shown in [Table L1] to [Table L13] (hereinafter, referred to as a compound group SX243).

A compound wherein in the compound (L-1), n is 0, $R^1$ is an isopropyl group, $R^{3b}$ is a difluoromethyl group, $R^{3c}$ is a fluorine atom, and Q is any of the substituents shown in [Table L1] to [Table L13] (hereinafter, referred to as a compound group SX244).

A compound wherein in the compound (L-1), n is 1, $R^1$ is an isopropyl group, $R^{3b}$ is a difluoromethyl group, $R^{3c}$ is a fluorine atom, and Q is any of the substituents shown in [Table L1] to [Table L13] (hereinafter, referred to as a compound group SX245).

A compound wherein in the compound (L-1), n is 2, $R^1$ is an isopropyl group, $R^{3b}$ is a difluoromethyl group, $R^{3c}$ is a fluorine atom, and Q is any of the substituents shown in [Table L1] to [Table L13] (hereinafter, referred to as a compound group SX246).

A compound wherein in the compound (L-1), n is 0, $R^1$ is a methylcyano group, $R^{3b}$ is a difluoromethyl group, $R^{3c}$ is a fluorine atom, and Q is any of the substituents shown in [Table L1] to [Table L13] (hereinafter, referred to as a compound group SX247).

A compound wherein in the compound (L-1), n is 1, $R^1$ is a methylcyano group, $R^{3b}$ is a difluoromethyl group, $R^{3c}$ is a fluorine atom, and Q is any of the substituents shown in [Table L1] to [Table L13] (hereinafter, referred to as a compound group SX248).

A compound wherein in the compound (L-1), n is 2, $R^1$ is a methylcyano group, $R^{3b}$ is a difluoromethyl group, $R^{3c}$ is a fluorine atom, and Q is any of the substituents shown in [Table L1] to [Table L13] (hereinafter, referred to as a compound group SX249).

A compound wherein in the compound (L-1), n is 0, $R^1$ is a benzyl group, $R^{3b}$ is a difluoromethyl group, $R^{3c}$ is a fluorine atom, and Q is any of the substituents shown in [Table L1] to [Table L13] (hereinafter, referred to as a compound group SX250).

A compound wherein in the compound (L-1), n is 1, $R^1$ is a benzyl group, $R^{3b}$ is a difluoromethyl group, $R^{3c}$ is a fluorine atom, and Q is any of the substituents shown in [Table L1] to [Table L13] (hereinafter, referred to as a compound group SX251).

A compound wherein in the compound (L-1), n is 2, $R^1$ is a benzyl group, $R^{3b}$ is a difluoromethyl group, $R^{3c}$ is a fluorine atom, and Q is any of the substituents shown in [Table L1] to [Table L13] (hereinafter, referred to as a compound group SX252).

A compound wherein in the compound (L-1), n is 0, $R^1$ is a methyl group, $R^{3b}$ is a hydrogen atom, $R^{3c}$ is a bromine

atom, and Q is any of the substituents shown in [Table L1] to [Table L13] (hereinafter, referred to as a compound group SX256).

A compound wherein in the compound (L-1), n is 1, $R^1$ is a methyl group, $R^{3b}$ is a hydrogen atom, $R^{3c}$ is a bromine atom, and Q is any of the substituents shown in [Table L1] to [Table L13] (hereinafter, referred to as a compound group SX257).

A compound wherein in the compound (L-1), n is 2, $R^1$ is a methyl group, $R^{3b}$ is a hydrogen atom, $R^{3c}$ is a bromine atom, and Q is any of the substituents shown in [Table L1] to [Table L13] (hereinafter, referred to as a compound group SX258).

A compound wherein in the compound (L-1), n is 0, $R^1$ is an ethyl group, $R^{3b}$ is a hydrogen atom, $R^{3c}$ is a bromine atom, and Q is any of the substituents shown in [Table L1] to [Table L13] (hereinafter, referred to as a compound group SX259).

A compound wherein in the compound (L-1), n is 1, $R^1$ is an ethyl group, $R^{3b}$ is a hydrogen atom, $R^{3c}$ is a bromine atom, and Q is any of the substituents shown in [Table L1] to [Table L13] (hereinafter, referred to as a compound group SX260).

A compound wherein in the compound (L-1), n is 2, $R^1$ is an ethyl group, $R^{3b}$ is a hydrogen atom, $R^{3c}$ is a bromine atom, and Q is any of the substituents shown in [Table L1] to [Table L13] (hereinafter, referred to as a compound group SX261).

A compound wherein in the compound (L-1), n is 0, $R^1$ is an isopropyl group, $R^{3b}$ is a hydrogen atom, $R^{3c}$ is a bromine atom, and Q is any of the substituents shown in [Table L1] to [Table L13] (hereinafter, referred to as a compound group SX262).

A compound wherein in the compound (L-1), n is 1, $R^1$ is an isopropyl group, $R^{3b}$ is a hydrogen atom, $R^{3c}$ is a bromine atom, and Q is any of the substituents shown in [Table L1] to [Table L13] (hereinafter, referred to as a compound group SX263).

A compound wherein in the compound (L-1), n is 2, $R^1$ is an isopropyl group, $R^{3b}$ is a hydrogen atom, $R^{3c}$ is a bromine atom, and Q is any of the substituents shown in [Table L1] to [Table L13] (hereinafter, referred to as a compound group SX264).

A compound wherein in the compound (L-1), n is 0, $R^1$ is a methylcyano group, $R^{3b}$ is a hydrogen atom, $R^{3c}$ is a bromine atom, and Q is any of the substituents shown in [Table L1] to [Table L13] (hereinafter, referred to as a compound group SX265).

A compound wherein in the compound (L-1), n is 1, $R^1$ is a methylcyano group, $R^{3b}$ is a hydrogen atom, $R^{3c}$ is a bromine atom, and Q is any of the substituents shown in [Table L1] to [Table L13] (hereinafter, referred to as a compound group SX266).

A compound wherein in the compound (L-1), n is 2, $R^1$ is a methylcyano group, $R^{3b}$ is a hydrogen atom, $R^{3c}$ is a bromine atom, and Q is any of the substituents shown in [Table L1] to [Table L13] (hereinafter, referred to as a compound group SX267).

A compound wherein in the compound (L-1), n is 0, $R^1$ is a benzyl group, $R^{3b}$ is a hydrogen atom, $R^{3c}$ is a bromine atom, and Q is any of the substituents shown in [Table L1] to [Table L13] (hereinafter, referred to as a compound group SX268).

A compound wherein in the compound (L-1), n is 1, $R^1$ is a benzyl group, $R^{3b}$ is a hydrogen atom, $R^{3c}$ is a bromine atom, and Q is any of the substituents shown in [Table L1] to [Table L13] (hereinafter, referred to as a compound group SX269).

A compound wherein in the compound (L-1), n is 2, $R^1$ is a benzyl group, $R^{3b}$ is a hydrogen atom, $R^{3c}$ is a bromine atom, and Q is any of the substituents shown in [Table L1] to [Table L13] (hereinafter, referred to as a compound group SX270).

A compound wherein in the compound (L-1), n is 0, $R^1$ is a methyl group, $R^{3b}$ is a fluorine atom, $R^{3c}$ is a bromine atom, and Q is any of the substituents shown in [Table L1] to [Table L13] (hereinafter, referred to as a compound group SX274).

A compound wherein in the compound (L-1), n is 1, $R^1$ is a methyl group, $R^{3b}$ is a fluorine atom, $R^{3c}$ is a bromine atom, and Q is any of the substituents shown in [Table L1] to [Table L13] (hereinafter, referred to as a compound group SX275).

A compound wherein in the compound (L-1), n is 2, $R^1$ is a methyl group, $R^{3b}$ is a fluorine atom, $R^{3c}$ is a bromine atom, and Q is any of the substituents shown in [Table L1] to [Table L13] (hereinafter, referred to as a compound group SX276).

A compound wherein in the compound (L-1), n is 0, $R^1$ is an ethyl group, $R^{3b}$ is a fluorine atom, $R^{3c}$ is a bromine atom, and Q is any of the substituents shown in [Table L1] to [Table L13] (hereinafter, referred to as a compound group SX277).

A compound wherein in the compound (L-1), n is 1, $R^1$ is an ethyl group, $R^{3b}$ is a fluorine atom, $R^{3c}$ is a bromine atom, and Q is any of the substituents shown in [Table L1] to [Table L13] (hereinafter, referred to as a compound group

SX278).

A compound wherein in the compound (L-1), n is 2, $R^1$ is an ethyl group, $R^{3b}$ is a fluorine atom, $R^{3c}$ is a bromine atom, and Q is any of the substituents shown in [Table L1] to [Table L13] (hereinafter, referred to as a compound group SX279).

A compound wherein in the compound (L-1), n is 0, $R^1$ is an isopropyl group, $R^{3b}$ is a fluorine atom, $R^{3c}$ is a bromine atom, and Q is any of the substituents shown in [Table L1] to [Table L13] (hereinafter, referred to as a compound group SX280).

A compound wherein in the compound (L-1), n is 1, $R^1$ is an isopropyl group, $R^{3b}$ is a fluorine atom, $R^{3c}$ is a bromine atom, and Q is any of the substituents shown in [Table L1] to [Table L13] (hereinafter, referred to as a compound group SX281).

A compound wherein in the compound (L-1), n is 2, $R^1$ is an isopropyl group, $R^{3b}$ is a fluorine atom, $R^{3c}$ is a bromine atom, and Q is any of the substituents shown in [Table L1] to [Table L13] (hereinafter, referred to as a compound group SX282).

A compound wherein in the compound (L-1), n is 0, $R^1$ is a methylcyano group, $R^{3b}$ is a fluorine atom, $R^{3c}$ is a bromine atom, and Q is any of the substituents shown in [Table L1] to [Table L13] (hereinafter, referred to as a compound group SX283).

A compound wherein in the compound (L-1), n is 1, $R^1$ is a methylcyano group, $R^{3b}$ is a fluorine atom, $R^{3c}$ is a bromine atom, and Q is any of the substituents shown in [Table L1] to [Table L13] (hereinafter, referred to as compound group SX284).

A compound wherein in the compound (L-1), n is 2, $R^1$ is a methylcyano group, $R^{3b}$ is a fluorine atom, $R^{3c}$ is a bromine atom, and Q is any of the substituents shown in [Table L1] to [Table L13] (hereinafter, referred to as a compound group SX285).

A compound wherein in the compound (L-1), n is 0, $R^1$ is a benzyl group, $R^{3b}$ is a fluorine atom, $R^{3c}$ is a bromine atom, and Q is any of the substituents shown in [Table L1] to [Table L13] (hereinafter, referred to as a compound group SX286).

A compound wherein in the compound (L-1), n is 1, $R^1$ is a benzyl group, $R^{3b}$ is a fluorine atom, $R^{3c}$ is a bromine atom, and Q is any of the substituents shown in [Table L1] to [Table L13] (hereinafter, referred to as a compound group SX287).

A compound wherein in the compound (L-1), n is 2, $R^1$ is a benzyl group, $R^{3b}$ is a fluorine atom, $R^{3c}$ is a bromine atom, and Q is any of the substituents shown in [Table L1] to [Table L13] (hereinafter, referred to as a compound group SX288).

A compound wherein in the compound (L-1), n is 0, $R^1$ is a methyl group, $R^{3b}$ is a chlorine atom, $R^{3c}$ is a bromine atom, and Q is any of the substituents shown in [Table L1] to [Table L13] (hereinafter, referred to as a compound group SX292).

A compound wherein in the compound (L-1), n is 1, $R^1$ is a methyl group, $R^{3b}$ is a chlorine atom, $R^{3c}$ is a bromine atom, and Q is any of the substituents shown in [Table L1] to [Table L13] (hereinafter, referred to as a compound group SX293).

A compound wherein in the compound (L-1), n is 2, $R^1$ is a methyl group, $R^{3b}$ is a chlorine atom, $R^{3c}$ is a bromine atom, and Q is any of the substituents shown in [Table L1] to [Table L13] (hereinafter, referred to as a compound group SX294).

A compound wherein in the compound (L-1), n is 0, $R^1$ is an ethyl group, $R^{3b}$ is a chlorine atom, $R^{3c}$ is a bromine atom, and Q is any of the substituents shown in [Table L1] to [Table L13] (hereinafter, referred to as a compound group SX295).

A compound wherein in the compound (L-1), n is 1, $R^1$ is an ethyl group, $R^{3b}$ is a chlorine atom, $R^{3c}$ is a bromine atom, and Q is any of the substituents shown in [Table L1] to [Table L13] (hereinafter, referred to as a compound group SX296).

A compound wherein in the compound (L-1), n is 2, $R^1$ is an ethyl group, $R^{3b}$ is a chlorine atom, $R^{3c}$ is a bromine atom, and Q is any of the substituents shown in [Table L1] to [Table L13] (hereinafter, referred to as a compound group SX297).

A compound wherein in the compound (L-1), n is 0, $R^1$ is an isopropyl group, $R^{3b}$ is a chlorine atom, $R^{3c}$ is a bromine atom, and Q is any of the substituents shown in [Table L1] to [Table L13] (hereinafter, referred to as a compound group SX298).

A compound wherein in the compound (L-1), n is 1, $R^1$ is an isopropyl group, $R^{3b}$ is a chlorine atom, $R^{3c}$ is a bromine atom, and Q is any of the substituents shown in [Table L1] to [Table L13] (hereinafter, referred to as a compound group SX299).

A compound wherein in the compound (L-1), n is 2, $R^1$ is an isopropyl group, $R^{3b}$ is a chlorine atom, $R^{3c}$ is a bromine atom, and Q is any of the substituents shown in [Table L1] to [Table L13] (hereinafter, referred to as a compound group SX300).

A compound wherein in the compound (L-1), n is 0, $R^1$ is a methylcyano group, $R^{3b}$ is a chlorine atom, $R^{3c}$ is a bromine atom, and Q is any of the substituents shown in [Table L1] to [Table L13] (hereinafter, referred to as a compound group SX301).

A compound wherein in the compound (L-1), n is 1, $R^1$ is a methylcyano group, $R^{3b}$ is a chlorine atom, $R^{3c}$ is a bromine atom, and Q is any of the substituents shown in [Table L1] to [Table L13] (hereinafter, referred to as a compound group SX302).

A compound wherein in the compound (L-1), n is 2, $R^1$ is a methylcyano group, $R^{3b}$ is a chlorine atom, $R^{3c}$ is a bromine atom, and Q is any of the substituents shown in [Table L1] to [Table L13] (hereinafter, referred to as a compound group SX303).

A compound wherein in the compound (L-1), n is 0, $R^1$ is a benzyl group, $R^{3b}$ is a chlorine atom, $R^{3c}$ is a bromine atom, and Q is any of the substituents shown in [Table L1] to [Table L13] (hereinafter, referred to as a compound group SX304).

A compound wherein in the compound (L-1), n is 1, $R^1$ is a benzyl group, $R^{3b}$ is a chlorine atom, $R^{3c}$ is a bromine atom, and Q is any of the substituents shown in [Table L1] to [Table L13] (hereinafter, referred to as a compound group SX305).

A compound wherein in the compound (L-1), n is 2, $R^1$ is a benzyl group, $R^{3b}$ is a chlorine atom, $R^{3c}$ is a bromine atom, and Q is any of the substituents shown in [Table L1] to [Table L13] (hereinafter, referred to as a compound group SX306).

A compound wherein in the compound (L-1), n is 0, $R^1$ is a methyl group, $R^{3b}$ is a bromine atom, $R^{3c}$ is a bromine atom, and Q is any of the substituents shown in [Table L1] to [Table L13] (hereinafter, referred to as a compound group SX310).

A compound wherein in the compound (L-1), n is 1, $R^1$ is a methyl group, $R^{3b}$ is a bromine atom, $R^{3c}$ is a bromine atom, and Q is any of the substituents shown in [Table L1] to [Table L13] (hereinafter, referred to as a compound group SX311).

A compound wherein in the compound (L-1), n is 2, $R^1$ is a methyl group, $R^{3b}$ is a bromine atom, $R^{3c}$ is a bromine atom, and Q is any of the substituents shown in [Table L1] to [Table L13] (hereinafter, referred to as a compound group SX312).

A compound wherein in the compound (L-1), n is 0, $R^1$ is an ethyl group, $R^{3b}$ is a bromine atom, $R^{3c}$ is a bromine atom, and Q is any of the substituents shown in [Table L1] to [Table L13] (hereinafter, referred to as a compound group SX313).

A compound wherein in the compound (L-1), n is 1, $R^1$ is an ethyl group, $R^{3b}$ is a bromine atom, $R^{3c}$ is a bromine atom, and Q is any of the substituents shown in [Table L1] to [Table L13] (hereinafter, referred to as a compound group SX314).

A compound wherein in the compound (L-1), n is 2, $R^1$ is an ethyl group, $R^{3b}$ is a bromine atom, $R^{3c}$ is a bromine atom, and Q is any of the substituents shown in [Table L1] to [Table L13] (hereinafter, referred to as a compound group SX315).

A compound wherein in the compound (L-1), n is 0, $R^1$ is an isopropyl group, $R^{3b}$ is a bromine atom, $R^{3c}$ is a bromine atom, and Q is any of the substituents shown in [Table L1] to [Table L13] (hereinafter, referred to as a compound group SX316).

A compound wherein in the compound (L-1), n is 1, $R^1$ is an isopropyl group, $R^{3b}$ is a bromine atom, $R^{3c}$ is a bromine atom, and Q is any of the substituents shown in [Table L1] to [Table L13] (hereinafter, referred to as a compound group SX317).

A compound wherein in the compound (L-1), n is 2, $R^1$ is an isopropyl group, $R^{3b}$ is a bromine atom, $R^{3c}$ is a bromine atom, and Q is any of the substituents shown in [Table L1] to [Table L13] (hereinafter, referred to as a compound group SX318).

A compound wherein in the compound (L-1), n is 0, $R^1$ is a methylcyano group, $R^{3b}$ is a bromine atom, $R^{3c}$ is a bromine atom, and Q is any of the substituents shown in [Table L1] to [Table L13] (hereinafter, referred to as a compound group SX319).

A compound wherein in the compound (L-1), n is 1, $R^1$ is a methylcyano group, $R^{3b}$ is a bromine atom, $R^{3c}$ is a bromine atom, and Q is any of the substituents shown in [Table L1] to [Table L13] (hereinafter, referred to as compound group SX320).

A compound wherein in the compound (L-1), n is 2, $R^1$ is a methylcyano group, $R^{3b}$ is a bromine atom, $R^{3c}$ is a bromine atom, and Q is any of the substituents shown in [Table L1] to [Table L13] (hereinafter, referred to as a compound group SX321).

A compound wherein in the compound (L-1), n is 0, $R^1$ is a benzyl group, $R^{3b}$ is a bromine atom, $R^{3c}$ is a bromine atom, and Q is any of the substituents shown in [Table L1] to [Table L13] (hereinafter, referred to as a compound group SX322).

A compound wherein in the compound (L-1), n is 1, $R^1$ is a benzyl group, $R^{3b}$ is a bromine atom, $R^{3c}$ is a bromine

atom, and Q is any of the substituents shown in [Table L1] to [Table L13] (hereinafter, referred to as a compound group SX323).

A compound wherein in the compound (L-1), n is 2, $R^1$ is a benzyl group, $R^{3b}$ is a bromine atom, $R^{3c}$ is a bromine atom, and Q is any of the substituents shown in [Table L1] to [Table L13] (hereinafter, referred to as a compound group SX324).

A compound wherein in the compound (L-1), n is 0, $R^1$ is a methyl group, $R^{3b}$ is an iodine atom, $R^{3c}$ is a bromine atom, and Q is any of the substituents shown in [Table L1] to [Table L13] (hereinafter, referred to as a compound group SX328).

A compound wherein in the compound (L-1), n is 1, $R^1$ is a methyl group, $R^{3b}$ is an iodine atom, $R^{3c}$ is a bromine atom, and Q is any of the substituents shown in [Table L1] to [Table L13] (hereinafter, referred to as a compound group SX329).

A compound wherein in the compound (L-1), n is 2, $R^1$ is a methyl group, $R^{3b}$ is an iodine atom, $R^{3c}$ is a bromine atom, and Q is any of the substituents shown in [Table L1] to [Table L13] (hereinafter, referred to as a compound group SX330).

A compound wherein in the compound (L-1), n is 0, $R^1$ is an ethyl group, $R^{3b}$ is an iodine atom, $R^{3c}$ is a bromine atom, and Q is any of the substituents shown in [Table L1] to [Table L13] (hereinafter, referred to as a compound group SX331).

A compound wherein in the compound (L-1), n is 1, $R^1$ is an ethyl group, $R^{3b}$ is an iodine atom, $R^{3c}$ is a bromine atom, and Q is any of the substituents shown in [Table L1] to [Table L13] (hereinafter, referred to as a compound group SX332).

A compound wherein in the compound (L-1), n is 2, $R^1$ is an ethyl group, $R^{3b}$ is an iodine atom, $R^{3c}$ is a bromine atom, and Q is any of the substituents shown in [Table L1] to [Table L13] (hereinafter, referred to as a compound group SX333).

A compound wherein in the compound (L-1), n is 0, $R^1$ is an isopropyl group, $R^{3b}$ is an iodine atom, $R^{3c}$ is a bromine atom, and Q is any of the substituents shown in [Table L1] to [Table L13] (hereinafter, referred to as a compound group SX334).

A compound wherein in the compound (L-1), n is 1, $R^1$ is an isopropyl group, $R^{3b}$ is an iodine atom, $R^{3c}$ is a bromine atom, and Q is any of the substituents shown in [Table L1] to [Table L13] (hereinafter, referred to as a compound group SX335).

A compound wherein in the compound (L-1), n is 2, $R^1$ is an isopropyl group, $R^{3b}$ is an iodine atom, $R^{3c}$ is a bromine atom, and Q is any of the substituents shown in [Table L1] to [Table L13] (hereinafter, referred to as a compound group SX336).

A compound wherein in the compound (L-1), n is 0, $R^1$ is a methylcyano group, $R^{3b}$ is an iodine atom, $R^{3c}$ is a bromine atom, and Q is any of the substituents shown in [Table L1] to [Table L13] (hereinafter, referred to as a compound group SX337).

A compound wherein in the compound (L-1), n is 1, $R^1$ is a methylcyano group, $R^{3b}$ is an iodine atom, $R^{3c}$ is a bromine atom, and Q is any of the substituents shown in [Table L1] to [Table L13] (hereinafter, referred to as a compound group SX338).

A compound wherein in the compound (L-1), n is 2, $R^1$ is a methylcyano group, $R^{3b}$ is an iodine atom, $R^{3c}$ is a bromine atom, and Q is any of the substituents shown in [Table L1] to [Table L13] (hereinafter, referred to as a compound group SX339).

A compound wherein in the compound (L-1), n is 0, $R^1$ is a benzyl group, $R^{3b}$ is an iodine atom, $R^{3c}$ is a bromine atom, and Q is any of the substituents shown in [Table L1] to [Table L13] (hereinafter, referred to as compound group SX340).

A compound wherein in the compound (L-1), n is 1, $R^1$ is a benzyl group, $R^{3b}$ is an iodine atom, $R^{3c}$ is a bromine atom, and Q is any of the substituents shown in [Table L1] to [Table L13] (hereinafter, referred to as compound group SX341).

A compound wherein in the compound (L-1), n is 2, $R^1$ is a benzyl group, $R^{3b}$ is an iodine atom, $R^{3c}$ is a bromine atom, and Q is any of the substituents shown in [Table L1] to [Table L13] (hereinafter, referred to as compound group SX342).

A compound wherein in the compound (L-1), n is 0, $R^1$ is a methyl group, $R^{3b}$ is a trifluoromethyl group, $R^{3c}$ is a bromine atom, and Q is any of the substituents shown in [Table L1] to [Table L13] (hereinafter, referred to as a compound group SX346).

A compound wherein in the compound (L-1), n is 1, $R^1$ is a methyl group, $R^{3b}$ is a trifluoromethyl group, $R^{3c}$ is a bromine atom, and Q is any of the substituents shown in [Table L1] to [Table L13] (hereinafter, referred to as a compound group SX347).

A compound wherein in the compound (L-1), n is 2, $R^1$ is a methyl group, $R^{3b}$ is a trifluoromethyl group, $R^{3c}$ is a bromine atom, and Q is any of the substituents shown in [Table L1] to [Table L13] (hereinafter, referred to as a compound

group SX348).

A compound wherein in the compound (L-1), n is 0, $R^1$ is an ethyl group, $R^{3b}$ is a trifluoromethyl group, $R^{3c}$ is a bromine atom, and Q is any of the substituents shown in [Table L1] to [Table L13] (hereinafter, referred to as a compound group SX349).

A compound wherein in the compound (L-1), n is 1, $R^1$ is an ethyl group, $R^{3b}$ is a trifluoromethyl group, $R^{3c}$ is a bromine atom, and Q is any of the substituents shown in [Table L1] to [Table L13] (hereinafter, referred to as a compound group SX350).

A compound wherein in the compound (L-1), n is 2, $R^1$ is an ethyl group, $R^{3b}$ is a trifluoromethyl group, $R^{3c}$ is a bromine atom, and Q is any of the substituents shown in [Table L1] to [Table L13] (hereinafter, referred to as a compound group SX351).

A compound wherein in the compound (L-1), n is 0, $R^1$ is an isopropyl group, $R^{3b}$ is a trifluoromethyl group, $R^{3c}$ is a bromine atom, and Q is any of the substituents shown in [Table L1] to [Table L13] (hereinafter, referred to as a compound group SX352).

A compound wherein in the compound (L-1), n is 1, $R^1$ is an isopropyl group, $R^{3b}$ is a trifluoromethyl group, $R^{3c}$ is a bromine atom, and Q is any of the substituents shown in [Table L1] to [Table L13] (hereinafter, referred to as a compound group SX353).

A compound wherein in the compound (L-1), n is 2, $R^1$ is an isopropyl group, $R^{3b}$ is a trifluoromethyl group, $R^{3c}$ is a bromine atom, and Q is any of the substituents shown in [Table L1] to [Table L13] (hereinafter, referred to as a compound group SX354).

A compound wherein in the compound (L-1), n is 0, $R^1$ is a methylcyano group, $R^{3b}$ is a trifluoromethyl group, $R^{3c}$ is a bromine atom, and Q is any of the substituents shown in [Table L1] to [Table L13] (hereinafter, referred to as a compound group SX355).

A compound wherein in the compound (L-1), n is 1, $R^1$ is a methylcyano group, $R^{3b}$ is a trifluoromethyl group, $R^{3c}$ is a bromine atom, and Q is any of the substituents shown in [Table L1] to [Table L13] (hereinafter, referred to as a compound group SX356).

A compound wherein in the compound (L-1), n is 2, $R^1$ is a methylcyano group, $R^{3b}$ is a trifluoromethyl group, $R^{3c}$ is a bromine atom, and Q is any of the substituents shown in [Table L1] to [Table L13] (hereinafter, referred to as a compound group SX357).

A compound wherein in the compound (L-1), n is 0, $R^1$ is a benzyl group, $R^{3b}$ is a trifluoromethyl group, $R^{3c}$ is a bromine atom, and Q is any of the substituents shown in [Table L1] to [Table L13] (hereinafter, referred to as a compound group SX358).

A compound wherein in the compound (L-1), n is 1, $R^1$ is a benzyl group, $R^{3b}$ is a trifluoromethyl group, $R^{3c}$ is a bromine atom, and Q is any of the substituents shown in [Table L1] to [Table L13] (hereinafter, referred to as a compound group SX359).

A compound wherein in the compound (L-1), n is 2, $R^1$ is a benzyl group, $R^{3b}$ is a trifluoromethyl group, $R^{3c}$ is a bromine atom, and Q is any of the substituents shown in [Table L1] to [Table L13] (hereinafter, referred to as a compound group SX360).

A compound wherein in the compound (L-1), n is 0, $R^1$ is a methyl group, $R^{3b}$ is a difluoromethyl group, $R^{3c}$ is a bromine atom, and Q is any of the substituents shown in [Table L1] to [Table L13] (hereinafter, referred to as a compound group SX364).

A compound wherein in the compound (L-1), n is 1, $R^1$ is a methyl group, $R^{3b}$ is a difluoromethyl group, $R^{3c}$ is a bromine atom, and Q is any of the substituents shown in [Table L1] to [Table L13] (hereinafter, referred to as a compound group SX365).

A compound wherein in the compound (L-1), n is 2, $R^1$ is a methyl group, $R^{3b}$ is a difluoromethyl group, $R^{3c}$ is a bromine atom, and Q is any of the substituents shown in [Table L1] to [Table L13] (hereinafter, referred to as a compound group SX366).

A compound wherein in the compound (L-1), n is 0, $R^1$ is an ethyl group, $R^{3b}$ is a difluoromethyl group, $R^{3c}$ is a bromine atom, and Q is any of the substituents shown in [Table L1] to [Table L13] (hereinafter, referred to as a compound group SX367).

A compound wherein in the compound (L-1), n is 1, $R^1$ is an ethyl group, $R^{3b}$ is a difluoromethyl group, $R^{3c}$ is a bromine atom, and Q is any of the substituents shown in [Table L1] to [Table L13] (hereinafter, referred to as a compound group SX368).

A compound wherein in the compound (L-1), n is 2, $R^1$ is an ethyl group, $R^{3b}$ is a difluoromethyl group, $R^{3c}$ is a bromine atom, and Q is any of the substituents shown in [Table L1] to [Table L13] (hereinafter, referred to as a compound group SX369).

A compound wherein in the compound (L-1), n is 0, $R^1$ is an isopropyl group, $R^{3b}$ is a difluoromethyl group, $R^{3c}$ is a bromine atom, and Q is any of the substituents shown in [Table L1] to [Table L13] (hereinafter, referred to as a compound group SX370).

A compound wherein in the compound (L-1), n is 1, $R^1$ is an isopropyl group, $R^{3b}$ is a difluoromethyl group, $R^{3c}$ is a bromine atom, and Q is any of the substituents shown in [Table L1] to [Table L13] (hereinafter, referred to as a compound group SX371).

A compound wherein in the compound (L-1), n is 2, $R^1$ is an isopropyl group, $R^{3b}$ is a difluoromethyl group, $R^{3c}$ is a bromine atom, and Q is any of the substituents shown in [Table L1] to [Table L13] (hereinafter, referred to as a compound group SX372).

A compound wherein in the compound (L-1), n is 0, $R^1$ is a methylcyano group, $R^{3b}$ is a difluoromethyl group, $R^{3c}$ is a bromine atom, and Q is any of the substituents shown in [Table L1] to [Table L13] (hereinafter, referred to as a compound group SX373).

A compound wherein in the compound (L-1), n is 1, $R^1$ is a methylcyano group, $R^{3b}$ is a difluoromethyl group, $R^{3c}$ is a bromine atom, and Q is any of the substituents shown in [Table L1] to [Table L13] (hereinafter, referred to as a compound group SX374).

A compound wherein in the compound (L-1), n is 2, $R^1$ is a methylcyano group, $R^{3b}$ is a difluoromethyl group, $R^{3c}$ is a bromine atom, and Q is any of the substituents shown in [Table L1] to [Table L13] (hereinafter, referred to as a compound group SX375).

A compound wherein in the compound (L-1), n is 0, $R^1$ is a benzyl group, $R^{3b}$ is a difluoromethyl group, $R^{3c}$ is a bromine atom, and Q is any of the substituents shown in [Table L1] to [Table L13] (hereinafter, referred to as a compound group SX376).

A compound wherein in the compound (L-1), n is 1, $R^1$ is a benzyl group, $R^{3b}$ is a difluoromethyl group, $R^{3c}$ is a bromine atom, and Q is any of the substituents shown in [Table L1] to [Table L13] (hereinafter, referred to as a compound group SX377).

A compound wherein in the compound (L-1), n is 2, $R^1$ is a benzyl group, $R^{3b}$ is a difluoromethyl group, $R^{3c}$ is a bromine atom, and Q is any of the substituents shown in [Table L1] to [Table L13] (hereinafter, referred to as a compound group SX378).

A compound wherein in the compound (L-1), n is 0, $R^1$ is a methyl group, $R^{3b}$ is a hydrogen atom, $R^{3c}$ is an iodine atom, and Q is any of the substituents shown in [Table L1] to [Table L13] (hereinafter, referred to as a compound group SX382).

A compound wherein in the compound (L-1), n is 1, $R^1$ is a methyl group, $R^{3b}$ is a hydrogen atom, $R^{3c}$ is an iodine atom, and Q is any of the substituents shown in [Table L1] to [Table L13] (hereinafter, referred to as a compound group SX383).

A compound wherein in the compound (L-1), n is 2, $R^1$ is a methyl group, $R^{3b}$ is a hydrogen atom, $R^{3c}$ is an iodine atom, and Q is any of the substituents shown in [Table L1] to [Table L13] (hereinafter, referred to as a compound group SX384).

A compound wherein in the compound (L-1), n is 0, $R^1$ is an ethyl group, $R^{3b}$ is a hydrogen atom, $R^{3c}$ is an iodine atom, and Q is any of the substituents shown in [Table L1] to [Table L13] (hereinafter, referred to as a compound group SX385).

A compound wherein in the compound (L-1), n is 1, $R^1$ is an ethyl group, $R^{3b}$ is a hydrogen atom, $R^{3c}$ is an iodine atom, and Q is any of the substituents shown in [Table L1] to [Table L13] (hereinafter, referred to as a compound group SX386).

A compound wherein in the compound (L-1), n is 2, $R^1$ is an ethyl group, $R^{3b}$ is a hydrogen atom, $R^{3c}$ is an iodine atom, and Q is any of the substituents shown in [Table L1] to [Table L13] (hereinafter, referred to as a compound group SX387).

A compound wherein in the compound (L-1), n is 0, $R^1$ is an isopropyl group, $R^{3b}$ is a hydrogen atom, $R^{3c}$ is an iodine atom, and Q is any of the substituents shown in [Table L1] to [Table L13] (hereinafter, referred to as a compound group SX388).

A compound wherein in the compound (L-1), n is 1, $R^1$ is an isopropyl group, $R^{3b}$ is a hydrogen atom, $R^{3c}$ is an iodine atom, and Q is any of the substituents shown in [Table L1] to [Table L13] (hereinafter, referred to as a compound group SX389).

A compound wherein in the compound (L-1), n is 2, $R^1$ is an isopropyl group, $R^{3b}$ is a hydrogen atom, $R^{3c}$ is an iodine atom, and Q is any of the substituents shown in [Table L1] to [Table L13] (hereinafter, referred to as a compound group SX390).

A compound wherein in the compound (L-1), n is 0, $R^1$ is a methylcyano group, $R^{3b}$ is a hydrogen atom, $R^{3c}$ is an iodine atom, and Q is any of the substituents shown in [Table L1] to [Table L13] (hereinafter, referred to as a compound group SX391).

A compound wherein in the compound (L-1), n is 1, $R^1$ is a methylcyano group, $R^{3b}$ is a hydrogen atom, $R^{3c}$ is an iodine atom, and Q is any of the substituents shown in [Table L1] to [Table L13] (hereinafter, referred to as a compound group SX392).

A compound wherein in the compound (L-1), n is 2, $R^1$ is a methylcyano group, $R^{3b}$ is a hydrogen atom, $R^{3c}$ is an

iodine atom, and Q is any of the substituents shown in [Table L1] to [Table L13] (hereinafter, referred to as a compound group SX393).

A compound wherein in the compound (L-1), n is 0, $R^1$ is a benzyl group, $R^{3b}$ is a hydrogen atom, $R^{3c}$ is an iodine atom, and Q is any of the substituents shown in [Table L1] to [Table L13] (hereinafter, referred to as a compound group SX394).

A compound wherein in the compound (L-1), n is 1, $R^1$ is a benzyl group, $R^{3b}$ is a hydrogen atom, $R^{3c}$ is an iodine atom, and Q is any of the substituents shown in [Table L1] to [Table L13] (hereinafter, referred to as a compound group SX395).

A compound wherein in the compound (L-1), n is 2, $R^1$ is a benzyl group, $R^{3b}$ is a hydrogen atom, $R^{3c}$ is an iodine atom, and Q is any of the substituents shown in [Table L1] to [Table L13] (hereinafter, referred to as a compound group SX396).

A compound wherein in the compound (L-1), n is 0, $R^1$ is a methyl group, $R^{3b}$ is a fluorine atom, $R^{3c}$ is an iodine atom, and Q is any of the substituents shown in [Table L1] to [Table L13] (hereinafter, referred to as a compound group SX400).

A compound wherein in the compound (L-1), n is 1, $R^1$ is a methyl group, $R^{3b}$ is a fluorine atom, $R^{3c}$ is an iodine atom, and Q is any of the substituents shown in [Table L1] to [Table L13] (hereinafter, referred to as a compound group SX401).

A compound wherein in the compound (L-1), n is 2, $R^1$ is a methyl group, $R^{3b}$ is a fluorine atom, $R^{3c}$ is an iodine atom, and Q is any of the substituents shown in [Table L1] to [Table L13] (hereinafter, referred to as a compound group SX402).

A compound wherein in the compound (L-1), n is 0, $R^1$ is an ethyl group, $R^{3b}$ is a fluorine atom, $R^{3c}$ is an iodine atom, and Q is any of the substituents shown in [Table L1] to [Table L13] (hereinafter, referred to as a compound group SX403).

A compound wherein in the compound (L-1), n is 1, $R^1$ is an ethyl group, $R^{3b}$ is a fluorine atom, $R^{3c}$ is an iodine atom, and Q is any of the substituents shown in [Table L1] to [Table L13] (hereinafter, referred to as a compound group SX404).

A compound wherein in the compound (L-1), n is 2, $R^1$ is an ethyl group, $R^{3b}$ is a fluorine atom, $R^{3c}$ is an iodine atom, and Q is any of the substituents shown in [Table L1] to [Table L13] (hereinafter, referred to as a compound group SX405).

A compound wherein in the compound (L-1), n is 0, $R^1$ is an isopropyl group, $R^{3b}$ is a fluorine atom, $R^{3c}$ is an iodine atom, and Q is any of the substituents shown in [Table L1] to [Table L13] (hereinafter, referred to as a compound group SX406).

A compound wherein in the compound (L-1), n is 1, $R^1$ is an isopropyl group, $R^{3b}$ is a fluorine atom, $R^{3c}$ is an iodine atom, and Q is any of the substituents shown in [Table L1] to [Table L13] (hereinafter, referred to as a compound group SX407).

A compound wherein in the compound (L-1), n is 2, $R^1$ is an isopropyl group, $R^{3b}$ is a fluorine atom, $R^{3c}$ is an iodine atom, and Q is any of the substituents shown in [Table L1] to [Table L13] (hereinafter, referred to as a compound group SX408).

A compound wherein in the compound (L-1), n is 0, $R^1$ is a methylcyano group, $R^{3b}$ is a fluorine atom, $R^{3c}$ is an iodine atom, and Q is any of the substituents shown in [Table L1] to [Table L13] (hereinafter, referred to as a compound group SX409).

A compound wherein in the compound (L-1), n is 1, $R^1$ is a methylcyano group, $R^{3b}$ is a fluorine atom, $R^{3c}$ is an iodine atom, and Q is any of the substituents shown in [Table L1] to [Table L13] (hereinafter, referred to as a compound group SX410).

A compound wherein in the compound (L-1), n is 2, $R^1$ is a methylcyano group, $R^{3b}$ is a fluorine atom, $R^{3c}$ is an iodine atom, and Q is any of the substituents shown in [Table L1] to [Table L13] (hereinafter, referred to as a compound group SX411).

A compound wherein in the compound (L-1), n is 0, $R^1$ is a benzyl group, $R^{3b}$ is a fluorine atom, $R^{3c}$ is an iodine atom, and Q is any of the substituents shown in [Table L1] to [Table L13] (hereinafter, referred to as a compound group SX412).

A compound wherein in the compound (L-1), n is 1, $R^1$ is a benzyl group, $R^{3b}$ is a fluorine atom, $R^{3c}$ is an iodine atom, and Q is any of the substituents shown in [Table L1] to [Table L13] (hereinafter, referred to as a compound group SX413).

A compound wherein in the compound (L-1), n is 2, $R^1$ is a benzyl group, $R^{3b}$ is a fluorine atom, $R^{3c}$ is an iodine atom, and Q is any of the substituents shown in [Table L1] to [Table L13] (hereinafter, referred to as a compound group SX414).

A compound wherein in the compound (L-1), n is 0, $R^1$ is a methyl group, $R^{3b}$ is a chlorine atom, $R^{3c}$ is an iodine atom, and Q is any of the substituents shown in [Table L1] to [Table L13] (hereinafter, referred to as a compound group

SX418).

A compound wherein in the compound (L-1), n is 1, $R^1$ is a methyl group, $R^{3b}$ is a chlorine atom, $R^{3c}$ is an iodine atom, and Q is any of the substituents shown in [Table L1] to [Table L13] (hereinafter, referred to as a compound group SX419).

A compound wherein in the compound (L-1), n is 2, $R^1$ is a methyl group, $R^{3b}$ is a chlorine atom, $R^{3c}$ is an iodine atom, and Q is any of the substituents shown in [Table L1] to [Table L13] (hereinafter, referred to as a compound group SX420).

A compound wherein in the compound (L-1), n is 0, $R^1$ is an ethyl group, $R^{3b}$ is a chlorine atom, $R^{3c}$ is an iodine atom, and Q is any of the substituents shown in [Table L1] to [Table L13] (hereinafter, referred to as a compound group SX421).

A compound wherein in the compound (L-1), n is 1, $R^1$ is an ethyl group, $R^{3b}$ is a chlorine atom, $R^{3c}$ is an iodine atom, and Q is any of the substituents shown in [Table L1] to [Table L13] (hereinafter, referred to as a compound group SX422).

A compound wherein in the compound (L-1), n is 2, $R^1$ is an ethyl group, $R^{3b}$ is a chlorine atom, $R^{3c}$ is an iodine atom, and Q is any of the substituents shown in [Table L1] to [Table L13] (hereinafter, referred to as a compound group SX423).

A compound wherein in the compound (L-1), n is 0, $R^1$ is an isopropyl group, $R^{3b}$ is a chlorine atom, $R^{3c}$ is an iodine atom, and Q is any of the substituents shown in [Table L1] to [Table L13] (hereinafter, referred to as a compound group SX424).

A compound wherein in the compound (L-1), n is 1, $R^1$ is an isopropyl group, $R^{3b}$ is a chlorine atom, $R^{3c}$ is an iodine atom, and Q is any of the substituents shown in [Table L1] to [Table L13] (hereinafter, referred to as a compound group SX425).

A compound wherein in the compound (L-1), n is 2, $R^1$ is an isopropyl group, $R^{3b}$ is a chlorine atom, $R^{3c}$ is an iodine atom, and Q is any of the substituents shown in [Table L1] to [Table L13] (hereinafter, referred to as a compound group SX426).

A compound wherein in the compound (L-1), n is 0, $R^1$ is a methylcyano group, $R^{3b}$ is a chlorine atom, $R^{3c}$ is an iodine atom, and Q is any of the substituents shown in [Table L1] to [Table L13] (hereinafter, referred to as a compound group SX427).

A compound wherein in the compound (L-1), n is 1, $R^1$ is a methylcyano group, $R^{3b}$ is a chlorine atom, $R^{3c}$ is an iodine atom, and Q is any of the substituents shown in [Table L1] to [Table L13] (hereinafter, referred to as a compound group SX428).

A compound wherein in the compound (L-1), n is 2, $R^1$ is a methylcyano group, $R^{3b}$ is a chlorine atom, $R^{3c}$ is an iodine atom, and Q is any of the substituents shown in [Table L1] to [Table L13] (hereinafter, referred to as a compound group SX429).

A compound wherein in the compound (L-1), n is 0, $R^1$ is a benzyl group, $R^{3b}$ is a chlorine atom, $R^{3c}$ is an iodine atom, and Q is any of the substituents shown in [Table L1] to [Table L13] (hereinafter, referred to as a compound group SX430).

A compound wherein in the compound (L-1), n is 1, $R^1$ is a benzyl group, $R^{3b}$ is a chlorine atom, $R^{3c}$ is an iodine atom, and Q is any of the substituents shown in [Table L1] to [Table L13] (hereinafter, referred to as a compound group SX431).

A compound wherein in the compound (L-1), n is 2, $R^1$ is a benzyl group, $R^{3b}$ is a chlorine atom, $R^{3c}$ is an iodine atom, and Q is any of the substituents shown in [Table L1] to [Table L13] (hereinafter, referred to as a compound group SX432).

A compound wherein in the compound (L-1), n is 0, $R^1$ is a methyl group, $R^{3b}$ is a bromine atom, $R^{3c}$ is an iodine atom, and Q is any of the substituents shown in [Table L1] to [Table L13] (hereinafter, referred to as a compound group SX436).

A compound wherein in the compound (L-1), n is 1, $R^1$ is a methyl group, $R^{3b}$ is a bromine atom, $R^{3c}$ is an iodine atom, and Q is any of the substituents shown in [Table L1] to [Table L13] (hereinafter, referred to as a compound group SX437).

A compound wherein in the compound (L-1), n is 2, $R^1$ is a methyl group, $R^{3b}$ is a bromine atom, $R^{3c}$ is an iodine atom, and Q is any of the substituents shown in [Table L1] to [Table L13] (hereinafter, referred to as a compound group SX438).

A compound wherein in the compound (L-1), n is 0, $R^1$ is an ethyl group, $R^{3b}$ is a bromine atom, $R^{3c}$ is an iodine atom, and Q is any of the substituents shown in [Table L1] to [Table L13] (hereinafter, referred to as a compound group SX439).

A compound wherein in the compound (L-1), n is 1, $R^1$ is an ethyl group, $R^{3b}$ is a bromine atom, $R^{3c}$ is an iodine atom, and Q is any of the substituents shown in [Table L1] to [Table L13] (hereinafter, referred to as a compound group SX440).

A compound wherein in the compound (L-1), n is 2, $R^1$ is an ethyl group, $R^{3b}$ is a bromine atom, $R^{3c}$ is an iodine atom, and Q is any of the substituents shown in [Table L1] to [Table L13] (hereinafter, referred to as a compound group SX441).

A compound wherein in the compound (L-1), n is 0, $R^1$ is an isopropyl group, $R^{3b}$ is a bromine atom, $R^{3c}$ is an iodine atom, and Q is any of the substituents shown in [Table L1] to [Table L13] (hereinafter, referred to as a compound group SX442).

A compound wherein in the compound (L-1), n is 1, $R^1$ is an isopropyl group, $R^{3b}$ is a bromine atom, $R^{3c}$ is an iodine atom, and Q is any of the substituents shown in [Table L1] to [Table L13] (hereinafter, referred to as a compound group SX443).

A compound wherein in the compound (L-1), n is 2, $R^1$ is an isopropyl group, $R^{3b}$ is a bromine atom, $R^{3c}$ is an iodine atom, and Q is any of the substituents shown in [Table L1] to [Table L13] (hereinafter, referred to as a compound group SX444).

A compound wherein in the compound (L-1), n is 0, $R^1$ is a methylcyano group, $R^{3b}$ is a bromine atom, $R^{3c}$ is an iodine atom, and Q is any of the substituents shown in [Table L1] to [Table L13] (hereinafter, referred to as a compound group SX445).

A compound wherein in the compound (L-1), n is 1, $R^1$ is a methylcyano group, $R^{3b}$ is a bromine atom, $R^{3c}$ is an iodine atom, and Q is any of the substituents shown in [Table L1] to [Table L13] (hereinafter, referred to as a compound group SX446).

A compound wherein in the compound (L-1), n is 2, $R^1$ is a methylcyano group, $R^{3b}$ is a bromine atom, $R^{3c}$ is an iodine atom, and Q is any of the substituents shown in [Table L1] to [Table L13] (hereinafter, referred to as a compound group SX447).

A compound wherein in the compound (L-1), n is 0, $R^1$ is a benzyl group, $R^{3b}$ is a bromine atom, $R^{3c}$ is an iodine atom, and Q is any of the substituents shown in [Table L1] to [Table L13] (hereinafter, referred to as compound group SX448).

A compound wherein in the compound (L-1), n is 1, $R^1$ is a benzyl group, $R^{3b}$ is a bromine atom, $R^{3c}$ is an iodine atom, and Q is any of the substituents shown in [Table L1] to [Table L13] (hereinafter, referred to as compound group SX449).

A compound wherein in the compound (L-1), n is 2, $R^1$ is a benzyl group, $R^{3b}$ is a bromine atom, $R^{3c}$ is an iodine atom, and Q is any of the substituents shown in [Table L1] to [Table L13] (hereinafter, referred to as compound group SX450).

A compound wherein in the compound (L-1), n is 0, $R^1$ is a methyl group, $R^{3b}$ is an iodine atom, $R^{3c}$ is an iodine atom, and Q is any of the substituents shown in [Table L1] to [Table L13] (hereinafter, referred to as a compound group SX454).

A compound wherein in the compound (L-1), n is 1, $R^1$ is a methyl group, $R^{3b}$ is an iodine atom, $R^{3c}$ is an iodine atom, and Q is any of the substituents shown in [Table L1] to [Table L13] (hereinafter, referred to as a compound group SX455).

A compound wherein in the compound (L-1), n is 2, $R^1$ is a methyl group, $R^{3b}$ is an iodine atom, $R^{3c}$ is an iodine atom, and Q is any of the substituents shown in [Table L1] to [Table L13] (hereinafter, referred to as a compound group SX456).

A compound wherein in the compound (L-1), n is 0, $R^1$ is an ethyl group, $R^{3b}$ is an iodine atom, $R^{3c}$ is an iodine atom, and Q is any of the substituents shown in [Table L1] to [Table L13] (hereinafter, referred to as a compound group SX457).

A compound wherein in the compound (L-1), n is 1, $R^1$ is an ethyl group, $R^{3b}$ is an iodine atom, $R^{3c}$ is an iodine atom, and Q is any of the substituents shown in [Table L1] to [Table L13] (hereinafter, referred to as a compound group SX458).

A compound wherein in the compound (L-1), n is 2, $R^1$ is an ethyl group, $R^{3b}$ is an iodine atom, $R^{3c}$ is an iodine atom, and Q is any of the substituents shown in [Table L1] to [Table L13] (hereinafter, referred to as a compound group SX459).

A compound wherein in the compound (L-1), n is 0, $R^1$ is an isopropyl group, $R^{3b}$ is an iodine atom, $R^{3c}$ is an iodine atom, and Q is any of the substituents shown in [Table L1] to [Table L13] (hereinafter, referred to as a compound group SX460).

A compound wherein in the compound (L-1), n is 1, $R^1$ is an isopropyl group, $R^{3b}$ is an iodine atom, $R^{3c}$ is an iodine atom, and Q is any of the substituents shown in [Table L1] to [Table L13] (hereinafter, referred to as a compound group SX461).

A compound wherein in the compound (L-1), n is 2, $R^1$ is an isopropyl group, $R^{3b}$ is an iodine atom, $R^{3c}$ is an iodine atom, and Q is any of the substituents shown in [Table L1] to [Table L13] (hereinafter, referred to as a compound group SX462).

A compound wherein in the compound (L-1), n is 0, $R^1$ is a methylcyano group, $R^{3b}$ is an iodine atom, $R^{3c}$ is an

iodine atom, and Q is any of the substituents shown in [Table L1] to [Table L13] (hereinafter, referred to as a compound group SX463).

A compound wherein in the compound (L-1), n is 1, $R^1$ is a methylcyano group, $R^{3b}$ is an iodine atom, $R^{3c}$ is an iodine atom, and Q is any of the substituents shown in [Table L1] to [Table L13] (hereinafter, referred to as a compound group SX464).

A compound wherein in the compound (L-1), n is 2, $R^1$ is a methylcyano group, $R^{3b}$ is an iodine atom, $R^{3c}$ is an iodine atom, and Q is any of the substituents shown in [Table L1] to [Table L13] (hereinafter, referred to as a compound group SX465).

A compound wherein in the compound (L-1), n is 0, $R^1$ is a benzyl group, $R^{3b}$ is an iodine atom, $R^{3c}$ is an iodine atom, and Q is any of the substituents shown in [Table L1] to [Table L13] (hereinafter, referred to as compound group SX466) .

A compound wherein in the compound (L-1), n is 1, $R^1$ is a benzyl group, $R^{3b}$ is an iodine atom, $R^{3c}$ is an iodine atom, and Q is any of the substituents shown in [Table L1] to [Table L13] (hereinafter, referred to as compound group SX467).

A compound wherein in the compound (L-1), n is 2, $R^1$ is a benzyl group, $R^{3b}$ is an iodine atom, $R^{3c}$ is an iodine atom, and Q is any of the substituents shown in [Table L1] to [Table L13] (hereinafter, referred to as compound group SX468).

A compound wherein in the compound (L-1), n is 0, $R^1$ is a methyl group, $R^{3b}$ is a trifluoromethyl group, $R^{3c}$ is an iodine atom, and Q is any of the substituents shown in [Table L1] to [Table L13] (hereinafter, referred to as a compound group SX472).

A compound wherein in the compound (L-1), n is 1, $R^1$ is a methyl group, $R^{3b}$ is a trifluoromethyl group, $R^{3c}$ is an iodine atom, and Q is any of the substituents shown in [Table L1] to [Table L13] (hereinafter, referred to as a compound group SX473).

A compound wherein in the compound (L-1), n is 2, $R^1$ is a methyl group, $R^{3b}$ is a trifluoromethyl group, $R^{3c}$ is an iodine atom, and Q is any of the substituents shown in [Table L1] to [Table L13] (hereinafter, referred to as a compound group SX474).

A compound wherein in the compound (L-1), n is 0, $R^1$ is an ethyl group, $R^{3b}$ is a trifluoromethyl group, $R^{3c}$ is an iodine atom, and Q is any of the substituents shown in [Table L1] to [Table L13] (hereinafter, referred to as a compound group SX475).

A compound wherein in the compound (L-1), n is 1, $R^1$ is an ethyl group, $R^{3b}$ is a trifluoromethyl group, $R^{3c}$ is an iodine atom, and Q is any of the substituents shown in [Table L1] to [Table L13] (hereinafter, referred to as a compound group SX476).

A compound wherein in the compound (L-1), n is 2, $R^1$ is an ethyl group, $R^{3b}$ is a trifluoromethyl group, $R^{3c}$ is an iodine atom, and Q is any of the substituents shown in [Table L1] to [Table L13] (hereinafter, referred to as a compound group SX477).

A compound wherein in the compound (L-1), n is 0, $R^1$ is an isopropyl group, $R^{3b}$ is a trifluoromethyl group, $R^{3c}$ is an iodine atom, and Q is any of the substituents shown in [Table L1] to [Table L13] (hereinafter, referred to as a compound group SX478).

A compound wherein in the compound (L-1), n is 1, $R^1$ is an isopropyl group, $R^{3b}$ is a trifluoromethyl group, $R^{3c}$ is an iodine atom, and Q is any of the substituents shown in [Table L1] to [Table L13] (hereinafter, referred to as a compound group SX479).

A compound wherein in the compound (L-1), n is 2, $R^1$ is an isopropyl group, $R^{3b}$ is a trifluoromethyl group, $R^{3c}$ is an iodine atom, and Q is any of the substituents shown in [Table L1] to [Table L13] (hereinafter, referred to as a compound group SX480).

A compound wherein in the compound (L-1), n is 0, $R^1$ is a methylcyano group, $R^{3b}$ is a trifluoromethyl group, $R^{3c}$ is an iodine atom, and Q is any of the substituents shown in [Table L1] to [Table L13] (hereinafter, referred to as a compound group SX481).

A compound wherein in the compound (L-1), n is 1, $R^1$ is a methylcyano group, $R^{3b}$ is a trifluoromethyl group, $R^{3c}$ is an iodine atom, and Q is any of the substituents shown in [Table L1] to [Table L13] (hereinafter, referred to as a compound group SX482).

A compound wherein in the compound (L-1), n is 2, $R^1$ is a methylcyano group, $R^{3b}$ is a trifluoromethyl group, $R^{3c}$ is an iodine atom, and Q is any of the substituents shown in [Table L1] to [Table L13] (hereinafter, referred to as a compound group SX483).

A compound wherein in the compound (L-1), n is 0, $R^1$ is a benzyl group, $R^{3b}$ is a trifluoromethyl group, $R^{3c}$ is an iodine atom, and Q is any of the substituents shown in [Table L1] to [Table L13] (hereinafter, referred to as a compound group SX484).

A compound wherein in the compound (L-1), n is 1, $R^1$ is a benzyl group, $R^{3b}$ is a trifluoromethyl group, $R^{3c}$ is an iodine atom, and Q is any of the substituents shown in [Table L1] to [Table L13] (hereinafter, referred to as a compound

group SX485).

A compound wherein in the compound (L-1), n is 2, $R^1$ is a benzyl group, $R^{3b}$ is a trifluoromethyl group, $R^{3c}$ is an iodine atom, and Q is any of the substituents shown in [Table L1] to [Table L13] (hereinafter, referred to as a compound group SX486).

A compound wherein in the compound (L-1), n is 0, $R^1$ is a methyl group, $R^{3b}$ is a difluoromethyl group, $R^{3c}$ is an iodine atom, and Q is any of the substituents shown in [Table L1] to [Table L13] (hereinafter, referred to as a compound group SX490).

A compound wherein in the compound (L-1), n is 1, $R^1$ is a methyl group, $R^{3b}$ is a difluoromethyl group, $R^{3c}$ is an iodine atom, and Q is any of the substituents shown in [Table L1] to [Table L13] (hereinafter, referred to as a compound group SX491).

A compound wherein in the compound (L-1), n is 2, $R^1$ is a methyl group, $R^{3b}$ is a difluoromethyl group, $R^{3c}$ is an iodine atom, and Q is any of the substituents shown in [Table L1] to [Table L13] (hereinafter, referred to as a compound group SX492).

A compound wherein in the compound (L-1), n is 0, $R^1$ is an ethyl group, $R^{3b}$ is a difluoromethyl group, $R^{3c}$ is an iodine atom, and Q is any of the substituents shown in [Table L1] to [Table L13] (hereinafter, referred to as a compound group SX493).

A compound wherein in the compound (L-1), n is 1, $R^1$ is an ethyl group, $R^{3b}$ is a difluoromethyl group, $R^{3c}$ is an iodine atom, and Q is any of the substituents shown in [Table L1] to [Table L13] (hereinafter, referred to as a compound group SX494).

A compound wherein in the compound (L-1), n is 2, $R^1$ is an ethyl group, $R^{3b}$ is a difluoromethyl group, $R^{3c}$ is an iodine atom, and Q is any of the substituents shown in [Table L1] to [Table L13] (hereinafter, referred to as a compound group SX495).

A compound wherein in the compound (L-1), n is 0, $R^1$ is an isopropyl group, $R^{3b}$ is a difluoromethyl group, $R^{3c}$ is an iodine atom, and Q is any of the substituents shown in [Table L1] to [Table L13] (hereinafter, referred to as a compound group SX496).

A compound wherein in the compound (L-1), n is 1, $R^1$ is an isopropyl group, $R^{3b}$ is a difluoromethyl group, $R^{3c}$ is an iodine atom, and Q is any of the substituents shown in [Table L1] to [Table L13] (hereinafter, referred to as a compound group SX497).

A compound wherein in the compound (L-1), n is 2, $R^1$ is an isopropyl group, $R^{3b}$ is a difluoromethyl group, $R^{3c}$ is an iodine atom, and Q is any of the substituents shown in [Table L1] to [Table L13] (hereinafter, referred to as a compound group SX498).

A compound wherein in the compound (L-1), n is 0, $R^1$ is a methylcyano group, $R^{3b}$ is a difluoromethyl group, $R^{3c}$ is an iodine atom, and Q is any of the substituents shown in [Table L1] to [Table L13] (hereinafter, referred to as a compound group SX499).

A compound wherein in the compound (L-1), n is 1, $R^1$ is a methylcyano group, $R^{3b}$ is a difluoromethyl group, $R^{3c}$ is an iodine atom, and Q is any of the substituents shown in [Table L1] to [Table L13] (hereinafter, referred to as a compound group SX500).

A compound wherein in the compound (L-1), n is 2, $R^1$ is a methylcyano group, $R^{3b}$ is a difluoromethyl group, $R^{3c}$ is an iodine atom, and Q is any of the substituents shown in [Table L1] to [Table L13] (hereinafter, referred to as a compound group SX501).

A compound wherein in the compound (L-1), n is 0, $R^1$ is a benzyl group, $R^{3b}$ is a difluoromethyl group, $R^{3c}$ is an iodine atom, and Q is any of the substituents shown in [Table L1] to [Table L13] (hereinafter, referred to as a compound group SX502).

A compound wherein in the compound (L-1), n is 1, $R^1$ is a benzyl group, $R^{3b}$ is a difluoromethyl group, $R^{3c}$ is an iodine atom, and Q is any of the substituents shown in [Table L1] to [Table L13] (hereinafter, referred to as a compound group SX503).

A compound wherein in the compound (L-1), n is 2, $R^1$ is a benzyl group, $R^{3b}$ is a difluoromethyl group, $R^{3c}$ is an iodine atom, and Q is any of the substituents shown in [Table L1] to [Table L13] (hereinafter, referred to as a compound group SX504).

A compound wherein in the compound (L-1), n is 0, $R^1$ is a methyl group, $R^{3b}$ is a hydrogen atom, $R^{3c}$ is a trifluoromethyl group, and Q is any of the substituents shown in [Table L1] to [Table L13] (hereinafter, referred to as a compound group SX508).

A compound wherein in the compound (L-1), n is 1, $R^1$ is a methyl group, $R^{3b}$ is a hydrogen atom, $R^{3c}$ is a trifluoromethyl group, and Q is any of the substituents shown in [Table L1] to [Table L13] (hereinafter, referred to as a compound group SX509).

A compound wherein in the compound (L-1), n is 2, $R^1$ is a methyl group, $R^{3b}$ is a hydrogen atom, $R^{3c}$ is a trifluoromethyl group, and Q is any of the substituents shown in [Table L1] to [Table L13] (hereinafter, referred to as a compound group SX510).

A compound wherein in the compound (L-1), n is 0, $R^1$ is an ethyl group, $R^{3b}$ is a hydrogen atom, $R^{3c}$ is a trifluoromethyl group, and Q is any of the substituents shown in [Table L1] to [Table L13] (hereinafter, referred to as a compound group SX511).

A compound wherein in the compound (L-1), n is 1, $R^1$ is an ethyl group, $R^{3b}$ is a hydrogen atom, $R^{3c}$ is a trifluoromethyl group, and Q is any of the substituents shown in [Table L1] to [Table L13] (hereinafter, referred to as a compound group SX512).

A compound wherein in the compound (L-1), n is 2, $R^1$ is an ethyl group, $R^{3b}$ is a hydrogen atom, $R^{3c}$ is a trifluoromethyl group, and Q is any of the substituents shown in [Table L1] to [Table L13] (hereinafter, referred to as a compound group SX513).

A compound wherein in the compound (L-1), n is 0, $R^1$ is an isopropyl group, $R^{3b}$ is a hydrogen atom, $R^{3c}$ is a trifluoromethyl group, and Q is any of the substituents shown in [Table L1] to [Table L13] (hereinafter, referred to as a compound group SX514).

A compound wherein in the compound (L-1), n is 1, $R^1$ is an isopropyl group, $R^{3b}$ is a hydrogen atom, $R^{3c}$ is a trifluoromethyl group, and Q is any of the substituents shown in [Table L1] to [Table L13] (hereinafter, referred to as a compound group SX515).

A compound wherein in the compound (L-1), n is 2, $R^1$ is an isopropyl group, $R^{3b}$ is a hydrogen atom, $R^{3c}$ is a trifluoromethyl group, and Q is any of the substituents shown in [Table L1] to [Table L13] (hereinafter, referred to as a compound group SX516).

A compound wherein in the compound (L-1), n is 0, $R^1$ is a methylcyano group, $R^{3b}$ is a hydrogen atom, $R^{3c}$ is a trifluoromethyl group, and Q is any of the substituents shown in [Table L1] to [Table L13] (hereinafter, referred to as a compound group SX517).

A compound wherein in the compound (L-1), n is 1, $R^1$ is a methylcyano group, $R^{3b}$ is a hydrogen atom, $R^{3c}$ is a trifluoromethyl group, and Q is any of the substituents shown in [Table L1] to [Table L13] (hereinafter, referred to as a compound group SX518).

A compound wherein in the compound (L-1), n is 2, $R^1$ is a methylcyano group, $R^{3b}$ is a hydrogen atom, $R^{3c}$ is a trifluoromethyl group, and Q is any of the substituents shown in [Table L1] to [Table L13] (hereinafter, referred to as a compound group SX519).

A compound wherein in the compound (L-1), n is 0, $R^1$ is a benzyl group, $R^{3b}$ is a hydrogen atom, $R^{3c}$ is a trifluoromethyl group, and Q is any of the substituents shown in [Table L1] to [Table L13] (hereinafter, referred to as a compound group SX520).

A compound wherein in the compound (L-1), n is 1, $R^1$ is a benzyl group, $R^{3b}$ is a hydrogen atom, $R^{3c}$ is a trifluoromethyl group, and Q is any of the substituents shown in [Table L1] to [Table L13] (hereinafter, referred to as a compound group SX521).

A compound wherein in the compound (L-1), n is 2, $R^1$ is a benzyl group, $R^{3b}$ is a hydrogen atom, $R^{3c}$ is a trifluoromethyl group, and Q is any of the substituents shown in [Table L1] to [Table L13] (hereinafter, referred to as a compound group SX522).

A compound wherein in the compound (L-1), n is 0, $R^1$ is a methyl group, $R^{3b}$ is a fluorine atom, $R^{3c}$ is a trifluoromethyl group, and Q is any of the substituents shown in [Table L1] to [Table L13] (hereinafter, referred to as a compound group SX526).

A compound wherein in the compound (L-1), n is 1, $R^1$ is a methyl group, $R^{3b}$ is a fluorine atom, $R^{3c}$ is a trifluoromethyl group, and Q is any of the substituents shown in [Table L1] to [Table L13] (hereinafter, referred to as a compound group SX527).

A compound wherein in the compound (L-1), n is 2, $R^1$ is a methyl group, $R^{3b}$ is a fluorine atom, $R^{3c}$ is a trifluoromethyl group, and Q is any of the substituents shown in [Table L1] to [Table L13] (hereinafter, referred to as a compound group SX528).

A compound wherein in the compound (L-1), n is 0, $R^1$ is an ethyl group, $R^{3b}$ is a fluorine atom, $R^{3c}$ is a trifluoromethyl group, and Q is any of the substituents shown in [Table L1] to [Table L13] (hereinafter, referred to as a compound group SX529).

A compound wherein in the compound (L-1), n is 1, $R^1$ is an ethyl group, $R^{3b}$ is a fluorine atom, $R^{3c}$ is a trifluoromethyl group, and Q is any of the substituents shown in [Table L1] to [Table L13] (hereinafter, referred to as a compound group SX530).

A compound wherein in the compound (L-1), n is 2, $R^1$ is an ethyl group, $R^{3b}$ is a fluorine atom, $R^{3c}$ is a trifluoromethyl group, and Q is any of the substituents shown in [Table L1] to [Table L13] (hereinafter, referred to as a compound group SX531).

A compound wherein in the compound (L-1), n is 0, $R^1$ is an isopropyl group, $R^{3b}$ is a fluorine atom, $R^{3c}$ is a trifluoromethyl group, and Q is any of the substituents shown in [Table L1] to [Table L13] (hereinafter, referred to as a compound group SX532).

A compound wherein in the compound (L-1), n is 1, $R^1$ is an isopropyl group, $R^{3b}$ is a fluorine atom, $R^{3c}$ is a

trifluoromethyl group, and Q is any of the substituents shown in [Table L1] to [Table L13] (hereinafter, referred to as a compound group SX533).

A compound wherein in the compound (L-1), n is 2, $R^1$ is an isopropyl group, $R^{3b}$ is a fluorine atom, $R^{3c}$ is a trifluoromethyl group, and Q is any of the substituents shown in [Table L1] to [Table L13] (hereinafter, referred to as a compound group SX534).

A compound wherein in the compound (L-1), n is 0, $R^1$ is a methylcyano group, $R^{3b}$ is a fluorine atom, $R^{3c}$ is a trifluoromethyl group, and Q is any of the substituents shown in [Table L1] to [Table L13] (hereinafter, referred to as a compound group SX535).

A compound wherein in the compound (L-1), n is 1, $R^1$ is a methylcyano group, $R^{3b}$ is a fluorine atom, $R^{3c}$ is a trifluoromethyl group, and Q is any of the substituents shown in [Table L1] to [Table L13] (hereinafter, referred to as a compound group SX536).

A compound wherein in the compound (L-1), n is 2, $R^1$ is a methylcyano group, $R^{3b}$ is a fluorine atom, $R^{3c}$ is a trifluoromethyl group, and Q is any of the substituents shown in [Table L1] to [Table L13] (hereinafter, referred to as a compound group SX537).

A compound wherein in the compound (L-1), n is 0, $R^1$ is a benzyl group, $R^{3b}$ is a fluorine atom, $R^{3c}$ is a trifluoromethyl group, and Q is any of the substituents shown in [Table L1] to [Table L13] (hereinafter, referred to as a compound group SX538).

A compound wherein in the compound (L-1), n is 1, $R^1$ is a benzyl group, $R^{3b}$ is a fluorine atom, $R^{3c}$ is a trifluoromethyl group, and Q is any of the substituents shown in [Table L1] to [Table L13] (hereinafter, referred to as a compound group SX539).

A compound wherein in the compound (L-1), n is 2, $R^1$ is a benzyl group, $R^{3b}$ is a fluorine atom, $R^{3c}$ is a trifluoromethyl group, and Q is any of the substituents shown in [Table L1] to [Table L13] (hereinafter, referred to as a compound group SX540).

A compound wherein in the compound (L-1), n is 0, $R^1$ is a methyl group, $R^{3b}$ is a chlorine atom, $R^{3c}$ is a trifluoromethyl group, and Q is any of the substituents shown in [Table L1] to [Table L13] (hereinafter, referred to as a compound group SX544).

A compound wherein in the compound (L-1), n is 1, $R^1$ is a methyl group, $R^{3b}$ is a chlorine atom, $R^{3c}$ is a trifluoromethyl group, and Q is any of the substituents shown in [Table L1] to [Table L13] (hereinafter, referred to as a compound group SX545).

A compound wherein in the compound (L-1), n is 2, $R^1$ is a methyl group, $R^{3b}$ is a chlorine atom, $R^{3c}$ is a trifluoromethyl group, and Q is any of the substituents shown in [Table L1] to [Table L13] (hereinafter, referred to as a compound group SX546).

A compound wherein in the compound (L-1), n is 0, $R^1$ is an ethyl group, $R^{3b}$ is a chlorine atom, $R^{3c}$ is a trifluoromethyl group, and Q is any of the substituents shown in [Table L1] to [Table L13] (hereinafter, referred to as a compound group SX547).

A compound wherein in the compound (L-1), n is 1, $R^1$ is an ethyl group, $R^{3b}$ is a chlorine atom, $R^{3c}$ is a trifluoromethyl group, and Q is any of the substituents shown in [Table L1] to [Table L13] (hereinafter, referred to as a compound group SX548).

A compound wherein in the compound (L-1), n is 2, $R^1$ is an ethyl group, $R^{3b}$ is a chlorine atom, $R^{3c}$ is a trifluoromethyl group, and Q is any of the substituents shown in [Table L1] to [Table L13] (hereinafter, referred to as a compound group SX549).

A compound wherein in the compound (L-1), n is 0, $R^1$ is an isopropyl group, $R^{3b}$ is a chlorine atom, $R^{3c}$ is a trifluoromethyl group, and Q is any of the substituents shown in [Table L1] to [Table L13] (hereinafter, referred to as a compound group SX550).

A compound wherein in the compound (L-1), n is 1, $R^1$ is an isopropyl group, $R^{3b}$ is a chlorine atom, $R^{3c}$ is a trifluoromethyl group, and Q is any of the substituents shown in [Table L1] to [Table L13] (hereinafter, referred to as a compound group SX551).

A compound wherein in the compound (L-1), n is 2, $R^1$ is an isopropyl group, $R^{3b}$ is a chlorine atom, $R^{3c}$ is a trifluoromethyl group, and Q is any of the substituents shown in [Table L1] to [Table L13] (hereinafter, referred to as a compound group SX552).

A compound wherein in the compound (L-1), n is 0, $R^1$ is a methylcyano group, $R^{3b}$ is a chlorine atom, $R^{3c}$ is a trifluoromethyl group, and Q is any of the substituents shown in [Table L1] to [Table L13] (hereinafter, referred to as a compound group SX553).

A compound wherein in the compound (L-1), n is 1, $R^1$ is a methylcyano group, $R^{3b}$ is a chlorine atom, $R^{3c}$ is a trifluoromethyl group, and Q is any of the substituents shown in [Table L1] to [Table L13] (hereinafter, referred to as a compound group SX554).

A compound wherein in the compound (L-1), n is 2, $R^1$ is a methylcyano group, $R^{3b}$ is a chlorine atom, $R^{3c}$ is a trifluoromethyl group, and Q is any of the substituents shown in [Table L1] to [Table L13] (hereinafter, referred to as a

compound group SX555).

A compound wherein in the compound (L-1), n is 0, $R^1$ is a benzyl group, $R^{3b}$ is a chlorine atom, $R^{3c}$ is a trifluoromethyl group, and Q is any of the substituents shown in [Table L1] to [Table L13] (hereinafter, referred to as a compound group SX556).

A compound wherein in the compound (L-1), n is 1, $R^1$ is a benzyl group, $R^{3b}$ is a chlorine atom, $R^{3c}$ is a trifluoromethyl group, and Q is any of the substituents shown in [Table L1] to [Table L13] (hereinafter, referred to as a compound group SX557).

A compound wherein in the compound (L-1), n is 2, $R^1$ is a benzyl group, $R^{3b}$ is a chlorine atom, $R^{3c}$ is a trifluoromethyl group, and Q is any of the substituents shown in [Table L1] to [Table L13] (hereinafter, referred to as a compound group SX558).

A compound wherein in the compound (L-1), n is 0, $R^1$ is a methyl group, $R^{3b}$ is a bromine atom, $R^{3c}$ is a trifluoromethyl group, and Q is any of the substituents shown in [Table L1] to [Table L13] (hereinafter, referred to as a compound group SX562).

A compound wherein in the compound (L-1), n is 1, $R^1$ is a methyl group, $R^{3b}$ is a bromine atom, $R^{3c}$ is a trifluoromethyl group, and Q is any of the substituents shown in [Table L1] to [Table L13] (hereinafter, referred to as a compound group SX563).

A compound wherein in the compound (L-1), n is 2, $R^1$ is a methyl group, $R^{3b}$ is a bromine atom, $R^{3c}$ is a trifluoromethyl group, and Q is any of the substituents shown in [Table L1] to [Table L13] (hereinafter, referred to as a compound group SX564).

A compound wherein in the compound (L-1), n is 0, $R^1$ is an ethyl group, $R^{3b}$ is a bromine atom, $R^{3c}$ is a trifluoromethyl group, and Q is any of the substituents shown in [Table L1] to [Table L13] (hereinafter, referred to as a compound group SX565).

A compound wherein in the compound (L-1), n is 1, $R^1$ is an ethyl group, $R^{3b}$ is a bromine atom, $R^{3c}$ is a trifluoromethyl group, and Q is any of the substituents shown in [Table L1] to [Table L13] (hereinafter, referred to as a compound group SX566).

A compound wherein in the compound (L-1), n is 2, $R^1$ is an ethyl group, $R^{3b}$ is a bromine atom, $R^{3c}$ is a trifluoromethyl group, and Q is any of the substituents shown in [Table L1] to [Table L13] (hereinafter, referred to as a compound group SX567).

A compound wherein in the compound (L-1), n is 0, $R^1$ is an isopropyl group, $R^{3b}$ is a bromine atom, $R^{3c}$ is a trifluoromethyl group, and Q is any of the substituents shown in [Table L1] to [Table L13] (hereinafter, referred to as a compound group SX568).

A compound wherein in the compound (L-1), n is 1, $R^1$ is an isopropyl group, $R^{3b}$ is a bromine atom, $R^{3c}$ is a trifluoromethyl group, and Q is any of the substituents shown in [Table L1] to [Table L13] (hereinafter, referred to as a compound group SX569).

A compound wherein in the compound (L-1), n is 2, $R^1$ is an isopropyl group, $R^{3b}$ is a bromine atom, $R^{3c}$ is a trifluoromethyl group, and Q is any of the substituents shown in [Table L1] to [Table L13] (hereinafter, referred to as a compound group SX570).

A compound wherein in the compound (L-1), n is 0, $R^1$ is a methylcyano group, $R^{3b}$ is a bromine atom, $R^{3c}$ is a trifluoromethyl group, and Q is any of the substituents shown in [Table L1] to [Table L13] (hereinafter, referred to as a compound group SX571).

A compound wherein in the compound (L-1), n is 1, $R^1$ is a methylcyano group, $R^{3b}$ is a bromine atom, $R^{3c}$ is a trifluoromethyl group, and Q is any of the substituents shown in [Table L1] to [Table L13] (hereinafter, referred to as a compound group SX572).

A compound wherein in the compound (L-1), n is 2, $R^1$ is a methylcyano group, $R^{3b}$ is a bromine atom, $R^{3c}$ is a trifluoromethyl group, and Q is any of the substituents shown in [Table L1] to [Table L13] (hereinafter, referred to as a compound group SX573).

A compound wherein in the compound (L-1), n is 0, $R^1$ is a benzyl group, $R^{3b}$ is a bromine atom, $R^{3c}$ is a trifluoromethyl group, and Q is any of the substituents shown in [Table L1] to [Table L13] (hereinafter, referred to as a compound group SX574).

A compound wherein in the compound (L-1), n is 1, $R^1$ is a benzyl group, $R^{3b}$ is a bromine atom, $R^{3c}$ is a trifluoromethyl group, and Q is any of the substituents shown in [Table L1] to [Table L13] (hereinafter, referred to as a compound group SX575).

A compound wherein in the compound (L-1), n is 2, $R^1$ is a benzyl group, $R^{3b}$ is a bromine atom, $R^{3c}$ is a trifluoromethyl group, and Q is any of the substituents shown in [Table L1] to [Table L13] (hereinafter, referred to as a compound group SX576).

A compound wherein in the compound (L-1), n is 0, $R^1$ is a methyl group, $R^{3b}$ is an iodine atom, $R^{3c}$ is a trifluoromethyl group, and Q is any of the substituents shown in [Table L1] to [Table L13] (hereinafter, referred to as a compound group SX580).

A compound wherein in the compound (L-1), n is 1, $R^1$ is a methyl group, $R^{3b}$ is an iodine atom, $R^{3c}$ is a trifluoromethyl group, and Q is any of the substituents shown in [Table L1] to [Table L13] (hereinafter, referred to as a compound group SX581).

A compound wherein in the compound (L-1), n is 2, $R^1$ is a methyl group, $R^{3b}$ is an iodine atom, $R^{3c}$ is a trifluoromethyl group, and Q is any of the substituents shown in [Table L1] to [Table L13] (hereinafter, referred to as a compound group SX582).

A compound wherein in the compound (L-1), n is 0, $R^1$ is an ethyl group, $R^{3b}$ is an iodine atom, $R^{3c}$ is a trifluoromethyl group, and Q is any of the substituents shown in [Table L1] to [Table L13] (hereinafter, referred to as a compound group SX583).

A compound wherein in the compound (L-1), n is 1, $R^1$ is an ethyl group, $R^{3b}$ is an iodine atom, $R^{3c}$ is a trifluoromethyl group, and Q is any of the substituents shown in [Table L1] to [Table L13] (hereinafter, referred to as a compound group SX584).

A compound wherein in the compound (L-1), n is 2, $R^1$ is an ethyl group, $R^{3b}$ is an iodine atom, $R^{3c}$ is a trifluoromethyl group, and Q is any of the substituents shown in [Table L1] to [Table L13] (hereinafter, referred to as a compound group SX585).

A compound wherein in the compound (L-1), n is 0, $R^1$ is an isopropyl group, $R^{3b}$ is an iodine atom, $R^{3c}$ is a trifluoromethyl group, and Q is any of the substituents shown in [Table L1] to [Table L13] (hereinafter, referred to as a compound group SX586).

A compound wherein in the compound (L-1), n is 1, $R^1$ is an isopropyl group, $R^{3b}$ is an iodine atom, $R^{3c}$ is a trifluoromethyl group, and Q is any of the substituents shown in [Table L1] to [Table L13] (hereinafter, referred to as a compound group SX587).

A compound wherein in the compound (L-1), n is 2, $R^1$ is an isopropyl group, $R^{3b}$ is an iodine atom, $R^{3c}$ is a trifluoromethyl group, and Q is any of the substituents shown in [Table L1] to [Table L13] (hereinafter, referred to as a compound group SX588).

A compound wherein in the compound (L-1), n is 0, $R^1$ is a methylcyano group, $R^{3b}$ is an iodine atom, $R^{3c}$ is a trifluoromethyl group, and Q is any of the substituents shown in [Table L1] to [Table L13] (hereinafter, referred to as a compound group SX589).

A compound wherein in the compound (L-1), n is 1, $R^1$ is a methylcyano group, $R^{3b}$ is an iodine atom, $R^{3c}$ is a trifluoromethyl group, and Q is any of the substituents shown in [Table L1] to [Table L13] (hereinafter, referred to as a compound group SX590).

A compound wherein in the compound (L-1), n is 2, $R^1$ is a methylcyano group, $R^{3b}$ is an iodine atom, $R^{3c}$ is a trifluoromethyl group, and Q is any of the substituents shown in [Table L1] to [Table L13] (hereinafter, referred to as a compound group SX591).

A compound wherein in the compound (L-1), n is 0, $R^1$ is a benzyl group, $R^{3b}$ is an iodine atom, $R^{3c}$ is a trifluoromethyl group, and Q is any of the substituents shown in [Table L1] to [Table L13] (hereinafter, referred to as a compound group SX592).

A compound wherein in the compound (L-1), n is 1, $R^1$ is a benzyl group, $R^{3b}$ is an iodine atom, $R^{3c}$ is a trifluoromethyl group, and Q is any of the substituents shown in [Table L1] to [Table L13] (hereinafter, referred to as a compound group SX593).

A compound wherein in the compound (L-1), n is 2, $R^1$ is a benzyl group, $R^{3b}$ is an iodine atom, $R^{3c}$ is a trifluoromethyl group, and Q is any of the substituents shown in [Table L1] to [Table L13] (hereinafter, referred to as a compound group SX594).

A compound wherein in the compound (L-1), n is 0, $R^1$ is a methyl group, $R^{3b}$ is a trifluoromethyl group, $R^{3c}$ is a trifluoromethyl group, and Q is any of the substituents shown in [Table L1] to [Table L13] (hereinafter, referred to as a compound group SX598).

A compound wherein in the compound (L-1), n is 1, $R^1$ is a methyl group, $R^{3b}$ is a trifluoromethyl group, $R^{3c}$ is a trifluoromethyl group, and Q is any of the substituents shown in [Table L1] to [Table L13] (hereinafter, referred to as a compound group SX599).

A compound wherein in the compound (L-1), n is 2, $R^1$ is a methyl group, $R^{3b}$ is a trifluoromethyl group, $R^{3c}$ is a trifluoromethyl group, and Q is any of the substituents shown in [Table L1] to [Table L13] (hereinafter, referred to as a compound group SX600).

A compound wherein in the compound (L-1), n is 0, $R^1$ is an ethyl group, $R^{3b}$ is a trifluoromethyl group, $R^{3c}$ is a trifluoromethyl group, and Q is any of the substituents shown in [Table L1] to [Table L13] (hereinafter, referred to as a compound group SX601).

A compound wherein in the compound (L-1), n is 1, $R^1$ is an ethyl group, $R^{3b}$ is a trifluoromethyl group, $R^{3c}$ is a trifluoromethyl group, and Q is any of the substituents shown in [Table L1] to [Table L13] (hereinafter, referred to as a compound group SX602).

A compound wherein in the compound (L-1), n is 2, $R^1$ is an ethyl group, $R^{3b}$ is a trifluoromethyl group, $R^{3c}$ is a

trifluoromethyl group, and Q is any of the substituents shown in [Table L1] to [Table L13] (hereinafter, referred to as a compound group SX603).

A compound wherein in the compound (L-1), n is 0, $R^1$ is an isopropyl group, $R^{3b}$ is a trifluoromethyl group, $R^{3c}$ is a trifluoromethyl group, and Q is any of the substituents shown in [Table L1] to [Table L13] (hereinafter, referred to as a compound group SX604).

A compound wherein in the compound (L-1), n is 1, $R^1$ is an isopropyl group, $R^{3b}$ is a trifluoromethyl group, $R^{3c}$ is a trifluoromethyl group, and Q is any of the substituents shown in [Table L1] to [Table L13] (hereinafter, referred to as a compound group SX605).

A compound wherein in the compound (L-1), n is 2, $R^1$ is an isopropyl group, $R^{3b}$ is a trifluoromethyl group, $R^{3c}$ is a trifluoromethyl group, and Q is any of the substituents shown in [Table L1] to [Table L13] (hereinafter, referred to as a compound group SX606).

A compound wherein in the compound (L-1), n is 0, $R^1$ is a methylcyano group, $R^{3b}$ is a trifluoromethyl group, $R^{3c}$ is a trifluoromethyl group, and Q is any of the substituents shown in [Table L1] to [Table L13] (hereinafter, referred to as a compound group SX607).

A compound wherein in the compound (L-1), n is 1, $R^1$ is a methylcyano group, $R^{3b}$ is a trifluoromethyl group, $R^{3c}$ is a trifluoromethyl group, and Q is any of the substituents shown in [Table L1] to [Table L13] (hereinafter, referred to as a compound group SX608).

A compound wherein in the compound (L-1), n is 2, $R^1$ is a methylcyano group, $R^{3b}$ is a trifluoromethyl group, $R^{3c}$ is a trifluoromethyl group, and Q is any of the substituents shown in [Table L1] to [Table L13] (hereinafter, referred to as a compound group SX609).

A compound wherein in the compound (L-1), n is 0, $R^1$ is a benzyl group, $R^{3b}$ is a trifluoromethyl group, $R^{3c}$ is a trifluoromethyl group, and Q is any of the substituents shown in [Table L1] to [Table L13] (hereinafter, referred to as a compound group SX610).

A compound wherein in the compound (L-1), n is 1, $R^1$ is a benzyl group, $R^{3b}$ is a trifluoromethyl group, $R^{3c}$ is a trifluoromethyl group, and Q is any of the substituents shown in [Table L1] to [Table L13] (hereinafter, referred to as a compound group SX611).

A compound wherein in the compound (L-1), n is 2, $R^1$ is a benzyl group, $R^{3b}$ is a trifluoromethyl group, $R^{3c}$ is a trifluoromethyl group, and Q is any of the substituents shown in [Table L1] to [Table L13] (hereinafter, referred to as a compound group SX612).

A compound wherein in the compound (L-1), n is 0, $R^1$ is a methyl group, $R^{3b}$ is a difluoromethyl group, $R^{3c}$ is a trifluoromethyl group, and Q is any of the substituents shown in [Table L1] to [Table L13] (hereinafter, referred to as a compound group SX616).

A compound wherein in the compound (L-1), n is 1, $R^1$ is a methyl group, $R^{3b}$ is a difluoromethyl group, $R^{3c}$ is a trifluoromethyl group, and Q is any of the substituents shown in [Table L1] to [Table L13] (hereinafter, referred to as a compound group SX617).

A compound wherein in the compound (L-1), n is 2, $R^1$ is a methyl group, $R^{3b}$ is a difluoromethyl group, $R^{3c}$ is a trifluoromethyl group, and Q is any of the substituents shown in [Table L1] to [Table L13] (hereinafter, referred to as a compound group SX618).

A compound wherein in the compound (L-1), n is 0, $R^1$ is an ethyl group, $R^{3b}$ is a difluoromethyl group, $R^{3c}$ is a trifluoromethyl group, and Q is any of the substituents shown in [Table L1] to [Table L13] (hereinafter, referred to as a compound group SX619).

A compound wherein in the compound (L-1), n is 1, $R^1$ is an ethyl group, $R^{3b}$ is a difluoromethyl group, $R^{3c}$ is a trifluoromethyl group, and Q is any of the substituents shown in [Table L1] to [Table L13] (hereinafter, referred to as a compound group SX620).

A compound wherein in the compound (L-1), n is 2, $R^1$ is an ethyl group, $R^{3b}$ is a difluoromethyl group, $R^{3c}$ is a trifluoromethyl group, and Q is any of the substituents shown in [Table L1] to [Table L13] (hereinafter, referred to as a compound group SX621).

A compound wherein in the compound (L-1), n is 0, $R^1$ is an isopropyl group, $R^{3b}$ is a difluoromethyl group, $R^{3c}$ is a trifluoromethyl group, and Q is any of the substituents shown in [Table L1] to [Table L13] (hereinafter, referred to as a compound group SX622).

A compound wherein in the compound (L-1), n is 1, $R^1$ is an isopropyl group, $R^{3b}$ is a difluoromethyl group, $R^{3c}$ is a trifluoromethyl group, and Q is any of the substituents shown in [Table L1] to [Table L13] (hereinafter, referred to as a compound group SX623).

A compound wherein in the compound (L-1), n is 2, $R^1$ is an isopropyl group, $R^{3b}$ is a difluoromethyl group, $R^{3c}$ is a trifluoromethyl group, and Q is any of the substituents shown in [Table L1] to [Table L13] (hereinafter, referred to as a compound group SX624).

A compound wherein in the compound (L-1), n is 0, $R^1$ is a methylcyano group, $R^{3b}$ is a difluoromethyl group, $R^{3c}$ is a trifluoromethyl group, and Q is any of the substituents shown in [Table L1] to [Table L13] (hereinafter, referred to as a

compound group SX625).

A compound wherein in the compound (L-1), n is 1, $R^1$ is a methylcyano group, $R^{3b}$ is a difluoromethyl group, $R^{3c}$ is a trifluoromethyl group, and Q is any of the substituents shown in [Table L1] to [Table L13] (hereinafter, referred to as a compound group SX626).

A compound wherein in the compound (L-1), n is 2, $R^1$ is a methylcyano group, $R^{3b}$ is a difluoromethyl group, $R^{3c}$ is a trifluoromethyl group, and Q is any of the substituents shown in [Table L1] to [Table L13] (hereinafter, referred to as a compound group SX627).

A compound wherein in the compound (L-1), n is 0, $R^1$ is a benzyl group, $R^{3b}$ is a difluoromethyl group, $R^{3c}$ is a trifluoromethyl group, and Q is any of the substituents shown in [Table L1] to [Table L13] (hereinafter, referred to as a compound group SX628).

A compound wherein in the compound (L-1), n is 1, $R^1$ is a benzyl group, $R^{3b}$ is a difluoromethyl group, $R^{3c}$ is a trifluoromethyl group, and Q is any of the substituents shown in [Table L1] to [Table L13] (hereinafter, referred to as a compound group SX629).

A compound wherein in the compound (L-1), n is 2, $R^1$ is a benzyl group, $R^{3b}$ is a difluoromethyl group, $R^{3c}$ is a trifluoromethyl group, and Q is any of the substituents shown in [Table L1] to [Table L13] (hereinafter, referred to as a compound group SX630).

A compound wherein in the compound (L-1), n is 0, $R^1$ is a methyl group, $R^{3b}$ is a hydrogen atom, $R^{3c}$ is a difluoromethyl group, and Q is any of the substituents shown in [Table L1] to [Table L13] (hereinafter, referred to as a compound group SX634).

A compound wherein in the compound (L-1), n is 1, $R^1$ is a methyl group, $R^{3b}$ is a hydrogen atom, $R^{3c}$ is a difluoromethyl group, and Q is any of the substituents shown in [Table L1] to [Table L13] (hereinafter, referred to as a compound group SX635).

A compound wherein in the compound (L-1), n is 2, $R^1$ is a methyl group, $R^{3b}$ is a hydrogen atom, $R^{3c}$ is a difluoromethyl group, and Q is any of the substituents shown in [Table L1] to [Table L13] (hereinafter, referred to as a compound group SX636).

A compound wherein in the compound (L-1), n is 0, $R^1$ is an ethyl group, $R^{3b}$ is a hydrogen atom, $R^{3c}$ is a difluoromethyl group, and Q is any of the substituents shown in [Table L1] to [Table L13] (hereinafter, referred to as a compound group SX637).

A compound wherein in the compound (L-1), n is 1, $R^1$ is an ethyl group, $R^{3b}$ is a hydrogen atom, $R^{3c}$ is a difluoromethyl group, and Q is any of the substituents shown in [Table L1] to [Table L13] (hereinafter, referred to as a compound group SX638).

A compound wherein in the compound (L-1), n is 2, $R^1$ is an ethyl group, $R^{3b}$ is a hydrogen atom, $R^{3c}$ is a difluoromethyl group, and Q is any of the substituents shown in [Table L1] to [Table L13] (hereinafter, referred to as a compound group SX639).

A compound wherein in the compound (L-1), n is 0, $R^1$ is an isopropyl group, $R^{3b}$ is a hydrogen atom, $R^{3c}$ is a difluoromethyl group, and Q is any of the substituents shown in [Table L1] to [Table L13] (hereinafter, referred to as a compound group SX640).

A compound wherein in the compound (L-1), n is 1, $R^1$ is an isopropyl group, $R^{3b}$ is a hydrogen atom, $R^{3c}$ is a difluoromethyl group, and Q is any of the substituents shown in [Table L1] to [Table L13] (hereinafter, referred to as a compound group SX641).

A compound wherein in the compound (L-1), n is 2, $R^1$ is an isopropyl group, $R^{3b}$ is a hydrogen atom, $R^{3c}$ is a difluoromethyl group, and Q is any of the substituents shown in [Table L1] to [Table L13] (hereinafter, referred to as a compound group SX642).

A compound wherein in the compound (L-1), n is 0, $R^1$ is a methylcyano group, $R^{3b}$ is a hydrogen atom, $R^{3c}$ is a difluoromethyl group, and Q is any of the substituents shown in [Table L1] to [Table L13] (hereinafter, referred to as a compound group SX643).

A compound wherein in the compound (L-1), n is 1, $R^1$ is a methylcyano group, $R^{3b}$ is a hydrogen atom, $R^{3c}$ is a difluoromethyl group, and Q is any of the substituents shown in [Table L1] to [Table L13] (hereinafter, referred to as a compound group SX644).

A compound wherein in the compound (L-1), n is 2, $R^1$ is a methylcyano group, $R^{3b}$ is a hydrogen atom, $R^{3c}$ is a difluoromethyl group, and Q is any of the substituents shown in [Table L1] to [Table L13] (hereinafter, referred to as a compound group SX645).

A compound wherein in the compound (L-1), n is 0, $R^1$ is a benzyl group, $R^{3b}$ is a hydrogen atom, $R^{3c}$ is a difluoromethyl group, and Q is any of the substituents shown in [Table L1] to [Table L13] (hereinafter, referred to as a compound group SX646).

A compound wherein in the compound (L-1), n is 1, $R^1$ is a benzyl group, $R^{3b}$ is a hydrogen atom, $R^{3c}$ is a difluoromethyl group, and Q is any of the substituents shown in [Table L1] to [Table L13] (hereinafter, referred to as a compound group SX647).

A compound wherein in the compound (L-1), n is 2, $R^1$ is a benzyl group, $R^{3b}$ is a hydrogen atom, $R^{3c}$ is a difluoromethyl group, and Q is any of the substituents shown in [Table L1] to [Table L13] (hereinafter, referred to as a compound group SX648).

A compound wherein in the compound (L-1), n is 0, $R^1$ is a methyl group, $R^{3b}$ is a fluorine atom, $R^{3c}$ is a difluoromethyl group, and Q is any of the substituents shown in [Table L1] to [Table L13] (hereinafter, referred to as a compound group SX652).

A compound wherein in the compound (L-1), n is 1, $R^1$ is a methyl group, $R^{3b}$ is a fluorine atom, $R^{3c}$ is a difluoromethyl group, and Q is any of the substituents shown in [Table L1] to [Table L13] (hereinafter, referred to as a compound group SX653).

A compound wherein in the compound (L-1), n is 2, $R^1$ is a methyl group, $R^{3b}$ is a fluorine atom, $R^{3c}$ is a difluoromethyl group, and Q is any of the substituents shown in [Table L1] to [Table L13] (hereinafter, referred to as a compound group SX654).

A compound wherein in the compound (L-1), n is 0, $R^1$ is an ethyl group, $R^{3b}$ is a fluorine atom, $R^{3c}$ is a difluoromethyl group, and Q is any of the substituents shown in [Table L1] to [Table L13] (hereinafter, referred to as a compound group SX655).

A compound wherein in the compound (L-1), n is 1, $R^1$ is an ethyl group, $R^{3b}$ is a fluorine atom, $R^{3c}$ is a difluoromethyl group, and Q is any of the substituents shown in [Table L1] to [Table L13] (hereinafter, referred to as a compound group SX656).

A compound wherein in the compound (L-1), n is 2, $R^1$ is an ethyl group, $R^{3b}$ is a fluorine atom, $R^{3c}$ is a difluoromethyl group, and Q is any of the substituents shown in [Table L1] to [Table L13] (hereinafter, referred to as a compound group SX657).

A compound wherein in the compound (L-1), n is 0, $R^1$ is an isopropyl group, $R^{3b}$ is a fluorine atom, $R^{3c}$ is a difluoromethyl group, and Q is any of the substituents shown in [Table L1] to [Table L13] (hereinafter, referred to as a compound group SX658).

A compound wherein in the compound (L-1), n is 1, $R^1$ is an isopropyl group, $R^{3b}$ is a fluorine atom, $R^{3c}$ is a difluoromethyl group, and Q is any of the substituents shown in [Table L1] to [Table L13] (hereinafter, referred to as a compound group SX659).

A compound wherein in the compound (L-1), n is 2, $R^1$ is an isopropyl group, $R^{3b}$ is a fluorine atom, $R^{3c}$ is a difluoromethyl group, and Q is any of the substituents shown in [Table L1] to [Table L13] (hereinafter, referred to as a compound group SX660).

A compound wherein in the compound (L-1), n is 0, $R^1$ is a methylcyano group, $R^{3b}$ is a fluorine atom, $R^{3c}$ is a difluoromethyl group, and Q is any of the substituents shown in [Table L1] to [Table L13] (hereinafter, referred to as a compound group SX661).

A compound wherein in the compound (L-1), n is 1, $R^1$ is a methylcyano group, $R^{3b}$ is a fluorine atom, $R^{3c}$ is a difluoromethyl group, and Q is any of the substituents shown in [Table L1] to [Table L13] (hereinafter, referred to as a compound group SX662).

A compound wherein in the compound (L-1), n is 2, $R^1$ is a methylcyano group, $R^{3b}$ is a fluorine atom, $R^{3c}$ is a difluoromethyl group, and Q is any of the substituents shown in [Table L1] to [Table L13] (hereinafter, referred to as a compound group SX663).

A compound wherein in the compound (L-1), n is 0, $R^1$ is a benzyl group, $R^{3b}$ is a fluorine atom, $R^{3c}$ is a difluoromethyl group, and Q is any of the substituents shown in [Table L1] to [Table L13] (hereinafter, referred to as a compound group SX664).

A compound wherein in the compound (L-1), n is 1, $R^1$ is a benzyl group, $R^{3b}$ is a fluorine atom, $R^{3c}$ is a difluoromethyl group, and Q is any of the substituents shown in [Table L1] to [Table L13] (hereinafter, referred to as a compound group SX665).

A compound wherein in the compound (L-1), n is 2, $R^1$ is a benzyl group, $R^{3b}$ is a fluorine atom, $R^{3c}$ is a difluoromethyl group, and Q is any of the substituents shown in [Table L1] to [Table L13] (hereinafter, referred to as a compound group SX666).

A compound wherein in the compound (L-1), n is 0, $R^1$ is a methyl group, $R^{3b}$ is a chlorine atom, $R^{3c}$ is a difluoromethyl group, and Q is any of the substituents shown in [Table L1] to [Table L13] (hereinafter, referred to as a compound group SX670).

A compound wherein in the compound (L-1), n is 1, $R^1$ is a methyl group, $R^{3b}$ is a chlorine atom, $R^{3c}$ is a difluoromethyl group, and Q is any of the substituents shown in [Table L1] to [Table L13] (hereinafter, referred to as a compound group SX671).

A compound wherein in the compound (L-1), n is 2, $R^1$ is a methyl group, $R^{3b}$ is a chlorine atom, $R^{3c}$ is a difluoromethyl group, and Q is any of the substituents shown in [Table L1] to [Table L13] (hereinafter, referred to as a compound group SX672).

A compound wherein in the compound (L-1), n is 0, $R^1$ is an ethyl group, $R^{3b}$ is a chlorine atom, $R^{3c}$ is a

difluoromethyl group, and Q is any of the substituents shown in [Table L1] to [Table L13] (hereinafter, referred to as a compound group SX673).

A compound wherein in the compound (L-1), n is 1, $R^1$ is an ethyl group, $R^{3b}$ is a chlorine atom, $R^{3c}$ is a difluoromethyl group, and Q is any of the substituents shown in [Table L1] to [Table L13] (hereinafter, referred to as a compound group SX674).

A compound wherein in the compound (L-1), n is 2, $R^1$ is an ethyl group, $R^{3b}$ is a chlorine atom, $R^{3c}$ is a difluoromethyl group, and Q is any of the substituents shown in [Table L1] to [Table L13] (hereinafter, referred to as a compound group SX675).

A compound wherein in the compound (L-1), n is 0, $R^1$ is an isopropyl group, $R^{3b}$ is a chlorine group, $R^{3c}$ is a difluoromethyl group, and Q is any of the substituents shown in [Table L1] to [Table L13] (hereinafter, referred to as a compound group SX676).

A compound wherein in the compound (L-1), n is 1, $R^1$ is an isopropyl group, $R^{3b}$ is a chlorine group, $R^{3c}$ is a difluoromethyl group, and Q is any of the substituents shown in [Table L1] to [Table L13] (hereinafter, referred to as a compound group SX677).

A compound wherein in the compound (L-1), n is 2, $R^1$ is an isopropyl group, $R^{3b}$ is a chlorine group, $R^{3c}$ is a difluoromethyl group, and Q is any of the substituents shown in [Table L1] to [Table L13] (hereinafter, referred to as a compound group SX678).

A compound wherein in the compound (L-1), n is 0, $R^1$ is a methylcyano group, $R^{3b}$ is a chlorine group, $R^{3c}$ is a difluoromethyl group, and Q is any of the substituents shown in [Table L1] to [Table L13] (hereinafter, referred to as a compound group SX679).

A compound wherein in the compound (L-1), n is 1, $R^1$ is a methylcyano group, $R^{3b}$ is a chlorine group, $R^{3c}$ is a difluoromethyl group, and Q is any of the substituents shown in [Table L1] to [Table L13] (hereinafter, referred to as a compound group SX680).

A compound wherein in the compound (L-1), n is 2, $R^1$ is a methylcyano group, $R^{3b}$ is a chlorine group, $R^{3c}$ is a difluoromethyl group, and Q is any of the substituents shown in [Table L1] to [Table L13] (hereinafter, referred to as a compound group SX681).

A compound wherein in the compound (L-1), n is 0, $R^1$ is a benzyl group, $R^{3b}$ is a chlorine atom, $R^{3c}$ is a difluoromethyl group, and Q is any of the substituents shown in [Table L1] to [Table L13] (hereinafter, referred to as a compound group SX682).

A compound wherein in the compound (L-1), n is 1, $R^1$ is a benzyl group, $R^{3b}$ is a chlorine atom, $R^{3c}$ is a difluoromethyl group, and Q is any of the substituents shown in [Table L1] to [Table L13] (hereinafter, referred to as a compound group SX683).

A compound wherein in the compound (L-1), n is 2, $R^1$ is a benzyl group, $R^{3b}$ is a chlorine atom, $R^{3c}$ is a difluoromethyl group, and Q is any of the substituents shown in [Table L1] to [Table L13] (hereinafter, referred to as a compound group SX684).

A compound wherein in the compound (L-1), n is 0, $R^1$ is a methyl group, $R^{3b}$ is a bromine atom, $R^{3c}$ is a difluoromethyl group, and Q is any of the substituents shown in [Table L1] to [Table L13] (hereinafter, referred to as a compound group SX688).

A compound wherein in the compound (L-1), n is 1, $R^1$ is a methyl group, $R^{3b}$ is a bromine atom, $R^{3c}$ is a difluoromethyl group, and Q is any of the substituents shown in [Table L1] to [Table L13] (hereinafter, referred to as a compound group SX689).

A compound wherein in the compound (L-1), n is 2, $R^1$ is a methyl group, $R^{3b}$ is a bromine atom, $R^{3c}$ is a difluoromethyl group, and Q is any of the substituents shown in [Table L1] to [Table L13] (hereinafter, referred to as a compound group SX690).

A compound wherein in the compound (L-1), n is 0, $R^1$ is an ethyl group, $R^{3b}$ is a bromine atom, $R^{3c}$ is a difluoromethyl group, and Q is any of the substituents shown in [Table L1] to [Table L13] (hereinafter, referred to as a compound group SX691).

A compound wherein in the compound (L-1), n is 1, $R^1$ is an ethyl group, $R^{3b}$ is a bromine atom, $R^{3c}$ is a difluoromethyl group, and Q is any of the substituents shown in [Table L1] to [Table L13] (hereinafter, referred to as a compound group SX692).

A compound wherein in the compound (L-1), n is 2, $R^1$ is an ethyl group, $R^{3b}$ is a bromine atom, $R^{3c}$ is a difluoromethyl group, and Q is any of the substituents shown in [Table L1] to [Table L13] (hereinafter, referred to as a compound group SX693).

A compound wherein in the compound (L-1), n is 0, $R^1$ is an isopropyl group, $R^{3b}$ is a bromine atom, $R^{3c}$ is a difluoromethyl group, and Q is any of the substituents shown in [Table L1] to [Table L13] (hereinafter, referred to as a compound group SX694).

A compound wherein in the compound (L-1), n is 1, $R^1$ is an isopropyl group, $R^{3b}$ is a bromine atom, $R^{3c}$ is a difluoromethyl group, and Q is any of the substituents shown in [Table L1] to [Table L13] (hereinafter, referred to as a

compound group SX695).

A compound wherein in the compound (L-1), n is 2, $R^1$ is an isopropyl group, $R^{3b}$ is a bromine atom, $R^{3c}$ is a difluoromethyl group, and Q is any of the substituents shown in [Table L1] to [Table L13] (hereinafter, referred to as a compound group SX696).

A compound wherein in the compound (L-1), n is 0, $R^1$ is a methylcyano group, $R^{3b}$ is a bromine atom, $R^{3c}$ is a difluoromethyl group, and Q is any of the substituents shown in [Table L1] to [Table L13] (hereinafter, referred to as a compound group SX697).

A compound wherein in the compound (L-1), n is 1, $R^1$ is a methylcyano group, $R^{3b}$ is a bromine atom, $R^{3c}$ is a difluoromethyl group, and Q is any of the substituents shown in [Table L1] to [Table L13] (hereinafter, referred to as a compound group SX698).

A compound wherein in the compound (L-1), n is 2, $R^1$ is a methylcyano group, $R^{3b}$ is a bromine atom, $R^{3c}$ is a difluoromethyl group, and Q is any of the substituents shown in [Table L1] to [Table L13] (hereinafter, referred to as a compound group SX699).

A compound wherein in the compound (L-1), n is 0, $R^1$ is a benzyl group, $R^{3b}$ is a bromine atom, $R^{3c}$ is a difluoromethyl group, and Q is any of the substituents shown in [Table L1] to [Table L13] (hereinafter, referred to as a compound group SX700).

A compound wherein in the compound (L-1), n is 1, $R^1$ is a benzyl group, $R^{3b}$ is a bromine atom, $R^{3c}$ is a difluoromethyl group, and Q is any of the substituents shown in [Table L1] to [Table L13] (hereinafter, referred to as a compound group SX701).

A compound wherein in the compound (L-1), n is 2, $R^1$ is a benzyl group, $R^{3b}$ is a bromine atom, $R^{3c}$ is a difluoromethyl group, and Q is any of the substituents shown in [Table L1] to [Table L13] (hereinafter, referred to as a compound group SX702).

A compound wherein in the compound (L-1), n is 0, $R^1$ is a methyl group, $R^{3b}$ is an iodine atom, $R^{3c}$ is a difluoromethyl group, and Q is any of the substituents shown in [Table L1] to [Table L13] (hereinafter, referred to as a compound group SX706).

A compound wherein in the compound (L-1), n is 1, $R^1$ is a methyl group, $R^{3b}$ is an iodine atom, $R^{3c}$ is a difluoromethyl group, and Q is any of the substituents shown in [Table L1] to [Table L13] (hereinafter, referred to as a compound group SX707).

A compound wherein in the compound (L-1), n is 2, $R^1$ is a methyl group, $R^{3b}$ is an iodine atom, $R^{3c}$ is a difluoromethyl group, and Q is any of the substituents shown in [Table L1] to [Table L13] (hereinafter, referred to as a compound group SX708).

A compound wherein in the compound (L-1), n is 0, $R^1$ is an ethyl group, $R^{3b}$ is an iodine atom, $R^{3c}$ is a difluoromethyl group, and Q is any of the substituents shown in [Table L1] to [Table L13] (hereinafter, referred to as a compound group SX709).

A compound wherein in the compound (L-1), n is 1, $R^1$ is an ethyl group, $R^{3b}$ is an iodine atom, $R^{3c}$ is a difluoromethyl group, and Q is any of the substituents shown in [Table L1] to [Table L13] (hereinafter, referred to as a compound group SX710).

A compound wherein in the compound (L-1), n is 2, $R^1$ is an ethyl group, $R^{3b}$ is an iodine atom, $R^{3c}$ is a difluoromethyl group, and Q is any of the substituents shown in [Table L1] to [Table L13] (hereinafter, referred to as a compound group SX711).

A compound wherein in the compound (L-1), n is 0, $R^1$ is an isopropyl group, $R^{3b}$ is an iodine atom, $R^{3c}$ is a difluoromethyl group, and Q is any of the substituents shown in [Table L1] to [Table L13] (hereinafter, referred to as a compound group SX712).

A compound wherein in the compound (L-1), n is 1, $R^1$ is an isopropyl group, $R^{3b}$ is an iodine atom, $R^{3c}$ is a difluoromethyl group, and Q is any of the substituents shown in [Table L1] to [Table L13] (hereinafter, referred to as a compound group SX713).

A compound wherein in the compound (L-1), n is 2, $R^1$ is an isopropyl group, $R^{3b}$ is an iodine atom, $R^{3c}$ is a difluoromethyl group, and Q is any of the substituents shown in [Table L1] to [Table L13] (hereinafter, referred to as a compound group SX714).

A compound wherein in the compound (L-1), n is 0, $R^1$ is a methylcyano group, $R^{3b}$ is an iodine atom, $R^{3c}$ is a difluoromethyl group, and Q is any of the substituents shown in [Table L1] to [Table L13] (hereinafter, referred to as a compound group SX715).

A compound wherein in the compound (L-1), n is 1, $R^1$ is a methylcyano group, $R^{3b}$ is an iodine atom, $R^{3c}$ is a difluoromethyl group, and Q is any of the substituents shown in [Table L1] to [Table L13] (hereinafter, referred to as a compound group SX716).

A compound wherein in the compound (L-1), n is 2, $R^1$ is a methylcyano group, $R^{3b}$ is an iodine atom, $R^{3c}$ is a difluoromethyl group, and Q is any of the substituents shown in [Table L1] to [Table L13] (hereinafter, referred to as a compound group SX717).

A compound wherein in the compound (L-1), n is 0, $R^1$ is a benzyl group, $R^{3b}$ is an iodine atom, $R^{3c}$ is a difluoromethyl group, and Q is any of the substituents shown in [Table L1] to [Table L13] (hereinafter, referred to as a compound group SX718).

A compound wherein in the compound (L-1), n is 1, $R^1$ is a benzyl group, $R^{3b}$ is an iodine atom, $R^{3c}$ is a difluoromethyl group, and Q is any of the substituents shown in [Table L1] to [Table L13] (hereinafter, referred to as a compound group SX719).

A compound wherein in the compound (L-1), n is 2, $R^1$ is a benzyl group, $R^{3b}$ is an iodine atom, $R^{3c}$ is a difluoromethyl group, and Q is any of the substituents shown in [Table L1] to [Table L13] (hereinafter, referred to as a compound group SX720).

A compound wherein in the compound (L-1), n is 0, $R^1$ is a methyl group, $R^{3b}$ is a trifluoromethyl group, $R^{3c}$ is a difluoromethyl group, and Q is any of the substituents shown in [Table L1] to [Table L13] (hereinafter, referred to as a compound group SX724).

A compound wherein in the compound (L-1), n is 1, $R^1$ is a methyl group, $R^{3b}$ is a trifluoromethyl group, $R^{3c}$ is a difluoromethyl group, and Q is any of the substituents shown in [Table L1] to [Table L13] (hereinafter, referred to as a compound group SX725).

A compound wherein in the compound (L-1), n is 2, $R^1$ is a methyl group, $R^{3b}$ is a trifluoromethyl group, $R^{3c}$ is a difluoromethyl group, and Q is any of the substituents shown in [Table L1] to [Table L13] (hereinafter, referred to as a compound group SX726).

A compound wherein in the compound (L-1), n is 0, $R^1$ is an ethyl group, $R^{3b}$ is a trifluoromethyl group, $R^{3c}$ is a difluoromethyl group, and Q is any of the substituents shown in [Table L1] to [Table L13] (hereinafter, referred to as a compound group SX727).

A compound wherein in the compound (L-1), n is 1, $R^1$ is an ethyl group, $R^{3b}$ is a trifluoromethyl group, $R^{3c}$ is a difluoromethyl group, and Q is any of the substituents shown in [Table L1] to [Table L13] (hereinafter, referred to as a compound group SX728).

A compound wherein in the compound (L-1), n is 2, $R^1$ is an ethyl group, $R^{3b}$ is a trifluoromethyl group, $R^{3c}$ is a difluoromethyl group, and Q is any of the substituents shown in [Table L1] to [Table L13] (hereinafter, referred to as a compound group SX729).

A compound wherein in the compound (L-1), n is 0, $R^1$ is an isopropyl group, $R^{3b}$ is a trifluoromethyl group, $R^{3c}$ is a difluoromethyl group, and Q is any of the substituents shown in [Table L1] to [Table L13] (hereinafter, referred to as a compound group SX730).

A compound wherein in the compound (L-1), n is 1, $R^1$ is an isopropyl group, $R^{3b}$ is a trifluoromethyl group, $R^{3c}$ is a difluoromethyl group, and Q is any of the substituents shown in [Table L1] to [Table L13] (hereinafter, referred to as a compound group SX731).

A compound wherein in the compound (L-1), n is 2, $R^1$ is an isopropyl group, $R^{3b}$ is a trifluoromethyl group, $R^{3c}$ is a difluoromethyl group, and Q is any of the substituents shown in [Table L1] to [Table L13] (hereinafter, referred to as a compound group SX732).

A compound wherein in the compound (L-1), n is 0, $R^1$ is a methylcyano group, $R^{3b}$ is a trifluoromethyl group, $R^{3c}$ is a difluoromethyl group, and Q is any of the substituents shown in [Table L1] to [Table L13] (hereinafter, referred to as a compound group SX733).

A compound wherein in the compound (L-1), n is 1, $R^1$ is a methylcyano group, $R^{3b}$ is a trifluoromethyl group, $R^{3c}$ is a difluoromethyl group, and Q is any of the substituents shown in [Table L1] to [Table L13] (hereinafter, referred to as a compound group SX734).

A compound wherein in the compound (L-1), n is 2, $R^1$ is a methylcyano group, $R^{3b}$ is a trifluoromethyl group, $R^{3c}$ is a difluoromethyl group, and Q is any of the substituents shown in [Table L1] to [Table L13] (hereinafter, referred to as a compound group SX735).

A compound wherein in the compound (L-1), n is 0, $R^1$ is a benzyl group, $R^{3b}$ is a trifluoromethyl group, $R^{3c}$ is a difluoromethyl group, and Q is any of the substituents shown in [Table L1] to [Table L13] (hereinafter, referred to as a compound group SX736).

A compound wherein in the compound (L-1), n is 1, $R^1$ is a benzyl group, $R^{3b}$ is a trifluoromethyl group, $R^{3c}$ is a difluoromethyl group, and Q is any of the substituents shown in [Table L1] to [Table L13] (hereinafter, referred to as a compound group SX737).

A compound wherein in the compound (L-1), n is 2, $R^1$ is a benzyl group, $R^{3b}$ is a trifluoromethyl group, $R^{3c}$ is a difluoromethyl group, and Q is any of the substituents shown in [Table L1] to [Table L13] (hereinafter, referred to as a compound group SX738).

A compound wherein in the compound (L-1), n is 0, $R^1$ is a methyl group, $R^{3b}$ is a difluoromethyl group, $R^{3c}$ is a difluoromethyl group, and Q is any of the substituents shown in [Table L1] to [Table L13] (hereinafter, referred to as a compound group SX742).

A compound wherein in the compound (L-1), n is 1, $R^1$ is a methyl group, $R^{3b}$ is a difluoromethyl group, $R^{3c}$ is a

difluoromethyl group, and Q is any of the substituents shown in [Table L1] to [Table L13] (hereinafter, referred to as a compound group SX743).

A compound wherein in the compound (L-1), n is 2, $R^1$ is a methyl group, $R^{3b}$ is a difluoromethyl group, $R^{3c}$ is a difluoromethyl group, and Q is any of the substituents shown in [Table L1] to [Table L13] (hereinafter, referred to as a compound group SX744).

A compound wherein in the compound (L-1), n is 0, $R^1$ is an ethyl group, $R^{3b}$ is a difluoromethyl group, $R^{3c}$ is a difluoromethyl group, and Q is any of the substituents shown in [Table L1] to [Table L13] (hereinafter, referred to as a compound group SX745).

A compound wherein in the compound (L-1), n is 1, $R^1$ is an ethyl group, $R^{3b}$ is a difluoromethyl group, $R^{3c}$ is a difluoromethyl group, and Q is any of the substituents shown in [Table L1] to [Table L13] (hereinafter, referred to as a compound group SX746).

A compound wherein in the compound (L-1), n is 2, $R^1$ is an ethyl group, $R^{3b}$ is a difluoromethyl group, $R^{3c}$ is a difluoromethyl group, and Q is any of the substituents shown in [Table L1] to [Table L13] (hereinafter, referred to as a compound group SX747).

A compound wherein in the compound (L-1), n is 0, $R^1$ is an isopropyl group, $R^{3b}$ is a difluoromethyl group, $R^{3c}$ is a difluoromethyl group, and Q is any of the substituents shown in [Table L1] to [Table L13] (hereinafter, referred to as a compound group SX748).

A compound wherein in the compound (L-1), n is 1, $R^1$ is an isopropyl group, $R^{3b}$ is a difluoromethyl group, $R^{3c}$ is a difluoromethyl group, and Q is any of the substituents shown in [Table L1] to [Table L13] (hereinafter, referred to as a compound group SX749).

A compound wherein in the compound (L-1), n is 2, $R^1$ is an isopropyl group, $R^{3b}$ is a difluoromethyl group, $R^{3c}$ is a difluoromethyl group, and Q is any of the substituents shown in [Table L1] to [Table L13] (hereinafter, referred to as a compound group SX750).

A compound wherein in the compound (L-1), n is 0, $R^1$ is a methylcyano group, $R^{3b}$ is a difluoromethyl group, $R^{3c}$ is a difluoromethyl group, and Q is any of the substituents shown in [Table L1] to [Table L13] (hereinafter, referred to as a compound group SX751).

A compound wherein in the compound (L-1), n is 1, $R^1$ is a methylcyano group, $R^{3b}$ is a difluoromethyl group, $R^{3c}$ is a difluoromethyl group, and Q is any of the substituents shown in [Table L1] to [Table L13] (hereinafter, referred to as a compound group SX752).

A compound wherein in the compound (L-1), n is 2, $R^1$ is a methylcyano group, $R^{3b}$ is a difluoromethyl group, $R^{3c}$ is a difluoromethyl group, and Q is any of the substituents shown in [Table L1] to [Table L13] (hereinafter, referred to as a compound group SX753).

A compound wherein in the compound (L-1), n is 0, $R^1$ is a benzyl group, $R^{3b}$ is a difluoromethyl group, $R^{3c}$ is a difluoromethyl group, and Q is any of the substituents shown in [Table L1] to [Table L13] (hereinafter, referred to as a compound group SX754).

A compound wherein in the compound (L-1), n is 1, $R^1$ is a benzyl group, $R^{3b}$ is a difluoromethyl group, $R^{3c}$ is a difluoromethyl group, and Q is any of the substituents shown in [Table L1] to [Table L13] (hereinafter, referred to as a compound group SX755).

A compound wherein in the compound (L-1), n is 2, $R^1$ is a benzyl group, $R^{3b}$ is a difluoromethyl group, $R^{3c}$ is a difluoromethyl group, and Q is any of the substituents shown in [Table L1] to [Table L13] (hereinafter, referred to as a compound group SX756).

[Table L14]

| |
|---|
| F |
| Cl |
| Br |
| I |
| Me |
| $CF_3$ |
| $CF_2H$ |
| $CH=CH_2$ |
| $CMe=CH_2$ |
| c-Pr |

(continued)

| |
|---|
| 1-CN-c-Pr |
| CHO |
| C(O)Me |
| CMe=N-OH |
| CMe=N-OMe |
| CMe=N-OEt |
| $NO_2$ |
| CN |

[Table L15]

| |
|---|
| Ph |
| 4-F-Ph |
| 4-Cl-Ph |
| OPh |
| $NH_2$ |
| NHMe |
| $NMe_2$ |
| NHC(O)Me |
| Py2 |
| Py3 |
| Py4 |
| OPy2 |
| OPy3 |
| OPy4 |
| $CMe_2CN$ |
| OMe |
| OEt |
| $OCF_2H$ |
| $OCF_3$ |
| $OCH_2CF_3$ |

[Table L16]

| |
|---|
| pyrimidin-2-yl |
| pyrimidin-4-yl |
| pyrimidin-5-yl |
| pyrazin-2-yl |
| pyridazin-3-yl |
| pyridazin-4-yl |
| 3-chloro-1H-pyrazol-1-yl |
| 4-chloro-1H-pyrazol-1-yl |
| 3-(trifluoromethyl)-1H-pyrazol-1-yl |

(continued)

| |
|---|
| 4-(trifluoromethyl)-1H-pyrazol-1-yl |
| 1,2,4-triazol-1-yl |

A compound wherein in the compound (L-1), n is 0, $R^1$ is a methyl group, $R^{3b}$ is any of the substituents shown in [Table L14] to [Table L16], $R^{3c}$ is a hydrogen atom, and Q is 3-(trifluoromethyl)isoxazol-5-yl (hereinafter, referred to as a compound group SX757).

A compound wherein in the compound (L-1), n is 1, $R^1$ is a methyl group, $R^{3b}$ is any of the substituents shown in [Table L14] to [Table L16], $R^{3c}$ is a hydrogen atom, and Q is 3-(trifluoromethyl)isoxazol-5-yl (hereinafter, referred to as a compound group SX758).

A compound wherein in the compound (L-1), n is 2, $R^1$ is a methyl group, $R^{3b}$ is any of the substituents shown in [Table L14] to [Table L16], $R^{3c}$ is a hydrogen atom, and Q is 3-(trifluoromethyl)isoxazol-5-yl (hereinafter, referred to as a compound group SX759).

A compound wherein in the compound (L-1), n is 0, $R^1$ is an ethyl group, $R^{3b}$ is any of the substituents shown in [Table L14] to [Table L16], $R^{3c}$ is a hydrogen atom, and Q is 3-(trifluoromethyl)isoxazol-5-yl (hereinafter, referred to as a compound group SX760).

A compound wherein in the compound (L-1), n is 1, $R^1$ is an ethyl group, $R^{3b}$ is any of the substituents shown in [Table L14] to [Table L16], $R^{3c}$ is a hydrogen atom, and Q is 3-(trifluoromethyl)isoxazol-5-yl (hereinafter, referred to as a compound group SX761).

A compound wherein in the compound (L-1), n is 2, $R^1$ is an ethyl group, $R^{3b}$ is any of the substituents shown in [Table L14] to [Table L16], $R^{3c}$ is a hydrogen atom, and Q is 3-(trifluoromethyl)isoxazol-5-yl (hereinafter, referred to as a compound group SX762).

A compound wherein in the compound (L-1), n is 0, $R^1$ is a methyl group, $R^{3b}$ is any of the substituents shown in [Table L14] to [Table L16], $R^{3c}$ is a fluorine atom, and Q is 3-(trifluoromethyl)isoxazol-5-yl (hereinafter, referred to as a compound group SX763).

A compound wherein in the compound (L-1), n is 1, $R^1$ is a methyl group, $R^{3b}$ is any of the substituents shown in [Table L14] to [Table L16], $R^{3c}$ is a fluorine atom, and Q is 3-(trifluoromethyl)isoxazol-5-yl (hereinafter, referred to as a compound group SX764).

A compound wherein in the compound (L-1), n is 2, $R^1$ is a methyl group, $R^{3b}$ is any of the substituents shown in [Table L14] to [Table L16], $R^{3c}$ is a fluorine atom, and Q is 3-(trifluoromethyl)isoxazol-5-yl (hereinafter, referred to as a compound group SX765).

A compound wherein in the compound (L-1), n is 0, $R^1$ is an ethyl group, $R^{3b}$ is any of the substituents shown in [Table L14] to [Table L16], $R^{3c}$ is a fluorine atom, and Q is 3-(trifluoromethyl)isoxazol-5-yl (hereinafter, referred to as a compound group SX766).

A compound wherein in the compound (L-1), n is 1, $R^1$ is an ethyl group, $R^{3b}$ is any of the substituents shown in [Table L14] to [Table L16], $R^{3c}$ is a fluorine atom, and Q is 3-(trifluoromethyl)isoxazol-5-yl (hereinafter, referred to as a compound group SX767).

A compound wherein in the compound (L-1), n is 2, $R^1$ is an ethyl group, $R^{3b}$ is any of the substituents shown in [Table L14] to [Table L16], $R^{3c}$ is a fluorine atom, and Q is 3-(trifluoromethyl)isoxazol-5-yl (hereinafter, referred to as a compound group SX768).

A compound wherein in the compound (L-1), n is 0, $R^1$ is a methyl group, $R^{3b}$ is any of the substituents shown in [Table L14] to [Table L16], $R^{3c}$ is a bromine atom, and Q is 3-(trifluoromethyl)isoxazol-5-yl (hereinafter, referred to as a compound group SX769).

A compound wherein in the compound (L-1), n is 1, $R^1$ is a methyl group, $R^{3b}$ is any of the substituents shown in [Table L14] to [Table L16], $R^{3c}$ is a bromine atom, and Q is 3-(trifluoromethyl)isoxazol-5-yl (hereinafter, referred to as a compound group SX770).

A compound wherein in the compound (L-1), n is 2, $R^1$ is a methyl group, $R^{3b}$ is any of the substituents shown in [Table L14] to [Table L16], $R^{3c}$ is a bromine atom, and Q is 3-(trifluoromethyl)isoxazol-5-yl (hereinafter, referred to as a compound group SX771).

A compound wherein in the compound (L-1), n is 0, $R^1$ is an ethyl group, $R^{3b}$ is any of the substituents shown in [Table L14] to [Table L16], $R^{3c}$ is a bromine atom, and Q is 3-(trifluoromethyl)isoxazol-5-yl (hereinafter, referred to as a compound group SX772).

A compound wherein in the compound (L-1), n is 1, $R^1$ is an ethyl group, $R^{3b}$ is any of the substituents shown in [Table L14] to [Table L16], $R^{3c}$ is a bromine atom, and Q is 3-(trifluoromethyl)isoxazol-5-yl (hereinafter, referred to as a compound group SX773).

A compound wherein in the compound (L-1), n is 2, $R^1$ is an ethyl group, $R^{3b}$ is any of the substituents shown in [Table L14] to [Table L16], $R^{3c}$ is a bromine atom, and Q is 3-(trifluoromethyl)isoxazol-5-yl (hereinafter, referred to as a

compound group SX774).

A compound wherein in the compound (L-1), n is 0, $R^1$ is a methyl group, $R^{3b}$ is any of the substituents shown in [Table L14] to [Table L16], $R^{3c}$ is an iodine atom, and Q is 3-(trifluoromethyl)isoxazol-5-yl (hereinafter, referred to as a compound group SX775).

A compound wherein in the compound (L-1), n is 1, $R^1$ is a methyl group, $R^{3b}$ is any of the substituents shown in [Table L14] to [Table L16], $R^{3c}$ is an iodine atom, and Q is 3-(trifluoromethyl)isoxazol-5-yl (hereinafter, referred to as a compound group SX776).

A compound wherein in the compound (L-1), n is 2, $R^1$ is a methyl group, $R^{3b}$ is any of the substituents shown in [Table L14] to [Table L16], $R^{3c}$ is an iodine atom, and Q is 3-(trifluoromethyl)isoxazol-5-yl (hereinafter, referred to as a compound group SX777).

A compound wherein in the compound (L-1), n is 0, $R^1$ is an ethyl group, $R^{3b}$ is any of the substituents shown in [Table L14] to [Table L16], $R^{3c}$ is an iodine atom, and Q is 3-(trifluoromethyl)isoxazol-5-yl (hereinafter, referred to as a compound group SX778).

A compound wherein in the compound (L-1), n is 1, $R^1$ is an ethyl group, $R^{3b}$ is any of the substituents shown in [Table L14] to [Table L16], $R^{3c}$ is an iodine atom, and Q is 3-(trifluoromethyl)isoxazol-5-yl (hereinafter, referred to as a compound group SX779).

A compound wherein in the compound (L-1), n is 2, $R^1$ is an ethyl group, $R^{3b}$ is any of the substituents shown in [Table L14] to [Table L16], $R^{3c}$ is an iodine atom, and Q is 3-(trifluoromethyl)isoxazol-5-yl (hereinafter, referred to as a compound group SX780).

A compound wherein in the compound (L-1), n is 0, $R^1$ is a methyl group, $R^{3b}$ is any of the substituents shown in [Table L14] to [Table L16], $R^{3c}$ is a trifluoromethyl group, and Q is 3-(trifluoromethyl)isoxazol-5-yl (hereinafter, referred to as a compound group SX781).

A compound wherein in the compound (L-1), n is 1, $R^1$ is a methyl group, $R^{3b}$ is any of the substituents shown in [Table L14] to [Table L16], $R^{3c}$ is a trifluoromethyl group, and Q is 3-(trifluoromethyl)isoxazol-5-yl (hereinafter, referred to as a compound group SX782).

A compound wherein in the compound (L-1), n is 2, $R^1$ is a methyl group, $R^{3b}$ is any of the substituents shown in [Table L14] to [Table L16], $R^{3c}$ is a trifluoromethyl group, and Q is 3-(trifluoromethyl)isoxazol-5-yl (hereinafter, referred to as a compound group SX783).

**A** compound wherein in the compound (L-1), n is 0, $R^1$ is an ethyl group, $R^{3b}$ is any of the substituents shown in [Table L14] to [Table L16], $R^{3c}$ is a trifluoromethyl group, and Q is 3-(trifluoromethyl)isoxazol-5-yl (hereinafter, referred to as a compound group SX784).

A compound wherein in the compound (L-1), n is 1, $R^1$ is an ethyl group, $R^{3b}$ is any of the substituents shown in [Table L14] to [Table L16], $R^{3c}$ is a trifluoromethyl group, and Q is 3-(trifluoromethyl)isoxazol-5-yl (hereinafter, referred to as a compound group SX785).

A compound wherein in the compound (L-1), n is 2, $R^1$ is an ethyl group, $R^{3b}$ is any of the substituents shown in [Table L14] to [Table L16], $R^{3c}$ is a trifluoromethyl group, and Q is 3-(trifluoromethyl)isoxazol-5-yl (hereinafter, referred to as a compound group SX786).

A compound wherein in the compound (L-1), n is 0, $R^1$ is a methyl group, $R^{3b}$ is any of the substituents shown in [Table L14] to [Table L16], $R^{3c}$ is a difluoromethyl group, and Q is 3-(trifluoromethyl)isoxazol-5-yl (hereinafter, referred to as a compound group SX787).

A compound wherein in the compound (L-1), n is 1, $R^1$ is a methyl group, $R^{3b}$ is any of the substituents shown in [Table L14] to [Table L16], $R^{3c}$ is a difluoromethyl group, and Q is 3-(trifluoromethyl)isoxazol-5-yl (hereinafter, referred to as a compound group SX788).

A compound wherein in the compound (L-1), n is 2, $R^1$ is a methyl group, $R^{3b}$ is any of the substituents shown in [Table L14] to [Table L16], $R^{3c}$ is a difluoromethyl group, and Q is 3-(trifluoromethyl)isoxazol-5-yl (hereinafter, referred to as a compound group SX789).

A compound wherein in the compound (L-1), n is 0, $R^1$ is an ethyl group, $R^{3b}$ is any of the substituents shown in [Table L14] to [Table L16], $R^{3c}$ is a difluoromethyl group, and Q is 3-(trifluoromethyl)isoxazol-5-yl (hereinafter, referred to as a compound group SX790).

A compound wherein in the compound (L-1), n is 1, $R^1$ is an ethyl group, $R^{3b}$ is any of the substituents shown in [Table L14] to [Table L16], $R^{3c}$ is a difluoromethyl group, and Q is 3-(trifluoromethyl)isoxazol-5-yl (hereinafter, referred to as a compound group SX791).

A compound wherein in the compound (L-1), n is 2, $R^1$ is an ethyl group, $R^{3b}$ is any of the substituents shown in [Table L14] to [Table L16], $R^{3c}$ is a difluoromethyl group, and Q is 3-(trifluoromethyl)isoxazol-5-yl (hereinafter, referred to as a compound group SX792).

A compound wherein in the compound (L-1), n is 0, $R^1$ is a methyl group, $R^{3b}$ is any of the substituents shown in [Table L14] to [Table L16], $R^{3c}$ is a hydrogen atom, and Q is 5-(trifluoromethyl)isoxazol-3-yl (hereinafter, referred to as a compound group SX793).

A compound wherein in the compound (L-1), n is 1, $R^1$ is a methyl group, $R^{3b}$ is any of the substituents shown in [Table L14] to [Table L16], $R^{3c}$ is a hydrogen atom, and Q is 5-(trifluoromethyl)isoxazol-3-yl (hereinafter, referred to as a compound group SX794).

A compound wherein in the compound (L-1), n is 2, $R^1$ is a methyl group, $R^{3b}$ is any of the substituents shown in [Table L14] to [Table L16], $R^{3c}$ is a hydrogen atom, and Q is 5-(trifluoromethyl)isoxazol-3-yl (hereinafter, referred to as a compound group SX795).

A compound wherein in the compound (L-1), n is 0, $R^1$ is an ethyl group, $R^{3b}$ is any of the substituents shown in [Table L14] to [Table L16], $R^{3c}$ is a hydrogen atom, and Q is 5-(trifluoromethyl)isoxazol-3-yl (hereinafter, referred to as a compound group SX796).

A compound wherein in the compound (L-1), n is 1, $R^1$ is an ethyl group, $R^{3b}$ is any of the substituents shown in [Table L14] to [Table L16], $R^{3c}$ is a hydrogen atom, and Q is 5-(trifluoromethyl)isoxazol-3-yl (hereinafter, referred to as a compound group SX797).

A compound wherein in the compound (L-1), n is 2, $R^1$ is an ethyl group, $R^{3b}$ is any of the substituents shown in [Table L14] to [Table L16], $R^{3c}$ is a hydrogen atom, and Q is 5-(trifluoromethyl)isoxazol-3-yl (hereinafter, referred to as a compound group SX798).

A compound wherein in the compound (L-1), n is 0, $R^1$ is a methyl group, $R^{3b}$ is any of the substituents shown in [Table L14] to [Table L16], $R^{3c}$ is a fluorine atom, and Q is 5-(trifluoromethyl)isoxazol-3-yl (hereinafter, referred to as a compound group SX799).

A compound wherein in the compound (L-1), n is 1, $R^1$ is a methyl group, $R^{3b}$ is any of the substituents shown in [Table L14] to [Table L16], $R^{3c}$ is a fluorine atom, and Q is 5-(trifluoromethyl)isoxazol-3-yl (hereinafter, referred to as a compound group SX800).

A compound wherein in the compound (L-1), n is 2, $R^1$ is a methyl group, $R^{3b}$ is any of the substituents shown in [Table L14] to [Table L16], $R^{3c}$ is a fluorine atom, and Q is 5-(trifluoromethyl)isoxazol-3-yl (hereinafter, referred to as a compound group SX801).

A compound wherein in the compound (L-1), n is 0, $R^1$ is an ethyl group, $R^{3b}$ is any of the substituents shown in [Table L14] to [Table L16], $R^{3c}$ is a fluorine atom, and Q is 5-(trifluoromethyl)isoxazol-3-yl (hereinafter, referred to as a compound group SX802).

A compound wherein in the compound (L-1), n is 1, $R^1$ is an ethyl group, $R^{3b}$ is any of the substituents shown in [Table L14] to [Table L16], $R^{3c}$ is a fluorine atom, and Q is 5-(trifluoromethyl)isoxazol-3-yl (hereinafter, referred to as a compound group SX803).

A compound wherein in the compound (L-1), n is 2, $R^1$ is an ethyl group, $R^{3b}$ is any of the substituents shown in [Table L14] to [Table L16], $R^{3c}$ is a fluorine atom, and Q is 5-(trifluoromethyl)isoxazol-3-yl (hereinafter, referred to as a compound group SX804).

A compound wherein in the compound (L-1), n is 0, $R^1$ is a methyl group, $R^{3b}$ is any of the substituents shown in [Table L14] to [Table L16], $R^{3c}$ is a bromine atom, and Q is 5-(trifluoromethyl)isoxazol-3-yl (hereinafter, referred to as a compound group SX805).

A compound wherein in the compound (L-1), n is 1, $R^1$ is a methyl group, $R^{3b}$ is any of the substituents shown in [Table L14] to [Table L16], $R^{3c}$ is a bromine atom, and Q is 5-(trifluoromethyl)isoxazol-3-yl (hereinafter, referred to as a compound group SX806).

A compound wherein in the compound (L-1), n is 2, $R^1$ is a methyl group, $R^{3b}$ is any of the substituents shown in [Table L14] to [Table L16], $R^{3c}$ is a bromine atom, and Q is 5-(trifluoromethyl)isoxazol-3-yl (hereinafter, referred to as a compound group SX807).

A compound wherein in the compound (L-1), n is 0, $R^1$ is an ethyl group, $R^{3b}$ is any of the substituents shown in [Table L14] to [Table L16], $R^{3c}$ is a bromine atom, and Q is 5-(trifluoromethyl)isoxazol-3-yl (hereinafter, referred to as a compound group SX808).

A compound wherein in the compound (L-1), n is 1, $R^1$ is an ethyl group, $R^{3b}$ is any of the substituents shown in [Table L14] to [Table L16], $R^{3c}$ is a bromine atom, and Q is 5-(trifluoromethyl)isoxazol-3-yl (hereinafter, referred to as a compound group SX809).

A compound wherein in the compound (L-1), n is 2, $R^1$ is an ethyl group, $R^{3b}$ is any of the substituents shown in [Table L14] to [Table L16], $R^{3c}$ is a bromine atom, and Q is 5-(trifluoromethyl)isoxazol-3-yl (hereinafter, referred to as a compound group SX810).

A compound wherein in the compound (L-1), n is 0, $R^1$ is a methyl group, $R^{3b}$ is any of the substituents shown in [Table L14] to [Table L16], $R^{3c}$ is an iodine atom, and Q is 5-(trifluoromethyl)isoxazol-3-yl (hereinafter, referred to as a compound group SX811).

A compound wherein in the compound (L-1), n is 1, $R^1$ is a methyl group, $R^{3b}$ is any of the substituents shown in [Table L14] to [Table L16], $R^{3c}$ is an iodine atom, and Q is 5-(trifluoromethyl)isoxazol-3-yl (hereinafter, referred to as a compound group SX812).

A compound wherein in the compound (L-1), n is 2, $R^1$ is a methyl group, $R^{3b}$ is any of the substituents shown in

[Table L14] to [Table L16], $R^{3c}$ is an iodine atom, and Q is 5-(trifluoromethyl)isoxazol-3-yl (hereinafter, referred to as a compound group SX813).

A compound wherein in the compound (L-1), n is 0, $R^1$ is an ethyl group, $R^{3b}$ is any of the substituents shown in [Table L14] to [Table L16], $R^{3c}$ is an iodine atom, and Q is 5-(trifluoromethyl)isoxazol-3-yl (hereinafter, referred to as a compound group SX814).

A compound wherein in the compound (L-1), n is 1, $R^1$ is an ethyl group, $R^{3b}$ is any of the substituents shown in [Table L14] to [Table L16], $R^{3c}$ is an iodine atom, and Q is 5-(trifluoromethyl)isoxazol-3-yl (hereinafter, referred to as a compound group SX815).

A compound wherein in the compound (L-1), n is 2, $R^1$ is an ethyl group, $R^{3b}$ is any of the substituents shown in [Table L14] to [Table L16], $R^{3c}$ is an iodine atom, and Q is 5-(trifluoromethyl)isoxazol-3-yl (hereinafter, referred to as a compound group SX816).

A compound wherein in the compound (L-1), n is 0, $R^1$ is a methyl group, $R^{3b}$ is any of the substituents shown in [Table L14] to [Table L16], $R^{3c}$ is a trifluoromethyl group, and Q is 5-(trifluoromethyl)isoxazol-3-yl (hereinafter, referred to as a compound group SX817).

A compound wherein in the compound (L-1), n is 1, $R^1$ is a methyl group, $R^{3b}$ is any of the substituents shown in [Table L14] to [Table L16], $R^{3c}$ is a trifluoromethyl group, and Q is 5-(trifluoromethyl)isoxazol-3-yl (hereinafter, referred to as a compound group SX818).

**A** compound wherein in the compound (L-1), n is 2, $R^1$ is a methyl group, $R^{3b}$ is any of the substituents shown in [Table L14] to [Table L16], $R^{3c}$ is a trifluoromethyl group, and Q is 5-(trifluoromethyl)isoxazol-3-yl (hereinafter, referred to as a compound group SX819).

A compound wherein in the compound (L-1), n is 0, $R^1$ is an ethyl group, $R^{3b}$ is any of the substituents shown in [Table L14] to [Table L16], $R^{3c}$ is a trifluoromethyl group, and Q is 5-(trifluoromethyl)isoxazol-3-yl (hereinafter, referred to as a compound group SX820).

A compound wherein in the compound (L-1), n is 1, $R^1$ is an ethyl group, $R^{3b}$ is any of the substituents shown in [Table L14] to [Table L16], $R^{3c}$ is a trifluoromethyl group, and Q is 5-(trifluoromethyl)isoxazol-3-yl (hereinafter, referred to as a compound group SX821).

A compound wherein in the compound (L-1), n is 2, $R^1$ is an ethyl group, $R^{3b}$ is any of the substituents shown in [Table L14] to [Table L16], $R^{3c}$ is a trifluoromethyl group, and Q is 5-(trifluoromethyl)isoxazol-3-yl (hereinafter, referred to as a compound group SX822).

A compound wherein in the compound (L-1), n is 0, $R^1$ is a methyl group, $R^{3b}$ is any of the substituents shown in [Table L14] to [Table L16], $R^{3c}$ is a difluoromethyl group, and Q is 5-(trifluoromethyl)isoxazol-3-yl (hereinafter, referred to as a compound group SX823).

A compound wherein in the compound (L-1), n is 1, $R^1$ is a methyl group, $R^{3b}$ is any of the substituents shown in [Table L14] to [Table L16], $R^{3c}$ is a difluoromethyl group, and Q is 5-(trifluoromethyl)isoxazol-3-yl (hereinafter, referred to as a compound group SX824).

A compound wherein in the compound (L-1), n is 2, $R^1$ is a methyl group, $R^{3b}$ is any of the substituents shown in [Table L14] to [Table L16], $R^{3c}$ is a difluoromethyl group, and Q is 5-(trifluoromethyl)isoxazol-3-yl (hereinafter, referred to as a compound group SX825).

A compound wherein in the compound (L-1), n is 0, $R^1$ is an ethyl group, $R^{3b}$ is any of the substituents shown in [Table L14] to [Table L16], $R^{3c}$ is a difluoromethyl group, and Q is 5-(trifluoromethyl)isoxazol-3-yl (hereinafter, referred to as a compound group SX826).

A compound wherein in the compound (L-1), n is 1, $R^1$ is an ethyl group, $R^{3b}$ is any of the substituents shown in [Table L14] to [Table L16], $R^{3c}$ is a difluoromethyl group, and Q is 5-(trifluoromethyl)isoxazol-3-yl (hereinafter, referred to as a compound group SX827).

A compound wherein in the compound (L-1), n is 2, $R^1$ is an ethyl group, $R^{3b}$ is any of the substituents shown in [Table L14] to [Table L16], $R^{3c}$ is a difluoromethyl group, and Q is 5-(trifluoromethyl)isoxazol-3-yl (hereinafter, referred to as a compound group SX828).

A compound wherein in the compound (L-1), n is 0, $R^1$ is a methyl group, $R^{3b}$ is any of the substituents shown in [Table L14] to [Table L16], $R^{3c}$ is a hydrogen atom, and Q is 1-(difluoromethyl)-1H-pyrazol-4-yl (hereinafter, referred to as a compound group SX829).

A compound wherein in the compound (L-1), n is 1, $R^1$ is a methyl group, $R^{3b}$ is any of the substituents shown in [Table L14] to [Table L16], $R^{3c}$ is a hydrogen atom, and Q is 1-(difluoromethyl)-1H-pyrazol-4-yl (hereinafter, referred to as a compound group SX830).

A compound wherein in the compound (L-1), n is 2, $R^1$ is a methyl group, $R^{3b}$ is any of the substituents shown in [Table L14] to [Table L16], $R^{3c}$ is a hydrogen atom, and Q is 1-(difluoromethyl)-1H-pyrazol-4-yl (hereinafter, referred to as a compound group SX831).

A compound wherein in the compound (L-1), n is 0, $R^1$ is an ethyl group, $R^{3b}$ is any of the substituents shown in [Table L14] to [Table L16], $R^{3c}$ is a hydrogen atom, and Q is 1-(difluoromethyl)-1H-pyrazol-4-yl (hereinafter, referred to as a

compound group SX832).

A compound wherein in the compound (L-1), n is 1, $R^1$ is an ethyl group, $R^{3b}$ is any of the substituents shown in [Table L14] to [Table L16], $R^{3c}$ is a hydrogen atom, and Q is 1-(difluoromethyl)-1H-pyrazol-4-yl (hereinafter, referred to as a compound group SX833).

A compound wherein in the compound (L-1), n is 2, $R^1$ is an ethyl group, $R^{3b}$ is any of the substituents shown in [Table L14] to [Table L16], $R^{3c}$ is a hydrogen atom, and Q is 1-(difluoromethyl)-1H-pyrazol-4-yl (hereinafter, referred to as a compound group SX834).

A compound wherein in the compound (L-1), n is 0, $R^1$ is a methyl group, $R^{3b}$ is any of the substituents shown in [Table L14] to [Table L16], $R^{3c}$ is a fluorine atom, and Q is 1-(difluoromethyl)-1H-pyrazol-4-yl (hereinafter, referred to as a compound group SX835).

A compound wherein in the compound (L-1), n is 1, $R^1$ is a methyl group, $R^{3b}$ is any of the substituents shown in [Table L14] to [Table L16], $R^{3c}$ is a fluorine atom, and Q is 1-(difluoromethyl)-1H-pyrazol-4-yl (hereinafter, referred to as a compound group SX836).

A compound wherein in the compound (L-1), n is 2, $R^1$ is a methyl group, $R^{3b}$ is any of the substituents shown in [Table L14] to [Table L16], $R^{3c}$ is a fluorine atom, and Q is 1-(difluoromethyl)-1H-pyrazol-4-yl (hereinafter, referred to as a compound group SX837).

A compound wherein in the compound (L-1), n is 0, $R^1$ is an ethyl group, $R^{3b}$ is any of the substituents shown in [Table L14] to [Table L16], $R^{3c}$ is a fluorine atom, and Q is 1-(difluoromethyl)-1H-pyrazol-4-yl (hereinafter, referred to as a compound group SX838).

A compound wherein in the compound (L-1), n is 1, $R^1$ is an ethyl group, $R^{3b}$ is any of the substituents shown in [Table L14] to [Table L16], $R^{3c}$ is a fluorine atom, and Q is 1-(difluoromethyl)-1H-pyrazol-4-yl (hereinafter, referred to as a compound group SX839).

A compound wherein in the compound (L-1), n is 2, $R^1$ is an ethyl group, $R^{3b}$ is any of the substituents shown in [Table L14] to [Table L16], $R^{3c}$ is a fluorine atom, and Q is 1-(difluoromethyl)-1H-pyrazol-4-yl (hereinafter, referred to as a compound group SX840).

A compound wherein in the compound (L-1), n is 0, $R^1$ is a methyl group, $R^{3b}$ is any of the substituents shown in [Table L14] to [Table L16], $R^{3c}$ is a bromine atom, and Q is 1-(difluoromethyl)-1H-pyrazol-4-yl (hereinafter, referred to as a compound group SX841).

A compound wherein in the compound (L-1), n is 1, $R^1$ is a methyl group, $R^{3b}$ is any of the substituents shown in [Table L14] to [Table L16], $R^{3c}$ is a bromine atom, and Q is 1-(difluoromethyl)-1H-pyrazol-4-yl (hereinafter, referred to as a compound group SX842).

A compound wherein in the compound (L-1), n is 2, $R^1$ is a methyl group, $R^{3b}$ is any of the substituents shown in [Table L14] to [Table L16], $R^{3c}$ is a bromine atom, and Q is 1-(difluoromethyl)-1H-pyrazol-4-yl (hereinafter, referred to as a compound group SX843).

A compound wherein in the compound (L-1), n is 0, $R^1$ is an ethyl group, $R^{3b}$ is any of the substituents shown in [Table L14] to [Table L16], $R^{3c}$ is a bromine atom, and Q is 1-(difluoromethyl)-1H-pyrazol-4-yl (hereinafter, referred to as a compound group SX844).

A compound wherein in the compound (L-1), n is 1, $R^1$ is an ethyl group, $R^{3b}$ is any of the substituents shown in [Table L14] to [Table L16], $R^{3c}$ is a bromine atom, and Q is 1-(difluoromethyl)-1H-pyrazol-4-yl (hereinafter, referred to as a compound group SX845).

A compound wherein in the compound (L-1), n is 2, $R^1$ is an ethyl group, $R^{3b}$ is any of the substituents shown in [Table L14] to [Table L16], $R^{3c}$ is a bromine atom, and Q is 1-(difluoromethyl)-1H-pyrazol-4-yl (hereinafter, referred to as a compound group SX846).

A compound wherein in the compound (L-1), n is 0, $R^1$ is a methyl group, $R^{3b}$ is any of the substituents shown in [Table L14] to [Table L16], $R^{3c}$ is an iodine atom, and Q is 1-(difluoromethyl)-1H-pyrazol-4-yl (hereinafter, referred to as a compound group SX847).

A compound wherein in the compound (L-1), n is 1, $R^1$ is a methyl group, $R^{3b}$ is any of the substituents shown in [Table L14] to [Table L16], $R^{3c}$ is an iodine atom, and Q is 1-(difluoromethyl)-1H-pyrazol-4-yl (hereinafter, referred to as a compound group SX848).

A compound wherein in the compound (L-1), n is 2, $R^1$ is a methyl group, $R^{3b}$ is any of the substituents shown in [Table L14] to [Table L16], $R^{3c}$ is an iodine atom, and Q is 1-(difluoromethyl)-1H-pyrazol-4-yl (hereinafter, referred to as a compound group SX849).

A compound wherein in the compound (L-1), n is 0, $R^1$ is an ethyl group, $R^{3b}$ is any of the substituents shown in [Table L14] to [Table L16], $R^{3c}$ is an iodine atom, and Q is 1-(difluoromethyl)-1H-pyrazol-4-yl (hereinafter, referred to as a compound group SX850).

A compound wherein in the compound (L-1), n is 1, $R^1$ is an ethyl group, $R^{3b}$ is any of the substituents shown in [Table L14] to [Table L16], $R^{3c}$ is an iodine atom, and Q is 1-(difluoromethyl)-1H-pyrazol-4-yl (hereinafter, referred to as a compound group SX851).

A compound wherein in the compound (L-1), n is 2, $R^1$ is an ethyl group, $R^{3b}$ is any of the substituents shown in [Table L14] to [Table L16], $R^{3c}$ is an iodine atom, and Q is 1-(difluoromethyl)-1H-pyrazol-4-yl (hereinafter, referred to as a compound group SX852).

A compound wherein in the compound (L-1), n is 0, $R^1$ is a methyl group, $R^{3b}$ is any of the substituents shown in [Table L14] to [Table L16], $R^{3c}$ is a trifluoromethyl group, and Q is 1-(difluoromethyl)-1H-pyrazol-4-yl (hereinafter, referred to as a compound group SX853).

A compound wherein in the compound (L-1), n is 1, $R^1$ is a methyl group, $R^{3b}$ is any of the substituents shown in [Table L14] to [Table L16], $R^{3c}$ is a trifluoromethyl group, and Q is 1-(difluoromethyl)-1H-pyrazol-4-yl (hereinafter, referred to as a compound group SX854).

A compound wherein in the compound (L-1), n is 2, $R^1$ is a methyl group, $R^{3b}$ is any of the substituents shown in [Table L14] to [Table L16], $R^{3c}$ is a trifluoromethyl group, and Q is 1-(difluoromethyl)-1H-pyrazol-4-yl (hereinafter, referred to as a compound group SX855).

A compound wherein in the compound (L-1), n is 0, $R^1$ is an ethyl group, $R^{3b}$ is any of the substituents shown in [Table L14] to [Table L16], $R^{3c}$ is a trifluoromethyl group, and Q is 1-(difluoromethyl)-1H-pyrazol-4-yl (hereinafter, referred to as a compound group SX856).

A compound wherein in the compound (L-1), n is 1, $R^1$ is an ethyl group, $R^{3b}$ is any of the substituents shown in [Table L14] to [Table L16], $R^{3c}$ is a trifluoromethyl group, and Q is 1-(difluoromethyl)-1H-pyrazol-4-yl (hereinafter, referred to as a compound group SX857).

A compound wherein in the compound (L-1), n is 2, $R^1$ is an ethyl group, $R^{3b}$ is any of the substituents shown in [Table L14] to [Table L16], $R^{3c}$ is a trifluoromethyl group, and Q is 1-(difluoromethyl)-1H-pyrazol-4-yl (hereinafter, referred to as a compound group SX858).

A compound wherein in the compound (L-1), n is 0, $R^1$ is a methyl group, $R^{3b}$ is any of the substituents shown in [Table L14] to [Table L16], $R^{3c}$ is a difluoromethyl group, and Q is 1-(difluoromethyl)-1H-pyrazol-4-yl (hereinafter, referred to as a compound group SX859).

A compound wherein in the compound (L-1), n is 1, $R^1$ is a methyl group, $R^{3b}$ is any of the substituents shown in [Table L14] to [Table L16], $R^{3c}$ is a difluoromethyl group, and Q is 1-(difluoromethyl)-1H-pyrazol-4-yl (hereinafter, referred to as a compound group SX860).

A compound wherein in the compound (L-1), n is 2, $R^1$ is a methyl group, $R^{3b}$ is any of the substituents shown in [Table L14] to [Table L16], $R^{3c}$ is a difluoromethyl group, and Q is 1-(difluoromethyl)-1H-pyrazol-4-yl (hereinafter, referred to as a compound group SX861).

A compound wherein in the compound (L-1), n is 0, $R^1$ is an ethyl group, $R^{3b}$ is any of the substituents shown in [Table L14] to [Table L16], $R^{3c}$ is a difluoromethyl group, and Q is 1-(difluoromethyl)-1H-pyrazol-4-yl (hereinafter, referred to as a compound group SX862).

A compound wherein in the compound (L-1), n is 1, $R^1$ is an ethyl group, $R^{3b}$ is any of the substituents shown in [Table L14] to [Table L16], $R^{3c}$ is a difluoromethyl group, and Q is 1-(difluoromethyl)-1H-pyrazol-4-yl (hereinafter, referred to as a compound group SX863).

A compound wherein in the compound (L-1), n is 2, $R^1$ is an ethyl group, $R^{3b}$ is any of the substituents shown in [Table L14] to [Table L16], $R^{3c}$ is a difluoromethyl group, and Q is 1-(difluoromethyl)-1H-pyrazol-4-yl (hereinafter, referred to as a compound group SX864).

A compound wherein in the compound (L-1), n is 0, $R^1$ is a methyl group, $R^{3b}$ is any of the substituents shown in [Table L14] to [Table L16], $R^{3c}$ is a hydrogen atom, and Q is 3-(difluoromethyl)-1-methyl-1H-pyrazol-4-yl (hereinafter, referred to as a compound group SX865).

A compound wherein in the compound (L-1), n is 1, $R^1$ is a methyl group, $R^{3b}$ is any of the substituents shown in [Table L14] to [Table L16], $R^{3c}$ is a hydrogen atom, and Q is 3-(difluoromethyl)-1-methyl-1H-pyrazol-4-yl (hereinafter, referred to as a compound group SX866).

A compound wherein in the compound (L-1), n is 2, $R^1$ is a methyl group, $R^{3b}$ is any of the substituents shown in [Table L14] to [Table L16], $R^{3c}$ is a hydrogen atom, and Q is 3-(difluoromethyl)-1-methyl-1H-pyrazol-4-yl (hereinafter, referred to as a compound group SX867).

A compound wherein in the compound (L-1), n is 0, $R^1$ is an ethyl group, $R^{3b}$ is any of the substituents shown in [Table L14] to [Table L16], $R^{3c}$ is a hydrogen atom, and Q is 3-(difluoromethyl)-1-methyl-1H-pyrazol-4-yl (hereinafter, referred to as a compound group SX868).

A compound wherein in the compound (L-1), n is 1, $R^1$ is an ethyl group, $R^{3b}$ is any of the substituents shown in [Table L14] to [Table L16], $R^{3c}$ is a hydrogen atom, and Q is 3-(difluoromethyl)-1-methyl-1H-pyrazol-4-yl (hereinafter, referred to as a compound group SX869).

A compound wherein in the compound (L-1), n is 2, $R^1$ is an ethyl group, $R^{3b}$ is any of the substituents shown in [Table L14] to [Table L16], $R^{3c}$ is a hydrogen atom, and Q is 3-(difluoromethyl)-1-methyl-1H-pyrazol-4-yl (hereinafter, referred to as a compound group SX870).

A compound wherein in the compound (L-1), n is 0, $R^1$ is a methyl group, $R^{3b}$ is any of the substituents shown in

[Table L14] to [Table L16], $R^{3c}$ is a fluorine atom, and Q is 3-(difluoromethyl)-1-methyl-1H-pyrazol-4-yl (hereinafter, referred to as a compound group SX871).

A compound wherein in the compound (L-1), n is 1, $R^1$ is a methyl group, $R^{3b}$ is any of the substituents shown in [Table L14] to [Table L16], $R^{3c}$ is a fluorine atom, and Q is 3-(difluoromethyl)-1-methyl-1H-pyrazol-4-yl (hereinafter, referred to as a compound group SX872).

A compound wherein in the compound (L-1), n is 2, $R^1$ is a methyl group, $R^{3b}$ is any of the substituents shown in [Table L14] to [Table L16], $R^{3c}$ is a fluorine atom, and Q is 3-(difluoromethyl)-1-methyl-1H-pyrazol-4-yl (hereinafter, referred to as a compound group SX873).

A compound wherein in the compound (L-1), n is 0, $R^1$ is an ethyl group, $R^{3b}$ is any of the substituents shown in [Table L14] to [Table L16], $R^{3c}$ is a fluorine atom, and Q is 3-(difluoromethyl)-1-methyl-1H-pyrazol-4-yl (hereinafter, referred to as a compound group SX874).

A compound wherein in the compound (L-1), n is 1, $R^1$ is an ethyl group, $R^{3b}$ is any of the substituents shown in [Table L14] to [Table L16], $R^{3c}$ is a fluorine atom, and Q is 3-(difluoromethyl)-1-methyl-1H-pyrazol-4-yl (hereinafter, referred to as a compound group SX875).

A compound wherein in the compound (L-1), n is 2, $R^1$ is an ethyl group, $R^{3b}$ is any of the substituents shown in [Table L14] to [Table L16], $R^{3c}$ is a fluorine atom, and Q is 3-(difluoromethyl)-1-methyl-1H-pyrazol-4-yl (hereinafter, referred to as a compound group SX876).

A compound wherein in the compound (L-1), n is 0, $R^1$ is a methyl group, $R^{3b}$ is any of the substituents shown in [Table L14] to [Table L16], $R^{3c}$ is a bromine atom, and Q is 3-(difluoromethyl)-1-methyl-1H-pyrazol-4-yl (hereinafter, referred to as a compound group SX877).

A compound wherein in the compound (L-1), n is 1, $R^1$ is a methyl group, $R^{3b}$ is any of the substituents shown in [Table L14] to [Table L16], $R^{3c}$ is a bromine atom, and Q is 3-(difluoromethyl)-1-methyl-1H-pyrazol-4-yl (hereinafter, referred to as a compound group SX878).

A compound wherein in the compound (L-1), n is 2, $R^1$ is a methyl group, $R^{3b}$ is any of the substituents shown in [Table L14] to [Table L16], $R^{3c}$ is a bromine atom, and Q is 3-(difluoromethyl)-1-methyl-1H-pyrazol-4-yl (hereinafter, referred to as a compound group SX879).

A compound wherein in the compound (L-1), n is 0, $R^1$ is an ethyl group, $R^{3b}$ is any of the substituents shown in [Table L14] to [Table L16], $R^{3c}$ is a bromine atom, and Q is 3-(difluoromethyl)-1-methyl-1H-pyrazol-4-yl (hereinafter, referred to as a compound group SX880).

A compound wherein in the compound (L-1), n is 1, $R^1$ is an ethyl group, $R^{3b}$ is any of the substituents shown in [Table L14] to [Table L16], $R^{3c}$ is a bromine atom, and Q is 3-(difluoromethyl)-1-methyl-1H-pyrazol-4-yl (hereinafter, referred to as a compound group SX881).

A compound wherein in the compound (L-1), n is 2, $R^1$ is an ethyl group, $R^{3b}$ is any of the substituents shown in [Table L14] to [Table L16], $R^{3c}$ is a bromine atom, and Q is 3-(difluoromethyl)-1-methyl-1H-pyrazol-4-yl (hereinafter, referred to as a compound group SX882).

A compound wherein in the compound (L-1), n is 0, $R^1$ is a methyl group, $R^{3b}$ is any of the substituents shown in [Table L14] to [Table L16], $R^{3c}$ is an iodine atom, and Q is 3-(difluoromethyl)-1-methyl-1H-pyrazol-4-yl (hereinafter, referred to as a compound group SX883).

A compound wherein in the compound (L-1), n is 1, $R^1$ is a methyl group, $R^{3b}$ is any of the substituents shown in [Table L14] to [Table L16], $R^{3c}$ is an iodine atom, and Q is 3-(difluoromethyl)-1-methyl-1H-pyrazol-4-yl (hereinafter, referred to as a compound group SX884).

A compound wherein in the compound (L-1), n is 2, $R^1$ is a methyl group, $R^{3b}$ is any of the substituents shown in [Table L14] to [Table L16], $R^{3c}$ is an iodine atom, and Q is 3-(difluoromethyl)-1-methyl-1H-pyrazol-4-yl (hereinafter, referred to as a compound group SX885).

A compound wherein in the compound (L-1), n is 0, $R^1$ is an ethyl group, $R^{3b}$ is any of the substituents shown in [Table L14] to [Table L16], $R^{3c}$ is an iodine atom, and Q is 3-(difluoromethyl)-1-methyl-1H-pyrazol-4-yl (hereinafter, referred to as a compound group SX886).

A compound wherein in the compound (L-1), n is 1, $R^1$ is an ethyl group, $R^{3b}$ is any of the substituents shown in [Table L14] to [Table L16], $R^{3c}$ is an iodine atom, and Q is 3-(difluoromethyl)-1-methyl-1H-pyrazol-4-yl (hereinafter, referred to as a compound group SX887).

A compound wherein in the compound (L-1), n is 2, $R^1$ is an ethyl group, $R^{3b}$ is any of the substituents shown in [Table L14] to [Table L16], $R^{3c}$ is an iodine atom, and Q is 3-(difluoromethyl)-1-methyl-1H-pyrazol-4-yl (hereinafter, referred to as a compound group SX888).

A compound wherein in the compound (L-1), n is 0, $R^1$ is a methyl group, $R^{3b}$ is any of the substituents shown in [Table L14] to [Table L16], $R^{3c}$ is a trifluoromethyl group, and Q is 3-(difluoromethyl)-1-methyl-1H-pyrazol-4-yl (hereinafter, referred to as a compound group SX889).

A compound wherein in the compound (L-1), n is 1, $R^1$ is a methyl group, $R^{3b}$ is any of the substituents shown in [Table L14] to [Table L16], $R^{3c}$ is a trifluoromethyl group, and Q is 3-(difluoromethyl)-1-methyl-1H-pyrazol-4-yl (hereinafter,

referred to as a compound group SX890).

A compound wherein in the compound (L-1), n is 2, $R^1$ is a methyl group, $R^{3b}$ is any of the substituents shown in [Table L14] to [Table L16], $R^{3c}$ is a trifluoromethyl group, and Q is 3-(difluoromethyl)-1-methyl-1H-pyrazol-4-yl (hereinafter, referred to as a compound group SX891).

A compound wherein in the compound (L-1), n is 0, $R^1$ is an ethyl group, $R^{3b}$ is any of the substituents shown in [Table L14] to [Table L16], $R^{3c}$ is a trifluoromethyl group, and Q is 3-(difluoromethyl)-1-methyl-1H-pyrazol-4-yl (hereinafter, referred to as a compound group SX892).

A compound wherein in the compound (L-1), n is 1, $R^1$ is an ethyl group, $R^{3b}$ is any of the substituents shown in [Table L14] to [Table L16], $R^{3c}$ is a trifluoromethyl group, and Q is 3-(difluoromethyl)-1-methyl-1H-pyrazol-4-yl (hereinafter, referred to as a compound group SX893).

A compound wherein in the compound (L-1), n is 2, $R^1$ is an ethyl group, $R^{3b}$ is any of the substituents shown in [Table L14] to [Table L16], $R^{3c}$ is a trifluoromethyl group, and Q is 3-(difluoromethyl)-1-methyl-1H-pyrazol-4-yl (hereinafter, referred to as a compound group SX894).

A compound wherein in the compound (L-1), n is 0, $R^1$ is a methyl group, $R^{3b}$ is any of the substituents shown in [Table L14] to [Table L16], $R^{3c}$ is a difluoromethyl group, and Q is 3-(difluoromethyl)-1-methyl-1H-pyrazol-4-yl (hereinafter, referred to as a compound group SX895).

A compound wherein in the compound (L-1), n is 1, $R^1$ is a methyl group, $R^{3b}$ is any of the substituents shown in [Table L14] to [Table L16], $R^{3c}$ is a difluoromethyl group, and Q is 3-(difluoromethyl)-1-methyl-1H-pyrazol-4-yl (hereinafter, referred to as a compound group SX896).

A compound wherein in the compound (L-1), n is 2, $R^1$ is a methyl group, $R^{3b}$ is any of the substituents shown in [Table L14] to [Table L16], $R^{3c}$ is a difluoromethyl group, and Q is 3-(difluoromethyl)-1-methyl-1H-pyrazol-4-yl (hereinafter, referred to as a compound group SX897).

A compound wherein in the compound (L-1), n is 0, $R^1$ is an ethyl group, $R^{3b}$ is any of the substituents shown in [Table L14] to [Table L16], $R^{3c}$ is a difluoromethyl group, and Q is 3-(difluoromethyl)-1-methyl-1H-pyrazol-4-yl (hereinafter, referred to as a compound group SX898).

A compound wherein in the compound (L-1), n is 1, $R^1$ is an ethyl group, $R^{3b}$ is any of the substituents shown in [Table L14] to [Table L16], $R^{3c}$ is a difluoromethyl group, and Q is 3-(difluoromethyl)-1-methyl-1H-pyrazol-4-yl (hereinafter, referred to as a compound group SX899).

A compound wherein in the compound (L-1), n is 2, $R^1$ is an ethyl group, $R^{3b}$ is any of the substituents shown in [Table L14] to [Table L16], $R^{3c}$ is a difluoromethyl group, and Q is 3-(difluoromethyl)-1-methyl-1H-pyrazol-4-yl (hereinafter, referred to as a compound group SX900).

A compound represented by a formula (L-2) (hereinafter, referred to as a compound (L-2))

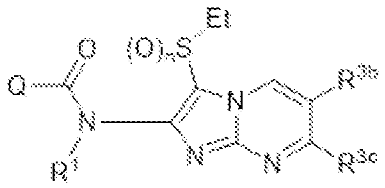

(L-2)

wherein n is 0, $R^1$ is a methyl group, $R^{3b}$ is a hydrogen atom, $R^{3c}$ is a hydrogen atom, and Q is any of the substituents shown in [Table L1] to [Table L13] (hereinafter, referred to as a compound group SX904).

A compound wherein in the compound (L-2), n is 1, $R^1$ is a methyl group, $R^{3b}$ is a hydrogen atom, $R^{3c}$ is a hydrogen atom, and Q is any of the substituents shown in [Table L1] to [Table L13] (hereinafter, referred to as a compound group SX905).

A compound wherein in the compound (L-2), n is 2, $R^1$ is a methyl group, $R^{3b}$ is a hydrogen atom, $R^{3c}$ is a hydrogen atom, and Q is any of the substituents shown in [Table L1] to [Table L13] (hereinafter, referred to as a compound group SX906).

A compound wherein in the compound (L-2), n is 0, $R^1$ is an ethyl group, $R^{3b}$ is a hydrogen atom, $R^{3c}$ is a hydrogen atom, and Q is any of the substituents shown in [Table L1] to [Table L13] (hereinafter, referred to as a compound group SX907).

A compound wherein in the compound (L-2), n is 1, $R^1$ is an ethyl group, $R^{3b}$ is a hydrogen atom, $R^{3c}$ is a hydrogen atom, and Q is any of the substituents shown in [Table L1] to [Table L13] (hereinafter, referred to as a compound group SX908).

A compound wherein in the compound (L-2), n is 2, $R^1$ is an ethyl group, $R^{3b}$ is a hydrogen atom, $R^{3c}$ is a hydrogen atom, and Q is any of the substituents shown in [Table L1] to [Table L13] (hereinafter, referred to as a compound group SX909).

A compound wherein in the compound (L-2), n is 0, $R^1$ is a benzyl group, $R^{3b}$ is a hydrogen atom, $R^{3c}$ is a

hydrogen atom, and Q is any of the substituents shown in [Table L1] to [Table L13] (hereinafter, referred to as a compound group SX910).

A compound wherein in the compound (L-2), n is 1, $R^1$ is a benzyl group, $R^{3b}$ is a hydrogen atom, $R^{3c}$ is a hydrogen atom, and Q is any of the substituents shown in [Table L1] to [Table L13] (hereinafter, referred to as a compound group SX911).

A compound wherein in the compound (L-2), n is 2, $R^1$ is a benzyl group, $R^{3b}$ is a hydrogen atom, $R^{3c}$ is a hydrogen atom, and Q is any of the substituents shown in [Table L1] to [Table L13] (hereinafter, referred to as a compound group SX912).

A compound wherein in the compound (L-2), n is 0, $R^1$ is a hydrogen atom, $R^{3b}$ is a fluorine atom, $R^{3c}$ is a hydrogen atom, and Q is any of the substituents shown in [Table L1] to [Table L13] (hereinafter, referred to as a compound group SX913).

A compound wherein in the compound (L-2), n is 1, $R^1$ is a hydrogen atom, $R^{3b}$ is a fluorine atom, $R^{3c}$ is a hydrogen atom, and Q is any of the substituents shown in [Table L1] to [Table L13] (hereinafter, referred to as a compound group SX914).

A compound wherein in the compound (L-2), n is 2, $R^1$ is a hydrogen atom, $R^{3b}$ is a fluorine atom, $R^{3c}$ is a hydrogen atom, and Q is any of the substituents shown in [Table L1] to [Table L13] (hereinafter, referred to as a compound group SX915).

A compound wherein in the compound (L-2), n is 0, $R^1$ is a methyl group, $R^{3b}$ is a fluorine atom, $R^{3c}$ is a hydrogen atom, and Q is any of the substituents shown in [Table L1] to [Table L13] (hereinafter, referred to as a compound group SX916).

A compound wherein in the compound (L-2), n is 1, $R^1$ is a methyl group, $R^{3b}$ is a fluorine atom, $R^{3c}$ is a hydrogen atom, and Q is any of the substituents shown in [Table L1] to [Table L13] (hereinafter, referred to as a compound group SX917).

A compound wherein in the compound (L-2), n is 2, $R^1$ is a methyl group, $R^{3b}$ is a fluorine atom, $R^{3c}$ is a hydrogen atom, and Q is any of the substituents shown in [Table L1] to [Table L13] (hereinafter, referred to as a compound group SX918).

A compound wherein in the compound (L-2), n is 0, $R^1$ is an ethyl group, $R^{3b}$ is a fluorine atom, $R^{3c}$ is a hydrogen atom, and Q is any of the substituents shown in [Table L1] to [Table L13] (hereinafter, referred to as a compound group SX919).

A compound wherein in the compound (L-2), n is 1, $R^1$ is an ethyl group, $R^{3b}$ is a fluorine atom, $R^{3c}$ is a hydrogen atom, and Q is any of the substituents shown in [Table L1] to [Table L13] (hereinafter, referred to as a compound group SX920).

A compound wherein in the compound (L-2), n is 2, $R^1$ is an ethyl group, $R^{3b}$ is a fluorine atom, $R^{3c}$ is a hydrogen atom, and Q is any of the substituents shown in [Table L1] to [Table L13] (hereinafter, referred to as a compound group SX921).

A compound wherein in the compound (L-2), n is 0, $R^1$ is a benzyl group, $R^{3b}$ is a fluorine atom, $R^{3c}$ is a hydrogen atom, and Q is any of the substituents shown in [Table L1] to [Table L13] (hereinafter, referred to as a compound group SX922).

A compound wherein in the compound (L-2), n is 1, $R^1$ is a benzyl group, $R^{3b}$ is a fluorine atom, $R^{3c}$ is a hydrogen atom, and Q is any of the substituents shown in [Table L1] to [Table L13] (hereinafter, referred to as a compound group SX923).

A compound wherein in the compound (L-2), n is 2, $R^1$ is a benzyl group, $R^{3b}$ is a fluorine atom, $R^{3c}$ is a hydrogen atom, and Q is any of the substituents shown in [Table L1] to [Table L13] (hereinafter, referred to as a compound group SX924).

A compound wherein in the compound (L-2), n is 0, $R^1$ is a methyl group, $R^{3b}$ is a chlorine atom, $R^{3c}$ is a hydrogen atom, and Q is any of the substituents shown in [Table L1] to [Table L13] (hereinafter, referred to as a compound group SX928).

A compound wherein in the compound (L-2), n is 1, $R^1$ is a methyl group, $R^{3b}$ is a chlorine atom, $R^{3c}$ is a hydrogen atom, and Q is any of the substituents shown in [Table L1] to [Table L13] (hereinafter, referred to as a compound group SX929).

A compound wherein in the compound (L-2), n is 2, $R^1$ is a methyl group, $R^{3b}$ is a chlorine atom, $R^{3c}$ is a hydrogen atom, and Q is any of the substituents shown in [Table L1] to [Table L13] (hereinafter, referred to as a compound group SX930).

A compound wherein in the compound (L-2), n is 0, $R^1$ is an ethyl group, $R^{3b}$ is a chlorine atom, $R^{3c}$ is a hydrogen atom, and Q is any of the substituents shown in [Table L1] to [Table L13] (hereinafter, referred to as a compound group SX931).

A compound wherein in the compound (L-2), n is 1, $R^1$ is an ethyl group, $R^{3b}$ is a chlorine atom, $R^{3c}$ is a hydrogen atom, and Q is any of the substituents shown in [Table L1] to [Table L13] (hereinafter, referred to as a compound group

SX932).

A compound wherein in the compound (L-2), n is 2, $R^1$ is an ethyl group, $R^{3b}$ is a chlorine atom, $R^{3c}$ is a hydrogen atom, and Q is any of the substituents shown in [Table L1] to [Table L13] (hereinafter, referred to as a compound group SX933).

A compound wherein in the compound (L-2), n is 0, $R^1$ is a benzyl group, $R^{3b}$ is a chlorine atom, $R^{3c}$ is a hydrogen atom, and Q is any of the substituents shown in [Table L1] to [Table L13] (hereinafter, referred to as a compound group SX934).

A compound wherein in the compound (L-2), n is 1, $R^1$ is a benzyl group, $R^{3b}$ is a chlorine atom, $R^{3c}$ is a hydrogen atom, and Q is any of the substituents shown in [Table L1] to [Table L13] (hereinafter, referred to as a compound group SX935).

A compound wherein in the compound (L-2), n is 2, $R^1$ is a benzyl group, $R^{3b}$ is a chlorine atom, $R^{3c}$ is a hydrogen atom, and Q is any of the substituents shown in [Table L1] to [Table L13] (hereinafter, referred to as a compound group SX936).

A compound wherein in the compound (L-2), n is 0, $R^1$ is a methyl group, $R^{3b}$ is a bromine atom, $R^{3c}$ is a hydrogen atom, and Q is any of the substituents shown in [Table L1] to [Table L13] (hereinafter, referred to as a compound group SX940).

A compound wherein in the compound (L-2), n is 1, $R^1$ is a methyl group, $R^{3b}$ is a bromine atom, $R^{3c}$ is a hydrogen atom, and Q is any of the substituents shown in [Table L1] to [Table L13] (hereinafter, referred to as a compound group SX941).

**A** compound wherein in the compound (L-2), n is **2,** $R^1$ is a methyl group, $R^{3b}$ is a bromine atom, $R^{3c}$ is a hydrogen atom, and **Q** is any of the substituents shown in [Table L1] to [Table L13] (hereinafter, referred to as a compound group SX942).

A compound wherein in the compound (L-2), n is 0, $R^1$ is an ethyl group, $R^{3b}$ is a bromine atom, $R^{3c}$ is a hydrogen atom, and **Q** is any of the substituents shown in [Table L1] to [Table L13] (hereinafter, referred to as a compound group SX943).

A compound wherein in the compound (L-2), n is 1, $R^1$ is an ethyl group, $R^{3b}$ is a bromine atom, $R^{3c}$ is a hydrogen atom, and Q is any of the substituents shown in [Table L1] to [Table L13] (hereinafter, referred to as a compound group SX944).

A compound wherein in the compound (L-2), n is 2, $R^1$ is an ethyl group, $R^{3b}$ is a bromine atom, $R^{3c}$ is a hydrogen atom, and Q is any of the substituents shown in [Table L1] to [Table L13] (hereinafter, referred to as a compound group SX945).

A compound wherein in the compound (L-2), n is 0, $R^1$ is a benzyl group, $R^{3b}$ is a bromine atom, $R^{3c}$ is a hydrogen atom, and Q is any of the substituents shown in [Table L1] to [Table L13] (hereinafter, referred to as a compound group SX946).

A compound wherein in the compound (L-2), n is 1, $R^1$ is a benzyl group, $R^{3b}$ is a bromine atom, $R^{3c}$ is a hydrogen atom, and Q is any of the substituents shown in [Table L1] to [Table L13] (hereinafter, referred to as a compound group SX947).

A compound wherein in the compound (L-2), n is 2, $R^1$ is a benzyl group, $R^{3b}$ is a bromine atom, $R^{3c}$ is a hydrogen atom, and Q is any of the substituents shown in [Table L1] to [Table L13] (hereinafter, referred to as a compound group SX948).

A compound wherein in the compound (L-2), n is 0, $R^1$ is a methyl group, $R^{3b}$ is an iodine atom, $R^{3c}$ is a hydrogen atom, and Q is any of the substituents shown in [Table L1] to [Table L13] (hereinafter, referred to as a compound group SX952).

A compound wherein in the compound (L-2), n is 1, $R^1$ is a methyl group, $R^{3b}$ is an iodine atom, $R^{3c}$ is a hydrogen atom, and Q is any of the substituents shown in [Table L1] to [Table L13] (hereinafter, referred to as a compound group SX953).

A compound wherein in the compound (L-2), n is 2, $R^1$ is a methyl group, $R^{3b}$ is an iodine atom, $R^{3c}$ is a hydrogen atom, and Q is any of the substituents shown in [Table L1] to [Table L13] (hereinafter, referred to as a compound group SX954).

A compound wherein in the compound (L-2), n is 0, $R^1$ is an ethyl group, $R^{3b}$ is an iodine atom, $R^{3c}$ is a hydrogen atom, and Q is any of the substituents shown in [Table L1] to [Table L13] (hereinafter, referred to as a compound group SX955).

A compound wherein in the compound (L-2), n is 1, $R^1$ is an ethyl group, $R^{3b}$ is an iodine atom, $R^{3c}$ is a hydrogen atom, and Q is any of the substituents shown in [Table L1] to [Table L13] (hereinafter, referred to as a compound group SX956).

A compound wherein in the compound (L-2), n is **2,** $R^1$ is an ethyl group, $R^{3b}$ is an iodine atom, $R^{3c}$ is a hydrogen atom, and Q is any of the substituents shown in [Table L1] to [Table L13] (hereinafter, referred to as a compound group SX957).

A compound wherein in the compound (L-2), n is 0, $R^1$ is a benzyl group, $R^{3b}$ is an iodine atom, $R^{3c}$ is a hydrogen atom, and Q is any of the substituents shown in [Table L1] to [Table L13] (hereinafter, referred to as a compound group SX958).

A compound wherein in the compound (L-2), n is 1, $R^1$ is a benzyl group, $R^{3b}$ is an iodine atom, $R^{3c}$ is a hydrogen atom, and Q is any of the substituents shown in [Table L1] to [Table L13] (hereinafter, referred to as a compound group SX959).

A compound wherein in the compound (L-2), n is 2, $R^1$ is a benzyl group, $R^{3b}$ is an iodine atom, $R^{3c}$ is a hydrogen atom, and Q is any of the substituents shown in [Table L1] to [Table L13] (hereinafter, referred to as a compound group SX960).

A compound wherein in the compound (L-2), n is 0, $R^1$ is a methyl group, $R^{3b}$ is a trifluoromethyl group, $R^{3c}$ is a hydrogen atom, and Q is any of the substituents shown in [Table L1] to [Table L13] (hereinafter, referred to as a compound group SX964).

A compound wherein in the compound (L-2), n is 1, $R^1$ is a methyl group, $R^{3b}$ is a trifluoromethyl group, $R^{3c}$ is a hydrogen atom, and Q is any of the substituents shown in [Table L1] to [Table L13] (hereinafter, referred to as a compound group SX965).

A compound wherein in the compound (L-2), n is 2, $R^1$ is a methyl group, $R^{3b}$ is a trifluoromethyl group, $R^{3c}$ is a hydrogen atom, and Q is any of the substituents shown in [Table L1] to [Table L13] (hereinafter, referred to as a compound group SX966).

A compound wherein in the compound (L-2), n is 0, $R^1$ is an ethyl group, $R^{3b}$ is a trifluoromethyl group, $R^{3c}$ is a hydrogen atom, and Q is any of the substituents shown in [Table L1] to [Table L13] (hereinafter, referred to as a compound group SX967).

A compound wherein in the compound (L-2), n is 1, $R^1$ is an ethyl group, $R^{3b}$ is a trifluoromethyl group, $R^{3c}$ is a hydrogen atom, and Q is any of the substituents shown in [Table L1] to [Table L13] (hereinafter, referred to as a compound group SX968).

A compound wherein in the compound (L-2), n is 2, $R^1$ is an ethyl group, $R^{3b}$ is a trifluoromethyl group, $R^{3c}$ is a hydrogen atom, and Q is any of the substituents shown in [Table L1] to [Table L13] (hereinafter, referred to as a compound group SX969).

A compound wherein in the compound (L-2), n is 0, $R^1$ is a benzyl group, $R^{3b}$ is a trifluoromethyl group, $R^{3c}$ is a hydrogen atom, and Q is any of the substituents shown in [Table L1] to [Table L13] (hereinafter, referred to as a compound group SX970).

A compound wherein in the compound (L-2), n is 1, $R^1$ is a benzyl group, $R^{3b}$ is a trifluoromethyl group, $R^{3c}$ is a hydrogen atom, and Q is any of the substituents shown in [Table L1] to [Table L13] (hereinafter, referred to as a compound group SX971).

A compound wherein in the compound (L-2), n is 2, $R^1$ is a benzyl group, $R^{3b}$ is a trifluoromethyl group, $R^{3c}$ is a hydrogen atom, and Q is any of the substituents shown in [Table L1] to [Table L13] (hereinafter, referred to as a compound group SX972).

A compound wherein in the compound (L-2), n is 0, $R^1$ is a methyl group, $R^{3b}$ is a difluoromethyl group, $R^{3c}$ is a hydrogen atom, and Q is any of the substituents shown in [Table L1] to [Table L13] (hereinafter, referred to as a compound group SX976).

A compound wherein in the compound (L-2), n is 1, $R^1$ is a methyl group, $R^{3b}$ is a difluoromethyl group, $R^{3c}$ is a hydrogen atom, and Q is any of the substituents shown in [Table L1] to [Table L13] (hereinafter, referred to as a compound group SX977).

A compound wherein in the compound (L-2), n is 2, $R^1$ is a methyl group, $R^{3b}$ is a difluoromethyl group, $R^{3c}$ is a hydrogen atom, and Q is any of the substituents shown in [Table L1] to [Table L13] (hereinafter, referred to as a compound group SX978).

A compound wherein in the compound (L-2), n is 0, $R^1$ is an ethyl group, $R^{3b}$ is a difluoromethyl group, $R^{3c}$ is a hydrogen atom, and Q is any of the substituents shown in [Table L1] to [Table L13] (hereinafter, referred to as a compound group SX979).

A compound wherein in the compound (L-2), n is 1, $R^1$ is an ethyl group, $R^{3b}$ is a difluoromethyl group, $R^{3c}$ is a hydrogen atom, and Q is any of the substituents shown in [Table L1] to [Table L13] (hereinafter, referred to as a compound group SX980).

A compound wherein in the compound (L-2), n is 2, $R^1$ is an ethyl group, $R^{3b}$ is a difluoromethyl group, $R^{3c}$ is a hydrogen atom, and Q is any of the substituents shown in [Table L1] to [Table L13] (hereinafter, referred to as a compound group SX981).

A compound wherein in the compound (L-2), n is 0, $R^1$ is a benzyl group, $R^{3b}$ is a difluoromethyl group, $R^{3c}$ is a hydrogen atom, and Q is any of the substituents shown in [Table L1] to [Table L13] (hereinafter, referred to as a compound group SX982).

A compound wherein in the compound (L-2), n is 1, $R^1$ is a benzyl group, $R^{3b}$ is a difluoromethyl group, $R^{3c}$ is a

hydrogen atom, and Q is any of the substituents shown in [Table L1] to [Table L13] (hereinafter, referred to as a compound group SX983).

A compound wherein in the compound (L-2), n is **2,** $R^1$ is a benzyl group, $R^{3b}$ is a difluoromethyl group, $R^{3c}$ is a hydrogen atom, and Q is any of the substituents shown in [Table L1] to [Table L13] (hereinafter, referred to **as** a compound group SX984).

A compound wherein in the compound (L-2), n is 0, $R^1$ is a methyl group, $R^{3b}$ is a hydrogen atom, $R^{3c}$ is a fluorine atom, and Q is any of the substituents shown in [Table L1] to [Table L13] (hereinafter, referred to as a compound group SX988).

A compound wherein in the compound (L-2), n is 1, $R^1$ is a methyl group, $R^{3b}$ is a hydrogen atom, $R^{3c}$ is a fluorine atom, and Q is any of the substituents shown in [Table L1] to [Table L13] (hereinafter, referred to as a compound group SX989).

A compound wherein in the compound (L-2), n is 2, $R^1$ is a methyl group, $R^{3b}$ is a hydrogen atom, $R^{3c}$ is a fluorine atom, and Q is any of the substituents shown in [Table L1] to [Table L13] (hereinafter, referred to as a compound group SX990).

A compound wherein in the compound (L-2), n is 0, $R^1$ is an ethyl group, $R^{3b}$ is a hydrogen atom, $R^{3c}$ is a fluorine atom, and Q is any of the substituents shown in [Table L1] to [Table L13] (hereinafter, referred to as a compound group SX991).

A compound wherein in the compound (L-2), n is 1, $R^1$ is an ethyl group, $R^{3b}$ is a hydrogen atom, $R^{3c}$ is a fluorine atom, and Q is any of the substituents shown in [Table L1] to [Table L13] (hereinafter, referred to as a compound group SX992).

A compound wherein in the compound (L-2), n is 2, $R^1$ is an ethyl group, $R^{3b}$ is a hydrogen atom, $R^{3c}$ is a fluorine atom, and Q is any of the substituents shown in [Table L1] to [Table L13] (hereinafter, referred to as a compound group SX993).

A compound wherein in the compound (L-2), n is 0, $R^1$ is a benzyl group, $R^{3b}$ is a hydrogen atom, $R^{3c}$ is a fluorine atom, and Q is any of the substituents shown in [Table L1] to [Table L13] (hereinafter, referred to as a compound group SX994).

A compound wherein in the compound (L-2), n is 1, $R^1$ is a benzyl group, $R^{3b}$ is a hydrogen atom, $R^{3c}$ is a fluorine atom, and Q is any of the substituents shown in [Table L1] to [Table L13] (hereinafter, referred to as a compound group SX995).

A compound wherein in the compound (L-2), n is 2, $R^1$ is a benzyl group, $R^{3b}$ is a hydrogen atom, $R^{3c}$ is a fluorine atom, and Q is any of the substituents shown in [Table L1] to [Table L13] (hereinafter, referred to as a compound group SX996).

A compound wherein in the compound (L-2), n is 0, $R^1$ is a methyl group, $R^{3b}$ is a fluorine atom, $R^{3c}$ is a fluorine atom, and Q is any of the substituents shown in [Table L1] to [Table L13] (hereinafter, referred to as a compound group SX1000).

A compound wherein in the compound (L-2), n is 1, $R^1$ is a methyl group, $R^{3b}$ is a fluorine atom, $R^{3c}$ is a fluorine atom, and Q is any of the substituents shown in [Table L1] to [Table L13] (hereinafter, referred to as a compound group SX1001).

A compound wherein in the compound (L-2), n is 2, $R^1$ is a methyl group, $R^{3b}$ is a fluorine atom, $R^{3c}$ is a fluorine atom, and Q is any of the substituents shown in [Table L1] to [Table L13] (hereinafter, referred to as a compound group SX1002).

A compound wherein in the compound (L-2), n is 0, $R^1$ is an ethyl group, $R^{3b}$ is a fluorine atom, $R^{3c}$ is a fluorine atom, and Q is any of the substituents shown in [Table L1] to [Table L13] (hereinafter, referred to as a compound group SX1003).

A compound wherein in the compound (L-2), n is 1, $R^1$ is an ethyl group, $R^{3b}$ is a fluorine atom, $R^{3c}$ is a fluorine atom, and Q is any of the substituents shown in [Table L1] to [Table L13] (hereinafter, referred to as a compound group SX1004).

A compound wherein in the compound (L-2), n is 2, $R^1$ is an ethyl group, $R^{3b}$ is a fluorine atom, $R^{3c}$ is a fluorine atom, and Q is any of the substituents shown in [Table L1] to [Table L13] (hereinafter, referred to as a compound group SX1005).

A compound wherein in the compound (L-2), n is 0, $R^1$ is a benzyl group, $R^{3b}$ is a fluorine atom, $R^{3c}$ is a fluorine atom, and Q is any of the substituents shown in [Table L1] to [Table L13] (hereinafter, referred to as a compound group SX1006).

A compound wherein in the compound (L-2), n is 1, $R^1$ is a benzyl group, $R^{3b}$ is a fluorine atom, $R^{3c}$ is a fluorine atom, and Q is any of the substituents shown in [Table L1] to [Table L13] (hereinafter, referred to as a compound group SX1007).

A compound wherein in the compound (L-2), n is 2, $R^1$ is a benzyl group, $R^{3b}$ is a fluorine atom, $R^{3c}$ is a fluorine atom, and Q is any of the substituents shown in [Table L1] to [Table L13] (hereinafter, referred to as a compound group

SX1008).

A compound wherein in the compound (L-2), n is 0, $R^1$ is a methyl group, $R^{3b}$ is a chlorine atom, $R^{3c}$ is a fluorine atom, and Q is any of the substituents shown in [Table L1] to [Table L13] (hereinafter, referred to as a compound group SX1012).

A compound wherein in the compound (L-2), n is 1, $R^1$ is a methyl group, $R^{3b}$ is a chlorine atom, $R^{3c}$ is a fluorine atom, and Q is any of the substituents shown in [Table L1] to [Table L13] (hereinafter, referred to as a compound group SX1013).

A compound wherein in the compound (L-2), n is 2, $R^1$ is a methyl group, $R^{3b}$ is a chlorine atom, $R^{3c}$ is a fluorine atom, and Q is any of the substituents shown in [Table L1] to [Table L13] (hereinafter, referred to as a compound group SX1014).

A compound wherein in the compound (L-2), n is 0, $R^1$ is an ethyl group, $R^{3b}$ is a chlorine atom, $R^{3c}$ is a fluorine atom, and Q is any of the substituents shown in [Table L1] to [Table L13] (hereinafter, referred to as a compound group SX1015).

A compound wherein in the compound (L-2), n is 1, $R^1$ is an ethyl group, $R^{3b}$ is a chlorine atom, $R^{3c}$ is a fluorine atom, and Q is any of the substituents shown in [Table L1] to [Table L13] (hereinafter, referred to as a compound group SX1016).

A compound wherein in the compound (L-2), n is 2, $R^1$ is an ethyl group, $R^{3b}$ is a chlorine atom, $R^{3c}$ is a fluorine atom, and Q is any of the substituents shown in [Table L1] to [Table L13] (hereinafter, referred to as a compound group SX1017).

A compound wherein in the compound (L-2), n is 0, $R^1$ is a benzyl group, $R^{3b}$ is a chlorine atom, $R^{3c}$ is a fluorine atom, and Q is any of the substituents shown in [Table L1] to [Table L13] (hereinafter, referred to as a compound group SX1018).

A compound wherein in the compound (L-2), n is 1, $R^1$ is a benzyl group, $R^{3b}$ is a chlorine atom, $R^{3c}$ is a fluorine atom, and Q is any of the substituents shown in [Table L1] to [Table L13] (hereinafter, referred to as a compound group SX1019).

A compound wherein in the compound (L-2), n is 2, $R^1$ is a benzyl group, $R^{3b}$ is a chlorine atom, $R^{3c}$ is a fluorine atom, and Q is any of the substituents shown in [Table L1] to [Table L13] (hereinafter, referred to as a compound group SX1020).

A compound wherein in the compound (L-2), n is 0, $R^1$ is a methyl group, $R^{3b}$ is a bromine atom, $R^{3c}$ is a fluorine atom, and Q is any of the substituents shown in [Table L1] to [Table L13] (hereinafter, referred to as a compound group SX1024).

A compound wherein in the compound (L-2), n is 1, $R^1$ is a methyl group, $R^{3b}$ is a bromine atom, $R^{3c}$ is a fluorine atom, and Q is any of the substituents shown in [Table L1] to [Table L13] (hereinafter, referred to as a compound group SX1025).

A compound wherein in the compound (L-2), n is 2, $R^1$ is a methyl group, $R^{3b}$ is a bromine atom, $R^{3c}$ is a fluorine atom, and Q is any of the substituents shown in [Table L1] to [Table L13] (hereinafter, referred to as a compound group SX1026).

A compound wherein in the compound (L-2), n is 0, $R^1$ is an ethyl group, $R^{3b}$ is a bromine atom, $R^{3c}$ is a fluorine atom, and Q is any of the substituents shown in [Table L1] to [Table L13] (hereinafter, referred to as a compound group SX1027).

A compound wherein in the compound (L-2), n is 1, $R^1$ is an ethyl group, $R^{3b}$ is a bromine atom, $R^{3c}$ is a fluorine atom, and Q is any of the substituents shown in [Table L1] to [Table L13] (hereinafter, referred to as a compound group SX1028).

A compound wherein in the compound (L-2), n is 2, $R^1$ is an ethyl group, $R^{3b}$ is a bromine atom, $R^{3c}$ is a fluorine atom, and Q is any of the substituents shown in [Table L1] to [Table L13] (hereinafter, referred to as a compound group SX1029).

A compound wherein in the compound (L-2), n is 0, $R^1$ is a benzyl group, $R^{3b}$ is a bromine atom, $R^{3c}$ is a fluorine atom, and Q is any of the substituents shown in [Table L1] to [Table L13] (hereinafter, referred to as a compound group SX1030).

A compound wherein in the compound (L-2), n is 1, $R^1$ is a benzyl group, $R^{3b}$ is a bromine atom, $R^{3c}$ is a fluorine atom, and Q is any of the substituents shown in [Table L1] to [Table L13] (hereinafter, referred to as a compound group SX1031).

A compound wherein in the compound (L-2), n is 2, $R^1$ is a benzyl group, $R^{3b}$ is a bromine atom, $R^{3c}$ is a fluorine atom, and Q is any of the substituents shown in [Table L1] to [Table L13] (hereinafter, referred to as a compound group SX1032).

A compound wherein in the compound (L-2), n is 0, $R^1$ is a methyl group, $R^{3b}$ is an iodine atom, $R^{3c}$ is a fluorine atom, and Q is any of the substituents shown in [Table L1] to [Table L13] (hereinafter, referred to as a compound group SX1036).

A compound wherein in the compound (L-2), n is 1, $R^1$ is a methyl group, $R^{3b}$ is an iodine atom, $R^{3c}$ is a fluorine atom, and Q is any of the substituents shown in [Table L1] to [Table L13] (hereinafter, referred to as a compound group SX1037).

A compound wherein in the compound (L-2), n is 2, $R^1$ is a methyl group, $R^{3b}$ is an iodine atom, $R^{3c}$ is a fluorine atom, and Q is any of the substituents shown in [Table L1] to [Table L13] (hereinafter, referred to as a compound group SX1038).

A compound wherein in the compound (L-2), n is 0, $R^1$ is an ethyl group, $R^{3b}$ is an iodine atom, $R^{3c}$ is a fluorine atom, and Q is any of the substituents shown in [Table L1] to [Table L13] (hereinafter, referred to as a compound group SX1039).

A compound wherein in the compound (L-2), n is 1, $R^1$ is an ethyl group, $R^{3b}$ is an iodine atom, $R^{3c}$ is a fluorine atom, and Q is any of the substituents shown in [Table L1] to [Table L13] (hereinafter, referred to as a compound group SX1040).

A compound wherein in the compound (L-2), n is 2, $R^1$ is an ethyl group, $R^{3b}$ is an iodine atom, $R^{3c}$ is a fluorine atom, and Q is any of the substituents shown in [Table L1] to [Table L13] (hereinafter, referred to as a compound group SX1041).

A compound wherein in the compound (L-2), n is 0, $R^1$ is a benzyl group, $R^{3b}$ is an iodine atom, $R^{3c}$ is a fluorine atom, and Q is any of the substituents shown in [Table L1] to [Table L13] (hereinafter, referred to as a compound group SX1042).

A compound wherein in the compound (L-2), n is 1, $R^1$ is a benzyl group, $R^{3b}$ is an iodine atom, $R^{3c}$ is a fluorine atom, and Q is any of the substituents shown in [Table L1] to [Table L13] (hereinafter, referred to as a compound group SX1043).

A compound wherein in the compound (L-2), n is 2, $R^1$ is a benzyl group, $R^{3b}$ is an iodine atom, $R^{3c}$ is a fluorine atom, and Q is any of the substituents shown in [Table L1] to [Table L13] (hereinafter, referred to as a compound group SX1044).

A compound wherein in the compound (L-2), n is 0, $R^1$ is a methyl group, $R^{3b}$ is a trifluoromethyl group, $R^{3c}$ is a fluorine atom, and Q is any of the substituents shown in [Table L1] to [Table L13] (hereinafter, referred to as a compound group SX1048).

A compound wherein in the compound (L-2), n is 1, $R^1$ is a methyl group, $R^{3b}$ is a trifluoromethyl group, $R^{3c}$ is a fluorine atom, and Q is any of the substituents shown in [Table L1] to [Table L13] (hereinafter, referred to as a compound group SX1049).

A compound wherein in the compound (L-2), n is 2, $R^1$ is a methyl group, $R^{3b}$ is a trifluoromethyl group, $R^{3c}$ is a fluorine atom, and Q is any of the substituents shown in [Table L1] to [Table L13] (hereinafter, referred to as a compound group SX1050).

A compound wherein in the compound (L-2), n is 0, $R^1$ is an ethyl group, $R^{3b}$ is a trifluoromethyl group, $R^{3c}$ is a fluorine atom, and Q is any of the substituents shown in [Table L1] to [Table L13] (hereinafter, referred to as a compound group SX1051).

A compound wherein in the compound (L-2), n is 1, $R^1$ is an ethyl group, $R^{3b}$ is a trifluoromethyl group, $R^{3c}$ is a fluorine atom, and Q is any of the substituents shown in [Table L1] to [Table L13] (hereinafter, referred to as a compound group SX1052).

A compound wherein in the compound (L-2), n is 2, $R^1$ is an ethyl group, $R^{3b}$ is a trifluoromethyl group, $R^{3c}$ is a fluorine atom, and Q is any of the substituents shown in [Table L1] to [Table L13] (hereinafter, referred to as a compound group SX1053).

A compound wherein in the compound (L-2), n is 0, $R^1$ is a benzyl group, $R^{3b}$ is a trifluoromethyl group, $R^{3c}$ is a fluorine atom, and Q is any of the substituents shown in [Table L1] to [Table L13] (hereinafter, referred to as a compound group SX1054).

A compound wherein in the compound (L-2), n is 1, $R^1$ is a benzyl group, $R^{3b}$ is a trifluoromethyl group, $R^{3c}$ is a fluorine atom, and Q is any of the substituents shown in [Table L1] to [Table L13] (hereinafter, referred to as a compound group SX1055).

A compound wherein in the compound (L-2), n is 2, $R^1$ is a benzyl group, $R^{3b}$ is a trifluoromethyl group, $R^{3c}$ is a fluorine atom, and Q is any of the substituents shown in [Table L1] to [Table L13] (hereinafter, referred to as a compound group SX1056).

A compound wherein in the compound (L-2), n is 0, $R^1$ is a methyl group, $R^{3b}$ is a difluoromethyl group, $R^{3c}$ is a fluorine atom, and Q is any of the substituents shown in [Table L1] to [Table L13] (hereinafter, referred to as a compound group SX1060).

A compound wherein in the compound (L-2), n is 1, $R^1$ is a methyl group, $R^{3b}$ is a difluoromethyl group, $R^{3c}$ is a fluorine atom, and Q is any of the substituents shown in [Table L1] to [Table L13] (hereinafter, referred to as a compound group SX1061).

A compound wherein in the compound (L-2), n is 2, $R^1$ is a methyl group, $R^{3b}$ is a difluoromethyl group, $R^{3c}$ is a

fluorine atom, and Q is any of the substituents shown in [Table L1] to [Table L13] (hereinafter, referred to as a compound group SX1062).

A compound wherein in the compound (L-2), n is 0, $R^1$ is an ethyl group, $R^{3b}$ is a difluoromethyl group, $R^{3c}$ is a fluorine atom, and Q is any of the substituents shown in [Table L1] to [Table L13] (hereinafter, referred to as a compound group SX1063).

A compound wherein in the compound (L-2), n is 1, $R^1$ is an ethyl group, $R^{3b}$ is a difluoromethyl group, $R^{3c}$ is a fluorine atom, and Q is any of the substituents shown in [Table L1] to [Table L13] (hereinafter, referred to as a compound group SX1064).

A compound wherein in the compound (L-2), n is 2, $R^1$ is an ethyl group, $R^{3b}$ is a difluoromethyl group, $R^{3c}$ is a fluorine atom, and Q is any of the substituents shown in [Table L1] to [Table L13] (hereinafter, referred to as a compound group SX1065).

A compound wherein in the compound (L-2), n is 0, $R^1$ is a benzyl group, $R^{3b}$ is a difluoromethyl group, $R^{3c}$ is a fluorine atom, and Q is any of the substituents shown in [Table L1] to [Table L13] (hereinafter, referred to **as** a compound group SX1066).

**A** compound wherein in the compound (L-2), n is 1, $R^1$ is a benzyl group, $R^{3b}$ is a difluoromethyl group, $R^{3c}$ is a fluorine atom, and **Q** is any of the substituents shown in [Table L1] to [Table L13] (hereinafter, referred to as a compound group SX1067).

A compound wherein in the compound (L-2), n is **2,** $R^1$ is a benzyl group, $R^{3b}$ is a difluoromethyl group, $R^{3c}$ is a fluorine atom, and **Q** is any of the substituents shown in [Table L1] to [Table L13] (hereinafter, referred to as a compound group SX1068).

A compound wherein in the compound (L-2), n is 0, $R^1$ is a methyl group, $R^{3b}$ is a hydrogen atom, $R^{3c}$ is a bromine atom, and Q is any of the substituents shown in [Table L1] to [Table L13] (hereinafter, referred to as a compound group SX1072).

A compound wherein in the compound (L-2), n is 1, $R^1$ is a methyl group, $R^{3b}$ is a hydrogen atom, $R^{3c}$ is a bromine atom, and Q is any of the substituents shown in [Table L1] to [Table L13] (hereinafter, referred to as a compound group SX1073).

A compound wherein in the compound (L-2), n is 2, $R^1$ is a methyl group, $R^{3b}$ is a hydrogen atom, $R^{3c}$ is a bromine atom, and Q is any of the substituents shown in [Table L1] to [Table L13] (hereinafter, referred to as a compound group SX1074).

A compound wherein in the compound (L-2), n is 0, $R^1$ is an ethyl group, $R^{3b}$ is a hydrogen atom, $R^{3c}$ is a bromine atom, and Q is any of the substituents shown in [Table L1] to [Table L13] (hereinafter, referred to as a compound group SX1075).

A compound wherein in the compound (L-2), n is 1, $R^1$ is an ethyl group, $R^{3b}$ is a hydrogen atom, $R^{3c}$ is a bromine atom, and Q is any of the substituents shown in [Table L1] to [Table L13] (hereinafter, referred to as a compound group SX1076).

A compound wherein in the compound (L-2), n is 2, $R^1$ is an ethyl group, $R^{3b}$ is a hydrogen atom, $R^{3c}$ is a bromine atom, and Q is any of the substituents shown in [Table L1] to [Table L13] (hereinafter, referred to as a compound group SX1077).

A compound wherein in the compound (L-2), n is 0, $R^1$ is a benzyl group, $R^{3b}$ is a hydrogen atom, $R^{3c}$ is a bromine atom, and Q is any of the substituents shown in [Table L1] to [Table L13] (hereinafter, referred to as a compound group SX1078).

A compound wherein in the compound (L-2), n is 1, $R^1$ is a benzyl group, $R^{3b}$ is a hydrogen atom, $R^{3c}$ is a bromine atom, and Q is any of the substituents shown in [Table L1] to [Table L13] (hereinafter, referred to as a compound group SX1079).

A compound wherein in the compound (L-2), n is 2, $R^1$ is a benzyl group, $R^{3b}$ is a hydrogen atom, $R^{3c}$ is a bromine atom, and Q is any of the substituents shown in [Table L1] to [Table L13] (hereinafter, referred to as a compound group SX1080).

A compound wherein in the compound (L-2), n is 0, $R^1$ is a methyl group, $R^{3b}$ is a fluorine atom, $R^{3c}$ is a bromine atom, and Q is any of the substituents shown in [Table L1] to [Table L13] (hereinafter, referred to as a compound group SX1084).

A compound wherein in the compound (L-2), n is 1, $R^1$ is a methyl group, $R^{3b}$ is a fluorine atom, $R^{3c}$ is a bromine atom, and Q is any of the substituents shown in [Table L1] to [Table L13] (hereinafter, referred to as a compound group SX1085).

A compound wherein in the compound (L-2), n is 2, $R^1$ is a methyl group, $R^{3b}$ is a fluorine atom, $R^{3c}$ is a bromine atom, and Q is any of the substituents shown in [Table L1] to [Table L13] (hereinafter, referred to as a compound group SX1086).

A compound wherein in the compound (L-2), n is 0, $R^1$ is an ethyl group, $R^{3b}$ is a fluorine atom, $R^{3c}$ is a bromine atom, and Q is any of the substituents shown in [Table L1] to [Table L13] (hereinafter, referred to as a compound group

SX1087).

A compound wherein in the compound (L-2), n is 1, $R^1$ is an ethyl group, $R^{3b}$ is a fluorine atom, $R^{3c}$ is a bromine atom, and Q is any of the substituents shown in [Table L1] to [Table L13] (hereinafter, referred to as a compound group SX1088).

A compound wherein in the compound (L-2), n is 2, $R^1$ is an ethyl group, $R^{3b}$ is a fluorine atom, $R^{3c}$ is a bromine atom, and Q is any of the substituents shown in [Table L1] to [Table L13] (hereinafter, referred to as a compound group SX1089).

A compound wherein in the compound (L-2), n is 0, $R^1$ is a benzyl group, $R^{3b}$ is a fluorine atom, $R^{3c}$ is a bromine atom, and Q is any of the substituents shown in [Table L1] to [Table L13] (hereinafter, referred to as a compound group SX1090).

A compound wherein in the compound (L-2), n is 1, $R^1$ is a benzyl group, $R^{3b}$ is a fluorine atom, $R^{3c}$ is a bromine atom, and Q is any of the substituents shown in [Table L1] to [Table L13] (hereinafter, referred to as a compound group SX1091).

A compound wherein in the compound (L-2), n is **2,** $R^1$ is a benzyl group, $R^{3b}$ is a fluorine atom, $R^{3c}$ is a bromine atom, and Q is any of the substituents shown in [Table L1] to [Table L13] (hereinafter, referred to as a compound group SX1092).

**A** compound wherein in the compound (L-2), n is **0,** $R^1$ is a methyl group, $R^{3b}$ is a chlorine atom, $R^{3c}$ is a bromine atom, and **Q** is any of the substituents shown in [Table L1] to [Table L13] (hereinafter, referred to as a compound group SX1096).

A compound wherein in the compound (L-2), n is 1, $R^1$ is a methyl group, $R^{3b}$ is a chlorine atom, $R^{3c}$ is a bromine atom, and **Q** is any of the substituents shown in [Table L1] to [Table L13] (hereinafter, referred to as a compound group SX1097).

A compound wherein in the compound (L-2), n is **2,** $R^1$ is a methyl group, $R^{3b}$ is a chlorine atom, $R^{3c}$ is a bromine atom, and **Q** is any of the substituents shown in [Table L1] to [Table L13] (hereinafter, referred to as a compound group SX1098).

A compound wherein in the compound (L-2), n is 0, $R^1$ is an ethyl group, $R^{3b}$ is a chlorine atom, $R^{3c}$ is a bromine atom, and Q is any of the substituents shown in [Table L1] to [Table L13] (hereinafter, referred to as a compound group SX1099).

A compound wherein in the compound (L-2), n is 1, $R^1$ is an ethyl group, $R^{3b}$ is a chlorine atom, $R^{3c}$ is a bromine atom, and Q is any of the substituents shown in [Table L1] to [Table L13] (hereinafter, referred to as a compound group SX1100).

A compound wherein in the compound (L-2), n is 2, $R^1$ is an ethyl group, $R^{3b}$ is a chlorine atom, $R^{3c}$ is a bromine atom, and Q is any of the substituents shown in [Table L1] to [Table L13] (hereinafter, referred to as a compound group SX1101).

A compound wherein in the compound (L-2), n is 0, $R^1$ is a benzyl group, $R^{3b}$ is a chlorine atom, $R^{3c}$ is a bromine atom, and Q is any of the substituents shown in [Table L1] to [Table L13] (hereinafter, referred to as a compound group SX1102).

A compound wherein in the compound (L-2), n is 1, $R^1$ is a benzyl group, $R^{3b}$ is a chlorine atom, $R^{3c}$ is a bromine atom, and Q is any of the substituents shown in [Table L1] to [Table L13] (hereinafter, referred to as a compound group SX1103).

A compound wherein in the compound (L-2), n is 2, $R^1$ is a benzyl group, $R^{3b}$ is a chlorine atom, $R^{3c}$ is a bromine atom, and Q is any of the substituents shown in [Table L1] to [Table L13] (hereinafter, referred to as a compound group SX1104).

A compound wherein in the compound (L-2), n is 0, $R^1$ is a methyl group, $R^{3b}$ is a bromine atom, $R^{3c}$ is a bromine atom, and Q is any of the substituents shown in [Table L1] to [Table L13] (hereinafter, referred to as a compound group SX1108).

A compound wherein in the compound (L-2), n is 1, $R^1$ is a methyl group, $R^{3b}$ is a bromine atom, $R^{3c}$ is a bromine atom, and Q is any of the substituents shown in [Table L1] to [Table L13] (hereinafter, referred to as a compound group SX1109).

A compound wherein in the compound (L-2), n is 2, $R^1$ is a methyl group, $R^{3b}$ is a bromine atom, $R^{3c}$ is a bromine atom, and Q is any of the substituents shown in [Table L1] to [Table L13] (hereinafter, referred to as a compound group SX1110).

A compound wherein in the compound (L-2), n is 0, $R^1$ is an ethyl group, $R^{3b}$ is a bromine atom, $R^{3c}$ is a bromine atom, and Q is any of the substituents shown in [Table L1] to [Table L13] (hereinafter, referred to as a compound group SX1111).

A compound wherein in the compound (L-2), n is 1, $R^1$ is an ethyl group, $R^{3b}$ is a bromine atom, $R^{3c}$ is a bromine atom, and Q is any of the substituents shown in [Table L1] to [Table L13] (hereinafter, referred to as a compound group SX1112).

A compound wherein in the compound (L-2), n is 2, $R^1$ is an ethyl group, $R^{3b}$ is a bromine atom, $R^{3c}$ is a bromine atom, and Q is any of the substituents shown in [Table L1] to [Table L13] (hereinafter, referred to as a compound group SX1113).

A compound wherein in the compound (L-2), n is 0, $R^1$ is a benzyl group, $R^{3b}$ is a bromine atom, $R^{3c}$ is a bromine atom, and Q is any of the substituents shown in [Table L1] to [Table L13] (hereinafter, referred to as a compound group SX1114).

A compound wherein in the compound (L-2), n is 1, $R^1$ is a benzyl group, $R^{3b}$ is a bromine atom, $R^{3c}$ is a bromine atom, and Q is any of the substituents shown in [Table L1] to [Table L13] (hereinafter, referred to as a compound group SX1115).

A compound wherein in the compound (L-2), n is 2, $R^1$ is a benzyl group, $R^{3b}$ is a bromine atom, $R^{3c}$ is a bromine atom, and Q is any of the substituents shown in [Table L1] to [Table L13] (hereinafter, referred to as a compound group SX1116).

A compound wherein in the compound (L-2), n is 0, $R^1$ is a methyl group, $R^{3b}$ is an iodine atom, $R^{3c}$ is a bromine atom, and Q is any of the substituents shown in [Table L1] to [Table L13] (hereinafter, referred to as a compound group SX1120).

A compound wherein in the compound (L-2), n is 1, $R^1$ is a methyl group, $R^{3b}$ is an iodine atom, $R^{3c}$ is a bromine atom, and Q is any of the substituents shown in [Table L1] to [Table L13] (hereinafter, referred to as a compound group SX1121).

A compound wherein in the compound (L-2), n is 2, $R^1$ is a methyl group, $R^{3b}$ is an iodine atom, $R^{3c}$ is a bromine atom, and Q is any of the substituents shown in [Table L1] to [Table L13] (hereinafter, referred to as a compound group SX1122).

A compound wherein in the compound (L-2), n is 0, $R^1$ is an ethyl group, $R^{3b}$ is an iodine atom, $R^{3c}$ is a bromine atom, and Q is any of the substituents shown in [Table L1] to [Table L13] (hereinafter, referred to as a compound group SX1123).

A compound wherein in the compound (L-2), n is 1, $R^1$ is an ethyl group, $R^{3b}$ is an iodine atom, $R^{3c}$ is a bromine atom, and **Q** is any of the substituents shown in [Table L1] to [Table L13] (hereinafter, referred to as a compound group SX1124).

A compound wherein in the compound (L-2), n is **2,** $R^1$ is an ethyl group, $R^{3b}$ is an iodine atom, $R^{3c}$ is a bromine atom, and Q is any of the substituents shown in [Table L1] to [Table L13] (hereinafter, referred to as a compound group SX1125).

A compound wherein in the compound (L-2), n is 0, $R^1$ is a benzyl group, $R^{3b}$ is an iodine atom, $R^{3c}$ is a bromine atom, and Q is any of the substituents shown in [Table L1] to [Table L13] (hereinafter, referred to as compound group SX1126).

A compound wherein in the compound (L-2), n is 1, $R^1$ is a benzyl group, $R^{3b}$ is an iodine atom, $R^{3c}$ is a bromine atom, and Q is any of the substituents shown in [Table L1] to [Table L13] (hereinafter, referred to as compound group SX1127).

A compound wherein in the compound (L-2), n is 2, $R^1$ is a benzyl group, $R^{3b}$ is an iodine atom, $R^{3c}$ is a bromine atom, and Q is any of the substituents shown in [Table L1] to [Table L13] (hereinafter, referred to as compound group SX1128).

A compound wherein in the compound (L-2), n is 0, $R^1$ is a methyl group, $R^{3b}$ is a trifluoromethyl group, $R^{3c}$ is a bromine atom, and Q is any of the substituents shown in [Table L1] to [Table L13] (hereinafter, referred to as a compound group SX1132).

A compound wherein in the compound (L-2), n is 1, $R^1$ is a methyl group, $R^{3b}$ is a trifluoromethyl group, $R^{3c}$ is a bromine atom, and Q is any of the substituents shown in [Table L1] to [Table L13] (hereinafter, referred to **as** a compound group SX1133).

A compound wherein in the compound (L-2), n is **2,** $R^1$ is a methyl group, $R^{3b}$ is a trifluoromethyl group, $R^{3c}$ is a bromine atom, and Q is any of the substituents shown in [Table L1] to [Table L13] (hereinafter, referred to as a compound group SX1134).

A compound wherein in the compound (L-2), n is 0, $R^1$ is an ethyl group, $R^{3b}$ is a trifluoromethyl group, $R^{3c}$ is a bromine atom, and Q is any of the substituents shown in [Table L1] to [Table L13] (hereinafter, referred to as a compound group SX1135).

A compound wherein in the compound (L-2), n is 1, $R^1$ is an ethyl group, $R^{3b}$ is a trifluoromethyl group, $R^{3c}$ is a bromine atom, and Q is any of the substituents shown in [Table L1] to [Table L13] (hereinafter, referred to as a compound group SX1136).

A compound wherein in the compound (L-2), n is **2,** $R^1$ is an ethyl group, $R^{3b}$ is a trifluoromethyl group, $R^{3c}$ is a bromine atom, and **Q** is any of the substituents shown in [Table L1] to [Table L13] (hereinafter, referred to as a compound group SX1137).

A compound wherein in the compound (L-2), n is 0, $R^1$ is a benzyl group, $R^{3b}$ is a trifluoromethyl group, $R^{3c}$ is a

bromine atom, and Q is any of the substituents shown in [Table L1] to [Table L13] (hereinafter, referred to **as** a compound group SX1138).

A compound wherein in the compound (L-2), n is 1, $R^1$ is a benzyl group, $R^{3b}$ is a trifluoromethyl group, $R^{3c}$ is a bromine atom, and Q is any of the substituents shown in [Table L1] to [Table L13] (hereinafter, referred to **as** a compound group SX1139).

**A** compound wherein in the compound (L-2), n is 2, $R^1$ is a benzyl group, $R^{3b}$ is a trifluoromethyl group, $R^{3c}$ is a bromine atom, and **Q** is any of the substituents shown in [Table L1] to [Table L13] (hereinafter, referred to as a compound group SX1140).

A compound wherein in the compound (L-2), n is 0, $R^1$ is a methyl group, $R^{3b}$ is a difluoromethyl group, $R^{3c}$ is a bromine atom, and **Q** is any of the substituents shown in [Table L1] to [Table L13] (hereinafter, referred to as a compound group SX1144).

A compound wherein in the compound (L-2), n is 1, $R^1$ is a methyl group, $R^{3b}$ is a difluoromethyl group, $R^{3c}$ is a bromine atom, and Q is any of the substituents shown in [Table L1] to [Table L13] (hereinafter, referred to as a compound group SX1145).

A compound wherein in the compound (L-2), n is **2,** $R^1$ is a methyl group, $R^{3b}$ is a difluoromethyl group, $R^{3c}$ is a bromine atom, and Q is any of the substituents shown in [Table L1] to [Table L13] (hereinafter, referred to **as** a compound group SX1146).

A compound wherein in the compound (L-2), n is 0, $R^1$ is an ethyl group, $R^{3b}$ is a difluoromethyl group, $R^{3c}$ is a bromine atom, and Q is any of the substituents shown in [Table L1] to [Table L13] (hereinafter, referred to as a compound group SX1147).

A compound wherein in the compound (L-2), n is 1, $R^1$ is an ethyl group, $R^{3b}$ is a difluoromethyl group, $R^{3c}$ is a bromine atom, and Q is any of the substituents shown in [Table L1] to [Table L13] (hereinafter, referred to as a compound group SX1148).

A compound wherein in the compound (L-2), n is 2, $R^1$ is an ethyl group, $R^{3b}$ is a difluoromethyl group, $R^{3c}$ is a bromine atom, and Q is any of the substituents shown in [Table L1] to [Table L13] (hereinafter, referred to as a compound group SX1149).

A compound wherein in the compound (L-2), n is 0, $R^1$ is a benzyl group, $R^{3b}$ is a difluoromethyl group, $R^{3c}$ is a bromine atom, and Q is any of the substituents shown in [Table L1] to [Table L13] (hereinafter, referred to as a compound group SX1150).

A compound wherein in the compound (L-2), n is 1, $R^1$ is a benzyl group, $R^{3b}$ is a difluoromethyl group, $R^{3c}$ is a bromine atom, and Q is any of the substituents shown in [Table L1] to [Table L13] (hereinafter, referred to as a compound group SX1151).

A compound wherein in the compound (L-2), n is 2, $R^1$ is a benzyl group, $R^{3b}$ is a difluoromethyl group, $R^{3c}$ is a bromine atom, and Q is any of the substituents shown in [Table L1] to [Table L13] (hereinafter, referred to **as** a compound group SX1152).

**A** compound wherein in the compound (L-2), n is **0,** $R^1$ is a methyl group, $R^{3b}$ is a hydrogen atom, $R^{3c}$ is an iodine atom, and **Q** is any of the substituents shown in [Table L1] to [Table L13] (hereinafter, referred to as a compound group SX1156).

A compound wherein in the compound (L-2), n is 1, $R^1$ is a methyl group, $R^{3b}$ is a hydrogen atom, $R^{3c}$ is an iodine atom, and **Q** is any of the substituents shown in [Table L1] to [Table L13] (hereinafter, referred to as a compound group SX1157).

A compound wherein in the compound (L-2), n is 2, $R^1$ is a methyl group, $R^{3b}$ is a hydrogen atom, $R^{3c}$ is an iodine atom, and Q is any of the substituents shown in [Table L1] to [Table L13] (hereinafter, referred to as a compound group SX1158).

A compound wherein in the compound (L-2), n is 0, $R^1$ is an ethyl group, $R^{3b}$ is a hydrogen atom, $R^{3c}$ is an iodine atom, and Q is any of the substituents shown in [Table L1] to [Table L13] (hereinafter, referred to as a compound group SX1159).

A compound wherein in the compound (L-2), n is 1, $R^1$ is an ethyl group, $R^{3b}$ is a hydrogen atom, $R^{3c}$ is an iodine atom, and Q is any of the substituents shown in [Table L1] to [Table L13] (hereinafter, referred to as a compound group SX1160).

A compound wherein in the compound (L-2), n is 2, $R^1$ is an ethyl group, $R^{3b}$ is a hydrogen atom, $R^{3c}$ is an iodine atom, and Q is any of the substituents shown in [Table L1] to [Table L13] (hereinafter, referred to as a compound group SX1161).

A compound wherein in the compound (L-2), n is 0, $R^1$ is a benzyl group, $R^{3b}$ is a hydrogen atom, $R^{3c}$ is an iodine atom, and Q is any of the substituents shown in [Table L1] to [Table L13] (hereinafter, referred to as a compound group SX1162).

A compound wherein in the compound (L-2), n is 1, $R^1$ is a benzyl group, $R^{3b}$ is a hydrogen atom, $R^{3c}$ is an iodine atom, and Q is any of the substituents shown in [Table L1] to [Table L13] (hereinafter, referred to as a compound group

SX1163).

A compound wherein in the compound (L-2), n is 2, $R^1$ is a benzyl group, $R^{3b}$ is a hydrogen atom, $R^{3c}$ is an iodine atom, and Q is any of the substituents shown in [Table L1] to [Table L13] (hereinafter, referred to as a compound group SX1164).

A compound wherein in the compound (L-2), n is 0, $R^1$ is a methyl group, $R^{3b}$ is a fluorine atom, $R^{3c}$ is an iodine atom, and Q is any of the substituents shown in [Table L1] to [Table L13] (hereinafter, referred to as a compound group SX1168).

A compound wherein in the compound (L-2), n is 1, $R^1$ is a methyl group, $R^{3b}$ is a fluorine atom, $R^{3c}$ is an iodine atom, and Q is any of the substituents shown in [Table L1] to [Table L13] (hereinafter, referred to as a compound group SX1169).

A compound wherein in the compound (L-2), n is 2, $R^1$ is a methyl group, $R^{3b}$ is a fluorine atom, $R^{3c}$ is an iodine atom, and Q is any of the substituents shown in [Table L1] to [Table L13] (hereinafter, referred to as a compound group SX1170).

A compound wherein in the compound (L-2), n is 0, $R^1$ is an ethyl group, $R^{3b}$ is a fluorine atom, $R^{3c}$ is an iodine atom, and Q is any of the substituents shown in [Table L1] to [Table L13] (hereinafter, referred to as a compound group SX1171).

A compound wherein in the compound (L-2), n is 1, $R^1$ is an ethyl group, $R^{3b}$ is a fluorine atom, $R^{3c}$ is an iodine atom, and Q is any of the substituents shown in [Table L1] to [Table L13] (hereinafter, referred to as a compound group SX1172).

A compound wherein in the compound (L-2), n is 2, $R^1$ is an ethyl group, $R^{3b}$ is a fluorine atom, $R^{3c}$ is an iodine atom, and Q is any of the substituents shown in [Table L1] to [Table L13] (hereinafter, referred to as a compound group SX1173).

A compound wherein in the compound (L-2), n is 0, $R^1$ is a benzyl group, $R^{3b}$ is a fluorine atom, $R^{3c}$ is an iodine atom, and Q is any of the substituents shown in [Table L1] to [Table L13] (hereinafter, referred to as a compound group SX1174).

A compound wherein in the compound (L-2), n is 1, $R^1$ is a benzyl group, $R^{3b}$ is a fluorine atom, $R^{3c}$ is an iodine atom, and Q is any of the substituents shown in [Table L1] to [Table L13] (hereinafter, referred to as a compound group SX1175).

A compound wherein in the compound (L-2), n is 2, $R^1$ is a benzyl group, $R^{3b}$ is a fluorine atom, $R^{3c}$ is an iodine atom, and Q is any of the substituents shown in [Table L1] to [Table L13] (hereinafter, referred to as a compound group SX1176).

A compound wherein in the compound (L-2), n is 0, $R^1$ is a methyl group, $R^{3b}$ is a chlorine atom, $R^{3c}$ is an iodine atom, and Q is any of the substituents shown in [Table L1] to [Table L13] (hereinafter, referred to as a compound group SX1180).

A compound wherein in the compound (L-2), n is 1, $R^1$ is a methyl group, $R^{3b}$ is a chlorine atom, $R^{3c}$ is an iodine atom, and Q is any of the substituents shown in [Table L1] to [Table L13] (hereinafter, referred to as a compound group SX1181).

A compound wherein in the compound (L-2), n is 2, $R^1$ is a methyl group, $R^{3b}$ is a chlorine atom, $R^{3c}$ is an iodine atom, and Q is any of the substituents shown in [Table L1] to [Table L13] (hereinafter, referred to as a compound group SX1182).

A compound wherein in the compound (L-2), n is 0, $R^1$ is an ethyl group, $R^{3b}$ is a chlorine atom, $R^{3c}$ is an iodine atom, and Q is any of the substituents shown in [Table L1] to [Table L13] (hereinafter, referred to as a compound group SX1183).

A compound wherein in the compound (L-2), n is 1, $R^1$ is an ethyl group, $R^{3b}$ is a chlorine atom, $R^{3c}$ is an iodine atom, and Q is any of the substituents shown in [Table L1] to [Table L13] (hereinafter, referred to as a compound group SX1184).

A compound wherein in the compound (L-2), n is 2, $R^1$ is an ethyl group, $R^{3b}$ is a chlorine atom, $R^{3c}$ is an iodine atom, and Q is any of the substituents shown in [Table L1] to [Table L13] (hereinafter, referred to as a compound group SX1185).

A compound wherein in the compound (L-2), n is 0, $R^1$ is a benzyl group, $R^{3b}$ is a chlorine atom, $R^{3c}$ is an iodine atom, and Q is any of the substituents shown in [Table L1] to [Table L13] (hereinafter, referred to as a compound group SX1186).

A compound wherein in the compound (L-2), n is 1, $R^1$ is a benzyl group, $R^{3b}$ is a chlorine atom, $R^{3c}$ is an iodine atom, and Q is any of the substituents shown in [Table L1] to [Table L13] (hereinafter, referred to as a compound group SX1187).

A compound wherein in the compound (L-2), n is 2, $R^1$ is a benzyl group, $R^{3b}$ is a chlorine atom, $R^{3c}$ is an iodine atom, and Q is any of the substituents shown in [Table L1] to [Table L13] (hereinafter, referred to as a compound group SX1188).

A compound wherein in the compound (L-2), n is 0, $R^1$ is a methyl group, $R^{3b}$ is a bromine atom, $R^{3c}$ is an iodine atom, and Q is any of the substituents shown in [Table L1] to [Table L13] (hereinafter, referred to as a compound group SX1192).

A compound wherein in the compound (L-2), n is 1, $R^1$ is a methyl group, $R^{3b}$ is a bromine atom, $R^{3c}$ is an iodine atom, and Q is any of the substituents shown in [Table L1] to [Table L13] (hereinafter, referred to as a compound group SX1193).

A compound wherein in the compound (L-2), n is 2, $R^1$ is a methyl group, $R^{3b}$ is a bromine atom, $R^{3c}$ is an iodine atom, and Q is any of the substituents shown in [Table L1] to [Table L13] (hereinafter, referred to as a compound group SX1194).

A compound wherein in the compound (L-2), n is 0, $R^1$ is an ethyl group, $R^{3b}$ is a bromine atom, $R^{3c}$ is an iodine atom, and Q is any of the substituents shown in [Table L1] to [Table L13] (hereinafter, referred to as a compound group SX1195).

A compound wherein in the compound (L-2), n is 1, $R^1$ is an ethyl group, $R^{3b}$ is a bromine atom, $R^{3c}$ is an iodine atom, and Q is any of the substituents shown in [Table L1] to [Table L13] (hereinafter, referred to as a compound group SX1196).

A compound wherein in the compound (L-2), n is 2, $R^1$ is an ethyl group, $R^{3b}$ is a bromine atom, $R^{3c}$ is an iodine atom, and Q is any of the substituents shown in [Table L1] to [Table L13] (hereinafter, referred to as a compound group SX1197).

A compound wherein in the compound (L-2), n is 0, $R^1$ is a benzyl group, $R^{3b}$ is a bromine atom, $R^{3c}$ is an iodine atom, and Q is any of the substituents shown in [Table L1] to [Table L13] (hereinafter, referred to as compound group SX1198).

A compound wherein in the compound (L-2), n is 1, $R^1$ is a benzyl group, $R^{3b}$ is a bromine atom, $R^{3c}$ is an iodine atom, and Q is any of the substituents shown in [Table L1] to [Table L13] (hereinafter, referred to as compound group SX1199).

A compound wherein in the compound (L-2), n is 2, $R^1$ is a benzyl group, $R^{3b}$ is a bromine atom, $R^{3c}$ is an iodine atom, and Q is any of the substituents shown in [Table L1] to [Table L13] (hereinafter, referred to as compound group SX1200).

A compound wherein in the compound (L-2), n is 0, $R^1$ is a methyl group, $R^{3b}$ is an iodine atom, $R^{3c}$ is an iodine atom, and Q is any of the substituents shown in [Table L1] to [Table L13] (hereinafter, referred to as a compound group SX1204).

A compound wherein in the compound (L-2), n is 1, $R^1$ is a methyl group, $R^{3b}$ is an iodine atom, $R^{3c}$ is an iodine atom, and Q is any of the substituents shown in [Table L1] to [Table L13] (hereinafter, referred to as a compound group SX1205).

A compound wherein in the compound (L-2), n is 2, $R^1$ is a methyl group, $R^{3b}$ is an iodine atom, $R^{3c}$ is an iodine atom, and Q is any of the substituents shown in [Table L1] to [Table L13] (hereinafter, referred to as a compound group SX1206).

A compound wherein in the compound (L-2), n is 0, $R^1$ is an ethyl group, $R^{3b}$ is an iodine atom, $R^{3c}$ is an iodine atom, and Q is any of the substituents shown in [Table L1] to [Table L13] (hereinafter, referred to as a compound group SX1207).

A compound wherein in the compound (L-2), n is 1, $R^1$ is an ethyl group, $R^{3b}$ is an iodine atom, $R^{3c}$ is an iodine atom, and Q is any of the substituents shown in [Table L1] to [Table L13] (hereinafter, referred to as a compound group SX1208).

A compound wherein in the compound (L-2), n is 2, $R^1$ is an ethyl group, $R^{3b}$ is an iodine atom, $R^{3c}$ is an iodine atom, and Q is any of the substituents shown in [Table L1] to [Table L13] (hereinafter, referred to as a compound group SX1209).

A compound wherein in the compound (L-2), n is 0, $R^1$ is a benzyl group, $R^{3b}$ is an iodine atom, $R^{3c}$ is an iodine atom, and Q is any of the substituents shown in [Table L1] to [Table L13] (hereinafter, referred to as compound group SX1210).

A compound wherein in the compound (L-2), n is 1, $R^1$ is a benzyl group, $R^{3b}$ is an iodine atom, $R^{3c}$ is an iodine atom, and Q is any of the substituents shown in [Table L1] to [Table L13] (hereinafter, referred to as compound group SX1211).

A compound wherein in the compound (L-2), n is 2, $R^1$ is a benzyl group, $R^{3b}$ is an iodine atom, $R^{3c}$ is an iodine atom, and Q is any of the substituents shown in [Table L1] to [Table L13] (hereinafter, referred to as compound group SX1212).

A compound wherein in the compound (L-2), n is 0, $R^1$ is a methyl group, $R^{3b}$ is a trifluoromethyl group, $R^{3c}$ is an iodine atom, and Q is any of the substituents shown in [Table L1] to [Table L13] (hereinafter, referred to as a compound group SX1216).

A compound wherein in the compound (L-2), n is 1, $R^1$ is a methyl group, $R^{3b}$ is a trifluoromethyl group, $R^{3c}$ is an

iodine atom, and Q is any of the substituents shown in [Table L1] to [Table L13] (hereinafter, referred to as a compound group SX1217).

A compound wherein in the compound (L-2), n is 2, $R^1$ is a methyl group, $R^{3b}$ is a trifluoromethyl group, $R^{3c}$ is an iodine atom, and Q is any of the substituents shown in [Table L1] to [Table L13] (hereinafter, referred to as a compound group SX1218).

A compound wherein in the compound (L-2), n is 0, $R^1$ is an ethyl group, $R^{3b}$ is a trifluoromethyl group, $R^{3c}$ is an iodine atom, and Q is any of the substituents shown in [Table L1] to [Table L13] (hereinafter, referred to as a compound group SX1219).

A compound wherein in the compound (L-2), n is 1, $R^1$ is an ethyl group, $R^{3b}$ is a trifluoromethyl group, $R^{3c}$ is an iodine atom, and Q is any of the substituents shown in [Table L1] to [Table L13] (hereinafter, referred to as a compound group SX1220).

A compound wherein in the compound (L-2), n is 2, $R^1$ is an ethyl group, $R^{3b}$ is a trifluoromethyl group, $R^{3c}$ is an iodine atom, and Q is any of the substituents shown in [Table L1] to [Table L13] (hereinafter, referred to as a compound group SX1221).

A compound wherein in the compound (L-2), n is 0, $R^1$ is a benzyl group, $R^{3b}$ is a trifluoromethyl group, $R^{3c}$ is an iodine atom, and Q is any of the substituents shown in [Table L1] to [Table L13] (hereinafter, referred to as a compound group SX1222).

A compound wherein in the compound (L-2), n is 1, $R^1$ is a benzyl group, $R^{3b}$ is a trifluoromethyl group, $R^{3c}$ is an iodine atom, and Q is any of the substituents shown in [Table L1] to [Table L13] (hereinafter, referred to as a compound group SX1223).

A compound wherein in the compound (L-2), n is 2, $R^1$ is a benzyl group, $R^{3b}$ is a trifluoromethyl group, $R^{3c}$ is an iodine atom, and Q is any of the substituents shown in [Table L1] to [Table L13] (hereinafter, referred to as a compound group SX1224).

A compound wherein in the compound (L-2), n is 0, $R^1$ is a methyl group, $R^{3b}$ is a difluoromethyl group, $R^{3c}$ is an iodine atom, and Q is any of the substituents shown in [Table L1] to [Table L13] (hereinafter, referred to as a compound group SX1228).

A compound wherein in the compound (L-2), n is 1, $R^1$ is a methyl group, $R^{3b}$ is a difluoromethyl group, $R^{3c}$ is an iodine atom, and Q is any of the substituents shown in [Table L1] to [Table L13] (hereinafter, referred to as a compound group SX1229).

A compound wherein in the compound (L-2), n is 2, $R^1$ is a methyl group, $R^{3b}$ is a difluoromethyl group, $R^{3c}$ is an iodine atom, and Q is any of the substituents shown in [Table L1] to [Table L13] (hereinafter, referred to as a compound group SX1230).

A compound wherein in the compound (L-2), n is 0, $R^1$ is an ethyl group, $R^{3b}$ is a difluoromethyl group, $R^{3c}$ is an iodine atom, and Q is any of the substituents shown in [Table L1] to [Table L13] (hereinafter, referred to as a compound group SX1231).

A compound wherein in the compound (L-2), n is 1, $R^1$ is an ethyl group, $R^{3b}$ is a difluoromethyl group, $R^{3c}$ is an iodine atom, and Q is any of the substituents shown in [Table L1] to [Table L13] (hereinafter, referred to as a compound group SX1232).

A compound wherein in the compound (L-2), n is 2, $R^1$ is an ethyl group, $R^{3b}$ is a difluoromethyl group, $R^{3c}$ is an iodine atom, and Q is any of the substituents shown in [Table L1] to [Table L13] (hereinafter, referred to as a compound group SX1233).

A compound wherein in the compound (L-2), n is 0, $R^1$ is a benzyl group, $R^{3b}$ is a difluoromethyl group, $R^{3c}$ is an iodine atom, and Q is any of the substituents shown in [Table L1] to [Table L13] (hereinafter, referred to as a compound group SX1234).

A compound wherein in the compound (L-2), n is 1, $R^1$ is a benzyl group, $R^{3b}$ is a difluoromethyl group, $R^{3c}$ is an iodine atom, and Q is any of the substituents shown in [Table L1] to [Table L13] (hereinafter, referred to as a compound group SX1235).

A compound wherein in the compound (L-2), n is 2, $R^1$ is a benzyl group, $R^{3b}$ is a difluoromethyl group, $R^{3c}$ is an iodine atom, and Q is any of the substituents shown in [Table L1] to [Table L13] (hereinafter, referred to as a compound group SX1236).

A compound wherein in the compound (L-2), n is 0, $R^1$ is a methyl group, $R^{3b}$ is a hydrogen atom, $R^{3c}$ is a trifluoromethyl group, and Q is any of the substituents shown in [Table L1] to [Table L13] (hereinafter, referred to as a compound group SX1240).

A compound wherein in the compound (L-2), n is 1, $R^1$ is a methyl group, $R^{3b}$ is a hydrogen atom, $R^{3c}$ is a trifluoromethyl group, and Q is any of the substituents shown in [Table L1] to [Table L13] (hereinafter, referred to as a compound group SX1241).

A compound wherein in the compound (L-2), n is 2, $R^1$ is a methyl group, $R^{3b}$ is a hydrogen atom, $R^{3c}$ is a trifluoromethyl group, and Q is any of the substituents shown in [Table L1] to [Table L13] (hereinafter, referred to as a

compound group SX1242).

A compound wherein in the compound (L-2), n is 0, $R^1$ is an ethyl group, $R^{3b}$ is a hydrogen atom, $R^{3c}$ is a trifluoromethyl group, and Q is any of the substituents shown in [Table L1] to [Table L13] (hereinafter, referred to as a compound group SX1243).

A compound wherein in the compound (L-2), n is 1, $R^1$ is an ethyl group, $R^{3b}$ is a hydrogen atom, $R^{3c}$ is a trifluoromethyl group, and Q is any of the substituents shown in [Table L1] to [Table L13] (hereinafter, referred to as a compound group SX1244).

A compound wherein in the compound (L-2), n is 2, $R^1$ is an ethyl group, $R^{3b}$ is a hydrogen atom, $R^{3c}$ is a trifluoromethyl group, and Q is any of the substituents shown in [Table L1] to [Table L13] (hereinafter, referred to as a compound group SX1245).

A compound wherein in the compound (L-2), n is 0, $R^1$ is a benzyl group, $R^{3b}$ is a hydrogen atom, $R^{3c}$ is a trifluoromethyl group, and Q is any of the substituents shown in [Table L1] to [Table L13] (hereinafter, referred to as a compound group SX1246).

A compound wherein in the compound (L-2), n is 1, $R^1$ is a benzyl group, $R^{3b}$ is a hydrogen atom, $R^{3c}$ is a trifluoromethyl group, and Q is any of the substituents shown in [Table L1] to [Table L13] (hereinafter, referred to as a compound group SX1247).

A compound wherein in the compound (L-2), n is 2, $R^1$ is a benzyl group, $R^{3b}$ is a hydrogen atom, $R^{3c}$ is a trifluoromethyl group, and Q is any of the substituents shown in [Table L1] to [Table L13] (hereinafter, referred to as a compound group SX1248).

A compound wherein in the compound (L-2), n is 0, $R^1$ is a methyl group, $R^{3b}$ is a fluorine atom, $R^{3c}$ is a trifluoromethyl group, and Q is any of the substituents shown in [Table L1] to [Table L13] (hereinafter, referred to as a compound group SX1252).

A compound wherein in the compound (L-2), n is 1, $R^1$ is a methyl group, $R^{3b}$ is a fluorine atom, $R^{3c}$ is a trifluoromethyl group, and Q is any of the substituents shown in [Table L1] to [Table L13] (hereinafter, referred to as a compound group SX1253).

A compound wherein in the compound (L-2), n is 2, $R^1$ is a methyl group, $R^{3b}$ is a fluorine atom, $R^{3c}$ is a trifluoromethyl group, and Q is any of the substituents shown in [Table L1] to [Table L13] (hereinafter, referred to as a compound group SX1254).

A compound wherein in the compound (L-2), n is 0, $R^1$ is an ethyl group, $R^{3b}$ is a fluorine atom, $R^{3c}$ is a trifluoromethyl group, and Q is any of the substituents shown in [Table L1] to [Table L13] (hereinafter, referred to as a compound group SX1255).

A compound wherein in the compound (L-2), n is 1, $R^1$ is an ethyl group, $R^{3b}$ is a fluorine atom, $R^{3c}$ is a trifluoromethyl group, and Q is any of the substituents shown in [Table L1] to [Table L13] (hereinafter, referred to as a compound group SX1256).

A compound wherein in the compound (L-2), n is 2, $R^1$ is an ethyl group, $R^{3b}$ is a fluorine atom, $R^{3c}$ is a trifluoromethyl group, and Q is any of the substituents shown in [Table L1] to [Table L13] (hereinafter, referred to as a compound group SX1257).

A compound wherein in the compound (L-2), n is 0, $R^1$ is a benzyl group, $R^{3b}$ is a fluorine atom, $R^{3c}$ is a trifluoromethyl group, and Q is any of the substituents shown in [Table L1] to [Table L13] (hereinafter, referred to as a compound group SX1258).

A compound wherein in the compound (L-2), n is 1, $R^1$ is a benzyl group, $R^{3b}$ is a fluorine atom, $R^{3c}$ is a trifluoromethyl group, and Q is any of the substituents shown in [Table L1] to [Table L13] (hereinafter, referred to as a compound group SX1259).

A compound wherein in the compound (L-2), n is 2, $R^1$ is a benzyl group, $R^{3b}$ is a fluorine atom, $R^{3c}$ is a trifluoromethyl group, and Q is any of the substituents shown in [Table L1] to [Table L13] (hereinafter, referred to as a compound group SX1260).

A compound wherein in the compound (L-2), n is 0, $R^1$ is a methyl group, $R^{3b}$ is a chlorine atom, $R^{3c}$ is a trifluoromethyl group, and Q is any of the substituents shown in [Table L1] to [Table L13] (hereinafter, referred to as a compound group SX1264).

A compound wherein in the compound (L-2), n is 1, $R^1$ is a methyl group, $R^{3b}$ is a chlorine atom, $R^{3c}$ is a trifluoromethyl group, and Q is any of the substituents shown in [Table L1] to [Table L13] (hereinafter, referred to as a compound group SX1265).

A compound wherein in the compound (L-2), n is 2, $R^1$ is a methyl group, $R^{3b}$ is a chlorine atom, $R^{3c}$ is a trifluoromethyl group, and Q is any of the substituents shown in [Table L1] to [Table L13] (hereinafter, referred to as a compound group SX1266).

A compound wherein in the compound (L-2), n is 0, $R^1$ is an ethyl group, $R^{3b}$ is a chlorine atom, $R^{3c}$ is a trifluoromethyl group, and Q is any of the substituents shown in [Table L1] to [Table L13] (hereinafter, referred to as a compound group SX1267).

A compound wherein in the compound (L-2), n is 1, $R^1$ is an ethyl group, $R^{3b}$ is a chlorine atom, $R^{3c}$ is a trifluoromethyl group, and Q is any of the substituents shown in [Table L1] to [Table L13] (hereinafter, referred to as a compound group SX1268).

A compound wherein in the compound (L-2), n is 2, $R^1$ is an ethyl group, $R^{3b}$ is a chlorine atom, $R^{3c}$ is a trifluoromethyl group, and Q is any of the substituents shown in [Table L1] to [Table L13] (hereinafter, referred to as a compound group SX1269).

A compound wherein in the compound (L-2), n is 0, $R^1$ is a benzyl group, $R^{3b}$ is a chlorine atom, $R^{3c}$ is a trifluoromethyl group, and Q is any of the substituents shown in [Table L1] to [Table L13] (hereinafter, referred to as a compound group SX1270).

A compound wherein in the compound (L-2), n is 1, $R^1$ is a benzyl group, $R^{3b}$ is a chlorine atom, $R^{3c}$ is a trifluoromethyl group, and Q is any of the substituents shown in [Table L1] to [Table L13] (hereinafter, referred to as a compound group SX1271).

A compound wherein in the compound (L-2), n is 2, $R^1$ is a benzyl group, $R^{3b}$ is a chlorine atom, $R^{3c}$ is a trifluoromethyl group, and Q is any of the substituents shown in [Table L1] to [Table L13] (hereinafter, referred to as a compound group SX1272).

A compound wherein in the compound (L-2), n is 0, $R^1$ is a methyl group, $R^{3b}$ is a bromine atom, $R^{3c}$ is a trifluoromethyl group, and Q is any of the substituents shown in [Table L1] to [Table L13] (hereinafter, referred to as a compound group SX1276).

A compound wherein in the compound (L-2), n is 1, $R^1$ is a methyl group, $R^{3b}$ is a bromine atom, $R^{3c}$ is a trifluoromethyl group, and Q is any of the substituents shown in [Table L1] to [Table L13] (hereinafter, referred to as a compound group SX1277).

A compound wherein in the compound (L-2), n is 2, $R^1$ is a methyl group, $R^{3b}$ is a bromine atom, $R^{3c}$ is a trifluoromethyl group, and Q is any of the substituents shown in [Table L1] to [Table L13] (hereinafter, referred to as a compound group SX1278).

A compound wherein in the compound (L-2), n is 0, $R^1$ is an ethyl group, $R^{3b}$ is a bromine atom, $R^{3c}$ is a trifluoromethyl group, and Q is any of the substituents shown in [Table L1] to [Table L13] (hereinafter, referred to as a compound group SX1279).

A compound wherein in the compound (L-2), n is 1, $R^1$ is an ethyl group, $R^{3b}$ is a bromine atom, $R^{3c}$ is a trifluoromethyl group, and Q is any of the substituents shown in [Table L1] to [Table L13] (hereinafter, referred to as a compound group SX1280).

A compound wherein in the compound (L-2), n is 2, $R^1$ is an ethyl group, $R^{3b}$ is a bromine atom, $R^{3c}$ is a trifluoromethyl group, and Q is any of the substituents shown in [Table L1] to [Table L13] (hereinafter, referred to as a compound group SX1281).

A compound wherein in the compound (L-2), n is 0, $R^1$ is a benzyl group, $R^{3b}$ is a bromine atom, $R^{3c}$ is a trifluoromethyl group, and Q is any of the substituents shown in [Table L1] to [Table L13] (hereinafter, referred to as a compound group SX1282).

A compound wherein in the compound (L-2), n is 1, $R^1$ is a benzyl group, $R^{3b}$ is a bromine atom, $R^{3c}$ is a trifluoromethyl group, and Q is any of the substituents shown in [Table L1] to [Table L13] (hereinafter, referred to as a compound group SX1283).

A compound wherein in the compound (L-2), n is 2, $R^1$ is a benzyl group, $R^{3b}$ is a bromine atom, $R^{3c}$ is a trifluoromethyl group, and Q is any of the substituents shown in [Table L1] to [Table L13] (hereinafter, referred to as a compound group SX1284).

A compound wherein in the compound (L-2), n is 0, $R^1$ is a methyl group, $R^{3b}$ is an iodine atom, $R^{3c}$ is a trifluoromethyl group, and Q is any of the substituents shown in [Table L1] to [Table L13] (hereinafter, referred to as a compound group SX1288).

A compound wherein in the compound (L-2), n is 1, $R^1$ is a methyl group, $R^{3b}$ is an iodine atom, $R^{3c}$ is a trifluoromethyl group, and Q is any of the substituents shown in [Table L1] to [Table L13] (hereinafter, referred to as a compound group SX1289).

A compound wherein in the compound (L-2), n is 2, $R^1$ is a methyl group, $R^{3b}$ is an iodine atom, $R^{3c}$ is a trifluoromethyl group, and Q is any of the substituents shown in [Table L1] to [Table L13] (hereinafter, referred to as a compound group SX1290).

A compound wherein in the compound (L-2), n is 0, $R^1$ is an ethyl group, $R^{3b}$ is an iodine atom, $R^{3c}$ is a trifluoromethyl group, and Q is any of the substituents shown in [Table L1] to [Table L13] (hereinafter, referred to as a compound group SX1291).

A compound wherein in the compound (L-2), n is 1, $R^1$ is an ethyl group, $R^{3b}$ is an iodine atom, $R^{3c}$ is a trifluoromethyl group, and Q is any of the substituents shown in [Table L1] to [Table L13] (hereinafter, referred to as a compound group SX1292).

A compound wherein in the compound (L-2), n is 2, $R^1$ is an ethyl group, $R^{3b}$ is an iodine atom, $R^{3c}$ is a

trifluoromethyl group, and Q is any of the substituents shown in [Table L1] to [Table L13] (hereinafter, referred to as a compound group SX1293).

A compound wherein in the compound (L-2), n is 0, $R^1$ is a benzyl group, $R^{3b}$ is an iodine atom, $R^{3c}$ is a trifluoromethyl group, and Q is any of the substituents shown in [Table L1] to [Table L13] (hereinafter, referred to as a compound group SX1294).

A compound wherein in the compound (L-2), n is 1, $R^1$ is a benzyl group, $R^{3b}$ is an iodine atom, $R^{3c}$ is a trifluoromethyl group, and Q is any of the substituents shown in [Table L1] to [Table L13] (hereinafter, referred to as a compound group SX1295).

A compound wherein in the compound (L-2), n is 2, $R^1$ is a benzyl group, $R^{3b}$ is an iodine atom, $R^{3c}$ is a trifluoromethyl group, and Q is any of the substituents shown in [Table L1] to [Table L13] (hereinafter, referred to as a compound group SX1296).

A compound wherein in the compound (L-2), n is 0, $R^1$ is a methyl group, $R^{3b}$ is a trifluoromethyl group, $R^{3c}$ is a trifluoromethyl group, and Q is any of the substituents shown in [Table L1] to [Table L13] (hereinafter, referred to as a compound group SX1300).

A compound wherein in the compound (L-2), n is 1, $R^1$ is a methyl group, $R^{3b}$ is a trifluoromethyl group, $R^{3c}$ is a trifluoromethyl group, and Q is any of the substituents shown in [Table L1] to [Table L13] (hereinafter, referred to as a compound group SX1301).

A compound wherein in the compound (L-2), n is 2, $R^1$ is a methyl group, $R^{3b}$ is a trifluoromethyl group, $R^{3c}$ is a trifluoromethyl group, and Q is any of the substituents shown in [Table L1] to [Table L13] (hereinafter, referred to as a compound group SX1302).

A compound wherein in the compound (L-2), n is 0, $R^1$ is an ethyl group, $R^{3b}$ is a trifluoromethyl group, $R^{3c}$ is a trifluoromethyl group, and Q is any of the substituents shown in [Table L1] to [Table L13] (hereinafter, referred to as a compound group SX1303).

A compound wherein in the compound (L-2), n is 1, $R^1$ is an ethyl group, $R^{3b}$ is a trifluoromethyl group, $R^{3c}$ is a trifluoromethyl group, and Q is any of the substituents shown in [Table L1] to [Table L13] (hereinafter, referred to as a compound group SX1304).

A compound wherein in the compound (L-2), n is 2, $R^1$ is an ethyl group, $R^{3b}$ is a trifluoromethyl group, $R^{3c}$ is a trifluoromethyl group, and Q is any of the substituents shown in [Table L1] to [Table L13] (hereinafter, referred to as a compound group SX1305).

A compound wherein in the compound (L-2), n is 0, $R^1$ is a benzyl group, $R^{3b}$ is a trifluoromethyl group, $R^{3c}$ is a trifluoromethyl group, and Q is any of the substituents shown in [Table L1] to [Table L13] (hereinafter, referred to as a compound group SX1306).

A compound wherein in the compound (L-2), n is 1, $R^1$ is a benzyl group, $R^{3b}$ is a trifluoromethyl group, $R^{3c}$ is a trifluoromethyl group, and Q is any of the substituents shown in [Table L1] to [Table L13] (hereinafter, referred to as a compound group SX1307).

A compound wherein in the compound (L-2), n is 2, $R^1$ is a benzyl group, $R^{3b}$ is a trifluoromethyl group, $R^{3c}$ is a trifluoromethyl group, and Q is any of the substituents shown in [Table L1] to [Table L13] (hereinafter, referred to as a compound group SX1308).

A compound wherein in the compound (L-2), n is 0, $R^1$ is a methyl group, $R^{3b}$ is a difluoromethyl group, $R^{3c}$ is a trifluoromethyl group, and Q is any of the substituents shown in [Table L1] to [Table L13] (hereinafter, referred to as a compound group SX1312).

A compound wherein in the compound (L-2), n is 1, $R^1$ is a methyl group, $R^{3b}$ is a difluoromethyl group, $R^{3c}$ is a trifluoromethyl group, and Q is any of the substituents shown in [Table L1] to [Table L13] (hereinafter, referred to as a compound group SX1313).

A compound wherein in the compound (L-2), n is 2, $R^1$ is a methyl group, $R^{3b}$ is a difluoromethyl group, $R^{3c}$ is a trifluoromethyl group, and Q is any of the substituents shown in [Table L1] to [Table L13] (hereinafter, referred to as a compound group SX1314).

A compound wherein in the compound (L-2), n is 0, $R^1$ is an ethyl group, $R^{3b}$ is a difluoromethyl group, $R^{3c}$ is a trifluoromethyl group, and Q is any of the substituents shown in [Table L1] to [Table L13] (hereinafter, referred to as a compound group SX1315).

A compound wherein in the compound (L-2), n is 1, $R^1$ is an ethyl group, $R^{3b}$ is a difluoromethyl group, $R^{3c}$ is a trifluoromethyl group, and Q is any of the substituents shown in [Table L1] to [Table L13] (hereinafter, referred to as a compound group SX1316).

A compound wherein in the compound (L-2), n is 2, $R^1$ is an ethyl group, $R^{3b}$ is a difluoromethyl group, $R^{3c}$ is a trifluoromethyl group, and Q is any of the substituents shown in [Table L1] to [Table L13] (hereinafter, referred to as a compound group SX1317).

A compound wherein in the compound (L-2), n is 0, $R^1$ is a benzyl group, $R^{3b}$ is a difluoromethyl group, $R^{3c}$ is a trifluoromethyl group, and Q is any of the substituents shown in [Table L1] to [Table L13] (hereinafter, referred to as a

compound group SX1318).

A compound wherein in the compound (L-2), n is 1, $R^1$ is a benzyl group, $R^{3b}$ is a difluoromethyl group, $R^{3c}$ is a trifluoromethyl group, and Q is any of the substituents shown in [Table L1] to [Table L13] (hereinafter, referred to as a compound group SX1319).

A compound wherein in the compound (L-2), n is 2, $R^1$ is a benzyl group, $R^{3b}$ is a difluoromethyl group, $R^{3c}$ is a trifluoromethyl group, and Q is any of the substituents shown in [Table L1] to [Table L13] (hereinafter, referred to as a compound group SX1320).

A compound wherein in the compound (L-2), n is 0, $R^1$ is a methyl group, $R^{3b}$ is a hydrogen atom, $R^{3c}$ is a difluoromethyl group, and Q is any of the substituents shown in [Table L1] to [Table L13] (hereinafter, referred to as a compound group SX1324).

A compound wherein in the compound (L-2), n is 1, $R^1$ is a methyl group, $R^{3b}$ is a hydrogen atom, $R^{3c}$ is a difluoromethyl group, and Q is any of the substituents shown in [Table L1] to [Table L13] (hereinafter, referred to as a compound group SX1325).

A compound wherein in the compound (L-2), n is 2, $R^1$ is a methyl group, $R^{3b}$ is a hydrogen atom, $R^{3c}$ is a difluoromethyl group, and Q is any of the substituents shown in [Table L1] to [Table L13] (hereinafter, referred to as a compound group SX1326).

A compound wherein in the compound (L-2), n is 0, $R^1$ is an ethyl group, $R^{3b}$ is a hydrogen atom, $R^{3c}$ is a difluoromethyl group, and Q is any of the substituents shown in [Table L1] to [Table L13] (hereinafter, referred to as a compound group SX1327).

A compound wherein in the compound (L-2), n is 1, $R^1$ is an ethyl group, $R^{3b}$ is a hydrogen atom, $R^{3c}$ is a difluoromethyl group, and Q is any of the substituents shown in [Table L1] to [Table L13] (hereinafter, referred to as a compound group SX1328).

A compound wherein in the compound (L-2), n is 2, $R^1$ is an ethyl group, $R^{3b}$ is a hydrogen atom, $R^{3c}$ is a difluoromethyl group, and Q is any of the substituents shown in [Table L1] to [Table L13] (hereinafter, referred to as a compound group SX1329).

A compound wherein in the compound (L-2), n is 0, $R^1$ is a benzyl group, $R^{3b}$ is a hydrogen atom, $R^{3c}$ is a difluoromethyl group, and Q is any of the substituents shown in [Table L1] to [Table L13] (hereinafter, referred to as a compound group SX1330).

A compound wherein in the compound (L-2), n is 1, $R^1$ is a benzyl group, $R^{3b}$ is a hydrogen atom, $R^{3c}$ is a difluoromethyl group, and Q is any of the substituents shown in [Table L1] to [Table L13] (hereinafter, referred to as a compound group SX1331).

A compound wherein in the compound (L-2), n is 2, $R^1$ is a benzyl group, $R^{3b}$ is a hydrogen atom, $R^{3c}$ is a difluoromethyl group, and Q is any of the substituents shown in [Table L1] to [Table L13] (hereinafter, referred to as a compound group SX1332).

A compound wherein in the compound (L-2), n is 0, $R^1$ is a methyl group, $R^{3b}$ is a fluorine atom, $R^{3c}$ is a difluoromethyl group, and Q is any of the substituents shown in [Table L1] to [Table L13] (hereinafter, referred to as a compound group SX1336).

A compound wherein in the compound (L-2), n is 1, $R^1$ is a methyl group, $R^{3b}$ is a fluorine atom, $R^{3c}$ is a difluoromethyl group, and Q is any of the substituents shown in [Table L1] to [Table L13] (hereinafter, referred to as a compound group SX1337).

A compound wherein in the compound (L-2), n is 2, $R^1$ is a methyl group, $R^{3b}$ is a fluorine atom, $R^{3c}$ is a difluoromethyl group, and Q is any of the substituents shown in [Table L1] to [Table L13] (hereinafter, referred to as a compound group SX1338).

A compound wherein in the compound (L-2), n is 0, $R^1$ is an ethyl group, $R^{3b}$ is a fluorine atom, $R^{3c}$ is a difluoromethyl group, and Q is any of the substituents shown in [Table L1] to [Table L13] (hereinafter, referred to as a compound group SX1339).

A compound wherein in the compound (L-2), n is 1, $R^1$ is an ethyl group, $R^{3b}$ is a fluorine atom, $R^{3c}$ is a difluoromethyl group, and Q is any of the substituents shown in [Table L1] to [Table L13] (hereinafter, referred to as a compound group SX1340).

A compound wherein in the compound (L-2), n is 2, $R^1$ is an ethyl group, $R^{3b}$ is a fluorine atom, $R^{3c}$ is a difluoromethyl group, and Q is any of the substituents shown in [Table L1] to [Table L13] (hereinafter, referred to as a compound group SX1341).

A compound wherein in the compound (L-2), n is 0, $R^1$ is a benzyl group, $R^{3b}$ is a fluorine atom, $R^{3c}$ is a difluoromethyl group, and Q is any of the substituents shown in [Table L1] to [Table L13] (hereinafter, referred to as a compound group SX1342).

A compound wherein in the compound (L-2), n is 1, $R^1$ is a benzyl group, $R^{3b}$ is a fluorine atom, $R^{3c}$ is a difluoromethyl group, and Q is any of the substituents shown in [Table L1] to [Table L13] (hereinafter, referred to as a compound group SX1343).

A compound wherein in the compound (L-2), n is 2, $R^1$ is a benzyl group, $R^{3b}$ is a fluorine atom, $R^{3c}$ is a difluoromethyl group, and Q is any of the substituents shown in [Table L1] to [Table L13] (hereinafter, referred to as a compound group SX1344).

A compound wherein in the compound (L-2), n is 0, $R^1$ is a methyl group, $R^{3b}$ is a chlorine atom, $R^{3c}$ is a difluoromethyl group, and Q is any of the substituents shown in [Table L1] to [Table L13] (hereinafter, referred to as a compound group SX1348).

A compound wherein in the compound (L-2), n is 1, $R^1$ is a methyl group, $R^{3b}$ is a chlorine atom, $R^{3c}$ is a difluoromethyl group, and Q is any of the substituents shown in [Table L1] to [Table L13] (hereinafter, referred to as a compound group SX1349).

A compound wherein in the compound (L-2), n is 2, $R^1$ is a methyl group, $R^{3b}$ is a chlorine atom, $R^{3c}$ is a difluoromethyl group, and Q is any of the substituents shown in [Table L1] to [Table L13] (hereinafter, referred to as a compound group SX1350).

A compound wherein in the compound (L-2), n is 0, $R^1$ is an ethyl group, $R^{3b}$ is a chlorine atom, $R^{3c}$ is a difluoromethyl group, and Q is any of the substituents shown in [Table L1] to [Table L13] (hereinafter, referred to as a compound group SX1351).

A compound wherein in the compound (L-2), n is 1, $R^1$ is an ethyl group, $R^{3b}$ is a chlorine atom, $R^{3c}$ is a difluoromethyl group, and Q is any of the substituents shown in [Table L1] to [Table L13] (hereinafter, referred to as a compound group SX1352).

A compound wherein in the compound (L-2), n is 2, $R^1$ is an ethyl group, $R^{3b}$ is a chlorine atom, $R^{3c}$ is a difluoromethyl group, and Q is any of the substituents shown in [Table L1] to [Table L13] (hereinafter, referred to as a compound group SX1353).

A compound wherein in the compound (L-2), n is 0, $R^1$ is a benzyl group, $R^{3b}$ is a chlorine atom, $R^{3c}$ is a difluoromethyl group, and Q is any of the substituents shown in [Table L1] to [Table L13] (hereinafter, referred to as a compound group SX1354).

A compound wherein in the compound (L-2), n is 1, $R^1$ is a benzyl group, $R^{3b}$ is a chlorine atom, $R^{3c}$ is a difluoromethyl group, and Q is any of the substituents shown in [Table L1] to [Table L13] (hereinafter, referred to as a compound group SX1355).

A compound wherein in the compound (L-2), n is 2, $R^1$ is a benzyl group, $R^{3b}$ is a chlorine atom, $R^{3c}$ is a difluoromethyl group, and Q is any of the substituents shown in [Table L1] to [Table L13] (hereinafter, referred to as a compound group SX1356).

A compound wherein in the compound (L-2), n is 0, $R^1$ is a methyl group, $R^{3b}$ is a bromine atom, $R^{3c}$ is a difluoromethyl group, and Q is any of the substituents shown in [Table L1] to [Table L13] (hereinafter, referred to as a compound group SX1360).

A compound wherein in the compound (L-2), n is 1, $R^1$ is a methyl group, $R^{3b}$ is a bromine atom, $R^{3c}$ is a difluoromethyl group, and Q is any of the substituents shown in [Table L1] to [Table L13] (hereinafter, referred to as a compound group SX1361).

A compound wherein in the compound (L-2), n is 2, $R^1$ is a methyl group, $R^{3b}$ is a bromine atom, $R^{3c}$ is a difluoromethyl group, and Q is any of the substituents shown in [Table L1] to [Table L13] (hereinafter, referred to as a compound group SX1362).

A compound wherein in the compound (L-2), n is 0, $R^1$ is an ethyl group, $R^{3b}$ is a bromine atom, $R^{3c}$ is a difluoromethyl group, and Q is any of the substituents shown in [Table L1] to [Table L13] (hereinafter, referred to as a compound group SX1363).

A compound wherein in the compound (L-2), n is 1, $R^1$ is an ethyl group, $R^{3b}$ is a bromine atom, $R^{3c}$ is a difluoromethyl group, and Q is any of the substituents shown in [Table L1] to [Table L13] (hereinafter, referred to as a compound group SX1364).

A compound wherein in the compound (L-2), n is 2, $R^1$ is an ethyl group, $R^{3b}$ is a bromine atom, $R^{3c}$ is a difluoromethyl group, and Q is any of the substituents shown in [Table L1] to [Table L13] (hereinafter, referred to as a compound group SX1365).

A compound wherein in the compound (L-2), n is 0, $R^1$ is a benzyl group, $R^{3b}$ is a bromine atom, $R^{3c}$ is a difluoromethyl group, and Q is any of the substituents shown in [Table L1] to [Table L13] (hereinafter, referred to as a compound group SX1366).

A compound wherein in the compound (L-2), n is 1, $R^1$ is a benzyl group, $R^{3b}$ is a bromine atom, $R^{3c}$ is a difluoromethyl group, and Q is any of the substituents shown in [Table L1] to [Table L13] (hereinafter, referred to as a compound group SX1367).

A compound wherein in the compound (L-2), n is 2, $R^1$ is a benzyl group, $R^{3b}$ is a bromine atom, $R^{3c}$ is a difluoromethyl group, and Q is any of the substituents shown in [Table L1] to [Table L13] (hereinafter, referred to as a compound group SX1368).

A compound wherein in the compound (L-2), n is 0, $R^1$ is a methyl group, $R^{3b}$ is an iodine atom, $R^{3c}$ is a

difluoromethyl group, and Q is any of the substituents shown in [Table L1] to [Table L13] (hereinafter, referred to as a compound group SX1372).

A compound wherein in the compound (L-2), n is 1, $R^1$ is a methyl group, $R^{3b}$ is an iodine atom, $R^{3c}$ is a difluoromethyl group, and Q is any of the substituents shown in [Table L1] to [Table L13] (hereinafter, referred to as a compound group SX1373).

A compound wherein in the compound (L-2), n is 2, $R^1$ is a methyl group, $R^{3b}$ is an iodine atom, $R^{3c}$ is a difluoromethyl group, and Q is any of the substituents shown in [Table L1] to [Table L13] (hereinafter, referred to as a compound group SX1374).

A compound wherein in the compound (L-2), n is 0, $R^1$ is an ethyl group, $R^{3b}$ is an iodine atom, $R^{3c}$ is a difluoromethyl group, and Q is any of the substituents shown in [Table L1] to [Table L13] (hereinafter, referred to as a compound group SX1375).

A compound wherein in the compound (L-2), n is 1, $R^1$ is an ethyl group, $R^{3b}$ is an iodine atom, $R^{3c}$ is a difluoromethyl group, and Q is any of the substituents shown in [Table L1] to [Table L13] (hereinafter, referred to as a compound group SX1376).

A compound wherein in the compound (L-2), n is 2, $R^1$ is an ethyl group, $R^{3b}$ is an iodine atom, $R^{3c}$ is a difluoromethyl group, and Q is any of the substituents shown in [Table L1] to [Table L13] (hereinafter, referred to as a compound group SX1377).

A compound wherein in the compound (L-2), n is 0, $R^1$ is a benzyl group, $R^{3b}$ is an iodine atom, $R^{3c}$ is a difluoromethyl group, and Q is any of the substituents shown in [Table L1] to [Table L13] (hereinafter, referred to as a compound group SX1378).

A compound wherein in the compound (L-2), n is 1, $R^1$ is a benzyl group, $R^{3b}$ is an iodine atom, $R^{3c}$ is a difluoromethyl group, and Q is any of the substituents shown in [Table L1] to [Table L13] (hereinafter, referred to as a compound group SX1379).

A compound wherein in the compound (L-2), n is 2, $R^1$ is a benzyl group, $R^{3b}$ is an iodine atom, $R^{3c}$ is a difluoromethyl group, and Q is any of the substituents shown in [Table L1] to [Table L13] (hereinafter, referred to as a compound group SX1380).

A compound wherein in the compound (L-2), n is 0, $R^1$ is a methyl group, $R^{3b}$ is a trifluoromethyl group, $R^{3c}$ is a difluoromethyl group, and Q is any of the substituents shown in [Table L1] to [Table L13] (hereinafter, referred to as a compound group SX1384).

A compound wherein in the compound (L-2), n is 1, $R^1$ is a methyl group, $R^{3b}$ is a trifluoromethyl group, $R^{3c}$ is a difluoromethyl group, and Q is any of the substituents shown in [Table L1] to [Table L13] (hereinafter, referred to as a compound group SX1385).

A compound wherein in the compound (L-2), n is 2, $R^1$ is a methyl group, $R^{3b}$ is a trifluoromethyl group, $R^{3c}$ is a difluoromethyl group, and Q is any of the substituents shown in [Table L1] to [Table L13] (hereinafter, referred to as a compound group SX1386).

A compound wherein in the compound (L-2), n is 0, $R^1$ is an ethyl group, $R^{3b}$ is a trifluoromethyl group, $R^{3c}$ is a difluoromethyl group, and Q is any of the substituents shown in [Table L1] to [Table L13] (hereinafter, referred to as a compound group SX1387).

A compound wherein in the compound (L-2), n is 1, $R^1$ is an ethyl group, $R^{3b}$ is a trifluoromethyl group, $R^{3c}$ is a difluoromethyl group, and Q is any of the substituents shown in [Table L1] to [Table L13] (hereinafter, referred to as a compound group SX1388).

A compound wherein in the compound (L-2), n is 2, $R^1$ is an ethyl group, $R^{3b}$ is a trifluoromethyl group, $R^{3c}$ is a difluoromethyl group, and Q is any of the substituents shown in [Table L1] to [Table L13] (hereinafter, referred to as a compound group SX1389).

A compound wherein in the compound (L-2), n is 0, $R^1$ is a benzyl group, $R^{3b}$ is a trifluoromethyl group, $R^{3c}$ is a difluoromethyl group, and Q is any of the substituents shown in [Table L1] to [Table L13] (hereinafter, referred to as a compound group SX1390).

A compound wherein in the compound (L-2), n is 1, $R^1$ is a benzyl group, $R^{3b}$ is a trifluoromethyl group, $R^{3c}$ is a difluoromethyl group, and Q is any of the substituents shown in [Table L1] to [Table L13] (hereinafter, referred to as a compound group SX1391).

A compound wherein in the compound (L-2), n is 2, $R^1$ is a benzyl group, $R^{3b}$ is a trifluoromethyl group, $R^{3c}$ is a difluoromethyl group, and Q is any of the substituents shown in [Table L1] to [Table L13] (hereinafter, referred to as a compound group SX1392).

A compound wherein in the compound (L-2), n is 0, $R^1$ is a methyl group, $R^{3b}$ is a difluoromethyl group, $R^{3c}$ is a difluoromethyl group, and Q is any of the substituents shown in [Table L1] to [Table L13] (hereinafter, referred to as a compound group SX1396).

A compound wherein in the compound (L-2), n is 1, $R^1$ is a methyl group, $R^{3b}$ is a difluoromethyl group, $R^{3c}$ is a difluoromethyl group, and Q is any of the substituents shown in [Table L1] to [Table L13] (hereinafter, referred to as a

compound group SX1397).

A compound wherein in the compound (L-2), n is 2, $R^1$ is a methyl group, $R^{3b}$ is a difluoromethyl group, $R^{3c}$ is a difluoromethyl group, and Q is any of the substituents shown in [Table L1] to [Table L13] (hereinafter, referred to as a compound group SX1398).

A compound wherein in the compound (L-2), n is 0, $R^1$ is an ethyl group, $R^{3b}$ is a difluoromethyl group, $R^{3c}$ is a difluoromethyl group, and Q is any of the substituents shown in [Table L1] to [Table L13] (hereinafter, referred to as a compound group SX1399).

A compound wherein in the compound (L-2), n is 1, $R^1$ is an ethyl group, $R^{3b}$ is a difluoromethyl group, $R^{3c}$ is a difluoromethyl group, and Q is any of the substituents shown in [Table L1] to [Table L13] (hereinafter, referred to as a compound group SX1400).

A compound wherein in the compound (L-2), n is 2, $R^1$ is an ethyl group, $R^{3b}$ is a difluoromethyl group, $R^{3c}$ is a difluoromethyl group, and Q is any of the substituents shown in [Table L1] to [Table L13] (hereinafter, referred to as a compound group SX1401).

A compound wherein in the compound (L-2), n is 0, $R^1$ is a benzyl group, $R^{3b}$ is a difluoromethyl group, $R^{3c}$ is a difluoromethyl group, and Q is any of the substituents shown in [Table L1] to [Table L13] (hereinafter, referred to as a compound group SX1402).

A compound wherein in the compound (L-2), n is 1, $R^1$ is a benzyl group, $R^{3b}$ is a difluoromethyl group, $R^{3c}$ is a difluoromethyl group, and Q is any of the substituents shown in [Table L1] to [Table L13] (hereinafter, referred to as a compound group SX1403).

A compound wherein in the compound (L-2), n is 2, $R^1$ is a benzyl group, $R^{3b}$ is a difluoromethyl group, $R^{3c}$ is a difluoromethyl group, and Q is any of the substituents shown in [Table L1] to [Table L13] (hereinafter, referred to as a compound group SX1404).

A compound represented by a formula (L-3) (hereinafter, referred to as a compound (L-3))

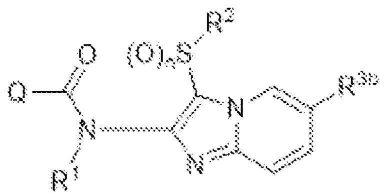 (L-3)

wherein n is 0, $R^1$ is a methyl group, $R^2$ is a methyl group, $R^{3b}$ is a hydrogen atom, and Q is any of the substituents shown in [Table L1] to [Table L13] (hereinafter, referred to as a compound group SX1408).

A compound wherein in the compound (L-3), n is 1, $R^1$ is a methyl group, $R^2$ is a methyl group, $R^{3b}$ is a hydrogen atom, and Q is any of the substituents shown in [Table L1] to [Table L13] (hereinafter, referred to as a compound group SX1409).

A compound wherein in the compound (L-3), n is 2, $R^1$ is a methyl group, $R^2$ is a methyl group, $R^{3b}$ is a hydrogen atom, and Q is any of the substituents shown in [Table L1] to [Table L13] (hereinafter, referred to as a compound group SX1410).

A compound wherein in the compound (L-3), n is 0, $R^1$ is an ethyl group, $R^2$ is a methyl group, $R^{3b}$ is a hydrogen atom, and Q is any of the substituents shown in [Table L1] to [Table L13] (hereinafter, referred to as a compound group SX1411).

A compound wherein in the compound (L-3), n is 1, $R^1$ is an ethyl group, $R^2$ is a methyl group, $R^{3b}$ is a hydrogen atom, and Q is any of the substituents shown in [Table L1] to [Table L13] (hereinafter, referred to as a compound group SX1412).

A compound wherein in the compound (L-3), n is 2, $R^1$ is an ethyl group, $R^2$ is a methyl group, $R^{3b}$ is a hydrogen atom, and Q is any of the substituents shown in [Table L1] to [Table L13] (hereinafter, referred to as a compound group SX1413).

A compound wherein in the compound (L-3), n is 0, $R^1$ is a benzyl group, $R^2$ is a methyl group, $R^{3b}$ is a hydrogen atom, and Q is any of the substituents shown in [Table L1] to [Table L13] (hereinafter, referred to as a compound group SX1414).

A compound wherein in the compound (L-3), n is 1, $R^1$ is a benzyl group, $R^2$ is a methyl group, $R^{3b}$ is a hydrogen atom, and Q is any of the substituents shown in [Table L1] to [Table L13] (hereinafter, referred to as a compound group SX1415).

A compound wherein in the compound (L-3), n is 2, $R^1$ is a benzyl group, $R^2$ is a methyl group, $R^{3b}$ is a hydrogen atom, and Q is any of the substituents shown in [Table L1] to [Table L13] (hereinafter, referred to as a compound group SX1416).

A compound wherein in the compound (L-3), n is 0, $R^1$ is a methyl group, $R^2$ is a methyl group, $R^{3b}$ is a fluorine

atom, and Q is any of the substituents shown in [Table L1] to [Table L13] (hereinafter, referred to as a compound group SX1420).

A compound wherein in the compound (L-3), n is 1, $R^1$ is a methyl group, $R^2$ is a methyl group, $R^{3b}$ is a fluorine atom, and Q is any of the substituents shown in [Table L1] to [Table L13] (hereinafter, referred to as a compound group SX1421).

A compound wherein in the compound (L-3), n is 2, $R^1$ is a methyl group, $R^2$ is a methyl group, $R^{3b}$ is a fluorine atom, and Q is any of the substituents shown in [Table L1] to [Table L13] (hereinafter, referred to as a compound group SX1422).

A compound wherein in the compound (L-3), n is 0, $R^1$ is an ethyl group, $R^2$ is a methyl group, $R^{3b}$ is a fluorine atom, and Q is any of the substituents shown in [Table L1] to [Table L13] (hereinafter, referred to as a compound group SX1423).

A compound wherein in the compound (L-3), n is 1, $R^1$ is an ethyl group, $R^2$ is a methyl group, $R^{3b}$ is a fluorine atom, and Q is any of the substituents shown in [Table L1] to [Table L13] (hereinafter, referred to as a compound group SX1424).

A compound wherein in the compound (L-3), n is 2, $R^1$ is an ethyl group, $R^2$ is a methyl group, $R^{3b}$ is a fluorine atom, and Q is any of the substituents shown in [Table L1] to [Table L13] (hereinafter, referred to as a compound group SX1425).

A compound wherein in the compound (L-3), n is 0, $R^1$ is a benzyl group, $R^2$ is a methyl group, $R^{3b}$ is a fluorine atom, and Q is any of the substituents shown in [Table L1] to [Table L13] (hereinafter, referred to as a compound group SX1426).

A compound wherein in the compound (L-3), n is 1, $R^1$ is a benzyl group, $R^2$ is a methyl group, $R^{3b}$ is a fluorine atom, and Q is any of the substituents shown in [Table L1] to [Table L13] (hereinafter, referred to as a compound group SX1427).

A compound wherein in the compound (L-3), n is 2, $R^1$ is a benzyl group, $R^2$ is a methyl group, $R^{3b}$ is a fluorine atom, and Q is any of the substituents shown in [Table L1] to [Table L13] (hereinafter, referred to as a compound group SX1428).

A compound wherein in the compound (L-3), n is 0, $R^1$ is a methyl group, $R^2$ is a methyl group, $R^{3b}$ is a chlorine atom, and Q is any of the substituents shown in [Table L1] to [Table L13] (hereinafter, referred to as a compound group SX1432).

A compound wherein in the compound (L-3), n is 1, $R^1$ is a methyl group, $R^2$ is a methyl group, $R^{3b}$ is a chlorine atom, and Q is any of the substituents shown in [Table L1] to [Table L13] (hereinafter, referred to as a compound group SX1433).

A compound wherein in the compound (L-3), n is 2, $R^1$ is a methyl group, $R^2$ is a methyl group, $R^{3b}$ is a chlorine atom, and Q is any of the substituents shown in [Table L1] to [Table L13] (hereinafter, referred to as a compound group SX1434).

A compound wherein in the compound (L-3), n is 0, $R^1$ is an ethyl group, $R^2$ is a methyl group, $R^{3b}$ is a chlorine atom, and Q is any of the substituents shown in [Table L1] to [Table L13] (hereinafter, referred to as a compound group SX1435).

A compound wherein in the compound (L-3), n is 1, $R^1$ is an ethyl group, $R^2$ is a methyl group, $R^{3b}$ is a chlorine atom, and Q is any of the substituents shown in [Table L1] to [Table L13] (hereinafter, referred to as a compound group SX1436).

A compound wherein in the compound (L-3), n is 2, $R^1$ is an ethyl group, $R^2$ is a methyl group, $R^{3b}$ is a chlorine atom, and Q is any of the substituents shown in [Table L1] to [Table L13] (hereinafter, referred to as a compound group SX1437).

A compound wherein in the compound (L-3), n is 0, $R^1$ is a benzyl group, $R^2$ is a methyl group, $R^{3b}$ is a chlorine atom, and Q is any of the substituents shown in [Table L1] to [Table L13] (hereinafter, referred to as a compound group SX1438).

A compound wherein in the compound (L-3), n is 1, $R^1$ is a benzyl group, $R^2$ is a methyl group, $R^{3b}$ is a chlorine atom, and Q is any of the substituents shown in [Table L1] to [Table L13] (hereinafter, referred to as a compound group SX1439).

A compound wherein in the compound (L-3), n is 2, $R^1$ is a benzyl group, $R^2$ is a methyl group, $R^{3b}$ is a chlorine atom, and Q is any of the substituents shown in [Table L1] to [Table L13] (hereinafter, referred to as a compound group SX1440).

A compound wherein in the compound (L-3), n is 0, $R^1$ is a methyl group, $R^2$ is a methyl group, $R^{3b}$ is a bromine atom, and Q is any of the substituents shown in [Table L1] to [Table L13] (hereinafter, referred to as a compound group SX1444).

A compound wherein in the compound (L-3), n is 1, $R^1$ is a methyl group, $R^2$ is a methyl group, $R^{3b}$ is a bromine atom, and Q is any of the substituents shown in [Table L1] to [Table L13] (hereinafter, referred to as a compound group

SX1445).

A compound wherein in the compound (L-3), n is 2, $R^1$ is a methyl group, $R^2$ is a methyl group, $R^{3b}$ is a bromine atom, and Q is any of the substituents shown in [Table L1] to [Table L13] (hereinafter, referred to as a compound group SX1446).

A compound wherein in the compound (L-3), n is 0, $R^1$ is an ethyl group, $R^2$ is a methyl group, $R^{3b}$ is a bromine atom, and Q is any of the substituents shown in [Table L1] to [Table L13] (hereinafter, referred to as a compound group SX1447).

A compound wherein in the compound (L-3), n is 1, $R^1$ is an ethyl group, $R^2$ is a methyl group, $R^{3b}$ is a bromine atom, and Q is any of the substituents shown in [Table L1] to [Table L13] (hereinafter, referred to as a compound group SX1448).

A compound wherein in the compound (L-3), n is 2, $R^1$ is an ethyl group, $R^2$ is a methyl group, $R^{3b}$ is a bromine atom, and Q is any of the substituents shown in [Table L1] to [Table L13] (hereinafter, referred to as a compound group SX1449).

A compound wherein in the compound (L-3), n is 0, $R^1$ is a benzyl group, $R^2$ is a methyl group, $R^{3b}$ is a bromine atom, and Q is any of the substituents shown in [Table L1] to [Table L13] (hereinafter, referred to as a compound group SX1450).

A compound wherein in the compound (L-3), n is 1, $R^1$ is a benzyl group, $R^2$ is a methyl group, $R^{3b}$ is a bromine atom, and Q is any of the substituents shown in [Table L1] to [Table L13] (hereinafter, referred to as a compound group SX1451).

A compound wherein in the compound (L-3), n is 2, $R^1$ is a benzyl group, $R^2$ is a methyl group, $R^{3b}$ is a bromine atom, and Q is any of the substituents shown in [Table L1] to [Table L13] (hereinafter, referred to as a compound group SX1452).

A compound wherein in the compound (L-3), n is 0, $R^1$ is a methyl group, $R^2$ is a methyl group, $R^{3b}$ is an iodine atom, and Q is any of the substituents shown in [Table L1] to [Table L13] (hereinafter, referred to as a compound group SX1456).

A compound wherein in the compound (L-3), n is 1, $R^1$ is a methyl group, $R^2$ is a methyl group, $R^{3b}$ is an iodine atom, and Q is any of the substituents shown in [Table L1] to [Table L13] (hereinafter, referred to as a compound group SX1457).

A compound wherein in the compound (L-3), n is 2, $R^1$ is a methyl group, $R^2$ is a methyl group, $R^{3b}$ is an iodine atom, and Q is any of the substituents shown in [Table L1] to [Table L13] (hereinafter, referred to as a compound group SX1458).

A compound wherein in the compound (L-3), n is 0, $R^1$ is an ethyl group, $R^2$ is a methyl group, $R^{3b}$ is an iodine atom, and Q is any of the substituents shown in [Table L1] to [Table L13] (hereinafter, referred to as a compound group SX1459).

A compound wherein in the compound (L-3), n is 1, $R^1$ is an ethyl group, $R^2$ is a methyl group, $R^{3b}$ is an iodine atom, and Q is any of the substituents shown in [Table L1] to [Table L13] (hereinafter, referred to as a compound group SX1460).

A compound wherein in the compound (L-3), n is 2, $R^1$ is an ethyl group, $R^2$ is a methyl group, $R^{3b}$ is an iodine atom, and Q is any of the substituents shown in [Table L1] to [Table L13] (hereinafter, referred to as a compound group SX1461).

A compound wherein in the compound (L-3), n is 0, $R^1$ is a benzyl group, $R^2$ is a methyl group, $R^{3b}$ is an iodine atom, and Q is any of the substituents shown in [Table L1] to [Table L13] (hereinafter, referred to as a compound group SX1462).

A compound wherein in the compound (L-3), n is 1, $R^1$ is a benzyl group, $R^2$ is a methyl group, $R^{3b}$ is an iodine atom, and Q is any of the substituents shown in [Table L1] to [Table L13] (hereinafter, referred to as a compound group SX1463).

A compound wherein in the compound (L-3), n is 2, $R^1$ is a benzyl group, $R^2$ is a methyl group, $R^{3b}$ is an iodine atom, and Q is any of the substituents shown in [Table L1] to [Table L13] (hereinafter, referred to as a compound group SX1464).

A compound wherein in the compound (L-3), n is 0, $R^1$ is a methyl group, $R^2$ is a methyl group, $R^{3b}$ is a trifluoromethyl group, and Q is any of the substituents shown in [Table L1] to [Table L13] (hereinafter, referred to as a compound group SX1468).

A compound wherein in the compound (L-3), n is 1, $R^1$ is a methyl group, $R^2$ is a methyl group, $R^{3b}$ is a trifluoromethyl group, and Q is any of the substituents shown in [Table L1] to [Table L13] (hereinafter, referred to as a compound group SX1469).

A compound wherein in the compound (L-3), n is 2, $R^1$ is a methyl group, $R^2$ is a methyl group, $R^{3b}$ is a trifluoromethyl group, and Q is any of the substituents shown in [Table L1] to [Table L13] (hereinafter, referred to as a compound group SX1470).

A compound wherein in the compound (L-3), n is 0, $R^1$ is an ethyl group, $R^2$ is a methyl group, $R^{3b}$ is a trifluoromethyl group, and Q is any of the substituents shown in [Table L1] to [Table L13] (hereinafter, referred to as a compound group SX1471).

A compound wherein in the compound (L-3), n is 1, $R^1$ is an ethyl group, $R^2$ is a methyl group, $R^{3b}$ is a trifluoromethyl group, and Q is any of the substituents shown in [Table L1] to [Table L13] (hereinafter, referred to as a compound group SX1472).

A compound wherein in the compound (L-3), n is 2, $R^1$ is an ethyl group, $R^2$ is a methyl group, $R^{3b}$ is a trifluoromethyl group, and Q is any of the substituents shown in [Table L1] to [Table L13] (hereinafter, referred to as a compound group SX1473).

A compound wherein in the compound (L-3), n is 0, $R^1$ is a benzyl group, $R^2$ is a methyl group, $R^{3b}$ is a trifluoromethyl group, and Q is any of the substituents shown in [Table L1] to [Table L13] (hereinafter, referred to as a compound group SX1474).

A compound wherein in the compound (L-3), n is 1, $R^1$ is a benzyl group, $R^2$ is a methyl group, $R^{3b}$ is a trifluoromethyl group, and Q is any of the substituents shown in [Table L1] to [Table L13] (hereinafter, referred to as a compound group SX1475).

A compound wherein in the compound (L-3), n is 2, $R^1$ is a benzyl group, $R^2$ is a methyl group, $R^{3b}$ is a trifluoromethyl group, and Q is any of the substituents shown in [Table L1] to [Table L13] (hereinafter, referred to as a compound group SX1476).

A compound wherein in the compound (L-3), n is 0, $R^1$ is a methyl group, $R^2$ is a methyl group, $R^{3b}$ is a difluoromethyl group, and Q is any of the substituents shown in [Table L1] to [Table L13] (hereinafter, referred to as a compound group SX1480).

A compound wherein in the compound (L-3), n is 1, $R^1$ is a methyl group, $R^2$ is a methyl group, $R^{3b}$ is a difluoromethyl group, and Q is any of the substituents shown in [Table L1] to [Table L13] (hereinafter, referred to as a compound group SX1481).

A compound wherein in the compound (L-3), n is 2, $R^1$ is a methyl group, $R^2$ is a methyl group, $R^{3b}$ is a difluoromethyl group, and Q is any of the substituents shown in [Table L1] to [Table L13] (hereinafter, referred to as a compound group SX1482).

A compound wherein in the compound (L-3), n is 0, $R^1$ is an ethyl group, $R^2$ is a methyl group, $R^{3b}$ is a difluoromethyl group, and Q is any of the substituents shown in [Table L1] to [Table L13] (hereinafter, referred to as a compound group SX1483).

A compound wherein in the compound (L-3), n is 1, $R^1$ is an ethyl group, $R^2$ is a methyl group, $R^{3b}$ is a difluoromethyl group, and Q is any of the substituents shown in [Table L1] to [Table L13] (hereinafter, referred to as a compound group SX1484).

A compound wherein in the compound (L-3), n is 2, $R^1$ is an ethyl group, $R^2$ is a methyl group, $R^{3b}$ is a difluoromethyl group, and Q is any of the substituents shown in [Table L1] to [Table L13] (hereinafter, referred to as a compound group SX1485).

A compound wherein in the compound (L-3), n is 0, $R^1$ is a benzyl group, $R^2$ is a methyl group, $R^{3b}$ is a difluoromethyl group, and Q is any of the substituents shown in [Table L1] to [Table L13] (hereinafter, referred to as a compound group SX1486).

A compound wherein in the compound (L-3), n is 1, $R^1$ is a benzyl group, $R^2$ is a methyl group, $R^{3b}$ is a difluoromethyl group, and Q is any of the substituents shown in [Table L1] to [Table L13] (hereinafter, referred to as a compound group SX1487).

A compound wherein in the compound (L-3), n is 2, $R^1$ is a benzyl group, $R^2$ is a methyl group, $R^{3b}$ is a difluoromethyl group, and Q is any of the substituents shown in [Table L1] to [Table L13] (hereinafter, referred to as a compound group SX1488).

A compound wherein in the compound (L-3), n is 0, $R^1$ is a methyl group, $R^2$ is a cyclopropyl group, $R^{3b}$ is a hydrogen atom, and Q is any of the substituents shown in [Table L1] to [Table L13] (hereinafter, referred to as a compound group SX1492).

A compound wherein in the compound (L-3), n is 1, $R^1$ is a methyl group, $R^2$ is a cyclopropyl group, $R^{3b}$ is a hydrogen atom, and Q is any of the substituents shown in [Table L1] to [Table L13] (hereinafter, referred to as a compound group SX1493).

A compound wherein in the compound (L-3), n is 2, $R^1$ is a methyl group, $R^2$ is a cyclopropyl group, $R^{3b}$ is a hydrogen atom, and Q is any of the substituents shown in [Table L1] to [Table L13] (hereinafter, referred to as a compound group SX1494).

A compound wherein in the compound (L-3), n is 0, $R^1$ is an ethyl group, $R^2$ is a cyclopropyl group, $R^{3b}$ is a hydrogen atom, and Q is any of the substituents shown in [Table L1] to [Table L13] (hereinafter, referred to as a compound group SX1495).

A compound wherein in the compound (L-3), n is 1, $R^1$ is an ethyl group, $R^2$ is a cyclopropyl group, $R^{3b}$ is a

hydrogen atom, and Q is any of the substituents shown in [Table L1] to [Table L13] (hereinafter, referred to as a compound group SX1496).

A compound wherein in the compound (L-3), n is 2, $R^1$ is an ethyl group, $R^2$ is a cyclopropyl group, $R^{3b}$ is a hydrogen atom, and Q is any of the substituents shown in [Table L1] to [Table L13] (hereinafter, referred to as a compound group SX1497).

A compound wherein in the compound (L-3), n is 0, $R^1$ is a benzyl group, $R^2$ is a cyclopropyl group, $R^{3b}$ is a hydrogen atom, and Q is any of the substituents shown in [Table L1] to [Table L13] (hereinafter, referred to as a compound group SX1498).

A compound wherein in the compound (L-3), n is 1, $R^1$ is a benzyl group, $R^2$ is a cyclopropyl group, $R^{3b}$ is a hydrogen atom, and Q is any of the substituents shown in [Table L1] to [Table L13] (hereinafter, referred to as a compound group SX1499).

A compound wherein in the compound (L-3), n is 2, $R^1$ is a benzyl group, $R^2$ is a cyclopropyl group, $R^{3b}$ is a hydrogen atom, and Q is any of the substituents shown in [Table L1] to [Table L13] (hereinafter, referred to as a compound group SX1500).

A compound wherein in the compound (L-3), n is 0, $R^1$ is a methyl group, $R^2$ is a cyclopropyl group, $R^{3b}$ is a fluorine atom, and Q is any of the substituents shown in [Table L1] to [Table L13] (hereinafter, referred to as a compound group SX1504).

A compound wherein in the compound (L-3), n is 1, $R^1$ is a methyl group, $R^2$ is a cyclopropyl group, $R^{3b}$ is a fluorine atom, and Q is any of the substituents shown in [Table L1] to [Table L13] (hereinafter, referred to as a compound group SX1505).

A compound wherein in the compound (L-3), n is 2, $R^1$ is a methyl group, $R^2$ is a cyclopropyl group, $R^{3b}$ is a fluorine atom, and Q is any of the substituents shown in [Table L1] to [Table L13] (hereinafter, referred to as a compound group SX1506).

A compound wherein in the compound (L-3), n is 0, $R^1$ is an ethyl group, $R^2$ is a cyclopropyl group, $R^{3b}$ is a fluorine atom, and Q is any of the substituents shown in [Table L1] to [Table L13] (hereinafter, referred to as a compound group SX1507).

A compound wherein in the compound (L-3), n is 1, $R^1$ is an ethyl group, $R^2$ is a cyclopropyl group, $R^{3b}$ is a fluorine atom, and Q is any of the substituents shown in [Table L1] to [Table L13] (hereinafter, referred to as a compound group SX1508).

A compound wherein in the compound (L-3), n is 2, $R^1$ is an ethyl group, $R^2$ is a cyclopropyl group, $R^{3b}$ is a fluorine atom, and Q is any of the substituents shown in [Table L1] to [Table L13] (hereinafter, referred to as a compound group SX1509).

A compound wherein in the compound (L-3), n is 0, $R^1$ is a benzyl group, $R^2$ is a cyclopropyl group, $R^{3b}$ is a fluorine atom, and Q is any of the substituents shown in [Table L1] to [Table L13] (hereinafter, referred to as a compound group SX1510).

A compound wherein in the compound (L-3), n is 1, $R^1$ is a benzyl group, $R^2$ is a cyclopropyl group, $R^{3b}$ is a fluorine atom, and Q is any of the substituents shown in [Table L1] to [Table L13] (hereinafter, referred to as a compound group SX1511).

A compound wherein in the compound (L-3), n is 2, $R^1$ is a benzyl group, $R^2$ is a cyclopropyl group, $R^{3b}$ is a fluorine atom, and Q is any of the substituents shown in [Table L1] to [Table L13] (hereinafter, referred to as a compound group SX1512).

A compound wherein in the compound (L-3), n is 0, $R^1$ is a methyl group, $R^2$ is a cyclopropyl group, $R^{3b}$ is a chlorine atom, and Q is any of the substituents shown in [Table L1] to [Table L13] (hereinafter, referred to as a compound group SX1516).

A compound wherein in the compound (L-3), n is 1, $R^1$ is a methyl group, $R^2$ is a cyclopropyl group, $R^{3b}$ is a chlorine atom, and Q is any of the substituents shown in [Table L1] to [Table L13] (hereinafter, referred to as a compound group SX1517).

A compound wherein in the compound (L-3), n is 2, $R^1$ is a methyl group, $R^2$ is a cyclopropyl group, $R^{3b}$ is a chlorine atom, and Q is any of the substituents shown in [Table L1] to [Table L13] (hereinafter, referred to as a compound group SX1518).

A compound wherein in the compound (L-3), n is 0, $R^1$ is an ethyl group, $R^2$ is a cyclopropyl group, $R^{3b}$ is a chlorine atom, and Q is any of the substituents shown in [Table L1] to [Table L13] (hereinafter, referred to as a compound group SX1519).

A compound wherein in the compound (L-3), n is 1, $R^1$ is an ethyl group, $R^2$ is a cyclopropyl group, $R^{3b}$ is a chlorine atom, and Q is any of the substituents shown in [Table L1] to [Table L13] (hereinafter, referred to as a compound group SX1520).

A compound wherein in the compound (L-3), n is 2, $R^1$ is an ethyl group, $R^2$ is a cyclopropyl group, $R^{3b}$ is a chlorine atom, and Q is any of the substituents shown in [Table L1] to [Table L13] (hereinafter, referred to as a compound group

SX1521).

A compound wherein in the compound (L-3), n is 0, $R^1$ is a benzyl group, $R^2$ is a cyclopropyl group, $R^{3b}$ is a chlorine atom, and Q is any of the substituents shown in [Table L1] to [Table L13] (hereinafter, referred to as a compound group SX1522).

A compound wherein in the compound (L-3), n is 1, $R^1$ is a benzyl group, $R^2$ is a cyclopropyl group, $R^{3b}$ is a chlorine atom, and Q is any of the substituents shown in [Table L1] to [Table L13] (hereinafter, referred to as a compound group SX1523).

A compound wherein in the compound (L-3), n is 2, $R^1$ is a benzyl group, $R^2$ is a cyclopropyl group, $R^{3b}$ is a chlorine atom, and Q is any of the substituents shown in [Table L1] to [Table L13] (hereinafter, referred to as a compound group SX1524).

A compound wherein in the compound (L-3), n is 0, $R^1$ is a methyl group, $R^2$ is a cyclopropyl group, $R^{3b}$ is a bromine atom, and Q is any of the substituents shown in [Table L1] to [Table L13] (hereinafter, referred to as a compound group SX1528).

A compound wherein in the compound (L-3), n is 1, $R^1$ is a methyl group, $R^2$ is a cyclopropyl group, $R^{3b}$ is a bromine atom, and Q is any of the substituents shown in [Table L1] to [Table L13] (hereinafter, referred to as a compound group SX1529).

A compound wherein in the compound (L-3), n is 2, $R^1$ is a methyl group, $R^2$ is a cyclopropyl group, $R^{3b}$ is a bromine atom, and Q is any of the substituents shown in [Table L1] to [Table L13] (hereinafter, referred to as a compound group SX1530).

A compound wherein in the compound (L-3), n is 0, $R^1$ is an ethyl group, $R^2$ is a cyclopropyl group, $R^{3b}$ is a bromine atom, and Q is any of the substituents shown in [Table L1] to [Table L13] (hereinafter, referred to as a compound group SX1531).

A compound wherein in the compound (L-3), n is 1, $R^1$ is an ethyl group, $R^2$ is a cyclopropyl group, $R^{3b}$ is a bromine atom, and Q is any of the substituents shown in [Table L1] to [Table L13] (hereinafter, referred to as a compound group SX1532).

A compound wherein in the compound (L-3), n is 2, $R^1$ is an ethyl group, $R^2$ is a cyclopropyl group, $R^{3b}$ is a bromine atom, and Q is any of the substituents shown in [Table L1] to [Table L13] (hereinafter, referred to as a compound group SX1533).

A compound wherein in the compound (L-3), n is 0, $R^1$ is a benzyl group, $R^2$ is a cyclopropyl group, $R^{3b}$ is a bromine atom, and Q is any of the substituents shown in [Table L1] to [Table L13] (hereinafter, referred to as a compound group SX1534).

A compound wherein in the compound (L-3), n is 1, $R^1$ is a benzyl group, $R^2$ is a cyclopropyl group, $R^{3b}$ is a bromine atom, and Q is any of the substituents shown in [Table L1] to [Table L13] (hereinafter, referred to as a compound group SX1535).

A compound wherein in the compound (L-3), n is 2, $R^1$ is a benzyl group, $R^2$ is a cyclopropyl group, $R^{3b}$ is a bromine atom, and Q is any of the substituents shown in [Table L1] to [Table L13] (hereinafter, referred to as a compound group SX1536).

A compound wherein in the compound (L-3), n is 0, $R^1$ is a methyl group, $R^2$ is a cyclopropyl group, $R^{3b}$ is an iodine atom, and Q is any of the substituents shown in [Table L1] to [Table L13] (hereinafter, referred to as a compound group SX1540).

A compound wherein in the compound (L-3), n is 1, $R^1$ is a methyl group, $R^2$ is a cyclopropyl group, $R^{3b}$ is an iodine atom, and Q is any of the substituents shown in [Table L1] to [Table L13] (hereinafter, referred to as a compound group SX1541).

A compound wherein in the compound (L-3), n is 2, $R^1$ is a methyl group, $R^2$ is a cyclopropyl group, $R^{3b}$ is an iodine atom, and Q is any of the substituents shown in [Table L1] to [Table L13] (hereinafter, referred to as a compound group SX1542).

A compound wherein in the compound (L-3), n is 0, $R^1$ is an ethyl group, $R^2$ is a cyclopropyl group, $R^{3b}$ is an iodine atom, and Q is any of the substituents shown in [Table L1] to [Table L13] (hereinafter, referred to as a compound group SX1543).

A compound wherein in the compound (L-3), n is 1, $R^1$ is an ethyl group, $R^2$ is a cyclopropyl group, $R^{3b}$ is an iodine atom, and Q is any of the substituents shown in [Table L1] to [Table L13] (hereinafter, referred to as a compound group SX1544).

A compound wherein in the compound (L-3), n is 2, $R^1$ is an ethyl group, $R^2$ is a cyclopropyl group, $R^{3b}$ is an iodine atom, and Q is any of the substituents shown in [Table L1] to [Table L13] (hereinafter, referred to as a compound group SX1545).

A compound wherein in the compound (L-3), n is 0, $R^1$ is a benzyl group, $R^2$ is a cyclopropyl group, $R^{3b}$ is an iodine atom, and Q is any of the substituents shown in [Table L1] to [Table L13] (hereinafter, referred to as a compound group SX1546).

A compound wherein in the compound (L-3), n is 1, $R^1$ is a benzyl group, $R^2$ is a cyclopropyl group, $R^{3b}$ is an iodine atom, and Q is any of the substituents shown in [Table L1] to [Table L13] (hereinafter, referred to as a compound group SX1547).

A compound wherein in the compound (L-3), n is 2, $R^1$ is a benzyl group, $R^2$ is a cyclopropyl group, $R^{3b}$ is an iodine atom, and Q is any of the substituents shown in [Table L1] to [Table L13] (hereinafter, referred to as a compound group SX1548).

A compound wherein in the compound (L-3), n is 0, $R^1$ is a methyl group, $R^2$ is a cyclopropyl group, $R^{3b}$ is a trifluoromethyl group, and Q is any of the substituents shown in [Table L1] to [Table L13] (hereinafter, referred to as a compound group SX1552).

A compound wherein in the compound (L-3), n is 1, $R^1$ is a methyl group, $R^2$ is a cyclopropyl group, $R^{3b}$ is a trifluoromethyl group, and Q is any of the substituents shown in [Table L1] to [Table L13] (hereinafter, referred to as a compound group SX1553).

A compound wherein in the compound (L-3), n is 2, $R^1$ is a methyl group, $R^2$ is a cyclopropyl group, $R^{3b}$ is a trifluoromethyl group, and Q is any of the substituents shown in [Table L1] to [Table L13] (hereinafter, referred to as a compound group SX1554).

A compound wherein in the compound (L-3), n is 0, $R^1$ is an ethyl group, $R^2$ is a cyclopropyl group, $R^{3b}$ is a trifluoromethyl group, and Q is any of the substituents shown in [Table L1] to [Table L13] (hereinafter, referred to as a compound group SX1555).

A compound wherein in the compound (L-3), n is 1, $R^1$ is an ethyl group, $R^2$ is a cyclopropyl group, $R^{3b}$ is a trifluoromethyl group, and Q is any of the substituents shown in [Table L1] to [Table L13] (hereinafter, referred to as a compound group SX1556).

A compound wherein in the compound (L-3), n is 2, $R^1$ is an ethyl group, $R^2$ is a cyclopropyl group, $R^{3b}$ is a trifluoromethyl group, and Q is any of the substituents shown in [Table L1] to [Table L13] (hereinafter, referred to as a compound group SX1557).

A compound wherein in the compound (L-3), n is 0, $R^1$ is a benzyl group, $R^2$ is a cyclopropyl group, $R^{3b}$ is a trifluoromethyl group, and Q is any of the substituents shown in [Table L1] to [Table L13] (hereinafter, referred to as a compound group SX1558).

A compound wherein in the compound (L-3), n is 1, $R^1$ is a benzyl group, $R^2$ is a cyclopropyl group, $R^{3b}$ is a trifluoromethyl group, and Q is any of the substituents shown in [Table L1] to [Table L13] (hereinafter, referred to as a compound group SX1559).

A compound wherein in the compound (L-3), n is 2, $R^1$ is a benzyl group, $R^2$ is a cyclopropyl group, $R^{3b}$ is a trifluoromethyl group, and Q is any of the substituents shown in [Table L1] to [Table L13] (hereinafter, referred to as a compound group SX1560).

A compound wherein in the compound (L-3), n is 0, $R^1$ is a methyl group, $R^2$ is a cyclopropyl group, $R^{3b}$ is a difluoromethyl group, and Q is any of the substituents shown in [Table L1] to [Table L13] (hereinafter, referred to as a compound group SX1564).

A compound wherein in the compound (L-3), n is 1, $R^1$ is a methyl group, $R^2$ is a cyclopropyl group, $R^{3b}$ is a difluoromethyl group, and Q is any of the substituents shown in [Table L1] to [Table L13] (hereinafter, referred to as a compound group SX1565).

A compound wherein in the compound (L-3), n is 2, $R^1$ is a methyl group, $R^2$ is a cyclopropyl group, $R^{3b}$ is a difluoromethyl group, and Q is any of the substituents shown in [Table L1] to [Table L13] (hereinafter, referred to as a compound group SX1566).

A compound wherein in the compound (L-3), n is 0, $R^1$ is an ethyl group, $R^2$ is a cyclopropyl group, $R^{3b}$ is a difluoromethyl group, and Q is any of the substituents shown in [Table L1] to [Table L13] (hereinafter, referred to as a compound group SX1567).

A compound wherein in the compound (L-3), n is 1, $R^1$ is an ethyl group, $R^2$ is a cyclopropyl group, $R^{3b}$ is a difluoromethyl group, and Q is any of the substituents shown in [Table L1] to [Table L13] (hereinafter, referred to as a compound group SX1568).

A compound wherein in the compound (L-3), n is 2, $R^1$ is an ethyl group, $R^2$ is a cyclopropyl group, $R^{3b}$ is a difluoromethyl group, and Q is any of the substituents shown in [Table L1] to [Table L13] (hereinafter, referred to as a compound group SX1569).

A compound wherein in the compound (L-3), n is 0, $R^1$ is a benzyl group, $R^2$ is a cyclopropyl group, $R^{3b}$ is a difluoromethyl group, and Q is any of the substituents shown in [Table L1] to [Table L13] (hereinafter, referred to as a compound group SX1570).

A compound wherein in the compound (L-3), n is 1, $R^1$ is a benzyl group, $R^2$ is a cyclopropyl group, $R^{3b}$ is a difluoromethyl group, and Q is any of the substituents shown in [Table L1] to [Table L13] (hereinafter, referred to as a compound group SX1571).

A compound wherein in the compound (L-3), n is 2, $R^1$ is a benzyl group, $R^2$ is a cyclopropyl group, $R^{3b}$ is a

difluoromethyl group, and Q is any of the substituents shown in [Table L1] to [Table L13] (hereinafter, referred to as a compound group SX1572).

A compound wherein in the compound (L-3), n is 0, $R^1$ is a methyl group, $R^2$ is a N,N-dimethylamino group, $R^{3b}$ is a hydrogen atom, and Q is any of the substituents shown in [Table L1] to [Table L13] (hereinafter, referred to as a compound group SX1576).

A compound wherein in the compound (L-3), n is 1, $R^1$ is a methyl group, $R^2$ is a N,N-dimethylamino group, $R^{3b}$ is a hydrogen atom, and Q is any of the substituents shown in [Table L1] to [Table L13] (hereinafter, referred to as a compound group SX1577).

A compound wherein in the compound (L-3), n is 2, $R^1$ is a methyl group, $R^2$ is a N,N-dimethylamino group, $R^{3b}$ is a hydrogen atom, and Q is any of the substituents shown in [Table L1] to [Table L13] (hereinafter, referred to as a compound group SX1578).

A compound wherein in the compound (L-3), n is 0, $R^1$ is an ethyl group, $R^2$ is a N,N-dimethylamino group, $R^{3b}$ is a hydrogen atom, and Q is any of the substituents shown in [Table L1] to [Table L13] (hereinafter, referred to as a compound group SX1579).

A compound wherein in the compound (L-3), n is 1, $R^1$ is an ethyl group, $R^2$ is a N,N-dimethylamino group, $R^{3b}$ is a hydrogen atom, and Q is any of the substituents shown in [Table L1] to [Table L13] (hereinafter, referred to as a compound group SX1580).

A compound wherein in the compound (L-3), n is 2, $R^1$ is an ethyl group, $R^2$ is a N,N-dimethylamino group, $R^{3b}$ is a hydrogen atom, and Q is any of the substituents shown in [Table L1] to [Table L13] (hereinafter, referred to as a compound group SX1581).

A compound wherein in the compound (L-3), n is 0, $R^1$ is a benzyl group, $R^2$ is a N,N-dimethylamino group, $R^{3b}$ is a hydrogen atom, and Q is any of the substituents shown in [Table L1] to [Table L13] (hereinafter, referred to as a compound group SX1582).

A compound wherein in the compound (L-3), n is 1, $R^1$ is a benzyl group, $R^2$ is a N,N-dimethylamino group, $R^{3b}$ is a hydrogen atom, and Q is any of the substituents shown in [Table L1] to [Table L13] (hereinafter, referred to as a compound group SX1583).

A compound wherein in the compound (L-3), n is 2, $R^1$ is a benzyl group, $R^2$ is a N,N-dimethylamino group, $R^{3b}$ is a hydrogen atom, and Q is any of the substituents shown in [Table L1] to [Table L13] (hereinafter, referred to as a compound group SX1584).

A compound wherein in the compound (L-3), n is 0, $R^1$ is a methyl group, $R^2$ is a N,N-dimethylamino group, $R^{3b}$ is a fluorine atom, and Q is any of the substituents shown in [Table L1] to [Table L13] (hereinafter, referred to as a compound group SX1588).

A compound wherein in the compound (L-3), n is 1, $R^1$ is a methyl group, $R^2$ is a N,N-dimethylamino group, $R^{3b}$ is a fluorine atom, and Q is any of the substituents shown in [Table L1] to [Table L13] (hereinafter, referred to as a compound group SX1589).

A compound wherein in the compound (L-3), n is 2, $R^1$ is a methyl group, $R^2$ is a N,N-dimethylamino group, $R^{3b}$ is a fluorine atom, and Q is any of the substituents shown in [Table L1] to [Table L13] (hereinafter, referred to as a compound group SX1590).

A compound wherein in the compound (L-3), n is 0, $R^1$ is an ethyl group, $R^2$ is a N,N-dimethylamino group, $R^{3b}$ is a fluorine atom, and Q is any of the substituents shown in [Table L1] to [Table L13] (hereinafter, referred to as a compound group SX1591).

A compound wherein in the compound (L-3), n is 1, $R^1$ is an ethyl group, $R^2$ is a N,N-dimethylamino group, $R^{3b}$ is a fluorine atom, and Q is any of the substituents shown in [Table L1] to [Table L13] (hereinafter, referred to as a compound group SX1592).

A compound wherein in the compound (L-3), n is 2, $R^1$ is an ethyl group, $R^2$ is a N,N-dimethylamino group, $R^{3b}$ is a fluorine atom, and Q is any of the substituents shown in [Table L1] to [Table L13] (hereinafter, referred to as a compound group SX1593).

A compound wherein in the compound (L-3), n is 0, $R^1$ is a benzyl group, $R^2$ is a N,N-dimethylamino group, $R^{3b}$ is a fluorine atom, and Q is any of the substituents shown in [Table L1] to [Table L13] (hereinafter, referred to as a compound group SX1594).

A compound wherein in the compound (L-3), n is 1, $R^1$ is a benzyl group, $R^2$ is a N,N-dimethylamino group, $R^{3b}$ is a fluorine atom, and Q is any of the substituents shown in [Table L1] to [Table L13] (hereinafter, referred to as a compound group SX1595).

A compound wherein in the compound (L-3), n is 2, $R^1$ is a benzyl group, $R^2$ is a N,N-dimethylamino group, $R^{3b}$ is a fluorine atom, and Q is any of the substituents shown in [Table L1] to [Table L13] (hereinafter, referred to as a compound group SX1596).

A compound wherein in the compound (L-3), n is 0, $R^1$ is a methyl group, $R^2$ is a N,N-dimethylamino group, $R^{3b}$ is a chlorine atom, and Q is any of the substituents shown in [Table L1] to [Table L13] (hereinafter, referred to as a compound

group SX1600).

A compound wherein in the compound (L-3), n is 1, $R^1$ is a methyl group, $R^2$ is a N,N-dimethylamino group, $R^{3b}$ is a chlorine atom, and Q is any of the substituents shown in [Table L1] to [Table L13] (hereinafter, referred to as a compound group SX1601).

A compound wherein in the compound (L-3), n is 2, $R^1$ is a methyl group, $R^2$ is a N,N-dimethylamino group, $R^{3b}$ is a chlorine atom, and Q is any of the substituents shown in [Table L1] to [Table L13] (hereinafter, referred to as a compound group SX1602).

A compound wherein in the compound (L-3), n is 0, $R^1$ is an ethyl group, $R^2$ is a N,N-dimethylamino group, $R^{3b}$ is a chlorine atom, and Q is any of the substituents shown in [Table L1] to [Table L13] (hereinafter, referred to as a compound group SX1603).

A compound wherein in the compound (L-3), n is 1, $R^1$ is an ethyl group, $R^2$ is a N,N-dimethylamino group, $R^{3b}$ is a chlorine atom, and Q is any of the substituents shown in [Table L1] to [Table L13] (hereinafter, referred to as a compound group SX1604).

A compound wherein in the compound (L-3), n is 2, $R^1$ is an ethyl group, $R^2$ is a N,N-dimethylamino group, $R^{3b}$ is a chlorine atom, and Q is any of the substituents shown in [Table L1] to [Table L13] (hereinafter, referred to as a compound group SX1605).

A compound wherein in the compound (L-3), n is 0, $R^1$ is a benzyl group, $R^2$ is a N,N-dimethylamino group, $R^{3b}$ is a chlorine atom, and Q is any of the substituents shown in [Table L1] to [Table L13] (hereinafter, referred to as a compound group SX1606).

A compound wherein in the compound (L-3), n is 1, $R^1$ is a benzyl group, $R^2$ is a N,N-dimethylamino group, $R^{3b}$ is a chlorine atom, and Q is any of the substituents shown in [Table L1] to [Table L13] (hereinafter, referred to as a compound group SX1607).

A compound wherein in the compound (L-3), n is 2, $R^1$ is a benzyl group, $R^2$ is a N,N-dimethylamino group, $R^{3b}$ is a chlorine atom, and Q is any of the substituents shown in [Table L1] to [Table L13] (hereinafter, referred to as a compound group SX1608).

A compound wherein in the compound (L-3), n is 0, $R^1$ is a methyl group, $R^2$ is a N,N-dimethylamino group, $R^{3b}$ is a bromine atom, and Q is any of the substituents shown in [Table L1] to [Table L13] (hereinafter, referred to as a compound group SX1612).

A compound wherein in the compound (L-3), n is 1, $R^1$ is a methyl group, $R^2$ is a N,N-dimethylamino group, $R^{3b}$ is a bromine atom, and Q is any of the substituents shown in [Table L1] to [Table L13] (hereinafter, referred to as a compound group SX1613).

A compound wherein in the compound (L-3), n is 2, $R^1$ is a methyl group, $R^2$ is a N,N-dimethylamino group, $R^{3b}$ is a bromine atom, and Q is any of the substituents shown in [Table L1] to [Table L13] (hereinafter, referred to as a compound group SX1614).

A compound wherein in the compound (L-3), n is 0, $R^1$ is an ethyl group, $R^2$ is a N,N-dimethylamino group, $R^{3b}$ is a bromine atom, and Q is any of the substituents shown in [Table L1] to [Table L13] (hereinafter, referred to as a compound group SX1615).

A compound wherein in the compound (L-3), n is 1, $R^1$ is an ethyl group, $R^2$ is a N,N-dimethylamino group, $R^{3b}$ is a bromine atom, and Q is any of the substituents shown in [Table L1] to [Table L13] (hereinafter, referred to as a compound group SX1616).

A compound wherein in the compound (L-3), n is 2, $R^1$ is an ethyl group, $R^2$ is a N,N-dimethylamino group, $R^{3b}$ is a bromine atom, and Q is any of the substituents shown in [Table L1] to [Table L13] (hereinafter, referred to as a compound group SX1617).

A compound wherein in the compound (L-3), n is 0, $R^1$ is a benzyl group, $R^2$ is a N,N-dimethylamino group, $R^{3b}$ is a bromine atom, and Q is any of the substituents shown in [Table L1] to [Table L13] (hereinafter, referred to as a compound group SX1618).

A compound wherein in the compound (L-3), n is 1, $R^1$ is a benzyl group, $R^2$ is a N,N-dimethylamino group, $R^{3b}$ is a bromine atom, and Q is any of the substituents shown in [Table L1] to [Table L13] (hereinafter, referred to as a compound group SX1619).

A compound wherein in the compound (L-3), n is 2, $R^1$ is a benzyl group, $R^2$ is a N,N-dimethylamino group, $R^{3b}$ is a bromine atom, and Q is any of the substituents shown in [Table L1] to [Table L13] (hereinafter, referred to as a compound group SX1620).

A compound wherein in the compound (L-3), n is 0, $R^1$ is a methyl group, $R^2$ is a N,N-dimethylamino group, $R^{3b}$ is an iodine atom, and Q is any of the substituents shown in [Table L1] to [Table L13] (hereinafter, referred to as a compound group SX1624).

A compound wherein in the compound (L-3), n is 1, $R^1$ is a methyl group, $R^2$ is a N,N-dimethylamino group, $R^{3b}$ is an iodine atom, and Q is any of the substituents shown in [Table L1] to [Table L13] (hereinafter, referred to as a compound group SX1625).

A compound wherein in the compound (L-3), n is 2, $R^1$ is a methyl group, $R^2$ is a N,N-dimethylamino group, $R^{3b}$ is an iodine atom, and Q is any of the substituents shown in [Table L1] to [Table L13] (hereinafter, referred to as a compound group SX1626).

A compound wherein in the compound (L-3), n is 0, $R^1$ is an ethyl group, $R^2$ is a N,N-dimethylamino group, $R^{3b}$ is an iodine atom, and Q is any of the substituents shown in [Table L1] to [Table L13] (hereinafter, referred to as a compound group SX1627).

A compound wherein in the compound (L-3), n is 1, $R^1$ is an ethyl group, $R^2$ is a N,N-dimethylamino group, $R^{3b}$ is an iodine atom, and Q is any of the substituents shown in [Table L1] to [Table L13] (hereinafter, referred to as a compound group SX1628).

A compound wherein in the compound (L-3), n is 2, $R^1$ is an ethyl group, $R^2$ is a N,N-dimethylamino group, $R^{3b}$ is an iodine atom, and Q is any of the substituents shown in [Table L1] to [Table L13] (hereinafter, referred to as a compound group SX1629).

A compound wherein in the compound (L-3), n is 0, $R^1$ is a benzyl group, $R^2$ is a N,N-dimethylamino group, $R^{3b}$ is an iodine atom, and Q is any of the substituents shown in [Table L1] to [Table L13] (hereinafter, referred to as a compound group SX1630).

A compound wherein in the compound (L-3), n is 1, $R^1$ is a benzyl group, $R^2$ is a N,N-dimethylamino group, $R^{3b}$ is an iodine atom, and Q is any of the substituents shown in [Table L1] to [Table L13] (hereinafter, referred to as a compound group SX1631).

A compound wherein in the compound (L-3), n is 2, $R^1$ is a benzyl group, $R^2$ is a N,N-dimethylamino group, $R^{3b}$ is an iodine atom, and Q is any of the substituents shown in [Table L1] to [Table L13] (hereinafter, referred to as a compound group SX1632).

A compound wherein in the compound (L-3), n is 0, $R^1$ is a methyl group, $R^2$ is a N,N-dimethylamino group, $R^{3b}$ is a trifluoromethyl group, and Q is any of the substituents shown in [Table L1] to [Table L13] (hereinafter, referred to as a compound group SX1636).

A compound wherein in the compound (L-3), n is 1, $R^1$ is a methyl group, $R^2$ is a N,N-dimethylamino group, $R^{3b}$ is a trifluoromethyl group, and Q is any of the substituents shown in [Table L1] to [Table L13] (hereinafter, referred to as a compound group SX1637).

A compound wherein in the compound (L-3), n is 2, $R^1$ is a methyl group, $R^2$ is a N,N-dimethylamino group, $R^{3b}$ is a trifluoromethyl group, and Q is any of the substituents shown in [Table L1] to [Table L13] (hereinafter, referred to as a compound group SX1638).

A compound wherein in the compound (L-3), n is 0, $R^1$ is an ethyl group, $R^2$ is a N,N-dimethylamino group, $R^{3b}$ is a trifluoromethyl group, and Q is any of the substituents shown in [Table L1] to [Table L13] (hereinafter, referred to as a compound group SX1639).

A compound wherein in the compound (L-3), n is 1, $R^1$ is an ethyl group, $R^2$ is a N,N-dimethylamino group, $R^{3b}$ is a trifluoromethyl group, and Q is any of the substituents shown in [Table L1] to [Table L13] (hereinafter, referred to as a compound group SX1640).

A compound wherein in the compound (L-3), n is 2, $R^1$ is an ethyl group, $R^2$ is a N,N-dimethylamino group, $R^{3b}$ is a trifluoromethyl group, and Q is any of the substituents shown in [Table L1] to [Table L13] (hereinafter, referred to as a compound group SX1641).

A compound wherein in the compound (L-3), n is 0, $R^1$ is a benzyl group, $R^2$ is a N,N-dimethylamino group, $R^{3b}$ is a trifluoromethyl group, and Q is any of the substituents shown in [Table L1] to [Table L13] (hereinafter, referred to as a compound group SX1642).

A compound wherein in the compound (L-3), n is 1, $R^1$ is a benzyl group, $R^2$ is a N,N-dimethylamino group, $R^{3b}$ is a trifluoromethyl group, and Q is any of the substituents shown in [Table L1] to [Table L13] (hereinafter, referred to as a compound group SX1643).

A compound wherein in the compound (L-3), n is 2, $R^1$ is a benzyl group, $R^2$ is a N,N-dimethylamino group, $R^{3b}$ is a trifluoromethyl group, and Q is any of the substituents shown in [Table L1] to [Table L13] (hereinafter, referred to as a compound group SX1644).

A compound wherein in the compound (L-3), n is 0, $R^1$ is a methyl group, $R^2$ is a N,N-dimethylamino group, $R^{3b}$ is a difluoromethyl group, and Q is any of the substituents shown in [Table L1] to [Table L13] (hereinafter, referred to as a compound group SX1648).

A compound wherein in the compound (L-3), n is 1, $R^1$ is a methyl group, $R^2$ is a N,N-dimethylamino group, $R^{3b}$ is a difluoromethyl group, and Q is any of the substituents shown in [Table L1] to [Table L13] (hereinafter, referred to as a compound group SX1649).

A compound wherein in the compound (L-3), n is 2, $R^1$ is a methyl group, $R^2$ is a N,N-dimethylamino group, $R^{3b}$ is a difluoromethyl group, and Q is any of the substituents shown in [Table L1] to [Table L13] (hereinafter, referred to as a compound group SX1650).

A compound wherein in the compound (L-3), n is 0, $R^1$ is an ethyl group, $R^2$ is a N,N-dimethylamino group, $R^{3b}$ is a

difluoromethyl group, and Q is any of the substituents shown in [Table L1] to [Table L13] (hereinafter, referred to as a compound group SX1651).

A compound wherein in the compound (L-3), n is 1, $R^1$ is an ethyl group, $R^2$ is a N,N-dimethylamino group, $R^{3b}$ is a difluoromethyl group, and Q is any of the substituents shown in [Table L1] to [Table L13] (hereinafter, referred to as a compound group SX1652).

A compound wherein in the compound (L-3), n is 2, $R^1$ is an ethyl group, $R^2$ is a N,N-dimethylamino group, $R^{3b}$ is a difluoromethyl group, and Q is any of the substituents shown in [Table L1] to [Table L13] (hereinafter, referred to as a compound group SX1653).

A compound wherein in the compound (L-3), n is 0, $R^1$ is a benzyl group, $R^2$ is a N,N-dimethylamino group, $R^{3b}$ is a difluoromethyl group, and Q is any of the substituents shown in [Table L1] to [Table L13] (hereinafter, referred to as a compound group SX1654).

A compound wherein in the compound (L-3), n is 1, $R^1$ is a benzyl group, $R^2$ is a N,N-dimethylamino group, $R^{3b}$ is a difluoromethyl group, and Q is any of the substituents shown in [Table L1] to [Table L13] (hereinafter, referred to as a compound group SX1655).

A compound wherein in the compound (L-3), n is 2, $R^1$ is a benzyl group, $R^2$ is a N,N-dimethylamino group, $R^{3b}$ is a difluoromethyl group, and Q is any of the substituents shown in [Table L1] to [Table L13] (hereinafter, referred to as a compound group SX1656).

A compound wherein in the compound (L-3), n is 0, $R^1$ is a methyl group, $R^2$ is a N-methylamino group, $R^{3b}$ is a hydrogen atom, and Q is any of the substituents shown in [Table L1] to [Table L13] (hereinafter, referred to as a compound group SX1660).

A compound wherein in the compound (L-3), n is 1, $R^1$ is a methyl group, $R^2$ is a N-methylamino group, $R^{3b}$ is a hydrogen atom, and Q is any of the substituents shown in [Table L1] to [Table L13] (hereinafter, referred to as a compound group SX1661).

A compound wherein in the compound (L-3), n is 2, $R^1$ is a methyl group, $R^2$ is a N-methylamino group, $R^{3b}$ is a hydrogen atom, and Q is any of the substituents shown in [Table L1] to [Table L13] (hereinafter, referred to as a compound group SX1662).

A compound wherein in the compound (L-3), n is 0, $R^1$ is an ethyl group, $R^2$ is a N-methylamino group, $R^{3b}$ is a hydrogen atom, and Q is any of the substituents shown in [Table L1] to [Table L13] (hereinafter, referred to as a compound group SX1663).

A compound wherein in the compound (L-3), n is 1, $R^1$ is an ethyl group, $R^2$ is a N-methylamino group, $R^{3b}$ is a hydrogen atom, and Q is any of the substituents shown in [Table L1] to [Table L13] (hereinafter, referred to as a compound group SX1664).

A compound wherein in the compound (L-3), n is 2, $R^1$ is an ethyl group, $R^2$ is a N-methylamino group, $R^{3b}$ is a hydrogen atom, and Q is any of the substituents shown in [Table L1] to [Table L13] (hereinafter, referred to as a compound group SX1665).

A compound wherein in the compound (L-3), n is 0, $R^1$ is a benzyl group, $R^2$ is a N-methylamino group, $R^{3b}$ is a hydrogen atom, and Q is any of the substituents shown in [Table L1] to [Table L13] (hereinafter, referred to as a compound group SX1666).

A compound wherein in the compound (L-3), n is 1, $R^1$ is a benzyl group, $R^2$ is a N-methylamino group, $R^{3b}$ is a hydrogen atom, and Q is any of the substituents shown in [Table L1] to [Table L13] (hereinafter, referred to as a compound group SX1667).

A compound wherein in the compound (L-3), n is 2, $R^1$ is a benzyl group, $R^2$ is a N-methylamino group, $R^{3b}$ is a hydrogen atom, and Q is any of the substituents shown in [Table L1] to [Table L13] (hereinafter, referred to as a compound group SX1668).

A compound wherein in the compound (L-3), n is 0, $R^1$ is a methyl group, $R^2$ is a N-methylamino group, $R^{3b}$ is a fluorine atom, and Q is any of the substituents shown in [Table L1] to [Table L13] (hereinafter, referred to as a compound group SX1672).

A compound wherein in the compound (L-3), n is 1, $R^1$ is a methyl group, $R^2$ is a N-methylamino group, $R^{3b}$ is a fluorine atom, and Q is any of the substituents shown in [Table L1] to [Table L13] (hereinafter, referred to as a compound group SX1673).

A compound wherein in the compound (L-3), n is 2, $R^1$ is a methyl group, $R^2$ is a N-methylamino group, $R^{3b}$ is a fluorine atom, and Q is any of the substituents shown in [Table L1] to [Table L13] (hereinafter, referred to as a compound group SX1674).

A compound wherein in the compound (L-3), n is 0, $R^1$ is an ethyl group, $R^2$ is a N-methylamino group, $R^{3b}$ is a fluorine atom, and Q is any of the substituents shown in [Table L1] to [Table L13] (hereinafter, referred to as a compound group SX1675).

A compound wherein in the compound (L-3), n is 1, $R^1$ is an ethyl group, $R^2$ is a N-methylamino group, $R^{3b}$ is a fluorine atom, and Q is any of the substituents shown in [Table L1] to [Table L13] (hereinafter, referred to as a compound

group SX1676).

A compound wherein in the compound (L-3), n is 2, $R^1$ is an ethyl group, $R^2$ is a N-methylamino group, $R^{3b}$ is a fluorine atom, and Q is any of the substituents shown in [Table L1] to [Table L13] (hereinafter, referred to as a compound group SX1677).

A compound wherein in the compound (L-3), n is 0, $R^1$ is a benzyl group, $R^2$ is a N-methylamino group, $R^{3b}$ is a fluorine atom, and Q is any of the substituents shown in [Table L1] to [Table L13] (hereinafter, referred to as a compound group SX1678).

A compound wherein in the compound (L-3), n is 1, $R^1$ is a benzyl group, $R^2$ is a N-methylamino group, $R^{3b}$ is a fluorine atom, and Q is any of the substituents shown in [Table L1] to [Table L13] (hereinafter, referred to as a compound group SX1679).

A compound wherein in the compound (L-3), n is 2, $R^1$ is a benzyl group, $R^2$ is a N-methylamino group, $R^{3b}$ is a fluorine atom, and Q is any of the substituents shown in [Table L1] to [Table L13] (hereinafter, referred to as a compound group SX1680).

A compound wherein in the compound (L-3), n is 0, $R^1$ is a methyl group, $R^2$ is a N-methylamino group, $R^{3b}$ is a chlorine atom, and Q is any of the substituents shown in [Table L1] to [Table L13] (hereinafter, referred to as a compound group SX1684).

A compound wherein in the compound (L-3), n is 1, $R^1$ is a methyl group, $R^2$ is a N-methylamino group, $R^{3b}$ is a chlorine atom, and Q is any of the substituents shown in [Table L1] to [Table L13] (hereinafter, referred to as a compound group SX1685).

A compound wherein in the compound (L-3), n is 2, $R^1$ is a methyl group, $R^2$ is a N-methylamino group, $R^{3b}$ is a chlorine atom, and Q is any of the substituents shown in [Table L1] to [Table L13] (hereinafter, referred to as a compound group SX1686).

A compound wherein in the compound (L-3), n is 0, $R^1$ is an ethyl group, $R^2$ is a N-methylamino group, $R^{3b}$ is a chlorine atom, and Q is any of the substituents shown in [Table L1] to [Table L13] (hereinafter, referred to as a compound group SX1687).

A compound wherein in the compound (L-3), n is 1, $R^1$ is an ethyl group, $R^2$ is a N-methylamino group, $R^{3b}$ is a chlorine atom, and Q is any of the substituents shown in [Table L1] to [Table L13] (hereinafter, referred to as a compound group SX1688).

A compound wherein in the compound (L-3), n is 2, $R^1$ is an ethyl group, $R^2$ is a N-methylamino group, $R^{3b}$ is a chlorine atom, and Q is any of the substituents shown in [Table L1] to [Table L13] (hereinafter, referred to as a compound group SX1689).

A compound wherein in the compound (L-3), n is 0, $R^1$ is a benzyl group, $R^2$ is a N-methylamino group, $R^{3b}$ is a chlorine atom, and Q is any of the substituents shown in [Table L1] to [Table L13] (hereinafter, referred to as a compound group SX1690).

A compound wherein in the compound (L-3), n is 1, $R^1$ is a benzyl group, $R^2$ is a N-methylamino group, $R^{3b}$ is a chlorine atom, and Q is any of the substituents shown in [Table L1] to [Table L13] (hereinafter, referred to as a compound group SX1691).

A compound wherein in the compound (L-3), n is 2, $R^1$ is a benzyl group, $R^2$ is a N-methylamino group, $R^{3b}$ is a chlorine atom, and Q is any of the substituents shown in [Table L1] to [Table L13] (hereinafter, referred to as a compound group SX1692).

A compound wherein in the compound (L-3), n is 0, $R^1$ is a methyl group, $R^2$ is a N-methylamino group, $R^{3b}$ is a bromine atom, and Q is any of the substituents shown in [Table L1] to [Table L13] (hereinafter, referred to as a compound group SX1696).

A compound wherein in the compound (L-3), n is 1, $R^1$ is a methyl group, $R^2$ is a N-methylamino group, $R^{3b}$ is a bromine atom, and Q is any of the substituents shown in [Table L1] to [Table L13] (hereinafter, referred to as a compound group SX1697).

A compound wherein in the compound (L-3), n is 2, $R^1$ is a methyl group, $R^2$ is a N-methylamino group, $R^{3b}$ is a bromine atom, and Q is any of the substituents shown in [Table L1] to [Table L13] (hereinafter, referred to as a compound group SX1698).

A compound wherein in the compound (L-3), n is 0, $R^1$ is an ethyl group, $R^2$ is a N-methylamino group, $R^{3b}$ is a bromine atom, and Q is any of the substituents shown in [Table L1] to [Table L13] (hereinafter, referred to as a compound group SX1699).

A compound wherein in the compound (L-3), n is 1, $R^1$ is an ethyl group, $R^2$ is a N-methylamino group, $R^{3b}$ is a bromine atom, and Q is any of the substituents shown in [Table L1] to [Table L13] (hereinafter, referred to as a compound group SX1700).

A compound wherein in the compound (L-3), n is 2, $R^1$ is an ethyl group, $R^2$ is a N-methylamino group, $R^{3b}$ is a bromine atom, and Q is any of the substituents shown in [Table L1] to [Table L13] (hereinafter, referred to as a compound group SX1701).

A compound wherein in the compound (L-3), n is 0, $R^1$ is a benzyl group, $R^2$ is a N-methylamino group, $R^{3b}$ is a bromine atom, and Q is any of the substituents shown in [Table L1] to [Table L13] (hereinafter, referred to as a compound group SX1702).

A compound wherein in the compound (L-3), n is 1, $R^1$ is a benzyl group, $R^2$ is a N-methylamino group, $R^{3b}$ is a bromine atom, and Q is any of the substituents shown in [Table L1] to [Table L13] (hereinafter, referred to as a compound group SX1703).

A compound wherein in the compound (L-3), n is 2, $R^1$ is a benzyl group, $R^2$ is a N-methylamino group, $R^{3b}$ is a bromine atom, and Q is any of the substituents shown in [Table L1] to [Table L13] (hereinafter, referred to as a compound group SX1704).

A compound wherein in the compound (L-3), n is 0, $R^1$ is a methyl group, $R^2$ is a N-methylamino group, $R^{3b}$ is an iodine atom, and Q is any of the substituents shown in [Table L1] to [Table L13] (hereinafter, referred to as a compound group SX1708).

A compound wherein in the compound (L-3), n is 1, $R^1$ is a methyl group, $R^2$ is a N-methylamino group, $R^{3b}$ is an iodine atom, and Q is any of the substituents shown in [Table L1] to [Table L13] (hereinafter, referred to as a compound group SX1709).

A compound wherein in the compound (L-3), n is 2, $R^1$ is a methyl group, $R^2$ is a N-methylamino group, $R^{3b}$ is an iodine atom, and Q is any of the substituents shown in [Table L1] to [Table L13] (hereinafter, referred to as a compound group SX1710).

A compound wherein in the compound (L-3), n is 0, $R^1$ is an ethyl group, $R^2$ is a N-methylamino group, $R^{3b}$ is an iodine atom, and Q is any of the substituents shown in [Table L1] to [Table L13] (hereinafter, referred to as a compound group SX1711).

A compound wherein in the compound (L-3), n is 1, $R^1$ is an ethyl group, $R^2$ is a N-methylamino group, $R^{3b}$ is an iodine atom, and Q is any of the substituents shown in [Table L1] to [Table L13] (hereinafter, referred to as a compound group SX1712).

A compound wherein in the compound (L-3), n is 2, $R^1$ is an ethyl group, $R^2$ is a N-methylamino group, $R^{3b}$ is an iodine atom, and Q is any of the substituents shown in [Table L1] to [Table L13] (hereinafter, referred to as a compound group SX1713).

A compound wherein in the compound (L-3), n is 0, $R^1$ is a benzyl group, $R^2$ is a N-methylamino group, $R^{3b}$ is an iodine atom, and Q is any of the substituents shown in [Table L1] to [Table L13] (hereinafter, referred to as a compound group SX1714).

A compound wherein in the compound (L-3), n is 1, $R^1$ is a benzyl group, $R^2$ is a N-methylamino group, $R^{3b}$ is an iodine atom, and Q is any of the substituents shown in [Table L1] to [Table L13] (hereinafter, referred to as a compound group SX1715).

A compound wherein in the compound (L-3), n is 2, $R^1$ is a benzyl group, $R^2$ is a N-methylamino group, $R^{3b}$ is an iodine atom, and Q is any of the substituents shown in [Table L1] to [Table L13] (hereinafter, referred to as a compound group SX1716).

A compound wherein in the compound (L-3), n is 0, $R^1$ is a methyl group, $R^2$ is a N-methylamino group, $R^{3b}$ is a trifluoromethyl group, and Q is any of the substituents shown in [Table L1] to [Table L13] (hereinafter, referred to as a compound group SX1720).

A compound wherein in the compound (L-3), n is 1, $R^1$ is a methyl group, $R^2$ is a N-methylamino group, $R^{3b}$ is a trifluoromethyl group, and Q is any of the substituents shown in [Table L1] to [Table L13] (hereinafter, referred to as a compound group SX1721).

A compound wherein in the compound (L-3), n is 2, $R^1$ is a methyl group, $R^2$ is a N-methylamino group, $R^{3b}$ is a trifluoromethyl group, and Q is any of the substituents shown in [Table L1] to [Table L13] (hereinafter, referred to as a compound group SX1722).

A compound wherein in the compound (L-3), n is 0, $R^1$ is an ethyl group, $R^2$ is a N-methylamino group, $R^{3b}$ is a trifluoromethyl group, and Q is any of the substituents shown in [Table L1] to [Table L13] (hereinafter, referred to as a compound group SX1723).

A compound wherein in the compound (L-3), n is 1, $R^1$ is an ethyl group, $R^2$ is a N-methylamino group, $R^{3b}$ is a trifluoromethyl group, and Q is any of the substituents shown in [Table L1] to [Table L13] (hereinafter, referred to as a compound group SX1724).

A compound wherein in the compound (L-3), n is 2, $R^1$ is an ethyl group, $R^2$ is a N-methylamino group, $R^{3b}$ is a trifluoromethyl group, and Q is any of the substituents shown in [Table L1] to [Table L13] (hereinafter, referred to as a compound group SX1725).

A compound wherein in the compound (L-3), n is 0, $R^1$ is a benzyl group, $R^2$ is a N-methylamino group, $R^{3b}$ is a trifluoromethyl group, and Q is any of the substituents shown in [Table L1] to [Table L13] (hereinafter, referred to as a compound group SX1726).

A compound wherein in the compound (L-3), n is 1, $R^1$ is a benzyl group, $R^2$ is a N-methylamino group, $R^{3b}$ is a

trifluoromethyl group, and Q is any of the substituents shown in [Table L1] to [Table L13] (hereinafter, referred to as a compound group SX1727).

A compound wherein in the compound (L-3), n is 2, $R^1$ is a benzyl group, $R^2$ is a N-methylamino group, $R^{3b}$ is a trifluoromethyl group, and Q is any of the substituents shown in [Table L1] to [Table L13] (hereinafter, referred to as a compound group SX1728).

A compound wherein in the compound (L-3), n is 0, $R^1$ is a methyl group, $R^2$ is a N-methylamino group, $R^{3b}$ is a difluoromethyl group, and Q is any of the substituents shown in [Table L1] to [Table L13] (hereinafter, referred to as a compound group SX1732).

A compound wherein in the compound (L-3), n is 1, $R^1$ is a methyl group, $R^2$ is a N-methylamino group, $R^{3b}$ is a difluoromethyl group, and Q is any of the substituents shown in [Table L1] to [Table L13] (hereinafter, referred to as a compound group SX1733).

A compound wherein in the compound (L-3), n is 2, $R^1$ is a methyl group, $R^2$ is a N-methylamino group, $R^{3b}$ is a difluoromethyl group, and Q is any of the substituents shown in [Table L1] to [Table L13] (hereinafter, referred to as a compound group SX1734).

A compound wherein in the compound (L-3), n is 0, $R^1$ is an ethyl group, $R^2$ is a N-methylamino group, $R^{3b}$ is a difluoromethyl group, and Q is any of the substituents shown in [Table L1] to [Table L13] (hereinafter, referred to as a compound group SX1735).

A compound wherein in the compound (L-3), n is 1, $R^1$ is an ethyl group, $R^2$ is a N-methylamino group, $R^{3b}$ is a difluoromethyl group, and Q is any of the substituents shown in [Table L1] to [Table L13] (hereinafter, referred to as a compound group SX1736).

A compound wherein in the compound (L-3), n is 2, $R^1$ is an ethyl group, $R^2$ is a N-methylamino group, $R^{3b}$ is a difluoromethyl group, and Q is any of the substituents shown in [Table L1] to [Table L13] (hereinafter, referred to as a compound group SX1737).

A compound wherein in the compound (L-3), n is 0, $R^1$ is a benzyl group, $R^2$ is a N-methylamino group, $R^{3b}$ is a difluoromethyl group, and Q is any of the substituents shown in [Table L1] to [Table L13] (hereinafter, referred to as a compound group SX1738).

A compound wherein in the compound (L-3), n is 1, $R^1$ is a benzyl group, $R^2$ is a N-methylamino group, $R^{3b}$ is a difluoromethyl group, and Q is any of the substituents shown in [Table L1] to [Table L13] (hereinafter, referred to as a compound group SX1739).

A compound wherein in the compound (L-3), n is 2, $R^1$ is a benzyl group, $R^2$ is a N-methylamino group, $R^{3b}$ is a difluoromethyl group, and Q is any of the substituents shown in [Table L1] to [Table L13] (hereinafter, referred to as a compound group SX1740).

A compound wherein in the compound (L-3), n is 0, $R^1$ is a methyl group, $R^2$ is a N-ethylamino group, $R^{3b}$ is a hydrogen atom, and Q is any of the substituents shown in [Table L1] to [Table L13] (hereinafter, referred to as a compound group SX1744).

A compound wherein in the compound (L-3), n is 1, $R^1$ is a methyl group, $R^2$ is a N-ethylamino group, $R^{3b}$ is a hydrogen atom, and Q is any of the substituents shown in [Table L1] to [Table L13] (hereinafter, referred to as a compound group SX1745).

A compound wherein in the compound (L-3), n is 2, $R^1$ is a methyl group, $R^2$ is a N-ethylamino group, $R^{3b}$ is a hydrogen atom, and Q is any of the substituents shown in [Table L1] to [Table L13] (hereinafter, referred to as a compound group SX1746).

A compound wherein in the compound (L-3), n is 0, $R^1$ is an ethyl group, $R^2$ is a N-ethylamino group, $R^{3b}$ is a hydrogen atom, and Q is any of the substituents shown in [Table L1] to [Table L13] (hereinafter, referred to as a compound group SX1747).

A compound wherein in the compound (L-3), n is 1, $R^1$ is an ethyl group, $R^2$ is a N-ethylamino group, $R^{3b}$ is a hydrogen atom, and Q is any of the substituents shown in [Table L1] to [Table L13] (hereinafter, referred to as a compound group SX1748).

A compound wherein in the compound (L-3), n is 2, $R^1$ is an ethyl group, $R^2$ is a N-ethylamino group, $R^{3b}$ is a hydrogen atom, and Q is any of the substituents shown in [Table L1] to [Table L13] (hereinafter, referred to as a compound group SX1749).

A compound wherein in the compound (L-3), n is 0, $R^1$ is a benzyl group, $R^2$ is a N-ethylamino group, $R^{3b}$ is a hydrogen atom, and Q is any of the substituents shown in [Table L1] to [Table L13] (hereinafter, referred to as a compound group SX1750).

A compound wherein in the compound (L-3), n is 1, $R^1$ is a benzyl group, $R^2$ is a N-ethylamino group, $R^{3b}$ is a hydrogen atom, and Q is any of the substituents shown in [Table L1] to [Table L13] (hereinafter, referred to as a compound group SX1751).

A compound wherein in the compound (L-3), n is 2, $R^1$ is a benzyl group, $R^2$ is a N-ethylamino group, $R^{3b}$ is a hydrogen atom, and Q is any of the substituents shown in [Table L1] to [Table L13] (hereinafter, referred to as a compound

group SX1752).

A compound wherein in the compound (L-3), n is 0, $R^1$ is a methyl group, $R^2$ is a N-methylamino group, $R^{3b}$ is a fluorine atom, and Q is any of the substituents shown in [Table L1] to [Table L13] (hereinafter, referred to as a compound group SX1756).

A compound wherein in the compound (L-3), n is 1, $R^1$ is a methyl group, $R^2$ is a N-methylamino group, $R^{3b}$ is a fluorine atom, and Q is any of the substituents shown in [Table L1] to [Table L13] (hereinafter, referred to as a compound group SX1757).

A compound wherein in the compound (L-3), n is 2, $R^1$ is a methyl group, $R^2$ is a N-methylamino group, $R^{3b}$ is a fluorine atom, and Q is any of the substituents shown in [Table L1] to [Table L13] (hereinafter, referred to as a compound group SX1758).

A compound wherein in the compound (L-3), n is 0, $R^1$ is an ethyl group, $R^2$ is a N-methylamino group, $R^{3b}$ is a fluorine atom, and Q is any of the substituents shown in [Table L1] to [Table L13] (hereinafter, referred to as a compound group SX1759).

A compound wherein in the compound (L-3), n is 1, $R^1$ is an ethyl group, $R^2$ is a N-methylamino group, $R^{3b}$ is a fluorine atom, and Q is any of the substituents shown in [Table L1] to [Table L13] (hereinafter, referred to as a compound group SX1760).

A compound wherein in the compound (L-3), n is 2, $R^1$ is an ethyl group, $R^2$ is a N-methylamino group, $R^{3b}$ is a fluorine atom, and Q is any of the substituents shown in [Table L1] to [Table L13] (hereinafter, referred to as a compound group SX1761).

A compound wherein in the compound (L-3), n is 0, $R^1$ is a benzyl group, $R^2$ is a N-ethylamino group, $R^{3b}$ is a fluorine atom, and Q is any of the substituents shown in [Table L1] to [Table L13] (hereinafter, referred to as a compound group SX1762).

A compound wherein in the compound (L-3), n is 1, $R^1$ is a benzyl group, $R^2$ is a N-ethylamino group, $R^{3b}$ is a fluorine atom, and Q is any of the substituents shown in [Table L1] to [Table L13] (hereinafter, referred to as a compound group SX1763).

A compound wherein in the compound (L-3), n is 2, $R^1$ is a benzyl group, $R^2$ is a N-ethylamino group, $R^{3b}$ is a fluorine atom, and Q is any of the substituents shown in [Table L1] to [Table L13] (hereinafter, referred to as a compound group SX1764).

A compound wherein in the compound (L-3), n is 0, $R^1$ is a methyl group, $R^2$ is a N-ethylamino group, $R^{3b}$ is a chlorine atom, and Q is any of the substituents shown in [Table L1] to [Table L13] (hereinafter, referred to as a compound group SX1768).

A compound wherein in the compound (L-3), n is 1, $R^1$ is a methyl group, $R^2$ is a N-ethylamino group, $R^{3b}$ is a chlorine atom, and Q is any of the substituents shown in [Table L1] to [Table L13] (hereinafter, referred to as a compound group SX1769).

A compound wherein in the compound (L-3), n is 2, $R^1$ is a methyl group, $R^2$ is a N-ethylamino group, $R^{3b}$ is a chlorine atom, and Q is any of the substituents shown in [Table L1] to [Table L13] (hereinafter, referred to as a compound group SX1770).

A compound wherein in the compound (L-3), n is 0, $R^1$ is an ethyl group, $R^2$ is a N-ethylamino group, $R^{3b}$ is a chlorine atom, and Q is any of the substituents shown in [Table L1] to [Table L13] (hereinafter, referred to as a compound group SX1771).

A compound wherein in the compound (L-3), n is 1, $R^1$ is an ethyl group, $R^2$ is a N-ethylamino group, $R^{3b}$ is a chlorine atom, and Q is any of the substituents shown in [Table L1] to [Table L13] (hereinafter, referred to as a compound group SX1772).

A compound wherein in the compound (L-3), n is 2, $R^1$ is an ethyl group, $R^2$ is a N-ethylamino group, $R^{3b}$ is a chlorine atom, and Q is any of the substituents shown in [Table L1] to [Table L13] (hereinafter, referred to as a compound group SX1773).

A compound wherein in the compound (L-3), n is 0, $R^1$ is a benzyl group, $R^2$ is a N-ethylamino group, $R^{3b}$ is a chlorine atom, and Q is any of the substituents shown in [Table L1] to [Table L13] (hereinafter, referred to as a compound group SX1774).

A compound wherein in the compound (L-3), n is 1, $R^1$ is a benzyl group, $R^2$ is a N-ethylamino group, $R^{3b}$ is a chlorine atom, and Q is any of the substituents shown in [Table L1] to [Table L13] (hereinafter, referred to as a compound group SX1775).

A compound wherein in the compound (L-3), n is 2, $R^1$ is a benzyl group, $R^2$ is a N-ethylamino group, $R^{3b}$ is a chlorine atom, and Q is any of the substituents shown in [Table L1] to [Table L13] (hereinafter, referred to as a compound group SX1776).

A compound wherein in the compound (L-3), n is 0, $R^1$ is a methyl group, $R^2$ is a N-ethylamino group, $R^{3b}$ is a bromine atom, and Q is any of the substituents shown in [Table L1] to [Table L13] (hereinafter, referred to as a compound group SX1780).

A compound wherein in the compound (L-3), n is 1, $R^1$ is a methyl group, $R^2$ is a N-ethylamino group, $R^{3b}$ is a bromine atom, and Q is any of the substituents shown in [Table L1] to [Table L13] (hereinafter, referred to as a compound group SX1781).

A compound wherein in the compound (L-3), n is 2, $R^1$ is a methyl group, $R^2$ is a N-ethylamino group, $R^{3b}$ is a bromine atom, and Q is any of the substituents shown in [Table L1] to [Table L13] (hereinafter, referred to as a compound group SX1782).

A compound wherein in the compound (L-3), n is 0, $R^1$ is an ethyl group, $R^2$ is a N-ethylamino group, $R^{3b}$ is a bromine atom, and Q is any of the substituents shown in [Table L1] to [Table L13] (hereinafter, referred to as a compound group SX1783).

A compound wherein in the compound (L-3), n is 1, $R^1$ is an ethyl group, $R^2$ is a N-ethylamino group, $R^{3b}$ is a bromine atom, and Q is any of the substituents shown in [Table L1] to [Table L13] (hereinafter, referred to as a compound group SX1784).

A compound wherein in the compound (L-3), n is 2, $R^1$ is an ethyl group, $R^2$ is a N-ethylamino group, $R^{3b}$ is a bromine atom, and Q is any of the substituents shown in [Table L1] to [Table L13] (hereinafter, referred to as a compound group SX1785).

A compound wherein in the compound (L-3), n is 0, $R^1$ is a benzyl group, $R^2$ is a N-ethylamino group, $R^{3b}$ is a bromine atom, and Q is any of the substituents shown in [Table L1] to [Table L13] (hereinafter, referred to as a compound group SX1786).

A compound wherein in the compound (L-3), n is 1, $R^1$ is a benzyl group, $R^2$ is a N-ethylamino group, $R^{3b}$ is a bromine atom, and Q is any of the substituents shown in [Table L1] to [Table L13] (hereinafter, referred to as a compound group SX1787).

A compound wherein in the compound (L-3), n is 2, $R^1$ is a benzyl group, $R^2$ is a N-ethylamino group, $R^{3b}$ is a bromine atom, and Q is any of the substituents shown in [Table L1] to [Table L13] (hereinafter, referred to as a compound group SX1788).

A compound wherein in the compound (L-3), n is 0, $R^1$ is a methyl group, $R^2$ is a N-ethylamino group, $R^{3b}$ is an iodine atom, and Q is any of the substituents shown in [Table L1] to [Table L13] (hereinafter, referred to as a compound group SX1792).

A compound wherein in the compound (L-3), n is 1, $R^1$ is a methyl group, $R^2$ is a N-ethylamino group, $R^{3b}$ is an iodine atom, and Q is any of the substituents shown in [Table L1] to [Table L13] (hereinafter, referred to as a compound group SX1793).

A compound wherein in the compound (L-3), n is 2, $R^1$ is a methyl group, $R^2$ is a N-ethylamino group, $R^{3b}$ is an iodine atom, and Q is any of the substituents shown in [Table L1] to [Table L13] (hereinafter, referred to as a compound group SX1794).

A compound wherein in the compound (L-3), n is 0, $R^1$ is an ethyl group, $R^2$ is a N-ethylamino group, $R^{3b}$ is an iodine atom, and Q is any of the substituents shown in [Table L1] to [Table L13] (hereinafter, referred to as a compound group SX1795).

A compound wherein in the compound (L-3), n is 1, $R^1$ is an ethyl group, $R^2$ is a N-ethylamino group, $R^{3b}$ is an iodine atom, and Q is any of the substituents shown in [Table L1] to [Table L13] (hereinafter, referred to as a compound group SX1796).

A compound wherein in the compound (L-3), n is 2, $R^1$ is an ethyl group, $R^2$ is a N-ethylamino group, $R^{3b}$ is an iodine atom, and Q is any of the substituents shown in [Table L1] to [Table L13] (hereinafter, referred to as a compound group SX1797).

A compound wherein in the compound (L-3), n is 0, $R^1$ is a benzyl group, $R^2$ is a N-ethylamino group, $R^{3b}$ is an iodine atom, and Q is any of the substituents shown in [Table L1] to [Table L13] (hereinafter, referred to as a compound group SX1798).

A compound wherein in the compound (L-3), n is 1, $R^1$ is a benzyl group, $R^2$ is a N-ethylamino group, $R^{3b}$ is an iodine atom, and Q is any of the substituents shown in [Table L1] to [Table L13] (hereinafter, referred to as a compound group SX1799).

A compound wherein in the compound (L-3), n is 2, $R^1$ is a benzyl group, $R^2$ is a N-ethylamino group, $R^{3b}$ is an iodine atom, and Q is any of the substituents shown in [Table L1] to [Table L13] (hereinafter, referred to as a compound group SX1800).

A compound wherein in the compound (L-3), n is 0, $R^1$ is a methyl group, $R^2$ is a N-ethylamino group, $R^{3b}$ is a trifluoromethyl group, and Q is any of the substituents shown in [Table L1] to [Table L13] (hereinafter, referred to as a compound group SX1804).

A compound wherein in the compound (L-3), n is 1, $R^1$ is a methyl group, $R^2$ is a N-ethylamino group, $R^{3b}$ is a trifluoromethyl group, and Q is any of the substituents shown in [Table L1] to [Table L13] (hereinafter, referred to as a compound group SX1805).

A compound wherein in the compound (L-3), n is 2, $R^1$ is a methyl group, $R^2$ is a N-ethylamino group, $R^{3b}$ is a

trifluoromethyl group, and Q is any of the substituents shown in [Table L1] to [Table L13] (hereinafter, referred to as a compound group SX1806).

A compound wherein in the compound (L-3), n is 0, $R^1$ is an ethyl group, $R^2$ is a N-ethylamino group, $R^{3b}$ is a trifluoromethyl group, and Q is any of the substituents shown in [Table L1] to [Table L13] (hereinafter, referred to as a compound group SX1807).

A compound wherein in the compound (L-3), n is 1, $R^1$ is an ethyl group, $R^2$ is a N-ethylamino group, $R^{3b}$ is a trifluoromethyl group, and Q is any of the substituents shown in [Table L1] to [Table L13] (hereinafter, referred to as a compound group SX1808).

A compound wherein in the compound (L-3), n is 2, $R^1$ is an ethyl group, $R^2$ is a N-ethylamino group, $R^{3b}$ is a trifluoromethyl group, and Q is any of the substituents shown in [Table L1] to [Table L13] (hereinafter, referred to as a compound group SX1809).

A compound wherein in the compound (L-3), n is 0, $R^1$ is a benzyl group, $R^2$ is a N-ethylamino group, $R^{3b}$ is a trifluoromethyl group, and Q is any of the substituents shown in [Table L1] to [Table L13] (hereinafter, referred to as a compound group SX1810).

A compound wherein in the compound (L-3), n is 1, $R^1$ is a benzyl group, $R^2$ is a N-ethylamino group, $R^{3b}$ is a trifluoromethyl group, and Q is any of the substituents shown in [Table L1] to [Table L13] (hereinafter, referred to as a compound group SX1811).

A compound wherein in the compound (L-3), n is 2, $R^1$ is a benzyl group, $R^2$ is a N-ethylamino group, $R^{3b}$ is a trifluoromethyl group, and Q is any of the substituents shown in [Table L1] to [Table L13] (hereinafter, referred to as a compound group SX1812).

A compound wherein in the compound (L-3), n is 0, $R^1$ is a methyl group, $R^2$ is a N-ethylamino group, $R^{3b}$ is a difluoromethyl group, and Q is any of the substituents shown in [Table L1] to [Table L13] (hereinafter, referred to as a compound group SX1816).

A compound wherein in the compound (L-3), n is 1, $R^1$ is a methyl group, $R^2$ is a N-ethylamino group, $R^{3b}$ is a difluoromethyl group, and Q is any of the substituents shown in [Table L1] to [Table L13] (hereinafter, referred to as a compound group SX1817).

A compound wherein in the compound (L-3), n is 2, $R^1$ is a methyl group, $R^2$ is a N-ethylamino group, $R^{3b}$ is a difluoromethyl group, and Q is any of the substituents shown in [Table L1] to [Table L13] (hereinafter, referred to as a compound group SX1818).

A compound wherein in the compound (L-3), n is 0, $R^1$ is an ethyl group, $R^2$ is a N-ethylamino group, $R^{3b}$ is a difluoromethyl group, and Q is any of the substituents shown in [Table L1] to [Table L13] (hereinafter, referred to as a compound group SX1819).

A compound wherein in the compound (L-3), n is 1, $R^1$ is an ethyl group, $R^2$ is a N-ethylamino group, $R^{3b}$ is a difluoromethyl group, and Q is any of the substituents shown in [Table L1] to [Table L13] (hereinafter, referred to as a compound group SX1820).

A compound wherein in the compound (L-3), n is 2, $R^1$ is an ethyl group, $R^2$ is a N-ethylamino group, $R^{3b}$ is a difluoromethyl group, and Q is any of the substituents shown in [Table L1] to [Table L13] (hereinafter, referred to as a compound group SX1821).

A compound wherein in the compound (L-3), n is 0, $R^1$ is a benzyl group, $R^2$ is a N-ethylamino group, $R^{3b}$ is a difluoromethyl group, and Q is any of the substituents shown in [Table L1] to [Table L13] (hereinafter, referred to as a compound group SX1822).

A compound wherein in the compound (L-3), n is 1, $R^1$ is a benzyl group, $R^2$ is a N-ethylamino group, $R^{3b}$ is a difluoromethyl group, and Q is any of the substituents shown in [Table L1] to [Table L13] (hereinafter, referred to as a compound group SX1823).

A compound wherein in the compound (L-3), n is 2, $R^1$ is a benzyl group, $R^2$ is a N-ethylamino group, $R^{3b}$ is a difluoromethyl group, and Q is any of the substituents shown in [Table L1] to [Table L13] (hereinafter, referred to as a compound group SX1824).

A compound wherein in the compound (L-3), n is 0, $R^1$ is a methyl group, $R^2$ is an amino group, $R^{3b}$ is a hydrogen atom, and Q is any of the substituents shown in [Table L1] to [Table L13] (hereinafter, referred to as a compound group SX1828).

A compound wherein in the compound (L-3), n is 1, $R^1$ is a methyl group, $R^2$ is an amino group, $R^{3b}$ is a hydrogen atom, and Q is any of the substituents shown in [Table L1] to [Table L13] (hereinafter, referred to as a compound group SX1829).

A compound wherein in the compound (L-3), n is 2, $R^1$ is a methyl group, $R^2$ is an amino group, $R^{3b}$ is a hydrogen atom, and Q is any of the substituents shown in [Table L1] to [Table L13] (hereinafter, referred to as a compound group SX1830).

A compound wherein in the compound (L-3), n is 0, $R^1$ is an ethyl group, $R^2$ is an amino group, $R^{31}$) is a hydrogen atom, and Q is any of the substituents shown in [Table L1] to [Table L13] (hereinafter, referred to as a compound group

SX1831).

A compound wherein in the compound (L-3), n is 1, $R^1$ is an ethyl group, $R^2$ is an amino group, $R^{3b}$ is a hydrogen atom, and Q is any of the substituents shown in [Table L1] to [Table L13] (hereinafter, referred to as a compound group SX1832).

A compound wherein in the compound (L-3), n is 2, $R^1$ is an ethyl group, $R^2$ is an amino group, $R^{3b}$ is a hydrogen atom, and Q is any of the substituents shown in [Table L1] to [Table L13] (hereinafter, referred to as a compound group SX1833).

A compound wherein in the compound (L-3), n is 0, $R^1$ is a benzyl group, $R^2$ is an amino group, $R^{3b}$ is a hydrogen atom, and Q is any of the substituents shown in [Table L1] to [Table L13] (hereinafter, referred to as a compound group SX1834).

A compound wherein in the compound (L-3), n is 1, $R^1$ is a benzyl group, $R^2$ is an amino group, $R^{3b}$ is a hydrogen atom, and Q is any of the substituents shown in [Table L1] to [Table L13] (hereinafter, referred to as a compound group SX1835).

A compound wherein in the compound (L-3), n is 2, $R^1$ is a benzyl group, $R^2$ is an amino group, $R^{3b}$ is a hydrogen atom, and Q is any of the substituents shown in [Table L1] to [Table L13] (hereinafter, referred to as a compound group SX1836).

A compound wherein in the compound (L-3), n is 0, $R^1$ is a methyl group, $R^2$ is an amino group, $R^{3b}$ is a fluorine atom, and Q is any of the substituents shown in [Table L1] to [Table L13] (hereinafter, referred to as a compound group SX1840).

A compound wherein in the compound (L-3), n is 1, $R^1$ is a methyl group, $R^2$ is an amino group, $R^{3b}$ is a fluorine atom, and Q is any of the substituents shown in [Table L1] to [Table L13] (hereinafter, referred to as a compound group SX1841).

A compound wherein in the compound (L-3), n is 2, $R^1$ is a methyl group, $R^2$ is an amino group, $R^{3b}$ is a fluorine atom, and Q is any of the substituents shown in [Table L1] to [Table L13] (hereinafter, referred to as a compound group SX1842).

A compound wherein in the compound (L-3), n is 0, $R^1$ is an ethyl group, $R^2$ is an amino group, $R^{3b}$ is a fluorine atom, and Q is any of the substituents shown in [Table L1] to [Table L13] (hereinafter, referred to as a compound group SX1843).

A compound wherein in the compound (L-3), n is 1, $R^1$ is an ethyl group, $R^2$ is an amino group, $R^{3b}$ is a fluorine atom, and Q is any of the substituents shown in [Table L1] to [Table L13] (hereinafter, referred to as a compound group SX1844).

A compound wherein in the compound (L-3), n is 2, $R^1$ is an ethyl group, $R^2$ is an amino group, $R^{3b}$ is a fluorine atom, and Q is any of the substituents shown in [Table L1] to [Table L13] (hereinafter, referred to as a compound group SX1845).

A compound wherein in the compound (L-3), n is 0, $R^1$ is a benzyl group, $R^2$ is an amino group, $R^{3b}$ is a fluorine atom, and Q is any of the substituents shown in [Table L1] to [Table L13] (hereinafter, referred to as a compound group SX1846).

A compound wherein in the compound (L-3), n is 1, $R^1$ is a benzyl group, $R^2$ is an amino group, $R^{3b}$ is a fluorine atom, and Q is any of the substituents shown in [Table L1] to [Table L13] (hereinafter, referred to as a compound group SX1847).

A compound wherein in the compound (L-3), n is 2, $R^1$ is a benzyl group, $R^2$ is an amino group, $R^{3b}$ is a fluorine atom, and Q is any of the substituents shown in [Table L1] to [Table L13] (hereinafter, referred to as a compound group SX1848).

A compound wherein in the compound (L-3), n is 0, $R^1$ is a methyl group, $R^2$ is an amino group, $R^{3b}$ is a chlorine atom, and Q is any of the substituents shown in [Table L1] to [Table L13] (hereinafter, referred to as a compound group SX1852).

A compound wherein in the compound (L-3), n is 1, $R^1$ is a methyl group, $R^2$ is an amino group, $R^{3b}$ is a chlorine atom, and Q is any of the substituents shown in [Table L1] to [Table L13] (hereinafter, referred to as a compound group SX1853).

A compound wherein in the compound (L-3), n is 2, $R^1$ is a methyl group, $R^2$ is an amino group, $R^{3b}$ is a chlorine atom, and Q is any of the substituents shown in [Table L1] to [Table L13] (hereinafter, referred to as a compound group SX1854).

A compound wherein in the compound (L-3), n is 0, $R^1$ is an ethyl group, $R^2$ is an amino group, $R^{3b}$ is a chlorine atom, and Q is any of the substituents shown in [Table L1] to [Table L13] (hereinafter, referred to as a compound group SX1855).

A compound wherein in the compound (L-3), n is 1, $R^1$ is an ethyl group, $R^2$ is an amino group, $R^{3b}$ is a chlorine atom, and Q is any of the substituents shown in [Table L1] to [Table L13] (hereinafter, referred to as a compound group SX1856).

A compound wherein in the compound (L-3), n is 2, $R^1$ is an ethyl group, $R^2$ is an amino group, $R^{3b}$ is a chlorine atom, and Q is any of the substituents shown in [Table L1] to [Table L13] (hereinafter, referred to as a compound group SX1857).

A compound wherein in the compound (L-3), n is 0, $R^1$ is a benzyl group, $R^2$ is an amino group, $R^{3b}$ is a chlorine atom, and Q is any of the substituents shown in [Table L1] to [Table L13] (hereinafter, referred to as a compound group SX1858).

A compound wherein in the compound (L-3), n is 1, $R^1$ is a benzyl group, $R^2$ is an amino group, $R^{3b}$ is a chlorine atom, and Q is any of the substituents shown in [Table L1] to [Table L13] (hereinafter, referred to as a compound group SX1859).

A compound wherein in the compound (L-3), n is 2, $R^1$ is a benzyl group, $R^2$ is an amino group, $R^{3b}$ is a chlorine atom, and Q is any of the substituents shown in [Table L1] to [Table L13] (hereinafter, referred to as a compound group SX1860).

A compound wherein in the compound (L-3), n is 0, $R^1$ is a methyl group, $R^2$ is an amino group, $R^{3b}$ is a bromine atom, and Q is any of the substituents shown in [Table L1] to [Table L13] (hereinafter, referred to as a compound group SX1864).

A compound wherein in the compound (L-3), n is 1, $R^1$ is a methyl group, $R^2$ is an amino group, $R^{3b}$ is a bromine atom, and Q is any of the substituents shown in [Table L1] to [Table L13] (hereinafter, referred to as a compound group SX1865).

A compound wherein in the compound (L-3), n is 2, $R^1$ is a methyl group, $R^2$ is an amino group, $R^{3b}$ is a bromine atom, and Q is any of the substituents shown in [Table L1] to [Table L13] (hereinafter, referred to as a compound group SX1866).

A compound wherein in the compound (L-3), n is 0, $R^1$ is an ethyl group, $R^2$ is an amino group, $R^{3b}$ is a bromine atom, and Q is any of the substituents shown in [Table L1] to [Table L13] (hereinafter, referred to as a compound group SX1867).

A compound wherein in the compound (L-3), n is 1, $R^1$ is an ethyl group, $R^2$ is an amino group, $R^{3b}$ is a bromine atom, and Q is any of the substituents shown in [Table L1] to [Table L13] (hereinafter, referred to as a compound group SX1868).

A compound wherein in the compound (L-3), n is 2, $R^1$ is an ethyl group, $R^2$ is an amino group, $R^{3b}$ is a bromine atom, and Q is any of the substituents shown in [Table L1] to [Table L13] (hereinafter, referred to as a compound group SX1869).

A compound wherein in the compound (L-3), n is 0, $R^1$ is a benzyl group, $R^2$ is an amino group, $R^{3b}$ is a bromine atom, and Q is any of the substituents shown in [Table L1] to [Table L13] (hereinafter, referred to as a compound group SX1870).

A compound wherein in the compound (L-3), n is 1, $R^1$ is a benzyl group, $R^2$ is an amino group, $R^{3b}$ is a bromine atom, and Q is any of the substituents shown in [Table L1] to [Table L13] (hereinafter, referred to as a compound group SX1871).

A compound wherein in the compound (L-3), n is 2, $R^1$ is a benzyl group, $R^2$ is an amino group, $R^{3b}$ is a bromine atom, and Q is any of the substituents shown in [Table L1] to [Table L13] (hereinafter, referred to as a compound group SX1872).

A compound wherein in the compound (L-3), n is 0, $R^1$ is a methyl group, $R^2$ is an amino group, $R^{3b}$ is an iodine atom, and Q is any of the substituents shown in [Table L1] to [Table L13] (hereinafter, referred to as a compound group SX1876).

A compound wherein in the compound (L-3), n is 1, $R^1$ is a methyl group, $R^2$ is an amino group, $R^{3b}$ is an iodine atom, and Q is any of the substituents shown in [Table L1] to [Table L13] (hereinafter, referred to as a compound group SX1877).

A compound wherein in the compound (L-3), n is 2, $R^1$ is a methyl group, $R^2$ is an amino group, $R^{3b}$ is an iodine atom, and Q is any of the substituents shown in [Table L1] to [Table L13] (hereinafter, referred to as a compound group SX1878).

A compound wherein in the compound (L-3), n is 0, $R^1$ is an ethyl group, $R^2$ is an amino group, $R^{3b}$ is an iodine atom, and Q is any of the substituents shown in [Table L1] to [Table L13] (hereinafter, referred to as a compound group SX1879).

A compound wherein in the compound (L-3), n is 1, $R^1$ is an ethyl group, $R^2$ is an amino group, $R^{3b}$ is an iodine atom, and Q is any of the substituents shown in [Table L1] to [Table L13] (hereinafter, referred to as a compound group SX1880).

A compound wherein in the compound (L-3), n is 2, $R^1$ is an ethyl group, $R^2$ is an amino group, $R^{3b}$ is an iodine atom, and Q is any of the substituents shown in [Table L1] to [Table L13] (hereinafter, referred to as a compound group SX1881).

A compound wherein in the compound (L-3), n is 0, $R^1$ is a benzyl group, $R^2$ is an amino group, $R^{3b}$ is an iodine

atom, and Q is any of the substituents shown in [Table L1] to [Table L13] (hereinafter, referred to as a compound group SX1882).

A compound wherein in the compound (L-3), n is 1, $R^1$ is a benzyl group, $R^2$ is an amino group, $R^{3b}$ is an iodine atom, and Q is any of the substituents shown in [Table L1] to [Table L13] (hereinafter, referred to as a compound group SX1883).

A compound wherein in the compound (L-3), n is 2, $R^1$ is a benzyl group, $R^2$ is an amino group, $R^{3b}$ is an iodine atom, and Q is any of the substituents shown in [Table L1] to [Table L13] (hereinafter, referred to as a compound group SX1884).

A compound wherein in the compound (L-3), n is 0, $R^1$ is a methyl group, $R^2$ is an amino group, $R^{3b}$ is a trifluoromethyl group, and Q is any of the substituents shown in [Table L1] to [Table L13] (hereinafter, referred to as a compound group SX1888).

A compound wherein in the compound (L-3), n is 1, $R^1$ is a methyl group, $R^2$ is an amino group, $R^{3b}$ is a trifluoromethyl group, and Q is any of the substituents shown in [Table L1] to [Table L13] (hereinafter, referred to as a compound group SX1889).

A compound wherein in the compound (L-3), n is 2, $R^1$ is a methyl group, $R^2$ is an amino group, $R^{3b}$ is a trifluoromethyl group, and Q is any of the substituents shown in [Table L1] to [Table L13] (hereinafter, referred to as a compound group SX1890).

A compound wherein in the compound (L-3), n is 0, $R^1$ is an ethyl group, $R^2$ is an amino group, $R^{3b}$ is a trifluoromethyl group, and Q is any of the substituents shown in [Table L1] to [Table L13] (hereinafter, referred to as a compound group SX1891).

A compound wherein in the compound (L-3), n is 1, $R^1$ is an ethyl group, $R^2$ is an amino group, $R^{3b}$ is a trifluoromethyl group, and Q is any of the substituents shown in [Table L1] to [Table L13] (hereinafter, referred to as a compound group SX1892).

A compound wherein in the compound (L-3), n is 2, $R^1$ is an ethyl group, $R^2$ is an amino group, $R^{3b}$ is a trifluoromethyl group, and Q is any of the substituents shown in [Table L1] to [Table L13] (hereinafter, referred to as a compound group SX1893).

A compound wherein in the compound (L-3), n is 0, $R^1$ is a benzyl group, $R^2$ is an amino group, $R^{3b}$ is a trifluoromethyl group, and Q is any of the substituents shown in [Table L1] to [Table L13] (hereinafter, referred to as a compound group SX1894).

A compound wherein in the compound (L-3), n is 1, $R^1$ is a benzyl group, $R^2$ is an amino group, $R^{3b}$ is a trifluoromethyl group, and Q is any of the substituents shown in [Table L1] to [Table L13] (hereinafter, referred to as a compound group SX1895).

A compound wherein in the compound (L-3), n is 2, $R^1$ is a benzyl group, $R^2$ is an amino group, $R^{3b}$ is a trifluoromethyl group, and Q is any of the substituents shown in [Table L1] to [Table L13] (hereinafter, referred to as a compound group SX1896).

A compound wherein in the compound (L-3), n is 0, $R^1$ is a methyl group, $R^2$ is an amino group, $R^{3b}$ is a difluoromethyl group, and Q is any of the substituents shown in [Table L1] to [Table L13] (hereinafter, referred to as a compound group SX1900).

A compound wherein in the compound (L-3), n is 1, $R^1$ is a methyl group, $R^2$ is an amino group, $R^{3b}$ is a difluoromethyl group, and Q is any of the substituents shown in [Table L1] to [Table L13] (hereinafter, referred to as a compound group SX1901).

A compound wherein in the compound (L-3), n is 2, $R^1$ is a methyl group, $R^2$ is an amino group, $R^{3b}$ is a difluoromethyl group, and Q is any of the substituents shown in [Table L1] to [Table L13] (hereinafter, referred to as a compound group SX1902).

A compound wherein in the compound (L-3), n is 0, $R^1$ is an ethyl group, $R^2$ is an amino group, $R^{3b}$ is a difluoromethyl group, and Q is any of the substituents shown in [Table L1] to [Table L13] (hereinafter, referred to as a compound group SX1903).

A compound wherein in the compound (L-3), n is 1, $R^1$ is an ethyl group, $R^2$ is an amino group, $R^{3b}$ is a difluoromethyl group, and Q is any of the substituents shown in [Table L1] to [Table L13] (hereinafter, referred to as a compound group SX1904).

A compound wherein in the compound (L-3), n is 2, $R^1$ is an ethyl group, $R^2$ is an amino group, $R^{3b}$ is a difluoromethyl group, and Q is any of the substituents shown in [Table L1] to [Table L13] (hereinafter, referred to as a compound group SX1905).

A compound wherein in the compound (L-3), n is 0, $R^1$ is a benzyl group, $R^2$ is an amino group, $R^{3b}$ is a difluoromethyl group, and Q is any of the substituents shown in [Table L1] to [Table L13] (hereinafter, referred to as a compound group SX1906).

A compound wherein in the compound (L-3), n is 1, $R^1$ is a benzyl group, $R^2$ is an amino group, $R^{3b}$ is a difluoromethyl group, and Q is any of the substituents shown in [Table L1] to [Table L13] (hereinafter, referred to as a

compound group SX1907).

A compound wherein in the compound (L-3), n is 2, $R^1$ is a benzyl group, $R^2$ is an amino group, $R^{3b}$ is a difluoromethyl group, and Q is any of the substituents shown in [Table L1] to [Table L13] (hereinafter, referred to as a compound group SX1908).

Then, formulation examples of the present compound will be described. Parts represent parts by weight. In addition, the present compound S represents a compound described in the compound groups SX1 to SX1908.

Formulation Example 1

35 parts of a mixture of polyoxyethylene alkyl ether sulfate ammonium salt and silica (weight ratio 1 : 1), 10 parts of any one of the present compounds S, and 55 parts of water are mixed, and finely pulverized by wet grinding method to provide a formulation.

Formulation Example 2

50 parts of any one of the present compounds S, 3 parts of calcium lignosulfonate, 2 parts of sodium lauryl sulfate, and 45 parts of silica are pulverized and mixed to provide a formulation.

Formulation Example 3

5 parts of any one of the present compounds S, 9 parts of polyoxyethylene styrylphenyl ether, 5 parts of polyoxyethylene decyl ether (addition number of ethylene oxide: 5), 6 parts of calcium dodecylbenzene sulfonate, and 75 parts of xylene are mixed to provide a formulation.

Formulation Example 4

2 parts of any one of the present compound S, 1 part of silica, 2 parts of calcium lignosulfonate, 30 parts of bentonite, and 65 parts of kaolin clay are pulverized and mixed, an appropriate amount of water is added thereto, the mixture is kneaded, granulated by a granulator, and then dried to provide a formulation.

Formulation Example 5

10 parts of any one of the present compound S is mixed with a mixture of 18 parts of benzyl alcohol and 9 parts of DMSO, 6.3 parts of GERONOL (registered trademark) TE250, 2.7 parts of Ethylan (registered trademark) NS-500LQ, and 54 parts of solvent naphtha are added thereto, and the mixture is mixed to provide a formulation.

Formulation Example 6

0.1 parts of any one of the present compound S and 39.9 parts of kerosene are mixed and dissolved, and placed in an aerosol container, and filled with 60 parts of liquefied petroleum gas (mixture of propane, butane, and isobutane and saturated vapor pressure: 0.47 MPa (25°C)) to provide a formulation.

Formulation Example 7

0.2 parts of any one of the present compound S, 50 parts of pyrethrum extract cake powder, 30 parts of tab powder, and 19.8 parts of wood powder are mixed, an appropriate amount of water is added thereto, and the mixture is kneaded, then subjected to an extruder to form a plate-shaped sheet, and formed into a spiral shape by a punching machine, thereby providing a formulation.

Then, the efficacy of the present compound against harmful arthropod is shown by Test Examples. In the following Test Examples, the test was performed at 25°C.

Test method 1

A test compound is formed into a formulation according to the method described in Formulation Example 1, and water containing 0.03% by volume of Shindain (registered trademark) is added thereto to prepare a diluted solution containing the test compound at a predetermined concentration.

A cucumber (Cucumis sativus) seedling (second true leaf stage) planted in a container is inoculated with about 30 cotton aphids (*Aphis gossypii*) (whole stage). One day later, the diluted solution is sprayed onto the seedlings at a rate of 10

mL/seedling. After 5 days, the number of surviving insects is examined, and the control value is determined by the following formula.

Controlling value (%) = {1 - (Cb × Tai)/(Cai × Tb)} × 100

Characters in the formula represent the following meanings.
Cb: number of test insects in a non-treated section
Cai: number of surviving insects in a non-treated section at the time of investigation
Tb: number of test insects in a treated section
Tai: number of surviving insects in a treated section at the time of investigation

Herein, the non-treated section means a section that is subjected to the same operation as that of the treated section except that the test compound is not used.

Test Example 1-1

As a result of performing a test according to test method 1 using the following compound of the present invention as a test compound at a predetermined concentration of 500 ppm, the following present compounds all exhibited a control value of 90% or more.

Present compound: 2, 3, 5, 6, 7, 8, 9, 10, 11, 13, 15, 16, 17, 19, 23, 24, 27, 30, 39, 40, 41, 42, 43, 44, 45, 46, 47, 48, 52, 53, 56, 57, 60, 61, 63, 64, and 67

Test method 2

The test compound is formulated according to the method described in Formulation Example 5, and water is added thereto to prepare a diluted solution containing the test compound at a predetermined concentration.

A cucumber (Cucumis sativus) seedling (second true leaf stage) planted in a container is irrigated with the diluted solution at a rate of 5 mL/seedling. After 7 days, the leaf surface of this seedling is inoculated with about 30 Aphis gossypii (whole stage). After 6 days, the number of surviving insects is examined, and the control value is determined by the following formula.

Controlling value (%) = {1 - (Cb × Tai)/(Cai × Tb)} × 100

Characters in the formula represent the following meanings.
Cb: number of test insects in a non-treated section
Cai: number of surviving insects in a non-treated section at the time of investigation
Tb: number of test insects in a treated section
Tai: number of surviving insects in a treated section at the time of investigation

Herein, the non-treated section means a section that is subjected to the same operation as that of the treated section except that the test compound is not used.

Test Example 2-1

As a result of performing a test according to test method 2 using the following compound of the present invention as a test compound at a predetermined concentration of 1000 ppm, the following present compounds all exhibited a control value of 90% or more.

Present compound: 7, 11, 16, 39, 40, 41, 42, 43, 44, 46, 47, 52, 56, 64, and 67

Test method 3

A test compound is formed into a formulation according to the method described in Formulation Example 1, and water containing 0.03% by volume of Shindain (registered trademark) is added thereto to prepare a diluted solution containing the test compound at a predetermined concentration.

A cabbage (Brassicae oleracea) seedling (2 to 3 true leaf stage) planted in a container is sprayed with the diluted

solution at a rate of 20 mL/seedling. Thereafter, the foliage portion of the seedling is cut out and placed in a container on which filter paper is laid. Thereto are released five second instar larvae of common cutworm *(Spodoptera litura).* After 5 days, the number of surviving insects is counted, and the mortality is determined from the following equation.

Mortality (%) = (1 - number of surviving insects/5) × 100

Test Example 3-1

As a result of performing a test according to test method 3 using the following compound of the present invention as a test compound at a predetermined concentration of 500 ppm, the following present compounds all exhibited a mortality of 80% or more.
Present compound: 6, 16, 40, and 48

Test method 4

A test compound is formed into a formulation according to the method described in Formulation Example 1, and water containing 0.03% by volume of Shindain (registered trademark) is added thereto to prepare a diluted solution containing the test compound at a predetermined concentration.
A cabbage *(Brassicae oleracea)* seedling (2 to 3 true leaf stage) planted in a container is sprayed with the diluted solution at a rate of 20 mL/seedling. Thereafter, the foliage portion of the seedling is cut out and placed in a container on which filter paper is laid. Thereto are released five second instar larvae of diamondback moth *(Plutella xylostella).* After 5 days, the number of surviving insects is counted, and the mortality is determined from the following equation.

Mortality (%) = (1 - number of surviving insects/5) × 100

Test Example 4-1

As a result of performing a test according to test method 4 using the following compound of the present invention as a test compound at a predetermined concentration of 500 ppm, the following present compounds all exhibited a mortality of 80% or more.
Present compound: 3, 5, 6, 7, 8, 9, 10, 11, 13, 16, 23, 24, 39, 40, 41, 42, 43, 44, 46, 47, 48, 50, 52, 53, 57, 60, 61, 63, and 64

Test method 5

Each 1 mg of the test compound is dissolved in 50 μL of a mixed solution of polyoxyethylene sorbitan monococoate and acetone (5 : 95 (volume ratio)). Water containing 0.03% by volume of Shindain (registered trademark) is added thereto to prepare a diluted solution containing the test compound at a predetermined concentration.
Young seedlings of maize (Zea mays) are immersed in the diluted solution for 30 seconds. Thereafter, the two fruits are placed in a petri dish (90 mm diameter), and thereto are released 10 second instar larvae of Western corn rootworm *(Diabrotica virgifera virgifera*). After 5 days, the number of dead insects is counted, and the mortality is determined from the following equation.

Mortality (%) = (number of dead insects/10) × 100

Test Example 5-1

As a result of performing a test according to test method 5 using the following compound of the present invention as a test compound at a predetermined concentration of 200 ppm, the following present compounds exhibited a mortality of 80% or more.
Present compound: 52

Test method 6

An acetone solution prepared such that the amount of the test compound is 800 ppm is poured into a container having an internal volume of 50 mL, and the inner surface of the container is uniformly coated such that the amount of the test compound is 40 mg/$m^2$, and then dried.
Five male imagoes of Blattella germanica are placed in the container, and the lid is closed. After a lapse of a predetermined time, the state of Blattella germanica is examined, and the mortality is determined. The mortality is

calculated by the following equation.

Mortality (%) = (number of dead insects/number of tested insects) × 100

Test Example 6-1

The results of the test performed according to Test method 6 are shown below. As a result of setting the predetermined time to 3 days and using the following present compound as a test compound, the following present compound showed a mortality of 80% or more.
Present compound: 23

Test method 7

An acetone solution prepared such that the amount of the test compound is 2000 ppm is poured into a container having an internal volume of 20 mL, and the inner surface of the container is uniformly coated such that the amount of the test compound is 100 mg/$m^2$, and then dried.
Five nymphs of Haemaphysalis longicornis are placed in the container, and the lid is closed. After a lapse of a predetermined time, the state of Haemaphysalis longicornis is examined to determine the mortality. The mortality is calculated by the following equation.

Mortality (%) = (number of dead ticks/number of tested ticks) × 100

Test Example 7-1

The results of the test performed according to Test method 7 are shown below. As a result of setting the predetermined time to 2 days and using the following present compound as a test compound, the following present compound showed a Mortality of 80% or more.
Present compound: 45

Test method 8

An acetone solution prepared such that the amount of the test compound is 800 ppm is poured into a container having an internal volume of 20 mL, and the inner surface of the container is uniformly coated such that the amount of the test compound is 40 mg/$m^2$, and then dried.
Five adult female Musca domestica are placed in the container, and the lid is closed. After a lapse of a predetermined time, the state of Musca domestica is examined, and the mortality is determined. The mortality is calculated by the following equation.

Mortality (%) = (number of dead insects/number of tested insects) × 100

Test Example 8-1

The results of the test performed according to Test method 8 are shown below. As a result of setting the predetermined time to 1 days and using the following present compound as a test compound, the following present compound all showed a mortality of 80% or more.
Present compound: 6, 16, and 52

Test method 9

A test compound is formed into a formulation according to the method described in Formulation Example 5, and water containing 0.03% by volume of Shindain (registered trademark) is added thereto to prepare a diluted solution containing the test compound at a predetermined concentration.
A cabbage (Brassicae oleracea) seedling (3 to 4 true leaf stage) planted in a container is sprayed with the diluted solution at a rate of 20 mL/seedling. Thereafter, 10 third instar larvae of common cutworm (Spodoptera litura) are released. After 6 days, the number of surviving insects is counted, and the mortality is determined from the following equation.

Mortality (%) = (1 - number of surviving insects/10) × 100

Test Example 9-1

As a result of performing a test according to test method 9 using the following compound of the present invention as a test compound at a predetermined concentration of 200 ppm, the following present compounds exhibited a mortality of 80% or more.
Present compound: 23

Test Example 9-2

The insecticidal effect on Spodoptera litura can be confirmed by performing a test according to Test method 9 using the present compound as a test compound at a predetermined concentration of 50 ppm.

Test method 10

A test compound is formed into a formulation according to the method described in Formulation Example 5, and water containing 0.03% by volume of Shindain (registered trademark) is added thereto to prepare a diluted solution containing the test compound at a predetermined concentration.
A cabbage (Brassicae oleracea) seedling (3 to 4 true leaf stage) planted in a container is sprayed with the diluted solution at a rate of 20 mL/seedling. Thereafter, 10 third instar larvae of diamondback moth *(Plutella xylostella)* are released. After 6 days, the number of surviving insects is counted, and the mortality is determined from the following equation.

Mortality (%) = (1 - number of surviving insects/10) × 100

Test Example 10-1

As a result of performing a test according to test method 10 using the following compound of the present invention as a test compound at a predetermined concentration of 200 ppm, the following present compounds all exhibited a mortality of 80% or more.
Present compound: 3, 6, 7, 10, 27, 36, 40, 41, 42, 43, 44, 47, 48, and 52

Test Example 10-2

As a result of performing a test according to test method 10 using the following compound of the present invention as a test compound at a predetermined concentration of 50 ppm, the following present compounds all exhibited a mortality of 80% or more.
Present compound: 7, 36, 40, and 52

Test method 11

A test compound is formed into a formulation according to the method described in Formulation Example 5, and water containing 0.03% by volume of Shindain (registered trademark) is added thereto to prepare a diluted solution containing the test compound at a predetermined concentration.
A cucumber (Cucumis sativus) seedling (second true leaf stage) planted in a container is inoculated with about 30 cotton aphids *(Aphis gossypii)* (whole stage). One day later, the diluted solution is sprayed onto the seedlings at a rate of 10 mL/seedling. After 5 days, the number of surviving insects is examined, and the control value is determined by the following formula.

Controlling value (%) = {1 - (Cb × Tai)/(Cai × Tb)} × 100

Characters in the formula represent the following meanings.
Cb: number of test insects in a non-treated section
Cai: number of surviving insects in a non-treated section at the time of investigation
Tb: number of test insects in a treated section
Tai: number of surviving insects in a treated section at the time of investigation

Herein, the non-treated section means a section that is subjected to the same operation as that of the treated section except that the test compound is not used.

Test Example 11-1

As a result of performing a test according to test method 11 using the following compound of the present invention as a test compound at a predetermined concentration of 200 ppm, the following present compounds all exhibited a control value of 90% or more.
Present compound: 2, 3, 4, 5, 6, 7, 8, 9, 10, 11, 13, 15, 16, 17, 18, 19, 23, 24, 25, 27, 29, 36, 39, 40, 41, 42, 43, 44, 45, 46, 47, 48, 51, 52, 53, 56, 57, 67, and 71

Test Example 11-2

As a result of performing a test according to test method 11 using the following compound of the present invention as a test compound at a predetermined concentration of 50 ppm, the following present compounds all exhibited a control value of 90% or more.
Present compound: 3, 4, 5, 6, 7, 8, 9, 10, 11, 18, 19, 27, 36, 39, 40, 41, 42, 43, 44, 45, 46, 47, 48, 52, 53, 56, 67

Test method 12

The test compound is formulated according to the method described in Formulation Example 1, and water is added thereto to prepare a diluted solution containing the test compound at a predetermined concentration.
30 last-instar larvae of Culex pipiens pallens are released into the diluted solution, and the state of the larvae of Culex pipiens is examined one day later to determine the Mortality. The mortality is calculated by the following equation.

Mortality (%) = (number of dead insects/number of tested insects) $\times$ 100

Test Example 12-1

As a result of performing a test according to test method 12 using the following compound of the present invention as a test compound at a predetermined concentration of 3.5 ppm, the following present compounds all exhibited a Mortality more than 90%.
Present compound: 3, 5, 6, 7, 8, 9, 10, 11, 13, 16, 19, 23, 24, 27, 39, 40, 42, 44, 45, 46, 48, 52, 55, 57, and 67

Test method 13

1 mg of the present compound is dissolved in 10 μL of a mixed solution of xylene : DMF : surfactant = 4 : 4 : 1 (volume ratio), and diluted with water containing 0.02% by volume of an adjuvant to prepare a diluted solution A containing the present compound at a predetermined concentration.
1 mg of the present component is dissolved in 10 μL of a mixed solution of xylene : DMF : surfactant = 4 : 4 : 1 (volume ratio), and diluted with water containing 0.02% by volume of an adjuvant to prepare a diluted solution B containing the present component at a predetermined concentration.
The diluted solution A and diluted solution B are mixed to provide a diluted solution C.
Leaf pieces of cucumber cotyledons (length 1.5 cm) are accommodated in each well of a 24 well microplate, 2 aphis gossypii and 8 larvae are released per well, and 20 pL of the diluted solution C is sprayed per well. This is defined as a treated section.
A well to which 20 μL of water containing 0.02% by volume of an adjuvant is sprayed instead of the diluted solution C is defined as a non-treated section.
After the diluted solution C is dried, the upper portion of the microplate is covered with a film sheet. After 5 days, the number of surviving insects in each well is examined.
The Controlling value is calculated from the following equation.

$$\text{Controlling value (\%)} = \{1 - (Tai)/(Cai)\} \times 100$$

Symbols in the equation represent the following meanings.

Cai: number of surviving insects in a non-treated section at the time of investigation
Tai: number of surviving insects in a treated section at the time of investigation

Specific diluted solutions C in which effects can be confirmed by Test method 13 are shown in the following 1) to 5).

1) Diluted solution C having a concentration of the present compound of 200 ppm and a concentration of the present component of 2000 ppm in the combination shown in the list A. In the list A, Comp X means any one compound selected from the compound groups SX1 to SX1908.
List A:

Comp X + Clothianidin; Comp X + Thiamethoxam; Comp X + Imidacloprid; Comp X + Thiacloprid; Comp **X** + Flupyradifurone; Comp **X** + Sulfoxaflor; Comp **X** + Triflumizole; Comp X + Diclodimezothiaz; Comp X + Beta-Cyfluthrin; Comp X + Tefluthrin; Comp X + Fipronil; Comp X + Chlorantraniliprole; Comp X + Cyantraniliprole; Comp X + Tetraniliprole; Comp X + Thiodicarb; Comp X + Carbofuran; Comp X + Flufenoxuron; Comp X + Afoxolaner; Comp X + Fluralaner; Comp X + Broflanilide; Comp X + Abamectin; Comp X + Fluopiram; Comp X + Fluensulfone; Comp X + Fluazinamidrazine; Comp X + Thioxazafen; Comp X + Flupyrimin; Comp X + Mycorrhizal Fungi; Comp X + Bradyrhizobium japonicum strain TA-11; Comp X + Bacillus firmus; Comp X + Bacillus firmus strain I-1582; Comp X + Bacillus amyloliquefaciens; Comp X + Bacillus amyloliquefaciens strain FZB42; Comp X + Bacillus amyloliquefaciens strain PTA4838; Comp X + Pasteuria nishizawae; Comp X + Pasteuria nishizawae strain Pn1; Comp X + Pasteuria penetrans; Comp X + Tebconazole; Comp X + Prothioconazole; Comp X + Metconazole; Comp X + Ipconazole; Comp X + Tricyclazole; Comp X + Difenoconazole; Comp X + Imazalil; Comp X + Triazmenol; Comp X + Tetraconazole; Comp X + Flutriafol; Comp X + Mandestrobin; Comp X + Azoxystrobin; Comp X + Pyraclostrobin; Comp X + Trifloxystrobin; Comp X + Fluoxastrobin; Comp X + Picoxystrobin; Comp X + Fenamidone; Comp X + Metalaxyl; Comp X + Metalaxyl-M; Comp X + Fluquioxonil; Comp X + Sedaxane; Comp X + Penflufen; Comp X + Fluxapyroxad; Comp X + Benzovindiflupyr; Comp X + Boscalid; Comp X + Carboxin; Comp X + Pencycuron; Comp X + Flutolanil; Comp X + Captan; Comp X + Thiram; Comp X + Tolclofos-methyl; Comp X + Thiabendazole; Comp X + Ethaboxam; Comp X + Mancozeb; Comp X + Picarbutilazole; Comp X + Oxathiapiprolin; Comp X + Silthiofam; and
Comp X + Flupyradifurone.

2) Diluted solution C having a concentration of the present compound of 200 ppm and a concentration of the present component of 200 ppm in the combination shown in the list A.
3) Diluted solution C having a concentration of the present compound of 500 ppm and a concentration of the present component of 50 ppm in the combination shown in the list A.
4) Diluted solution C having a concentration of the present compound of 500 ppm and a concentration of the present component of 5 ppm in the combination shown in the list A.
5) Diluted solution C having a concentration of the present compound of 500 ppm and a concentration of the present component of 0.5 ppm in the combination shown in the list A.

INDUSTRIAL APPLICABILITY

The present compound exhibits an excellent control effect on harmful arthropod.

## Claims

**1.** A compound represented by the formula (I)

[Formula 1]

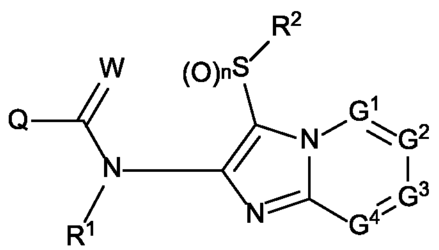

(I)

[wherein:

W represents an oxygen atom or a sulfur atom;

$R^1$ represents a C1-C6 chain hydrocarbon group optionally substituted with one or more substituent(s) selected from Group D, a C3-C7 cycloalkyl group optionally substituted with one or more substituent(s) selected from Group E, a phenyl group optionally substituted with one or more substituent(s) selected from Group F, a 5- or 6-membered aromatic heterocyclic group optionally substituted with one or more substituent(s) selected from Group F, $C(O)R^4$, $C(O)OR^4$, or $C(O)NR^4R^5$;

$R^4$ and $R^5$ are identical to or different from each other, and each represents a C1-C6 chain hydrocarbon group optionally substituted with one or more halogen atom(s), a phenyl group optionally substituted with one or more substituent(s) selected from Group F, a 5- or 6-membered heterocyclic group optionally substituted with one or more substituent(s) selected from Group F, or a hydrogen atom;

$R^2$ represents a C1-C6 alkyl group optionally substituted with one or more halogen atom(s), a cyclopropyl group, a cyclopropylmethyl group, or $NR^6R^7$;

$R^6$ and $R^7$ are identical to or different from each other, and each represents a C1-C6 alkyl group optionally substituted with one or more halogen atom(s), a C3-C6 cycloalkyl group optionally substituted with one or more halogen atom(s), or a hydrogen atom, or

$R^6$ and $R^7$ may be combined with the nitrogen atom to which they are attached to form an aziridinyl group, an azetidinyl group, a pyrrolidinyl group, or a piperidyl group;

n represents 0, 1, or 2;

$G^1$ represents a nitrogen atom or $CR^{3a}$;

$G^2$ represents a nitrogen atom or $CR^{3b}$;

$G^3$ represents a nitrogen atom or $CR^{3c}$;

$G^4$ represents a nitrogen atom or $CR^{3d}$;

$R^{3a}$, $R^{3b}$, $R^{3c}$, and $R^{3d}$ are identical to or different from each other, and each represents a C1-C6 chain hydrocarbon group optionally substituted with one or more substituent(s) selected from Group H, a C3-C7 cycloalkyl group optionally substituted with one or more substituent(s) selected from Group J, a phenyl group optionally substituted with one or more substituent(s) selected from Group K, a 5- or 6-membered aromatic heterocyclic group optionally substituted with one or more substituent (s) selected from Group K, $OR^8$, $NR^8R^9$, $NR^{8a}R^{9a}$, $NR^{10}NR^8R^9$, $NR^{10}OR^9$, $NR^9C(O)R^{11}$, $NR^{10}NR^9C(O)R^{11}$, $NR^9C(O)OR^{12}$, $NR^{10}NR^9C(O)OR^{12}$, $NR^9C(O)NR^{13}R^{14}$, $NR^{10}NR^9C(O)NR^{13}R^{14}$, $N{=}CHNR^{13}R^{14}$, $N{=}S(O)_pR^{15}R^{16}$, $C(O)R^{11}$, $C(O)OR^{17}$, $C(O)NR^{13}R^{14}$, $C(O)NR^9S(O)_2R^{18}$, $CR^{19}{=}NOR^{17}$, $NR^9CR^{10}{=}NOR^{17}$, $S(O)_mR^{18}$, a cyano group, a nitro group, a hydrogen atom, or a halogen atom;

p represents 0 or 1;

m represents 0, 1, or 2;

$R^8$ represents a C1-C6 chain hydrocarbon group optionally substituted with one or more substituent(s) selected from Group L, a C3-C7 cycloalkyl group optionally substituted with one or more substituent(s) selected from Group M, a C3-C7 cycloalkenyl group optionally substituted with one or more substituent(s) selected from Group M, a phenyl group optionally substituted with one or more substituent(s) selected from Group $J^2$, a 6-membered aromatic heterocyclic group optionally substituted with one or more substituent(s) selected from Group $J^2$, a hydrogen atom, or $S(O)_2R^{18}$;

$R^{11}$ represents a C1-C6 chain hydrocarbon group optionally substituted with one or more halogen atom(s), a C3-C7 cycloalkyl group optionally substituted with one or more halogen atom(s), a (C3-C6 cycloalkyl)C1-C3 alkyl group optionally substituted with one or more halogen atom(s), a phenyl group optionally substituted with one or more substituent(s) selected from Group $J^2$, a 5- or 6-membered aromatic heterocyclic group optionally substituted with one or more substituent(s) selected from Group $J^2$, or a hydrogen atom;

$R^{12}$ represents a C1-C6 chain hydrocarbon group optionally substituted with one or more halogen atom(s), a C3-C7 cycloalkyl group optionally substituted with one or more halogen atom(s), a (C3-C6 cycloalkyl)C1-C3 alkyl group optionally substituted with one or more halogen atom(s), or a phenyl C1-C3 alkyl group {wherein the phenyl portion of said phenyl C1-C3 alkyl group may be substituted with one or more substituent(s) selected from Group $J^2$};

$R^{13}$ represents a C1-C6 alkyl group optionally substituted with one or more halogen atom(s), or a hydrogen atom;

$R^{14}$ represents a C1-C6 chain hydrocarbon group optionally substituted with one or more substituent(s) selected from Group L, a C3-C7 cycloalkyl group optionally substituted with one or more substituent(s) selected from Group M, $S(O)_2R^{18}$, or a hydrogen atom;

$R^{15}$ and $R^{16}$ are identical to or different from each other, and each represents a C1-C6 alkyl group optionally substituted with one or more halogen atom(s);

$R^{17}$ represents a C1-C6 chain hydrocarbon group optionally substituted with one or more halogen atom(s), a

phenyl group optionally substituted with one or more substituent(s) selected from Group $J^2$, or a hydrogen atom; $R^{18}$ represents a C1-C6 chain hydrocarbon group optionally substituted with one or more halogen atom(s), or a phenyl group optionally substituted with one or more substituent(s) selected from Group $J^2$;
$R^{19}$ represents a C1-C6 chain hydrocarbon group optionally substituted with one or more halogen atom(s), a halogen atom, $OR^{20}$, $NR^{21}R^{22}$, or a hydrogen atom;
$R^{20}$ represents a C1-C6 chain hydrocarbon group optionally substituted with one or more halogen atom(s);
$R^9$, $R^{10}$, $R^{21}$, and $R^{22}$ are identical to or different from each other, and each represents a C1-C6 chain hydrocarbon group optionally substituted with one or more halogen atom(s), or a hydrogen atom;
$R^{8a}$ and $R^{9a}$ are combined with the nitrogen atom to which they are attached to form a 3-7 membered nonaromatic heterocyclic group optionally substituted with one or more substituent(s) selected from Group P};
Q represents $Q^1$ or $Q^2$;

[Formula 2]

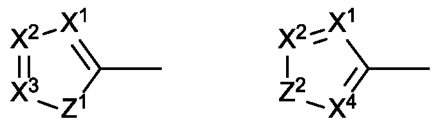

$Q^1$ $Q^2$

wherein,
$Z^1$ represents $NR^{3j}$, an oxygen atom, or a sulfur atom;
$Z^2$ represents $NR^{3k}$, an oxygen atom, or a sulfur atom;
when Q is $Q^1$ and $Z^1$ represents $NR^{3j}$, $X^1$ represents a nitrogen atom or $CR^{3e}$, $X^2$ represents a nitrogen atom or $CR^{3f}$, and $X^3$ represents a nitrogen atom or $CR^{3g}$;
when Q is $Q^1$ and $Z^1$ represents an oxygen atom, $X^1$ represents a nitrogen atom or $CR^{3e}$, $X^2$ represents a nitrogen atom or $CR^{3f}$, and $X^3$ represents a nitrogen atom or $CR^{3g}$ (provided that not all of $X^1$, $X^2$, and $X^3$ represent nitrogen atoms);
when Q is $Q^1$ and $Z^1$ represents a sulfur atom, $X^1$ represents a nitrogen atom or $CR^{3e}$, $X^2$ represents a nitrogen atom or $CR^{3f}$, and $X^3$ represents a nitrogen atom or $CR^{3g}$ (provided that not all of $X^1$, $X^2$, and $X^3$ represent nitrogen atoms);
when Q is $Q^2$ and $Z^2$ represents $NR^{3k}$, $X^1$ represents a nitrogen atom or $CR^{3e}$, $X^2$ represents a nitrogen atom or $CR^{3f}$, and $X^4$ represents a nitrogen atom or $CR^{3h}$;
when Q is $Q^2$ and $Z^2$ represents an oxygen atom, $X^1$ represents a nitrogen atom or $CR^{3e}$, $X^2$ represents a nitrogen atom or $CR^{3f}$, and $X^4$ represents a nitrogen atom or $CR^{3h}$ (provided that not all of $X^1$, $X^2$, and $X^4$ represent nitrogen atoms) ;
when Q is $Q^2$ and $Z^2$ represents a sulfur atom, $X^1$ represents a nitrogen atom or $CR^{3e}$, $X^2$ represents a nitrogen atom or $CR^{3f}$, and $X^4$ represents a nitrogen atom or $CR^{3h}$ (provided that not all of $X^1$, $X^2$, and $X^4$ represent nitrogen atoms);
$R^{3j}$ represents a methyl group optionally substituted with one or more substituent(s) selected from Group T, a C2-C4 chain hydrocarbon group optionally substituted with one or more substituent(s) selected from Group U, a C5-C6 chain hydrocarbon group optionally substituted with one or more substituent(s) selected from Group V, a C3-C7 cycloalkyl group optionally substituted with one or more substituent(s) selected from Group $K^2$, a phenyl group optionally substituted with one or more substituent(s) selected from Group X, a 5- or 6-membered aromatic heterocyclic group optionally substituted with one or more substituent(s) selected from Group X, $C(O)R^{23}$, $C(O)OR^{24}$, $C(O)NR^{25}R^{26}$, $C(O)NR^{27}S(O)_2R^{28}$, $CR^{29}$=$NOR^{24}$, or $S(O)_kR^{28}$;
k represents 0, 1, or 2;
$R^{23}$ represents a C1-C6 chain hydrocarbon group optionally substituted with one or more halogen atom(s), a C3-C7 cycloalkyl group optionally substituted with one or more halogen atom(s), a (C3-C6 cycloalkyl)C1-C3 alkyl group optionally substituted with one or more halogen atom(s), a phenyl group optionally substituted with one or more substituent(s) selected from Group X, a 5- or 6-membered aromatic heterocyclic group optionally substituted with one or more substituent(s) selected from Group X, or a hydrogen atom;
$R^{24}$ represents a C1-C6 chain hydrocarbon group optionally substituted with one or more halogen atom(s), a phenyl group optionally substituted with one or more substituent(s) selected from Group X, or a hydrogen atom;
$R^{25}$ represents a C1-C6 alkyl group optionally substituted with one or more halogen atom(s), or a hydrogen atom;

$R^{26}$ represents a C1-C6 chain hydrocarbon group optionally substituted with one or more substituent(s) selected from Group V, a C3-C7 cycloalkyl group optionally substituted with one or more substituent(s) selected from Group $K^2$, $S(O)_vR^{28}$, or a hydrogen atom;
v represents 0, 1, or 2;
$R^{28}$ represents a C1-C6 chain hydrocarbon group optionally substituted with one or more halogen atom(s), or a phenyl group optionally substituted with one or more substituent(s) selected from Group X;
$R^{29}$ represents a C1-C6 chain hydrocarbon group optionally substituted with one or more halogen atom(s), a halogen atom, $OR^{30}$, $NR^{31}R^{32}$, or a hydrogen atom;
$R^{30}$ represents a C1-C6 chain hydrocarbon group optionally substituted with one or more halogen atom(s);
$R^{27}$, $R^{31}$, and $R^{32}$ are identical to or different from each other, and each represents a C1-C6 chain hydrocarbon group optionally substituted with one or more halogen atom(s), or a hydrogen atom;
$R^{3k}$ represents a C1-C6 chain hydrocarbon group optionally substituted with one or more substituent(s) selected from Group Y, **a** C3-C7 cycloalkyl group optionally substituted with one or more substituent(s) selected from Group $M^2$, a phenyl group optionally substituted with one or more substituent(s) selected from Group $A^2$, a 5- or 6-membered aromatic heterocyclic group optionally substituted with one or more substituent(s) selected from Group $A^2$, $C(O)R^{52}$, $C(O)OR^{53}$, $C(O)NR^{54}R^{55}$, $C(O)NR^{56}S(O)_2R^{57}$, $CR^{58}=NOR^{53}$, $S(O)_tR^{57}$, or a hydrogen atom;
t represents 0, 1, or 2;
$R^{52}$ represents a C1-C6 chain hydrocarbon group optionally substituted with one or more halogen atom(s), a C3-C7 cycloalkyl group optionally substituted with one or more halogen atom(s), a (C3-C6 cycloalkyl)C1-C3 alkyl group optionally substituted with one or more halogen atom(s), a phenyl group optionally substituted with one or more substituent(s) selected from Group $A^2$, a 5- or 6-membered aromatic heterocyclic group optionally substituted with one or more substituent(s) selected from Group $A^2$, or a hydrogen atom;
$R^{53}$ represents a C1-C6 chain hydrocarbon group optionally substituted with one or more halogen atom(s), a phenyl group optionally substituted with one or more substituent(s) selected from Group $A^2$, or a hydrogen atom;
$R^{54}$ represents a C1-C6 alkyl group optionally substituted with one or more halogen atom(s), or a hydrogen atom;
$R^{55}$ represents a C1-C6 chain hydrocarbon group optionally substituted with one or more substituent(s) selected from Group Y, a C3-C7 cycloalkyl group optionally substituted with one or more substituent(s) selected from Group $M^2$, $S(O)_yR^{28}$, or a hydrogen atom;
y represents 0, 1, or 2;
$R^{57}$ represents a C1-C6 chain hydrocarbon group optionally substituted with one or more halogen atom(s), or a phenyl group optionally substituted with one or more substituent(s) selected from Group $A^2$;
$R^{58}$ represents a C1-C6 chain hydrocarbon group optionally substituted with one or more halogen atom(s), a halogen atom, $OR^{59}$, $NR^{60}R^{61}$, or a hydrogen atom;
$R^{59}$ represents a C1-C6 chain hydrocarbon group optionally substituted with one or more halogen atom(s);
$R^{56}$, $R^{60}$, and $R^{61}$ are identical to or different from each other, and each represents a C1-C6 chain hydrocarbon group optionally substituted with one or more halogen atom(s), or a hydrogen atom;
$R^{3e}$, $R^{3f}$, $R^{3g}$, and $R^{3h}$ are identical to or different from each other, and each represents a C1-C6 chain hydrocarbon group optionally substituted with one or more substituent(s) selected from Group $B^2$, a C3-C7 cycloalkyl group optionally substituted with one or more substituent(s) selected from Group $D^2$, a phenyl group optionally substituted with one or more substituent(s) selected from Group $E^2$, a 5- or 6-membered aromatic heterocyclic group optionally substituted with one or more substituent (s) selected from Group $E^2$, $OR^{33}$, $NR^{33}R^{34}$, $NR^{33a}R^{34a}$, $NR^{35}NR^{33}R^{34}$, $NR^{35}OR^{34}$, $NR^{34}C(O)R^{36}$, $NR^{35}NR^{34}C(O)R^{36}$, $NR^{34}C(O)OR^{37}$, $NR^{35}NR^{34}C(O)OR^{37}$, $NR^{34}C(O)NR^{38}R^{39}$, $NR^{35}NR^{34}C(O)NR^{38}R^{39}$, $N=CHNR^{38}R^{39}$, $N=S(O)_qR^{40}R^{41}$, $C(O)R^{36}$, $C(O)OR^{42}$, $C(O)NR^{38}R^{39}$, $C(O)NR^{34}S(O)_2R^{43}$, $CR^{44}=NOR^{42}$, $NR^{34}CR^{35}=NOR^{42}$, $S(O)_rR^{43}$, a cyano group, a nitro group, a halogen atom, or a hydrogen atom;
q represents 0 or 1;
r represents 0, 1, or 2;
$R^{33}$ represents a C1-C6 chain hydrocarbon group optionally substituted with one or more substituent(s) selected from Group $B^2$, a C3-C7 cycloalkyl group optionally substituted with one or more substituent(s) selected from Group $D^2$, a C3-C7 cycloalkenyl group optionally substituted with one or more substituent(s) selected from Group $D^2$, a phenyl group optionally substituted with one or more substituent(s) selected from Group $E^2$, a 6-membered aromatic heterocyclic group optionally substituted with one or more substituent(s) selected from Group $E^2$, a hydrogen atom, or $S(O)_2R^{43}$;
$R^{36}$ represents a C1-C6 chain hydrocarbon group optionally substituted with one or more halogen atom(s), a C3-C7 cycloalkyl group optionally substituted with one or more halogen atom(s), a (C3-C6 cycloalkyl)C1-C3 alkyl group optionally substituted with one or more halogen atom(s), a phenyl group optionally substituted with one or more substituent(s) selected from Group $E^2$, a 5- or 6-membered aromatic heterocyclic group optionally

substituted with one or more substituent(s) selected from Group $E^2$, or a hydrogen atom;

$R^{37}$ represents a C1-C6 chain hydrocarbon group optionally substituted with one or more halogen atom(s), a C3-C7 cycloalkyl group optionally substituted with one or more halogen atom(s), a (C3-C6 cycloalkyl)C1-C3 alkyl group, or a phenyl C1-C3 alkyl group {wherein the phenyl portion of said phenyl C1-C3 alkyl group may be substituted with one or more substituent(s) selected from Group $E^2$};

$R^{38}$ represents a C1-C6 alkyl group optionally substituted with one or more halogen atom(s), or a hydrogen atom;

$R^{39}$ represents a C1-C6 chain hydrocarbon group optionally substituted with one or more substituent(s) selected from Group $B^2$, a C3-C7 cycloalkyl group optionally substituted with one or more substituent(s) selected from Group $D^2$, $S(O)_2R^{43}$, or a hydrogen atom;

$R^{40}$ and $R^{41}$ are identical to or different from each other, and each represents a C1-C6 alkyl group optionally substituted with one or more halogen atom(s);

$R^{42}$ represents a C1-C6 chain hydrocarbon group optionally substituted with one or more halogen atom(s), a phenyl group optionally substituted with one or more substituent(s) selected from Group $E^2$, or a hydrogen atom;

$R^{43}$ represents a C1-C6 chain hydrocarbon group optionally substituted with one or more halogen atom(s), or a phenyl group optionally substituted with one or more substituent(s) selected from Group $E^2$;

$R^{44}$ represents a C1-C6 chain hydrocarbon group optionally substituted with one or more halogen atom(s), a halogen atom, $OR^{45}$, $NR^{46}R^{47}$, or a hydrogen atom;

$R^{45}$ represents a C1-C6 chain hydrocarbon group optionally substituted with one or more halogen atom(s);

$R^{34}$, $R^{35}$, $R^{46}$, and $R^{47}$ are identical to or different from each other, and each represents a C1-C6 chain hydrocarbon group optionally substituted with one or more halogen atom(s), or a hydrogen atom;

$R^{33a}$ and $R^{34a}$ are combined with the nitrogen atom to which they are attached to form a 3-7 membered nonaromatic heterocyclic group optionally substituted with one or more substituent(s) selected from Group $F^2$;

Group D: a group consisting of a C3-C6 cycloalkyl group optionally substituted with one or more substituent(s) selected from the group consisting of halogen atoms and C1-C3 alkyl groups, a C1-C6 alkoxy group optionally substituted with one or more halogen atom(s), a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), a C2-C6 alkylcarbonyl group optionally substituted with one or more halogen atom(s), a C2-C6 alkoxycarbonyl group optionally substituted with one or more halogen atom(s), a phenyl group optionally substituted with one or more substituent(s) selected from Group $L^2$, a 5- or 6-membered aromatic heterocyclic group optionally substituted with one or more substituent(s) selected from Group $L^2$, a hydroxy group, a cyano group, and a halogen atom;

Group E: a group consisting of a C1-C6 alkyl group optionally substituted with one or more halogen atom(s), a **C1-C6 alkoxy group optionally substituted with one or more** halogen atom(s), a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), and a halogen atom;

Group F: a group consisting of a C1-C6 alkyl group optionally substituted with one or more halogen atom(s), a C1-C6 alkoxy group optionally substituted with one or more halogen atom(s), a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), a C1-C6 alkylamino group optionally substituted with one or more halogen atom(s), a di(C1-C6 alkyl)amino group optionally substituted with one or more halogen atom(s), a C2-C6 alkylcarbonyl group optionally substituted with one or more halogen atom(s), a C2-C6 alkoxycarbonyl group optionally substituted with one or more halogen atom(s), a hydroxy group, a sulfanyl group, an amino group, a cyano group, a nitro group, and a halogen atom;

Group H: a group consisting of a C1-C6 alkoxy group optionally substituted with one or more halogen atom(s), a C3-C6 alkenyloxy group optionally substituted with one or more halogen atom(s), a C3-C6 alkynyloxy group optionally substituted with one or more halogen atom(s), a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), a C3-C6 cycloalkyl group optionally substituted with one or more halogen atom(s), a cyano group, a hydroxy group, and a halogen atom;

Group J: a group consisting of a C1-C6 chain hydrocarbon group optionally substituted with one or more halogen atom(s), a C1-C6 alkoxy group optionally substituted with one or more halogen atom(s), a C3-C6 alkenyloxy group optionally substituted with one or more halogen atom(s), a C3-C6 alkynyloxy group optionally substituted with one or more halogen atom(s), a halogen atom, an oxo group, a hydroxy group, a cyano group, and a nitro group;

Group K: a group consisting of a C1-C6 alkyl group optionally substituted with one or more halogen atom(s), a 5- or 6-membered aromatic heterocyclic group optionally substituted with one or more substituent(s) selected from

Group $J^2$, $OR^{49}$, $NR^{48}R^{49}$, $C(O)R^{49}$, $C(O)NR^{48}R^{49}$, $OC(O)R^{48}$, $OC(O)OR^{48}$, $NR^{49}C(O)R^{48}$, $NR^{49}C(O)OR^{48}$, $C(O)OR^{49}$, a halogen atom, a nitro group, a cyano group, and an amino group;

$R^{48}$ represents a C1-C6 alkyl group optionally **substituted with one or more halogen atom(s), or a C3-C6 cycloalkyl group optionally substituted with one or more halogen atom(s),**

$R^{49}$ represents a C1-C6 alkyl group optionally substituted with one or more halogen atom(s), a C3-C6 cycloalkyl group optionally substituted with one or more halogen atom(s), or a hydrogen atom;

Group L: a group consisting of a C1-C6 alkoxy group optionally substituted with one or more halogen atom(s), a phenyl group optionally substituted with one or more substituent(s) selected from Group $J^2$, a 5- or 6-membered aromatic heterocyclic group optionally substituted with one or more substituent(s) selected from Group $J^2$, a C3-C7 cycloalkyl group optionally substituted with one or more halogen atom(s), a 3-7 membered nonaromatic heterocyclic group optionally substituted with one or more substituent(s) selected from Group $H^2$, an amino group, $NHR^{50}$, $NR^{58}R^{51}$, a halogen atom, and a cyano group;

$R^{50}$ and $R^{51}$ are identical to or different from each other, and each represents a C1-C6 alkyl group optionally substituted with one or more halogen atom(s);

Group M: a group consisting of a C1-C6 alkyl group optionally substituted with one or more halogen atom(s), a halogen atom, and a cyano group;

Group P: a group consisting of a C1-C6 chain hydrocarbon group optionally substituted with one or more halogen atom(s), a C1-C6 alkoxy group optionally substituted with one or more halogen atom(s), a C3-C6 alkenyloxy group optionally substituted with one or more halogen atom(s), a C3-C6 alkynyloxy group optionally substituted with one or more halogen atom(s), a halogen atom, an oxo group, a hydroxy group, a cyano group, and a nitro group;

Group T: a group consisting of a C3-C6 cycloalkyl group optionally substituted with one or more substituent(s) selected from the group consisting of fluorine atoms and C1-C3 alkyl groups, a C1-C6 alkoxy group optionally substituted with one or more halogen atom(s), a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), a hydroxy group, a sulfanyl group, and a halogen atom;

Group U: a group consisting of a C3-C6 cycloalkyl group optionally substituted with one or more substituent(s) selected from the group consisting of fluorine atoms and C1-C3 alkyl groups, a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), a sulfanyl group, and a fluorine atom;

Group V: a group consisting of a C3-C6 cycloalkyl group optionally substituted with one or more substituent(s) selected from the group consisting of halogen atoms and C1-C3 alkyl groups, a C1-C6 alkoxy group optionally substituted with one or more halogen atom(s), a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), a C2-C6 alkylcarbonyl group optionally substituted with one or more halogen atom(s), a C2-C6 alkoxycarbonyl group optionally substituted with one or more halogen atom(s), a hydroxy group, a sulfanyl group, a cyano group, and a halogen atom;

Group X: a group consisting of a C1-C6 alkyl group optionally substituted with one or more halogen atom(s), a C1-C6 alkoxy group optionally substituted with one or more halogen atom(s), a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), a C1-C6 alkylamino group optionally substituted with one or more halogen atom(s), a di(C1-C6 alkyl)amino group optionally substituted with one or more halogen atom(s), a C2-C6 alkylcarbonyl group optionally substituted with one or more halogen atom(s), a C2-C6 alkoxycarbonyl group optionally substituted with one or more halogen atom(s), a hydroxy group, a sulfanyl group, an amino group, a cyano group, a nitro group, and a halogen atom;

Group Y: a group consisting of a C3-C6 cycloalkyl group optionally substituted with one or more substituent(s) selected from the group consisting of halogen atoms and C1-C3 alkyl groups, a C1-C6 alkoxy group optionally substituted with one or more halogen atom(s), a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), a C2-C6 alkylcarbonyl group optionally substituted with one or more halogen atom(s), a C2-C6 alkoxycarbonyl group optionally substituted with one or more halogen atom(s), a hydroxy group, a sulfanyl group, a cyano group, and a halogen atom;

Group $A^2$: a group consisting of a C1-C6 alkyl group optionally substituted with one or more halogen atom(s), a C1-C6 alkoxy group optionally substituted with one or more halogen atom(s), a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen

atom(s), a C1-C6 alkylamino group optionally substituted with one or more halogen atom(s), a di(C1-C6 alkyl) amino group optionally substituted with one or more halogen atom(s), a C2-C6 alkylcarbonyl group optionally substituted with one or more halogen atom(s), a C2-C6 alkoxycarbonyl group optionally substituted with one or more halogen atom(s), a hydroxy group, a sulfanyl group, an amino group, a cyano group, a nitro group, and a halogen atom;

Group $B^2$: a group consisting of a C3-C6 cycloalkyl group optionally substituted with one or more substituent(s) selected from the group consisting of halogen atoms and C1-C3 alkyl groups, a C1-C6 alkoxy group optionally substituted with one or more halogen atom(s), a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one **or more halogen atom(s), a C2-C6 alkylcarbonyl group** optionally substituted with one or more halogen atom(s), a C2-C6 alkoxycarbonyl group optionally substituted with one or more halogen atom(s), a hydroxy group, a sulfanyl group, a cyano group, and a halogen atom;

Group $D^2$: a group consisting of a C1-C6 alkyl group optionally substituted with one or more halogen atom(s), a C1-C6 alkoxy group optionally substituted with one or more halogen atom(s), a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), and a halogen atom;

Group $E^2$: a group consisting of a C1-C6 alkyl group optionally substituted with one or more halogen atom(s), a C1-C6 alkoxy group optionally substituted with one or more halogen atom(s), a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), a C1-C6 alkylamino group optionally substituted with one or more halogen atom(s), a di(C1-C6 alkyl) amino group optionally substituted with one or more halogen atom(s), a C2-C6 alkylcarbonyl group optionally substituted with one or more halogen atom(s), a C2-C6 alkoxycarbonyl group optionally substituted with one or more halogen atom(s), a hydroxy group, a sulfanyl group, an amino group, a cyano group, a nitro group, and a halogen atom;

Group $F^2$: a group consisting of a C1-C6 chain hydrocarbon group optionally substituted with one or more halogen atom(s), a C1-C6 alkoxy group optionally substituted with one or more halogen atom(s), a C3-C6 alkenyloxy group optionally substituted with one or more halogen atom(s), a C3-C6 alkynyloxy group optionally substituted with one or more halogen atom(s), a halogen atom, an oxo group, a hydroxy group, a cyano group, and a nitro group;

Group $H^2$: a group consisting of a C1-C6 chain hydrocarbon group optionally substituted with one or more halogen atom(s), a C1-C6 alkoxy group optionally substituted with one or more halogen atom(s), a C3-C6 alkenyloxy group optionally substituted with one or more halogen atom(s), a C3-C6 alkynyloxy group optionally substituted with one or more halogen atom(s), and a halogen atom;

Group $J^2$: a group consisting of a C1-C6 chain hydrocarbon group optionally substituted with one or more halogen atom(s), a hydroxy group, a C1-C6 alkoxy group optionally substituted with one or more halogen atom(s), a C3-C6 alkenyloxy group optionally substituted with one or more halogen atom(s), a C3-C6 alkynyloxy group optionally substituted with one or more halogen atom(s), a sulfanyl group, a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), an amino group, $NHR^{50}$, $NR^{50}R^{51}$, $C(O)R^{50}$, $OC(O)R^{50}$, $C(O)OR^{50}$, a cyano group, a nitro group, and a halogen atom;

Group $K^2$: a group consisting of a C1-C6 alkyl group optionally substituted with one or more halogen atom(s), a C1-C6 alkoxy group optionally substituted with one or more halogen atom(s), a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), and a fluorine atom;

Group $L^2$: a group consisting of a C1-C6 alkyl group optionally substituted with one or more halogen atom(s), a C1-C6 alkoxy group optionally substituted with one or more halogen atom(s), a C1-C6 alkylsulfanyl group optionally substituted with one or more halogen atom(s), a C1-C6 alkylsulfinyl group optionally substituted with one or more halogen atom(s), a C1-C6 alkylsulfonyl group optionally substituted with one or more halogen atom(s), a C1-C6 alkylamino group optionally substituted with one or more halogen atom(s), a di(C1-C6 alkyl)amino optionally substituted with one or more halogen atom(s) group, a C2-C6 alkylcarbonyl group optionally substituted with one or more halogen atom(s), a C2-C6 alkoxycarbonyl group optionally substituted with one or more halogen atom(s), a hydroxy group, a sulfanyl group, an amino group, a cyano group, a nitro group, and a halogen atom;

Group $M^2$: a group consisting of a C1-C6 alkyl group optionally substituted with one or more halogen atom(s), a C1-C6 alkoxy group optionally substituted with one or more halogen atom(s), a C1-C6 alkylsulfanyl group optionally substituted with one or more halogen atom(s), a C1-C6 alkylsulfinyl group optionally substituted with

one or more halogen atom(s), a C1-C6 alkylsulfonyl group optionally substituted with one or more halogen atom(s), and a halogen atom]
or an N-oxide thereof.

**2.** The compound according to claim 1 or an N-oxide thereof, wherein $G^1$ is $CR^{3a}$, $G^2$ is $CR^{3b}$, $G^3$ is $CR^{3c}$ and $G^4$ is a nitrogen atom or $CR^{3d}$.

**3.** The compound according to claim 1 or an N-oxide thereof, wherein $G^1$ is $CR^{3a}$, $G^2$ is $CR^{3b}$, $G^3$ is $CR^{3c}$ and $G^4$ is $CR^{3d}$.

**4.** The compound according to any one of claims 1 to 3 or an N-oxide thereof, wherein Q is $Q^1$, in $Q^1$, a combination of $Z^1$, $X^1$, $X^2$ and $X^3$ is

a combination where $Z^1$ is an oxygen atom, $X^1$ is a nitrogen atom, $X^2$ is $CR^{3f}$, and $X^3$ is $CR^{3g}$;
a combination where $Z^1$ is an oxygen atom, $X^1$ is $CR^{3e}$, $X^2$ is a nitrogen atom, and $X^3$ is $CR^{3g}$;
a combination where $Z^1$ is an oxygen atom, $X^1$ is $CR^{3e}$, $X^2$ is $CR^{3f}$, and $X^3$ is a nitrogen atom;
a combination where $Z^1$ is a sulfur atom, $X^1$ is $CR^{3e}$, $X^2$ is a nitrogen atom, and $X^3$ is $CR^{3g}$;
a combination where $Z^1$ is a sulfur atom, $X^1$ is a nitrogen atom, $X^2$ is $CR^{3f}$, and $X^3$ is $CR^{3g}$;
a combination where $Z^1$ is $NR^{3j}$, $X^1$ is a nitrogen atom, $X^2$ is $CR^{3f}$, and $X^3$ is $CR^{3g}$;
a combination where $Z^1$ is $NR^{3j}$, $X^1$ is $CR^{3e}$, $X^2$ is a nitrogen atom, and $X^3$ is $CR^{3g}$; or
a combination where $Z^1$ is $NR^{3j}$, $X^1$ is $CR^{3e}$, $X^2$ is $CR^{3f}$, and $X^3$ is a nitrogen atom.

**5.** The compound according to any one of claims 1 to 3 or an N-oxide thereof, wherein Q is $Q^2$, in $Q^2$, a combination of $Z^2$, $X^1$, $X^2$ and $X^4$ is

a combination where $Z^2$ is an oxygen atom, $X^1$ is a nitrogen atom, $X^2$ is $CR^{3f}$, and $X^4$ is $CR^{3h}$;
a combination where $Z^2$ is an oxygen atom, $X^1$ is $CR^{3e}$, $X^2$ is a nitrogen atom, and $X^4$ is $CR^{3h}$;
a combination where $Z^2$ is an oxygen atom, $X^1$ is $CR^{3e}$, $X^2$ is $CR^{3f}$, and $X^4$ is a nitrogen atom;
a combination where $Z^2$ is $NR^{3k}$, $X^1$ is a nitrogen atom, $X^2$ is $CR^{3f}$, and $X^4$ is $CR^{3h}$;
a combination where $Z^2$ is $NR^{3k}$, $X^1$ is $CR^{3e}$, $X^2$ is a nitrogen atom, and $X^4$ is $CR^{3h}$;
a combination where $Z^2$ is $NR^{3k}$, $X^1$ is $CR^{3e}$, $X^2$ is $CR^{3f}$, and $X^4$ is a nitrogen atom;
a combination where $Z^2$ is $NR^{3k}$, $X^1$ is $CR^{3e}$, $X^2$ is $CR^{3f}$, and $X^4$ is $CR^{3h}$; or
a combination where $Z^2$ is $NR^{3k}$, $X^1$ is a nitrogen atom, $X^2$ is a nitrogen atom, and $X^4$ is $CR^{3h}$.

**6.** The compound according to any one of claims 1 to 5 or an N-oxide thereof, wherein $R^2$ is an ethyl group.

**7.** The compound according to any one of claims 1 to 6 or an N-oxide thereof, wherein W is an oxygen atom.

**8.** A composition for controlling a harmful arthropod comprising the compound according to any one of claims 1 to 7 or an N-oxide thereof.

**9.** A composition comprising one or more ingredients selected from the group consisting of the following Groups (a), (b), (c) and (d), and the compound according to any one of claims 1 to 7 or an N-oxide thereof:

Group (a): a group consisting of insecticidal ingredients, miticidal ingredients, and nematicidal ingredients;
Group (b): fungicidal ingredients;
Group (c): plant growth modulating ingredients; and
Group (d): repellent ingredients.

**10.** A method for controlling a harmful arthropod, which comprises applying an effective amount of the compound according to any one of claims 1 to 7 or an N-oxide thereof, or an effective amount of the composition according to claim 9 to a harmful arthropod or a habitat where the harmful arthropod lives.

**11.** A seed or vegetative reproductive organ carrying an effective amount of the compound according to any one of claims 1 to 7 or an N-oxide thereof or an effective amount of the composition according to claim 9.

**12.** A compound represented by formula (II)

[Formula 3]

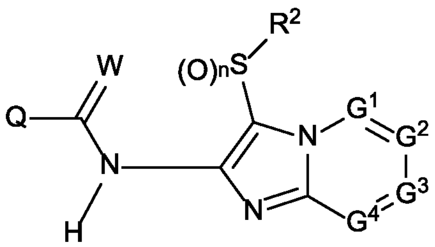

( II )

[wherein the symbols have the same meanings as defined in claim 1] or a salt thereof.

**INTERNATIONAL SEARCH REPORT**

International application No.
**PCT/JP2023/035679**

**A. CLASSIFICATION OF SUBJECT MATTER**

***C07D 471/04***(2006.01)i; ***A01N 43/90***(2006.01)i; ***A01P 7/02***(2006.01)i; ***A01P 7/04***(2006.01)i; ***A61K 31/437***(2006.01)i; ***A61P 33/14***(2006.01)i
FI: C07D471/04 108E; A01P7/02; A01P7/04; A01N43/90 103; A61P33/14; A61K31/437; C07D471/04 CSP

According to International Patent Classification (IPC) or to both national classification and IPC

**B. FIELDS SEARCHED**

Minimum documentation searched (classification system followed by classification symbols)

C07D471/04; A01N43/90; A01P7/02; A01P7/04; A61K31/437; A61P33/14

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| Y | WO 2022/071434 A1 (SUMITOMO CHEMICAL CO., LTD.) 07 April 2022 (2022-04-07) claim 1, production examples, reference production examples | 1-12 |
| Y | JP 2022-069685 A (SUMITOMO CHEMICAL CO., LTD.) 11 May 2022 (2022-05-11) claim 1, production examples, reference production examples | 1-12 |
| Y | JP 2020-519603 A (FMC CORP.) 02 July 2020 (2020-07-02) claim 1, paragraphs [0037], [0169] | 1-12 |
| Y | JP 2018-024660 A (NISSAN CHEM. IND., LTD.) 15 February 2018 (2018-02-15) claim 1, paragraphs [0251], [0252] | 1-12 |
| A | JP 2020-189891 A (SUMITOMO CHEMICAL CO., LTD.) 26 November 2020 (2020-11-26) entire text | 1-12 |
| A | JP 2018-177759 A (NISSAN CHEM. CORP.) 15 November 2018 (2018-11-15) entire text | 1-12 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **28 November 2023** | **19 December 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br>Telephone No. |

Form PCT/ISA/210 (second sheet) (January 2015)

INTERNATIONAL SEARCH REPORT

International application No.
PCT/JP2023/035679

C. DOCUMENTS CONSIDERED TO BE RELEVANT

| Category* | Citation of document, with indication, where appropriate, of the relevant passages | Relevant to claim No. |
|---|---|---|
| A | WO 2016/129684 A1 (NISSAN CHEM. IND., LTD.) 18 August 2016 (2016-08-18) entire text | 1-12 |
| A | WO 2022/071428 A1 (SUMITOMO CHEMICAL CO., LTD.) 07 April 2022 (2022-04-07) entire text | 1-12 |
| A | WO 2020/203763 A1 (SUMITOMO CHEMICAL CO., LTD.) 08 October 2020 (2020-10-08) entire text | 1-12 |
| A | WO 2020/251013 A1 (AGRO-KANESHO CO., LTD.) 17 December 2020 (2020-12-17) entire text | 1-12 |
| A | WO 2015/117912 A1 (SYNGENTA PARTICIPATIONS AG) 13 August 2015 (2015-08-13) entire text | 1-12 |

Form PCT/ISA/210 (second sheet) (January 2015)

**INTERNATIONAL SEARCH REPORT**
**Information on patent family members**

International application No.
**PCT/JP2023/035679**

| Patent document cited in search report | Publication date (day/month/year) | Patent family member(s) | Publication date (day/month/year) |
|---|---|---|---|
| WO 2022/071434 A1 | 07 April 2022 | CN 116456818 A<br>claim 1, production examples, reference production examples | |
| JP 2022-069685 A | 11 May 2022 | (Family: none) | |
| JP 2020-519603 A | 02 July 2020 | US 2020/0093133 A1<br>claim 1<br>WO 2018/208595 A1<br>EP 3621964 A1<br>KR 10-2020-0004385 A<br>CN 110869370 A | |
| JP 2018-024660 A | 15 February 2018 | (Family: none) | |
| JP 2020-189891 A | 26 November 2020 | (Family: none) | |
| JP 2018-177759 A | 15 November 2018 | TW 201819374 A<br>entire text<br>AR 109314 A | |
| WO 2016/129684 A1 | 18 August 2016 | US 2018/0022760 A1<br>entire text<br>US 2019/0375765 A1<br>US 2021/0017194 A1<br>EP 3257853 A1<br>CN 107207506 A<br>KR 10-2017-0117079 A | |
| WO 2022/071428 A1 | 07 April 2022 | CN 116456817 A<br>entire text | |
| WO 2020/203763 A1 | 08 October 2020 | US 2022/0217979 A1<br>entire text<br>EP 3949708 A1<br>KR 10-2021-0143201 A<br>CN 113905607 A | |
| WO 2020/251013 A1 | 17 December 2020 | US 2022/0330550 A1<br>entire text<br>EP 3985004 A1<br>CN 114008045 A<br>KR 10-2022-0024013 A | |
| WO 2015/117912 A1 | 13 August 2015 | (Family: none) | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2022158626 A
- WO 2016129684 A
- US 20180009778 A
- WO 2016121970 A
- WO 2015157093 A
- WO 2016109706 A
- WO 2018052136 A

### Non-patent literature cited in the description

- *Organic & Biomolecular Chemistry*, 2017, vol. 15, 4199
- *Europian Journal of Medicinal Chemistry*, 2016, vol. 123, 916